(19) Europäisches Patentamt
European Patent Office
Office européen des brevets

(11) **EP 3 039 174 B1**

(12) **EUROPEAN PATENT SPECIFICATION**

(45) Date of publication and mention
of the grant of the patent:
**16.10.2019 Bulletin 2019/42**

(51) Int Cl.:
*C12Q 1/6886* (2018.01)     *C12Q 1/6883* (2018.01)

(21) Application number: **14840036.9**

(22) Date of filing: **28.08.2014**

(86) International application number:
**PCT/US2014/053306**

(87) International publication number:
**WO 2015/031694 (05.03.2015 Gazette 2015/09)**

(54) **OLIGONUCLEOTIDE PROBES AND USES THEREOF**

OLIGONUKLEOTIDSONDEN UND VERWENDUNGEN DAVON

SONDES OLIGONUCLÉOTIDIQUES ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2013 US 201361871107 P
06.09.2013 US 201361874621 P
06.11.2013 US 201361900975 P
05.12.2013 US 201361912471 P
06.01.2014 US 201461924192 P
06.03.2014 US 201461949216 P
03.04.2014 US 201461974949 P
07.05.2014 US 201461990085 P
16.05.2014 US 201461994704 P
14.07.2014 US 201462024436 P**

(43) Date of publication of application:
**06.07.2016 Bulletin 2016/27**

(73) Proprietor: **Caris Science, Inc.
Irving, TX 75039 (US)**

(72) Inventors:
• **SPETZLER, David**
**Paradise Valley, AZ 85253 (US)**
• **DOMENYUK, Valeriy**
**Phoenix, AZ 85042 (US)**
• **XIAO, Nianqing**
**Rockville, MD 20850 (US)**
• **STARK, Adam**
**Phoenix, AZ 85040 (US)**
• **ZHONG, Zhenyu**
**Peoria, AZ 85383 (US)**

(74) Representative: **Patent Boutique LLP
10A Printing House Yard
Hackney Road
London E2 7PR (GB)**

(56) References cited:
**WO-A1-2011/066589     WO-A2-2010/056337
WO-A2-2011/088226     WO-A2-2013/022995**

• **TERESA JANAS ET AL: "The selection of aptamers specific for membrane molecular targets", CELLULAR & MOLECULAR BIOLOGY LETTERS, vol. 16, no. 1, 1 March 2011 (2011-03-01), pages 25-39, XP055001923, ISSN: 1425-8153, DOI: 10.2478/s11658-010-0023-3**
• **HENNING ULRICH ET AL: "Disease-specific biomarker discovery by aptamers", CYTOMETRY PART A, vol. 75A, no. 9, 1 September 2009 (2009-09-01), pages 727-733, XP055032998, ISSN: 1552-4922, DOI: 10.1002/cyto.a.20766**
• **MAO YE ET AL: "Generating Aptamers by Cell-SELEX for Applications in Molecular Medicine", INTERNATIONAL JOURNAL OF MOLECULAR SCIENCES, vol. 13, no. 12, 12 March 2012 (2012-03-12), pages 3341-3353, XP055241055, DOI: 10.3390/ijms13033341**
• **SEFAH ET AL.: 'DEVELOPMENT OF DNA APTAMERS USING CELL -SELEX' NAT PROTOC vol. 5, no. 6, 2010, pages 1169 - 1185, XP055257422**
• **TROY ET AL.: 'Understanding barriers to Borrelia burgdorferi dissemination during infection using massively parallel sequencing.' INFECT IMMUN vol. 81, no. 7, July 2013, pages 2347 - 2357, XP055257424**

Remarks:
The complete document including Reference Tables and the Sequence Listing can be downloaded from the EPO website

**Description**

**BACKGROUND**

The disclosure relates generally to the field of aptamers capable of binding to microvesicle surface antigens, which are useful as therapeutics in and diagnostics of cancer and/or other diseases or disorders in which microvesicles implicated. The disclosure further relates to materials and methods for the administration of aptamers capable of binding to microvesicles. The microvesicles may be derived from cells indicative of cancer, e.g., a breast cancer.

Aptamers are multi-meric nucleic acid molecules having specific binding affinity to molecules, which may be through interactions other than classic Watson-Crick base pairing. The terms aptamer, oligonucleotide, polynucleotide, or the like may be used interchangeably herein.

Aptamers, like peptides generated by phage display or monoclonal antibodies ("mAbs"), are capable of specifically binding to selected targets and modulating the target's activity, e.g., through binding aptamers may block their target's ability to function. Created by an in vitro selection process from pools of random sequence oligonucleotides, aptamers have been generated for over 100 proteins including growth factors, transcription factors, enzymes, immunoglobulins, and receptors. A typical aptamer is 10-15 kDa in size (30-45 nucleotides), binds its target with sub-nanomolar affinity, and discriminates against closely related targets (e.g., aptamers will typically not bind other proteins from the same gene family). A series of structural studies have shown that aptamers are capable of using the same types of binding interactions (e.g., hydrogen bonding, electrostatic complementarity, hydrophobic contacts, steric exclusion) that drive affinity and specificity in antibody-antigen complexes.

Aptamers have a number of desirable characteristics for use as therapeutics and diagnostics including high specificity and affinity, biological efficacy, and excellent pharmacokinetic properties. In addition, they offer specific competitive advantages over antibodies and other protein biologies, for example:

Speed and control. Aptamers are produced by an entirely in vitro process, allowing for the rapid generation of initial leads, including therapeutic leads. In vitro selection allows the specificity and affinity of the aptamer to be tightly controlled and allows the generation of leads, including leads against both toxic and non-immunogenic targets.

Toxicity and Immunogenicity. Aptamers as a class have demonstrated little or no toxicity or immunogenicity. In chronic dosing of rats or woodchucks with high levels of aptamer (10 mg/kg daily for 90 days), no toxicity is observed by any clinical, cellular, or biochemical measure. Whereas the efficacy of many monoclonal antibodies can be severely limited by immune response to antibodies themselves, it is extremely difficult to elicit antibodies to aptamers most likely because aptamers cannot be presented by T-cells via the MHC and the immune response is generally trained not to recognize nucleic acid fragments.

Administration. Whereas most currently approved antibody therapeutics are administered by intravenous infusion (typically over 2-4 hours), aptamers can be administered by subcutaneous injection (aptamer bioavailability via subcutaneous administration is >80% in monkey studies (Tucker et al., J. Chromatography B. 732: 203-212, 1999)). This difference is primarily due to the comparatively low solubility and thus large volumes necessary for most therapeutic mAbs. With good solubility (>150 mg/mL) and comparatively low molecular weight (aptamer: 10-50 kDa; antibody: 150 kDa), a weekly dose of aptamer may be delivered by injection in a volume of less than 0.5 mL. In addition, the small size of aptamers allows them to penetrate into areas of conformational constrictions that do not allow for antibodies or antibody fragments to penetrate, presenting yet another advantage of aptamer-based therapeutics or prophylaxis.

Scalability and cost. Aptamers are chemically synthesized and are readily scaled as needed to meet production demand for diagnostic or therapeutic applications. Whereas difficulties in scaling production are currently limiting the availability of some biologics and the capital cost of a large-scale protein production plant is enormous, a single large-scale oligonucleotide synthesizer can produce upwards of 100 kg/year and requires a relatively modest initial investment. The current cost of goods for aptamer synthesis at the kilogram scale is estimated at $100/g, comparable to that for highly optimized antibodies.

Stability. Aptamers are chemically robust. They are intrinsically adapted to regain activity following exposure to factors such as heat and denaturants and can be stored for extended periods (>1 yr) at room temperature as lyophilized powders.

WO 2010/056337 A2 (CARIS MPI) discloses a set of aptamers with the capacity of characterizing a disease in a sample of microvesicles derived from a patient's sample (e.g. paragraphs [0731] - [0769]).

**SUMMARY OF THE INVENTION**

Compositions and methods provide aptamers that bind biomarkers of interest such as microvesicle surface antigens or functional fragments of microvesicle surface antigens. Aptamers may be used in diagnostic, prognostic or theranostic processes to screen a biological sample for the presence or levels of microvesicle surface antigens determined to provide a diagnostic readout. The diagnosis may be related to a cancer, e.g., a breast cancer. Aptamers may be chemically modified or composed in a pharmaceutical composition for therapeutic applications.

In an aspect, the disclosure provides an oligonucleotide at least 50, 55, 60, 65, 70, 75, 80, 85, 90, 95, 96, 97, 98, 99 or 100 percent homologous to SEQ ID NO. 10558. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NO. 10558.

In another aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, or 500000 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to a sequence selected from SEQ ID NOs. 10559-510558. In a related aspect, the disclosure provides an oligonucleotide probe library comprising at least 50%, 60%, 70%, 80%, 90%, 95%, 96%, 97%, 98% or at least 99% of the oligonucleotides listed in SEQ ID NOs. 10559-510558.

In still another aspect, the disclosure provides an oligonucleotide comprising a nucleic acid sequence or a portion thereof that is at least 50, 55, 60, 65, 70, 75, 80, 85, 90, 95, 96, 97, 98, 99 or 100 percent homologous to a sequence selected from SEQ ID NOs. 510559-510578. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NOs. 510559-510578.

In yet another aspect, the disclosure provides an oligonucleotide comprising a nucleic acid sequence or a portion thereof that is at least 50, 55, 60, 65, 70, 75, 80, 85, 90, 95, 96, 97, 98, 99 or 100 percent homologous to a sequence selected from SEQ ID NOs. 510579-510598. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NOs. 510579-510598.

In an aspect, the disclosure provides an oligonucleotide comprising a nucleic acid sequence or a portion thereof that is at least 50, 55, 60, 65, 70, 75, 80, 85, 90, 95, 96, 97, 98, 99 or 100 percent homologous to a sequence selected from SEQ ID NOs. 510599-510763. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 110, 120, 125, 130, 140, 150, 160 or 165 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NOs. 510599-510763. In still another related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 110, 120, 125, 130, 140, 150, 160 or 165 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to the SEQ ID NOs. in a row in **Table 16.**

The nucleic acid sequences disclosed herein can also be modified as desired so long as the functional aspects are still maintained (e.g., binding to various targets or ability to characterize a phenotype). Such a modification may be referred to as a "functional modification" herein. A functional modification may enhance or have minimal or no effect on functional aspects of an oligonucleotide. For example, the oligonucleotides may comprise DNA or RNA, incorporate various non-natural nucleotides, incorporate other chemical modifications, or comprise various deletions or insertions. Such modifications may facilitate synthesis, stability, delivery, labeling, etc, or may have little to no effect in practice. In some cases, some nucleotides in an oligonucleotide may be substituted while maintaining functional aspects of the oligonucleotide. Similarly, 5' and 3' flanking regions may be substituted. In still other cases, only a portion of an oligonucleotide may be determined to direct its functionality such that other portions can be deleted or substituted. Numerous techniques to synthesize and modify nucleotides and polynucleotides, including various chemical modifications, are disclosed herein or are known in the art.

In an aspect, the disclosure also provides a method comprising contacting an oligonucleotide or plurality of oligonucleotides with a sample and detecting the presence or level of binding of the oligonucleotide or plurality of oligonucleotides to a target in the sample, wherein the oligonucleotide or plurality of oligonucleotides can be those provided by the disclosure above. The sample may comprise a biological sample, an organic sample, an inorganic sample, a tissue, a cell culture, a bodily fluid, blood, serum, a cell, a microvesicle, a protein complex, a lipid complex, a carbohydrate, or any combination, fraction or variation thereof. The target may comprise a cell, an organelle, a protein complex, a lipoprotein, a carbohydrate, a microvesicle, a membrane fragment, a small molecule, a heavy metal, a toxin, or a drug.

In a related aspect, the disclosure provides a method comprising: a) contacting a biological sample comprising microvesicles with an oligonucleotide probe library, wherein optionally the oligonucleotide probe library comprises an oligonucleotide or plurality of oligonucleotides those provided above; b) identifying oligonucleotides bound to at least a portion of the microvesicles; and c) characterizing the sample based on a profile of the identified oligonucleotides.

In another aspect, the disclosure provides a method comprising: a) contacting a sample with an oligonucleotide probe library comprising at least $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences oligonucleotides to form a mixture in solution, wherein the oligonucleotides are capable of binding a plurality of entities in the sample to form complexes, wherein optionally the oligonucleotide probe library comprises an oligonucleotide or plurality of oligonucleotides as disclosed above; b) partitioning the complexes formed in step (a) from the mixture; and c) detecting oligonucleotides present in the complexes partitioned in step (b) to identify an oligonucleotide profile for the sample. In an embodiment, the detecting step comprises performing sequencing of all or some of the oligonucleotides in the complexes, amplification of all or some of the oligonucleotides in the complexes, and/or hybridization of all or some of the oligonucleotides in the complexes to an array. The array can be any useful array, such as a planar or particle-based array.

In still another aspect, the disclosure provides a method for generating an enriched oligonucleotide probe library comprising: a) contacting a first oligonucleotide library with a biological test sample and a biological control sample, wherein complexes are formed between biological entities present in the biological samples and a plurality of oligonucleotides present in the first oligonucleotide library; b) partitioning the complexes formed in step (a) and isolating the oligonucleotides in the complexes to produce a subset of oligonucleotides for each of the biological test sample and biological control sample; c) contacting the subsets of oligonucleotides in (b) with the biological test sample and biological control sample wherein complexes are formed between biological entities present in the biological samples and a second plurality of oligonucleotides present in the subsets of oligonucleotides to generate a second subset group of oligonucleotides; and d) optionally repeating steps b)-c), one, two, three or more times to produce a respective third, fourth, fifth or more subset group of oligonucleotides, thereby producing the enriched oligonucleotide probe library. In a related aspect, the disclosure provides a plurality of oligonucleotides comprising at least 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, or 500000 different oligonucleotide sequences, wherein the plurality results from the method in this paragraph, wherein the library is capable of distinguishing a first phenotype from a second phenotype. The first phenotype may comprise a disease or disorder and the second phenotype may comprise a healthy state; or the first phenotype may comprise a disease or disorder and the second phenotype may comprise a different disease or disorder; or the first phenotype may comprise a stage or progression of a disease or disorder and the second phenotype may comprise a different stage or progression of the same disease or disorder; or the first phenotype may compriss a positive response to a therapy and the second phenotype may comprise a negative response to the same therapy.

In yet another aspect, the invention provides a method of characterizing a disease or disorder, comprising: a) contacting a biological test sample with a plurality of oligonucleotides, wherein the plurality of oligonucleotides comprises 164 different oligonucleotide sequences, and wherein each different member of the plurality of oligonucleotides comprises a different sequence that is any one of SED ID Nos. 510599-510763 or a sequence that is 95 percent homologous to any one of SED ID Nos. 510599-510763 ; b) detecting a presence or level of complexes formed in step (a) between the plurality of oligonucleotides and a target in the biological test sample; and c) comparing the presence or level detected in step (b) to a reference level from a biological control sample, thereby characterizing the disease or disorder. The step of detecting may comprise performing sequencing of all or some of the oligonucleotides in the complexes, amplification of all or some of the oligonucleotides in the complexes, and/or hybridization of all or some of the oligonucleotides in the complexes to an array. The sequencing may be high-throughput or next generation sequencing.

In the methods of the invention, the biological test sample and biological control sample may each comprise a tissue sample, a cell culture, or a biological fluid. In some embodiments, the biological fluid comprises a bodily fluid. Useful bodily fluids within the method of the invention comprise peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood. In some preferred embodiments, the bodily fluid comprises blood, serum or plasma. The biological fluid may comprise microvesicles. In such case, the complexes may be formed between the oligonucleotide or plurality of oligonucleotides and at least one of the microvesicles.

The biological test sample and biological control sample may further comprise isolated microvesicles, wherein optionally the microvesicles are isolated using at least one of chromatography, filtration, ultrafiltration, centrifugation, ultracentrifugation, flow cytometry, affinity capture (e.g., to a planar surface, column or bead), polymer precipitation, and using microfluidics. The vesicles can also be isolated after contact with the oligonucleotide or plurality of oligonucleotides.

In various embodiments of the methods of the invention, the plurality of oligonucleotides binds a polypeptide or fragment thereof. The polypeptide or fragment thereof can be soluble or membrane bound, wherein optionally the membrane comprises a microvesicle membrane. The membrane could also be from a cell or a fragment of a cell of vesicle. In some embodiments, the polypeptide or fragment thereof comprises a biomarker in **Table 3** or **Table 4.** For example, the polypeptide or fragment thereof could be a general vesicle marker such as in **Table 3** or a tissue-related or disease-related marker such as in **Table** 4. The oligonucleotide or plurality of oligonucleotides may bind a microvesicle surface antigen in the biological sample. For example, the oligonucleotide or plurality of oligonucleotides can be enriched from a naive library against microvesicles.

The disease or disorder detected by the methods provided here may comprise any appropriate disease or disorder of interest, including without limitation Breast Cancer, Alzheimer's disease, bronchial asthma, Transitional cell carcinoma of the bladder, Giant cellular osteoblastoclastoma, Brain Tumor, Colorectal adenocarcinoma, Chronic obstructive pulmonary disease (COPD), Squamous cell carcinoma of the cervix, acute myocardial infarction (AMI) / acute heart failure, Chron's Disease, diabetes mellitus type II, Esophageal carcinoma, Squamous cell carcinoma of the larynx, Acute and chronic leukemia of the bone marrow, Lung carcinoma, Malignant lymphoma, Multiple Sclerosis, Ovarian carcinoma, Parkinson disease, Prostate adenocarcinoma, psoriasis, Rheumatoid Arthritis, Renal cell carcinoma, Squamous cell carcinoma of skin, Adenocarcinoma of the stomach, carcinoma of the thyroid gland, Testicular cancer, ulcerative colitis, or Uterine adenocarcinoma.

In some embodiments, the disease or disorder comprises a cancer, a premalignant condition, an inflammatory disease, an immune disease, an autoimmune disease or disorder, a cardiovascular disease or disorder, neurological disease or disorder, infectious disease or pain. The cancer can include without limitation one of acute lymphoblastic leukemia; acute myeloid leukemia; adrenocortical carcinoma; AIDS-related cancers; AIDS-related lymphoma; anal cancer; appendix cancer; astrocytomas; atypical teratoid/rhabdoid tumor; basal cell carcinoma; bladder cancer; brain stem glioma; brain tumor (including brain stem glioma, central nervous system atypical teratoid/rhabdoid tumor, central nervous system embryonal tumors, astrocytomas, craniopharyngioma, ependymoblastoma, ependymoma, medulloblastoma, medulloepithelioma, pineal parenchymal tumors of intermediate differentiation, supratentorial primitive neuroectodermal tumors and pineoblastoma); breast cancer; bronchial tumors; Burkitt lymphoma; cancer of unknown primary site; carcinoid tumor; carcinoma of unknown primary site; central nervous system atypical teratoid/rhabdoid tumor; central nervous system embryonal tumors; cervical cancer; childhood cancers; chordoma; chronic lymphocytic leukemia; chronic myelogenous leukemia; chronic myeloproliferative disorders; colon cancer; colorectal cancer; craniopharyngioma; cutaneous T-cell lymphoma; endocrine pancreas islet cell tumors; endometrial cancer; ependymoblastoma; ependymoma; esophageal cancer; esthesioneuroblastoma; Ewing sarcoma; extracranial germ cell tumor; extragonadal germ cell tumor; extrahepatic bile duct cancer; gallbladder cancer; gastric (stomach) cancer; gastrointestinal carcinoid tumor; gastrointestinal stromal cell tumor; gastrointestinal stromal tumor (GIST); gestational trophoblastic tumor; glioma; hairy cell leukemia; head and neck cancer; heart cancer; Hodgkin lymphoma; hypopharyngeal cancer; intraocular melanoma; islet cell tumors; Kaposi sarcoma; kidney cancer; Langerhans cell histiocytosis; laryngeal cancer; lip cancer; liver cancer; lung cancer; malignant fibrous histiocytoma bone cancer; medulloblastoma; medulloepithelioma; melanoma; Merkel cell carcinoma; Merkel cell skin carcinoma; mesothelioma; metastatic squamous neck cancer with occult primary; mouth cancer; multiple endocrine neoplasia syndromes; multiple myeloma; multiple myeloma/plasma cell neoplasm; mycosis fungoides; myelodysplastic syndromes; myeloproliferative neoplasms; nasal cavity cancer; nasopharyngeal cancer; neuroblastoma; Non-Hodgkin lymphoma; nonmelanoma skin cancer; non-small cell lung cancer; oral cancer; oral cavity cancer; oropharyngeal cancer; osteosarcoma; other brain and spinal cord tumors; ovarian cancer; ovarian epithelial cancer; ovarian germ cell tumor; ovarian low malignant potential tumor; pancreatic cancer; papillomatosis; paranasal sinus cancer; parathyroid cancer; pelvic cancer; penile cancer; pharyngeal cancer; pineal parenchymal tumors of intermediate differentiation; pineoblastoma; pituitary tumor; plasma cell neoplasm/multiple myeloma; pleuropulmonary blastoma; primary central nervous system (CNS) lymphoma; primary hepatocellular liver cancer; prostate cancer; rectal cancer; renal cancer; renal cell (kidney) cancer; renal cell cancer; respiratory tract cancer; retinoblastoma; rhabdomyosarcoma; salivary gland cancer; Sezary syndrome; small cell lung cancer; small intestine cancer; soft tissue sarcoma; squamous cell carcinoma; squamous neck cancer; stomach (gastric) cancer; supratentorial primitive neuroectodermal tumors; T-cell lymphoma; testicular cancer; throat cancer; thymic carcinoma; thymoma; thyroid cancer; transitional cell cancer; transitional cell cancer of the renal pelvis and ureter; trophoblastic tumor; ureter cancer; urethral cancer; uterine cancer; uterine sarcoma; vaginal cancer; vulvar cancer; Waldenström macroglobulinemia; or Wilm's tumor. The premalignant condition can include without limitation Barrett's Esophagus. The autoimmune disease can include without limitation one of inflammatory bowel disease (IBD), Crohn's disease (CD), ulcerative colitis (UC), pelvic inflammation, vasculitis, psoriasis, diabetes, autoimmune hepatitis, multiple sclerosis, myasthenia gravis, Type I diabetes, rheumatoid arthritis, psoriasis, systemic lupus erythematosis (SLE), Hashimoto's Thyroiditis, Grave's disease, Ankylosing Spondylitis Sjogrens Disease, CREST syndrome, Scleroderma, Rheumatic Disease, organ rejection, Primary Sclerosing Cholangitis, or sepsis. The cardiovascular disease can include without limitation one of atherosclerosis, congestive heart failure, vulnerable plaque, stroke, ischemia, high blood pressure, stenosis, vessel occlusion or a thrombotic event. The neurological disease can include without limitation one of Multiple Sclerosis (MS), Parkinson's Disease (PD), Alzheimer's Disease (AD), schizophrenia, bipolar disorder, depression, autism, Prion Disease, Pick's disease, dementia, Huntington disease (HD), Down's syndrome, cerebrovascular disease, Rasmussen's encephalitis, viral meningitis, neurospsychiatric systemic lupus erythematosus (NPSLE), amyotrophic lateral sclerosis, Creutzfeldt-Jacob disease, Gerstmann-Straussler-Scheinker disease, transmissible spongiform encephalopathy, ischemic reperfusion damage (e.g. stroke), brain trauma, microbial infection, or chronic fatigue syndrome. The pain can include without limitation one of fibromyalgia, chronic neuropathic pain, or peripheral neuropathic pain. The infectious disease can include without limitation one of a bacterial infection, viral infection, yeast infection, Whipple's Disease, Prion Disease, cirrhosis, methicillin-resistant staphylococcus aureus, HIV, HCV, hepatitis, syphilis, meningitis, malaria, tuberculosis, or influenza. One of skill will appreciate that the methods of the invention can be used to assess any number of these or other related diseases and disorders.

In some embodiments of the invention, the methods of use thereof are useful for characterizing certain diseases or disease states. As desired, a pool of oligonucleotides useful for characterizing various diseases is assembled to create a master pool that can be used to probe useful for characterizing the various diseases. For example, the combination of SEQ ID NOs. 510599-510763 provided herein comprise such a pool. One of skill will also appreciate that pools of oligonucleotides useful for characterizing specific diseases or disorders can be created as well. The oligonucleotides and pools thereof can be modified as described herein.

In an embodiment, the disease or disorder comprises a breast cancer and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30 or at least 40 of SEQ ID NOs. 510559-510598. The oligonucleotides may incorporate various chemical modifications, additions, deletions, insertions, substitutions or other modifications so long as functional aspects of the oligonucleotides are enhanced or maintained in whole or in part.

In another embodiment, and the disease or disorder comprises Alzheimer's disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510604, 510605, 510608, 510609, 510612, 510614, 510629, 510632, 510633, 510634, 510641, 510642, 510643, 510646, 510648, 510649, 510651, 510652, 510653, 510655, 510661, 510667, 510673, 510675, 510676, 510677, 510678, 510679, 510681, 510683, 510685, 510687, 510688, 510690, 510694, 510696, 510702,510707, 510709, 510726, 510727, 510728, 510729, 510730, 510731, 510732, 510737, 510740, 510748, 510749, 510751, 510752, 510754, 510756, 510757, 510758, 510761, and 510762. In a related embodiment, the disease or disorder comprises Alzheimer's disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510601, 510603, 510606, 510608, 510609, 510611, 510613, 510614, 510615, 510619, 510621, 510625, 510628, 510629, 510630, 510632, 510634, 510635, 510636, 510637, 510644, 510647, 510648, 510651, 510652, 510654, 510657, 510665, 510666, 510667, 510668, 510677, 510678, 510679, 510687, 510692, 510693, 510696, 510698, 510699, 510701, 510702, 510707, 510708, 510710, 510713, 510716, 510725, 510726, 510728, 510731, 510732, 510733, 510734, 510736, 510741, 510748, 510749, 510755, 510757, 510758, 510761, and 510762. The oligonucleotides may incorporate various chemical modifications, additions, deletions, insertions, substitutions or other modifications so long as functional aspects of the oligonucleotides are enhanced or maintained in whole or in part.

The disease or disorder may comprise bronchial asthma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510614, 510619, 510623, 510627, 510631, 510633, 510635, 510647, 510655, 510656, 510660, 510672, 510689, 510690, 510693, 510698, 510701, 510702, 510707, 510709, 510710, 510713, 510720, 510723, 510724, 510726, 510728, 510729, 510730, 510731, 510734, 510735, 510738, 510743, 510744, 510745, 510746, 510748, 510749, 510750, 510751, 510752, 510754, 510755, 510757, 510758, 510759, 510760, 510761, and 510762. The disease or disorder may also comprise bronchial asthma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510608, 510609, 510610, 510611, 510617, 510619, 510622, 510631, 510632, 510634, 510635, 510637, 510642, 510643, 510644, 510652, 510655, 510657, 510658, 510665, 510668, 510673, 510675, 510676, 510677, 510678, 510679, 510683, 510691, 510701, 510703, 510704, 510706, 510708, 510709, 510714, 510725, 510736, 510737, 510740, 510741, 510742, and 510756. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises a transitional cell carcinoma of the bladder and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510619, 510623, 510631, 510632, 510635, 510641, 510642, 510647, 510656, 510657, 510658, 510659, 510673, 510674, 510683, 510686, 510693, 510695, 510701, 510702, 510707, 510708, 510711, 510716, 510722, 510725, 510726, 510728, 510731, 510734, 510737, 510744, 510748, 510749, 510751, 510752, 510753, 510756, 510757, 510758, 510761, and 510762. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a giant cellular osteoblastoclastoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510612, 510620, 510635, 510637, 510641, 510644, 510648, 510658, 510662, 510663, 510667, 510668, 510670, 510676, 510678, 510679, 510682, 510683, 510685, 510686, 510699, 510708, 510712, 510722, 510737, and 510753. The oligonucleotides may incorporate various chemical modifications, additions, deletions, insertions, substitutions or other modifications so long as functional aspects of the oligonucleotides are enhanced or maintained in whole or in part.

The disease or disorder may comprise a brain tumor and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510619, 510624, 510628, 510639, 510641, 510645, 510647, 510648, 510655, 510657, 510665, 510668, 510673, 510674, 510689, 510695, 510698, 510699, 510705, 510710, 510711, 510712, 510713, 510716, 510734, 510737, 510738, and 510762. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a colorectal adenocarcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510603, 510607, 510611, 510616, 510618, 510619, 510623, 510624, 510641, 510644, 510646, 510647, 510655, 510656, 510672, 510673, 510690, 510693, 510695, 510698, 510701, 510702, 510709, 510711, 510714, 510716, 510719, 510723, 510724, 510725, 510726, 510730, 510731, 510734, 510735, 510737, 510738, 510743, 510744, 510748, 510749, 510751, 510752, 510754, 510755, 510757, 510758, 510760, 510761, 510762, and 510763. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises a chronic obstructive pulmonary disease (COPD) and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510604, 510608, 510609, 510612, 510614, 510616, 510619, 510620, 510629, 510634, 510637, 510640, 510647, 510649, 510653, 510661, 510666, 510667, 510669, 510673, 510678, 510682, 510689, 510690, 510701, 510707, 510715, 510723, 510727, 510728, 510749, 510754, 510755, 510757, 510758, 510762, and 510763. In a related embodiment, the disease or disorder comprises a chronic obstructive pulmonary disease (COPD) and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510609, 510611, 510613, 510620, 510634, 510637, 510647, 510648, 510654, 510664, 510668, 510679, 510694, 510696, 510698, 510699, 510701, 510705, 510706, 510710, 510718, 510734, and 510741. The oligonucleotides may incorporate various chemical modifications, additions, deletions, insertions, substitutions or other modifications so long as functional aspects of the oligonucleotides are enhanced or maintained in whole or in part.

The disease or disorder can be a squamous cell carcinoma of the cervix and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510615, 510619, 510623, 510626, 510630, 510632, 510633, 510635, 510638, 510639, 510641, 510642, 510644, 510647, 510650, 510655, 510656, 510657, 510661, 510673, 510674, 510677, 510683, 510688, 510693, 510695, 510698, 510711, 510712, 510714, 510716, 510717, 510722, 510725, 510729, 510731, 510734, 510737, 510743, 510745, 510747, 510753, 510755, 510756, 510758, 510759, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise an acute myocardial infarction (AMI) or acute heart failure and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510608, 510613, 510626, 510629, 510631, 510634, 510635, 510638, 510639, 510646, 510648, 510656, 510667, 510669, 510671, 510672, 510675, 510682, 510689, 510698, 510701, 510707, 510710, 510715, 510721, 510723, 510727, 510728, 510730, 510731, 510734, 510735, 510743, 510744, 510748, 510749, 510751, 510752, 510757, 510758, 510760, 510761, and 510762. In a related embodiment, the disease or disorder comprises an acute myocardial infarction (AMI) or acute heart failure and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510601, 510606, 510607, 510608, 510609, 510611, 510614, 510616, 510619, 510622, 510624, 510626, 510635, 510636, 510637, 510640, 510641, 510643, 510644, 510648, 510651, 510665, 510668, 510669, 510672, 510675, 510677, 510678, 510679, 510682, 510692, 510695, 510696, 510698, 510699, 510701, 510703, 510707, 510710, 510725, 510726, 510728, 510729, 510730, 510731, 510733, 510734, 510736, 510743, 510750, 510751, 510752, 510755, 510757, 510758, 510759, 510760, 510761, and 510762. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises Chron's Disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510602, 510608, 510611, 510624, 510644, 510653, 510656, 510659, 510669, 510671, 510676, 510686, 510689, 510690, 510697, 510698, 510700, 510713, 510727, 510728, 510729, 510731, 510734, 510744, 510751, and 510757. In a related embodiment, the disease or disorder comprises Chron's Disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510610, 510611, 510615, 510618, 510621, 510623, 510625, 510626, 510628, 510631, 510632, 510635, 510637, 510638, 510643, 510647, 510648, 510649, 510653, 510654, 510655, 510657, 510658, 510666, 510667, 510668, 510672, 510675, 510677, 510678, 510679, 510680, 510682, 510684, 510689, 510691, 510694, 510696, 510698, 510701, 510707, 510708, 510709, 510710, 510713, 510714, 510715, 510719, 510725, 510727, 510728, 510729, 510730, 510731, 510734, 510736, 510738, 510743, 510744, 510748, 510750, 510751, 510755, 510757, 510758, 510759, 510760, 510761, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise diabetes mellitus type II and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510604, 510608, 510610, 510611, 510614, 510616, 510620, 510624, 510629, 510632, 510634, 510640, 510649, 510667, 510669, 510670, 510671, 510678, 510685, 510700, 510701, 510702, 510707, 510709, 510718, 510721, 510723, 510726, 510727, 510728, 510729, 510730, 510731, 510733, 510735, 510743, 510748, 510749, 510752, 510754, 510755, 510757, 510758, 510760, 510761, 510762, and 510763. Relatedly, the disease or disorder may comprise diabetes mellitus type II and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510613, 510632, 510635, 510636, 510641, 510645, 510647, 510648, 510654, 510660, 510664, 510667, 510668, 510670, 510675, 510684, 510691, 510695, 510696, 510706, 510710, 510734, and 510749. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises an esophageal carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510602, 510619, 510623, 510632, 510635, 510638, 510641, 510644, 510653, 510656, 510661, 510671, 510682, 510689, 510693, 510698, 510714, 510722, 510725, 510731, 510734, 510738, 510753, and 510761. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a squamous cell carcinoma of the larynx and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510605, 510607, 510612, 510614, 510619, 510623, 510632, 510635, 510641, 510642, 510655, 510656, 510657, 510659, 510661, 510668, 510673, 510674, 510689, 510690, 510693, 510695, 510698, 510708, 510712, 510732, 510734, 510737, 510738, 510745, 510747, 510753, and 510755. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises an acute or chronic leukemia of the bone marrow and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510605, 510607, 510610, 510612, 510628, 510631, 510633, 510641, 510644, 510650, 510664, 510670, 510673, 510674, 510675, 510681, 510684, 510685, 510686, 510701, 510711, 510712, 510717, 510718, 510719, 510720, 510721, 510724, 510729, 510732, 510739, 510740, 510743, 510745, 510746, 510747, 510752, 510754, and 510763. The oligonucleotides may incorporate various chemical modifications, additions, deletions, insertions, substitutions or other modifications so long as functional aspects of the oligonucleotides are enhanced or maintained in whole or in part.

In yet another embodiment, the disease or disorder comprises a lung carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510604, 510626, 510628, 510631, 510633, 510635, 510649, 510650, 510654, 510668, 510672, 510674, 510677, 510699, 510701, 510702, 510710, 510712, 510715, 510717, 510719, 510720, 510721, 510723, 510724, 510726, 510727, 510733, 510735, 510738, 510743, 510744, 510745, 510746, 510747, 510750, 510751, 510754, 510755, 510758, 510760, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise a malignant lymphoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510611, 510618, 510623, 510624, 510631, 510632, 510636, 510638, 510641, 510644, 510645, 510647, 510656, 510660, 510662, 510672, 510673, 510675, 510690, 510693, 510701, 510702, 510707, 510708, 510713, 510717, 510719, 510721, 510723, 510724, 510725, 510726, 510728, 510729, 510730, 510731, 510734, 510735, 510737, 510743, 510744, 510749, 510751, 510752, 510754, 510755, 510756, 510757, 510758, 510760, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder may also comprise multiple sclerosis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510612, 510620, 510646, 510653, 510655, 510661, 510663, 510669, 510675, 510682, 510685, 510686, 510690, 510699, 510701, 510710, 510713, 510731, 510738, 510747, 510758, and 510762. Relatedly, the disease or disorder may comprise multiple sclerosis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510604, 510609, 510610, 510613, 510617, 510618, 510622, 510632, 510635, 510647, 510670, 510675, 510677, 510687, 510690, 510692, 510695, 510701, 510706, 510708, 510731, and 510733. The oligonucleotides may incorporate various chemical modifications, additions, deletions, insertions, substitutions or other modifications so long as functional aspects of the oligonucleotides are enhanced or maintained in whole or in part.

In an embodiment, the disease or disorder comprises an ovarian carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510618, 510626, 510628, 510633, 510638, 510641, 510642, 510643, 510645, 510646, 510647, 510650, 510652, 510658, 510665, 510666, 510673, 510674, 510677, 510682, 510683, 510689, 510705, 510707, 510712, 510717, 510722, 510724, 510729, 510732, 510735, 510737, 510745, 510746, 510747, 510753, and 510756. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises Parkinson disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510601, 510604, 510608, 510609, 510612, 510614, 510624, 510631, 510633, 510634, 510640, 510641, 510642, 510649, 510650, 510651, 510653, 510667, 510673, 510675, 510676, 510677, 510683, 510686, 510689, 510694, 510700, 510703, 510704, 510705, 510706, 510707, 510709, 510713, 510715, 510721, 510723, 510724, 510726, 510727, 510729, 510730, 510731, 510732, 510734, 510735, 510736, 510737, 510739, 510740, 510741, 510742, 510744, 510745, 510746, 510747, 510748, 510751, 510752, 510754, 510756, 510757, 510758, 510759, 510760, 510761, and 510762. In a related embodiment, the disease or disorder comprises Parkinson disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510606, 510608, 510609, 510610, 510614, 510616, 510632, 510634, 510643, 510644, 510647, 510648, 510654, 510662, 510664, 510665, 510667, 510668, 510670, 510677, 510678, 510679, 510687, 510692, 510696, 510698, 510699, 510701, 510703, 510704, 510706, 510708, 510710, 510722, 510727, 510734, 510736, 510741, 510742, 510753, and 510756. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises a prostate adenocarcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510603, 510607, 510619, 510623, 510624, 510628, 510641, 510642, 510644, 510647, 510650, 510655, 510656, 510657, 510669, 510673, 510674, 510677, 510684, 510688, 510689, 510690, 510695, 510698, 510701, 510709, 510710, 510718, 510726, 510734, 510737, 510738, 510739, 510757, and 510762. The oligonucleotides may incorporate various chemical modifications, additions, deletions, insertions, substitutions or other modifications so long as functional aspects of the oligonucleotides are enhanced or maintained in whole or in part.

In yet another embodiment, the disease or disorder comprises psoriasis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510601, 510608, 510609, 510611, 510614, 510620, 510624, 510626, 510631, 510633, 510634, 510635, 510638, 510647, 510649, 510650, 510653, 510656, 510665, 510666, 510667, 510669, 510672, 510674, 510675, 510680, 510685, 510689, 510698, 510702, 510707, 510711, 510712, 510715, 510717, 510719, 510720, 510721, 510723, 510724, 510726, 510727, 510728, 510729, 510730, 510731, 510734, 510735, 510743, 510744, 510745, 510746, 510747, 510748, 510749, 510750, 510751, 510752, 510754, 510755, 510757, 510758, 510759, 510760, 510761, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder can be psoriasis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510601, 510604, 510613, 510621, 510627, 510637, 510641, 510644, 510648, 510650, 510652, 510663, 510667, 510668, 510676, 510680, 510681, 510699, 510701, 510703, 510705, 510709, 510710, 510722, 510734, 510739, 510750, and 510753. The oligonucleotides may incorporate various chemical modifications, additions, deletions, insertions, substitutions or other modifications so long as functional aspects of the oligonucleotides are enhanced or maintained in whole or in part.

The disease or disorder can also be rheumatoid arthritis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510603, 510604, 510607, 510608, 510609, 510614, 510616, 510622, 510625, 510627, 510629, 510630, 510634, 510635, 510636, 510637, 510640, 510642, 510646, 510649, 510650, 510651, 510653, 510656, 510664, 510665, 510667, 510671, 510675, 510677, 510678, 510679, 510682, 510689, 510699, 510707, 510726, 510727, 510728, 510729, 510731, 510733, 510737, 510738, 510748, 510755, 510758, 510761, and 510762. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises rheumatoid arthritis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510601, 510603, 510604, 510606, 510607, 510608, 510609, 510610, 510611, 510612, 510613, 510614, 510616, 510617, 510618, 510622, 510623, 510625, 510629, 510630, 510634, 510635, 510636, 510637, 510638, 510639, 510640, 510641, 510643, 510644, 510645, 510646, 510648, 510649, 510651, 510652, 510653, 510654, 510658, 510662, 510663, 510664, 510665, 510666, 510667, 510668, 510669, 510671, 510673, 510677, 510678, 510679, 510682, 510685, 510692, 510696, 510697, 510698, 510699, 510701, 510703, 510710, 510716, 510722, 510725, 510726, 510727, 510730, 510733, 510734, 510738, 510741, 510743, 510753, 510755, and 510757. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a renal cell carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510603, 510604, 510606, 510608, 510614, 510616, 510622, 510628, 510629, 510630, 510632, 510634, 510635, 510640, 510644, 510645, 510646, 510652, 510653, 510656, 510664, 510665, 510666, 510667, 510671, 510675, 510677, 510683, 510685, 510687, 510690, 510701, 510722, 510726, 510728, 510729, 510730, 510731, 510732, 510733, 510734, 510738, 510748, 510749, 510752, 510753, 510758, 510761, and 510762. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises a squamous cell carcinoma of skin and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510618, 510622, 510626, 510639, 510641, 510642, 510650, 510658, 510673, 510674, 510683, 510696, 510708, 510712, 510717, 510720, 510722, 510723, 510724, 510727, 510729, 510743, 510745, 510746, 510747, 510752, 510753, 510754, 510755, 510756, 510759, 510761, and 510763. The oligonucleotides may incorporate various chemical modifications, additions, deletions, insertions, substitutions or other modifications so long as functional aspects of the oligonucleotides are enhanced or maintained in whole or in part.

In various embodiments, the disease or disorder comprises an adenocarcinoma of the stomach and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510604, 510608, 510609, 510612, 510626, 510631, 510632, 510634, 510639, 510653, 510658, 510663, 510667, 510681, 510689, 510692, 510693, 510696, 510698, 510699, 510705, 510711, 510715, 510717, 510719, 510723, 510725, 510729, 510731, 510734, 510735, 510736, 510743, 510746, 510748, 510751, 510754, 510757, 510760, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise a carcinoma of the thyroid gland and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510602, 510603, 510614, 510626, 510638, 510641, 510644, 510646, 510653, 510658, 510659, 510661, 510662, 510674, 510689, 510693, 510695, 510698, 510699, 510701, 510704, 510705, 510708, 510717, 510718, 510722, 510727, 510731, 510734, 510736, 510739, 510740, 510741, 510747, 510753, and 510755. The oligonucleotides may incorporate various chemical modifications, additions, deletions, insertions, substitutions or other modifications so long as functional aspects of the oligonucleotides are enhanced or maintained in whole or in part.

The disease or disorder may also comprise a testicular cancer and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510603, 510607, 510615, 510616, 510618, 510619, 510621, 510622, 510623, 510624, 510630, 510636, 510637, 510641, 510644, 510645, 510647, 510650, 510654, 510655, 510656, 510657, 510659, 510662, 510665, 510670, 510673, 510674, 510681, 510684, 510685, 510687, 510688, 510689, 510690, 510692, 510693, 510695, 510696, 510698, 510700, 510701, 510704, 510707, 510712, 510713, 510717, 510718, 510726, 510734, 510737, 510738, 510739, 510740, 510742, 510747, 510756, and 510757. The oligonucleotides may incorporate various chemical modifications, additions, deletions, insertions, substitutions or other modifications so long as functional aspects of the oligonucleotides are enhanced or maintained in whole or in part.

The disease or disorder can be ulcerative colitis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510607, 510611, 510612, 510616, 510620, 510621, 510622, 510624, 510626, 510632, 510633, 510636, 510646, 510655, 510659, 510672, 510673, 510675, 510676, 510677, 510682, 510684, 510691, 510692, 510702, 510703, 510704, 510705, 510706, 510707, 510709, 510710, 510715, 510721, 510723, 510724, 510728, 510729, 510730, 510731, 510735, 510736, 510737, 510739, 510740, 510741, 510743, 510744, 510748, 510751, 510752, 510754, 510757, 510758, 510759, 510760, 510761, and 510762. In a related embodiment, the disease or disorder comprises ulcerative colitis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510610, 510611, 510612, 510619, 510622, 510623, 510634, 510635, 510641, 510647, 510648, 510654, 510657, 510664, 510668, 510670, 510671, 510676, 510677, 510678, 510679, 510689, 510691, 510696, 510701, 510703, 510704, 510710, 510722, 510734, 510742, and 510753. The oligonucleotides may incorporate various chemical modifications, additions, deletions, insertions, substitutions or other modifications so long as functional aspects of the oligonucleotides are enhanced or maintained in whole or in part.

The disease or disorder can also be a uterine adenocarcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510612, 510621, 510626, 510637, 510641, 510644, 510650, 510653, 510669, 510675, 510684, 510686, 510687, 510696, 510714, 510717, 510722, 510739, 510743, 510745, 510746, 510753, 510755, and 510762. The oligonucleotides may incorporate various chemical modifications, additions, deletions, insertions, substitutions or other modifications so long as functional aspects of the oligonucleotides are enhanced or maintained in whole or in part.

In an aspect, the invention provides a kit comprising at least one reagent for carrying out the methods of the invention provided herein, wherein the at least one reagent comprises the plurality of oligonucleotides. In a similar aspect, the invention contemplates use of a reagent for carrying out the methods of the invention provided herein. The plurality of oligonucleotides can be those disclosed herein. The reagent may comprise various other useful components including without limitation one or more of: a) a reagent configured to isolate a microvesicle, optionally wherein the at least one reagent configured to isolate a microvesicle comprises a binding agent to a microvesicle antigen, a column, a substrate, a filtration unit, a polymer, polyethylene glycol, PEG4000, PEG8000, a particle or a bead; b) at least one oligonucleotide configured to act as a primer or probe in order to amplify, sequence, hybridize or detect the oligonucleotide or plurality of oligonucleotides; and c) a reagent configured to remove one or more abundant protein from a sample, wherein optionally the one or more abundant protein comprises at least one of albumin, immunoglobulin, fibrinogen and fibrin.

In another aspect, the disclosure provides an aptamer comprising a nucleic acid sequence that is at least about 50, 55, 60, 65, 70, 75, 80, 85, 90, 95, 96, 97, 98, 99 or 100 percent homologous of any of: a) SEQ ID NOs. 8-21 or a variable sequence thereof as described in **Table 8;** b) SEQ ID NOs. 24-43 or a variable sequence thereof as described in **Table 11;** c) SEQ ID NOs. 44-10527; d) SEQ ID NOs. 10528-10557 or a variable sequence thereof as described in **Table 12;** or e) a functional fragment of any preceding sequence. The aptamer may recognize a microvesicle surface antigen. The aptamers may be identified herein in the form of DNA or RNA. Unless otherwise specified, one of skill in the art will appreciate that an aptamer may generally be synthesized in various forms of nucleic acid. The aptamers may also carry various chemical modifications and remain within the scope of the disclosure.

An aptamer may be modified to comprise at least one chemical modification. The modification may include without limitation a chemical substitution at a sugar position; a chemical substitution at a phosphate position; and a chemical substitution at a base position of the nucleic acid. The modification may be selected from the group consisting of: biotinylation, incorporation of a fluorescent label, incorporation of a modified nucleotide, a 2'-modified pyrimidine, 3' capping, conjugation to an amine linker, conjugation to a high molecular weight, non-immunogenic compound, conjugation to a lipophilic compound, conjugation to a drug, conjugation to a cytotoxic moiety, and labeling with a radioisotope, or other modification as disclosed herein. The position of the modification can be varied as desired. For example, the biotinylation, fluorescent label, or cytotoxic moiety can be conjugated to the 5' end of the aptamer. The biotinylation, fluorescent label, or cytotoxic moiety can also be conjugated to the 3' end of the aptamer.

The cytotoxic moiety may be encapsulated in a nanoparticle. The nanoparticle can be selected from the group consisting of: liposomes, dendrimers, and comb polymers. The cytotoxic moiety may comprise a small molecule cytotoxic moiety. The small molecule cytotoxic moiety can include without limtation vinblastine hydrazide, calicheamicin, vinca alkaloid, a cryptophycin, a tubulysin, dolastatin-10, dolastatin-15, auristatin E, rhizoxin, epothilone B, epithilone D, taxoids, maytansinoids and any variants and derivatives thereof. The cytotoxic moiety may comprise a protein toxin. For example, the protein toxin can be selected from the group consisting of diphtheria toxin, ricin, abrin, gelonin, and Pseudomonas exotoxin A. Non-immunogenic, high molecular weight compounds include polyalkylene glycols, e.g., polyethylene glycol. Appropriate radioisotopes include yttrium-90, indium-111, iodine-131, lutetium-177, copper-67, rhenium-186, rhenium-188, bismuth-212, bismuth-213, astatine-211, and actinium-225. The aptamer may be labeled with a gamma-emitting radioisotope.

An active agent may be conjugated to the aptamer. For example, the active agent may be a therapeutic agent or a diagnostic agent. The therapeutic agent may be selected from the group consisting of tyrosine kinase inhibitors, kinase inhibitors, biologically active agents, biological molecules, radionuclides, adriamycin, ansamycin antibiotics, asparaginase, bleomycin, busulphan, cisplatin, carboplatin, carmustine, capecotabine, chlorambucil, cytarabine, cyclophosphamide, camptothecin, dacarbazine, dactinomycin, daunorubicin, dexrazoxane, docetaxel, doxorubicin, etoposide, epothilones, floxuridine, fludarabine, fluorouracil, gemcitabine, hydroxyurea, idarubicin, ifosfamide, irinotecan, lomustine, mechlorethamine, mercaptopurine, melphalan, methotrexate, rapamycin (sirolimus), mitomycin, mitotane, mitoxantrone, nitrosurea, paclitaxel, pamidronate, pentostatin, plicamycin, procarbazine, rituximab, streptozocin, teniposide, thioguanine, thiotepa, taxanes, vinblastine, vincristine, vinorelbine, taxol, combretastatins, discodermolides, transplatinum, anti-vascular endothelial growth factor compounds ("anti-VEGFs"), anti-epidermal growth factor receptor compounds ("anti-EGFRs"), 5-fluorouracil and derivatives, radionuclides, polypeptide toxins, apoptosis inducers, therapy sensitizers, enzyme or active fragment thereof, and combinations thereof.

The disclosure further provides a pharmaceutical composition comprising a therapeutically effective amount of the aptamer described above or a salt thereof, and a pharmaceutically acceptable carrier or diluent. The disclosure also provides a pharmaceutical composition comprising a therapeutically effective amount of the aptamer or a salt thereof, and a pharmaceutically acceptable carrier or diluent. Relatedly, the disclosure provides a method of treating or ameliorating a disease or disorder, comprising administering the pharmaceutical composition to a subject in need thereof. Administering a therapeutically effective amount of the composition to the subject may result in: (a) an enhancement of the delivery of the active agent to a disease site relative to delivery of the active agent alone; or (b) an enhancement of microvesicles clearance resulting in a decrease of at least 10%, 20%, 30%, 40%, 50%, 60%, 70%, 80%, or 90% in a blood level of microvesicles targeted by the aptamer; or (c) an decrease in biological activity of microvesicles targeted by the aptamer of at least 10%, 20%, 30%, 40%, 50%, 60%, 70%, 80%, or 90%. In an aspect, the biological activity of microvesicles comprises immune suppression or transfer of genetic information. The disease or disorder can include without limitation those disclosed herein. For example, the disease or disorder may comprise a neoplastic, proliferative, or inflammatory, metabolic, cardiovascular, or neurological disease or disorder. See above and further section "Phenotypes."

The disclosure further provides a kit comprising an aptamer disclosed herein, or a pharmaceutical composition thereof.

In an aspect, the disclosure provides a method comprising contacting the aptamer as described above with a biological sample and detecting the presence or absence of binding of the aptamer to a microvesicle in the biological sample. As disclosed herein, the biological sample can be a tissue, fluid or cell culture sample. For example, the biological sample may comprise blood or a blood component. In some embodiments, the aptamer is conjugated to a substrate prior to the contacting with the biological sample. For example, the substrate may comprise a bead or a plate well. The aptamer may also be conjugated to a detectable label. Various configurations of the method are provided herein. See, e.g.,

**FIGs. 2A-2B** and **16A.**

In a related aspect, the disclosure provides a method of detecting a presence or level of a microvesicle population in a biological sample suspected of containing the microvesicle population, comprising contacting the biological sample with one or more binding agent specific to the microvesicle population and one or more aptamer as described above, and detecting microvesicles that are recognized by both the one or more binding agent and the one or more aptamer, thereby detecting the presence or level of the microvesicle population in the biological sample.

The biological sample can be a tissue sample, a cell culture, or a bodily fluid. The bodily fluid can be any useful fluid, including without limitation one or more of peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, and umbilical cord blood. In some embodiments, the bodily fluid comprises blood, serum or plasma.

Any useful binding agent can be used in the disclosed methods. In some aspects, the one or more binding agent comprises an antibody or aptamer to a microvesicle surface antigen selected from **Table 3**, **Table 4**, and a combination thereof. For example, the one or more binding agent may be an antibody or aptamer to a microvesicle surface antigen selected from the group consisting of EpCam, CD9, PCSA, CD63, CD81, PSMA, B7H3, PSCA, ICAM, STEAP, KLK2, SSX2, SSX4, PBP, SPDEF, EGFR, and a combination thereof. The one or more binding agent can also comprise an antibody or aptamer to a microvesicle surface antigen selected from the group consisting of EGFR, PBP, EpCAM, KLK2, and a combination thereof.

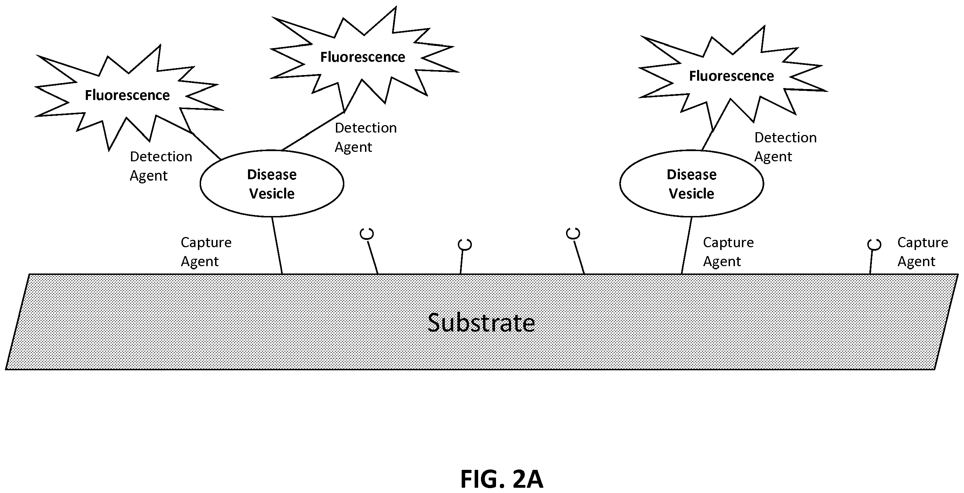

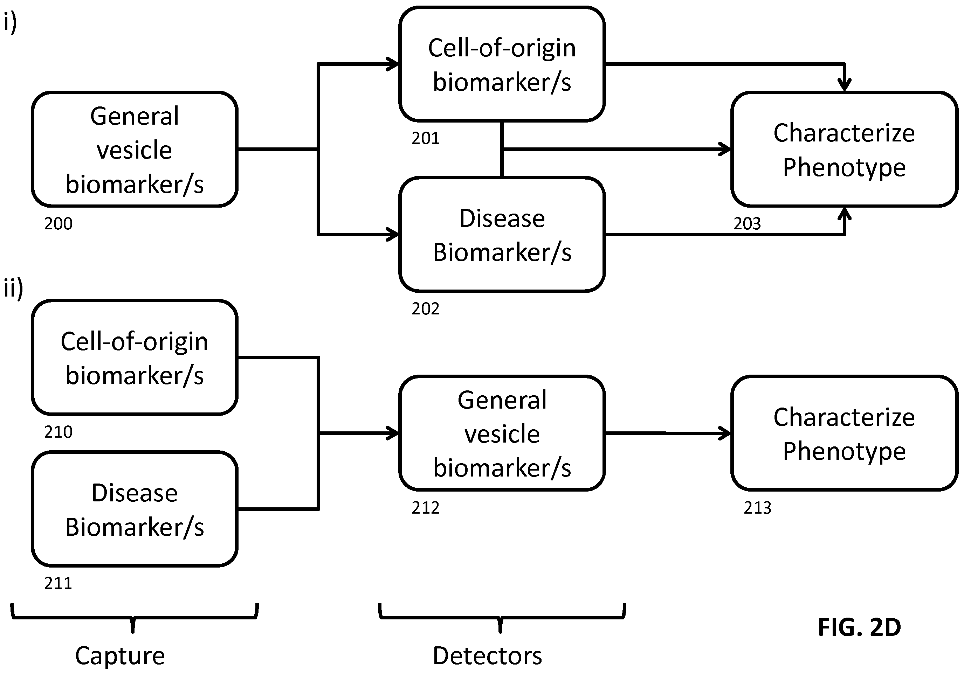

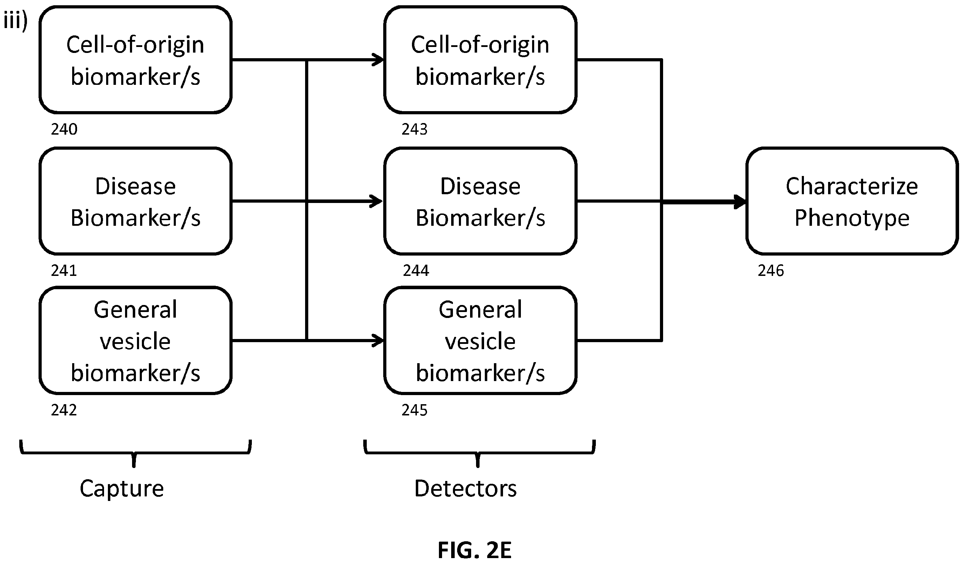

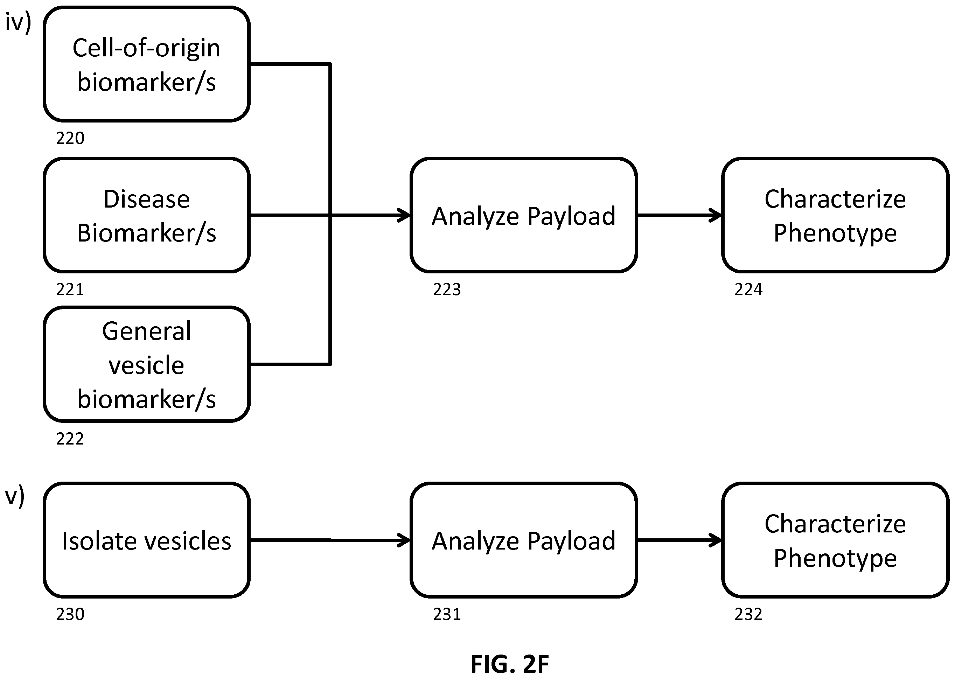

For example, a "sandwich" format can be used. See, e.g., **FIGs. 2A-2B.** The one or more binding agent may be conjugated to a substrate prior to the contacting with the biological sample. In this configuration, the one or more aptamer may be conjugated to a detectable label to serve as a detector agent. The one or more binding agent may be conjugated to a detectable label. In this configuration, the one or more aptamer may be conjugated to a substrate prior to the contacting with the biological sample to serve as a capture agent. Furthermore, the one or more aptamer can be conjugated to a substrate prior to the contacting with the biological sample, and/or the one or more aptamer is conjugated to a detectable label. In such cases, the one or more aptamer can act as either or both of a capture agent and a detection agent.

In another related aspect, the invention provides a method of characterizing a disease or disorder, comprising: (a) contacting a biological test sample with a plurality of oligonucleotides, wherein the plurality of oligonucleotides comprises 164 different oligonucleotide sequences, and wherein each different member of the plurality of oligonucleotides comprises a different sequence that is any one of SEQ ID Nos. 510599-510763, or a sequence that is 95 percent homologous to any one of SEQ ID Nos. 510599-510763; (b) detecting a presence or level of a complex between the plurality of oligonucleotides and a target in the biological test sample formed in step (a); and (c) comparing the presence or level detected in step (b) to a reference level from a biological control sample, thereby characterizing the disease or disorder. The reference level may be derived from a level of the target in a healthy sample individual, e.g., one that does not have or is not known to have the disease or disorder. The reference level may also be derived from an individual or sample having a treated, controlled, or alternate disease.

The biological test sample and biological control sample may each comprise a tissue sample, a cell culture, or a biological fluid. In some embodiments, the biological fluid comprises a bodily fluid. The bodily fluid can be any useful fluid, including without limitation one or more of peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood. In some embodiments, the bodily fluid comprises blood, serum or plasma. The biological fluid may comprise or be suspected to comprise microvesicles.

The one or more aptamer may bind a polypeptide or fragment thereof. The binding may be promiscuous or selective as desired. The polypeptide or fragment thereof can be soluble or membrane bound, e.g., in the membrane of a microvesicle or cell fragment. The polypeptide or fragment thereof comprises a biomarker in **Table 3** or **Table 4.** The one or more aptamer can bind a microvesicle surface antigen in the biological sample.

The method provided herein of characterizing a disease or disorder may include providing a diagnosis, prognosis or theranosis of the disease or disorder. The disease or disorder can include without limitation those disclosed herein. For example, the disease or disorder may comprise a cancer, a premalignant condition, an inflammatory disease, an immune disease, an autoimmune disease or disorder, a cardiovascular disease or disorder, a neurological disease or disorder, an infectious disease, and/or pain. See, e.g., section "Phenotypes" herein.

The invention further provides a kit comprising a reagent for carrying out the methods of the invention, wherein the at least one reagent comprises the plurality of oligonucleotides. For example, the reagent may comprise an aptamer disclosed herein. The reagent may further comprise other useful components disclosed herein including without limitation at least one of: a) a reagent configured to isolate a microvesicle, optionally wherein the at least one reagent configured to isolate a microvesicle comprises a binding agent to a microvesicle antigen, a column, a substrate, a filtration unit, a polymer, polyethylene glycol, PEG4000, PEG8000, a particle or a bead; b) at least one oligonucleotide configured to act as a primer or probe in order to amplify, sequence, hybridize or detect the oligonucleotide or plurality of oligonucleotides; and c) a reagent configured to remove one or more abundant protein from a sample, wherein optionally the one or more abundant protein comprises at least one of albumin, immunoglobulin, fibrinogen and fibrin.

In an aspect, the disclosure provides a composition comprising an input oligonucleotide library for assessing a cellular or extracellular vesicle sample comprising at least two subset oligonucleotide libraries to generate the input oligonucleotide library, wherein at least one of the at least two subset oligonucleotide libraries are manufactured with amounts of nucleotides with a total G and C content that is not equal to 50%. For example, the total G and C content can be less than 50%, or the the total G and C content can be more than 50%. At least one subset library may be manufactured with a total G and C content that is more than 50% and another subset library may be manufactured with a total G and C content less than 50%. The at least two subset libraries may comprise three subset libraries manufactured with 25%, 50% and 75% G and C content, respectively. The at least two subset libraries can be manufactured with amounts of nucleotides similar or equal to at least two rows in **Table 13.** The nucleotides can consist of naturally occurring nucleotides or modified naturally occurring nucleotides as desired. The nucleotides can also comprise non-naturally occurring nucleotides. Such input oligonucleotide libraries may be referred to as "GC" libraries herein.

In a related aspect, the disclosure provides a method of generating an input oligonucleotide library, comprising contacting the at least two subset oligonucleotide libraries to generate the input oligonucleotide library. The input oligonucleotide library can be screened or enriched to provide various aptamers or oligonucleotide probe libraries using the methods described herein.

In an aspect, the disclosure provides an oligonucleotide having a sequence that comprises a 5' transposon adapter region, an offset region comprising 0 or more nucleotides located 5' to the transposon adapter region, a variable region located 5' to the offset region, and a left primer region located 5' to the variable region. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences, wherein each of the oligonucleotide sequences comprises a 5' transposon adapter region, an offset region comprising 0 or more nucleotides located 5' to the transposon adapter region, a variable region located 5' to the offset region, and a left primer region located 5' to the variable region. Such oligonucleotides may be referred to herein as "balanced" oligonucleotides.

The balanced oligonucleotide or plurality of oligonucleotides can be such that: a) the transposon adapter region comprises 20-40 nucleotides; b) the offset region compries 0, 1, 2, 3, 4, 5, 6, 7, 8, 9 or 10 nucleotides; c) the variable region comprises 20-50 nucleotides; and/or d) the left primer region comprises 20-40 nucleotides. The balanced oligonucleotides may be such that: a) the transposon adapter region comprises a nucleic acid sequence that is at least about 50, 55, 60, 65, 70, 75, 80, 85, 90, 95, 96, 97, 98, 99 or 100 percent homologous to the sequence 5'-TCGTCGGCAGCGTCAGATGTGTATAAGAGACAG (SEQ ID NO. 510764); b) the offset region compries 1, 2, 3, 4, 5, 6, 7, 8, 9 or 10 nucleotides that are at least about 50, 55, 60, 65, 70, 75, 80, 85, 90, 95, 96, 97, 98, 99 or 100 percent homologous to at least one of the following sequences: 5'-T (SEQ ID NO. 510765), 5'-CT (SEQ ID NO. 510766) and 5'-ACT (SEQ ID NO. 510767); c) the variable region comprises 20, 21, 22, 23, 24, 25 26 27, 28, 29, 30, 31, 32, 33, 34, 35, 36, 37, 38, 39, 40, 41, 42, 43, 44, 45, 46, 47, 48, 49 or 50 nucleotides; and/or d) the left primer region comprises a nucleic acid sequence that is at least about 50, 55, 60, 65, 70, 75, 80, 85, 90, 95, 96, 97, 98, 99 or 100 percent homologous to a sequence selected from SEQ ID NOs. 510768 and 510769. The variable region may comprise "GC" library sequences as provided herein.

In an aspect, the disclosure provides a method of identifying an aptamer, comprising performing a selection or enrichment process using a balanced oligonucleotide or plurality of balanced oligonucleotides as a naive input library. Such selection or enrichment processes are described or provided herein, including without limitation SELEX and variations thereof. The selection process may comprise at least one round of positive selection against a target of interest and optionally at least one round of negative selection against a target other than the target of interest.

In another aspect, the disclosure provides a method comprising contacting a balanced oligonucleotide or plurality of balanced oligonucleotides with a sample and detecting the presence or level of binding of the oligonucleotide or plurality of oligonucleotides to a target in the sample. The sample may comprise a biological sample, such as an organic sample, an inorganic sample, a tissue, a cell culture, a bodily fluid, blood, serum, a cell, a microvesicle, a protein complex, a lipid complex, a carbohydrate, or any combination, fraction or variation thereof. The target can be any useful target, including without limitation a cell, an organelle, a protein complex, a lipoprotein, a carbohydrate, a microvesicle, a membrane fragment, a small molecule, a heavy metal, a toxin, or a drug.

In a related aspect, the disclosure provides a method comprising: a) contacting a biological sample comprising microvesicles with an oligonucleotide probe library, wherein the oligonucleotide probe library comprises a balanced oligonucleotide or plurality of balanced oligonucleotides; b) identifying oligonucleotides bound to at least a portion of the microvesicles; and c) characterizing the sample based on a profile of the identified oligonucleotides.

In another related aspect, the disclosure provides a method comprising: a) contacting a sample with an oligonucleotide probe library comprising at least $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences oligonucleotides to form a mixture in solution, wherein the oligonucleotides are capable of binding a plurality of entities in the sample to form complexes, wherein the oligonucleotide probe library comprises a balanced oligonucleotide or plurality of balanced oligonucleotides; d) partitioning the complexes formed in step (a) from the mixture; and c) detecting oligonucleotides present in the complexes partitioned in step (b) to identify an oligonucleotide profile for the sample. In a related aspect, the disclosure provides a method of characterizing a disease or disorder, comprising: a) contacting a biological test sample with a balanced oligonucleotide or plurality of balanced oligonucleotides; b) detecting a presence or level of complexes formed in step (a) between the balanced oligonucleotide or plurality of balanced oligonucleotides and a target in the biological test sample; and c) comparing the presence or level detected in step (b) to a reference level from a biological control sample, thereby characterizing the disease or disorder. The step of detecting may comprise performing sequencing of all or some of the oligonucleotides in the complexes, amplification of all or some of the oligonucleotides in the complexes, and/or hybridization of all or some of the oligonucleotides in the complexes to an array. In some embodiments, the sequencing comprises high-throughput sequencing.

In the methods comprising use of a balanced oligonucleotide or plurality of balanced oligonucleotides, the biological test sample and biological control sample may each comprise a tissue sample, a cell culture, or a biological fluid. In some embodiments, the biological fluid comprises a bodily fluid. Useful bodily fluids within the method of the invention comprise peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood. In some preferred embodiments, the bodily fluid comprises blood, serum or plasma. The biological fluid may comprise microvesicles. In such case, the complexes may be formed between the oligonucleotide or plurality of oligonucleotides and at least one of the microvesicles. The biological test sample and biological control sample may further comprise isolated microvesicles, wherein optionally the microvesicles are isolated using at least one of chromatography, filtration, ultrafiltration, centrifugation, ultracentrifugation, flow cytometry, affinity capture (e.g., to a planar surface, column or bead), polymer precipitation, and using microfluidics. The vesicles can also be isolated after contact with the oligonucleotide or plurality of oligonucleotides.

In various aspects of the methods comprising use of a balanced oligonucleotide or plurality of balanced oligonucleotides, the oligonucleotide or plurality of oligonucleotides binds a polypeptide or fragment thereof. The polypeptide or fragment thereof can be soluble or membrane bound, wherein optionally the membrane comprises a microvesicle membrane. The membrane could also be from a cell or a fragment of a cell of vesicle. The polypeptide or fragment thereof may comprise a biomarker in **Table 3** or **Table 4.** For example, the polypeptide or fragment thereof could be a general vesicle marker such as in **Table 3** or a tissue-related or disease-related marker such as in **Table 4.** The oligonucleotide or plurality of oligonucleotides may bind a microvesicle surface antigen in the biological sample. For example, the oligonucleotide or plurality of oligonucleotides can be enriched from a naive library against microvesicles.

The disease or disorder detected by the oligonucleotide, plurality of oligonucleotides, or methods provided herein comprising use of a balanced oligonucleotide or plurality of balanced oligonucleotides may comprise any appropriate disease or disorder of interest. For example, the disease or disorder may comprise a cancer, a premalignant condition, an inflammatory disease, an immune disease, an autoimmune disease or disorder, a cardiovascular disease or disorder, a neurological disease or disorder, an infectious disease, and/or pain. See, e.g., section "Phenotypes" herein. The disease or disorder may include without limitation Breast Cancer, Alzheimer's disease, bronchial asthma, Transitional cell carcinoma of the bladder, Giant cellular osteoblastoclastoma, Brain Tumor, Colorectal adenocarcinoma, Chronic obstructive pulmonary disease (COPD), Squamous cell carcinoma of the cervix, acute myocardial infarction (AMI) / acute heart failure, Chron's Disease, diabetes mellitus type II, Esophageal carcinoma, Squamous cell carcinoma of the larynx, Acute and chronic leukemia of the bone marrow, Lung carcinoma, Malignant lymphoma, Multiple Sclerosis, Ovarian carcinoma, Parkinson disease, Prostate adenocarcinoma, psoriasis, Rheumatoid Arthritis, Renal cell carcinoma, Squamous cell carcinoma of skin, Adenocarcinoma of the stomach, carcinoma of the thyroid gland, Testicular cancer, ulcerative colitis, or Uterine adenocarcinoma.

The disclosure further provides a kit comprising a reagent for carrying out the methods herein and also use of the reagent for carrying out the methods related to GC libraries or balanced oligonucleotides. For example, the reagent may comprise one or more oligonucleotide having a sequence derived from a GC libraries and/or a balanced oligonucleotide library. The reagent may further comprise other useful components disclosed herein including without limitation at least one of: a) a reagent configured to isolate a microvesicle, optionally wherein the at least one reagent configured to isolate a microvesicle comprises a binding agent to a microvesicle antigen, a column, a substrate, a filtration unit, a polymer, polyethylene glycol, PEG4000, PEG8000, a particle or a bead; b) at least one oligonucleotide configured to act as a primer or probe in order to amplify, sequence, hybridize or detect the oligonucleotide or plurality of oligonucleotides; and c) a reagent configured to remove one or more abundant protein from a sample, wherein optionally the one or more abundant protein comprises at least one of albumin, immunoglobulin, fibrinogen and fibrin.

## BRIEF DESCRIPTION OF THE DRAWINGS

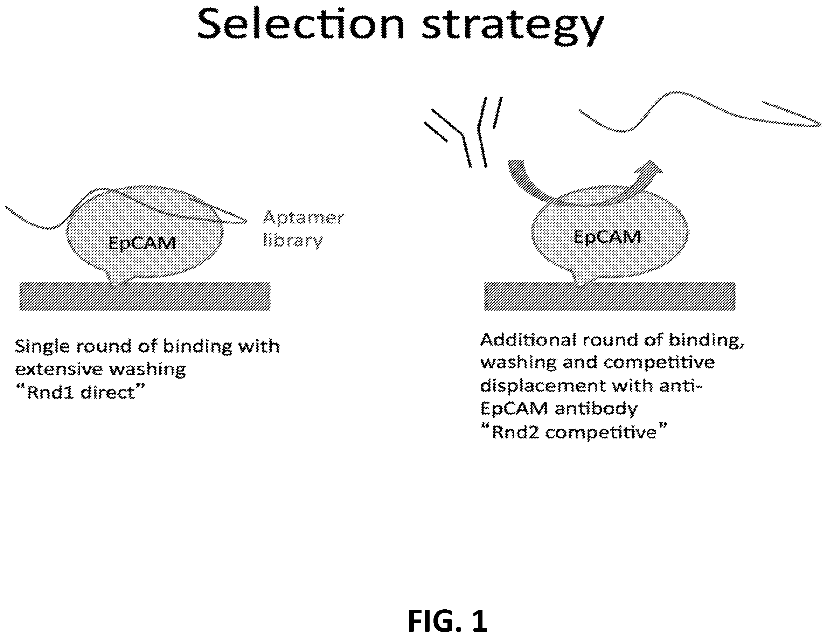

**FIG. 1** illustrates a competitive assay selection strategy: the random pool of aptamer (the library) is incubated with the target protein, in this case, EpCAM. After washing and elution from the target, the eluted aptamers are again added to the target and allowed to bind. The antibody is then added to the reaction, competing with the aptamers at the epitope of the antibody. The aptamers displaced by the antibody are then collected.

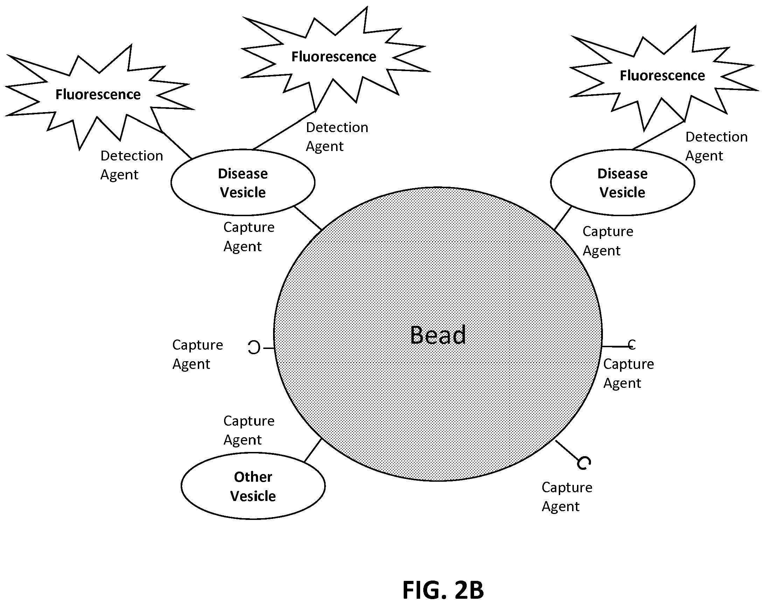

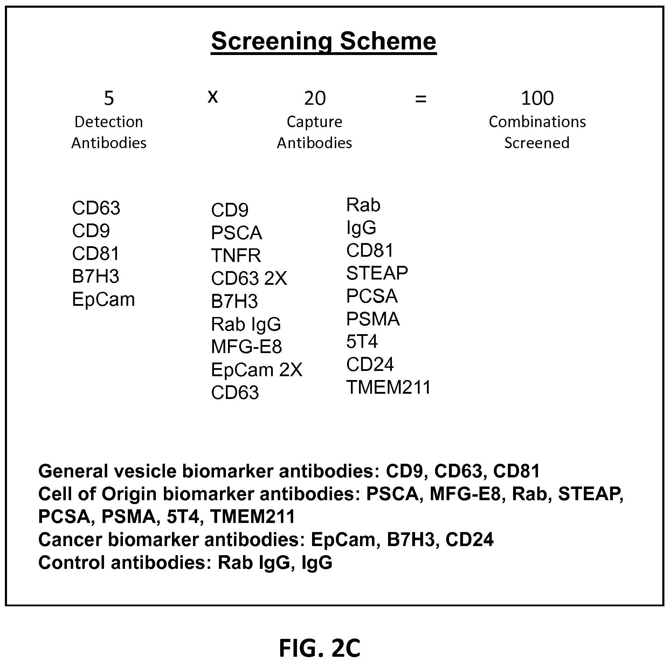

**FIGs. 2A-2F** illustrate methods of assessing biomarkers such as microvesicle surface antigens. **FIG. 2A** is a schematic of a planar substrate coated with a capture agent, such as an aptamer or antibody, which captures vesicles expressing the target antigen of the capture agent. The capture agent may bind a protein expressed on the surface of vesicles shed from diseased cells ("disease vesicle"). The detection agent, which may also be an aptamer or antibody, carries a detectable label, here a fluorescent signal. The detection agent binds to the captured vesicle and provides a detectable signal via its fluorescent label. The detection agent can detect an antigen that is generally associated with vesicles, or is associated with a cell-of-origin or a disease, e.g., a cancer. **FIG. 2B** is a schematic of a particle bead conjugated with a capture agent, which captures vesicles expressing the target antigen of the capture agent. The capture agent may bind a protein expressed on the surface of vesicles shed from diseased cells ("disease vesicle"). The detection agent, which may also be an aptamer or antibody, carries a detectable label, here a fluorescent signal. The detection agent binds to the captured vesicle and provides a detectable signal via its fluorescent label. The detection agent can detect an antigen that is generally associated with vesicles, or is associated with a cell-of-origin or a disease, e.g., a cancer. **FIG. 2C** is an example of a screening scheme that can be performed by using different combinations of capture and detection agents to the indicated biomarkers. The biomarker combinations can be detected using assays as shown in **FIGs. 2A-2B. FIGs. 2D-2E** present illustrative schemes for capturing and detecting vesicles to characterize a phenotype. **FIG. 2F** presents illustrative schemes for assessing vesicle payload to characterize a phenotype.

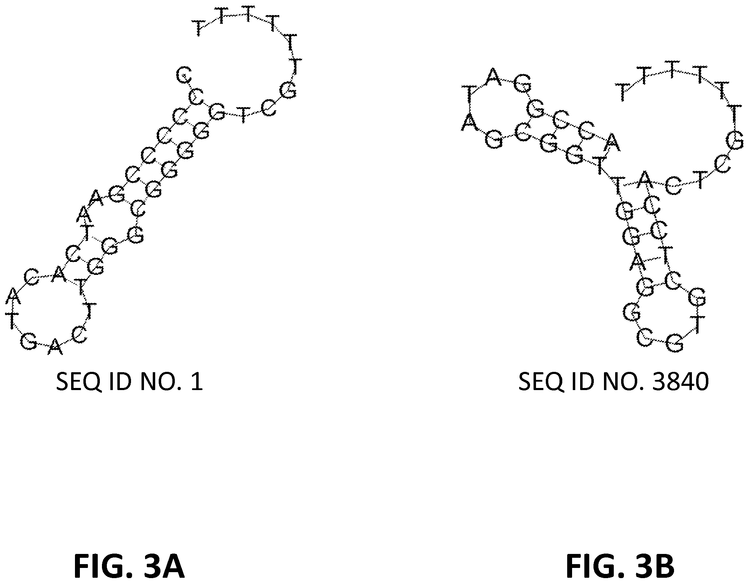

**FIGs. 3A-3B** illustrate a non-limiting example of an aptamer nucleotide sequence and its secondary structure. **FIG. 3A** illustrates a secondary structure of a 32-mer oligonucleotide, Aptamer 4, with sequence 5'-CCCCCCGAATCACATGACTTGGGCGGGGGTCG (SEQ ID NO. 1). In the figure, the sequence is shown with 6 thymine nucleotides added to the end, which can act as a spacer to attach a biotin molecule. This particular oligo has a high binding affinity to the target, EpCAM (*see* **Table 5**). Additional candidate EpCAM binders are identified by modeling the entire database of sequenced oligos to the secondary structure of this oligo. **FIG. 3B** illustrates another 32-mer oligo with sequence 5'-ACCGGATAGCGGTTGGAGGCGTGCTCCACTCG (SEQ ID NO. 2) that has a different secondary structure than the aptamer in **FIG. 3A.** This aptamer is also shown with a 6-thymine tail.

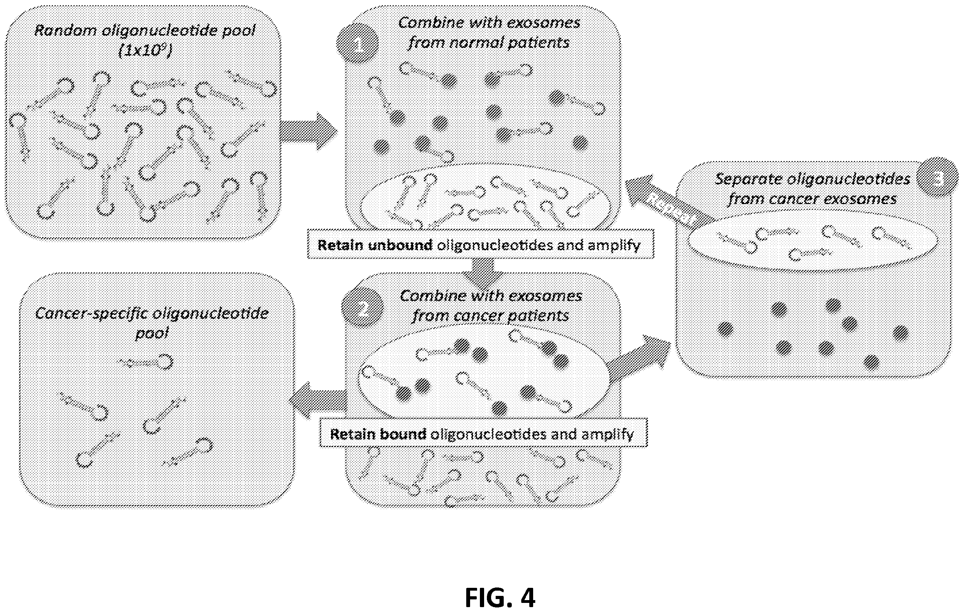

**FIG. 4** illustrates a process for producing a target-specific set of aptamers using a cell subtraction method, wherein the target is a biomarker associated with a specific disease. In Step 1, a random pool of oligonucleotides is contacted with a biological sample from a normal patient. In Step 2, the oligos that did not bind in Step 1 are added to a biological sample isolated from diseased patients. The bound oligos from this step are then eluted, captured via their biotin linkage and then combined again with normal biological sample. The unbound oligos are then added again to disease-derived biological sample and isolated. This process can be repeated iteratively. The final eluted aptamers are tested against patient samples to measure the sensitivity and specificity of the set. Biological samples can include blood, including plasma or serum, or other components of the circulatory system, such as microvesicles.

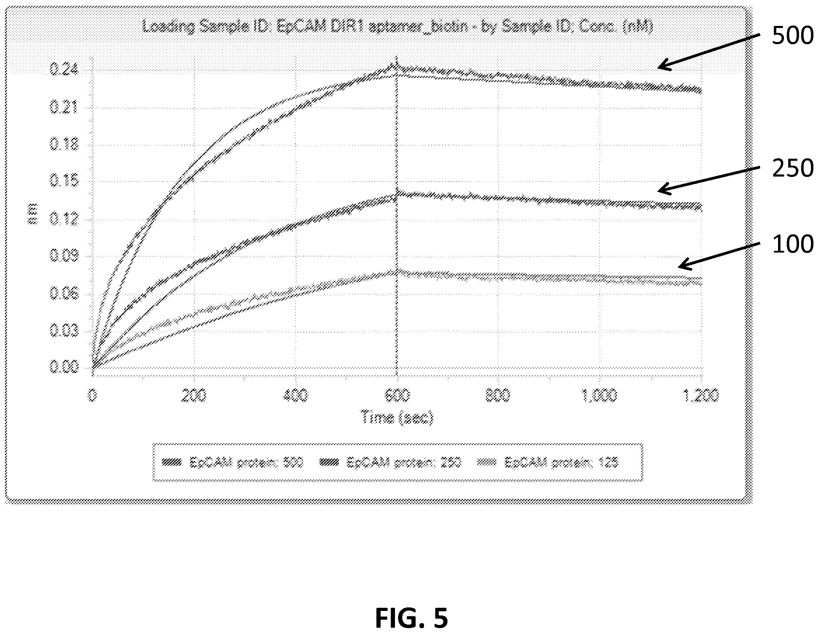

**FIG. 5** illustrates results from a binding assay showing the binding affinity of an exemplary aptamer (Aptamer ID BTX176881 (SEQ ID NO: 3)) to the target EpCAM protein at various target concentrations. The aptamer to be tested is fixed to a substrate using a biotin tail and is incubated with various concentrations of target (125, 250 and 500 nM). The test is performed on a surface plasmon resonance machine (SPR). The SPR machine detects association and disassociation of the aptamer and the target. Target is applied until the association and disassociation events are equal, resulting in a plateau of the curve. The equations describing the curve at each concentration can then be used to calculate the $K_D$ of the aptamer (*see* **Table 5**).

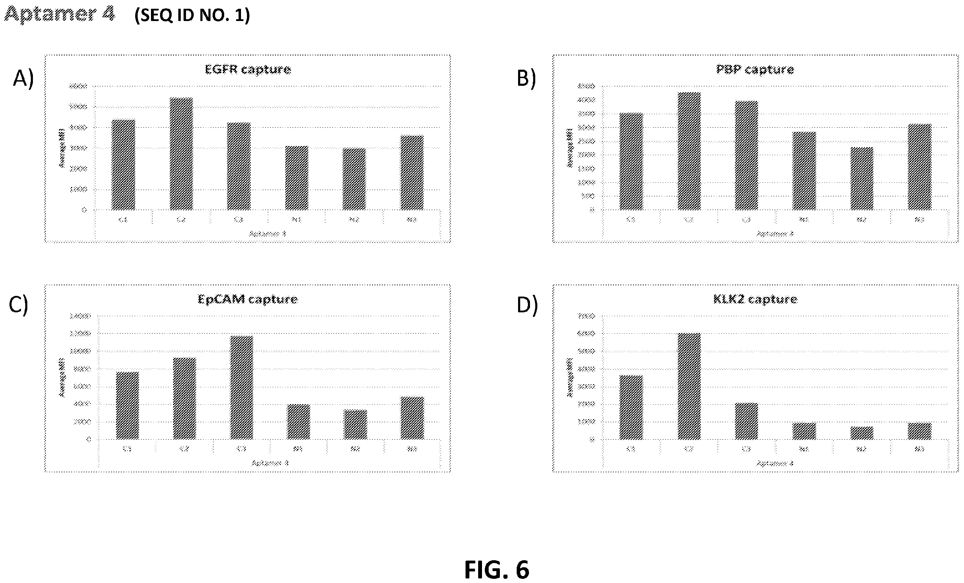

**FIG. 6** illustrates the use of an anti-EpCAM aptamer (Aptamer 4; SEQ ID NO. 1) to detect a microvesicle population. Vesicles in patient plasma samples were captured using bead-conjugated antibodies to the indicated microvesicle surface antigens: A) EGFR; B) PBP; C) EpCAM; D) KLK2. Fluorescently labeled Aptamer 4 was used as a detector in the microbead assay. The figure shows average median fluorescence values (MFI values) for three cancer (C1-C3) and three normal samples (N1-N3) in each plot. In each plot, the samples from left to right are ordered as: C1, C2, C3, N1, N2, N3.

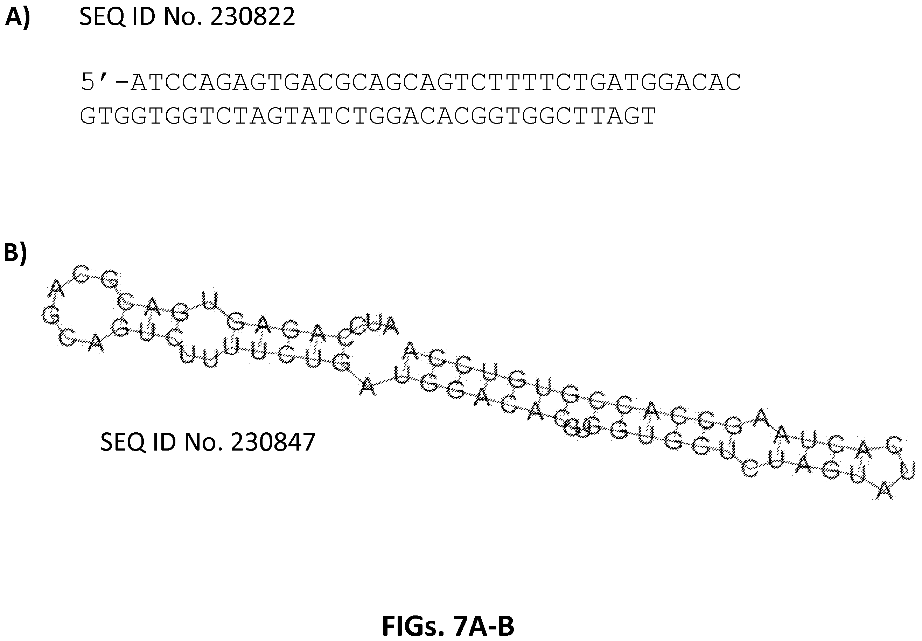

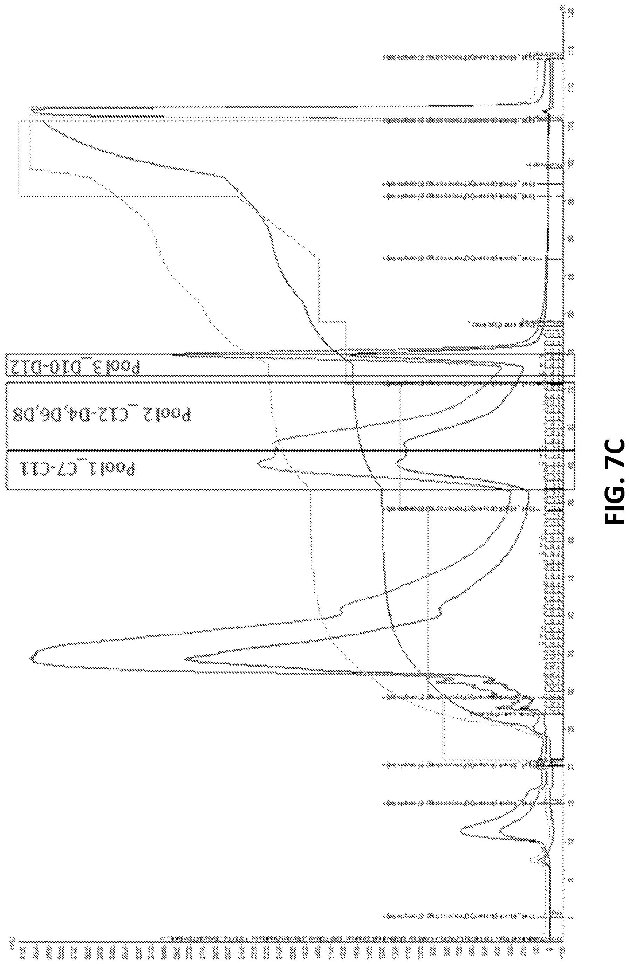

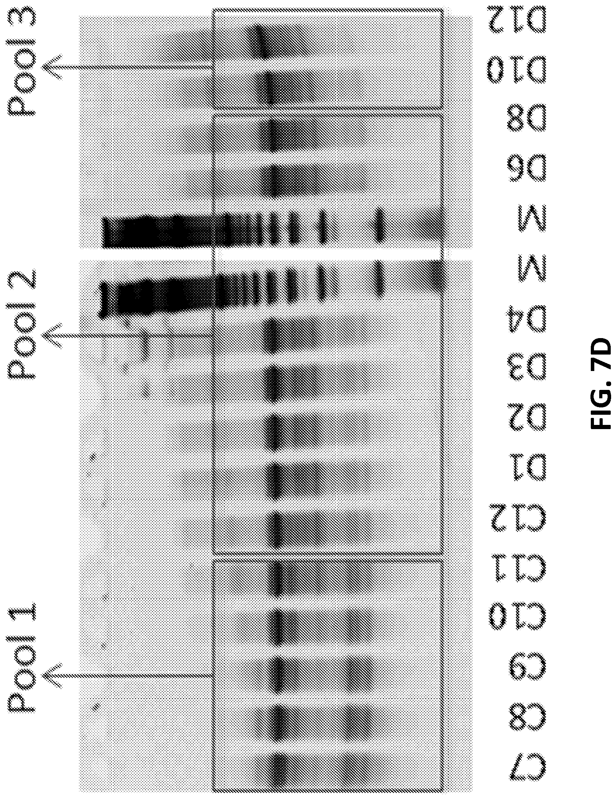

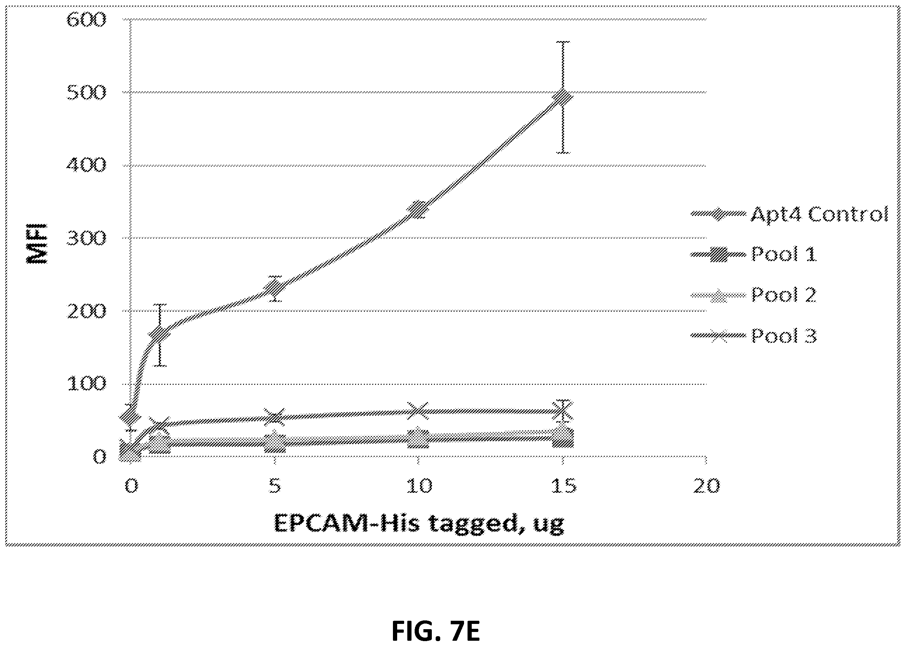

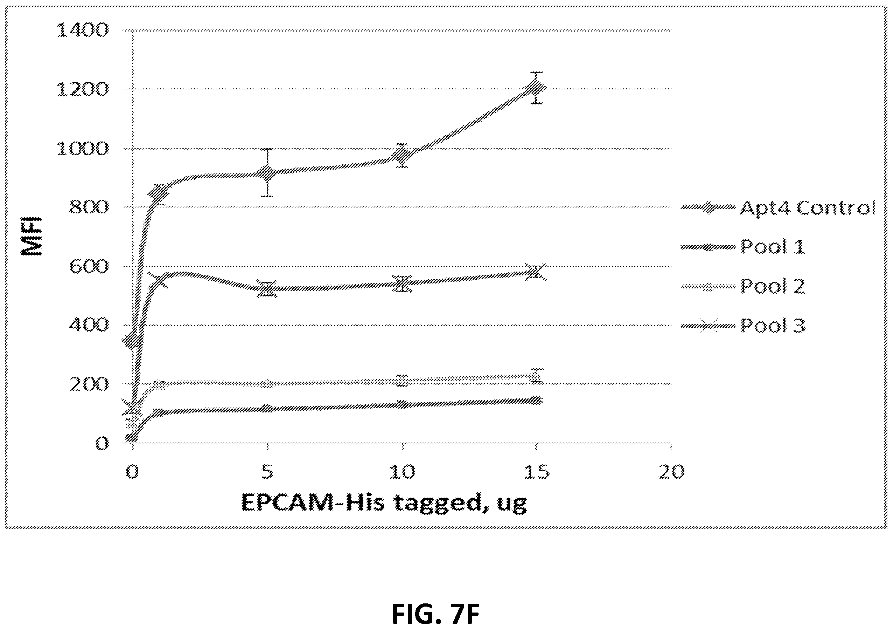

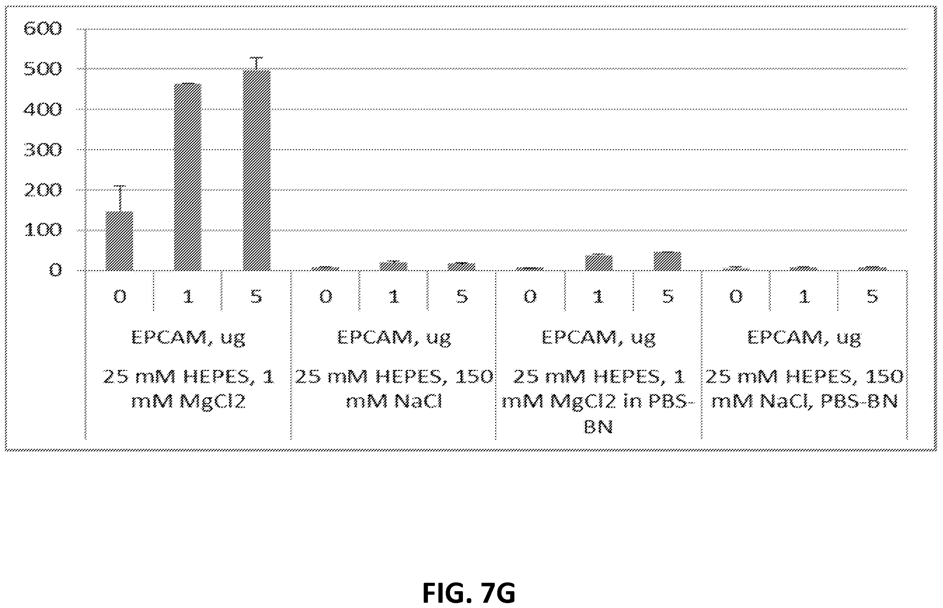

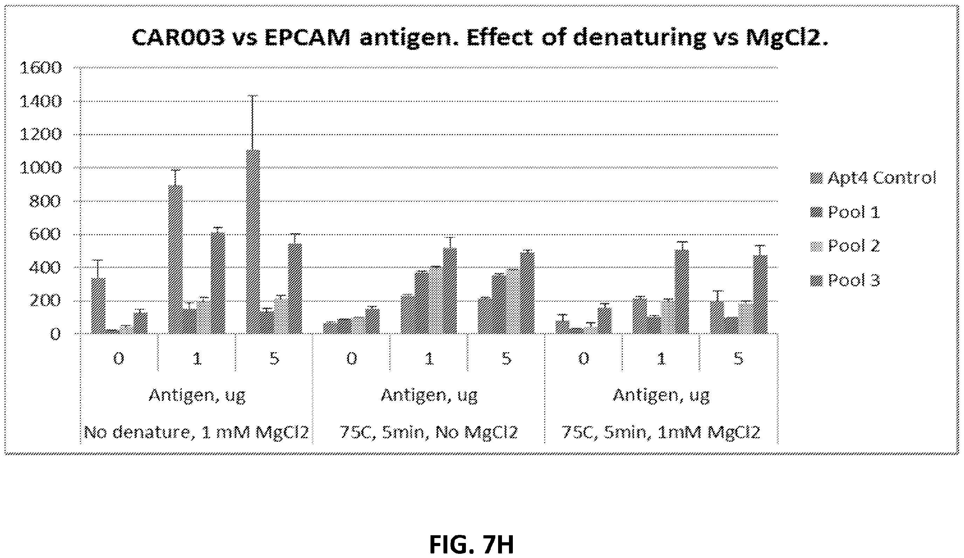

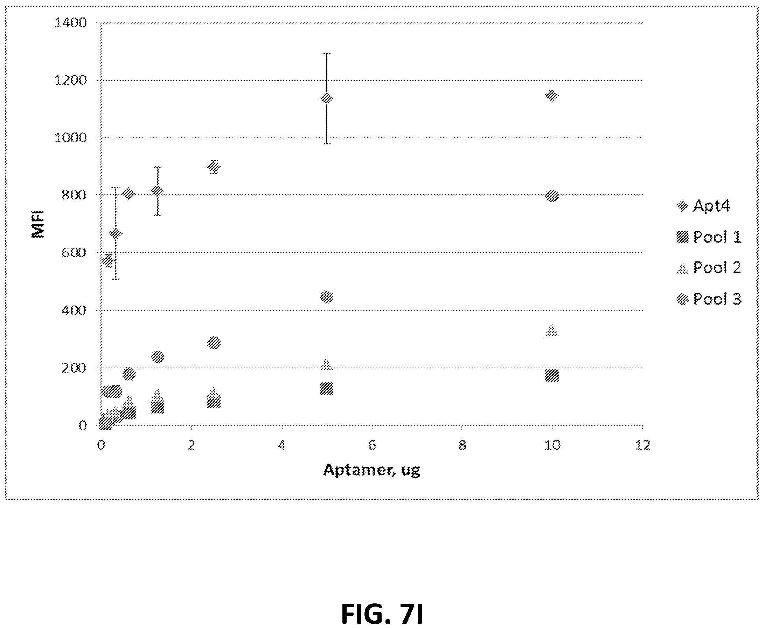

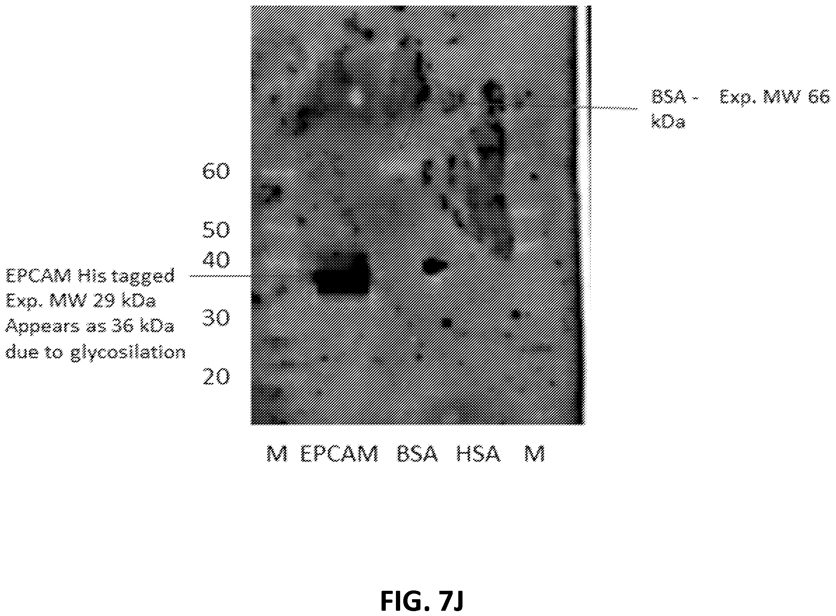

**FIG. 7A** illustrates the sequence of EPCAM aptamer CAR003 (SEQ ID NO. 4). **FIG. 7B** illustrates the optimal secondary structure of CAR003 with a minimum free energy ($\Delta$G) of -30.00 kcal/mol. For purposes of illustration, the aptamer is shown as an RNA aptamer (SEQ ID NO. 5) corresponding to the DNA sequence in **FIG. 7A**. **FIG. 7C** illustrates aptamer pool purification. The figure comprises an FPLC chromatogram with all product and fractions assigned in pools after checking quality on gel. **FIG. 7D** illustrates a SYBR GOLD stained gel with different FPLC fractions of CAR003 aptamer after synthesis. Different fractions were combined in pools based on amount of un-finished chains in order high to low (pool 1 - pool 3). The pools 1-3 correspond to those indicated in **FIG. 7C**. **FIG. 7E-F** illustrate binding of CAR003 to EPCAM protein in 25 mM HEPES with PBS-BN **(FIG. 7E)** or in 25 mM HEPES with 1 mM MgCl$_2$ **(FIG. 7F)**. **FIG. 7G** illustrates CAR003 binding to EpCAM in the indicated salts with and without addition of bovine serum albumin (BSA). **FIG. 7H** illustrates the effect of denaturing on CAR003 binding to EPCAM protein. In each group of four bars, the aptamer is from left to right: Aptamer 4, CAR003 Pool 1, CAR003 Pool 2, and CAR003 Pool 3. **FIG. 7I** illustrates titration of aptamers against EPCAM recombinant protein (constant input 5 $\mu$g). **FIG. 7J** illustrates a Western blot with CAR003 aptamer versus EPCAM his-tagged protein, BSA, and HSA (5 $\mu$g each). The gel was blocked 0.5% F127 and probed with -50 $\mu$g/ml CAR003 biotinylated aptamer, fraction 3. The blot was visualized with NeutrAvidin-HRP followed by SuperSignal West Femto Chemiluminescent Substrate.

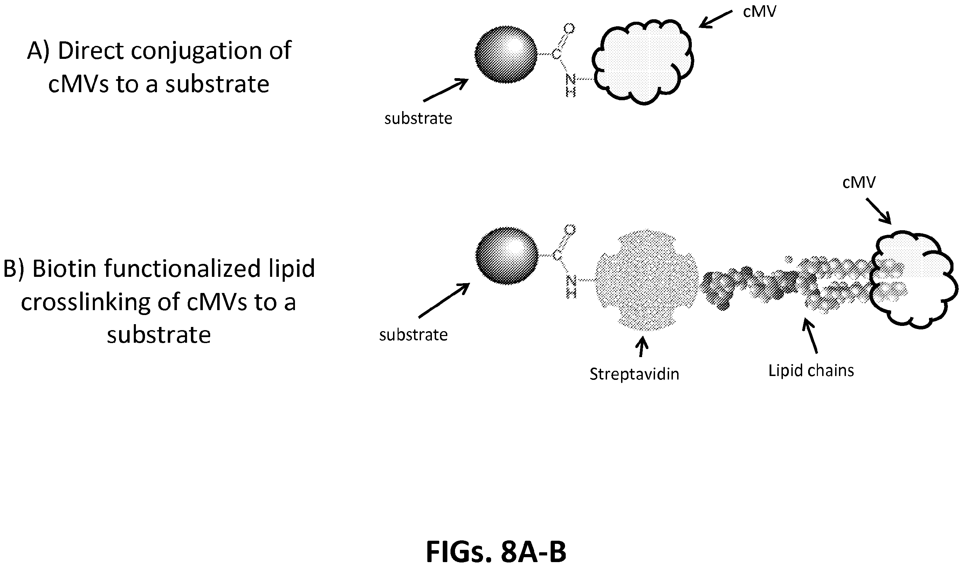

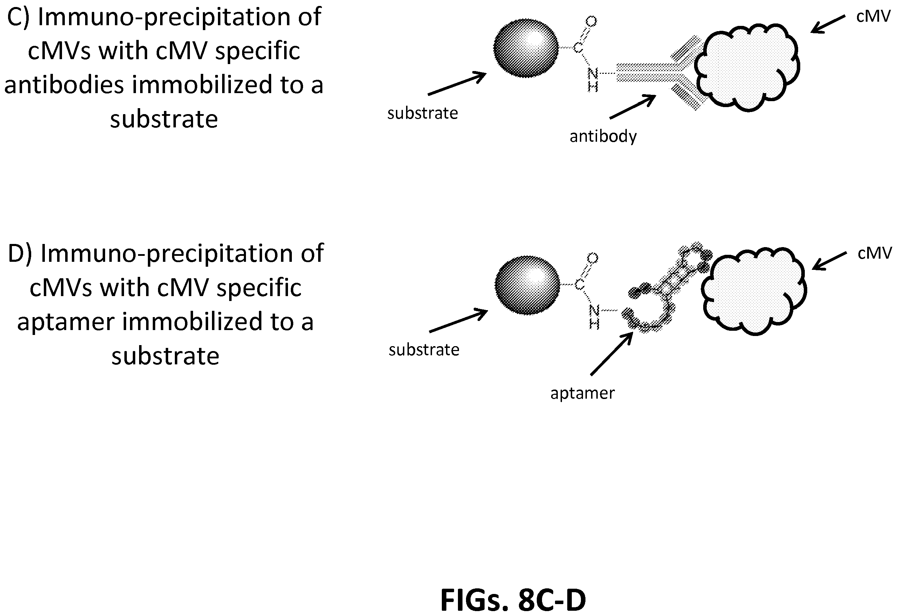

**FIGs. 8A-8D** illustrates methods to attach microvesicles to a substrate. **FIG. 8A** illustrates direct conjugation of a carboxylated microsphere to a vesicle surface antigen. **FIG. 8B** illustrates anchoring of a microvesicle to a microsphere via a biotin functionalized lipid anchor. **FIG. 8C** illustrates antibody binding to a vesicle surface antigen, wherein the antibody is conjugated to a carboxylated microsphere. **FIG. 8D** illustrates aptamer binding to a vesicle surface antigen, wherein the aptamer is conjugated to a carboxylated microsphere.

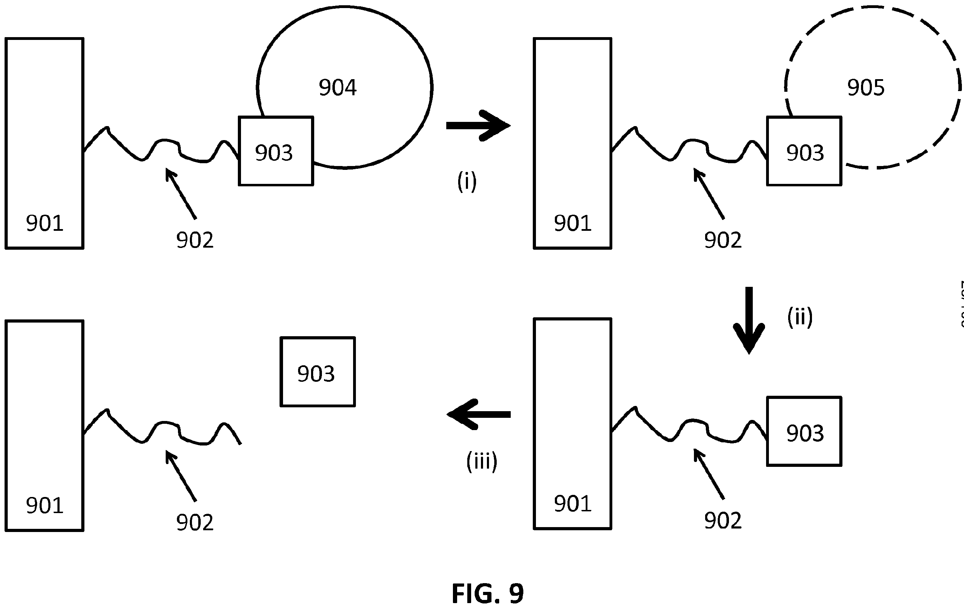

**FIG. 9** comprises a schematic for identifying a target of a selected aptamer, such as an aptamer selected by the above process. The figure shows a binding agent **902,** here an aptamer for purposes of illustration, tethered to a substrate **901**. The binding agent **902** can be covalently attached to substrate **901**. The binding agent **902** may also be non-covalently attached. For example, binding agent **902** can comprise a label which can be attracted to the substrate, such as a biotin group which can form a complex with an avidin/streptavidin molecule that is covalently attached to the substrate. The binding agent **902** binds to a surface antigen **903** of microvesicle **904.** In the step signified by arrow (i), the microvesicle is disrupted while leaving the complex between the binding agent **902** and surface antigen **903** intact. Disrupted microvesicle **905** is removed, e.g., via washing or buffer exchange, in the step signified by arrow (ii). In the step signified by arrow (iii), the surface antigen **903** is released from the binding agent **902**. The surface antigen **903** can be analyzed to determine its identity.

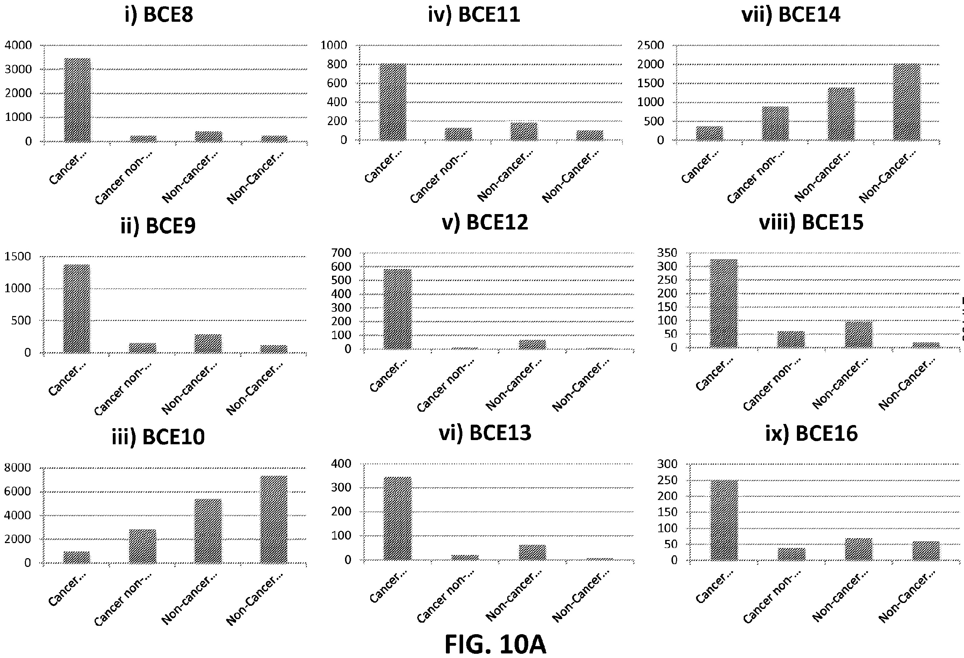

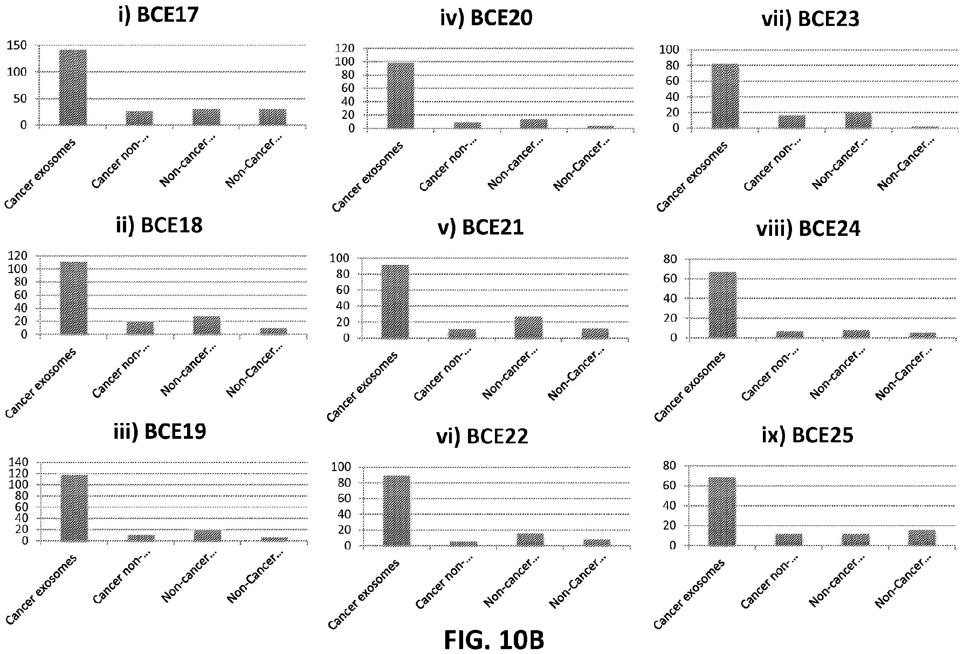

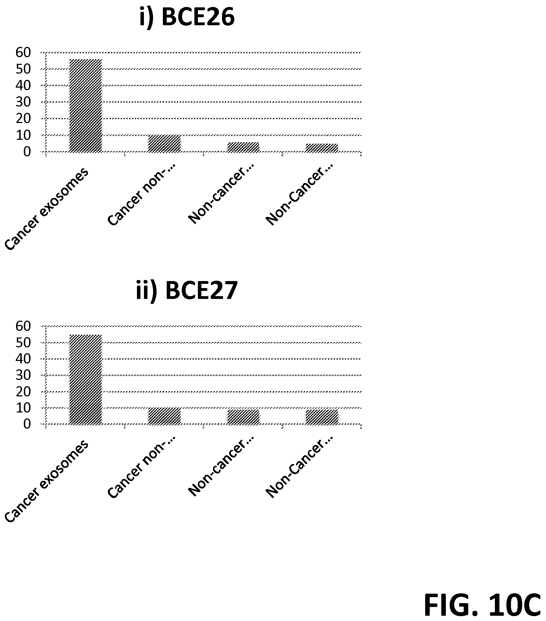

**FIGs. 10A-C** illustrate binding of selected aptamers against microbeads conjugated to various input sample. The aptamers were selected from an aptamer library as binding to microbeads conjugated to breast cancer-derived microvesicles. Experimental details are in the Examples herein. Each plot shows a different aptamer. The Y-axis indicates level of binding. In each group of samples, binding of 9 purified aptamer candidates is shown. The input sample is indicated on the X axis from left to right as follows: 1) Cancer Exosome: aptamer binding to microbeads conjugated to microvesicles isolated from plasma samples from breast cancer patients; 2) Cancer Non-exosome: aptamer binding to microbeads conjugated to plasma samples from breast cancer patients after removal of microvesicles by ultracentrifugation; 3) Non-Cancer Exosome: aptamer binding to microbeads conjugated to microvesicles isolated from plasma samples from normal (i.e., non-breast cancer) patients; 4) Non-Cancer Non-Exosome: aptamer binding to microbeads conjugated to plasma samples from breast cancer patients after removal of microvesicles by ultracentrifugation.

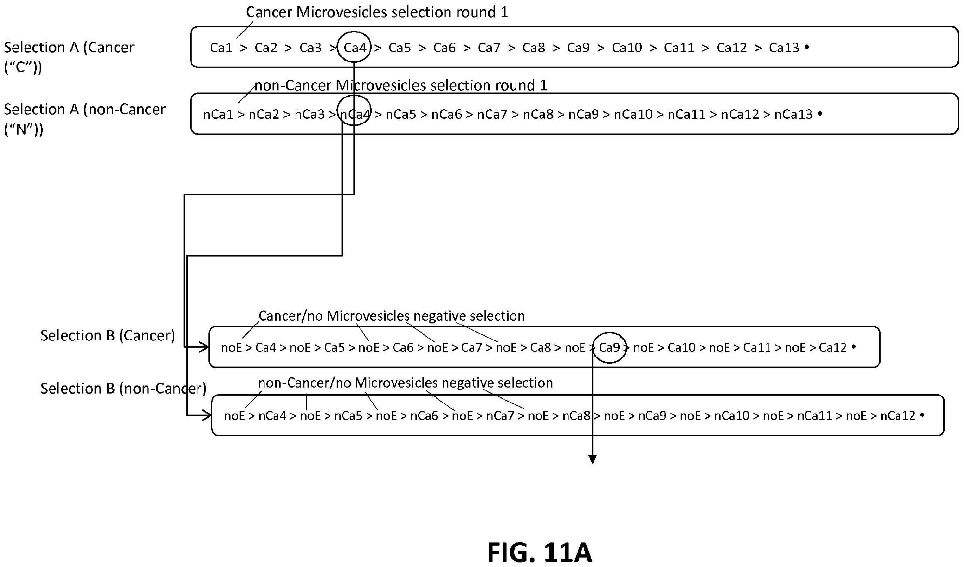

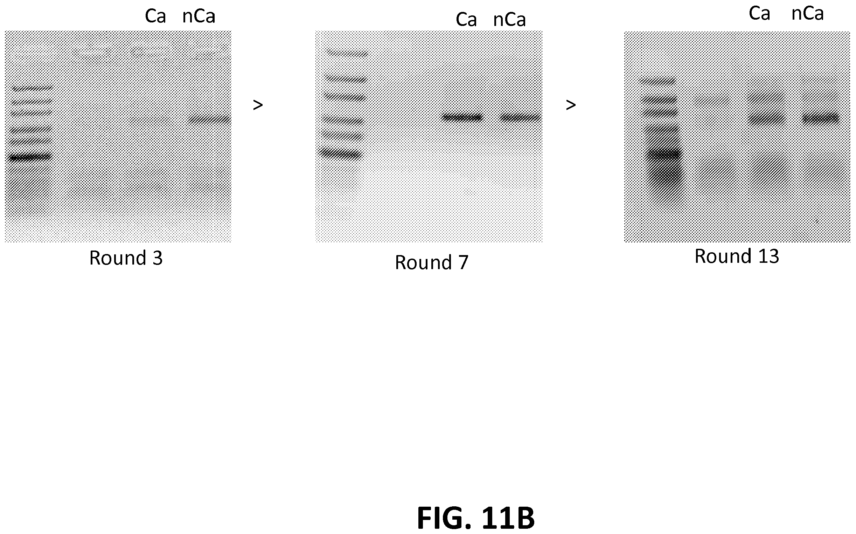

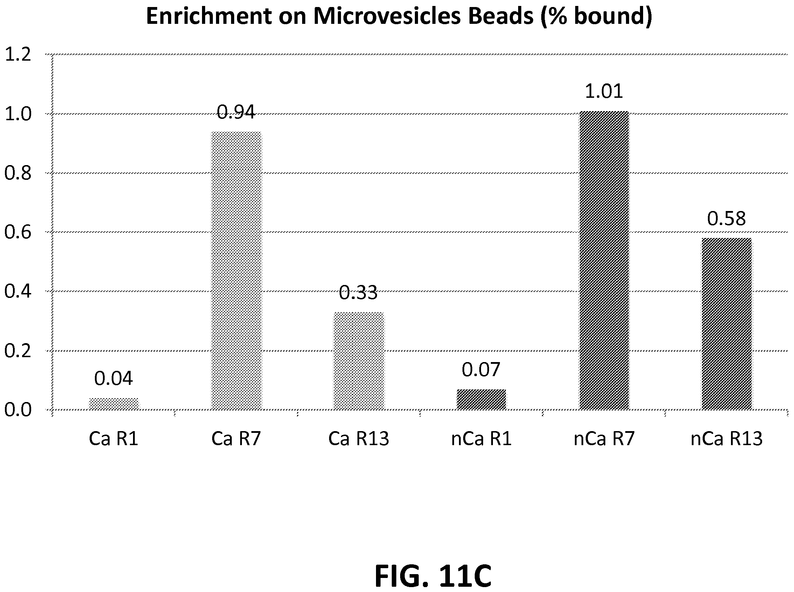

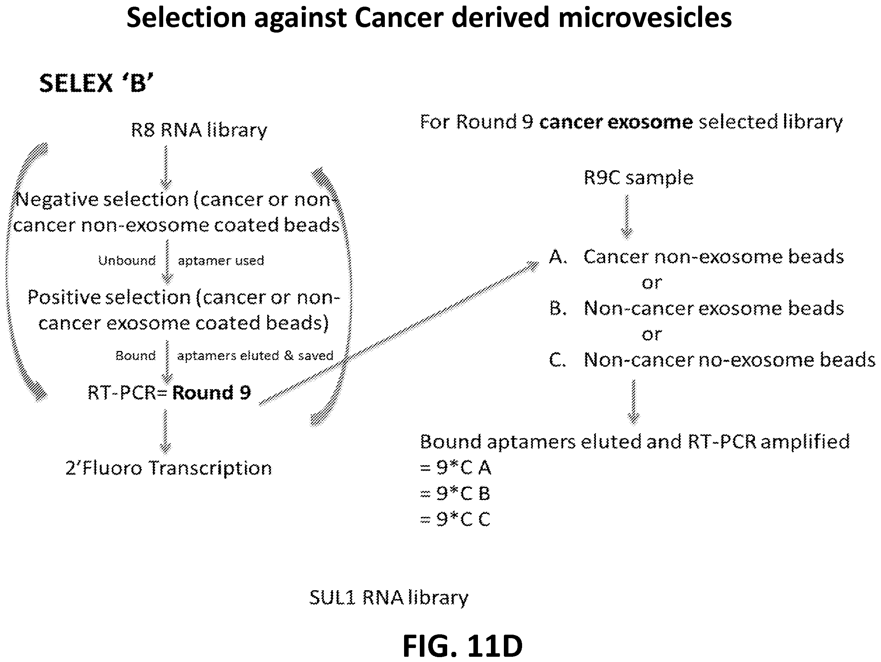

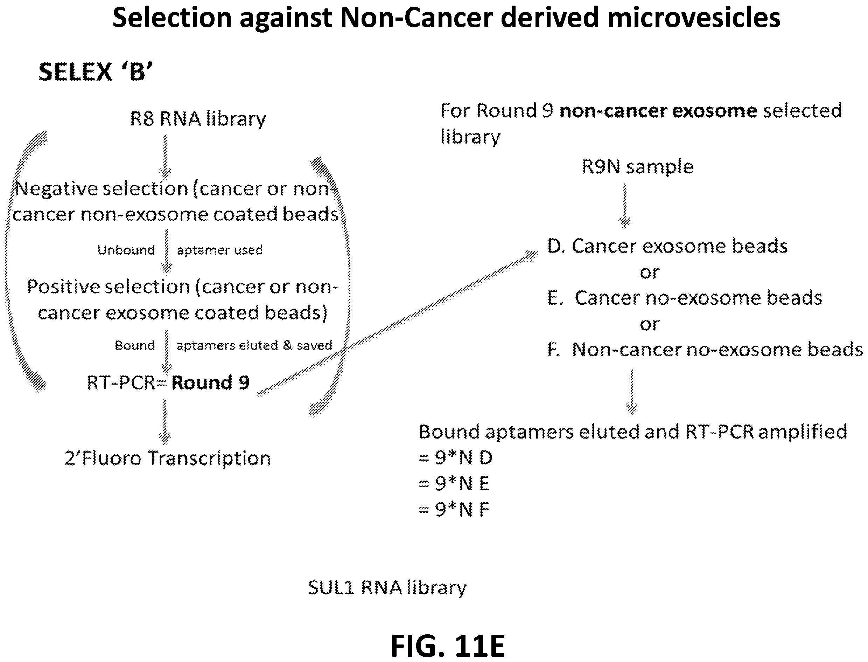

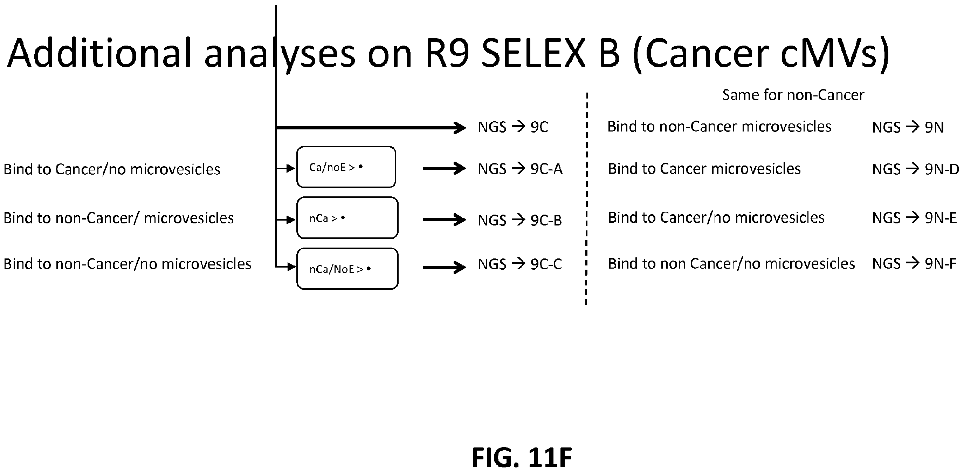

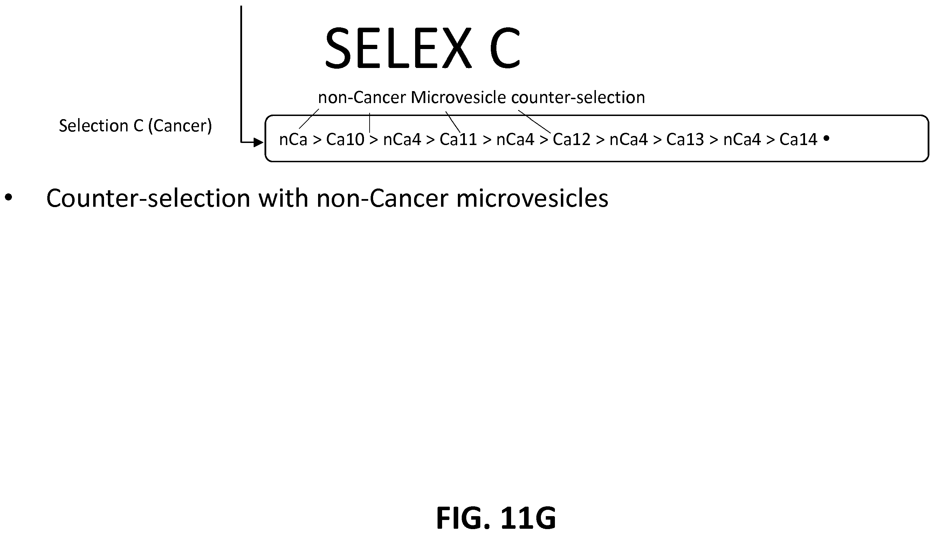

**FIGs. 11A-G** illustrate selection schemes used to enrich a starting aptamer library to a target of interest. In **FIG. 11A**, "Selection A" indicates 13 rounds of positive selection against microvesicles isolated from the plasma of breast cancer patients and non-breast cancer individuals. Ca1 refers to round 1 of cancer vesicle selection, etc. nCa1 refers to round 1 of non-cancer vesicle selection, etc. "Selection B" indicates a derivative wherein the aptamer pools after 3 rounds of positive selection in "Selection A" were used as input for 9 additions rounds (i.e., rounds 4-12) of positive and negative selection against microvesicles isolated from the plasma of breast cancer patients and non-breast cancer individuals. "noE" indicates the negative selection rounds. **FIG. 11B** show the recovered aptamer library after the indicated rounds of enrichment as run on agarose gels. Aptamers selected against cancer vesicles ("Ca") and non-cancer microvesicles ("nCa") are shown. **FIG. 11C** illustrates enrichment of aptamers that bind to cancer vesicles (three leftmost columns "Ca") and non-cancer microvesicles (three rightmost columns "nCa") through the rounds of selection 1 (R1), 7 (R7) and 13 (R13). Labeled aptamers after the indicated round of selection were incubated with bead-captured microvesicles. The number of aptamers binding to the bead-captured microvesicles was determined. The Y-axis indicates the percentage of bound aptamers compared to total input. The fall off from rounds 7 to 13 may indicate removal of non-specific binders. **FIG. 11D** and **FIG. 11E** indicate additional selection done for cancer and non-cancer microvesicles, respectively, after rounds 9 of "Selection B." **FIG. 11F** presents another view of **FIG. 11D. FIG. 11G** shows a third selection "Selection C" performed after rounds 9 of "Selection B" with alternating rounds of selection against cancer-derived (Ca#) and non-cancer derived (nCa#) microvesicles.

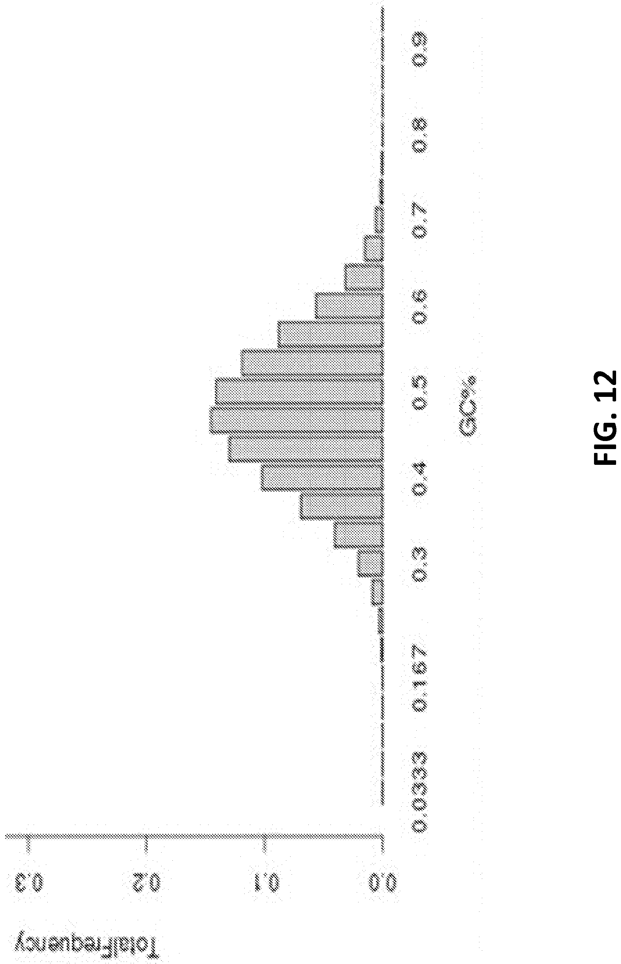

**FIG. 12** illustrates distribution of aptamer frequency observed in a selection steps versus sequence GC content. The library in this figure comprised of randomly generated sequences 30 nucleotides in length flanked by primer annealing sequences.

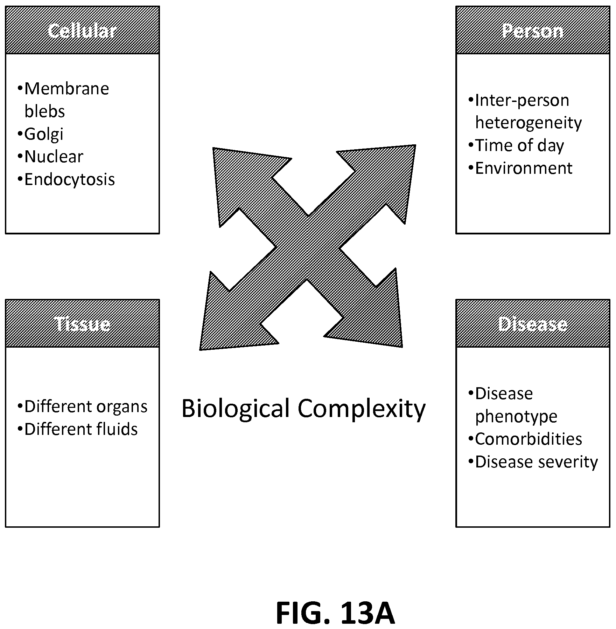

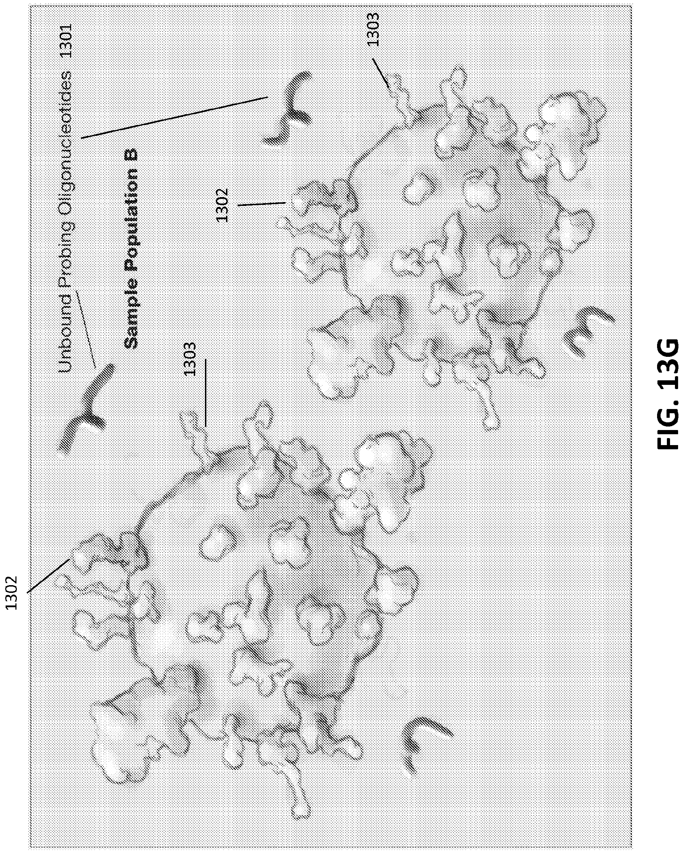

**FIGs. 13A-I** illustrate development and use of an oligonucleotide probe library to distinguish biological sample types.

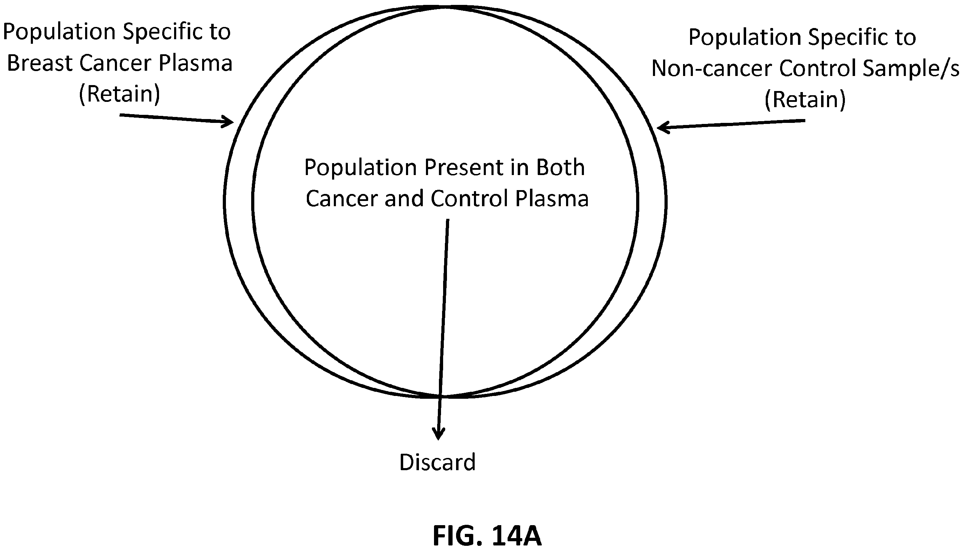

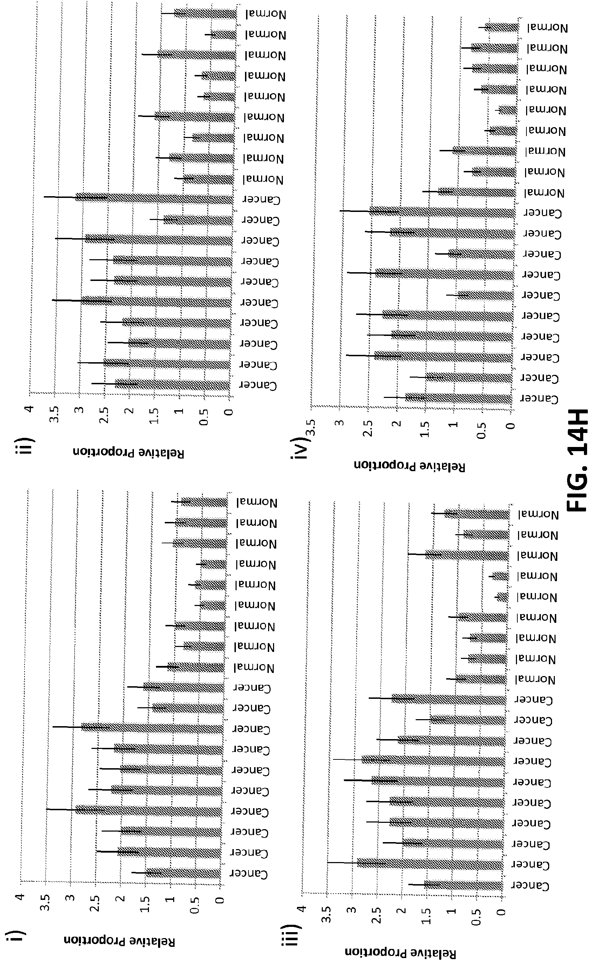

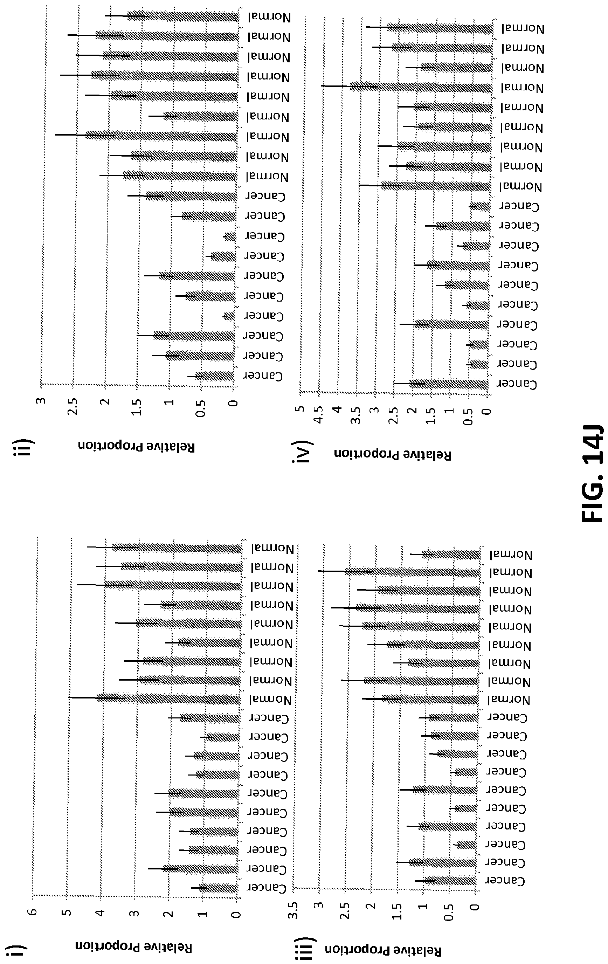

**FIGs. 14A-L** illustrate application of the approach outlined in **FIGs. 13A-I** applied to distinguish breast cancer and normal (i.e., non-cancer) samples by probing plasma-derived microvesicles with an oligonucleotide probe library.

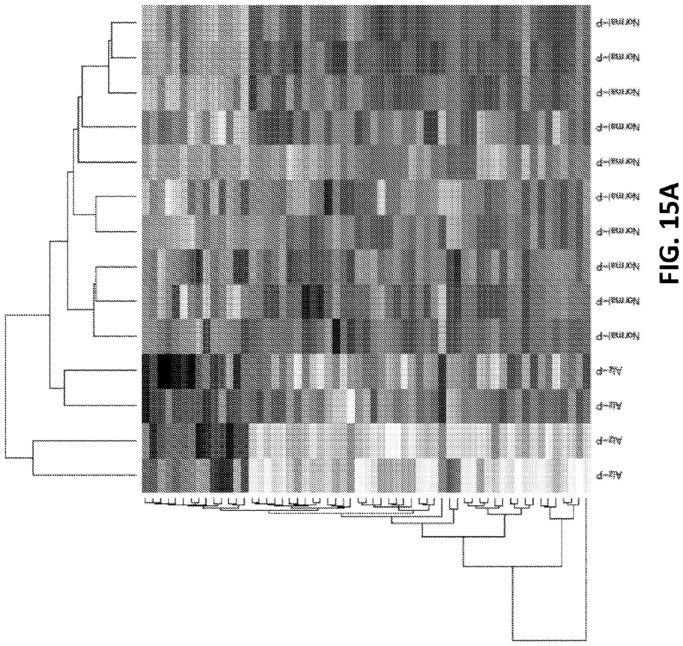

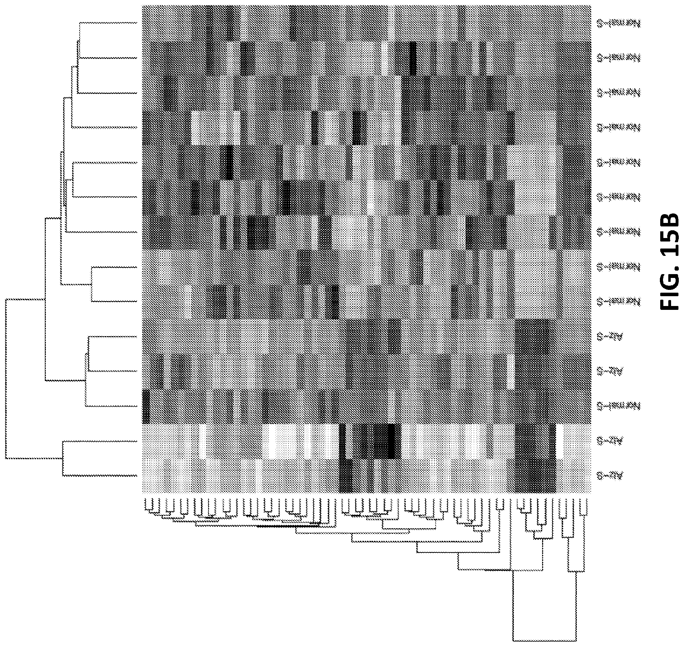

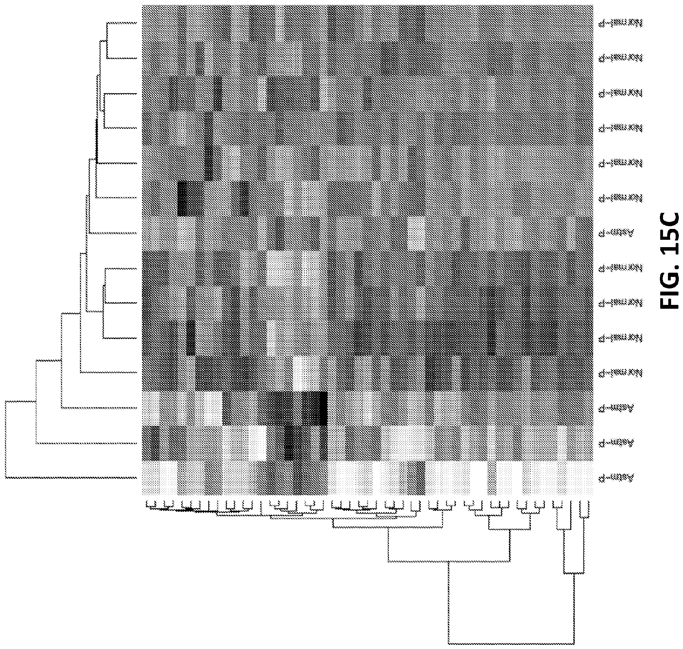

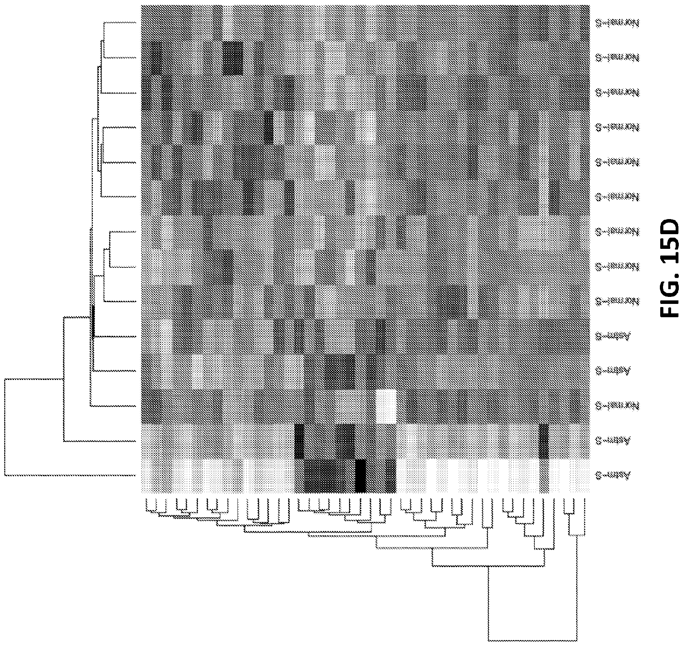

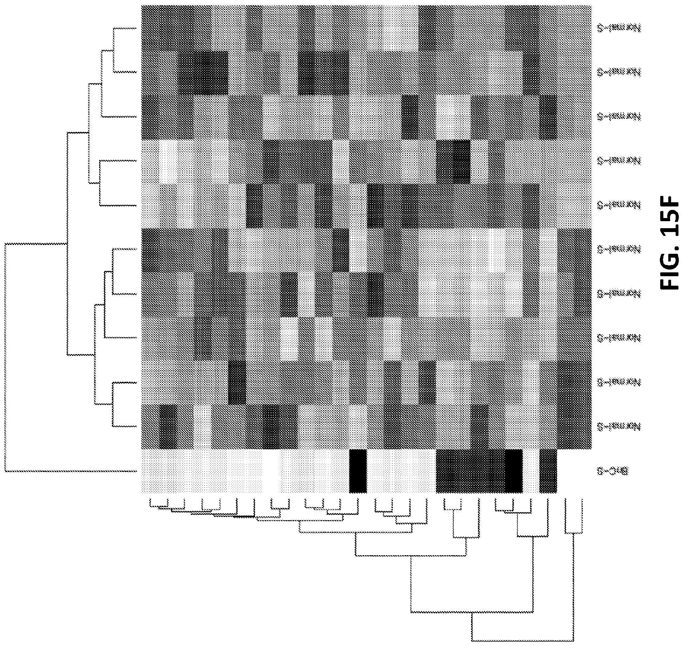

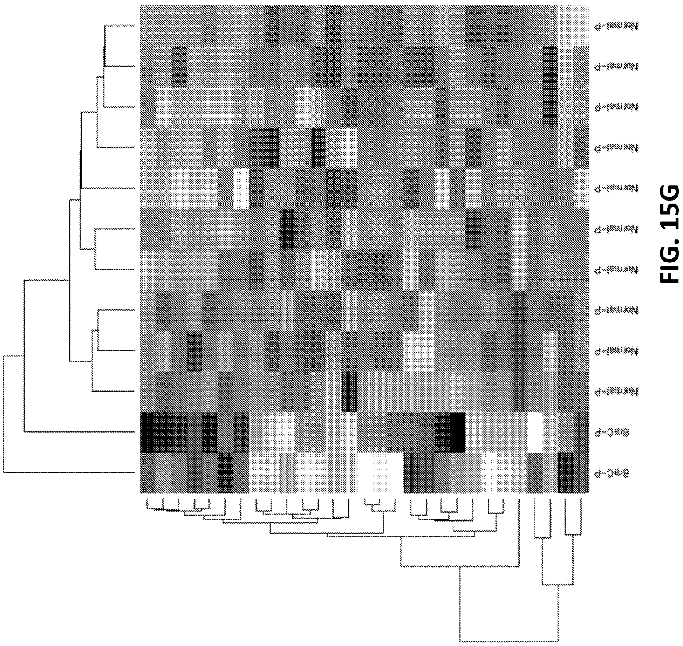

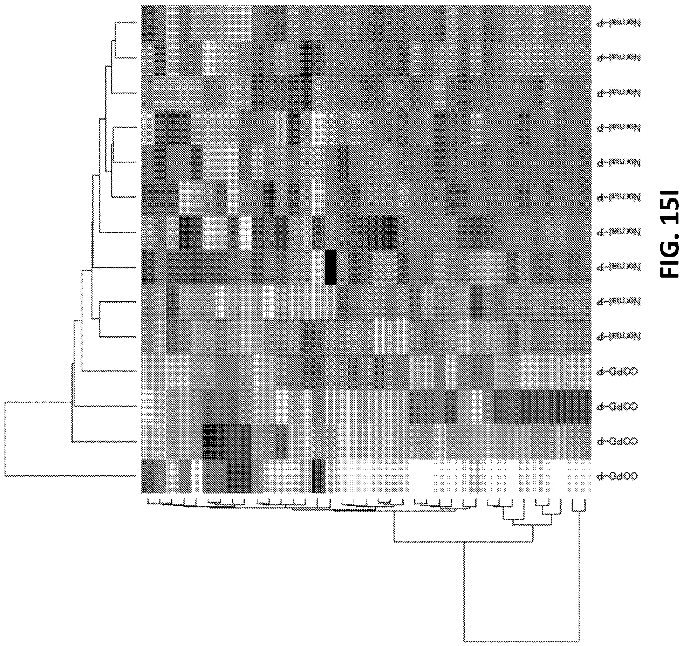

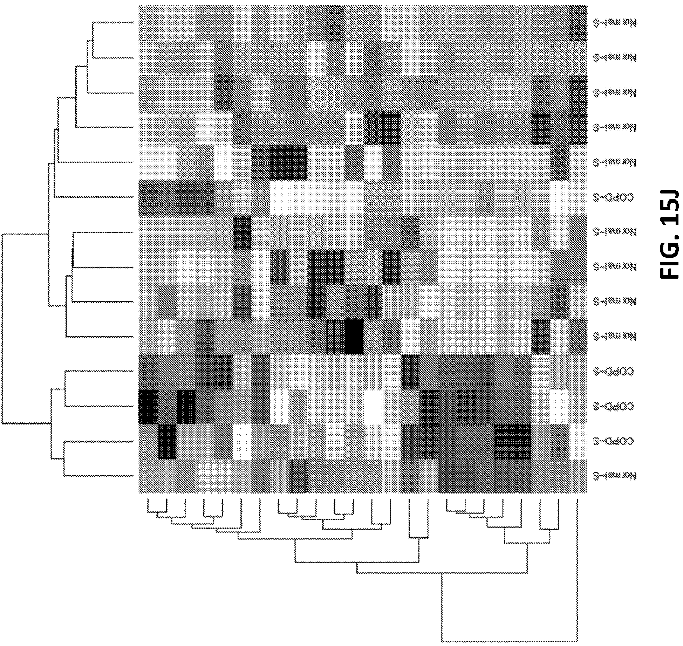

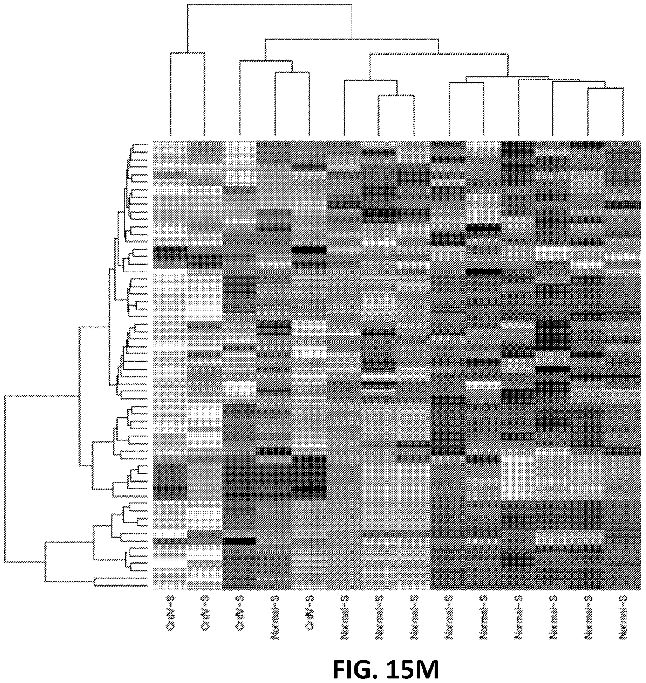

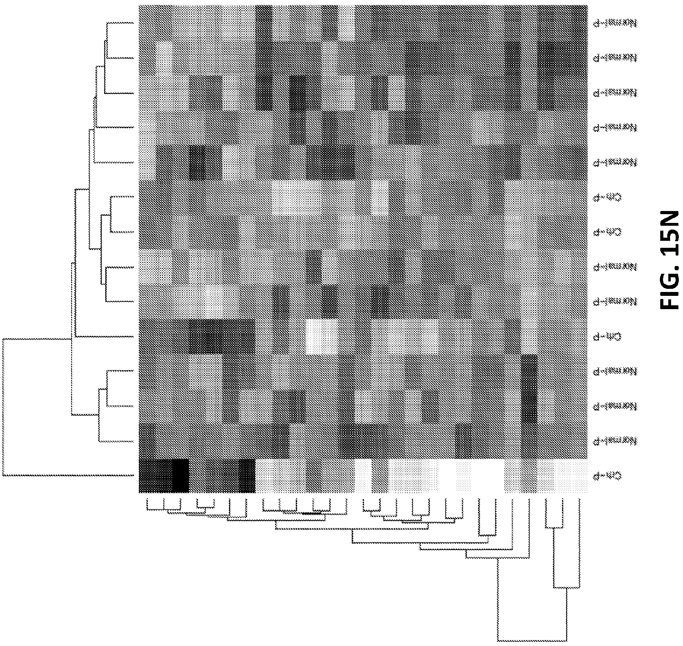

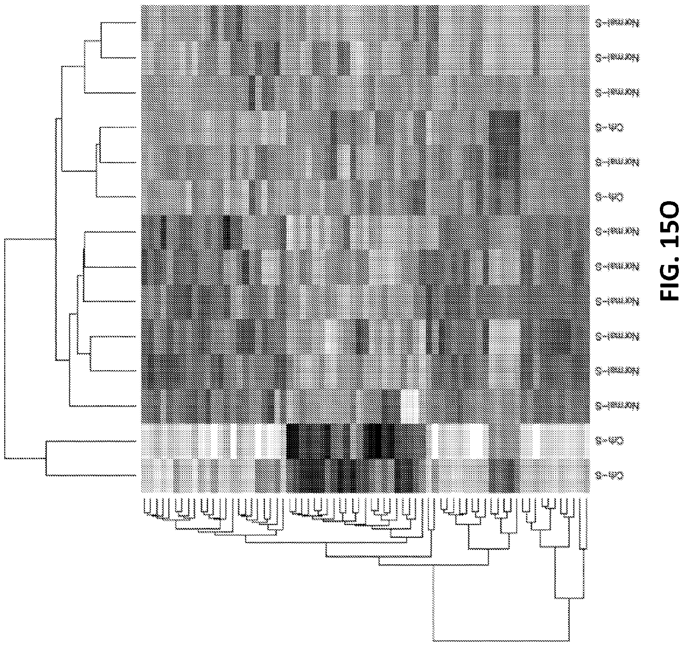

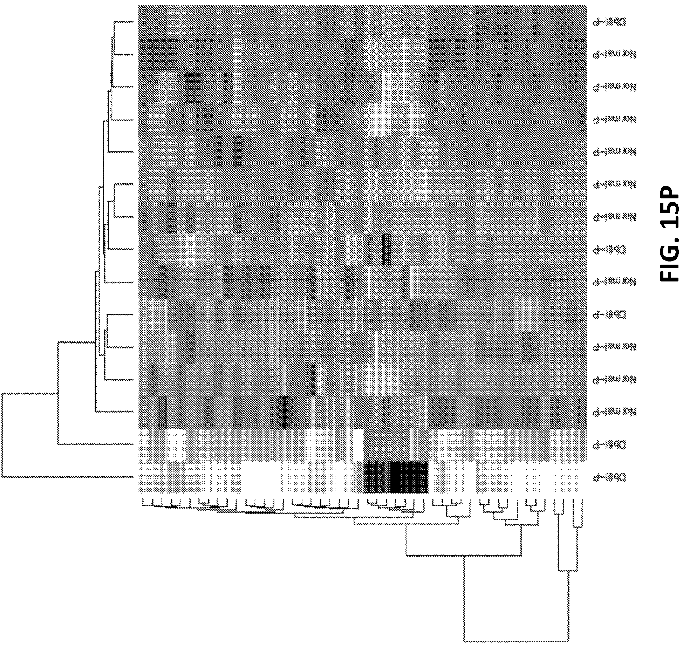

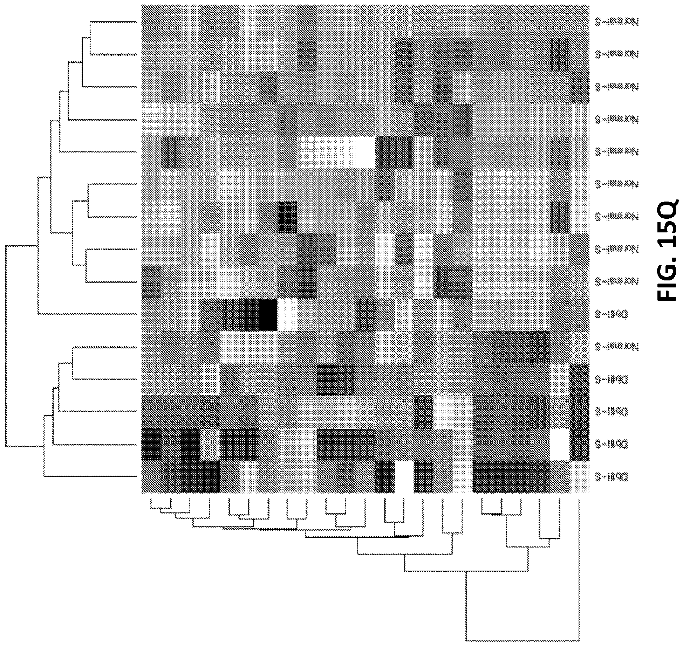

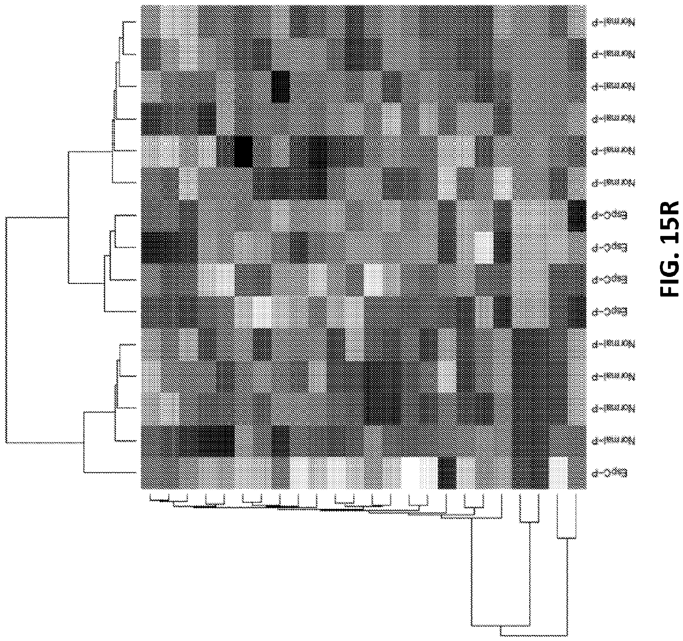

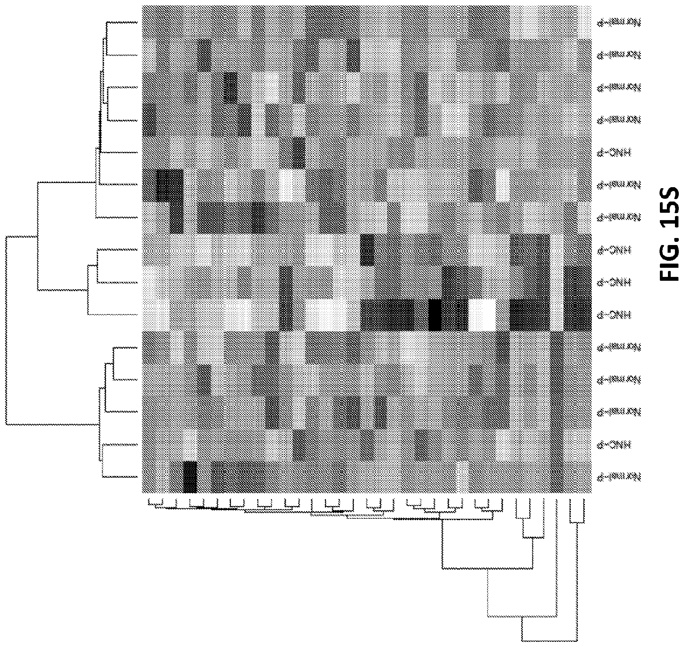

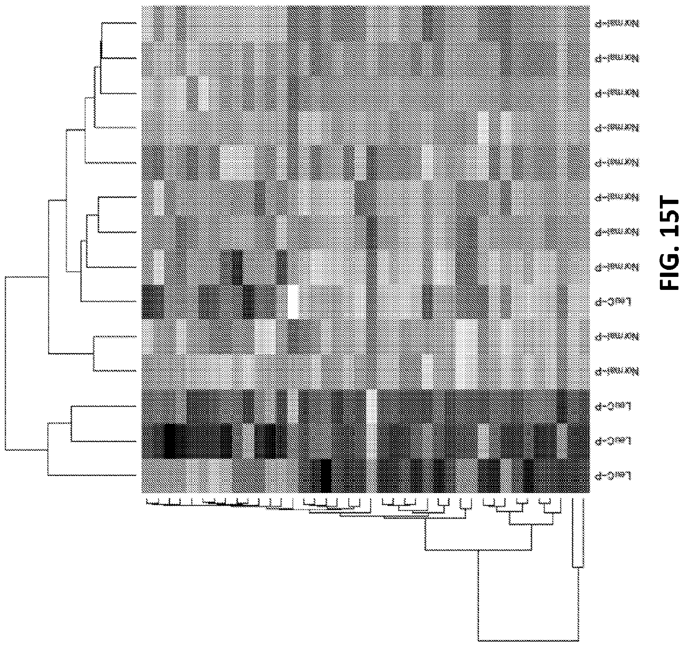

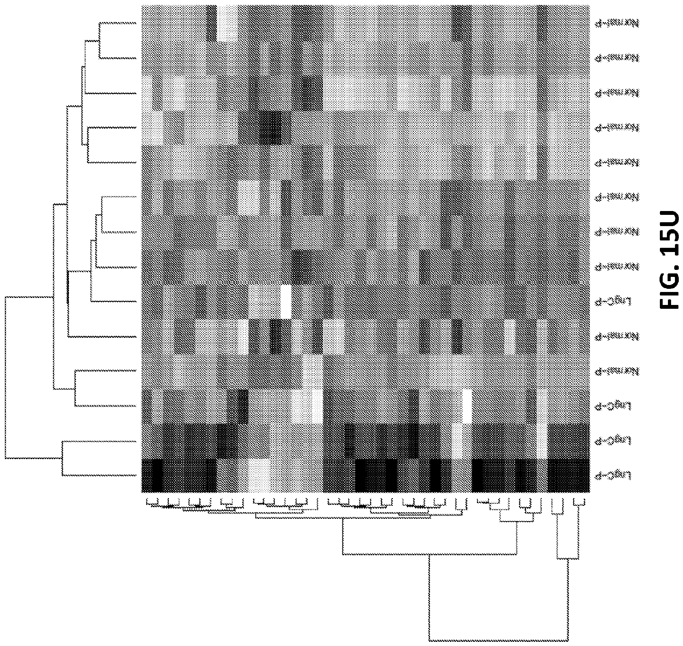

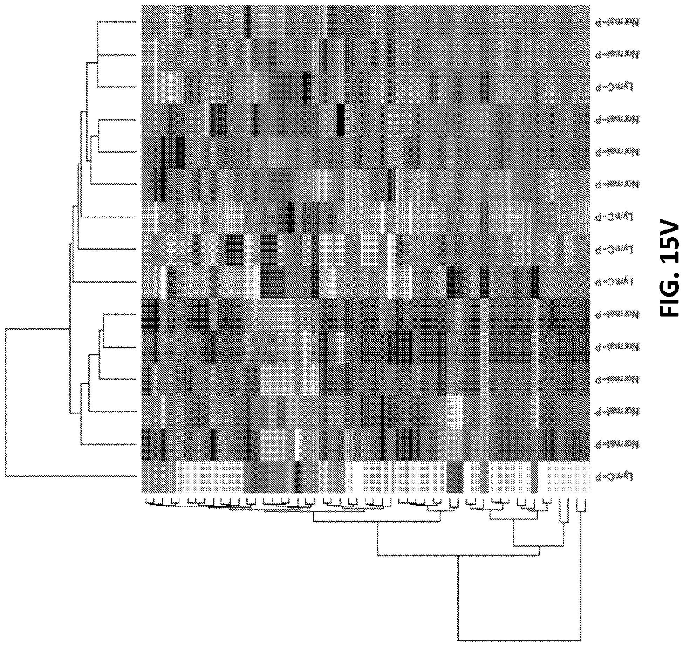

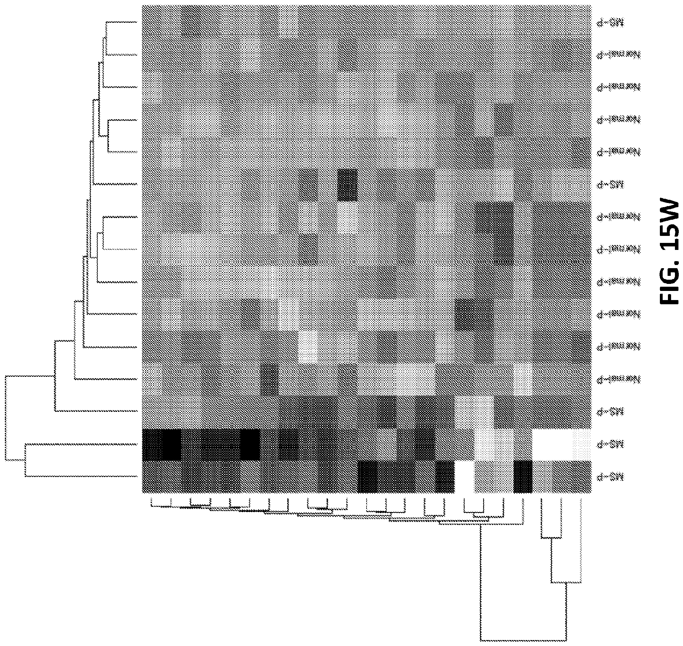

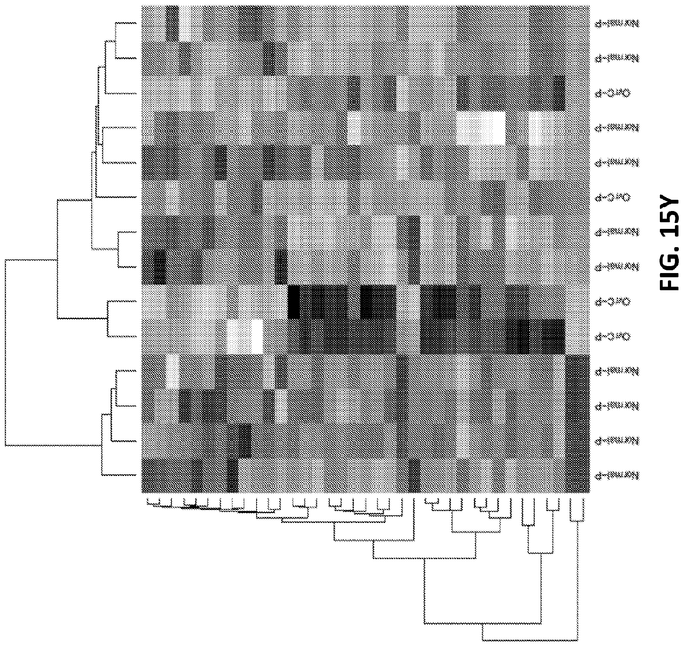

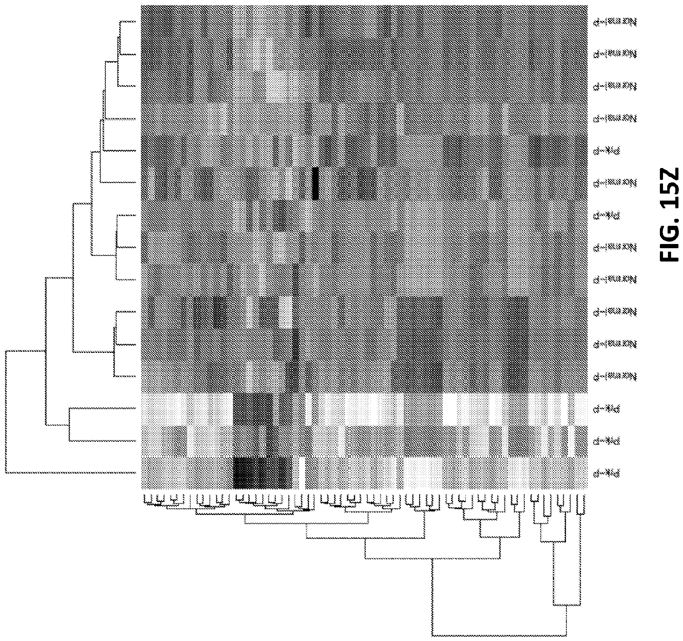

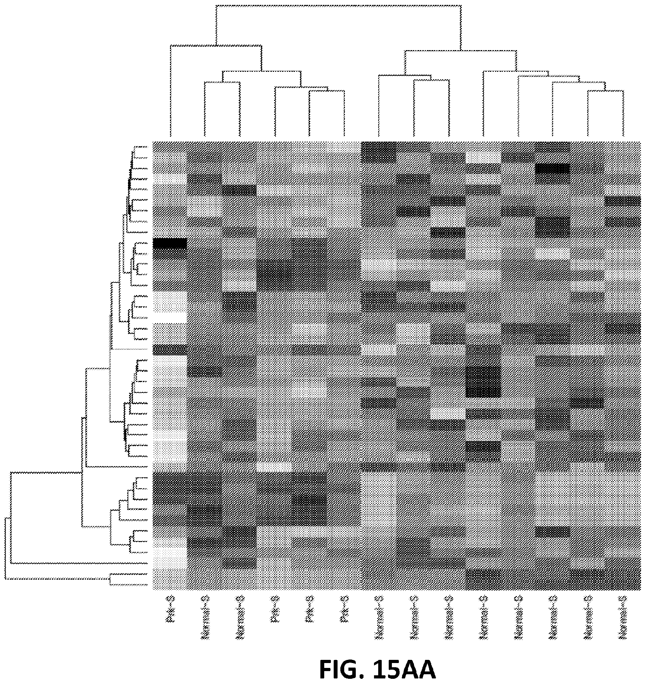

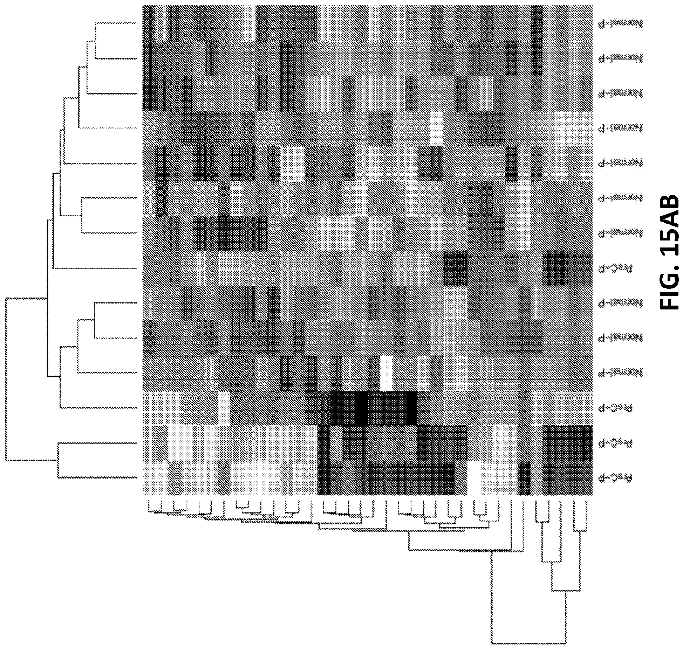

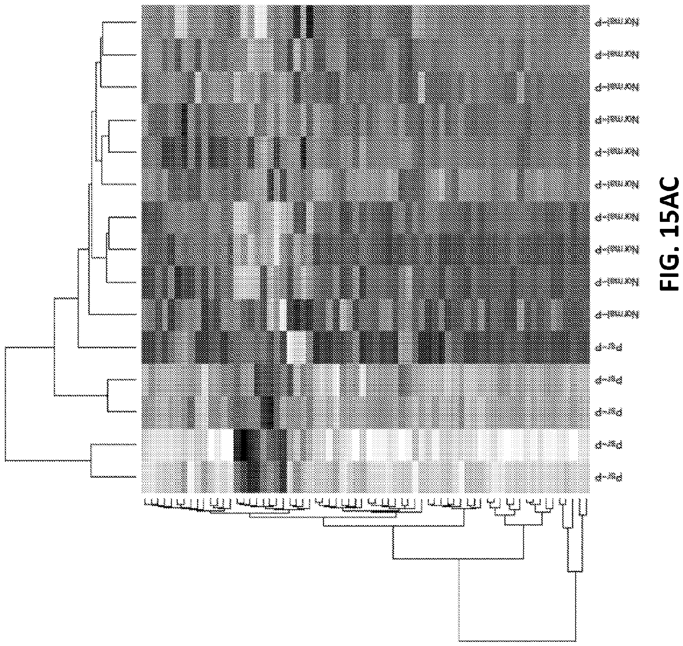

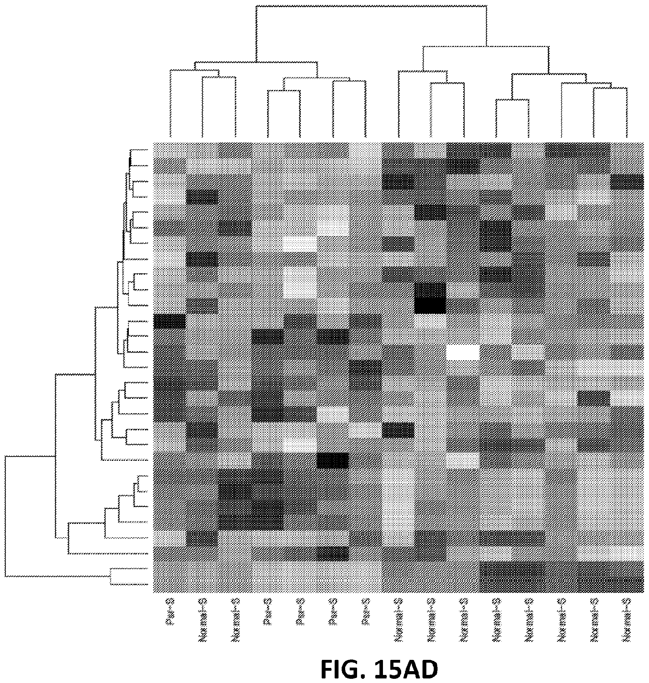

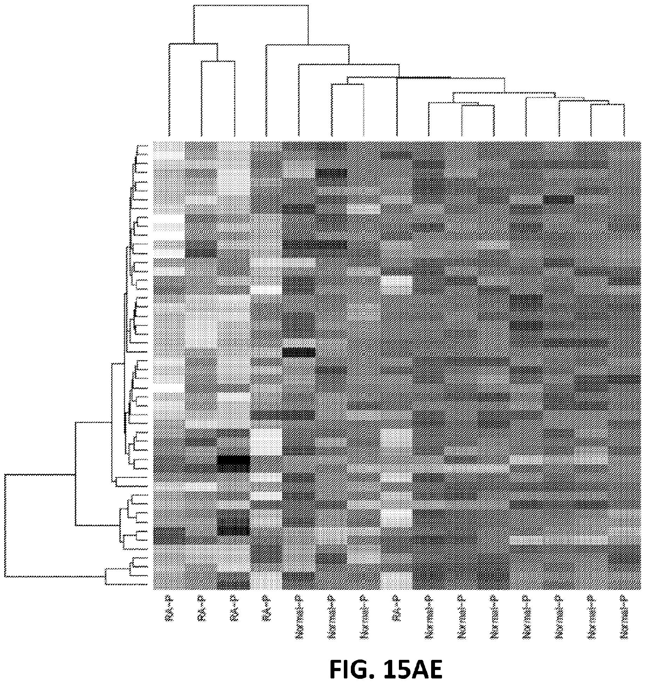

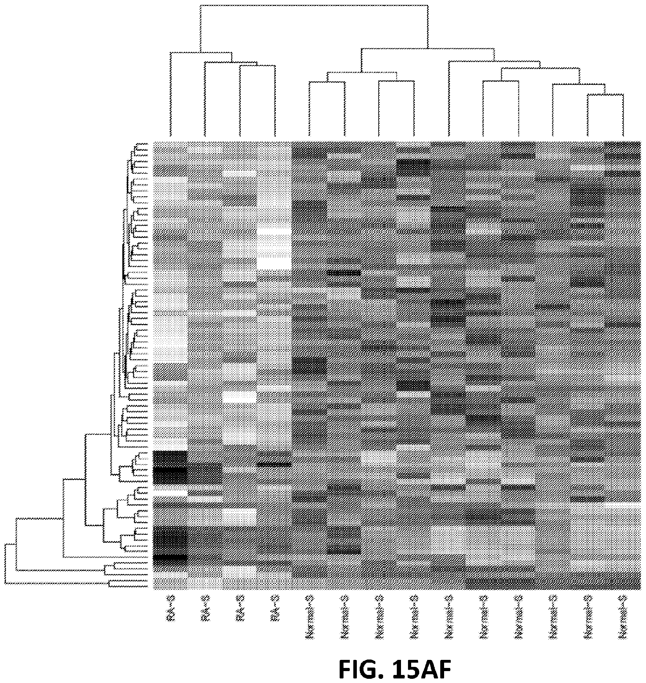

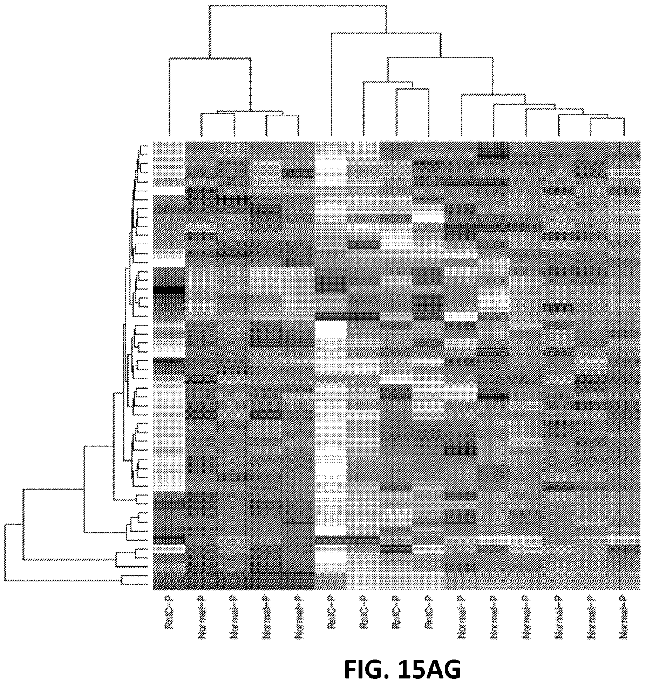

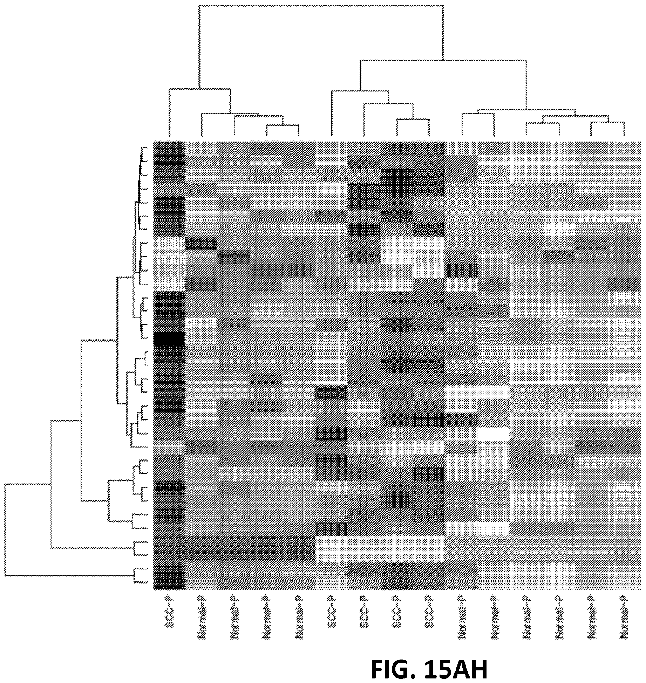

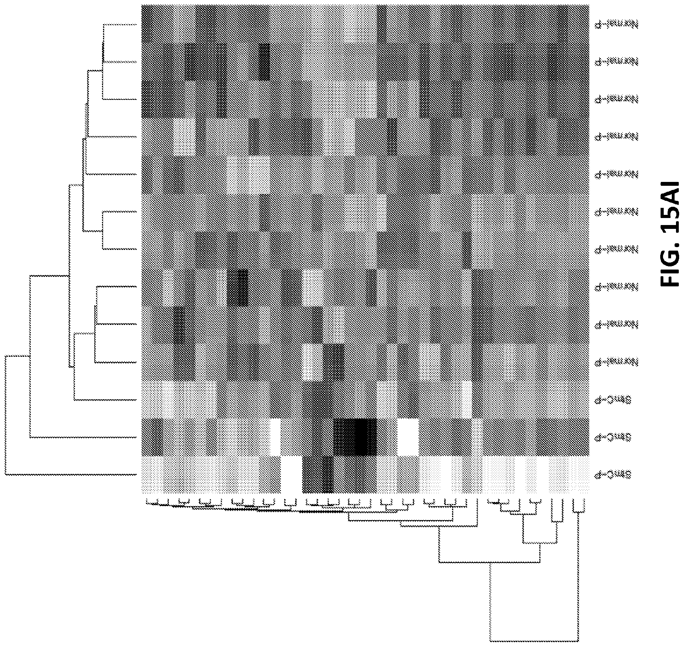

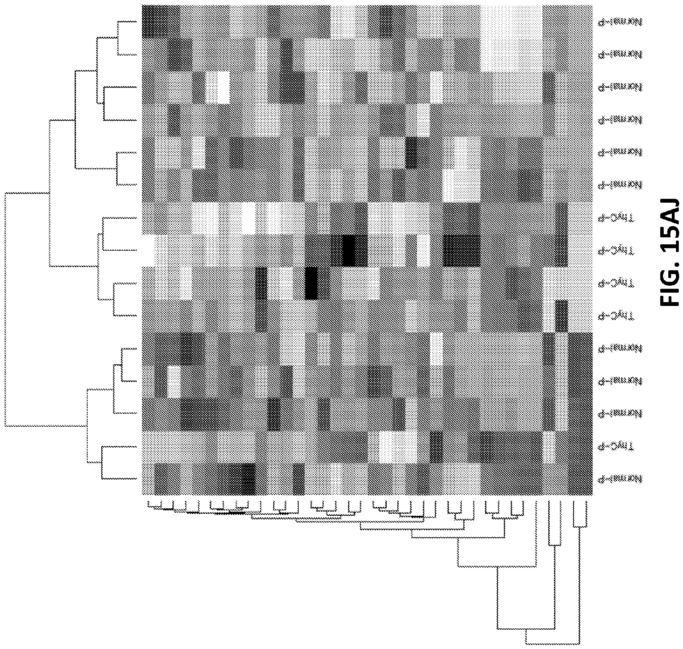

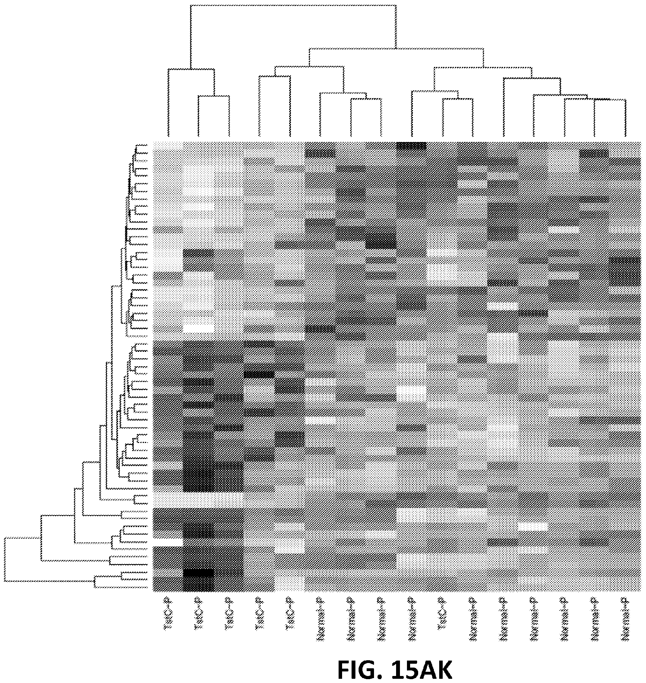

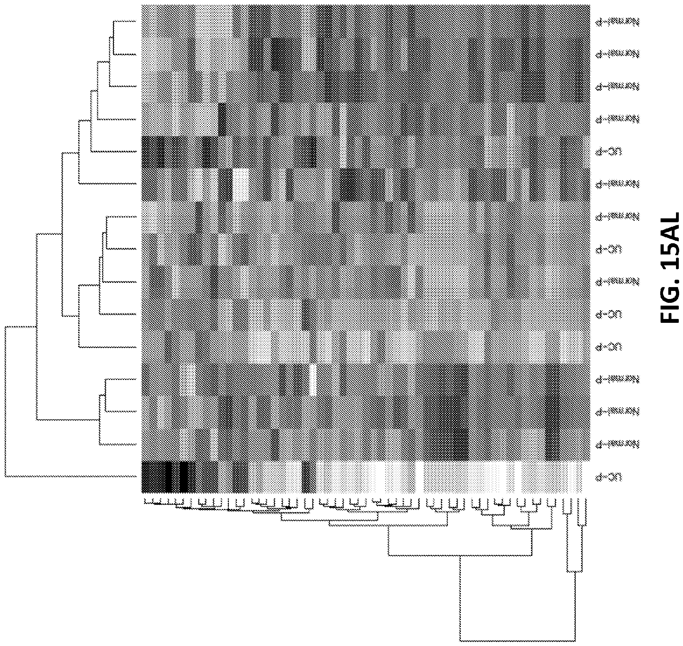

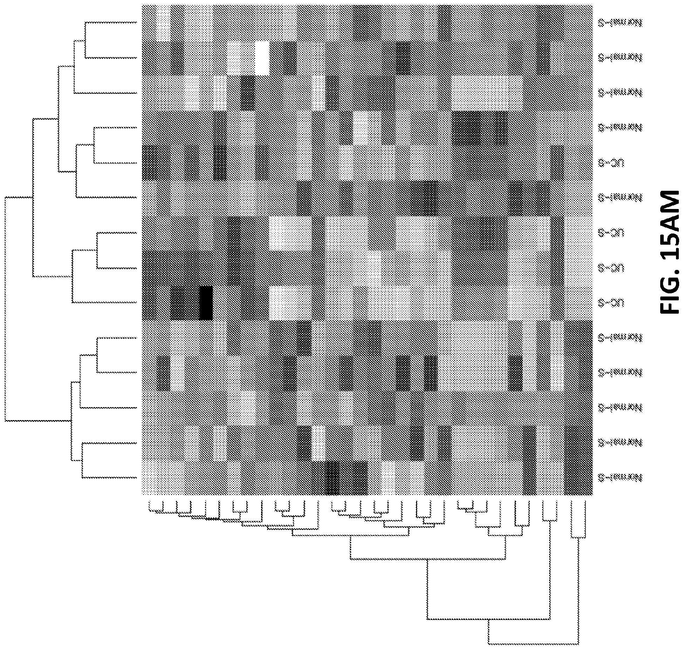

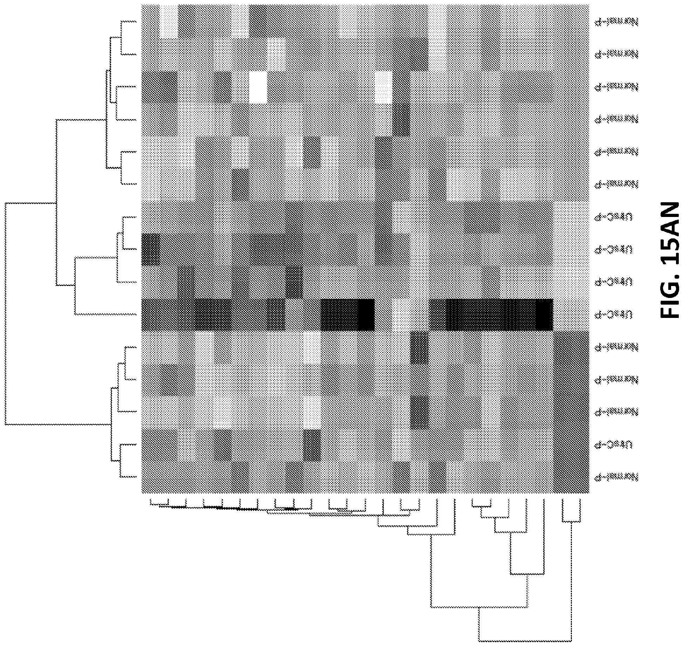

**FIGs. 15A-AN** comprise heat maps created using cluster analysis of oligonucleotide probe library analysis for various indications versus normal samples.

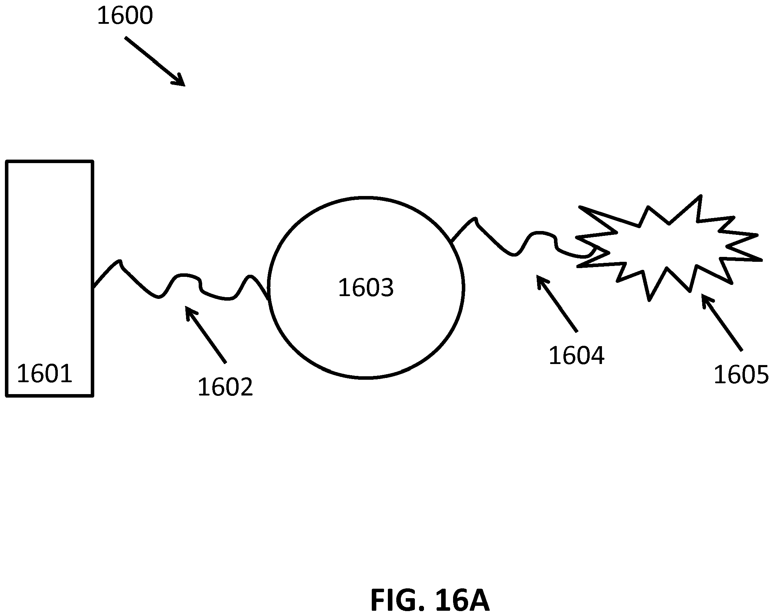

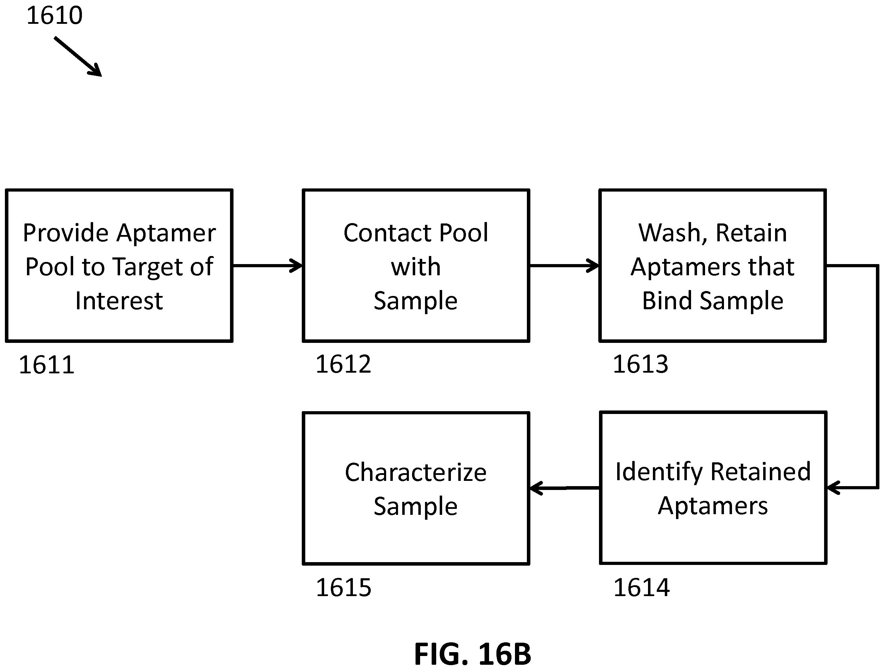

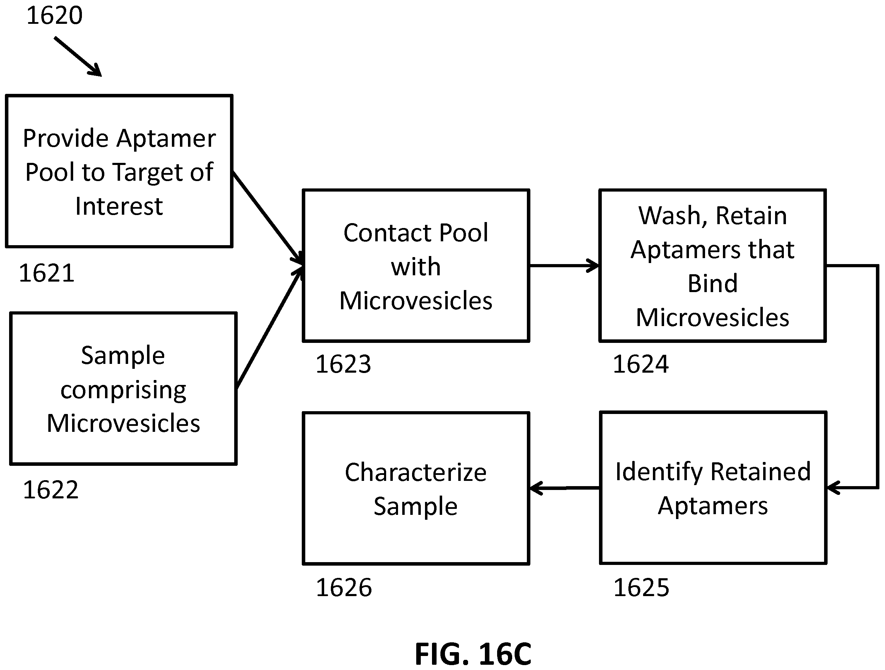

**FIGs. 16A-C** illustrate use of aptamers in methods of characterizing a phenotype. **FIG. 16A** is a schematic **1600** showing an assay configuration that can be used to detect and/or quantify a target of interest. In the figure, capture aptamer **1602** is attached to substrate **1601**. Target of interest **1603** is bound by capture aptamer **1602**. Detection aptamer **1604** is also bound to target of interest **1603**. Detection aptamer **1604** carries label **1605** which can be detected to identify target captured to substrate **1601** via capture aptamer **1602**. **FIG. 16B** is a schematic **1610** showing use of an aptamer pool to characterize a phenotype. A pool of aptamers to a target of interest is provided **1611**. The pool is contacted with a test sample to be characterized **1612**. The mixture is washed to remove unbound aptamers. The remaining aptamers are disassociated and collected **1613**. The collected aptamers are identified **1614** and the identity of the retained aptamers is used to characterize the phenotype **1615**. **FIG. 16C** is a schematic **1620** showing an implementation of the method in **FIG. 16B**. A pool of aptamers identified as binding a microvesicle population is provided **1621**. The input sample comprises microvesicles that are isolated from a test sample **1622**. The pool is contacted with the isolated microvesicles to be characterized **1623**. The mixture is washed to remove unbound aptamers and the remaining aptamers are disassociated and collected **1625**. The collected aptamers are identified and the identity of the retained aptamers is used to characterize the phenotype **1626**.

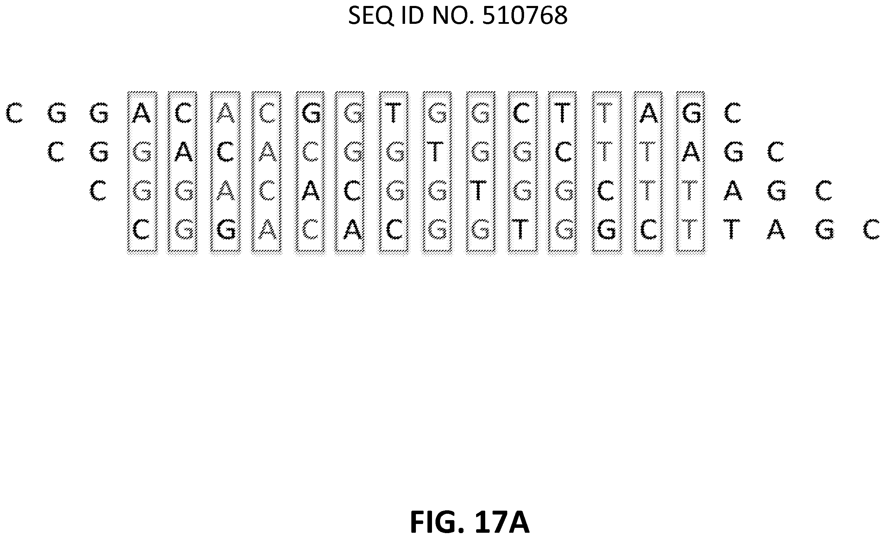

**FIGs. 17A-H** illustrate an optimized aptamer library design for high throughput (Next Generation) sequencing.

## DETAILED DESCRIPTION OF THE INVENTION

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present Specification will control.

Disclosed herein are compositions and methods that can be used to assess a biomarker profile, which can include a presence or level of one or more biomarkers. The compositions and methods comprise the use of aptamers that bind microvesicle surface antigens or a functional fragment thereof. The antigens typically comprise proteins or polypeptides but can be any component displayed on a microvesicle surface including lipids and/or carbohydrates. In general, aptamers disclosed are nucleic acid molecules, including DNA and RNA, and variations thereof. The methods disclosed comprise diagnostic processes and techniques using one or more aptamer, to determine level or presence of relevant microvesicle surface antigens or a functional fragment thereof. Alternatively, an aptamer can also be used as a binding agent to capture, isolate, or enrich, a cell, cell fragment, vesicle or any other fragment or complex that comprises the surface antigens or functional fragments thereof.

The compositions and methods comprise individual oligonucleotides that are identified for use in assessing a biomarker profile. The disclosure further discloses compositions and methods of oligonucleotide pools that can be used to detect a biomarker profile in a given sample.

Aptamers and sequences disclosed in the compositions and methods may be identified herein in the form of DNA or RNA. Unless otherwise specified, one of skill in the art will appreciate that an aptamer may generally be synthesized as either form of nucleic acid and carry various chemical modifications and remain within the scope of the disclosure. The term aptamer may be used in the art to refer to a single oligonucleotide that binds specifically to a target of interest through mechanisms other than Watson crick base pairing, similar to binding of a monoclonal antibody to a particular antigen. Within the scope of this disclosure and unless stated explicitly or otherwise implicit in context, the terms aptamer and oligonucleotide can be used interchangeably to refer to an oligonucleotide capable of distinguishing biological entities of interest (e.g, biomarkers) whether or not the specific entity has been identified or whether the precise mode of binding has been determined.

An aptamer can also be used to provide *in vitro* or *in vivo* detection or imaging, to provide any appropriate diagnostic readout (e.g., diagnostic, prognostic or theranostic).

Separately, an aptamer can also be used in for treatment or as a therapeutic to specifically target a cell, tissue or organ.

**Aptamers**

SELEX. A suitable method for generating an aptamer is with the process entitled "Systematic Evolution of Ligands by Exponential Enrichment" ("SELEX") generally described in, e.g., U.S. patent application Ser. No. 07/536,428, filed Jun. 11, 1990, now abandoned, U.S. Pat. No. 5,475,096 entitled "Nucleic Acid Ligands", and U.S. Pat. No. 5,270,163 (see also WO 91/19813) entitled "Nucleic Acid Ligands". Each SELEX-identified nucleic acid ligand, i.e., each aptamer, is a specific ligand of a given target compound or molecule. The SELEX process is based on the unique insight that nucleic acids have sufficient capacity for forming a variety of two- and three-dimensional structures and sufficient chemical versatility available within their monomers to act as ligands (i.e., form specific binding pairs) with virtually any chemical compound, whether monomeric or polymeric. Molecules of any size or composition can serve as targets.

SELEX relies as a starting point upon a large library or pool of single stranded oligonucleotides comprising randomized sequences. The oligonucleotides can be modified or unmodified DNA, RNA, or DNA/RNA hybrids. In some examples, the pool comprises 100% random or partially random oligonucleotides. In other examples, the pool comprises random or partially random oligonucleotides containing at least one fixed and/or conserved sequence incorporated within randomized sequence. In other examples, the pool comprises random or partially random oligonucleotides containing at least one fixed and/or conserved sequence at its 5' and/or 3' end which may comprise a sequence shared by all the molecules of the oligonucleotide pool. Fixed sequences are sequences such as hybridization sites for PCR primers, promoter sequences for RNA polymerases (e.g., T3, T4, T7, and SP6), restriction sites, or homopolymeric sequences, such as poly A or poly T tracts, catalytic cores, sites for selective binding to affinity columns, and other sequences to facilitate cloning and/or sequencing of an oligonucleotide of interest. Conserved sequences are sequences, other than the previously described fixed sequences, shared by a number of aptamers that bind to the same target.

The oligonucleotides of the pool preferably include a randomized sequence portion as well as fixed sequences useful for efficient amplification. Typically the oligonucleotides of the starting pool contain fixed 5' and 3' terminal sequences which flank an internal region of 30-50 random nucleotides. The randomized nucleotides can be produced in a number of ways including chemical synthesis and size selection from randomly cleaved cellular nucleic acids. Sequence variation in test nucleic acids can also be introduced or increased by mutagenesis before or during the selection/amplification iterations.

The random sequence portion of the oligonucleotide can be of any length and can comprise ribonucleotides and/or deoxyribonucleotides and can include modified or non-natural nucleotides or nucleotide analogs. See, e.g. U.S. Pat. No. 5,958,691; U.S. Pat. No. 5,660,985; U.S. Pat. No. 5,958,691; U.S. Pat. No. 5,698,687; U.S. Pat. No. 5,817,635; U.S. Pat. No. 5,672,695, and PCT Publication WO 92/07065. Random oligonucleotides can be synthesized from phosphodiester-linked nucleotides using solid phase oligonucleotide synthesis techniques well known in the art. See, e.g., Froehler et al., Nucl. Acid Res. 14:5399-5467 (1986) and Froehler et al., Tet. Lett. 27:5575-5578 (1986). Random oligonucleotides can also be synthesized using solution phase methods such as triester synthesis methods. See, e.g., Sood et al., Nucl. Acid Res. 4:2557 (1977) and Hirose et al., Tet. Lett., 28:2449 (1978). Typical syntheses carried out on automated DNA synthesis equipment yield $10^{14}$-$10^{16}$ individual molecules, a number sufficient for most SELEX experiments. Sufficiently large regions of random sequence in the sequence design increases the likelihood that each synthesized molecule is likely to represent a unique sequence.

The starting library of oligonucleotides may be generated by automated chemical synthesis on a DNA synthesizer. To synthesize randomized sequences, mixtures of all four nucleotides are added at each nucleotide addition step during the synthesis process, allowing for random incorporation of nucleotides. As stated above, random oligonucleotides may comprise entirely random sequences; however, random oligonucleotides can comprise stretches of nonrandom or partially random sequences. Partially random sequences can be created by adding the four nucleotides in different molar ratios at each addition step.

The starting library of oligonucleotides may be for example, RNA, DNA, or RNA/DNA hybrid. In those instances where an RNA library is to be used as the starting library it is typically generated by transcribing a DNA library in vitro using T7 RNA polymerase or modified T7 RNA polymerases and purified. The library is then mixed with the target under conditions favorable for binding and subjected to step-wise iterations of binding, partitioning and amplification, using the same general selection scheme, to achieve virtually any desired criterion of binding affinity and selectivity. More specifically, starting with a mixture containing the starting pool of nucleic acids, the SELEX method includes steps of: (a) contacting the mixture with the target under conditions favorable for binding; (b) partitioning unbound nucleic acids from those nucleic acids which have bound specifically to target molecules; (c) dissociating the nucleic acid-target complexes; (d) amplifying the nucleic acids dissociated from the nucleic acid-target complexes to yield a ligand-enriched mixture of nucleic acids; and (e) reiterating the steps of binding, partitioning, dissociating and amplifying through as many cycles as desired to yield highly specific, high affinity nucleic acid ligands to the target molecule. In those instances where RNA aptamers are being selected, the SELEX method further comprises the steps of: (i) reverse transcribing the nucleic acids dissociated from the nucleic acid-target complexes before amplification in step (d); and (ii) transcribing the amplified nucleic acids from step (d) before restarting the process.

Within a nucleic acid mixture containing a large number of possible sequences and structures, there is a wide range of binding affinities for a given target. A nucleic acid mixture comprising, for example, a 20 nucleotide randomized segment can have $4^{20}$ candidate possibilities. Those which have the higher affinity constants for the target are most likely to bind to the target. After partitioning, dissociation and amplification, a second nucleic acid mixture is generated, enriched for the higher binding affinity candidates. Additional rounds of selection progressively favor better ligands until the resulting nucleic acid mixture is predominantly composed of only one or a few sequences. These can then be cloned, sequenced and individually tested for binding affinity as pure ligands or aptamers.

Cycles of selection and amplification are repeated until a desired goal is achieved. In the most general case, selection/amplification is continued until no significant improvement in binding strength is achieved on repetition of the cycle. The method is typically used to sample approximately $10^{14}$ different nucleic acid species but may be used to sample as many as about $10^{18}$ different nucleic acid species. Generally, nucleic acid aptamer molecules are selected in a 5 to 20 cycle procedure. Heterogeneity may be introduced only in the initial selection stages and does not occur throughout the replicating process.

In one aspect of SELEX, the selection process is so efficient at isolating those nucleic acid ligands that bind most strongly to the selected target, that only one cycle of selection and amplification is required. Such an efficient selection may occur, for example, in a chromatographic-type process wherein the ability of nucleic acids to associate with targets bound on a column operates in such a manner that the column is sufficiently able to allow separation and isolation of the highest affinity nucleic acid ligands.

In many cases, it is not necessarily desirable to perform the iterative steps of SELEX until a single nucleic acid ligand is identified. The target-specific nucleic acid ligand solution may include a family of nucleic acid structures or motifs that have a number of conserved sequences and a number of sequences which can be substituted or added without significantly affecting the affinity of the nucleic acid ligands to the target. By terminating the SELEX process prior to completion, it is possible to determine the sequence of a number of members of the nucleic acid ligand solution family. The disclosure provides for the identification of aptamer pools and uses thereof that jointly can be used to characterize a test sample. For example, the aptamer pools can be identified through rounds of positive and negative selection to identify microvesicle indicative of a disease or condition. The disclosure further provides use of such aptamer pools to detect and/or quantify such microvesicles in a sample, thereby allowing a diagnosis, prognosis or theranosis to be provided.

A variety of nucleic acid primary, secondary and tertiary structures are known to exist. The structures or motifs that have been shown most commonly to be involved in non-Watson-Crick type interactions are referred to as hairpin loops, symmetric and asymmetric bulges, pseudoknots and myriad combinations of the same. Almost all known cases of such motifs suggest that they can be formed in a nucleic acid sequence of no more than 30 nucleotides. For this reason, it is often preferred that SELEX procedures with contiguous randomized segments be initiated with nucleic acid sequences containing a randomized segment of between about 20 to about 50 nucleotides and in some aspects, about 30 to about 40 nucleotides. In one example, the 5'-fixed:random:3'-fixed sequence comprises a random sequence of about 30 to about 50 nucleotides.

The core SELEX method has been modified to achieve a number of specific objectives. For example, U.S. Pat. No. 5,707,796 describes the use of SELEX in conjunction with gel electrophoresis to select nucleic acid molecules with specific structural characteristics, such as bent DNA. U.S. Pat. No. 5,763,177 describes SELEX based methods for selecting nucleic acid ligands containing photoreactive groups capable of binding and/or photocrosslinking to and/or photoinactivating a target molecule. U.S. Pat. No. 5,567,588 and U.S. Pat. No. 5,861,254 describe SELEX based methods which achieve highly efficient partitioning between oligonucleotides having high and low affinity for a target molecule. U.S. Pat. No. 5,496,938 describes methods for obtaining improved nucleic acid ligands after the SELEX process has been performed. U.S. Pat. No. 5,705,337 describes methods for covalently linking a ligand to its target.

SELEX can also be used to obtain nucleic acid ligands that bind to more than one site on the target molecule, and to obtain nucleic acid ligands that include non-nucleic acid species that bind to specific sites on the target. SELEX provides means for isolating and identifying nucleic acid ligands which bind to any envisionable target, including large and small biomolecules such as nucleic acid-binding proteins and proteins not known to bind nucleic acids as part of their biological function as well as cofactors and other small molecules. For example, U.S. Pat. No. 5,580,737 discloses nucleic acid sequences identified through SELEX which are capable of binding with high affinity to caffeine and the closely related analog, theophylline.

Counter-SELEX is a method for improving the specificity of nucleic acid ligands to a target molecule by eliminating nucleic acid ligand sequences with cross-reactivity to one or more non-target molecules. Counter-SELEX is comprised of the steps of: (a) preparing a candidate mixture of nucleic acids; (b) contacting the candidate mixture with the target, wherein nucleic acids having an increased affinity to the target relative to the candidate mixture may be partitioned from the remainder of the candidate mixture; (c) partitioning the increased affinity nucleic acids from the remainder of the candidate mixture; (d) dissociating the increased affinity nucleic acids from the target; e) contacting the increased affinity nucleic acids with one or more non-target molecules such that nucleic acid ligands with specific affinity for the non-target molecule(s) are removed; and (f) amplifying the nucleic acids with specific affinity only to the target molecule to yield a mixture of nucleic acids enriched for nucleic acid sequences with a relatively higher affinity and specificity for binding to the target molecule. As described above for SELEX, cycles of selection and amplification are repeated until a desired goal is achieved.

One potential problem encountered in the use of nucleic acids as therapeutics and vaccines is that oligonucleotides in their phosphodiester form may be quickly degraded in body fluids by intracellular and extracellular enzymes such as endonucleases and exonucleases before the desired effect is manifest. The SELEX method thus encompasses the identification of high-affinity nucleic acid ligands containing modified nucleotides conferring improved characteristics on the ligand, such as improved in vivo stability or improved delivery characteristics. Examples of such modifications include chemical substitutions at the ribose and/or phosphate and/or base positions. SELEX identified nucleic acid ligands containing modified nucleotides are described, e.g., in U.S. Pat. No. 5,660,985, which describes oligonucleotides containing nucleotide derivatives chemically modified at the 2' position of ribose, 5 position of pyrimidines, and 8 position of purines, U.S. Pat. No. 5,756,703 which describes oligonucleotides containing various 2'-modified pyrimidines, and U.S. Pat. No. 5,580,737 which describes highly specific nucleic acid ligands containing one or more nucleotides modified with 2'-amino (2'--$NH_2$), 2'-fluoro (2'-F), and/or 2'-O-methyl (2'-OMe) substituents.

Modifications of the nucleic acid ligands contemplated include, but are not limited to, those which provide other chemical groups that incorporate additional charge, polarizability, hydrophobicity, hydrogen bonding, electrostatic interaction, and fluxionality to the nucleic acid ligand bases or to the nucleic acid ligand as a whole. Modifications to generate oligonucleotide populations which are resistant to nucleases can also include one or more substitute internucleotide linkages, altered sugars, altered bases, or combinations thereof. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, phosphorothioate or allyl phosphate modifications, methylations, and unusual base-pairing combinations such as the isobases isocytidine and isoguanosine. Modifications can also include 3' and 5' modifications such as capping.

In one aspect, oligonucleotides are provided in which the P(O)O group is replaced by P(O)S ("thioate"), P(S)S ("dithioate"), P(O)$NR_2$ ("amidate"), P(O)R, P(O)OR', CO or $CH_2$ ("formacetal") or 3'-amine (--NH--$CH_2$--$CH_2$--), wherein each R or R' is independently H or substituted or unsubstituted alkyl. Linkage groups can be attached to adjacent nucleotides through an --O--, --N--, or --S-- linkage. Not all linkages in the oligonucleotide are required to be identical. As used herein, the term phosphorothioate encompasses one or more non-bridging oxygen atoms in a phosphodiester bond replaced by one or more sulfur atoms.

The oligonucleotides may comprise modified sugar groups, for example, one or more of the hydroxyl groups is replaced with halogen, aliphatic groups, or functionalized as ethers or amines. The 2'-position of the furanose residue may be substituted by any of an O-methyl, O-alkyl, O-allyl, S-alkyl, S-allyl, or halo group. Methods of synthesis of 2'-modified sugars are described, e.g., in Sproat, et al., Nucl. Acid Res. 19:733-738 (1991); Cotten, et al., Nucl. Acid Res. 19:2629-2635 (1991); and Hobbs, et al., Biochemistry 12:5138-5145 (1973). Other modifications are known to one of ordinary skill in the art. Such modifications may be pre-SELEX process modifications or post-SELEX process modifications (modification of previously identified unmodified ligands) or may be made by incorporation into the SELEX process.

Pre-SELEX process modifications or those made by incorporation into the SELEX process yield nucleic acid ligands with both specificity for their SELEX target and improved stability, e.g., in vivo stability. Post-SELEX process modifications made to nucleic acid ligands may result in improved stability, e.g., in vivo stability without adversely affecting the binding capacity of the nucleic acid ligand.

The SELEX method encompasses combining selected oligonucleotides with other selected oligonucleotides and non-oligonucleotide functional units as described in U.S. Pat. No. 5,637,459 and U.S. Pat. No. 5,683,867. The SELEX method further encompasses combining selected nucleic acid ligands with lipophilic or non-immunogenic high molecular weight compounds in a diagnostic or therapeutic complex, as described, e.g., in U.S. Pat. No. 6,011,020, U.S. Pat. No. 6,051,698, and PCT Publication No. WO 98/18480. These patents and applications teach the combination of a broad array of shapes and other properties, with the efficient amplification and replication properties of oligonucleotides, and with the desirable properties of other molecules.

The identification of nucleic acid ligands to small, flexible peptides via the SELEX method has also been explored. Small peptides have flexible structures and usually exist in solution in an equilibrium of multiple conformers, and thus it was initially thought that binding affinities may be limited by the conformational entropy lost upon binding a flexible peptide. However, the feasibility of identifying nucleic acid ligands to small peptides in solution was demonstrated in U.S. Pat. No. 5,648,214. In this patent, high affinity RNA nucleic acid ligands to substance P, an 11 amino acid peptide, were identified.

The aptamers with specificity and binding affinity to the target(s) can be selected by the SELEX N process as described herein. As part of the SELEX process, the sequences selected to bind to the target are then optionally minimized to determine the minimal sequence having the desired binding affinity. The selected sequences and/or the minimized sequences are optionally optimized by performing random or directed mutagenesis of the sequence to increase binding affinity or alternatively to determine which positions in the sequence are essential for binding activity. Additionally, selections can be performed with sequences incorporating modified nucleotides to stabilize the aptamer molecules against degradation in vivo.

### 2' Modified SELEX

In order for an aptamer to be suitable for use as a therapeutic, it is preferably inexpensive to synthesize, safe and stable in vivo. Wild-type RNA and DNA aptamers are typically not stable is vivo because of their susceptibility to degradation by nucleases. Resistance to nuclease degradation can be greatly increased by the incorporation of modifying groups at the 2'-position.

Fluoro and amino groups have been successfully incorporated into oligonucleotide pools from which aptamers have been subsequently selected. However, these modifications greatly increase the cost of synthesis of the resultant aptamer, and may introduce safety concerns in some cases because of the possibility that the modified nucleotides could be recycled into host DNA by degradation of the modified oligonucleotides and subsequent use of the nucleotides as substrates for DNA synthesis.

Aptamers that contain 2'-O-methyl ("2'-OMe") nucleotides, as disclosed herein, may overcome one or more potential drawbacks. Oligonucleotides containing 2'-OMe nucleotides are nuclease-resistant and inexpensive to synthesize. Although 2'-OMe nucleotides are ubiquitous in biological systems, natural polymerases do not accept 2'-OMe NTPs as substrates under physiological conditions, thus there are no safety concerns over the recycling of 2'-OMe nucleotides into host DNA. The SELEX method used to generate 2'-modified aptamers is described, e.g., in U.S. Provisional Patent Application Ser. No. 60/430,761, filed Dec. 3, 2002, U.S. Provisional Patent Application Ser. No. 60/487,474, filed Jul. 15, 2003, U.S. Provisional Patent Application Ser. No. 60/517,039, filed Nov. 4, 2003, U.S. patent application Ser. No. 10/729,581, filed Dec. 3, 2003, and U.S. patent application Ser. No. 10/873,856, filed Jun. 21, 2004, entitled "Method for in vitro Selection of 2'-O-methyl substituted Nucleic Acids,".

### METHODS

### Biomarker Detection and Diagnostics

The aptamers can be used in various methods to assess presence or level of biomarkers in a biological sample, e.g., biological entities of interest such as proteins, nucleic acids, or microvesicles. The aptamer functions as a binding agent to assess presence or level of the cognate target molecule. Therefore, in various aspects of the disclosure directed to diagnostics, prognostics or theranostics, one or more aptamers are configured in a ligand-target based assay, where one or more aptamer is contacted with a selected biological sample, where the or more aptamer associates with or binds to its target molecules. Aptamers are used to identify candidate biosignatures based on the biological samples assessed and biomarkers detected. Aptamers may themselves provide a biosignature for a particular condition or disease. A biosignature refers to a biomarker profile of a biological sample comprising a presence, level or other characteristic that can be assessed (including without limitation a sequence, mutation, rearrangement, translocation, deletion, epigenetic modification, methylation, post-translational modification, allele, activity, complex partners, stability, half life, and the like) of one or more biomarker of interest. Biosignatures can be used to evaluate diagnostic and/or prognostic criteria such as presence of disease, disease staging, disease monitoring, disease stratification, or surveillance for detection, metastasis or recurrence or progression of disease. For example, methods using aptamers against microvesicle surface antigen are useful for correlating a biosignature comprising microvesicle antigens to a selected condition or disease. A biosignature can also be used clinically in making decisions concerning treatment modalities including therapeutic intervention. A biosignature can further be used clinically to make treatment decisions, including whether to perform surgery or what treatment standards should be used along with surgery (e.g., either pre-surgery or post-surgery). As an illustrative example, a biosignature of circulating biomarkers that indicates an aggressive form of cancer may call for a more aggressive surgical procedure and/or more aggressive therapeutic regimen to treat the patient.

A biosignature can be used in any methods disclosed herein, e.g., to assess whether a subject is afflicted with disease, is at risk for developing disease or to assess the stage or progression of the disease. For example, a biosignature can be used to assess whether a subject has prostate cancer, colon cancer, or other cancer as described herein.

Furthermore, a biosignature can be used to determine a stage of a disease or condition, such as colon cancer. The biosignature/biomarker profile comprising a microvesicle can include assessment of payload within the microvesicle. For example, one or more aptamer can be used to capture a microvesicle population, thereby providing readout of microvesicle antigens, and then the payload content within the captured microvesicles can be assessed, thereby providing further biomarker readout of the payload content.

A biosignature for characterizing a phenotype may comprise any number of useful criteria. As described further below, the term "phenotype" as used herein can mean any trait or characteristic that is attributed to a biosignature / biomarker profile. A phenotype can be detected or identified in part or in whole using the methods of the invention. In some embodiments, at least one criterion is used for each biomarker. In some embodiments, at least 1, 2, 3, 4, 5, 6, 7, 8, 9, 10, 15, 20, 30, 40, 50, 60, 70, 80, 90 or at least 100 criteria are used. For example, for the characterizing of a cancer, a number of different criteria can be used when the subject is diagnosed with a cancer: 1) if the amount of microRNA in a sample from a subject is higher than a reference value; 2) if the amount of a microRNA within cell type specific vesicles (i.e. vesicles derived from a specific tissue or organ) is higher than a reference value; or 3) if the amount of microRNA within vesicles with one or more cancer specific biomarkers is higher than a reference value. Similar rules can apply if the amount of microRNA is less than or the same as the reference. The method can further include a quality control measure, such that the results are provided for the subject if the samples meet the quality control measure. In some embodiments, if the criteria are met but the quality control is questionable, the subject is reassessed.

*Theranostics*

A biosignature can be used in therapy related diagnostics to provide tests useful to diagnose a disease or choose the correct treatment regimen, such as provide a theranosis. Theranostics includes diagnostic testing that provides the ability to affect therapy or treatment of a diseased state. Theranostics testing provides a theranosis in a similar manner that diagnostics or prognostic testing provides a diagnosis or prognosis, respectively. As used herein, theranostics encompasses any desired form of therapy related testing, including predictive medicine, personalized medicine, integrated medicine, pharmacodiagnostics and Dx/Rx partnering. Therapy related tests can be used to predict and assess drug response in individual subjects, i.e., to provide personalized medicine. Predicting a drug response can be determining whether a subject is a likely responder or a likely non-responder to a candidate therapeutic agent, e.g., before the subject has been exposed or otherwise treated with the treatment. Assessing a drug response can be monitoring a response to a drug, e.g., monitoring the subject's improvement or lack thereof over a time course after initiating the treatment. Therapy related tests are useful to select a subject for treatment who is particularly likely to benefit from the treatment or to provide an early and objective indication of treatment efficacy in an individual subject. Thus, a biosignature as disclosed herein may indicate that treatment should be altered to select a more promising treatment, thereby avoiding the great expense of delaying beneficial treatment and avoiding the financial and morbidity costs of administering an ineffective drug(s).

The methods of the invention can be used to identify or detect a biosignature that associated with selected diseases and disorders, which include, but are not limited to cardiovascular disease, cancer, infectious diseases, sepsis, neurological diseases, central nervous system related diseases, endovascular related diseases, and autoimmune related diseases. Therapy related diagnostics also aid in the prediction of drug toxicity, drug resistance or drug response. Therapy related tests may be developed in any suitable diagnostic testing format, which include, but are not limited to, e.g., immunohistochemical tests, clinical chemistry, immunoassay, cell-based technologies, nucleic acid tests or body imaging methods. Therapy related tests can further include but are not limited to, testing that aids in the determination of therapy, testing that monitors for therapeutic toxicity, or response to therapy testing. Thus, a biosignature can be used to predict or monitor a subject's response to a treatment. A biosignature can be determined at different time points for a subject after initiating, removing, or altering a particular treatment.

In some embodiments, the methods of the invention provide for a determination or prediction as to whether a subject is responding to a treatment is made based on a change in the amount of one or more components of a biosignature (i.e., the microRNA, vesicles and/or biomarkers of interest), an amount of one or more components of a particular biosignature, or the biosignature detected for the components. In another embodiment, a subject's condition is monitored by determining a biosignature at different time points. The progression, regression, or recurrence of a condition is determined. Response to therapy can also be measured over a time course. Thus, the invention provides a method of monitoring a status of a disease or other medical condition in a subject, comprising isolating or detecting a biosignature from a biological sample from the subject, detecting the overall amount of the components of a particular biosignature, or detecting the biosignature of one or more components (such as the presence, absence, or expression level of a biomarker). The biosignatures are used to monitor the status of the disease or condition.

One or more novel biosignatures of a vesicle can also be identified. For example, one or more vesicles can be isolated from a subject that responds to a drug treatment or treatment regimen and compared to a reference, such as another subject that does not respond to the drug treatment or treatment regimen. Differences between the biosignatures can be determined and used to identify other subjects as responders or non-responders to a particular drug or treatment regimen.

In A biosignature may be used to determine whether a particular disease or condition is resistant to a drug, in which case a physician need not waste valuable time with such drug treatment. To obtain early validation of a drug choice or treatment regimen, a biosignature is determined for a sample obtained from a subject. The biosignature is used to assess whether the particular subject's disease has the biomarker associated with drug resistance. Such a determination enables doctors to devote critical time as well as the patient's financial resources to effective treatments.

Biosignatures can be used in the theranosis of a cancer, such as identifying whether a subject suffering from cancer is a likely responder or non-responder to a particular cancer treatment. The subject methods can be used to theranose cancers including those listed herein, e.g., in the "Phenotypes" section below. These include without limitation lung cancer, non-small cell lung cancer small cell lung cancer (including small cell carcinoma (oat cell cancer), mixed small cell/large cell carcinoma, and combined small cell carcinoma), colon cancer, breast cancer, prostate cancer, liver cancer, pancreatic cancer, brain cancer, kidney cancer, ovarian cancer, stomach cancer, melanoma, bone cancer, gastric cancer, breast cancer, glioma, glioblastoma, hepatocellular carcinoma, papillary renal carcinoma, head and neck squamous cell carcinoma, leukemia, lymphoma, myeloma, or other solid tumors.

A biosignature of circulating biomarkers, including markers associated with a component present in a biological sample (e.g., cell, cell-fragment, cell-derived extracellular vesicle), in a sample from a subject suffering from a cancer can be used select a candidate treatment for the subject. The biosignature can be determined according to the methods of the invention presented herein. In some embodiments, the candidate treatment comprises a standard of care for the cancer. The treatment can be a cancer treatment such as radiation, surgery, chemotherapy or a combination thereof. The cancer treatment can be a therapeutic such as anti-cancer agents and chemotherapeutic regimens. Further drug associations and rules that are used in embodiments of the invention are found in U.S. Patent Application 12/658,770, filed February 12, 2010; International PCT Patent Publication WO/2010/093465, filed February 11, 2010; International PCT Patent Publication WO/2011/056688, filed October 27, 2010; and U.S. Provisional Patent Application 61/427,788, filed December 28, 2010. See, e.g., "Table 4: Rules Summary for Treatment Selection" of WO/2011/056688.

**Biomarker Detection**

The compositions and methods can be used to assess any useful biomarkers in a biological sample for charactering a phenotype associated with the sample. Such biomarkers include all sorts of biological entities such as proteins, nucleic acids, lipids, carbohydrates, complexes of any thereof, and microvesicles. Various molecules associated with a microvesicle surface or enclosed within the microvesicle (referred to herein as "payload") can serve as biomarkers. The microvesicles themselves can also be used as biomarkers.

The aptamers can be used to assess levels or presence of a microvesicle population. See, e.g., **FIGs. 16B-16C.** The aptamers can also be used to assess levels or presence of their specific target molecule. See, e.g., **FIG. 16A.** In addition, aptamers are used to capture or isolated a component present in a biological sample that has the aptamer's target molecule present. For example, if a given microvesicle surface antigen is present on a cell, cell fragment or cell-derived extracellular vesicle. A binding agent to the biomarker, including without limitation an aptamer, may be used to capture or isolate the cell, cell fragment or cell-derived extracellular vesicles. See, e.g., **FIGs. 2A-2B, 16A.** Such captured or isolated entities may be further characterized to assess additional surface antigens or internal "payload" molecules present (i.e., nucleic acid molecules, lipids, sugars, polypeptides or functional fragments thereof, or anything else present in the cellular milieu that may be used as a biomarker), where one or more biomarkers provide a biosignature to assess a desired phenotype, such a s disease or condition. See, e.g., **FIG. 2F.** Therefore, aptamers are used not only to assess one or more microvesicle surface antigen of interest but are also used to separate a component present in a biological sample, where the components themselves can be further assessed to identify a candidate biosignature.

The methods of the disclosure can comprise multiplex analysis of at least 2, 3, 4, 5, 6, 7, 8, 9, 10, 11, 12, 13, 14, 15, 16, 17, 18, 19, 20, 25, 50, 75 or 100 different biomarkers. For example, an assay of a heterogeneous population of vesicles can be performed with a plurality of particles that are differentially labeled. There can be at least 2, 3, 4, 5, 6, 7, 8, 9, 10, 11, 12, 13, 14, 15, 16, 17, 18, 19, 20, 25, 50, 75 or 100 differentially labeled particles. The particles may be externally labeled, such as with a tag, or they may be intrinsically labeled. Each differentially labeled particle can be coupled to a capture agent, such as a binding agent, for a vesicle, resulting in capture of a vesicle. The multiple capture agents can be selected to characterize a phenotype of interest, including capture agents against general vesicle biomarkers, cell-of-origin specific biomarkers, and disease biomarkers. One or more biomarkers of the captured vesicle can then be detected by a plurality of binding agents. The binding agent can be directly labeled to facilitate detection. Alternatively, the binding agent is labeled by a secondary agent. For example, the binding agent may be an antibody for a biomarker on the vesicle, wherein the binding agent is linked to biotin. A secondary agent comprises streptavidin linked to a reporter and can be added to detect the biomarker. The captured vesicle may be assayed for at least 2, 3, 4, 5, 6, 7, 8, 9, 10, 11, 12, 13, 14, 15, 16, 17, 18, 19, 20, 25, 50, 75 or 100 different biomarkers. For example, multiple detectors, i.e., detection of multiple biomarkers of a captured vesicle or population of vesicles, can increase the signal obtained, permitted increased sensitivity, specificity, or both, and the use of smaller amounts of samples. Detection can be with more than one biomarker, including without limitation more than one general vesicle marker such as in **Table 3.**

An immunoassay based method (e.g., sandwich assay) can be used to detect a biomarker of a vesicle. An example includes ELISA. A binding agent can be bound to a well. For example, a binding agent such as an aptamer or antibody to an antigen of a vesicle can be attached to a well. A biomarker on the captured vesicle can be detected based on the methods described herein. **FIG. 2A** shows an illustrative schematic for a sandwich-type of immunoassay. The capture agent can be against a vesicle antigen of interest, e.g., a general vesicle biomarker, a cell-of-origin marker, or a disease marker. In the figure, the captured vesicles are detected using fluorescently labeled binding agent (detection agent) against vesicle antigens of interest. Multiple capture binding agents can be used, e.g., in distinguishable addresses on an array or different wells of an immunoassay plate. The detection binding agents can be against the same antigen as the capture binding agent, or can be directed against other markers. The capture binding agent can be any useful binding agent, e.g., tethered aptamers, antibodies or lectins, and/or the detector antibodies can be similarly substituted, e.g., with detectable (e.g., labeled) aptamers, antibodies, lectins or other binding proteins or entities. One or more capture agents to a general vesicle biomarker, a cell-of-origin marker, and/or a disease marker may be used along with detection agents against general vesicle biomarker, such as tetraspanin molecules including without limitation one or more of CD9, CD63 and CD81, or other markers in **Table 3** herein. Examples of microvesicle surface antigens are disclosed herein, e.g. in **Tables 3** or **4,** or are known in the art, and examples useful in methods and compositions are disclosed of International Patent Publication No. WO/2011/127219, entitled "Circulating Biomarkers for Disease" and filed April 6, 2011.

**FIG. 2D** presents an illustrative schematic for analyzing vesicles according to the methods. Capture agents are used to capture vesicles, detectors are used to detect the captured vesicles, and the level or presence of the captured and detected microvesicles is used to characterize a phenotype. Capture agents, detectors and characterizing phenotypes can be any of those described herein. For example, capture agents include antibodies or aptamers tethered to a substrate that recognize a vesicle antigen of interest, detectors include labeled antibodies or aptamers to a vesicle antigen of interest, and characterizing a phenotype includes a diagnosis, prognosis, or theranosis of a disease. In the scheme shown in **FIG. 2D i),** a population of vesicles is captured with one or more capture agents against general vesicle biomarkers **(200).** The captured vesicles are then labeled with detectors against cell-of-origin biomarkers **(201)** and/or disease specific biomarkers **(202).** If only cell-of-origin detectors are used **(201),** the biosignature used to characterize the phenotype **(203)** can include the general vesicle markers **(200)** and the cell-of-origin biomarkers **(201).** If only disease detectors are used **(202),** the biosignature used to characterize the phenotype **(203)** can include the general vesicle markers **(200)** and the disease biomarkers **(202).** Alternately, detectors are used to detect both cell-of-origin biomarkers **(201)** and disease specific biomarkers **(202).** In this case, the biosignature used to characterize the phenotype **(203)** can include the general vesicle markers **(200),** the cell-of-origin biomarkers **(201)** and the disease biomarkers **(202).** The biomarkers combinations are selected to characterize the phenotype of interest and can be selected from the biomarkers and phenotypes described herein, e.g., in **Tables 3** or **4.**

In the scheme shown in **FIG. 2D ii),** a population of vesicles is captured with one or more capture agents against cell-of-origin biomarkers **(210)** and/or disease biomarkers **(211).** The captured vesicles are then detected using detectors against general vesicle biomarkers **(212).** If only cell-of-origin capture agents are used **(210),** the biosignature used to characterize the phenotype **(213)** can include the cell-of-origin biomarkers **(210)** and the general vesicle markers **(212).** If only disease biomarker capture agents are used **(211),** the biosignature used to characterize the phenotype **(213)** can include the disease biomarkers **(211)** and the general vesicle biomarkers **(212).** Alternately, capture agents to one or more cell-of-origin biomarkers **(210)** and one or more disease specific biomarkers **(211)** are used to capture vesicles. In this case, the biosignature used to characterize the phenotype **(213)** can include the cell-of-origin biomarkers **(210),** the disease biomarkers **(211),** and the general vesicle markers **(213).** The biomarkers combinations are selected to characterize the phenotype of interest and can be selected from the biomarkers and phenotypes described herein.

The methods comprise capture and detection of microvesicles of interest using any combination of useful biomarkers. For example, a microvesicle population can be captured using one or more binding agent to any desired combination of cell of origin, disease specific, or general vesicle markers. The captured microvesicles can then be detected using one or more binding agent to any desired combination of cell of origin, disease specific, or general vesicle markers. **FIG. 2E** represents a flow diagram of such configurations. Any one or more of a cell-of-origin biomarker **(240),** disease biomarkers **(241),** and general vesicle biomarker **(242)** is used to capture a microvesicle population. Thereafter, any one or more of a cell-of-origin biomarker **(243),** disease biomarkers **(244),** and general vesicle biomarker **(245)** is used to detect the captured microvesicle population. The biosignature of captured and detected microvesicles is then used to characterize a phenotype. The biomarkers combinations are selected to characterize the phenotype of interest and can be selected from the biomarkers and phenotypes described herein.

A microvesicle payload molecule can be assessed as a member of a biosignature panel. A payload molecule comprises any of the biological entities contained within a cell, cell fragment or vesicle membrane. These entities include without limitation nucleic acids, e.g., mRNA, microRNA, or DNA fragments; protein, e.g., soluble and membrane associated proteins; carbohydrates; lipids; metabolites; and various small molecules, e.g., hormones. The payload can be part of the cellular milieu that is encapsulated as a vesicle is formed in the cellular environment. In some aspects, the payload is analyzed in addition to detecting vesicle surface antigens. Specific populations of vesicles can be captured as described above then the payload in the captured vesicles can be used to characterize a phenotype. For example, vesicles captured on a substrate can be further isolated to assess the payload therein. Alternately, the vesicles in a sample are detected and sorted without capture. The vesicles so detected can be further isolated to assess the payload therein. Vesicle populations may be sorted by flow cytometry and the payload in the sorted vesicles is analyzed. In the scheme shown in **FIG. 2F iv)**, a population of vesicles is captured and/or detected **(220)** using one or more of cell-of-origin biomarkers **(220)**, disease biomarkers **(221)**, and/or general vesicle markers **(222)**. The payload of the isolated vesicles is assessed **(223)**. A biosignature detected within the payload can be used to characterize a phenotype **(224)**. In a non-limiting example, a vesicle population can be analyzed in a plasma sample from a patient using antibodies against one or more vesicle antigens of interest. The antibodies can be capture antibodies which are tethered to a substrate to isolate a desired vesicle population. Alternately, the antibodies can be directly labeled and the labeled vesicles isolated by sorting with flow cytometry. The presence or level of microRNA or mRNA extracted from the isolated vesicle population can be used to detect a biosignature. The biosignature is then used to diagnose, prognose or theranose the patient.

Vesicle or cellular payload may be analyzed in a population (e.g., cells or vesicles) without first capturing or detected subpopulations of vesicles. For example, a cellular or extracellular vesicle population can be generally isolated from a sample using centrifugation, filtration, chromatography, or other techniques as described herein and known in the art. The payload of such sample components can be analyzed thereafter to detect a biosignature and characterize a phenotype. In the scheme shown in **FIG. 2F v)**, a population of vesicles is isolated **(230)** and the payload of the isolated vesicles is assessed **(231)**. A biosignature detected within the payload can be used to characterize a phenotype **(232)**. In a non-limiting example, a vesicle population is isolated from a plasma sample from a patient using size exclusion and membrane filtration. The presence or level of microRNA or mRNA extracted from the vesicle population is used to detect a biosignature. The biosignature is then used to diagnose, prognose or theranose the patient.

The biomarkers used to detect a vesicle population can be selected to detect a microvesicle population of interest, e.g., a population of vesicles that provides a diagnosis, prognosis or theranosis of a selected condition or disease, including but not limited to a cancer, a premalignant condition, an inflammatory disease, an immune disease, an autoimmune disease or disorder, a cardiovascular disease or disorder, neurological disease or disorder, infectious disease or pain. See Section "Phenotypes" herein for more detail. The biomarkers may be selected from the group consisting of EpCam (epithelial cell adhesion molecule), CD9 (tetraspanin CD9 molecule), PCSA (prostate cell specific antigen, *see* Rokhlin et al., 5E10: a prostate-specific surface-reactive monoclonal antibody. Cancer Lett. 1998 131:129-36), CD63 (tetraspanin CD63 molecule), CD81 (tetraspanin CD81 molecule), PSMA (FOLH1, folate hydrolase (prostate-specific membrane antigen) 1), B7H3 (CD276 molecule), PSCA (prostate stem cell antigen), ICAM (intercellular adhesion molecule), STEAP (STEAP1, six transmembrane epithelial antigen of the prostate 1), KLK2 (kallikrein-related peptidase 2), SSX2 (synovial sarcoma, X breakpoint 2), SSX4 (synovial sarcoma, X breakpoint 4), PBP (prostatic binding protein), SPDEF (SAM pointed domain containing ets transcription factor), EGFR (epidermal growth factor receptor), and a combination thereof. One or more of these markers can provide a biosignature for a specific condition, such as to detect a cancer, including without limitation a carcinoma, a prostate cancer, a breast cancer, a lung cancer, a colorectal cancer, an ovarian cancer, melanoma, a brain cancer, or other type of cancer as disclosed herein. A binding agent to one or more of these markers may be used to capture a microvesicle population, and an aptamer may be used to assist in detection of the capture vesicles as described herein. An aptamer may be used to capture a microvesicle population, and a binding agent to one or more of these markers may be used to assist in detection of the capture vesicles as described herein. The binding agents can be any useful binding agent as disclosed herein or known in the art, e.g., antibodies or aptamers.

The methods of characterizing a phenotype can employ a combination of techniques to assess a component or population of components present in a biological sample of interest. For example, an aptamer can be used to assess a single cell, or a single extracellular vesicle or a population of cells or population of vesicles. A sample may be split into various aliquots, where each is analyzed separately. For example, protein content of one or more aliquot is determined and microRNA content of one or more other aliquot is determined. The protein content and microRNA content can be combined to characterize a phenotype. A component present in a biological sample of interest may be isolated and the payload therein may be assessed (e.g., capture a population of subpopulation of vesicles using an aptamer and further assess nucleic acid or proteins present in the isolated vesicles).

A population of vesicles with a given surface marker can be isolated by using a binding agent to a microvesicle surface marker. See, e.g., **FIGs. 2A, 2B, 16A.** The binding agent can be an aptamer that was identified to target the microvesicle surface marker using the methods. The isolated vesicles is assessed for additional biomarkers such as surface content or payload, which can be contemporaneous to detection of the aptamer-specific target or the assessment of additional biomarkers can be before or subsequent to aptamer-specific target detection.

A biosignature can be detected qualitatively or quantitatively by detecting a presence, level or concentration of a circulating biomarker, e.g., a microRNA, protein, vesicle or other biomarker, as disclosed herein. These biosignature components can be detected using a number of techniques known to those of skill in the art. For example, a biomarker can be detected by microarray analysis, polymerase chain reaction (PCR) (including PCR-based methods such as real time polymerase chain reaction (RT-PCR), quantitative real time polymerase chain reaction (Q-PCR/qPCR) and the like), hybridization with allele-specific probes, enzymatic mutation detection, ligation chain reaction (LCR), oligonucleotide ligation assay (OLA), flow-cytometric heteroduplex analysis, chemical cleavage of mismatches, mass spectrometry, nucleic acid sequencing, single strand conformation polymorphism (SSCP), denaturing gradient gel electrophoresis (DGGE), temperature gradient gel electrophoresis (TGGE), restriction fragment polymorphisms, serial analysis of gene expression (SAGE), or combinations thereof. A biomarker, such as a nucleic acid, can be amplified prior to detection. A biomarker can also be detected by immunoassay, immunoblot, immunoprecipitation, enzyme-linked immunosorbent assay (ELISA; EIA), radioimmunoassay (RIA), flow cytometry, or electron microscopy (EM).

Biosignatures can be detected using aptamers that function as either as capture agents and detection agents, as described herein. A capture agent can comprise an antibody, aptamer or other entity which recognizes a biomarker and can be used for capturing the biomarker. Biomarkers that can be captured include circulating biomarkers, e.g., a protein, nucleic acid, lipid or biological complex in solution in a bodily fluid. Similarly, the capture agent can be used for capturing a vesicle. A detection agent can comprise an antibody or other entity which recognizes a biomarker and can be used for detecting the biomarker vesicle, or which recognizes a vesicle and is useful for detecting a vesicle. The detection agent may be labeled and the label may be detected, thereby detecting the biomarker or vesicle. The detection agent can be a binding agent, e.g., an antibody or aptamer. The detection agent may comprise a small molecule such as a membrane protein labeling agent. See, e.g., the membrane protein labeling agents disclosed in Alroy et al., US. Patent Publication US 2005/0158708. Vesicles may be isolated or captured as described herein, and one or more membrane protein labeling agent may be used to detect the vesicles. In many cases, the antigen or other vesicle-moiety that is recognized by the capture and detection agents are interchangeable.

For example, a vesicle having a cell-of-origin specific antigen on its surface and a cancer-specific antigen on its surface, may be captured using a binding agent that is specific to a cells-specific antigen, e.g., by tethering the capture antibody or aptamer to a substrate, and then the vesicle may be detected using a binding agent to a disease-specific antigen, e.g., by labeling the binding agent used for detection with a fluorescent dye and detecting the fluorescent radiation emitted by the dye.

It will be apparent to one of skill in the art that where the target molecule for a binding agent (such as an aptamer) is informative as to assessing a condition or disease, the same binding agent can be used to both capture a component comprising the target molecule (e.g., microvesicle surface antigen of interest) and also be modified to comprise a detectable label so as to detect the target molecule, e.g., binding agenti-antigen-binding agent$_2$*, wherein the * signifies a detectable label; binding agent$_1$ and binding agent$_2$ may be the same binding agent or a different binding agent (e.g., same aptamer or different aptamer). In addition, binding agent$_1$ and binding agent$_2$ can be selected from wholly different categories of binding agents (e.g., antibody, aptamer, synthetic antibody, peptide-nucleic acid molecule, or any molecule that is configured to specifically bind to or associate with its target molecule). Such binding molecules can be selected solely based on their binding specificity for a target molecule.

Techniques of detecting biomarkers or capturing sample components using an aptamer include the use of a planar substrate such as an array (e.g., biochip or microarray), with molecules immobilized to the substrate as capture agents that facilitate the detection of a particular biosignature. The array can be provided as part of a kit for assaying one or more biomarkers. Additional examples of binding agents described above and useful in the compositions and methods are disclosed in International Patent Publication No. WO/2011/127219, entitled "Circulating Biomarkers for Disease" and filed April 6, 2011. Aptamers can be included in an array for detection and diagnosis of diseases including presymptomatic diseases. In some embodiments, an array comprises a custom array comprising biomolecules selected to specifically identify biomarkers of interest. Customized arrays can be modified to detect biomarkers that increase statistical performance, e.g., additional biomolecules that identifies a biosignature which lead to improved cross-validated error rates in multivariate prediction models (e.g., logistic regression, discriminant analysis, or regression tree models). In some embodiments, customized array(s) are constructed to study the biology of a disease, condition or syndrome and profile biosignatures in defined physiological states. Markers for inclusion on the customized array be chosen based upon statistical criteria, e.g., having a desired level of statistical significance in differentiating between phenotypes or physiological states. In some embodiments, standard significance of p-value = 0.05 is chosen to exclude or include biomolecules on the microarray. The p-values can be corrected for multiple comparisons. As an illustrative example, nucleic acids extracted from samples from a subject with or without a disease can be hybridized to a high density microarray that binds to thousands of gene sequences. Nucleic acids whose levels are significantly different between the samples with or without the disease can be selected as biomarkers to distinguish samples as having the disease or not. A customized array can be constructed to detect the selected biomarkers. In some embodiments, customized arrays comprise low density microarrays, which refer to arrays with lower number of addressable binding agents, e.g., tens or hundreds instead of thousands. Low density arrays can be formed on a substrate. In some embodiments, customizable low density arrays use PCR amplification in plate wells, e.g., TaqMan® Gene Expression Assays (Applied Biosystems by Life Technologies Corporation, Carlsbad, CA).

An aptamer or other useful binding agent may be linked directly or indirectly to a solid surface or substrate. See, e.g., **FIGs. 2A-2B, 9, 16A.** A solid surface or substrate can be any physically separable solid to which a binding agent can be directly or indirectly attached including, but not limited to, surfaces provided by microarrays and wells, particles such as beads, columns, optical fibers, wipes, glass and modified or functionalized glass, quartz, mica, diazotized membranes (paper or nylon), polyformaldehyde, cellulose, cellulose acetate, paper, ceramics, metals, metalloids, semiconductive materials, quantum dots, coated beads or particles, other chromatographic materials, magnetic particles; plastics (including acrylics, polystyrene, copolymers of styrene or other materials, polypropylene, polyethylene, polybutylene, polyurethanes, Teflon material, etc.), polysaccharides, nylon or nitrocellulose, resins, silica or silica-based materials including silicon and modified silicon, carbon, metals, inorganic glasses, plastics, ceramics, conducting polymers (including polymers such as polypyrole and polyindole); micro or nanostructured surfaces such as nucleic acid tiling arrays, nanotube, nanowire, or nanoparticulate decorated surfaces; or porous surfaces or gels such as methacrylates, acrylamides, sugar polymers, cellulose, silicates, or other fibrous or stranded polymers. In addition, as is known the art, the substrate may be coated using passive or chemically-derivatized coatings with any number of materials, including polymers, such as dextrans, acrylamides, gelatins or agarose. Such coatings can facilitate the use of the array with a biological sample.

As provided in the examples, below, an aptamer or other useful binding agent can be conjugated to a detectable entity or label. Appropriate labels include without limitation a magnetic label, a fluorescent moiety, an enzyme, a chemiluminescent probe, a metal particle, a non-metal colloidal particle, a polymeric dye particle, a pigment molecule, a pigment particle, an electrochemically active species, semiconductor nanocrystal or other nanoparticles including quantum dots or gold particles, fluorophores, quantum dots, or radioactive labels. Protein labels include green fluorescent protein (GFP) and variants thereof (e.g., cyan fluorescent protein and yellow fluorescent protein); and luminescent proteins such as luciferase, as described below. Radioactive labels include without limitation radioisotopes (radionu-clides), such as $^3$H, $^{11}$C, $^{14}$C, $^{18}$F, $^{32}$P, $^{35}$S, $^{64}$Cu, $^{68}$Ga, $^{86}$Y, $^{99}$Tc, $^{111}$In, $^{123}$I, $^{124}$I, $^{125}$I, $^{131}$I, $^{133}$Xe, $^{177}$Lu, $^{211}$At, or $^{213}$Bi. Fluorescent labels include without limitation a rare earth chelate (e.g., europium chelate), rhodamine; fluorescein types including without limitation FITC, 5-carboxyfluorescein, 6-carboxy fluorescein; a rhodamine type including without limitation TAMRA; dansyl; Lissamine; cyanines; phycoerythrins; Texas Red; Cy3, Cy5, dapoxyl, NBD, Cascade Yellow, dansyl, PyMPO, pyrene, 7-diethylaminocoumarin-3-carboxylic acid and other coumarin derivatives, Marina Blue™, Pacific Blue™, Cascade Blue™, 2-anthracenesulfonyl, PyMPO, 3,4,9,10-perylene-tetracarboxylic acid, 2,7-difluorofluorescein (Oregon Green™ 488-X), 5-carboxyfluorescein, Texas Red™-X, Alexa Fluor 430, 5-carboxytetramethylrhodamine (5-TAMRA), 6-carboxytetramethylrhodamine (6-TAMRA), BODIPY FL, bimane, and Alexa Fluor 350, 405, 488, 500, 514, 532, 546, 555, 568, 594, 610, 633, 647, 660, 680, 700, and 750, and derivatives thereof, among many others. See, e.g., "The Handbook--A Guide to Fluorescent Probes and Labeling Technologies," Tenth Edition, available on the internet at probes (dot) invitrogen (dot) com/handbook. The fluorescent label can be one or more of FAM, dRHO, 5-FAM, 6FAM, dR6G, JOE, HEX, VIC, TET, dTAMRA, TAMRA, NED, dROX, PET, BHQ, Gold540 and LIZ.

Using conventional techniques, an aptamer can be directly or indirectly labeled, e.g., the label is attached to the aptamer through biotin-streptavidin (e.g., synthesize a biotinylated aptamer, which is then capable of binding a streptavidin molecule that is itself conjugated to a detectable label; non-limiting example is streptavidin, phycoerythrin conjugated (SAPE)). Methods for chemical coupling using multiple step procedures include biotinylation, coupling of trinitrophenol (TNP) or digoxigenin using for example succinimide esters of these compounds. Biotinylation can be accomplished by, for example, the use of D-biotinyl-N-hydroxysuccinimide. Succinimide groups react effectively with amino groups at pH values above 7, and preferentially between about pH 8.0 and about pH 8.5. Alternatively, an aptamer is not labeled, but is later contacted with a second antibody that is labeled after the first antibody is bound to an antigen of interest.

Various enzyme-substrate labels may also be used in conjunction with a method of the invention. Such enzyme-substrate labels are available commercially (e.g., U.S. Pat. No. 4,275,149). The enzyme generally catalyzes a chemical alteration of a chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Examples of enzymatic labels include luciferases (e.g., firefly luciferase and bacterial luciferase; U.S. Pat. No. 4,737,456), luciferin, 2,3-dihydrophthalazinediones, malate dehydrogenase, urease, peroxidase such as horseradish peroxidase (HRP), alkaline phosphatase (AP), β-galactosidase, glucoamylase, lysozyme, saccharide oxidases (e.g. glucose oxidase, galactose oxidase, and glucose-6-phosphate dehydrogenase), heterocyclic oxidases (such as uricase and xanthine oxidase), lactoperoxidase, microperoxidase, and the like. Examples of enzyme-substrate combinations include, but are not limited to, horseradish peroxidase (HRP) with hydrogen peroxidase as a substrate, wherein the hydrogen peroxidase oxidizes a dye precursor (e.g., orthophenylene diamine (OPD) or 3,3',5,5'-tetramethylbenzidine hydrochloride (TMB)); alkaline phosphatase (AP) with para-nitrophenyl phosphate as chromogenic substrate; and β-D-galactosidase (β-D-Gal) with a chromogenic substrate (e.g., p-nitrophenyl-β-D-galactosidase) or fluorogenic substrate 4-methylumbelliferyl-β-D-galactosidase.

Aptamer(s) can be linked to a substrate such as a planar substrate. A planar array generally contains addressable locations (e.g., pads, addresses, or micro-locations) of biomolecules in an array format. The size of the array will depend on the composition and end use of the array. Arrays can be made containing from 2 different molecules to many thousands. Generally, the array comprises from two to as many as 100,000 or more molecules, depending on the end use of the array and the method of manufacture. A microarray for use with the invention comprises at least one biomolecule that identifies or captures a biomarker present in a biosignature of interest, e.g., a microRNA or other biomolecule or vesicle that makes up the biosignature. In some arrays, multiple substrates are used, either of different or identical compositions. Accordingly, planar arrays may comprise a plurality of smaller substrates.

The present invention can make use of many types of arrays for detecting a biomarker, e.g., a biomarker associated with a biosignature of interest. Useful arrays or microarrays include without limitation DNA microarrays, such as cDNA microarrays, oligonucleotide microarrays and SNP microarrays, microRNA arrays, protein microarrays, antibody microarrays, tissue microarrays, cellular microarrays (also called transfection microarrays), chemical compound microarrays, and carbohydrate arrays (glycoarrays). These arrays are described in more detail above. In some embodiments, microarrays comprise biochips that provide high-density immobilized arrays of recognition molecules (e.g., aptamers or antibodies), where biomarker binding is monitored indirectly (e.g., via fluorescence).

An array or microarray that can be used to detect one or more biomarkers of a biosignature and comprising one or more aptamers can be made according to the methods described in U.S. Pat. Nos. 6,329,209; 6,365,418; 6,406,921; 6,475,808; and 6,475,809, and U.S. Patent Application Ser. No. 10/884,269. Custom arrays to detect specific selections of sets of biomarkers described herein can be made using the methods described in these patents. Commercially available microarrays can also be used to carry out the methods of the invention, including without limitation those from Affymetrix (Santa Clara, CA), Illumina (San Diego, CA), Agilent (Santa Clara, CA), Exiqon (Denmark), or Invitrogen (Carlsbad, CA). Custom and/or commercial arrays include arrays for detection proteins, nucleic acids, and other biological molecules and entities (e.g., cells, vesicles, virii) as described herein.

Multiple capture molecules may be disposed on an array, e.g., proteins, peptides or additional nucleic acid molecules. The proteins may be immobilized using methods and materials that minimize the denaturing of the proteins, that minimize alterations in the activity of the proteins, or that minimize interactions between the protein and the surface on which they are immobilized. The capture molecules can comprise one or more aptamer. An array may be constructed for the hybridization of a pool of aptamers. The array can then be used to identify pool members that bind a sample, thereby facilitating characterization of a phenotype. See **FIGs. 16B-16C** and related disclosure for further details.

Array surfaces useful may be of any desired shape, form, or size. Non-limiting examples of surfaces include chips, continuous surfaces, curved surfaces, flexible surfaces, films, plates, sheets, or tubes. Surfaces can have areas ranging from approximately a square micron to approximately 500 cm$^2$. The area, length, and width of surfaces may be varied according to the requirements of the assay to be performed. Considerations may include, for example, ease of handling, limitations of the material(s) of which the surface is formed, requirements of detection systems, requirements of deposition systems (e.g., arrayers), or the like.

It may be desirable to employ a physical means for separating groups or arrays of binding islands or immobilized biomolecules: such physical separation facilitates exposure of different groups or arrays to different solutions of interest. Therefore, arrays may be situated within microwell plates having any number of wells. In such cases, the bottoms of the wells may serve as surfaces for the formation of arrays, or arrays may be formed on other surfaces and then placed into wells. In certain cases, such as where a surface without wells is used, binding islands may be formed or molecules may be immobilized on a surface and a gasket having holes spatially arranged so that they correspond to the islands or biomolecules may be placed on the surface. Such a gasket is preferably liquid tight. A gasket may be placed on a surface at any time during the process of making the array and may be removed if separation of groups or arrays is no longer desired.

The immobilized molecules can bind to one or more biomarkers or vesicles present in a biological sample contacting the immobilized molecules. The immobilized molecules may modify or may be modified by molecules present in the one or more vesicles contacting the immobilized molecules. Contacting the sample typically comprises overlaying the sample upon the array.

Modifications or binding of molecules in solution or immobilized on an array can be detected using detection techniques known in the art. Examples of such techniques include immunological techniques such as competitive binding assays and sandwich assays; fluorescence detection using instruments such as confocal scanners, confocal microscopes, or CCD-based systems and techniques such as fluorescence, fluorescence polarization (FP), fluorescence resonant energy transfer (FRET), total internal reflection fluorescence (TIRF), fluorescence correlation spectroscopy (FCS); colorimetric/spectrometric techniques; surface plasmon resonance, by which changes in mass of materials adsorbed at surfaces are measured; techniques using radioisotopes, including conventional radioisotope binding and scintillation proximity assays (SPA); mass spectroscopy, such as matrix-assisted laser desorption/ionization mass spectroscopy (MALDI) and MALDI-time of flight (TOF) mass spectroscopy; ellipsometry, which is an optical method of measuring thickness of protein films; quartz crystal microbalance (QCM), a very sensitive method for measuring mass of materials adsorbing to surfaces; scanning probe microscopies, such as atomic force microscopy (AFM), scanning force microscopy (SFM) or scanning electron microscopy (SEM); and techniques such as electrochemical, impedance, acoustic, microwave, and IR/Raman detection. See, e.g., Mere L, et al., "Miniaturized FRET assays and microfluidics: key components for ultra-high-throughput screening, " Drug Discovery Today 4(8):363-369 (1999), and references cited therein; Lakowicz J R, Principles of Fluorescence Spectroscopy, 2nd Edition, Plenum Press (1999), or Jain KK: Integrative Omics, Pharmacoproteomics, and Human Body Fluids. In: Thongboonkerd V, ed., ed. Proteomics of Human Body Fluids: Principles, Methods and Applications. Volume 1: Totowa, N.J.: Humana Press, 2007.

Microarray technology can be combined with mass spectroscopy (MS) analysis and other tools. Electrospray interface to a mass spectrometer can be integrated with a capillary in a microfluidics device. For example, one commercially available system contains eTag reporters that are fluorescent labels with unique and well-defined electrophoretic mobilities; each label is coupled to biological or chemical probes via cleavable linkages. The distinct mobility address of each eTag reporter allows mixtures of these tags to be rapidly deconvoluted and quantitated by capillary electrophoresis. This system allows concurrent gene expression, protein expression, and protein function analyses from the same sample Jain KK: Integrative Omics, Pharmacoproteomics, and Human Body Fluids. In: Thongboonkerd V, ed., ed. Proteomics of Human Body Fluids: Principles, Methods and Applications. Volume 1: Totowa, N.J.: Humana Press, 2007.

A biochip can include components for a microfluidic or nanofluidic assay. A microfluidic device can be used for isolating or analyzing biomarkers, such as determining a biosignature. Microfluidic systems allow for the miniaturization and compartmentalization of one or more processes for isolating, capturing or detecting a vesicle, detecting a microRNA, detecting a circulating biomarker, detecting a biosignature, and other processes. The microfluidic devices can use one or more detection reagents in at least one aspect of the system, and such a detection reagent can be used to detect one or more biomarkers. The device may detect a biomarker on an isolated or bound vesicle. Various probes, antibodies, proteins, or other binding agents can be used to detect a biomarker within the microfluidic system. The detection agents may be immobilized in different compartments of the microfluidic device or be entered into a hybridization or detection reaction through various channels of the device.

A vesicle in a microfluidic device can be lysed and its contents detected within the microfluidic device, such as proteins or nucleic acids, e.g., DNA or RNA such as miRNA or mRNA. The nucleic acid may be amplified prior to detection, or directly detected, within the microfluidic device. Thus microfluidic system can also be used for multiplexing detection of various biomarkers. Vesicles may be captured within the microfluidic device, the captured vesicles may be lysed, and a biosignature of microRNA from the vesicle payload may be determined. The biosignature can further comprise the capture agent used to capture the vesicle.

Novel nanofabrication techniques are opening up the possibilities for biosensing applications that rely on fabrication of high-density, precision arrays, e.g., nucleotide-based chips and protein arrays otherwise known as heterogeneous nanoarrays. Nanofluidics allows a further reduction in the quantity of fluid analyte in a microchip to nanoliter levels, and the chips used here are referred to as nanochips. See, e.g., Unger Met al., Biotechniques 1999; 27(5):1008-14, Kartalov EP et al., Biotechniques 2006; 40(1):85-90. Commercially available nanochips currently provide simple one step assays such as total cholesterol, total protein or glucose assays that can be run by combining sample and reagents, mixing and monitoring of the reaction. Gel-free analytical approaches based on liquid chromatography (LC) and nanoLC separations (Cutillas et al. Proteomics, 2005;5:101-112 and Cutillas et al., Mol Cell Proteomics 2005;4:1038-1051) can be used in combination with the nanochips.

An array suitable for identifying a disease, condition, syndrome or physiological status can be included in a kit. A kit can include, an aptamer, including as non-limiting examples, one or more reagents useful for preparing molecules for immobilization onto binding islands or areas of an array, reagents useful for detecting binding of a vesicle to immobilized molecules, and instructions for use.

Further disclosed herein is a rapid detection device that facilitates the detection of a particular biosignature in a biological sample. The device can integrate biological sample preparation with polymerase chain reaction (PCR) on a chip. The device can facilitate the detection of a particular biosignature of a vesicle in a biological sample, and an example is provided as described in Pipper et al., Angewandte Chemie, 47(21), p. 3900-3904 (2008). A biosignature can be incorporated using micro-/nano-electrochemical system (MEMS/NEMS) sensors and oral fluid for diagnostic applications as described in Li et al., Adv Dent Res 18(1): 3-5 (2005).

**Particle arrays**

As an alternative to planar arrays, assays using particles, such as bead based assays are also capable of use with an aptamer. Aptamers are easily conjugated with commercially available beads. See, e.g., Srinivas et al. Anal. Chem. 2011 Oct. 21, Aptamer functionalized Microgel Particles for Protein Detection; See also, review article on aptamers as therapeutic and diagnostic agents, Brody and Gold, Rev. Mol. Biotech. 2000, 74:5-13.

Multiparametric assays or other high throughput detection assays using bead coatings with cognate ligands and reporter molecules with specific activities consistent with high sensitivity automation can be used. In a bead based assay system, a binding agent for a biomarker or vesicle, such as a capture agent (e.g. capture antibody), can be immobilized on an addressable microsphere. Each binding agent for each individual binding assay can be coupled to a distinct type of microsphere (i.e., microbead) and the assay reaction takes place on the surface of the microsphere, such as depicted in **FIG. 2B**. A binding agent for a vesicle can be a capture antibody coupled to a bead. Dyed microspheres with discrete fluorescence intensities are loaded separately with their appropriate binding agent or capture probes. The different bead sets carrying different binding agents can be pooled as desired to generate custom bead arrays. Bead arrays are then incubated with the sample in a single reaction vessel to perform the assay.

Bead-based assays can also be used with one or more aptamers. A bead substrate can provide a platform for attaching one or more binding agents, including aptamer(s). For multiplexing, multiple different bead sets (e.g., Illumina, Luminex) can have different binding agents (specific to different target molecules). For example, a bead can be conjugated to an aptamer used to detect the presence (quantitatively or qualitatively) of an antigen of interest, or it can also be used to isolate a component present in a selected biological sample (e.g., cell, cell-fragment or vesicle comprising the target molecule to which the aptamer is configured to bind or associate). Any molecule of organic origin can be successfully conjugated to a polystyrene bead through use of commercially available kits.

One or more aptamers can be used with any bead based substrate, including but not limited to magnetic capture method, fluorescence activated cell sorting (FACS) or laser cytometry. Magnetic capture methods can include, but are not limited to, the use of magnetically activated cell sorter (MACS) microbeads or magnetic columns. Examples of bead or particle based methods that can be modified to use an aptamer include methods and bead systems described in U.S. Patent Nos. 4,551,435, 4,795,698, 4,925,788, 5,108,933, 5,186,827, 5,200,084 or 5,158,871; 7,399,632; 8,124,015; 8,008,019; 7,955,802; 7,445,844; 7,274,316; 6,773,812; 6,623,526; 6,599,331; 6,057,107; 5,736,330; International Patent Publication No. WO/2012/174282; WO/1993/022684.

**Flow Cytometry**

Isolation or detection of circulating biomarkers, e.g., protein antigens, from a biological sample, or of the biomarker-comprising cells, cell fragments or vesicles may also be achieved using an aptamer in a cytometry process. As a non-limiting example, aptamers can be used in an assay comprising using a particle such as a bead or microsphere The disclosure provides aptamers as binding agents, which may be conjugated to the particle. Flow cytometry can be used for sorting microscopic particles suspended in a stream of fluid. As particles pass through they can be selectively charged and on their exit can be deflected into separate paths of flow. It is therefore possible to separate populations from an original mix, such as a biological sample, with a high degree of accuracy and speed. Flow cytometry allows simultaneous multiparametric analysis of the physical and/or chemical characteristics of single cells flowing through an optical/electronic detection apparatus. A beam of light, usually laser light, of a single frequency (color) is directed onto a hydrodynamically focused stream of fluid. A number of detectors are aimed at the point where the stream passes through the light beam; one in line with the light beam (Forward Scatter or FSC) and several perpendicular to it (Side Scatter or SSC) and one or more fluorescent detectors.

Each suspended particle passing through the beam scatters the light in some way, and fluorescent chemicals in the particle may be excited into emitting light at a lower frequency than the light source. This combination of scattered and fluorescent light is picked up by the detectors, and by analyzing fluctuations in brightness at each detector (one for each fluorescent emission peak), it is possible to deduce various facts about the physical and chemical structure of each individual particle. FSC correlates with the cell size and SSC depends on the inner complexity of the particle, such as shape of the nucleus, the amount and type of cytoplasmic granules or the membrane roughness. Some flow cytometers have eliminated the need for fluorescence and use only light scatter for measurement.

Flow cytometers can analyze several thousand particles every second in "real time" and can actively separate out and isolate particles having specified properties. They offer high-throughput automated quantification, and separation, of the set parameters for a high number of single cells during each analysis session. Flow cytometers can have multiple lasers and fluorescence detectors, allowing multiple labels to be used to more precisely specify a target population by their phenotype. Thus, a flow cytometer, such as a multicolor flow cytometer, can be used to detect one or more vesicles with multiple fluorescent labels or colors. In some embodiments, the flow cytometer can also sort or isolate different vesicle populations, such as by size or by different markers.

The flow cytometer may have one or more lasers, such as 1, 2, 3, 4, 5, 6, 7, 8, 9, 10 or more lasers. In some embodiments, the flow cytometer can detect more than one color or fluorescent label, such as at least 2, 3, 4, 5, 6, 7, 8, 9, 10, 11, 12, 13, 14, 15, 16, 17, 18, 19, or 20 different colors or fluorescent labels. For example, the flow cytometer can have at least 2, 3, 4, 5, 6, 7, 8, 9, 10, 11, 12, 13, 14, 15, 16, 17, 18, 19, or 20 fluorescence detectors.

Examples of commercially available flow cytometers that can be used to detect or analyze one or more vesicles, to sort or separate different populations of vesicles, include, but are not limited to the MoFlo™ XDP Cell Sorter (Beckman Coulter, Brea, CA), MoFlo™ Legacy Cell Sorter (Beckman Coulter, Brea, CA), BD FACSAria™ Cell Sorter (BD Biosciences, San Jose, CA), BD™ LSRII (BD Biosciences, San Jose, CA), and BD FACSCalibur™ (BD Biosciences, San Jose, CA). Use of multicolor or multi-fluor cytometers can be used in multiplex analysis of vesicles, as further described below. In some embodiments, the flow cytometer can sort, and thereby collect or sort more than one population of vesicles based one or more characteristics. For example, two populations of vesicles differ in size, such that the vesicles within each population have a similar size range and can be differentially detected or sorted. In another embodiment, two different populations of vesicles are differentially labeled.

The data resulting from flow-cytometers can be plotted in 1 dimension to produce histograms or seen in 2 dimensions as dot plots or in 3 dimensions with newer software. The regions on these plots can be sequentially separated by a series of subset extractions which are termed gates. Specific gating protocols exist for diagnostic and clinical purposes especially in relation to hematology. The plots are often made on logarithmic scales. Because different fluorescent dye's emission spectra overlap, signals at the detectors have to be compensated electronically as well as computationally. Fluorophores for labeling biomarkers may include those described in Ormerod, Flow Cytometry 2nd ed., Springer-Verlag, New York (1999), and in Nida et al., Gynecologic Oncology 2005;4 889-894. In a multiplexed assay, including but not limited to a flow cytometry assay, one or more different target molecules can be assessed. In some embodiments, at least one of the target molecules is a biomarker, e.g., a microvesicle surface antigen, assessed using an aptamer.

## Microfluidics

One or more aptamer can be disposed on any useful planar or bead substrate. In one aspect one or more aptamer is disposed on a microfluidic device, thereby facilitating assessing, characterizing or isolating a component of a biological sample comprising a polypeptide antigen of interest or a functional fragment thereof. For example, the circulating antigen or a cell, cell fragment or cell-derived vesicles comprising the antigen can be assessed using one or more aptamers (alternatively along with additional binding agents). Microfluidic devices, which may also be referred to as "lab-on-a-chip" systems, biomedical micro-electro-mechanical systems (bioMEMs), or multicomponent integrated systems, can be used for isolating and analyzing a vesicle. Such systems miniaturize and compartmentalize processes that allow for binding of vesicles, detection of biosignatures, and other processes.

A microfluidic device can also be used for isolation of a vesicle through size differential or affinity selection. For example, a microfluidic device can use one more channels for isolating a vesicle from a biological sample based on size or by using one or more binding agents for isolating a vesicle from a biological sample. A biological sample can be introduced into one or more microfluidic channels, which selectively allows the passage of a vesicle. The selection can be based on a property of the vesicle, such as the size, shape, deformability, or biosignature of the vesicle.

In one embodiment, a heterogeneous population of vesicles can be introduced into a microfluidic device, and one or more different homogeneous populations of vesicles can be obtained. For example, different channels can have different size selections or binding agents to select for different vesicle populations. Thus, a microfluidic device can isolate a plurality of vesicles wherein at least a subset of the plurality of vesicles comprises a different biosignature from another subset of the plurality of vesicles. For example, the microfluidic device can isolate at least 2, 3, 4, 5, 6, 7, 8, 9, 10, 15, 20, 25, 30, 40, 50, 60, 70, 80, 90, or 100 different subsets of vesicles, wherein each subset of vesicles comprises a different biosignature.

In some embodiments, the microfluidic device can comprise one or more channels that permit further enrichment or selection of a vesicle. A population of vesicles that has been enriched after passage through a first channel can be introduced into a second channel, which allows the passage of the desired vesicle or vesicle population to be further enriched, such as through one or more binding agents present in the second channel.

Array-based assays and bead-based assays can be used with microfluidic device. For example, the binding agent can be coupled to beads and the binding reaction between the beads and vesicle can be performed in a microfluidic device. Multiplexing can also be performed using a microfluidic device. Different compartments can comprise different binding agents for different populations of vesicles, where each population is of a different cell-of-origin specific vesicle population. In one embodiment, each population has a different biosignature. The hybridization reaction between the microsphere and vesicle can be performed in a microfluidic device and the reaction mixture can be delivered to a detection device. The detection device, such as a dual or multiple laser detection system can be part of the microfluidic system and can use a laser to identify each bead or microsphere by its color-coding, and another laser can detect the hybridization signal associated with each bead.

Any appropriate microfluidic device can be used in the methods of the invention. Examples of microfluidic devices that may be used, or adapted for use with vesicles, include but are not limited to those described in U.S. Pat. Nos. 7,591,936, 7,581,429, 7,579,136, 7,575,722, 7,568,399, 7,552,741, 7,544,506, 7,541,578, 7,518,726, 7,488,596, 7,485,214, 7,467,928, 7,452,713, 7,452,509, 7,449,096, 7,431,887, 7,422,725, 7,422,669, 7,419,822, 7,419,639, 7,413,709, 7,411,184, 7,402,229, 7,390,463, 7,381,471, 7,357,864, 7,351,592, 7,351,380, 7,338,637, 7,329,391, 7,323,140, 7,261,824, 7,258,837, 7,253,003, 7,238,324, 7,238,255, 7,233,865, 7,229,538, 7,201,881, 7,195,986, 7,189,581, 7,189,580, 7,189,368, 7,141,978, 7,138,062, 7,135,147, 7,125,711, 7,118,910, 7,118,661, 7,640,947, 7,666,361, 7,704,735; and International Patent Publication WO 2010/072410. Another example for use with methods disclosed herein is described in Chen et al., "Microfluidic isolation and transcriptome analysis of serum vesicles, " Lab on a Chip, Dec. 8, 2009 DOI: 10.1039/b916199f.

Other microfluidic devices for use with the invention include devices comprising elastomeric layers, valves and pumps, including without limitation those disclosed in U.S. Patent Nos. 5,376,252, 6,408,878, 6,645,432, 6,719,868, 6,793,753, 6,899,137, 6,929,030, 7,040,338, 7,118,910, 7,144,616, 7,216,671, 7,250,128, 7,494,555, 7,501,245, 7,601,270, 7,691,333, 7,754,010, 7,837,946; U.S. Patent Application Nos. 2003/0061687, 2005/0084421, 2005/0112882, 2005/0129581, 2005/0145496, 2005/0201901, 2005/0214173, 2005/0252773, 2006/0006067; and EP Patent Nos. 0527905 and 1065378. In some instances, much or all of the devices are composed of elastomeric material. Certain devices are designed to conduct thermal cycling reactions (e.g., PCR) with devices that include one or more elastomeric valves to regulate solution flow through the device. The devices can comprise arrays of reaction sites thereby allowing a plurality of reactions to be performed. Thus, the devices can be used to assess circulating microRNAs in a multiplex fashion, including microRNAs isolated from vesicles. In an embodiment, the microfluidic device comprises (a) a first plurality of flow channels formed in an elastomeric substrate; (b) a second plurality of flow channels formed in the elastomeric substrate that intersect the first plurality of flow channels to define an array of reaction sites, each reaction site located at an intersection of one of the first and second flow channels; (c) a plurality of isolation valves disposed along the first and second plurality of flow channels and spaced between the reaction sites that can be actuated to isolate a solution within each of the reaction sites from solutions at other reaction sites, wherein the isolation valves comprise one or more control channels that each overlay and intersect one or more of the flow channels; and (d) means for simultaneously actuating the valves for isolating the reaction sites from each other. Various modifications to the basic structure of the device are envisioned. MicroRNAs can be detected in each of the reaction sites by using PCR methods. For example, the method can comprise the steps of: (i) providing a microfluidic device, the microfluidic device comprising: a first fluidic channel having a first end and a second end in fluid communication with each other through the channel; a plurality of flow channels, each flow channel terminating at a terminal wall; wherein each flow channel branches from and is in fluid communication with the first fluidic channel, wherein an aqueous fluid that enters one of the flow channels from the first fluidic channel can flow out of the flow channel only through the first fluidic channel; and, an inlet in fluid communication with the first fluidic channel, the inlet for introducing a sample fluid; wherein each flow channel is associated with a valve that when closed isolates one end of the flow channel from the first fluidic channel, whereby an isolated reaction site is formed between the valve and the terminal wall; a control channel; wherein each the valve is a deflectable membrane which is deflected into the flow channel associated with the valve when an actuating force is applied to the control channel, thereby closing the valve; and wherein when the actuating force is applied to the control channel a valve in each of the flow channels is closed, so as to produce the isolated reaction site in each flow channel; (ii) introducing the sample fluid into the inlet, the sample fluid filling the flow channels; (iii) actuating the valve to separate the sample fluid into the separate portions within the flow channels; (iv) amplifying the nucleic acid in the sample fluid; (v) analyzing the portions of the sample fluid to determine whether the amplifying produced the reaction. The sample fluid can contain an amplifiable nucleic acid target, e.g., a microRNA, and the conditions can be polymerase chain reaction (PCR) conditions, so that the reaction results in a PCR product being formed.

The microfluidic device can have one or more binding agents attached to a surface in a channel, or present in a channel. For example, the microchannel can have one or more capture agents, such as a capture agent for one or more general microvesicle antigen in **Table 3** or a cell-of-origin or cancer related antigen in **Table 4,** including without limitation EpCam, CD9, PCSA, CD63, CD81, PSMA, B7H3, PSCA, ICAM, STEAP, KLK2, SSX2, SSX4, PBP, SPDEF, and EGFR. The capture agent may be an aptamer selected by the methods. The surface of the channel can also be contacted with a blocking aptamer. A microchannel surface may be treated with avidin and a capture agent, that is biotinylated can be injected into the channel to bind the avidin. The capture agents may be present in chambers or other components of a microfluidic device. The capture agents can also be attached to beads that can be manipulated to move through the microfluidic channels. The capture agents may be attached to magnetic beads. The beads can be manipulated using magnets.

A biological sample can be flowed into the microfluidic device, or a microchannel, at rates such as at least about 1, 2, 3, 4, 5, 6, 7, 8, 9, 10, 11, 12, 13, 14, 15, 16, 17, 18, 19, 20, 25, 30, 35, 40, 45, or 50 μl per minute, such as between about 1-50, 5-40, 5-30, 3-20 or 5-15 μl per minute. One or more vesicles can be captured and directly detected in the microfluidic device. Alternatively, the captured vesicle may be released and exit the microfluidic device prior to analysis. In another embodiment, one or more captured vesicles are lysed in the microchannel and the lysate can be analyzed, e.g., to examine payload within the vesicles. Lysis buffer can be flowed through the channel and lyse the captured vesicles. For example, the lysis buffer can be flowed into the device or microchannel at rates such as at least about a, 2, 3, 4, 5, 6, 7, 8, 9, 10, 11, 12, 13, 14, 15, 16, 17, 18, 19, 20, 25, 26, 27, 28, 29, 30, 35, 40, 45, or 50 μl per

minute, such as between about 1-50, 5-40, 10-30, 5-30 or 10-35 μl per minute. The lysate can be collected and analyzed, such as performing RT-PCR, PCR, mass spectrometry, Western blotting, or other assays, to detect one or more biomarkers of the vesicle.

**Phenotypes**

Disclosed herein are products and processes for characterizing a phenotype using the methods of the invention. The term "phenotype" as used herein can mean any trait or characteristic that is attributed to a biomarker profile that is identified using in part or in whole the methods of the invention. For example, a phenotype can be a diagnostic, prognostic or theranostic determination based on a characterized biomarker profile for a sample obtained from a subject. A phenotype can be any observable characteristic or trait of, such as a disease or condition, a stage of a disease or condition, susceptibility to a disease or condition, prognosis of a disease stage or condition, a physiological state, or response / potential response to therapeutics. A phenotype can result from a subject's genetic makeup as well as the influence of environmental factors and the interactions between the two, as well as from epigenetic modifications to nucleic acid sequences.

A phenotype in a subject can be characterized by obtaining a biological sample from a subject and analyzing the sample using the methods of the invention. For example, characterizing a phenotype for a subject or individual can include detecting a disease or condition (including pre-symptomatic early stage detecting), determining a prognosis, diagnosis, or theranosis of a disease or condition, or determining the stage or progression of a disease or condition. Characterizing a phenotype can include identifying appropriate treatments or treatment efficacy for specific diseases, conditions, disease stages and condition stages, predictions and likelihood analysis of disease progression, particularly disease recurrence, metastatic spread or disease relapse. A phenotype can also be a clinically distinct type or subtype of a condition or disease, such as a cancer or tumor. Phenotype determination can also be a determination of a physiological condition, or an assessment of organ distress or organ rejection, such as post-transplantation. The compositions and methods described herein allow assessment of a subject on an individual basis, which can provide benefits of more efficient and economical decisions in treatment.

In an aspect, the disclosure relates to the analysis of biomarkers such as microvesicles to provide a diagnosis, prognosis, and/or theranosis of a disease or condition. Theranostics includes diagnostic testing that provides the ability to affect therapy or treatment of a disease or disease state. Theranostics testing provides a theranosis in a similar manner that diagnostics or prognostic testing provides a diagnosis or prognosis, respectively. As used herein, theranostics encompasses any desired form of therapy related testing, including predictive medicine, personalized medicine, integrated medicine, pharmacodiagnostics and Dx/Rx partnering. Therapy related tests can be used to predict and assess drug response in individual subjects, i.e., to provide personalized medicine. Predicting a drug response can be determining whether a subject is a likely responder or a likely non-responder to a candidate therapeutic agent, e.g., before the subject has been exposed or otherwise treated with the treatment. Assessing a drug response can be monitoring a response to a drug, e.g., monitoring the subject's improvement or lack thereof over a time course after initiating the treatment. Therapy related tests are useful to select a subject for treatment who is particularly likely to benefit from the treatment or to provide an early and objective indication of treatment efficacy in an individual subject. Thus, analysis using the methods of the invention may indicate that treatment should be altered to select a more promising treatment, thereby avoiding the great expense of delaying beneficial treatment and avoiding the financial and morbidity costs of administering an ineffective drug(s).

Thus, the methods of the invention may help predict whether a subject is likely to respond to a treatment for a disease or disorder. Characterizating a phenotype includes predicting the responder / non-responder status of the subject, wherein a responder responds to a treatment for a disease and a non-responder does not respond to the treatment. Biomarkers such as microvesicles can be analyzed in the subject and compared against that of previous subjects that were known to respond or not to a treatment. If the biomarker profile in the subject more closely aligns with that of previous subjects that were known to respond to the treatment, the subject can be characterized, or predicted, as a responder to the treatment. Similarly, if the biomarker profile in the subject more closely aligns with that of previous subjects that did not respond to the treatment, the subject can be characterized, or predicted as a non-responder to the treatment. The treatment can be for any appropriate disease, disorder or other condition, including without limitation those disclosed herein.

The phenotype may comprise a disease or condition such as those listed in **Tables 1** or **16.** For example, the phenotype can comprise detecting the presence of or likelihood of developing a tumor, neoplasm, or cancer, or characterizing the tumor, neoplasm, or cancer (e.g., stage, grade, aggressiveness, likelihood of metastatis or recurrence, etc). Cancers that can be detected or assessed by methods or compositions described herein include, but are not limited to, breast cancer, ovarian cancer, lung cancer, colon cancer, hyperplastic polyp, adenoma, colorectal cancer, high grade dysplasia, low grade dysplasia, prostatic hyperplasia, prostate cancer, melanoma, pancreatic cancer, brain cancer (such as a glioblastoma), hematological malignancy, hepatocellular carcinoma, cervical cancer, endometrial cancer, head and neck cancer, esophageal cancer, gastrointestinal stromal tumor (GIST), renal cell carcinoma (RCC) or gastric cancer. The colorectal cancer can be CRC Dukes B or Dukes C-D. The hematological malignancy can be B-Cell Chronic Lymphocytic Leukemia, B-Cell Lymphoma-DLBCL, B-Cell Lymphoma-DLBCL-germinal center-like, B-Cell Lymphoma-DLBCL-activated B-cell-like, and Burkitt's lymphoma.

The phenotype can be a premalignant condition, such as actinic keratosis, atrophic gastritis, leukoplakia, erythroplasia, Lymphomatoid Granulomatosis, preleukemia, fibrosis, cervical dysplasia, uterine cervical dysplasia, xeroderma pigmentosum, Barrett's Esophagus, colorectal polyp, or other abnormal tissue growth or lesion that is likely to develop into a malignant tumor. Transformative viral infections such as HIV and HPV also present phenotypes that can be assessed according to the invention.

A cancer characterized by the methods of the invention can comprise, without limitation, a carcinoma, a sarcoma, a lymphoma or leukemia, a germ cell tumor, a blastoma, or other cancers. Carcinomas include without limitation epithelial neoplasms, squamous cell neoplasms squamous cell carcinoma, basal cell neoplasms basal cell carcinoma, transitional cell papillomas and carcinomas, adenomas and adenocarcinomas (glands), adenoma, adenocarcinoma, linitis plastica insulinoma, glucagonoma, gastrinoma, vipoma, cholangiocarcinoma, hepatocellular carcinoma, adenoid cystic carcinoma, carcinoid tumor of appendix, prolactinoma, oncocytoma, hurthle cell adenoma, renal cell carcinoma, grawitz tumor, multiple endocrine adenomas, endometrioid adenoma, adnexal and skin appendage neoplasms, mucoepidermoid neoplasms, cystic, mucinous and serous neoplasms, cystadenoma, pseudomyxoma peritonei, ductal, lobular and medullary neoplasms, acinar cell neoplasms, complex epithelial neoplasms, warthin's tumor, thymoma, specialized gonadal neoplasms, sex cord stromal tumor, thecoma, granulosa cell tumor, arrhenoblastoma, sertoli leydig cell tumor, glomus tumors, paraganglioma, pheochromocytoma, glomus tumor, nevi and melanomas, melanocytic nevus, malignant melanoma, melanoma, nodular melanoma, dysplastic nevus, lentigo maligna melanoma, superficial spreading melanoma, and malignant acral lentiginous melanoma. Sarcoma includes without limitation Askin's tumor, botryodies, chondrosarcoma, Ewing's sarcoma, malignant hemangio endothelioma, malignant schwannoma, osteosarcoma, soft tissue sarcomas including: alveolar soft part sarcoma, angiosarcoma, cystosarcoma phyllodes, dermatofibrosarcoma, desmoid tumor, desmoplastic small round cell tumor, epithelioid sarcoma, extraskeletal chondrosarcoma, extraskeletal osteosarcoma, fibrosarcoma, hemangiopericytoma, hemangiosarcoma, kaposi's sarcoma, leiomyosarcoma, liposarcoma, lymphangiosarcoma, lymphosarcoma, malignant fibrous histiocytoma, neurofibrosarcoma, rhabdomyosarcoma, and synovialsarcoma. Lymphoma and leukemia include without limitation chronic lymphocytic leukemia/small lymphocytic lymphoma, B-cell prolymphocytic leukemia, lymphoplasmacytic lymphoma (such as waldenström macroglobulinemia), splenic marginal zone lymphoma, plasma cell myeloma, plasmacytoma, monoclonal immunoglobulin deposition diseases, heavy chain diseases, extranodal marginal zone B cell lymphoma, also called malt lymphoma, nodal marginal zone B cell lymphoma (nmzl), follicular lymphoma, mantle cell lymphoma, diffuse large B cell lymphoma, mediastinal (thymic) large B cell lymphoma, intravascular large B cell lymphoma, primary effusion lymphoma, burkitt lymphoma/leukemia, T cell prolymphocytic leukemia, T cell large granular lymphocytic leukemia, aggressive NK cell leukemia, adult T cell leukemia/lymphoma, extranodal NK/T cell lymphoma, nasal type, enteropathy-type T cell lymphoma, hepatosplenic T cell lymphoma, blastic NK cell lymphoma, mycosis fungoides / sezary syndrome, primary cutaneous CD30-positive T cell lymphoproliferative disorders, primary cutaneous anaplastic large cell lymphoma, lymphomatoid papulosis, angioimmunoblastic T cell lymphoma, peripheral T cell lymphoma, unspecified, anaplastic large cell lymphoma, classical hodgkin lymphomas (nodular sclerosis, mixed cellularity, lymphocyte-rich, lymphocyte depleted or not depleted), and nodular lymphocyte-predominant hodgkin lymphoma. Germ cell tumors include without limitation germinoma, dysgerminoma, seminoma, nongerminomatous germ cell tumor, embryonal carcinoma, endodermal sinus turmor, choriocarcinoma, teratoma, polyembryoma, and gonadoblastoma. Blastoma includes without limitation nephroblastoma, medulloblastoma, and retinoblastoma. Other cancers include without limitation labial carcinoma, larynx carcinoma, hypopharynx carcinoma, tongue carcinoma, salivary gland carcinoma, gastric carcinoma, adenocarcinoma, thyroid cancer (medullary and papillary thyroid carcinoma), renal carcinoma, kidney parenchyma carcinoma, cervix carcinoma, uterine corpus carcinoma, endometrium carcinoma, chorion carcinoma, testis carcinoma, urinary carcinoma, melanoma, brain tumors such as glioblastoma, astrocytoma, meningioma, medulloblastoma and peripheral neuroectodermal tumors, gall bladder carcinoma, bronchial carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, and plasmocytoma.

In a further embodiment, the cancer under analysis may be a lung cancer including non-small cell lung cancer and small cell lung cancer (including small cell carcinoma (oat cell cancer), mixed small cell/large cell carcinoma, and combined small cell carcinoma), colon cancer, breast cancer, prostate cancer, liver cancer, pancreas cancer, brain cancer, kidney cancer, ovarian cancer, stomach cancer, skin cancer, bone cancer, gastric cancer, breast cancer, pancreatic cancer, glioma, glioblastoma, hepatocellular carcinoma, papillary renal carcinoma, head and neck squamous cell carcinoma, leukemia, lymphoma, myeloma, or a solid tumor.

In embodiments, the cancer comprises an acute lymphoblastic leukemia; acute myeloid leukemia; adrenocortical carcinoma; AIDS-related cancers; AIDS-related lymphoma; anal cancer; appendix cancer; astrocytomas; atypical teratoid/rhabdoid tumor; basal cell carcinoma; bladder cancer; brain stem glioma; brain tumor (including brain stem glioma, central nervous system atypical teratoid/rhabdoid tumor, central nervous system embryonal tumors, astrocytomas, craniopharyngioma, ependymoblastoma, ependymoma, medulloblastoma, medulloepithelioma, pineal parenchymal tumors of intermediate differentiation, supratentorial primitive neuroectodermal tumors and pineoblastoma); breast cancer; bronchial tumors; Burkitt lymphoma; cancer of unknown primary site; carcinoid tumor; carcinoma of unknown primary site; central nervous system atypical teratoid/rhabdoid tumor; central nervous system embryonal tumors; cervical cancer; childhood cancers; chordoma; chronic lymphocytic leukemia; chronic myelogenous leukemia; chronic myeloproliferative disorders; colon cancer; colorectal cancer; craniopharyngioma; cutaneous T-cell lymphoma; endocrine pancreas islet cell tumors; endometrial cancer; ependymoblastoma; ependymoma; esophageal cancer; esthesioneuroblastoma; Ewing sarcoma; extracranial germ cell tumor; extragonadal germ cell tumor; extrahepatic bile duct cancer; gallbladder cancer; gastric (stomach) cancer; gastrointestinal carcinoid tumor; gastrointestinal stromal cell tumor; gastrointestinal stromal tumor (GIST); gestational trophoblastic tumor; glioma; hairy cell leukemia; head and neck cancer; heart cancer; Hodgkin lymphoma; hypopharyngeal cancer; intraocular melanoma; islet cell tumors; Kaposi sarcoma; kidney cancer; Langerhans cell histiocytosis; laryngeal cancer; lip cancer; liver cancer; malignant fibrous histiocytoma bone cancer; medulloblastoma; medulloepithelioma; melanoma; Merkel cell carcinoma; Merkel cell skin carcinoma; mesothelioma; metastatic squamous neck cancer with occult primary; mouth cancer; multiple endocrine neoplasia syndromes; multiple myeloma; multiple myeloma/plasma cell neoplasm; mycosis fungoides; myelodysplastic syndromes; myeloproliferative neoplasms; nasal cavity cancer; nasopharyngeal cancer; neuroblastoma; Non-Hodgkin lymphoma; nonmelanoma skin cancer; non-small cell lung cancer; oral cancer; oral cavity cancer; oropharyngeal cancer; osteosarcoma; other brain and spinal cord tumors; ovarian cancer; ovarian epithelial cancer; ovarian germ cell tumor; ovarian low malignant potential tumor; pancreatic cancer; papillomatosis; paranasal sinus cancer; parathyroid cancer; pelvic cancer; penile cancer; pharyngeal cancer; pineal parenchymal tumors of intermediate differentiation; pineoblastoma; pituitary tumor; plasma cell neoplasm/multiple myeloma; pleuropulmonary blastoma; primary central nervous system (CNS) lymphoma; primary hepatocellular liver cancer; prostate cancer; rectal cancer; renal cancer; renal cell (kidney) cancer; renal cell cancer; respiratory tract cancer; retinoblastoma; rhabdomyosarcoma; salivary gland cancer; Sezary syndrome; small cell lung cancer; small intestine cancer; soft tissue sarcoma; squamous cell carcinoma; squamous neck cancer; stomach (gastric) cancer; supratentorial primitive neuroectodermal tumors; T-cell lymphoma; testicular cancer; throat cancer; thymic carcinoma; thymoma; thyroid cancer; transitional cell cancer; transitional cell cancer of the renal pelvis and ureter; trophoblastic tumor; ureter cancer; urethral cancer; uterine cancer; uterine sarcoma; vaginal cancer; vulvar cancer; Waldenström macroglobulinemia; or Wilm's tumor. The methods of the invention can be used to characterize these and other cancers. Thus, characterizing a phenotype can be providing a diagnosis, prognosis or theranosis of one of the cancers disclosed herein.

In some embodiments, the cancer comprises an acute myeloid leukemia (AML), breast carcinoma, cholangiocarcinoma, colorectal adenocarcinoma, extrahepatic bile duct adenocarcinoma, female genital tract malignancy, gastric adenocarcinoma, gastroesophageal adenocarcinoma, gastrointestinal stromal tumors (GIST), glioblastoma, head and neck squamous carcinoma, leukemia, liver hepatocellular carcinoma, low grade glioma, lung bronchioloalveolar carcinoma (BAC), lung non-small cell lung cancer (NSCLC), lung small cell cancer (SCLC), lymphoma, male genital tract malignancy, malignant solitary fibrous tumor of the pleura (MSFT), melanoma, multiple myeloma, neuroendocrine tumor, nodal diffuse large B-cell lymphoma, non epithelial ovarian cancer (non-EOC), ovarian surface epithelial carcinoma, pancreatic adenocarcinoma, pituitary carcinomas, oligodendroglioma, prostatic adenocarcinoma, retroperitoneal or peritoneal carcinoma, retroperitoneal or peritoneal sarcoma, small intestinal malignancy, soft tissue tumor, thymic carcinoma, thyroid carcinoma, or uveal melanoma. The methods of the invention can be used to characterize these and other cancers. Thus, characterizing a phenotype can be providing a diagnosis, prognosis or theranosis of one of the cancers disclosed herein.

The phenotype can also be an inflammatory disease, immune disease, or autoimmune disease. For example, the disease may be inflammatory bowel disease (IBD), Crohn's disease (CD), ulcerative colitis (UC), pelvic inflammation, vasculitis, psoriasis, diabetes, autoimmune hepatitis, Multiple Sclerosis, Myasthenia Gravis, Type I diabetes, Rheumatoid Arthritis, Psoriasis, Systemic Lupus Erythematosis (SLE), Hashimoto's Thyroiditis, Grave's disease, Ankylosing Spondylitis Sjogrens Disease, CREST syndrome, Scleroderma, Rheumatic Disease, organ rejection, Primary Sclerosing Cholangitis, or sepsis.

The phenotype can also comprise a cardiovascular disease, such as atherosclerosis, congestive heart failure, vulnerable plaque, stroke, or ischemia. The cardiovascular disease or condition can be high blood pressure, stenosis, vessel occlusion or a thrombotic event.

The phenotype can also comprise a neurological disease, such as Multiple Sclerosis (MS), Parkinson's Disease (PD), Alzheimer's Disease (AD), schizophrenia, bipolar disorder, depression, autism, Prion Disease, Pick's disease, dementia, Huntington disease (HD), Down's syndrome, cerebrovascular disease, Rasmussen's encephalitis, viral meningitis, neurospsychiatric systemic lupus erythematosus (NPSLE), amyotrophic lateral sclerosis, Creutzfeldt-Jacob disease, Gerstmann-Straussler-Scheinker disease, transmissible spongiform encephalopathy, ischemic reperfusion damage (e.g. stroke), brain trauma, microbial infection, or chronic fatigue syndrome. The phenotype may also be a condition such as fibromyalgia, chronic neuropathic pain, or peripheral neuropathic pain.

The phenotype may also comprise an infectious disease, such as a bacterial, viral or yeast infection. For example, the disease or condition may be Whipple's Disease, Prion Disease, cirrhosis, methicillin-resistant staphylococcus aureus, HIV, hepatitis, syphilis, meningitis, malaria, tuberculosis, or influenza. Viral proteins, such as HIV or HCV-like particles can be assessed in a vesicle, to characterize a viral condition.

The phenotype can also comprise a perinatal or pregnancy related condition (e.g. preeclampsia or preterm birth), metabolic disease or condition, such as a metabolic disease or condition associated with iron metabolism. For example, hepcidin can be assayed in a vesicle to characterize an iron deficiency. The metabolic disease or condition can also be diabetes, inflammation, or a perinatal condition.

The methods of the invention can be used to characterize these and other diseases and disorders that can be assessed via biomarkers. Thus, characterizing a phenotype can be providing a diagnosis, prognosis or theranosis of one of the diseases and disorders disclosed herein.

**Subject**

One or more phenotypes of a subject can be determined by analyzing one or more vesicles, such as vesicles, in a biological sample obtained from the subject. A subject or patient can include, but is not limited to, mammals such as bovine, avian, canine, equine, feline, ovine, porcine, or primate animals (including humans and non-human primates). A subject can also include a mammal of importance due to being endangered, such as a Siberian tiger; or economic importance, such as an animal raised on a farm for consumption by humans, or an animal of social importance to humans, such as an animal kept as a pet or in a zoo. Examples of such animals include, but are not limited to, carnivores such as cats and dogs; swine including pigs, hogs and wild boars; ruminants or ungulates such as cattle, oxen, sheep, giraffes, deer, goats, bison, camels or horses. Also included are birds that are endangered or kept in zoos, as well as fowl and more particularly domesticated fowl, i.e. poultry, such as turkeys and chickens, ducks, geese, guinea fowl. Also included are domesticated swine and horses (including race horses). In addition, any animal species connected to commercial activities are also included such as those animals connected to agriculture and aquaculture and other activities in which disease monitoring, diagnosis, and therapy selection are routine practice in husbandry for economic productivity and/or safety of the food chain.

The subject can have a pre-existing disease or condition, such as cancer. Alternatively, the subject may not have any known pre-existing condition. The subject may also be non-responsive to an existing or past treatment, such as a treatment for cancer.

**Samples**

A sample used and/or assessed via the methods of the invention includes any relevant biological sample that can be used for biomarker assessment, including without limitation sections of tissues such as biopsy or tissue removed during surgical or other procedures, bodily fluids, autopsy samples, frozen sections taken for histological purposes, and cell cultures. Such samples include blood and blood fractions or products (e.g., serum, buffy coat, plasma, platelets, red blood cells, and the like), sputum, malignant effusion, cheek cells tissue, cultured cells (e.g., primary cultures, explants, and transformed cells), stool, urine, other biological or bodily fluids (e.g., prostatic fluid, gastric fluid, intestinal fluid, renal fluid, lung fluid, cerebrospinal fluid, and the like), etc. The sample can comprise biological material that is a fresh frozen & formalin fixed paraffin embedded (FFPE) block, formalin-fixed paraffin embedded, or is within an RNA preservative + formalin fixative. More than one sample of more than one type can be used for each patient.

The sample used in the methods described herein can be a formalin fixed paraffin embedded (FFPE) sample. The FFPE sample can be one or more of fixed tissue, unstained slides, bone marrow core or clot, core needle biopsy, malignant fluids and fine needle aspirate (FNA). In an embodiment, the fixed tissue comprises a tumor containing formalin fixed paraffin embedded (FFPE) block from a surgery or biopsy. In another embodiment, the unstained slides comprise unstained, charged, unbaked slides from a paraffin block. In another embodiment, bone marrow core or clot comprises a decalcified core. A formalin fixed core and/or clot can be paraffin-embedded. In still another embodiment, the core needle biopsy comprises 1, 2, 3, 4, 5, 6, 7, 8, 9, 10 or more, e.g., 3-4, paraffin embedded biopsy samples. An 18 gauge needle biopsy can be used. The malignant fluid can comprise a sufficient volume of fresh pleural/ascitic fluid to produce a 5x5x2mm cell pellet. The fluid can be formalin fixed in a paraffin block. In an embodiment, the core needle biopsy comprises 1, 2, 3, 4, 5, 6, 7, 8, 9, 10 or more, e.g., 4-6, paraffin embedded aspirates.

A sample may be processed according to techniques understood by those in the art. A sample can be without limitation fresh, frozen or fixed cells or tissue. In some embodiments, a sample comprises formalin-fixed paraffin-embedded (FFPE) tissue, fresh tissue or fresh frozen (FF) tissue. A sample can comprise cultured cells, including primary or immortalized cell lines derived from a subject sample. A sample can also refer to an extract from a sample from a subject. For example, a sample can comprise DNA, RNA or protein extracted from a tissue or a bodily fluid. Many techniques and commercial kits are available for such purposes. The fresh sample from the individual can be treated with an agent to preserve RNA prior to further processing, e.g., cell lysis and extraction. Samples can include frozen samples collected for other purposes. Samples can be associated with relevant information such as age, gender, and clinical symptoms present in the subject; source of the sample; and methods of collection and storage of the sample. A sample is typically obtained from a subject.

A biopsy comprises the process of removing a tissue sample for diagnostic or prognostic evaluation, and to the tissue specimen itself. Any biopsy technique known in the art can be applied to the molecular profiling methods of the present invention. The biopsy technique applied can depend on the tissue type to be evaluated (e.g., colon, prostate, kidney, bladder, lymph node, liver, bone marrow, blood cell, lung, breast, etc.), the size and type of the tumor (e.g., solid or suspended, blood or ascites), among other factors. Representative biopsy techniques include, but are not limited to, excisional biopsy, incisional biopsy, needle biopsy, surgical biopsy, and bone marrow biopsy. An "excisional biopsy" refers to the removal of an entire tumor mass with a small margin of normal tissue surrounding it. An "incisional biopsy" refers to the removal of a wedge of tissue that includes a cross-sectional diameter of the tumor. Molecular profiling can use a "core-needle biopsy" of the tumor mass, or a "fine-needle aspiration biopsy" which generally obtains a suspension of cells from within the tumor mass. Biopsy techniques are discussed, for example, in Harrison's Principles of Internal Medicine, Kasper, et al., eds., 16th ed., 2005, Chapter 70, and throughout Part V.

Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998) and methodology as set forth in U.S. Pat. Nos. 4,666,828; 4,683,202; 4,801,531; 5,192,659 and 5,272,057. Polymerase chain reaction (PCR) can be carried out generally as in PCR Protocols: A Guide to Methods and Applications, Academic Press, San Diego, Calif. (1990).

The biological sample assessed using the methods of the invention can be any useful bodily or biological fluid, including but not limited to peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids, cells, cell culture, or a cell culture supernatant. A biological sample may also include the blastocyl cavity, umbilical cord blood, or maternal circulation which may be of fetal or maternal origin. The biological sample may also be a cell culture, tissue sample or biopsy from which vesicles and other circulating biomarkers may be obtained. For example, cells of interest can be cultured and vesicles isolated from the culture. In various embodiments, biomarkers or more particularly biosignatures disclosed herein can be assessed directly from such biological samples (e.g., identification of presence or levels of nucleic acid or polypeptide biomarkers or functional fragments thereof) using various methods, such as extraction of nucleic acid molecules from blood, plasma, serum or any of the foregoing biological samples, use of protein or antibody arrays to identify polypeptide (or functional fragment) biomarker(s), as well as other array, sequencing, PCR and proteomic techniques known in the art for identification and assessment of nucleic acid and polypeptide molecules. In addition, one or more components present in such samples can be first isolated or enriched and further processed to assess the presence or levels of selected biomarkers, to assess a given biosignature (e.g., isolated microvesicles prior to profiling for protein and/or nucleic acid biomarkers).

**Table 1** presents a non-limiting listing of diseases, conditions, or biological states and corresponding biological samples that may be used for analysis according to the methods of the invention.

**Table 1: Examples of Biological Samples for Various Diseases, Conditions, or Biological States**

| Illustrative Disease, Condition or Biological State | Illustrative Biological Samples |
|---|---|
| *Cancers/neoplasms affecting the following tissue types/ bodily systems:* breast, lung, ovarian, colon, rectal, prostate, pancreatic, brain, bone, connective tissue, glands, skin, lymph, nervous system, endocrine, germ cell, genitourinary, hematologic/blood, bone marrow, muscle, eye, esophageal, fat tissue, thyroid, pituitary, spinal cord, bile duct, heart, gall bladder, bladder, testes, cervical, endometrial, renal, ovarian, digestive/gastrointestinal, stomach, head and neck, liver, leukemia, respiratory/thorasic, cancers of unknown primary (CUP) | Tumor, blood, serum, plasma, cerebrospinal fluid (CSF), urine, sputum, ascites, synovial fluid, semen, nipple aspirates, saliva, bronchoalveolar lavage fluid, tears, oropharyngeal washes, feces, peritoneal fluids, pleural effusion, sweat, tears, aqueous humor, pericardial fluid, lymph, chyme, chyle, bile, stool water, amniotic fluid, breast milk, pancreatic juice, cerumen, Cowper's fluid or pre-ejaculatory fluid, female ejaculate, interstitial fluid, menses, mucus, pus, sebum, vaginal lubrication, vomit |
| *Neurodegenerative/neurological disorders:* Parkinson's disease, Alzheimer's Disease and multiple sclerosis, Schizophrenia, and bipolar disorder, spasticity disorders, epilepsy | Blood, serum, plasma, CSF, urine |
| *Cardiovascular Disease:* atherosclerosis, cardiomyopathy, endocarditis, vunerable plaques, infection | Blood, serum, plasma, CSF, urine |
| *Stroke:* ischemic, intracerebral hemorrhage, subarachnoid hemorrhage, transient ischemic attacks (TIA) | Blood, serum, plasma, CSF, urine |
| *Pain disorders:* peripheral neuropathic pain and chronic neuropathic pain, and fibromyalgia, | Blood, serum, plasma, CSF, urine |
| *Autoimmune disease:* systemic and localized diseases, rheumatic disease, Lupus, Sjogren's syndrome | Blood, serum, plasma, CSF, urine, synovial fluid |
| *Digestive system abnormalities:* Barrett's esophagus, irritable bowel syndrome, ulcerative colitis, Crohn's disease, Diverticulosis and Diverticulitis, Celiac Disease | Blood, serum, plasma, CSF, urine |
| *Endocrine disorders:* diabetes mellitus, various forms of Thyroiditis, adrenal disorders, pituitary disorders | Blood, serum, plasma, CSF, urine |
| *Diseases and disorders of the skin:* psoriasis | Blood, serum, plasma, CSF, urine, synovial fluid, tears |
| *Urological disorders:* benign prostatic hypertrophy (BPH), polycystic kidney disease, interstitial cystitis | Blood, serum, plasma, urine |
| *Hepatic disease/injury:* Cirrhosis, induced hepatotoxicity (due to exposure to natural or synthetic chemical sources) | Blood, serum, plasma, urine |
| *Kidney disease/injury:* acute, sub-acute, chronic conditions, Podocyte injury, focal segmental glomerulosclerosis | Blood, serum, plasma, urine |
| Endometriosis | Blood, serum, plasma, urine, vaginal fluids |
| Osteoporosis | Blood, serum, plasma, urine, synovial fluid |
| Pancreatitis | Blood, serum, plasma, urine, pancreatic juice |
| Asthma | Blood, serum, plasma, urine, sputum, bronchiolar lavage fluid |
| Allergies | Blood, serum, plasma, urine, sputum, bronchiolar lavage fluid |
| Prion-related diseases | Blood, serum, plasma, CSF, urine |
| *Viral Infections:* HIV/AIDS | Blood, serum, plasma, urine |
| Sepsis | Blood, serum, plasma, urine, tears, nasal lavage |
| Organ rejection/transplantation | Blood, serum, plasma, urine, various lavage fluids |

(continued)

| Illustrative Disease, Condition or Biological State | Illustrative Biological Samples |
|---|---|
| *Differentiating conditions:* adenoma versus hyperplastic polyp, irritable bowel syndrome (IBS) versus normal, classifying Dukes stages A, B, C, and/or D of colon cancer, adenoma with low-grade hyperplasia versus high-grade hyperplasia, adenoma versus normal, colorectal cancer versus normal, IBS versus. ulcerative colitis (UC) versus Crohn's disease (CD), | Blood, serum, plasma, urine, sputum, feces, colonic lavage fluid |
| *Pregnancy related physiological states, conditions, or affiliated diseases:* genetic risk, adverse pregnancy outcomes | Maternal serum, plasma, amniotic fluid, cord blood |

The methods of the invention can be used to characterize a phenotype using a blood sample or blood derivative. Blood derivatives include plasma and serum. Blood plasma is the liquid component of whole blood, and makes up approximately 55% of the total blood volume. It is composed primarily of water with small amounts of minerals, salts, ions, nutrients, and proteins in solution. In whole blood, red blood cells, leukocytes, and platelets are suspended within the plasma. Blood serum refers to blood plasma without fibrinogen or other clotting factors (i.e., whole blood minus both the cells and the clotting factors).

The biological sample may be obtained through a third party, such as a party not performing the analysis of the biomarkers, whether direct assessment of a biological sample or by profiling one or more vesicles obtained from the biological sample. For example, the sample may be obtained through a clinician, physician, or other health care manager of a subject from which the sample is derived. Alternatively, the biological sample may obtained by the same party analyzing the vesicle. In addition, biological samples be assayed, are archived (e.g., frozen) or ortherwise stored in under preservative conditions.

Furthermore, a biological sample can comprise a vesicle or cell membrane fragment that is derived from a cell of origin and available extracellularly in a subject's biological fluid or extracellular milieu.

Methods of the invention can include assessing one or more vesicles, including assessing vesicle populations. A vesicle, as used herein, is a membrane vesicle that is shed from cells. Vesicles or membrane vesicles include without limitation: circulating microvesicles (cMVs), microvesicle, exosome, nanovesicle, dexosome, bleb, blebby, prostasome, microparticle, intralumenal vesicle, membrane fragment, intralumenal endosomal vesicle, endosomal-like vesicle, exocytosis vehicle, endosome vesicle, endosomal vesicle, apoptotic body, multivesicular body, secretory vesicle, phospholipid vesicle, liposomal vesicle, argosome, texasome, secresome, tolerosome, melanosome, oncosome, or exocytosed vehicle. Furthermore, although vesicles may be produced by different cellular processes, the methods of the invention are not limited to or reliant on any one mechanism, insofar as such vesicles are present in a biological sample and are capable of being characterized by the methods disclosed herein. Unless otherwise specified, methods that make use of a species of vesicle can be applied to other types of vesicles. Vesicles comprise spherical structures with a lipid bilayer similar to cell membranes which surrounds an inner compartment which can contain soluble components, sometimes referred to as the payload. In some embodiments, the methods of the invention make use of exosomes, which are small secreted vesicles of about 40-100 nm in diameter. For a review of membrane vesicles, including types and characterizations, see Thery et al., Nat Rev Immunol. 2009 Aug;9(8):581-93. Some properties of different types of vesicles include those in **Table 2:**

**Table 2: Vesicle Properties**

| Feature | Exosomes | Microvesicles | Ectosomes | Membrane particles | Exosome-like vesicles | Apoptotic vesicles |
|---|---|---|---|---|---|---|
| Size | 50-100 nm | 100-1,000 nm | 50-200 nm | 50-80 nm | 20-50 nm | 50-500 nm |
| Density in sucrose | 1.13-1.19 g/ml | | | 1.04-1.07 g/ml | 1.1 g/ml | 1.16-1.28 g/ml |
| EM appearance | Cup shape | Irregular shape, electron dense | Bilamellar round structures | Round | Irregular shape | Heterogeneous |
| Sedimentation | 100,000 g | 10,000 g | 160,000-200,000 g | 100,000-200,000 g | 175,000 g | 1,200 g, 10,000 g, 100,000 g |
| Lipid composition | Enriched in cholesterol, sphingomyelin and ceramide; contains lipid rafts; expose PPS | Expose PPS | Enriched in cholesterol and diacylglycerol; expose PPS | | No lipid rafts | |
| Major protein markers | Tetraspanins (e.g., CD63, CD9), Alix, TSG101 | Integrins, selectins and CD40 ligand | CR1 and proteolytic enzymes; no CD63 | CD133; no CD63 | TNFRI | Histones |
| Intracellular origin | Internal compartments (endosomes) | Plasma membrane | Plasma membrane | Plasma membrane | | |
| *Abbreviations:* phosphatidylserine (PPS); electron microscopy (EM) | | | | | | |

Vesicles include shed membrane bound particles, or "microparticles," that are derived from either the plasma membrane or an internal membrane. Vesicles can be released into the extracellular environment from cells. Cells releasing vesicles include without limitation cells that originate from, or are derived from, the ectoderm, endoderm, or mesoderm. The cells may have undergone genetic, environmental, and/or any other variations or alterations. For example, the cell can be tumor cells. A vesicle can reflect any changes in the source cell, and thereby reflect changes in the originating cells, e.g., cells having various genetic mutations. In one mechanism, a vesicle is generated intracellularly when a segment of the cell membrane spontaneously invaginates and is ultimately exocytosed (see for example, Keller et al., Immunol. Lett. 107 (2): 102-8 (2006)). Vesicles also include cell-derived structures bounded by a lipid bilayer membrane arising from both herniated evagination (blebbing) separation and sealing of portions of the plasma membrane or from the export of any intracellular membrane-bounded vesicular structure containing various membrane-associated proteins of tumor origin, including surface-bound molecules derived from the host circulation that bind selectively to the tumor-derived proteins together with molecules contained in the vesicle lumen, including but not limited to tumor-derived microRNAs or intracellular proteins. Blebs and blebbing are further described in Charras et al., Nature Reviews Molecular and Cell Biology, Vol. 9, No. 11, p. 730-736 (2008). A vesicle shed into circulation or bodily fluids from tumor cells may be referred to as a "circulating tumor-derived vesicle." When such vesicle is an exosome, it may be referred to as a circulating-tumor derived exosome (CTE). In some instances, a vesicle can be derived from a specific cell of origin. CTE, as with a cell-of-origin specific vesicle, typically have one or more unique biomarkers that permit isolation of the CTE or cell-of-origin specific vesicle, e.g., from a bodily fluid and sometimes in a specific manner. For example, a cell or tissue specific markers are used to identify the cell of origin. Examples of such cell or tissue specific markers are disclosed herein and can further be accessed in the Tissue-specific Gene Expression and Regulation (TiGER) Database, available at bioinfo.wilmer.jhu.edu/tiger/; Liu et al. (2008) TiGER: a database for tissue-specific gene expression and regulation. BMC Bioinformatics. 9:271; TissueDistributionDBs, available at genome.dkfz-heidelberg.de/menu/tissue_db/index.html.

A vesicle can have a diameter of greater than about 10 nm, 20 nm, or 30 nm. A vesicle can have a diameter of greater than 40 nm, 50 nm, 100 nm, 200 nm, 500 nm, 1000 nm, 1500 nm, 2000 nm or greater than 10,000 nm. A vesicle can have a diameter of about 20-2000 nm, about 20-1500 nm, about 30-1000 nm, about 30-800 nm, about 30-200 nm, or about 30-100 nm. In some embodiments, the vesicle has a diameter of less than 10,000 nm, 2000 nm, 1500 nm, 1000 nm, 800 nm, 500 nm, 200 nm, 100 nm, 50 nm, 40 nm, 30 nm, 20 nm or less than 10 nm. As used herein the term "about" in reference to a numerical value means that variations of 10% above or below the numerical value are within the range ascribed to the specified value. Typical sizes for various types of vesicles are shown in **Table 2.** Vesicles can be assessed to measure the diameter of a single vesicle or any number of vesicles. For example, the range of diameters of a vesicle population or an average diameter of a vesicle population can be determined. Vesicle diameter can be assessed using methods known in the art, e.g., imaging technologies such as electron microscopy. In an embodiment, a diameter of one or more vesicles is determined using optical particle detection. See, e.g., U.S. Patent 7,751,053, entitled "Optical Detection and Analysis of Particles" and issued July 6, 2010; and U.S. Patent 7,399,600, entitled "Optical Detection and Analysis of Particles" and issued July 15, 2010.

In some embodiments, the methods of the invention comprise assessing vesicles directly such as in a biological sample without prior isolation, purification, or concentration from the biological sample. For example, the amount of vesicles in the sample can by itself provide a biosignature that provides a diagnostic, prognostic or theranostic determination. Alternatively, the vesicle in the sample may be isolated, captured, purified, or concentrated from a sample prior to analysis. As noted, isolation, capture or purification as used herein comprises partial isolation, partial capture or partial purification apart from other components in the sample. Vesicle isolation can be performed using various techniques as described herein, e.g., chromatography, filtration, centrifugation, flow cytometry, affinity capture (e.g., to a planar surface or bead), and/or using microfluidics. **FIGs. 13A-I** and **16B-16C** present an overview of a method for assessing microvesicles using an aptamer pool.

Vesicles such as exosomes can be assessed to provide a phenotypic characterization by comparing vesicle characteristics to a reference. In some embodiments, surface antigens on a vesicle are assessed. The surface antigens can provide an indication of the anatomical origin and/or cellular of the vesicles and other phenotypic information, e.g., tumor status. For example, wherein vesicles found in a patient sample, e.g., a bodily fluid such as blood, serum or plasma, are assessed for surface antigens indicative of colorectal origin and the presence of cancer. The surface antigens may comprise any informative biological entity that can be detected on the vesicle membrane surface, including without limitation surface proteins, lipids, carbohydrates, and other membrane components. For example, positive detection of colon derived vesicles expressing tumor antigens can indicate that the patient has colorectal cancer. As such, methods of the invention can be used to characterize any disease or condition associated with an anatomical or cellular origin, by assessing, for example, disease-specific and cell-specific biomarkers of one or more vesicles obtained from a subject.

In another embodiment, the methods of the invention comprise assessing one or more vesicle payload to provide a phenotypic characterization. The payload with a vesicle comprises any informative biological entity that can be detected as encapsulated within the vesicle, including without limitation proteins and nucleic acids, e.g., genomic or cDNA, mRNA, or functional fragments thereof, as well as microRNAs (miRs). In addition, methods of the invention are directed to detecting vesicle surface antigens (in addition or exclusive to vesicle payload) to provide a phenotypic characterization. For example, vesicles can be characterized by using binding agents (e.g., antibodies or aptamers) that are specific to vesicle surface antigens, and the bound vesicles can be further assessed to identify one or more payload components disclosed therein. As described herein, the levels of vesicles with surface antigens of interest or with payload of interest can be compared to a reference to characterize a phenotype. For example, overexpression in a sample of cancer-related surface antigens or vesicle payload, e.g., a tumor associated mRNA or microRNA, as compared to a reference, can indicate the presence of cancer in the sample. The biomarkers assessed can be present or absent, increased or reduced based on the selection of the desired target sample and comparison of the target sample to the desired reference sample. Non-limiting examples of target samples include: disease; treated/not-treated; different time points, such as a in a longitudinal study; and non-limiting examples of reference sample: non-disease; normal; different time points; and sensitive or resistant to candidate treatment(s).

**Microvesicle Isolation and Analysis**

*Sample Processing*

A vesicle or a population of vesicles may be isolated, purified, concentrated or otherwise enriched prior to and/or during analysis. Unless otherwise specified, the terms "purified," "isolated," or similar as used herein in reference to vesicles or biomarker components are intended to include partial or complete purification or isolation of such components from a cell or organism. Analysis of a vesicle can include quantitiating the amount one or more vesicle populations of a biological sample. For example, a heterogeneous population of vesicles can be quantitated, or a homogeneous population of vesicles, such as a population of vesicles with a particular biomarker profile, a particular biosignature, or derived from a particular cell type can be isolated from a heterogeneous population of vesicles and quantitated. Analysis of a vesicle can also include detecting, quantitatively or qualitatively, one or more particular biomarker profile or biosignature of a vesicle, as described herein.

A vesicle can be stored and archived, such as in a bio-fluid bank and retrieved for analysis as desired. A vesicle may also be isolated from a biological sample that has been previously harvested and stored from a living or deceased subject. In addition, a vesicle may be isolated from a biological sample which has been collected as described in King et al., Breast Cancer Res 7(5): 198-204 (2005). A vesicle can be isolated from an archived or stored sample. Alternatively, a vesicle may be isolated from a biological sample and analyzed without storing or archiving of the sample. Furthermore, a third party may obtain or store the biological sample, or obtain or store the vesicle for analysis.

An enriched population of vesicles can be obtained from a biological sample. For example, vesicles may be concentrated or isolated from a biological sample using size exclusion chromatography, density gradient centrifugation, differential centrifugation, nanomembrane ultrafiltration, immunoabsorbent capture, affinity purification, microfluidic separation, or combinations thereof.

Size exclusion chromatography, such as gel permeation columns, centrifugation or density gradient centrifugation, and filtration methods can be used. For example, a vesicle can be isolated by differential centrifugation, anion exchange and/or gel permeation chromatography (for example, as described in US Patent Nos. 6,899,863 and 6,812,023), sucrose density gradients, organelle electrophoresis (for example, as described in U.S. Patent No. 7,198,923), magnetic activated cell sorting (MACS), or with a nanomembrane ultrafiltration concentrator. Various combinations of isolation or concentration methods can be used.

Highly abundant proteins, such as albumin and immunoglobulin in blood samples, may hinder isolation of vesicles from a biological sample. For example, a vesicle can be isolated from a biological sample using a system that uses multiple antibodies that are specific to the most abundant proteins found in a biological sample, such as blood. Such a system can remove up to several proteins at once, thus unveiling the lower abundance species such as cell-of-origin specific vesicles. This type of system can be used for isolation of vesicles from biological samples such as blood, cerebrospinal fluid or urine. The isolation of vesicles from a biological sample may also be enhanced by high abundant protein removal methods as described in Chromy et al. J Proteome Res 2004; 3:1120-1127. In another embodiment, the isolation of vesicles from a biological sample may also be enhanced by removing serum proteins using glycopeptide capture as described in Zhang et al, Mol Cell Proteomics 2005;4:144-155. In addition, vesicles from a biological sample such as urine may be isolated by differential centrifugation followed by contact with antibodies directed to cytoplasmic or anti-cytoplasmic epitopes as described in Pisitkun et al., Proc Natl Acad Sci USA, 2004;101:13368-13373.

Plasma contains a large variety of proteins including albumin, immunoglobulins, and clotting proteins such as fibrinogen. About 60% of plasma protein comprises the protein albumin (e.g., human serum albumin or HSA), which contributes to osmotic pressure of plasma to assist in the transport of lipids and steroid hormones. Globulins make up about 35% of plasma proteins and are used in the transport of ions, hormones and lipids assisting in immune function. About 4% of plasma protein comprises fibrinogen which is essential in the clotting of blood and can be converted into the insoluble protein fibrin. Other types of blood proteins include: Prealbumin, Alpha 1 antitrypsin, Alpha 1 acid glycoprotein, Alpha 1 fetoprotein, Haptoglobin, Alpha 2 macroglobulin, Ceruloplasmin, Transferrin, complement proteins C3 and C4, Beta 2 microglobulin, Beta lipoprotein, Gamma globulin proteins, C-reactive protein (CRP), Lipoproteins (chylomicrons, VLDL, LDL, HDL), other globulins (types alpha, beta and gamma), Prothrombin and Mannose-binding lectin (MBL). Any of these proteins, including classes of proteins, or derivatives thereof (such as fibrin which is derived from the cleavage of fibrinogen) can be selectively depleted from a biological sample prior to further analysis performed on the sample. Without being bound by theory, removal of such background proteins may facilitate more sensitive, accurate, or precise detection of the biomarkers of interest in the sample.

Abundant proteins in blood or blood derivatives (e.g., plasma or serum) include without limitation albumin, IgG, transferrin, fibrinogen, IgA, $\alpha_2$-Macroglobulin, IgM, $\alpha_1$-Antitrypsin, complement C3, haptoglobulin, apolipoprotein A1, apolipoprotein A3, apolipoprotein B, $\alpha_1$-Acid Glycoprotein, ceruloplasmin, complement C4, C1q, IgD, prealbumin (transthyretin), and plasminogen. Such proteins can be depleted using commercially available columns and kits. Examples of such columns comprise the Multiple Affinity Removal System from Agilent Technologies (Santa Clara, CA). This system include various cartridges designed to deplete different protein profiles, including the following cartridges with performance characteristics according to the manufacturer: Human 14, which eliminates approximately 94% of total protein (albumin, IgG, antitrypsin, IgA, transferrin, haptoglobin, fibrinogen, alpha2-macroglobulin, alpha1-acid glycoprotein (orosomucoid), IgM, apolipoprotein AI, apolipoprotein AII, complement C3 and transthyretin); Human 7, which eliminates approximately 85 - 90% of total protein (albumin, IgG, IgA, transferrin, haptoglobin, antitrypsin, and fibrinogen); Human 6, which eliminates approximately 85 - 90% of total protein (albumin, IgG, IgA, transferrin, haptoglobin, and antitrypsin); Human Albumin/IgG, which eliminates approximately 69% of total protein (albumin and IgG); and Human Albumin, which eliminates approximately 50-55% of total protein (albumin). The ProteoPrep® 20 Plasma Immunodepletion Kit from Sigma-Aldrich is intended to specifically remove the 20 most abundant proteins from human plasma or serum, which is about remove 97-98% of the total protein mass in plasma or serum (Sigma-Aldrich, St. Louis, MO). According to the manufacturer, the ProteoPrep® 20 removes: albumin, IgG, transferrin, fibrinogen, IgA, $\alpha_2$-Macroglobulin, IgM, $\alpha_1$-Antitrypsin, complement C3, haptoglobulin, apolipoprotein A1, A3 and B; $\alpha_1$-Acid Glycoprotein, ceruloplasmin, complement C4, C1q; IgD, prealbumin, and plasminogen. Sigma-Aldrich also manufactures ProteoPrep® columns to remove albumin (HSA) and immunoglobulins (IgG). The ProteomeLab IgY-12 High Capacity Proteome Partitioning kits from Beckman Coulter (Fullerton, CA) are specifically designed to remove twelve highly abundant proteins (Albumin, IgG, Transferrin, Fibrinogen, IgA, $\alpha2$-macroglobulin, IgM, $\alpha1$-Antitrypsin, Haptoglobin, Orosomucoid, Apolipoprotein A-I, Apolipoprotein A-II) from the human biological fluids such as serum and plasma. Generally, such systems rely on immunodepletion to remove the target proteins, e.g., using small ligands and/or full antibodies. The PureProteome™ Human Albumin/Immunoglobulin Depletion Kit from Millipore (EMD Millipore Corporation, Billerica, MA, USA) is a magnetic bead based kit that enables high depletion efficiency (typically >99%) of Albumin and all Immunoglobulins (i.e., IgG, IgA, IgM, IgE and IgD) from human serum or plasma samples. The ProteoExtract® Albumin/IgG Removal Kit, also from Millipore, is designed to deplete >80% of albumin and IgG from body fluid samples. Other similar protein depletion products include without limitation the following: Aurum™ Affi-Gels Blue mini kit (Bio-Rad, Hercules, CA, USA); Vivapure® anti-HSA/IgG kit (Sartorius Stedim Biotech, Goettingen, Germany), Qproteome albumin/IgG depletion kit (Qiagen, Hilden, Germany); Seppro® MIXED12-LC20 column (GenWay Biotech, San Diego, CA, USA); Abundant Serum Protein Depletion Kit (Norgen Biotek Corp., Ontario, Canada); GBC Human Albumin/IgG/Transferrin 3 in 1 Depletion Column/Kit (Good Biotech Corp., Taiwan). These systems and similar systems can be used to remove abundant proteins from a biological sample, thereby improving the ability to detect low abundance circulating biomarkers such as proteins and vesicles.

Thromboplastin is a plasma protein aiding blood coagulation through conversion of prothrombin to thrombin. Thrombin in turn acts as a serine protease that converts soluble fibrinogen into insoluble strands of fibrin, as well as catalyzing many other coagulation-related reactions. Thus, thromboplastin is a protein that can be used to facilitate precipitation of fibrinogen/fibrin (blood clotting factors) out of plasma. In addition to or as an alternative to immunoaffinity protein removal, a blood sample can be treated with thromboplastin to deplete fibrinogen/fibrin. Thromboplastin removal can be performed in addition to or as an alternative to immunoaffinity protein removal as described above using methods known in the art. Precipitation of other proteins and/or other sample particulate can also improve detection of circulating biomarkers such as vesicles in a sample. For example, ammonium sulfate treatment as known in the art can be used to precipitate immunoglobulins and other highly abundant proteins.

In an aspect, the disclosure provides a method of detecting a presence or level of one or more circulating biomarker such as a microvesicle in a biological sample, comprising: (a) providing a biological sample comprising or suspected to comprise the one or more circulating biomarker; (b) selectively depleting one or more abundant protein from the biological sample provided in step (a); (c) performing affinity selection of the one or more circulating biomarker from the sample depleted in step (b), thereby detecting the presence or level of one or more circulating biomarker. The biological sample may comprise a bodily fluid, e.g., peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, umbilical cord blood, or a derivative of any thereof. In some embodiments, the biological sample comprises peripheral blood, serum or plasma. Illustrative protocols and results from selectively depleting one or more abundant protein from blood plasma prior to vesicle detection can be found in **Example 40** of International Patent Publication No. WO/2014/082083, filed November 26, 2013,.

An abundant protein may comprise a protein in the sample that is present in the sample at a high enough concentration to potentially interfere with downstream processing or analysis. Typically, an abundant protein is not the target of any further analysis of the sample. The abundant protein may constitute at least $10^{-5}$, $10^{-4}$, $10^{-1}$, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, 96, 97, 98 or at least 99% of the total protein mass in the sample. In some embodiments, the abundant protein is present at less than $10^{-5}$% of the total protein mass in the sample, e.g., in the case of a rare target of interest. As described herein, in the case of blood or a derivative thereof, the one or more abundant protein may comprise one or more of albumin, IgG, transferrin, fibrinogen, fibrin, IgA, $\alpha$2-Marcroglobulin, IgM, $\alpha$1-Antitrypsin, complement C3, haptoglobulin, apolipoprotein A1, A3 and B; $\alpha$1-Acid Glycoprotein, ceruloplasmin, complement C4, C1q, IgD, prealbumin (transthyretin), plasminogen, a derivative of any thereof, and a combination thereof. The one or more abundant protein in blood or a blood derivative may also comprise one or more of Albumin, Immunoglobulins, Fibrinogen, Prealbumin, Alpha 1 antitrypsin, Alpha 1 acid glycoprotein, Alpha 1 fetoprotein, Haptoglobin, Alpha 2 macroglobulin, Ceruloplasmin, Transferrin, complement proteins C3 and C4, Beta 2 microglobulin, Beta lipoprotein, Gamma globulin proteins, C-reactive protein (CRP), Lipoproteins (chylomicrons, VLDL, LDL, HDL), other globulins (types alpha, beta and gamma), Prothrombin, Mannose-binding lectin (MBL), a derivative of any thereof, and a combination thereof.

In some embodiments, selectively depleting the one or more abundant protein comprises contacting the biological sample with thromboplastin to initiate precipitation of fibrin. The one or more abundant protein may also be depleted by immunoaffinity, precipitation, or a combination thereof. For example, the sample can be treated with thromboplastin to precipitate fibrin, and then the sample may be passed through a column to remove HSA, IgG, and other abundant proteins as desired.

"Selectively depleting" the one or more abundant protein comprises depleting the abundant protein from the sample at a higher percentage than depletion another entity in the sample, such as another protein or microvesicle, including a target of interest for downstream processing or analysis. Selectively depleting the one or more abundant protein may comprise depleting the abundant protein at a 1.1-fold, 1.2-fold, 1.3-fold, 1.4-fold, 1.5-fold, 1.6-fold, 1.7-fold, 1.8-fold, 1.9-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, 20-fold, 25-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1000-fold, $10^4$-fold, $10^5$-fold, $10^6$-fold, $10^7$-fold, $10^8$-fold, $10^9$-fold, $10^{10}$-fold, $10^{11}$-fold, $10^{12}$-fold, $10^{13}$-fold, $10^{14}$-fold, $10^{15}$-fold, $10^{16}$-fold, $10^{17}$-fold, $10^{18}$-fold, $10^{19}$-fold, $10^{20}$-fold, or higher rate than another entity in the sample, such as another protein or microvesicle, including a target of interest for downstream processing or analysis. In an embodiment, there is little to no observable depletion of the target of interest as compared to the depletion of the abundant protein. In some embodiments, selectively depleting the one or more abundant protein from the biological sample comprises depleting at least 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% or 100% of the one or more abundant protein.

Removal of highly abundant proteins and other non-desired entities can further be facilitated with a non-stringent size exclusion step. For example, the sample can be processed using a high molecular weight cutoff size exclusion step to preferentially enrich high molecular weight vesicles apart from lower molecular weight proteins and other entities. In some embodiments, a sample is processed with a column (e.g., a gel filtration column) or filter having a molecular weight cutoff (MWCO) of 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, or greater than 10000 kiloDaltons (kDa). In an embodiment, a 700 kDa filtration column is used. In such a step, the vesicles will be retained or flow more slowly than the column or filter than the lower molecular weight entities. Such columns and filters are known in the art.

Isolation or enrichment of a vesicle from a biological sample can also be enhanced by use of sonication (for example, by applying ultrasound), detergents, other membrane-activating agents, or any combination thereof. For example, ultrasonic energy can be applied to a potential tumor site, and without being bound by theory, release of vesicles from a tissue can be increased, allowing an enriched population of vesicles that can be analyzed or assessed from a biological sample using one or more methods disclosed herein.

With methods of detecting circulating biomarkers as described here, e.g., antibody affinity isolation, the consistency of the results can be optimized as desired using various concentration or isolation procedures. Such steps can include agitation such as shaking or vortexing, different isolation techniques such as polymer based isolation, e.g., with PEG, and concentration to different levels during filtration or other steps. It will be understood by those in the art that such treatments can be applied at various stages of testing the vesicle containing sample. In one embodiment, the sample itself, e.g., a bodily fluid such as plasma or serum, is vortexed. In some embodiments, the sample is vortexed after one or more sample treatment step, e.g., vesicle isolation, has occurred. Agitation can occur at some or all appropriate sample treatment steps as desired. Additives can be introduced at the various steps to improve the process, e.g., to control aggregation or degradation of the biomarkers of interest.

The results can also be optimized as desireable by treating the sample with various agents. Such agents include additives to control aggregation and/or additives to adjust pH or ionic strength. Additives that control aggregation include blocking agents such as bovine serum albumin (BSA), milk or StabilGuard® (a BSA-free blocking agent; Product code SG02, Surmodics, Eden Prairie, MN), chaotropic agents such as guanidium hydro chloride, and detergents or surfactants. Useful ionic detergents include sodium dodecyl sulfate (SDS, sodium lauryl sulfate (SLS)), sodium laureth sulfate (SLS, sodium lauryl ether sulfate (SLES)), ammonium lauryl sulfate (ALS), cetrimonium bromide, cetrimonium chloride, cetrimonium stearate, and the like. Useful non-ionic (zwitterionic) detergents include polyoxyethylene glycols, polysorbate 20 (also known as Tween 20), other polysorbates (e.g., 40, 60, 65, 80, etc), Triton-X (e.g., X100, X114), 3-[(3-cholamidopropyl)dimethylammonio]-1-propanesulfonate (CHAPS), CHAPSO, deoxycholic acid, sodium deoxycholate, NP-40, glycosides, octyl-thio-glucosides, maltosides, and the like. In some embodiments, Pluronic F-68, a surfactant shown to reduce platelet aggregation, is used to treat samples containing vesicles during isolation and/or detection. F68 can be used from a 0.1% to 10% concentration, e.g., a 1%, 2.5% or 5% concentration. The pH and/or ionic strength of the solution can be adjusted with various acids, bases, buffers or salts, including without limitation sodium chloride (NaCl), phosphate-buffered saline (PBS), tris-buffered saline (TBS), sodium phosphate, potassium chloride, potassium phosphate, sodium citrate and saline-sodium citrate (SSC) buffer. In some embodiments, NaCl is added at a concentration of 0.1% to 10%, e.g., 1%, 2.5% or 5% final concentration. In some embodiments, Tween 20 is added to 0.005 to 2% concentration, e.g., 0.05%, 0.25% or 0.5 % final concentration. Blocking agents for use may comprise inert proteins, e.g., milk proteins, non-fat dry milk protein, albumin, BSA, casein, or serum such as newborn calf serum (NBCS), goat serum, rabbit serum or salmon serum. The proteins can be added at a 0.1% to 10% concentration, e.g., 1%, 2%, 3%, 3.5%, 4%, 5%, 6%, 7%, 8%, 9% or 10% concentration. In some embodiments, BSA is added to 0.1% to 10% concentration, e.g., 1%, 2%, 3%, 3.5%, 4%, 5%, 6%, 7%, 8%, 9% or 10% concentration. In an embodiment, the sample is treated according to the methodology presented in U.S. Patent Application 11/632946, filed July 13, 2005. Commercially available blockers may be used, such as SuperBlock, StartingBlock, Protein-Free from Pierce (a division of Thermo Fisher Scientific, Rockford, IL). In some embodiments, SSC/detergent (e.g., 20X SSC with 0.5% Tween 20 or 0.1% Triton-X 100) is added to 0.1% to 10% concentration, e.g., at 1.0% or 5.0% concentration.

The methods of detecting vesicles and other circulating biomarkers can be optimized as desired with various combinations of protocols and treatments as described herein. A detection protocol can be optimized by various combinations of agitation, isolation methods, and additives. In some embodiments, the patient sample is vortexed before and after isolation steps, and the sample is treated with blocking agents including BSA and/or F68. Such treatments may reduce the formation of large aggregates or protein or other biological debris and thus provide a more consistent detection reading.

### Filtration and Ultrafiltration

A vesicle can be isolated from a biological sample by filtering a biological sample from a subject through a filtration module and collecting from the filtration module a retentate comprising the vesicle, thereby isolating the vesicle from the biological sample. The method can comprise filtering a biological sample from a subject through a filtration module comprising a filter (also referred to herein as a selection membrane); and collecting from the filtration module a retentate comprising the vesicle, thereby isolating the vesicle from the biological sample. For example, in one embodiment, the filter retains molecules greater than about 100 kiloDaltons. In such cases, microvesicles are generally found within the retentate of the filtration process whereas smaller entities such as proteins, protein complexes, nucleic acids, etc, pass through into the filtrate.

The method can be used when determining a biosignature of one or more microvesicle. The method can also further comprise contacting the retentate from the filtration to a plurality of substrates, wherein each substrate is coupled to one or more capture agents, and each subset of the plurality of substrates comprises a different capture agent or combination of capture agents than another subset of the plurality of substrates.

Also disclosed herein is a method of determining a biosignature of a vesicle in a sample comprising: filtering a biological sample from a subject with a disorder through a filtration module, collecting from the filtration module a retentate comprising one or more vesicles, and determining a biosignature of the one or more vesicles. In one embodiment, the filtration module comprises a filter that retains molecules greater than about 100 or 150 kiloDaltons.

The method disclosed herein can further comprise characterizing a phenotype in a subject by filtering a biological sample from a subject through a filtration module, collecting from the filtration module a retentate comprising one or more vesicles; detecting a biosignature of the one or more vesicles; and characterizing a phenotype in the subject based on the biosignature, wherein characterizing is with at least 70% sensitivity. Characterizing may comprise determining an amount of one or more vesicle having the biosignature. Furthermore, the characterizing can be from about 80% to 100% sensitivity.

Also disclosed herein is a method for multiplex analysis of a plurality of vesicles. The method may comprise filtering a biological sample from a subject through a filtration module; collecting from the filtration module a retentate comprising the plurality of vesicles, applying the plurality of vesicles to a plurality of capture agents, wherein the plurality of capture agents is coupled to a plurality of substrates, and each subset of the plurality of substrates is differentially labeled from another subset of the plurality of substrates; capturing at least a subset of the plurality of vesicles; and determining a biosignature for at least a subset of the captured vesicles. Each substrate may be coupled to one or more capture agents, and each subset of the plurality of substrates may comprise a different capture agent or combination of capture agents as compared to another subset of the plurality of substrates. In some embodiments, at least a subset of the plurality of substrates is intrinsically labeled, such as comprising one or more labels. The substrate can be a particle or bead, or any combination thereof. In some embodiments, the filter retains molecules greater than 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, or greater than 10000 kiloDaltons (kDa). In one embodiment, the filtration module comprises a filter that retains molecules greater than about 100 or 150 kiloDaltons. In one embodiment, the filtration module comprises a filter that retains molecules greater than about 9, 20, 100 or 150 kiloDaltons. In still another embodiment, the filtration module comprises a filter that retains molecules greater than about 7000 kDa.

The method for multiplex analysis of a plurality of vesicles may comprise filtering a biological sample from a subject through a filtration module, wherein the filtration module comprises a filter that retains molecules greater than about 100 kiloDaltons; collecting from the filtration module a retentate comprising the plurality of vesicles; applying the plurality of vesicles to a plurality of capture agents, wherein the plurality of capture agents is coupled to a microarray; capturing at least a subset of the plurality of vesicles on the microarray; and determining a biosignature for at least a subset of the captured vesicles. In some embodiments, the filter retains molecules greater than 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, or greater than 10000 kiloDaltons (kDa). In one embodiment, the filtration module comprises a filter that retains molecules greater than about 100 or 150 kiloDaltons. In one embodiment, the filtration module comprises a filter that retains molecules greater than about 9, 20, 100 or 150 kiloDaltons. In still another embodiment, the filtration module comprises a filter that retains molecules greater than about 7000 kDa.

The biological sample can be clarified prior to isolation by filtration. Clarification comprises selective removal of cellular debris and other undesirable materials. For example, cellular debris and other components that may interfere with detection of the circulating biomarkers can be removed. The clarification can be by low-speed centrifugation, such as at about 5,000x g, 4,000x g, 3,000x g, 2,000x g, 1,000x g, or less. The supernatant, or clarified biological sample, containing the vesicle can then be collected and filtered to isolate the vesicle from the clarified biological sample. In some embodiments, the biological sample is not clarified prior to isolation of a vesicle by filtration.

In some embodiments, isolation of a vesicle from a sample does not use high-speed centrifugation, such as ultracentrifugation. For example, isolation may not require the use of centrifugal speeds, such as about 100,000x g or more. In some embodiments, isolation of a vesicle from a sample uses speeds of less than 50,000 x g, 40,000 x g, 30,000 x g, 20,000 x g, 15,000 x g, 12,000 x g, or 10,000 x g.

Any number of applicable filter configurations can be used to filter a sample of interest. In some embodiments, the filtration module used to isolate the circulating biomarkers from the biological sample is a fiber-based filtration cartridge. For example, the fiber can be a hollow polymeric fiber, such as a polypropylene hollow fiber. A biological sample can be introduced into the filtration module by pumping the sample fluid, such as a biological fluid as disclosed herein, into the module with a pump device, such as a peristaltic pump. The pump flow rate can vary, such as at about 0.25, 0.5, 1, 1.5, 2, 2.5, 3, 3.5, 4, 4.5, 5, 6, 7, 8, 9, or 10 mL/minute. The flow rate can be adjusted given the configuration, e.g., size and throughput, of the filtration module.

The filtration module can be a membrane filtration module. For example, the membrane filtration module can comprise a filter disc membrane, such as a hydrophilic polyvinylidene difluoride (PVDF) filter disc membrane housed in a stirred cell apparatus (e.g., comprising a magnetic stirrer). In some embodiments, the sample moves through the filter as a result of a pressure gradient established on either side of the filter membrane.

The filter can comprise a material having low hydrophobic absorptivity and/or high hydrophilic properties. For example, the filter can have an average pore size for vesicle retention and permeation of most proteins as well as a surface that is hydrophilic, thereby limiting protein adsorption. For example, the filter can comprise a material selected from the group consisting of polypropylene, PVDF, polyethylene, polyfluoroethylene, cellulose, secondary cellulose acetate, polyvinylalcohol, and ethylenevinyl alcohol (EVAL®, Kuraray Co., Okayama, Japan). Additional materials that can be used in a filter include, but are not limited to, polysulfone and polyethersulfone.

The filtration module can have a filter that retains molecules greater than about 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, or 900 kiloDaltons (kDa), such as a filter that has a MWCO (molecular weight cut off) of about 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, or 900 kDa, respectively. In embodiments, the filtration module has a MWCO of 1000 kDa, 1500 kDa, 2000 kDa, 2500 kDa, 3000 kDa, 3500 kDa, 4000 kDa, 4500 kDa, 5000 kDa, 5500 kDa, 6000 kDa, 6500 kDa, 7000 kDa, 7500 kDa, 8000 kDa, 8500 kDa, 9000 kDa, 9500 kDa, 10000 kDa, or greater than 10000 kDa. Ultrafiltration membranes with a range of MWCO of 9 kDa, 20 kDa and/or 150 kDa can be used. In some embodiments, the filter within the filtration module has an average pore diameter of about 0.01 $\mu$m to about 0.15 $\mu$m, and in some embodiments from about 0.05 $\mu$m to about 0.12 $\mu$m. In some embodiments, the filter has an average pore diameter of about 0.06 $\mu$m, 0.07 $\mu$m, 0.08 $\mu$m, 0.09 $\mu$m, 0.1 $\mu$m, 0.11 $\mu$m or 0.2 $\mu$m.

The filtration module can be a commerically available column, such as a column typically used for concentrating proteins or for isolating proteins (e.g., ultrafiltration). Examples include, but are not limited to, columns from Millpore (Billerica, MA), such as Amicon® centrifugal filters, or from Pierce® (Rockford, IL), such as Pierce Concentrator filter devices. Useful columns from Pierce include disposable ultrafiltration centrifugal devices with a MWCO of 9 kDa, 20 kDa and/or 150 kDa. These concentrators consist of a high-performance regenerated cellulose membrane welded to a conical device. The filters can be as described in U.S. Patents 6,269,957 or 6,357,601.

The retentate comprising the isolated vesicle can be collected from the filtration module. The retentate can be collected by flushing the retentate from the filter. Selection of a filter composition having hydrophilic surface properties, thereby limiting protein adsorption, can be used, without being bound by theory, for easier collection of the retentate and minimize use of harsh or time-consuming collection techniques.

The collected retentate can then be used subsequent analysis, such as assessing a biosignature of one or more vesicles in the retentate, as further described herein. The analysis can be directly performed on the collected retentate. Alternatively, the collected retentate can be further concentrated or purified, prior to analysis of one or more vesicles. For example, the retentate can be further concentrated or vesicles further isolated from the retentate using size exclusion chromatography, density gradient centrifugation, differential centrifugation, immunoabsorbent capture, affinity purification, microfluidic separation, or combinations thereof, such as described herein. In some embodiments, the retentate can undergo another step of filtration. Alternatively, prior to isolation of a vesicle using a filter, the vesicle is concentrated or isolated using techniques including without limitation size exclusion chromatography, density gradient centrifugation, differential centrifugation, immunoabsorbent capture, affinity purification, microfluidic separation, or combinations thereof.

Combinations of filters can be used for concentrating and isolating biomarkers. For example, the biological sample may first be filtered through a filter having a porosity or pore size of between about 0.01 $\mu$m to about 10 $\mu$m, e.g., 0.01 $\mu$m to about 2 $\mu$m or about 0.05 $\mu$m to about 1.5 $\mu$m, and then the sample is filtered. For example, prior to filtering a biological sample through a filtration module with a filter that retains molecules greater than about 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, or greater than 10000 kiloDaltons (kDa), such as a filter that has a MWCO (molecular weight cut off) of about 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, or greater than 10000 kDa, respectively, the biological sample may first be filtered through a filter having a porosity or pore size of between about 0.01 $\mu$m to about 10 $\mu$m, e.g., 0.01 $\mu$m to about 2 $\mu$m or about 0.05 $\mu$m to about 1.5 $\mu$m. In some embodiments, the filter has a pore size of about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0, 3.0, 4.0, 5.0, 6.0, 7.0, 8.0, 9.0 or 10.0 $\mu$m. The filter may be a syringe filter. Thus, in one embodiment, the method comprises filtering the biological sample through a filter, such as a syringe filter, wherein the syringe filter has a porosity of greater than about 1 $\mu$m, prior to filtering the sample through a filtration module comprising a filter that retains molecules greater than about 100 or 150 kiloDaltons. In an embodiment, the filter is 1.2 $\mu$M filter and the filtration is followed by passage of the sample through a 7 ml or 20 ml concentrator column with a 150 kDa cutoff. Multiple concentrator columns may be used, e.g., in series. For example, a 7000 MWCO filtration unit can be used before a 150 MWCO unit.

The filtration module can be a component of a microfluidic device. Microfluidic devices, which may also be referred to as "lab-on-a-chip" systems, biomedical micro-electro-mechanical systems (bioMEMs), or multicomponent integrated systems, can be used for isolating, and analyzing, vesicles. Such systems miniaturize and compartmentalize processes that allow for binding of vesicles, detection of biomarkers, and other processes, such as further described herein.

The filtration module and assessment can be as described in Grant, R., et al., A filtration-based protocol to isolate human Plasma Membrane-derived Vesicles and exosomes from blood plasma, J Immunol Methods (2011) 371:143-51 (Epub 2011 Jun 30).

A microfluidic device can also be used for isolation of a vesicle by comprising a filtration module. For example, a microfluidic device can use one more channels for isolating a vesicle from a biological sample based on size from a biological sample. A biological sample can be introduced into one or more microfluidic channels, which selectively allows the passage of vesicles. The microfluidic device can further comprise binding agents, or more than one filtration module to select vesicles based on a property of the vesicles, for example, size, shape, deformability, biomarker profile, or biosignature.

The retentate from a filtration step can be further processed before assessment of microvesicles or other biomarkers therein. In an embodiment, the retentate is diluted prior to biomarker assessment, e.g., with an appropriate diluent such as a biologically compatible buffer. In some cases, the retentate is serially diluted. In an aspect, the disclosure provides a method for detecting a microvesicle population from a biological sample comprising: a) concentrating the biological sample using a selection membrane having a pore size of from 0.01 $\mu$m to about 10 $\mu$m, or a molecular weight cut off (MWCO) from about 1 kDa to 10000 kDa; b) diluting a retentate from the concentration step into one or more aliquots; and c) contacting each of the one or more aliquots of retentate with one or more binding agent specific to a molecule of at least one microvesicle in the microvesicle population. In a related aspect, the disclosure provides a method for detecting a microvesicle population from a biological sample comprising: a) concentrating the biological sample using a selection membrane having a pore size of from 0.01 $\mu$m to about 10 $\mu$m, or a molecular weight cut off (MWCO) from about 1 kDa to 10000 kDa; and b) contacting one or more aliquots of the retentate from the concentrating step with one or more binding agent specific to a molecule of at least one microvesicle in the microvesicle population.

The selection membrane can be sized to retain the desired biomarkers in the retentate or to allow the desired biomarkers to pass through the filter into the filtrate. The filter membrane can be chosen to have a certain pore size or MWCO value. The selection membrane can have a pore size of about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0, 3.0, 4.0, 5.0, 6.0, 7.0, 8.0, 9.0 or 10.0 $\mu$m. The selection membrane can also have a MWCO of about 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, 900, 1000, 2000, 3000, 4000, 5000, 6000, 7000, 8000, 9000 or 10000 kDa.

The retentate can be separated and/or diluted into any number of desired aliquots. For example, multiple aliquots without any dilution or the same dilution can be used to determine reproducibility. In another example, multiple aliquots at different dilutions can be used to construct a concentration curve. In an embodiment, the retentate is separated and/or diluted into at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 150, 200, 250, 300, 350 or 400 aliquots. The aliquots can be at a same dilution or at different dilutions.

A dilution factor is the ratio of the final volume of a mixture (the mixture of the diluents and aliquot) divided by the initial volume of the aliquot. The retentate can be diluted into one or more aliquots at a dilution factor of about 0, 1, 2, 3, 4, 5, 6, 7, 8, 9, 10, 15, 20, 25, 30, 40, 50, 60, 70, 80, 90, 100, 110, 120, 130, 140, 150, 160, 170, 180, 190, 200, 250, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, 20000, 30000, 40000, 50000, 60000, 70000, 80000, 90000 and/or 100000. For example, the retentate can be diluted into one or more aliquot at a dilution factor of about 500.

To estimate a concentration or form a curve, the retentate can be diluted into multiple aliquots. In an embodiment of the method, the retentate is diluted into one or more aliquots at a dilution factor of about 100, 250, 500, 1000, 10000 and 100000. As desired, the method can further comprise detecting an amount of microvesicles in each aliquot of retentate, e.g., that formed a complex with the one or more binding agent. The curve can be used to determine a linear range of the amount of microvesicles in each aliquot detected versus dilution factor. A concentration of the detected microvesicles for the biological sample can be determined using the amount of microvesicles determined in one or more aliquot within the linear range. The concentration can be compared to a reference concentration, e.g., in order to characterize a phenotype as described herein.

The disclosure also provides a related method comprising filtering a biological sample from a subject through a filtration module and collecting a filtrate comprising the vesicle, thereby isolating the vesicle from the biological sample. In such cases cells and other large entities can be retained in the retentate while microvesicles pass through into the filtrate. It will be appreciated that strategies to retain and filter microvesicles can be used in concert. For example, a sample can be filtered with a selection membrane that allows microvesicles to pass through, thereby isolating the microvesicles from large particles (cells, complexes, etc). The filtrate comprising the microvesicle can then be filtered using a selection membrane that retains microvesicles, thereby isolating the microvesicles from smaller particles (proteins, nucleic acids, etc). The isolated microvesicles can be further assessed according to the methods of the invention, e.g., to characterize a phenotype.

*Precipitation*

Vesicles can be isolated using a polymeric precipitation method. In one embodiment, the sample containing the vesicles is contacted with a formulation of polyethylene glycol (PEG). The polymeric formulation is incubated with the vesicle containing sample then precipitated by centrifugation. The PEG can bind to the vesicles and can be treated to specifically capture vesicles by addition of a capture moiety, e.g., a pegylated-binding protein such as an antibody. One of skill will appreciate that other polymers in addition to PEG can be used, e.g., PEG derivatives including methoxypolyethylene glycols, poly (ethylene oxide), and various polymers of formula $HO\text{-}CH_2\text{-}(CH_2\text{-}O\text{-}CH_2\text{-})n\text{-}CH_2\text{-}OH$ having different molecular weights. The efficiency of isolation may depend on various factors including the length of the polymer chains and concentration of polymer used. In preferred embodiments, PEG4000 or PEG8000 may be used at a concentration of 1%, 2%, 3%, 4%, 5%, 6%, 7%, 8%, 9%, or 10%, e.g., 4% or 8%.

In some embodiments of the invention, the vesicles are concentrated from a sample using the polymer precipitation method and the isolated vesicles are further separated using another approach. The second step can be used to identify a subpopulation of vesicles, e.g., that display certain biomarkers. The second separation step can comprise size exclusion, a binding agent, an antibody capture step, microbeads, as described herein.

In an embodiment, vesicles are isolated according to the ExoQuick™ and ExoQuick-TC™ kits from System Biosciences, Mountain View, CA USA. These kits use a polymer-based precipitation method to pellet vesicles. Similarly, the vesicles can be isolated using the Total Exosome Isolation (from Serum) or Total Exosome Isolation (from Cell Culture Media) kits from Invitrogen / Life Technologies (Carlsbad, CA USA). The Total Exosome Isolation reagent forces less-soluble components such as vesicles out of solution, allowing them to be collected by a short, low-speed centrifugation. The reagent is added to the biological sample, and the solution is incubated overnight at 2 °C to 8 °C. The precipitated vesicles are recovered by standard centrifugation.

## Binding Agents

Binding agents (also referred to as binding reagents) include agents that are capable of binding a target biomarker. A binding agent can be specific for the target biomarker, meaning the agent is capable of binding a target biomarker. The target can be any useful biomarker disclosed herein, such as a biomarker on the vesicle surface. In some aspects, the target is a single molecule, such as a single protein, so that the binding agent is specific to the single protein. In other aspects, the target can be a group of molecules, such as a family or proteins having a similar epitope or moiety, so that the binding agent is specific to the family or group of proteins. The group of molecules can also be a class of molecules, such as protein, DNA or RNA. The binding agent can be a capture agent used to capture a vesicle by binding a component or biomarker of a vesicle. In some aspects, a capture agent comprises an antibody or fragment thereof, or an aptamer, that binds to an antigen on a vesicle. The capture agent can be optionally coupled to a substrate and used to isolate a vesicle, as further described herein.

A binding agent is an agent that binds to a circulating biomarker, such as a vesicle or a component of a vesicle. The binding agent can be used as a capture agent and/or a detection agent. A capture agent can bind and capture a circulating biomarker, such as by binding a component or biomarker of a vesicle. For example, the capture agent can be a capture antibody or capture antigen that binds to an antigen on a vesicle. A detection agent can bind to a circulating biomarker thereby facilitating detection of the biomarker. For example, a capture agent comprising an antibody or aptamer that is sequestered to a substrate can be used to capture a vesicle in a sample, and a detection agent comprising an antibody or aptamer that carries a label can be used to detect the captured vesicle via detection of the detection agent's label. A vesicle may be assessed using capture and detection agents that recognize the same vesicle biomarkers. For example, a vesicle population can be captured using a tetraspanin such as by using an anti-CD9 antibody bound to a substrate, and the captured vesicles can be detected using a fluorescently labeled anti-CD9 antibody to label the captured vesicles. A vesicle may be assessed using capture and detection agents that recognize different vesicle biomarkers. For example, a vesicle population can be captured using a cell-specific marker such as by using an anti-PCSA antibody bound to a substrate, and the captured vesicles can be detected using a fluorescently labeled anti-CD9 antibody to label the captured vesicles. Similarly, the vesicle population can be captured using a general vesicle marker such as by using an anti-CD9 antibody bound to a substate, and the captured vesicles can be detected using a fluorescently labeled antibody to a cell-specific or disease specific marker to label the captured vesicles.

The biomarkers recognized by the binding agent are sometimes referred to herein as an antigen. Unless otherwise specified, antigen as used herein is meant to encompass any entity that is capable of being bound by a binding agent, regardless of the type of binding agent or the immunogenicity of the biomarker. The antigen further encompasses a functional fragment thereof. For example, an antigen can encompass a protein biomarker capable of being bound by a binding agent, including a fragment of the protein that is capable of being bound by a binding agent.

A vesicle may be captured using a capture agent that binds to a biomarker on a vesicle. The capture agent can be coupled to a substrate and used to isolate a vesicle, as further described herein. A capture agent may be used for affinity capture or isolation of a vesicle present in a substance or sample.

A binding agent can be used after a vesicle is concentrated or isolated from a biological sample. For example, a vesicle can first be isolated from a biological sample before a vesicle with a specific biosignature is isolated or detected.

The vesicle with a specific biosignature can be isolated or detected using a binding agent for the biomarker. A vesicle with the specific biomarker can be isolated or detected from a heterogeneous population of vesicles. Alternatively, a binding agent may be used on a biological sample comprising vesicles without a prior isolation or concentration step. For example, a binding agent is used to isolate or detect a vesicle with a specific biosignature directly from a biological sample.

A binding agent can be a nucleic acid, protein, or other molecule that can bind to a component of a vesicle. The binding agent can comprise DNA, RNA, monoclonal antibodies, polyclonal antibodies, Fabs, Fab', single chain antibodies, synthetic antibodies, aptamers (DNA/RNA), peptoids, zDNA, peptide nucleic acids (PNAs), locked nucleic acids (LNAs), lectins, synthetic or naturally occurring chemical compounds (including but not limited to drugs, labeling reagents), dendrimers, or a combination thereof. For example, the binding agent can be a capture antibody. In aspects of the disclosure, the binding agent comprises a membrane protein labeling agent. See, e.g., the membrane protein labeling agents disclosed in Alroy et al., US. Patent Publication US 2005/0158708. In an embodiment, vesicles are isolated or captured as described herein, and one or more membrane protein labeling agent is used to detect the vesicles.

In some instances, a single binding agent can be employed to isolate or detect a vesicle. In other instances, a combination of different binding agents may be employed to isolate or detect a vesicle. For example, at least 2, 3, 4, 5, 6, 7, 8, 9, 10, 11, 12, 13, 14, 15, 16, 17, 18, 19, 20, 25, 50, 75 or 100 different binding agents may be used to isolate or detect a vesicle from a biological sample. Furthermore, the one or more different binding agents for a vesicle can form a biosignature of a vesicle, as further described below.

Different binding agents can also be used for multiplexing. For example, isolation or detection of more than one population of vesicles can be performed by isolating or detecting each vesicle population with a different binding agent. Different binding agents can be bound to different particles, wherein the different particles are labeled. An array comprising different binding agents can be used for multiplex analysis, wherein the different binding agents are differentially labeled or can be ascertained based on the location of the binding agent on the array. Multiplexing can be accomplished up to the resolution capability of the labels or detection method, such as described below. The binding agents can be used to detect the vesicles, such as for detecting cell-of-origin specific vesicles. A binding agent or multiple binding agents can themselves form a binding agent profile that provides a biosignature for a vesicle. One or more binding agents can be selected from Fig. 2 of International Patent Publication No. WO/2011/127219, entitled "Circulating Biomarkers for Disease" and filed April 6, 2011. For example, if a vesicle population is detected or isolated using two, three, four or more binding agents in a differential detection or isolation of a vesicle from a heterogeneous population of vesicles, the particular binding agent profile for the vesicle population provides a biosignature for the particular vesicle population. The vesicle can be detected using any number of binding agents in a multiplex fashion. Thus, the binding agent can also be used to form a biosignature for a vesicle. The biosignature can be used to characterize a phenotype.

The binding agent can be a lectin. Lectins are proteins that bind selectively to polysaccharides and glycoproteins and are widely distributed in plants and animals. For example, lectins such as those derived from Galanthus nivalis in the form of Galanthus nivalis agglutinin ("GNA"), Narcissus pseudonarcissus in the form of Narcissus pseudonarcissus agglutinin ("NPA") and the blue green algae Nostoc ellipsosporum called "cyanovirin" (Boyd et al. Antimicrob Agents Chemother 41 (7): 1521 1530, 1997; Hammar et al. Ann N Y Acad Sci 724: 166 169, 1994; Kaku et al. Arch Biochem Biophys 279(2): 298 304, 1990) can be used to isolate a vesicle. These lectins can bind to glycoproteins having a high mannose content (Chervenak et al. Biochemistry 34(16): 5685 5695, 1995). High mannose glycoprotein refers to glycoproteins having mannose-mannose linkages in the form of $\alpha$-1$\rightarrow$3 or $\alpha$-1$\rightarrow$6 mannose-mannose linkages.

The binding agent can be an agent that binds one or more lectins. Lectin capture can be applied to the isolation of the biomarker cathepsin D since it is a glycosylated protein capable of binding the lectins Galanthus nivalis agglutinin (GNA) and concanavalin A (ConA).

Methods and devices for using lectins to capture vesicles are described in International Patent Publications WO/2011/066589, entitled "METHODS AND SYSTEMS FOR ISOLATING, STORING, AND ANALYZING VESICLES" and filed November 30, 2010; WO/2010/065765, entitled "AFFINITY CAPTURE OF CIRCULATING BIOMARKERS" and filed December 3, 2009; WO/2010/141862, entitled "METHODS AND MATERIALS FOR ISOLATING EXOSOMES" and filed June 4, 2010; and WO/2007/103572, entitled "EXTRACORPOREAL REMOVAL OF MICRO VESICULAR PARTICLES" and filed March 9, 2007.

The binding agent can be an antibody. For example, a vesicle may be isolated using one or more antibodies specific for one or more antigens present on the vesicle. For example, a vesicle can have CD63 on its surface, and an antibody, or capture antibody, for CD63 can be used to isolate the vesicle. Alternatively, a vesicle derived from a tumor cell can express EpCam, the vesicle can be isolated using an antibody for EpCam and CD63. Other antibodies for isolating vesicles can include an antibody, or capture antibody, to CD9, PSCA, TNFR, CD63, B7H3, MFG-E8, EpCam, Rab, CD81, STEAP, PCSA, PSMA, or 5T4. Other antibodies for isolating vesicles can include an antibody, or capture antibody, to DR3, STEAP, epha2, TMEM211, MFG-E8, Tissue Factor (TF), unc93A, A33, CD24, NGAL, EpCam, MUC17, TROP2, or TETS.

In some aspects, the capture agent is an antibody to CD9, CD63, CD81, PSMA, PCSA, B7H3, EpCam, PSCA, ICAM, STEAP, or EGFR. The capture agent can also be used to identify a biomarker of a vesicle. For example, a capture agent such as an antibody to CD9 would identify CD9 as a biomarker of the vesicle. In some aspects, a plurality of capture agents can be used, such as in multiplex analysis. The plurality of captures agents can comprise binding agents to one or more of: CD9, CD63, CD81, PSMA, PCSA, B7H3, EpCam, PSCA, ICAM, STEAP, and EGFR. In some aspects, the plurality of capture agents comprise binding agents to CD9, CD63, CD81, PSMA, PCSA, B7H3, MFG-E8, and/or EpCam. In yet other aspects, the plurality of capture agents comprises binding agents to CD9, CD63, CD81, PSMA, PCSA, B7H3, EpCam, PSCA, ICAM, STEAP, and/or EGFR. The plurality of capture agents comprises binding agents to TMEM211, MFG-E8, Tissue Factor (TF), and/or CD24.

The antibodies referenced herein can be immunoglobulin molecules or immunologically active portions of immunoglobulin molecules, i.e., molecules that contain an antigen binding site that specifically binds an antigen and synthetic antibodies. The immunoglobulin molecules can be of any class (e.g., IgG, IgE, IgM, IgD or IgA) or subclass of immunoglobulin molecule. Antibodies include, but are not limited to, polyclonal, monoclonal, bispecific, synthetic, humanized and chimeric antibodies, single chain antibodies, Fab fragments and F(ab')$_2$ fragments, Fv or Fv' portions, fragments produced by a Fab expression library, anti-idiotypic (anti-Id) antibodies, or epitope-binding fragments of any of the above. An antibody, or generally any molecule, "binds specifically" to an antigen (or other molecule) if the antibody binds preferentially to the antigen, and, e.g., has less than about 30%, 20%, 10%, 5% or 1% cross-reactivity with another molecule.

The binding agent can also be a polypeptide or peptide. Polypeptide is used in its broadest sense and may include a sequence of subunit amino acids, amino acid analogs, or peptidomimetics. The subunits may be linked by peptide bonds. The polypeptides may be naturally occurring, processed forms of naturally occurring polypeptides (such as by enzymatic digestion), chemically synthesized or recombinantly expressed. The polypeptides for use in the methods may be chemically synthesized using standard techniques. The polypeptides may comprise D-amino acids (which are resistant to L- amino acid-specific proteases), a combination of D- and L-amino acids, $\beta$ amino acids, or various other designer or non-naturally occurring amino acids (e.g., $\beta$-methyl amino acids, C$\alpha$-methyl amino acids, and N$\alpha$-methyl amino acids, etc.) to convey special properties. Synthetic amino acids may include ornithine for lysine, and norleucine for leucine or isoleucine. In addition, the polypeptides can have peptidomimetic bonds, such as ester bonds, to prepare polypeptides with novel properties. For example, a polypeptide may be generated that incorporates a reduced peptide bond, i.e., R$_1$-CH$_2$-NH-R$_2$, where R$_1$ and R$_2$ are amino acid residues or sequences. A reduced peptide bond may be introduced as a dipeptide subunit. Such a polypeptide would be resistant to protease activity, and would possess an extended half- live in vivo. Polypeptides can also include peptoids (N-substituted glycines), in which the side chains are appended to nitrogen atoms along the molecule's backbone, rather than to the $\alpha$-carbons, as in amino acids. Polypeptides and peptides are intended to be used interchangeably throughout this application, i.e. where the term peptide is used, it may also include polypeptides and where the term polypeptides is used, it may also include peptides. The term "protein" is also intended to be used interchangeably throughout this application with the terms "polypeptides" and "peptides" unless otherwise specified.

A vesicle may be isolated, captured or detected using a binding agent. The binding agent can be an agent that binds a vesicle "housekeeping protein," or general vesicle biomarker. The biomarker can be CD63, CD9, CD81, CD82, CD37, CD53, Rab-5b, Annexin V, MFG-E8 or other commonly observed vesicle markers include those listed in **Table 3.** Furthermore, any of the markers disclosed herein or in **Table 3** can be selected in identifying a candidate biosignature for a disease or condition, where the one or more selected biomarkers have a direct or indirect role or function in mechanisms involved in the disease or condition.

The binding agent can also be an agent that binds to a vesicle derived from a specific cell type, such as a tumor cell (e.g. binding agent for Tissue factor, EpCam, B7H3, RAGE or CD24) or a specific cell-of-origin. The binding agent used to isolate or detect a vesicle can be a binding agent for an antigen selected from **Fig. 1** of International Patent Publication No. WO/2011/127219, entitled "Circulating Biomarkers for Disease" and filed April 6, 2011. The binding agent for a vesicle can also be selected from those listed in **Fig. 2** of International Patent Publication No. WO/2011/127219. The binding agent can be for an antigen such as a tetraspanin, MFG-E8, Annexin V, 5T4, B7H3, caveolin, CD63, CD9, E-Cadherin, Tissue factor, MFG-E8, TMEM211, CD24, PSCA, PCSA, PSMA, Rab-5B, STEAP, TNFR1, CD81, EpCam, CD59, CD81, ICAM, EGFR, or CD66. A binding agent for a platelet can be a glycoprotein such as GpIa-IIa, GpIIb-IIIa, GpIIIb, GpIb, or GpIX. A binding agent can be for an antigen comprisine one or more of CD9, Erb2, Erb4, CD81, Erb3, MUC16, CD63, DLL4, HLA-Drpe, B7H3, IFNAR, 5T4, PCSA, MICB, PSMA, MFG-E8, Muc1, PSA, Muc2, Unc93a, VEGFR2, EpCAM, VEGF A, TMPRSS2, RAGE, PSCA, CD40, Muc17, IL-17-RA, and CD80. For example, the binding agent can be one or more of CD9, CD63, CD81, B7H3, PCSA, MFG-E8, MUC2, EpCam, RAGE and Muc17. One or more binding agents, such as one or more binding agents for two or more of the antigens, can be used for isolating or detecting a vesicle. The binding agent used can be selected based on the desire of isolating or detecting a vesicle derived from a particular cell type or cell-of-origin specific vesicle. The binding agent can be to one or more vesicle marker in **Table 4.**

A binding agent can also be linked directly or indirectly to a solid surface or substrate. A solid surface or substrate can be any physically separable solid to which a binding agent can be directly or indirectly attached including, but not limited to, surfaces provided by microarrays and wells, particles such as beads, columns, optical fibers, wipes, glass and modified or functionalized glass, quartz, mica, diazotized membranes (paper or nylon), polyformaldehyde, cellulose, cellulose acetate, paper, ceramics, metals, metalloids, semiconductive materials, quantum dots, coated beads or particles, other chromatographic materials, magnetic particles; plastics (including acrylics, polystyrene, copolymers of styrene or other materials, polypropylene, polyethylene, polybutylene, polyurethanes, polytetrafluoroethylene (PTFE, Teflon®), etc.), polysaccharides, nylon or nitrocellulose, resins, silica or silica-based materials including silicon and modified silicon, carbon, metals, inorganic glasses, plastics, ceramics, conducting polymers (including polymers such as polypyrole and polyindole); micro or nanostructured surfaces such as nucleic acid tiling arrays, nanotube, nanowire, or nanoparticulate decorated surfaces; or porous surfaces or gels such as methacrylates, acrylamides, sugar polymers, cellulose, silicates, or other fibrous or stranded polymers. In addition, as is known the art, the substrate may be coated using passive or chemically-derivatized coatings with any number of materials, including polymers, such as dextrans, acrylamides, gelatins or agarose. Such coatings can facilitate the use of the array with a biological sample.

For example, an antibody used to isolate a vesicle can be bound to a solid substrate such as a well, such as commercially available plates (e.g. from Nunc, Milan Italy). Each well can be coated with the antibody. The antibody used to isolate a vesicle may be bound to a solid substrate such as an array. The array can have a predetermined spatial arrangement of molecule interactions, binding islands, biomolecules, zones, domains or spatial arrangements of binding islands or binding agents deposited within discrete boundaries. Further, the term array may be used herein to refer to multiple arrays arranged on a surface, such as would be the case where a surface bore multiple copies of an array. Such surfaces bearing multiple arrays may also be referred to as multiple arrays or repeating arrays.

Arrays typically contain addressable moieties that can detect the presense of an entity, e.g., a vesicle in the sample via a binding event. An array may be referred to as a microarray. Arrays or microarrays include without limitation DNA microarrays, such as cDNA microarrays, oligonucleotide microarrays and SNP microarrays, microRNA arrays, protein microarrays, antibody microarrays, tissue microarrays, cellular microarrays (also called transfection microarrays), chemical compound microarrays, and carbohydrate arrays (glycoarrays). DNA arrays typically comprise addressable nucleotide sequences that can bind to sequences present in a sample. MicroRNA arrays, e.g., the MMChips array from the University of Louisville or commercial systems from Agilent, can be used to detect microRNAs. Protein microarrays can be used to identify protein-protein interactions, including without limitation identifying substrates of protein kinases, transcription factor protein-activation, or to identify the targets of biologically active small molecules. Protein arrays may comprise an array of different protein molecules, commonly antibodies, or nucleotide sequences that bind to proteins of interest. In a non-limiting example, a protein array can be used to detect vesicles having certain proteins on their surface. Antibody arrays comprise antibodies spotted onto the protein chip that are used as capture molecules to detect proteins or other biological materials from a sample, e.g., from cell or tissue lysate solutions. For example, antibody arrays can be used to detect vesicle-associated biomarkers from bodily fluids, e.g., serum or urine. Tissue microarrays comprise separate tissue cores assembled in array fashion to allow multiplex histological analysis. Cellular microarrays, also called transfection microarrays, comprise various capture agents, such as antibodies, proteins, or lipids, which can interact with cells to facilitate their capture on addressable locations. Cellular arrays can also be used to capture vesicles due to the similarity between a vesicle and cellular membrane. Chemical compound microarrays comprise arrays of chemical compounds and can be used to detect protein or other biological materials that bind the compounds. Carbohydrate arrays (glycoarrays) comprise arrays of carbohydrates and can detect, e.g., protein that bind sugar moieties. One of skill will appreciate that similar technologies or improvements can be used according to the methods of the invention.

A binding agent can also be bound to particles such as beads or microspheres. For example, an antibody specific for a component of a vesicle can be bound to a particle, and the antibody-bound particle is used to isolate a vesicle from a biological sample. The microspheres may be magnetic or fluorescently labeled. In addition, a binding agent for isolating vesicles can be a solid substrate itself. For example, latex beads, such as aldehyde/sulfate beads (Interfacial Dynamics, Portland, OR) can be used.

A binding agent bound to a magnetic bead can also be used to isolate a vesicle. For example, a biological sample such as serum from a patient can be collected for colon cancer screening. The sample can be incubated with anti-CCSA-3 (Colon Cancer-Specific Antigen) coupled to magnetic microbeads. A low-density microcolumn can be placed in the magnetic field of a MACS Separator and the column is then washed with a buffer solution such as Tris-buffered saline. The magnetic immune complexes can then be applied to the column and unbound, non-specific material can be discarded. The CCSA-3 selected vesicle can be recovered by removing the column from the separator and placing it on a collection tube. A buffer can be added to the column and the magnetically labeled vesicle can be released by applying the plunger supplied with the column. The isolated vesicle can be diluted in IgG elution buffer and the complex can then be centrifuged to separate the microbeads from the vesicle. The pelleted isolated cell-of-origin specific vesicle can be resuspended in buffer such as phosphate-buffered saline and quantitated. Alternatively, due to the strong adhesion force between the antibody captured cell-of-origin specific vesicle and the magnetic microbeads, a proteolytic enzyme such as trypsin can be used for the release of captured vesicles without the need for centrifugation. The proteolytic enzyme can be incubated with the antibody captured cell-of-origin specific vesicles for at least a time sufficient to release the vesicles.

A binding agent, such as an antibody, for isolating vesicles is preferably contacted with the biological sample comprising the vesicles of interest for at least a time sufficient for the binding agent to bind to a component of the vesicle. For example, an antibody may be contacted with a biological sample for various intervals ranging from seconds days, including but not limited to, about 10 minutes, 30 minutes, 1 hour, 3 hours, 5 hours, 7 hours, 10 hours, 15 hours, 1 day, 3 days, 7 days or 10 days.

A binding agent, such as an antibody specific to an antigen listed in **Fig. 1** of International Patent Publication No. WO/2011/127219, entitled "Circulating Biomarkers for Disease" and filed April 6, 2011, or a binding agent listed in **Fig. 2** of International Patent Publication No. WO/2011/127219, can be labeled to facilitate detection. Appropriate labels include without limitation a magnetic label, a fluorescent moiety, an enzyme, a chemiluminescent probe, a metal particle, a non-metal colloidal particle, a polymeric dye particle, a pigment molecule, a pigment particle, an electrochemically active species, semiconductor nanocrystal or other nanoparticles including quantum dots or gold particles, fluorophores, quantum dots, or radioactive labels. Various protein, radioactive, fluorescent, enzymatic, and other labels are described further above.

A binding agent can be directly or indirectly labeled, e.g., the label is attached to the antibody through biotin-streptavidin. Alternatively, an antibody is not labeled, but is later contacted with a second antibody that is labeled after the first antibody is bound to an antigen of interest.

Depending on the method of isolation or detection used, the binding agent may be linked to a solid surface or substrate, such as arrays, particles, wells and other substrates described above. Methods for direct chemical coupling of antibodies, to the cell surface are known in the art, and may include, for example, coupling using glutaraldehyde or maleimide activated antibodies. Methods for chemical coupling using multiple step procedures include biotinylation, coupling of trinitrophenol (TNP) or digoxigenin using for example succinimide esters of these compounds. Biotinylation can be accomplished by, for example, the use of D-biotinyl-N-hydroxysuccinimide. Succinimide groups react effectively with amino groups at pH values above 7, and preferentially between about pH 8.0 and about pH 8.5. Biotinylation can be accomplished by, for example, treating the cells with dithiothreitol followed by the addition of biotin maleimide.

### Particle-based Assays

As an alternative to planar arrays, assays using particles or microspheres, such as bead based assays, are capable of use with a binding agent. For example, antibodies or aptamers are easily conjugated with commercially available beads. *See, e.g.,* Fan et al., Illumina universal bead arrays. Methods Enzymol. 2006 410:57-73; Srinivas et al. Anal. Chem. 2011 Oct. 21, Aptamerfunctionalized Microgel Particles for Protein Detection*; See also,* review article on aptamers as therapeutic and diagnostic agents, Brody and Gold, Rev. Mol. Biotech. 2000, 74:5-13.

Multiparametric assays or other high throughput detection assays using bead coatings with cognate ligands and reporter molecules with specific activities consistent with high sensitivity automation can be used. In a bead based assay system, a binding agent for a biomarker or vesicle, such as a capture agent (e.g. capture antibody), can be immobilized on an addressable microsphere. Each binding agent for each individual binding assay can be coupled to a distinct type of microsphere (i.e., microbead) and the assay reaction takes place on the surface of the microsphere, such as depicted in **FIG. 2B.** A binding agent for a vesicle can be a capture antibody or aptamer coupled to a bead. Dyed microspheres with discrete fluorescence intensities are loaded separately with their appropriate binding agent or capture probes. The different bead sets carrying different binding agents can be pooled as desired to generate custom bead arrays. Bead arrays are then incubated with the sample in a single reaction vessel to perform the assay.

Various particle/bead substrates and systems useful for the methods of the invention are described further above.

### Flow Cytometry

Flow cytometry, which is described in further detail above, may be used to assess a microvesicle population in a biological sample. If desired, the microvesicle population can be sorted from other particles (e.g., cell debris, protein aggregates, etc) in a sample by labeling the vesicles using one or more general vesicle marker. The general vesicle marker can be a marker in **Table 3.** Commonly used vesicle markers include tetraspanins such as CD9, CD63 and/or CD81. Vesicles comprising one or more tetraspanin are sometimes refered to as "Tet+" herein to indicate that the vesicles are tetraspanin-positive. The sorted microvesicles can be further assessed using methodology described herein. E.g., surface antigens on the sorted microvesicles can be detected using flow or other methods. Payload within the sorted microvesicles may be assessed. As an illustrative example, a population of microvesicles is contacted with a labeled binding agent to a surface antigen of interest, the contacted microvesicles are sorted using flow cytometry, and payload with the microvesicles is assessed. The payload may be polypeptides, nucleic acids (e.g., mRNA or microRNA) or other biological entities as desired. Such assessment is used to characterize a phenotype as described herein, e.g., to diagnose, prognose or theranose a cancer.

Flow sorting may be used to distinguish microvesicle populations from other biological complexes. In a non-limiting example, Ago2+/Tet+ and Ago2+/Tet- particles are detected using flow methodology to separate Ago2+ vesicles from vesicle-free Ago2+ complexes, respectively.

*Multiplexing*

Multiplex experiments comprise experiments that can simultaneously measure multiple analytes in a single assay. Vesicles and associated biomarkers can be assessed in a multiplex fashion. Different binding agents can be used for multiplexing different circulating biomarkers, e.g., microRNA, protein, or vesicle populations. Different biomarkers, e.g., different vesicle populations, can be isolated or detected using different binding agents. Each population in a biological sample can be labeled with a different signaling label, such as a fluorophore, quantum dot, or radioactive label, such as described above. The label can be directly conjugated to a binding agent or indirectly used to detect a binding agent that binds a vesicle. The number of populations detected in a multiplexing assay is dependent on the resolution capability of the labels and the summation of signals, as more than two differentially labeled vesicle populations that bind two or more affinity elements can produce summed signals.

Multiplexing of at least 2, 3, 4, 5, 6, 7, 8, 9, 10, 11, 12, 13, 14, 15, 16, 17, 18, 19, 20, 25, 50, 75 or 100 different circulating biomarkers may be performed. For example, one population of vesicles specific to a cell-of-origin can be assayed along with a second population of vesicles specific to a different cell-of-origin, where each population is labeled with a different label. Alternatively, a population of vesicles with a particular biomarker or biosignature can be assayed along with a second population of vesicles with a different biomarker or biosignature. In some cases, hundreds or thousands of vesicles are assessed in a single assay.

Multiplex analysis may be performed by applying a plurality of vesicles comprising more than one population of vesicles to a plurality of substrates, such as beads. Each bead is coupled to one or more capture agents. The plurality of beads is divided into subsets, where beads with the same capture agent or combination of capture agents form a subset of beads, such that each subset of beads has a different capture agent or combination of capture agents than another subset of beads. The beads can then be used to capture vesicles that comprise a component that binds to the capture agent. The different subsets can be used to capture different populations of vesicles. The captured vesicles can then be analyzed by detecting one or more biomarkers.

Flow cytometry can be used in combination with a particle-based or bead based assay. Multiparametric immunoassays or other high throughput detection assays using bead coatings with cognate ligands and reporter molecules with specific activities consistent with high sensitivity automation can be used. For example, beads in each subset can be differentially labeled from another subset. In a particle based assay system, a binding agent or capture agent for a vesicle, such as a capture antibody, can be immobilized on addressable beads or microspheres. Each binding agent for each individual binding assay (such as an immunoassay when the binding agent is an antibody) can be coupled to a distinct type of microsphere (i.e., microbead) and the binding assay reaction takes place on the surface of the microspheres. Microspheres can be distinguished by different labels, for example, a microsphere with a specific capture agent would have a different signaling label as compared to another microsphere with a different capture agent. For example, microspheres can be dyed with discrete fluorescence intensities such that the fluorescence intensity of a microsphere with a specific binding agent is different than that of another microsphere with a different binding agent. Biomarkers bound by different capture agents can be differentially detected using different labels.

A microsphere can be labeled or dyed with at least 2 different labels or dyes. The microsphere may be labeled with at least 3, 4, 5, 6, 7, 8, 9, or 10 different labels. Different microspheres in a plurality of microspheres can have more than one label or dye, wherein various subsets of the microspheres have various ratios and combinations of the labels or dyes permitting detection of different microspheres with different binding agents. For example, the various ratios and combinations of labels and dyes can permit different fluorescent intensities. Alternatively, the various ratios and combinations maybe used to generate different detection patters to identify the binding agent. The microspheres can be labeled or dyed externally or may have intrinsic fluorescence or signaling labels. Beads can be loaded separately with their appropriate binding agents and thus, different vesicle populations can be isolated based on the different binding agents on the differentially labeled microspheres to which the different binding agents are coupled.

Multiplex analysis can be performed using a planar substrate, wherein the substrate comprises a plurality of capture agents. The plurality of capture agents can capture one or more populations of vesicles, and one or more biomarkers of the captured vesicles detected. The planar substrate can be a microarray or other substrate as further described herein.

*Binding Agents*

A vesicle may be isolated or detected using a binding agent for a novel component of a vesicle, such as an antibody for a novel antigen specific to a vesicle of interest. Novel antigens that are specific to a vesicle of interest may be isolated or identified using different test compounds of known composition bound to a substrate, such as an array or a plurality of particles, which can allow a large amount of chemical/structural space to be adequately sampled using only a small fraction of the space. The novel antigen identified can also serve as a biomarker for the vesicle. For example, a novel antigen identified for a cell-of-origin specific vesicle can be a useful biomarker.

The term "agent" or "reagent" as used in respect to contacting a sample can mean any entity designed to bind, hybridize, associate with or otherwise detect or facilitate detection of a target molecule, including target polypeptides, peptides, nucleic acid molecules, leptins, lipids, or any other biological entity that can be detected as described herein or as known in the art. Examples of such agents/reagents are well known in the art, and include but are not limited to universal or specific nucleic acid primers, nucleic acid probes, antibodies, aptamers, peptoid, peptide nucleic acid, locked nucleic acid, lectin, dendrimer, chemical compound, or other entities described herein or known in the art.

A binding agent can be identified by screening either a homogeneous or heterogeneous vesicle population against test compounds. Since the composition of each test compound on the substrate surface is known, this constitutes a screen for affinity elements. For example, a test compound array comprises test compounds at specific locations on the substrate addressable locations, and can be used to identify one or more binding agents for a vesicle. The test compounds can all be unrelated or related based on minor variations of a core sequence or structure. The different test compounds may include variants of a given test compound (such as polypeptide isoforms), test compounds that are structurally or compositionally unrelated, or a combination thereof.

A test compound can be a peptoid, polysaccharide, organic compound, inorganic compound, polymer, lipids, nucleic acid, polypeptide, antibody, protein, polysaccharide, or other compound. The test compound can be natural or synthetic. The test compound can comprise or consist of linear or branched heteropolymeric compounds based on any of a number of linkages or combinations of linkages (e.g., amide, ester, ether, thiol, radical additions, metal coordination, etc.), dendritic structures, circular structures, cavity structures or other structures with multiple nearby sites of attachment that serve as scaffolds upon which specific additions are made. Thes test compound can be spotted on a substrate or synthesized in situ, using standard methods in the art. In addition, the test compound can be spotted or synthesized in situ in combinations in order to detect useful interactions, such as cooperative binding.

The test compound can be a polypeptide with known amino acid sequence, thus, detection of a test compound binding with a vesicle can lead to identification of a polypeptide of known amino sequence that can be used as a binding agent. For example, a homogenous population of vesicles can be applied to a spotted array on a slide containing between a few and 1,000,000 test polypeptides having a length of variable amino acids. The polypeptides can be attached to the surface through the C-terminus. The sequence of the polypeptides can be generated randomly from 19 amino acids, excluding cysteine. The binding reaction can include a non-specific competitor, such as excess bacterial proteins labeled with another dye such that the specificity ratio for each polypeptide binding target can be determined. The polypeptides with the highest specificity and binding can be selected. The identity of the polypeptide on each spot is known, and thus can be readily identified. Once the novel antigens specific to the homogeneous vesicle population, such as a cell-of-origin specific vesicle is identified, such cell-of-origin specific vesicles may subsequently be isolated using such antigens in methods described hereafter.

An array can also be used for identifying an antibody as a binding agent for a vesicle. Test antibodies can be attached to an array and screened against a heterogeneous population of vesicles to identify antibodies that can be used to isolate or identify a vesicle. A homogeneous population of vesicles such as cell-of-origin specific vesicles can also be screened with an antibody array. Other than identifying antibodies to isolate or detect a homogeneous population of vesicles, one or more protein biomarkers specific to the homogenous population can be identified. Commercially available platforms with test antibodies pre-selected or custom selection of test antibodies attached to the array can be used. For example, an antibody array from Full Moon Biosystems can be screened using prostate cancer cell derived vesicles identifying antibodies to Bcl-XL, ERCC1, Keratin 15, CD81/TAPA-1, CD9, Epithelial Specific Antigen (ESA), and Mast Cell Chymase as binding agents, and the proteins identified can be used as biomarkers for the vesicles. The biomarker can be present or absent, underexpressed or overexpressed, mutated, or modified in or on a vesicle and used in characterizing a condition.

An antibody or synthetic antibody to be used as a binding agent can also be identified through a peptide array. Another method is the use of synthetic antibody generation through antibody phage display. M13 bacteriophage libraries of antibodies (e.g. Fabs) are displayed on the surfaces of phage particles as fusions to a coat protein. Each phage particle displays a unique antibody and also encapsulates a vector that contains the encoding DNA. Highly diverse libraries can be constructed and represented as phage pools, which can be used in antibody selection for binding to immobilized antigens. Antigen-binding phages are retained by the immobilized antigen, and the nonbinding phages are removed by washing. The retained phage pool can be amplified by infection of an *Escherichia coli* host and the amplified pool can be used for additional rounds of selection to eventually obtain a population that is dominated by antigen-binding clones. At this stage, individual phase clones can be isolated and subjected to DNA sequencing to decode the sequences of the displayed antibodies. Through the use of phase display and other methods known in the art, high affinity designer antibodies for vesicles can be generated.

Bead-based assays can also be used to identify novel binding agents to isolate or detect a vesicle. A test antibody or peptide can be conjugated to a particle. For example, a bead can be conjugated to an antibody or peptide and used to detect and quantify the proteins expressed on the surface of a population of vesicles in order to discover and specifically select for novel antibodies that can target vesicles from specific tissue or tumor types. Any molecule of organic origin can be successfully conjugated to a polystyrene bead through use of a commercially available kit according to manufacturer's instructions. Each bead set can be colored a certain detectable wavelength and each can be linked to a known antibody or peptide which can be used to specifically measure which beads are linked to exosomal proteins matching the epitope of previously conjugated antibodies or peptides. The beads can be dyed with discrete fluorescence intensities such that each bead with a different intensity has a different binding agent as described above.

For example, a purified vesicle preparation can be diluted in assay buffer to an appropriate concentration according to empirically determined dynamic range of assay. A sufficient volume of coupled beads can be prepared and approximately 1 $\mu$l of the antibody-coupled beads can be aliquoted into a well and adjusted to a final volume of approximately 50 $\mu$l. Once the antibody-conjugated beads have been added to a vacuum compatible plate, the beads can be washed to ensure proper binding conditions. An appropriate volume of vesicle preparation can then be added to each well being tested and the mixture incubated, such as for 15-18 hours. A sufficient volume of detection antibodies using detection antibody diluent solution can be prepared and incubated with the mixture for 1 hour or more. The beads can then be washed before the addition of detection antibody (biotin expressing) mixture composed of streptavidin phycoereythin. The beads can then be washed and vacuum aspirated several times before analysis on a suspension array system using software provided with an instrument. The identity of antigens that can be used to selectively extract the vesicles can then be elucidated from the analysis.

Assays using imaging systems can be used to detect and quantify proteins expressed on the surface of a vesicle in order to discover and specifically select for and enrich vesicles from specific tissue, cell or tumor types. Antibodies, peptides or cells conjugated to multiple well multiplex carbon coated plates can be used. Simultaneous measurement of many analytes in a well can be achieved through the use of capture antibodies arrayed on the patterned carbon working surface. Analytes can then be detected with antibodies labeled with reagents in electrode wells with an enhanced electro-chemiluminescent plate. Any molecule of organic origin can be successfully conjugated to the carbon coated plate. Proteins expressed on the surface of vesicles can be identified from this assay and can be used as targets to specifically select for and enrich vesicles from specific tissue or tumor types.

The binding agent can also be an aptamer to a specific target. The term "specific" as used herein in regards to a binding agent can mean that an agent has a greater affinity for its target than other targets, typically with a much great affinity, but does not require that the binding agent is absolutely specific for its target.

### *Microfluidics*

The methods for isolating or identifying vesicles can be used in combination with microfluidic devices. The methods of isolating or detecting a vesicle, such as disclosed herein, can be performed using a microfluidic device. Microfluidic devices, which may also be referred to as "lab-on-a-chip" systems, biomedical micro-electro-mechanical systems (bioMEMs), or multicomponent integrated systems, can be used for isolating and analyzing a vesicle. Such systems miniaturize and compartmentalize processes that allow for binding of vesicles, detection of biosignatures, and other processes.

A microfluidic device can also be used for isolation of a vesicle through size differential or affinity selection. For example, a microfluidic device can use one more channels for isolating a vesicle from a biological sample based on size or by using one or more binding agents for isolating a vesicle from a biological sample. A biological sample can be introduced into one or more microfluidic channels, which selectively allows the passage of a vesicle. The selection can be based on a property of the vesicle, such as the size, shape, deformability, or biosignature of the vesicle.

A heterogeneous population of vesicles can be introduced into a microfluidic device, and one or more different homogeneous populations of vesicles can be obtained. For example, different channels can have different size selections or binding agents to select for different vesicle populations. Thus, a microfluidic device can isolate a plurality of vesicles wherein at least a subset of the plurality of vesicles comprises a different biosignature from another subset of the plurality of vesicles. For example, the microfluidic device can isolate at least 2, 3, 4, 5, 6, 7, 8, 9, 10, 15, 20, 25, 30, 40, 50, 60, 70, 80, 90, or 100 different subsets of vesicles, wherein each subset of vesicles comprises a different biosignature.

The microfluidic device can comprise one or more channels that permit further enrichment or selection of a vesicle. A population of vesicles that has been enriched after passage through a first channel can be introduced into a second channel, which allows the passage of the desired vesicle or vesicle population to be further enriched, such as through one or more binding agents present in the second channel.

Array-based assays and bead-based assays can be used with microfluidic device. For example, the binding agent can be coupled to beads and the binding reaction between the beads and vesicle can be performed in a microfluidic device. Multiplexing can also be performed using a microfluidic device. Different compartments can comprise different binding agents for different populations of vesicles, where each population is of a different cell-of-origin specific vesicle population. Each population may have a different biosignature. The hybridization reaction between the microsphere and vesicle can be performed in a microfluidic device and the reaction mixture can be delivered to a detection device. The detection device, such as a dual or multiple laser detection system can be part of the microfluidic system and can use a laser to identify each bead or microsphere by its color-coding, and another laser can detect the hybridization signal associated with each bead.

Various microfluidic devices and methods are described above.

### *Combined Isolation Methodology*

One of skill will appreciate that various methods of sample treatment and isolating and concentrating circulating biomarkers such as vesicles can be combined as desired. For example, a biological sample can be treated to prevent aggregation, remove undesired particulate and/or deplete highly abundant proteins. The steps used can be chosen to optimize downstream analysis steps. Next, biomarkers such as vesicles can be isolated, e.g., by chromotography, centrifugation, density gradient, filtration, precipitation, or affinity techniques. Any number of the later steps can be combined, e.g., a sample could be subjected to one or more of chromotography, centrifugation, density gradient, filtration and precipitation in order to isolate or concentrate all or most microvesicles. In a subsequent step, affinity techniques, e.g., using binding agents to one or more target of interest, can be used to isolate or identify microvesicles carrying desired biomarker profiles. Microfluidic systems can be employed to perform some or all of these steps.

An exemplary yet non-limiting isolation scheme for isolating and analysis of microvesicles includes the following: Plasma or serum collection -> highly abundant protein removal -> ultrafiltration - > nanomembrane concentration -> flow cytometry or particle-based assay.

Using the methods disclosed herein or known in the art, circulating biomarkers such as vesicles can be isolated or concentrated by at least about 2-fold, 3-fold, 1-fold, 2-fold, 3-fold, 4-fold, 5-fold, 6-fold, 7-fold, 8-fold, 9-fold, 10-fold, 12-fold, 15-fold, 20-fold, 25-fold, 30-fold, 35-fold, 40-fold, 45-fold, 50-fold, 55-fold, 60-fold, 65-fold, 70-fold, 75-fold, 80-fold, 90-fold, 95-fold, 100-fold, 110-fold, 120-fold, 125-fold, 130-fold, 140-fold, 150-fold, 160-fold, 170-fold, 175-fold, 180-fold, 190-fold, 200-fold, 225-fold, 250-fold, 275-fold, 300-fold, 325-fold, 350-fold, 375-fold, 400-fold, 425-fold, 450-fold, 475-fold, 500-fold, 525-fold, 550-fold, 575-fold, 600-fold, 625-fold, 650-fold, 675-fold, 700-fold, 725-fold, 750-fold, 775-fold, 800-fold, 825-fold, 850-fold, 875-fold, 900-fold, 925-fold, 950-fold, 975-fold, 1000-fold, 1500-fold, 2000-fold, 2500-fold, 3000-fold, 4000-fold, 5000-fold, 6000-fold, 7000-fold, 8000-fold, 9000-fold, or at least 10,000-fold. In some embodiments, the vesicles are be isolated or concentrated concentrated by at least 1 order of magnitude, 2 orders of magnitude, 3 orders of magnitude, 4 orders of magnitude, 5 orders of magnitude, 6 orders of magnitude, 7 orders of magnitude, 8 orders of magnitude, 9 orders of magnitude, or 10 orders of magnitude or more.

Once concentrated or isolated, the circulating biomarkers can be assessed, e.g., in order to characterize a phenotype as described herein. In some embodiments, the concentration or isolation steps themselves shed light on the phenotype of interest. For example, affinity methods can detect the presence or level of specific biomarkers of interest.

The various isolation and detection systems described herein can be used to isolate or detect circulating biomarkers such as vesicles that are informative for diagnosis, prognosis, disease stratification, theranosis, prediction of responder / non-responder status, disease monitoring, treatment monitoring and the like as related to such diseases and disorders. In a non-limiting example, a sample can be run through a chromatography column to isolate vesicles based on a property such as size of electrophoretic motility, and the vesicles can then be passed through a microfluidic device. Binding agents can be used before, during or after these steps.

The methods can be used with microvesicles isolated or detected using such methods as described herein. In various non-limiting examples: an aptamer can be used as a capture and/or detector agent for a biomarker such as a protein or microvesicle; a sample such as a bodily fluid can be contacted with an oligonucleotide probe library before microvesicles in the sample are isolated using one or more technique described herein (e.g., chromatography, centrifugation, flow cytometry, filtration, affinity isolation, polymer precipitation, etc); microvesicles in a sample are isolated using one or more technique described herein (e.g., chromatography, centrifugation, flow cytometry, filtration, affinity isolation, polymer precipitation, etc) before contacting the microvesicles with an aptamer or oligonucleotide probe library. Contaminants such as highly abundant proteins can be removed in whole or in part at any appropriate step in such processes. These and various other useful iterations of such techniques for assessment of microvesicles and other biomarkers are contemplated.

**Biomarkers**

As described herein, the methods and compositions can be used in assays to detect the presence or level of one or more biomarker of interest. The biomarker can be any useful biomarker disclosed herein or known to those of skill in the art. In an embodiment, the biomarker comprises a protein or polypeptide. As used herein, "protein," "polypeptide" and "peptide" are used interchangeably unless stated otherwise. The biomarker can be a nucleic acid, including DNA, RNA, and various subspecies of any thereof as disclosed herein or known in the art. The biomarker can comprise a lipid. The biomarker can comprise a carbohydrate. The biomarker can also be a complex, e.g., a complex comprising protein, nucleic acids, lipids and/or carbohydrates. In some embodiments, the biomarker comprises a microvesicle. In an aspect, the disclosure provides a method wherein a pool of aptamers is used to assess the presence and/or level of a population of microvesicles of interest without knowing the precise microvesicle antigen targeted by each member of the pool. See, e.g., **FIGs. 16B-C.** In other cases, biomarkers associated with microvesicles are assessed according to the methods. See, e.g., **FIGs. 2A-2F; FIG. 16A.**

A biosignature comprising more than one biomarker can comprise one type of biomarker or multiple types of biomarkers. As a non-limiting example, a biosignature can comprise multiple proteins, multiple nucleic acids, multiple lipids, multiple carbohydrates, multiple biomarker complexes, multiple microvesicles, or a combination of any thereof. For example, the biosignature may comprise one or more microvesicle, one or more protein, and one or more microRNA, wherein the one or more protein and/or one or more microRNA is optionally in association with the microvesicle as a surface antigen and/or payload, as appropriate.

In some embodiments, vesicles are detected using vesicle surface antigens. A commonly expressed vesicle surface antigen can be referred to as a "housekeeping protein," or general vesicle biomarker. The biomarker can be CD63, CD9, CD81, CD82, CD37, CD53, Rab-5b, Annexin V or MFG-E8. Tetraspanins, a family of membrane proteins with four transmembrane domains, can be used as general vesicle biomarkers. The tetraspanins include CD151, CD53, CD37, CD82, CD81, CD9 and CD63. There have been over 30 tetraspanins identified in mammals, including the TSPAN1 (TSP-1), TSPAN2 (TSP-2), TSPAN3 (TSP-3), TSPAN4 (TSP-4, NAG-2), TSPAN5 (TSP-5), TSPAN6 (TSP-6), TSPAN7 (CD231, TALLA-1, A15), TSPAN8 (CO-029), TSPAN9 (NET-5), TSPAN10 (Oculospanin), TSPAN11 (CD151-like), TSPAN12 (NET-2), TSPAN13 (NET-6), TSPAN14, TSPAN15 (NET-7), TSPAN16 (TM4-B), TSPAN17, TSPAN18, TSPAN19, TSPAN20 (UP1b, UPK1B), TSPAN21 (UP1a, UPK1A), TSPAN22 (RDS, PRPH2), TSPAN23 (ROM1), TSPAN24 (CD151), TSPAN25 (CD53), TSPAN26 (CD37), TSPAN27 (CD82), TSPAN28 (CD81), TSPAN29 (CD9), TSPAN30 (CD63), TSPAN31 (SAS), TSPAN32 (TSSC6), TSPAN33, and TSPAN34. Other commonly observed vesicle markers include those listed in **Table 3.** One or more of these proteins can be useful biomarkers for the characterizing a phenotype using the subject methods and compositions.

**Table 3: Proteins Observed in Vesicles from Multiple Cell Types**

| Class | Protein |
|---|---|
| **Antigen Presentation** | MHC class I, MHC class II, Integrins, Alpha 4 beta 1, Alpha M beta 2, Beta 2 |

**Table 3: Proteins Observed in Vesicles from Multiple Cell Types**

| Class | Protein |
|---|---|
| **Antigen Presentation** | MHC class I, MHC class II, Integrins, Alpha 4 beta 1, Alpha M beta 2, Beta 2 |
| **Immunoglobulin family** | ICAM1/CD54, P-selection |
| **Cell-surface peptidases** | Dipeptidylpeptidase IV/CD26, Aminopeptidase n/CD13 |
| **Tetraspanins** | CD151, CD53, CD37, CD82, CD81, CD9 and CD63 |
| **Heat-shock proteins** | Hsp70, Hsp84/90 |

(continued)

| Class | Protein |
|---|---|
| Cytoskeletal proteins | Actin, Actin-binding proteins, Tubulin |
| Membrane transport and fusion | Annexin I, Annexin II, Annexin IV, Annexin V, Annexin VI, RAB7/RAP1 B/RADGDI |
| Signal transduction | Gi2alpha/14-3-3, CBL/LCK |
| Abundant membrane proteins | CD63, GAPDH, CD9, CD81, ANXA2, ENO1, SDCBP, MSN, MFGE8, EZR, GK, ANXA1, LAMP2, DPP4, TSG101, HSPA1A, GDI2, CLTC, LAMP1, Cd86, ANPEP, TFRC, SLC3A2, RDX, RAP1B, RAB5C, RAB5B, MYH9, ICAM1, FN1, RAB11B, PIGR, LGALS3, ITGB1, EHD1, CLIC1, ATP1A1, ARF1, RAP1A, P4HB, MUC1, KRT10, HLA-A, FLOT1, CD59, C1orf58, BASP1, TACSTD1, STOM |
| Other Transmembrane Proteins | Cadherins: CDH1, CDH2, CDH12, CDH3, Deomoglein, DSG1, DSG2, DSG3, DSG4, Desmocollin, DSC1, DSC2, DSC3, Protocadherins, PCDH1, PCDH10, PCDH11x, PCDH11y, PCDH12, FAT, FAT2, FAT4, PCDH15, PCDH17, PCDH18, PCDH19; PCDH20; PCDH7, PCDH8, PCDH9, PCDHA1, PCDHA10, PCDHA11, PCDHA12, PCDHA13, PCDHA2, PCDHA3, PCDHA4, PCDHA5, PCDHA6, PCDHA7, PCDHA8, PCDHA9, PCDHAC1, PCDHAC2, PCDHB1, PCDHB10, PCDHB11, PCDHB12, PCDHB13, PCDHB14, PCDHB15, PCDHB16, PCDHB17, PCDHB18, PCDHB2, PCDHB3, PCDHB4, PCDHB5, PCDHB6, PCDHB7, PCDHB8, PCDHB9, PCDHGA1, PCDHGA10, PCDHGA11, PCDHGA12, PCDHGA2; PCDHGA3, PCDHGA4, PCDHGA5, PCDHGA6, PCDHGA7, PCDHGA8, PCDHGA9, PCDHGB1, PCDHGB2, PCDHGB3, PCDHGB4, PCDHGB5, PCDHGB6, PCDHGB7, PCDHGC3, PCDHGC4, PCDHGC5, CDH9 (cadherin 9, type 2 (T1-cadherin)), CDH10 (cadherin 10, type 2 (T2-cadherin)), CDH5 (VE-cadherin (vascular endothelial)), CDH6 (K-cadherin (kidney)), CDH7 (cadherin 7, type 2), CDH8 (cadherin 8, type 2), CDH11 (OB-cadherin (osteoblast)), CDH13 (T-cadherin - H-cadherin (heart)), CDH15 (M-cadherin (myotubule)), CDH16 (KSP-cadherin), CDH17 (LI cadherin (liver-intestine)), CDH18 (cadherin 18, type 2), CDH19 (cadherin 19, type 2), CDH20 (cadherin 20, type 2), CDH23 (cadherin 23, (neurosensory epithelium)), CDH10, CDH11, CDH13, CDH15, CDH16, CDH17, CDH18, CDH19, CDH20, CDH22, CDH23, CDH24, CDH26, CDH28, CDH4, CDH5, CDH6, CDH7, CDH8, CDH9, CELSR1, CELSR2, CELSR3, CLSTN1, CLSTN2, CLSTN3, DCHS1, DCHS2, LOC389118, PCLKC, RESDA1, RET |

Any of the types of biomarkers described herein can be used and/or assessed via the subject methods and compositions. Exemplary biomarkers include without limitation those in **Table 4.** The markers can be detected as protein or as mRNA, which can be circulating freely or in a complex with other biological molecules. As appropriate, the markers in **Table 4** can also be used for capture and/or detection of vesicles for characterizing phenotypes as disclosed herein. In some cases, multiple capture and/or detectors are used to enhance the characterization. See, e.g., **FIGs. 2D-E.** The markers can be detected as vesicle surface antigens and/or vesicle payload. The "Illustrative Class" indicates indications for which the markers are known markers. Those of skill will appreciate that the markers can also be used in alternate settings in certain instances. For example, a marker which can be used to characterize one type disease may also be used to characterize another disease as appropriate. Consider a non-limiting example of a tumor marker which can be used as a biomarker for tumors from various lineages. The biomarker references in **Table 4** are those commonly used in the art. Gene aliases and descriptions can be found using a variety of online databases, including GeneCards® (www.genecards.org), HUGO Gene Nomenclature (www.genenames.org), Entrez Gene (www.ncbi.nlm.nih.gov/entrez/query.fcgi?db=gene), UniProtKB/Swiss-Prot (www.uniprot.org), UniProtKB/TrEMBL (www.uniprot.org), OMIM (www.ncbi.nlm.nih.gov/entrez/query.fcgi?db=OMIM), GeneLoc (genecards.weizmann.ac.il/geneloc/), and Ensembl (www.ensembl.org). Generally, gene symbols and names below correspond to those approved by HUGO, and protein names are those recommended by UniProtKB/Swiss-Prot. Common alternatives are provided as well. In some cases, biomarkers are referred to by Ensembl reference numbers, which are of the form "ENSG" followed by a number, e.g., ENSG00000005893 which corresponds to LAMP2. In **Table 4,** solely for sake of brevity, "E." is sometimes used to represent "ENSG00000". For example, "E.005893 represents "ENSG00000005893." Where a protein name indicates a precursor, the mature protein is also implied. Throughout the application, gene and protein symbols may be used interchangeably and the meaning can be derived from context as necessary.

## Table 4: Illustrative Biomarkers

| Illustrative Class | Biomarkers |
|---|---|
| Drug associated targets and prognostic markers | ABCC1, ABCG2, ACE2, ADA, ADH1C, ADH4, AGT, AR, AREG, ASNS, BCL2, BCRP, BDCA1, beta III tubulin, BIRC5, B-RAF, BRCA1, BRCA2, CA2, caveolin, CD20, CD25, CD33, CD52, CDA, CDKN2A, CDKN1A, CDKN1B, CDK2, CDW52, CES2, CK 14, CK 17, CK 5/6, c-KIT, c-Met, c-Myc, COX-2, Cyclin D1, DCK, DHFR, DNMT1, DNMT3A, DNMT3B, E-Cadherin, ECGF1, EGFR, EML4-ALK fusion, EPHA2, Epiregulin, ER, ERBR2, ERCC1, ERCC3, EREG, ESR1, FLT1, folate receptor, FOLR1, FOLR2, FSHB, FSHPRH1, FSHR, FYN, GART, GNA11, GNAQ, GNRH1, GNRHR1, GSTP1, HCK, HDAC1, hENT-1, Her2/Neu, HGF, HIF1A, HIG1, HSP90, HSP90AA1, HSPCA, IGF-1R, IGFRBP, IGFRBP3, IGFRBP4, IGFRBP5, IL13RA1, IL2RA, KDR, Ki67, KIT, K-RAS, LCK, LTB, Lymphotoxin Beta Receptor, LYN, MET, MGMT, MLH1, MMR, MRP1, MS4A1, MSH2, MSH5, Myc, NFKB1, NFKB2, NFKBIA, NRAS, ODC1, OGFR, p16, p21, p27, p53, p95, PARP-1, PDGFC, PDGFR, PDGFRA, PDGFRB, PGP, PGR, PI3K, POLA, POLA1, PPARG, PPARGC1, PR, PTEN, PTGS2, PTPN12, RAF1, RARA, ROS1, RRM1, RRM2, RRM2B, RXRB, RXRG, SIK2, SPARC, SRC, SSTR1, SSTR2, SSTR3, SSTR4, SSTR5, Survivin, TK1, TLE3, TNF, TOP1, TOP2A, TOP2B, TS, TUBB3, TXN, TXNRD1, TYMS, VDR, VEGF, VEGFA, VEGFC, VHL, YES1, ZAP70 |
| Drug associated targets and prognostic markers | ABL1, STK11, FGFR2, ERBB4, SMARCB1, CDKN2A, CTNNB1, FGFR1, FLT3, NOTCH1, NPM1, SRC, SMAD4, FBXW7, PTEN, TP53, AKT1, ALK, APC, CDH1, C-Met, HRAS, IDH1, JAK2, MPL, PDGFRA, SMO, VHL, ATM, CSF1R, FGFR3, GNAS, ERBB2, HNF1A, JAK3, KDR, MLH1, PTPN11, RB1, RET, c-Kit, EGFR, PIK3CA, NRAS, GNA11, GNAQ, KRAS, BRAF |
| Drug associated targets and prognostic markers | ALK, AR, BRAF, cKIT, cMET, EGFR, ER, ERCC1, GNA11, HER2, IDH1, KRAS, MGMT, MGMT promoter methylation, NRAS, PDGFRA, Pgp, PIK3CA, PR, PTEN, ROS1, RRM1, SPARC, TLE3, TOP2A, TOPO1, TS, TUBB3, VHL |
| Drug associated targets | ABL1, AKT1, ALK, APC, AR, ATM, BRAF, BRAF, BRCA1, BRCA2, CDH1, cKIT, cMET, CSF1R, CTNNB1, EGFR, EGFR (H-score), EGFRvIII, ER, ERBB2 (HER2), ERBB4, ERCC1, FBXW7, FGFR1, FGFR2, FLT3, GNA11, GNAQ, GNAS, HER2, HNF1A, HRAS, IDH1, IDH2, JAK2, JAK3, KDR (VEGFR2), KRAS, MGMT, MGMT Promoter Methylation, microsatellite instability (MSI), MLH1, MPL, MSH2, MSH6, NOTCH1, NPM1, NRAS, PD-1, PDGFRA, PD-L1, Pgp, PIK3CA, PMS2, PR, PTEN, PTPN11, RB1, RET, ROS1, RRM1, SMAD4, |

| | |
|---|---|
| | SMARCB1, SMO, SPARC, STK11, TLE3, TOP2A, TOPO1, TP53, TS, TUBB3, VHL |
| Drug associated targets | 1p19q co-deletion, ABL1, AKT1, ALK, APC, AR, ARAF, ATM, BAP1, BRAF, BRCA1, BRCA2, CDH1, CHEK1, CHEK2, cKIT, cMET, CSF1R, CTNNB1, DDR2, EGFR, EGFRvIII, ER, ERBB2 (HER2), ERBB3, ERBB4, ERCC1, FBXW7, FGFR1, FGFR2, FLT3, GNA11, GNAQ, GNAS, H3K36me3, HER2, HNF1A, HRAS, IDH1, IDH2, JAK2, JAK3, KDR (VEGFR2), KRAS, MDMT, MGMT, MGMT Methylation, Microsatellite instability, MLH1, MPL, MSH2, MSH6, NF1, NOTCH1, NPM1, NRAS, NY-ESO-1, PD-1, PDGFRA, PD-L1, Pgp, PIK3CA, PMS2, PR, PTEN, PTPN11, RAF1, RB1, RET, ROS1, ROS1, RRM1, SMAD4, SMARCB1, SMO, SPARC, STK11, TLE3, TOP2A, TOPO1, TP53, TRKA, TS, TUBB3 , VHL, WT1 |
| Drug associated targets | ABL1, AKT1, ALK, APC, AR, ATM, BRAF, BRAF, BRCA1, BRCA2, CDH1, cKIT, cMET, CSF1R, CTNNB1, EGFR, EGFR (H-score), EGFRvIII, ER, ERBB2 (HER2), ERBB4, ERCC1, FBXW7, FGFR1, FGFR2, FLT3, GNA11, GNAQ, GNAS, HER2, HNF1A, HRAS, IDH1, IDH2, JAK2, JAK3, KDR (VEGFR2), KRAS, MGMT, MGMT Promoter Methylation, microsatellite instability (MSI), MLH1, MPL, MSH2, MSH6, NOTCH1, NPM1, NRAS, PD-1, PDGFRA, PD-L1, Pgp, PIK3CA, PMS2, PR, PTEN, PTPN11, RB1, RET, ROS1, RRM1, SMAD4, SMARCB1, SMO, SPARC, STK11, TLE3, TOP2A, TOPO1, TP53, TS, TUBB3, VHL |
| Drug associated targets | TOP2A, Chromosome 17 alteration, PBRM1 (PB1/BAF180), BAP1, SETD2 (ANTI-HISTONE H3), MDM2, Chromosome 12 alteration, ALK, CTLA4, CD3, NY-ESO-1, MAGE-A, TP, EGFR |
| 5-aminosalicyclic acid (5-ASA) efficacy | μ-protocadherin, KLF4, CEBPα |
| Cancer treatment associated markers | AR, AREG (Amphiregulin), BRAF, BRCA1, cKIT, cMET, EGFR, EGFR w/T790M, EML4-ALK, ER, ERBB3, ERBB4, ERCC1, EREG, GNA11, GNAQ, hENT-1, Her2, Her2 Exon 20 insert, IGF1R, Ki67, KRAS, MGMT, MGMT methylation, MSH2, MSI, NRAS, PGP (MDR1), PIK3CA, PR, PTEN, ROS1, ROS1 translocation, RRM1, SPARC, TLE3, TOPO1, TOPO2A, TS, TUBB3, VEGFR2 |
| Cancer treatment associated markers | AR, AREG, BRAF, BRCA1, cKIT, cMET, EGFR, EGFR w/T790M, EML4-ALK, ER, ERBB3, ERBB4, ERCC1, EREG, GNA11, GNAQ, Her2, Her2 Exon 20 insert, IGFR1, Ki67, KRAS, MGMT-Me, MSH2, MSI, NRAS, PGP (MDR-1), PIK3CA, PR, PTEN, ROS1 translocation, RRM1, SPARC, TLE3, TOPO1, TOPO2A, TS, TUBB3, VEGFR2 |
| Colon cancer treatment associated markers | AREG, BRAF, EGFR, EML4-ALK, ERCC1, EREG, KRAS, MSI, NRAS, PIK3CA, PTEN, TS, VEGFR2 |
| Colon cancer treatment associated markers | AREG, BRAF, EGFR, EML4-ALK, ERCC1, EREG, KRAS, MSI, NRAS, PIK3CA, PTEN, TS, VEGFR2 |
| Melanoma treatment associated markers | BRAF, cKIT, ERBB3, ERBB4, ERCC1, GNA11, GNAQ, MGMT, MGMT methylation, NRAS, PIK3CA, TUBB3, VEGFR2 |
| Melanoma treatment associated markers | BRAF, cKIT, ERBB3, ERBB4, ERCC1, GNA11, GNAQ, MGMT-Me, NRAS, PIK3CA, TUBB3, VEGFR2 |
| Ovarian cancer treatment associated markers | BRCA1, cMET, EML4-ALK, ER, ERBB3, ERCC1, hENT-1, HER2, IGF1R, PGP(MDR1), PIK3CA, PR, PTEN, RRM1, TLE3, TOPO1, TOPO2A, TS |

| | |
|---|---|
| Ovarian cancer treatment associated markers | BRCA1, cMET, EML4-ALK (translocation), ER, ERBB3, ERCC1, HER2, PIK3CA, PR, PTEN, RRM1, TLE3, TS |
| Breast cancer treatment associated markers | BRAF, BRCA1, EGFR, EGFR T790M, EML4-ALK, ER, ERBB3, ERCC1, HER2, Ki67, PGP (MDR1), PIK3CA, PR, PTEN, ROS1, ROS1 translocation, RRM1, TLE3, TOPO1, TOPO2A, TS |
| Breast cancer treatment associated markers | BRAF, BRCA1, EGFR w/T790M, EML4-ALK, ER, ERBB3, ERCC1, HER2, Ki67, KRAS, PIK3CA, PR, PTEN, ROS1 translocation, RRM1, TLE3, TOPO1, TOPO2A, TS |
| NSCLC cancer treatment associated markers | BRAF, BRCA1, cMET, EGFR, EGFR w/T790M, EML4-ALK, ERCC1, Her2 Exon 20 insert, KRAS, MSH2, PIK3CA, PTEN, ROS1 (trans), RRM1, TLE3, TS, VEGFR2 |
| NSCLC cancer treatment associated markers | BRAF, cMET, EGFR, EGFR w/T790M, EML4-ALK, ERCC1, Her2 Exon 20 insert, KRAS, MSH2, PIK3CA, PTEN, ROS1 translocation, RRM1, TLE3, TS |
| Mutated in cancers | AKT1, ALK, APC, ATM, BRAF, CDH1, CDKN2A, c-Kit, C-Met, CSF1R, CTNNB1, EGFR, ERBB2, ERBB4, FBXW7, FGFR1, FGFR2, FGFR3, FLT3, GNA11, GNAQ, GNAS, HNF1A, HRAS, IDH1, JAK2, JAK3, KDR, KRAS, MLH1, MPL, NOTCH1, NPM1, NRAS, PDGFRA, PIK3CA, PTEN, PTPN11, RB1, RET, SMAD4, SMARCB1, SMO, SRC, STK11, TP53, VHL |
| Mutated in cancers | ALK, BRAF, BRCA1, BRCA2, EGFR, ERRB2, GNA11, GNAQ, IDH1, IDH2, KIT, KRAS, MET, NRAS, PDGFRA, PIK3CA, PTEN, RET, SRC, TP53 |
| Mutated in cancers | AKT1, HRAS, GNAS, MEK1, MEK2, ERK1, ERK2, ERBB3, CDKN2A, PDGFRB, IFG1R, FGFR1, FGFR2, FGFR3, ERBB4, SMO, DDR2, GRB1, PTCH, SHH, PD1, UGT1A1, BIM, ESR1, MLL, AR, CDK4, SMAD4 |
| Mutated in cancers | ABL, APC, ATM, CDH1, CSFR1, CTNNB1, FBXW7, FLT3, HNF1A, JAK2, JAK3, KDR, MLH1, MPL, NOTCH1, NPM1, PTPN11, RB1, SMARCB1, STK11, VHL |
| Mutated in cancers | ABL1, AKT1, AKT2, AKT3, ALK, APC, AR, ARAF, ARFRP1, ARID1A, ARID2, ASXL1, ATM, ATR, ATRX, AURKA, AURKB, AXL, BAP1, BARD1, BCL2, BCL2L2, BCL6, BCOR, BCORL1, BLM, BRAF, BRCA1, BRCA2, BRIP1, BTK, CARD11, CBFB, CBL, CCND1, CCND2, CCND3, CCNE1, CD79A, CD79B, CDC73, CDH1, CDK12, CDK4, CDK6, CDK8, CDKN1B, CDKN2A, CDKN2B, CDKN2C, CEBPA, CHEK1, CHEK2, CIC, CREBBP, CRKL, CRLF2, CSF1R, CTCF, CTNNA1, CTNNB1, DAXX, DDR2, DNMT3A, DOT1L, EGFR, EMSY (C11orf30), EP300, EPHA3, EPHA5, EPHB1, ERBB2, ERBB3, ERBB4, ERG, ESR1, EZH2, FAM123B (WTX), FAM46C, FANCA, FANCC, FANCD2, FANCE, FANCF, FANCG, FANCL, FBXW7, FGF10, FGF14, FGF19, FGF23, FGF3, FGF4, FGF6, FGFR1, FGFR2, FGFR3, FGFR4, FLT1, FLT3, FLT4, FOXL2, GATA1, GATA2, GATA3, GID4 (C17orf39), GNA11, GNA13, GNAQ, GNAS, GPR124, GRIN2A, GSK3B, HGF, HRAS, IDH1, IDH2, IGF1R, IKBKE, IKZF1, IL7R, INHBA, IRF4, IRS2, JAK1, JAK2, JAK3, JUN, KAT6A (MYST3), KDM5A, KDM5C, KDM6A, KDR, KEAP1, KIT, KLHL6, KRAS, LRP1B, MAP2K1, MAP2K2, MAP2K4, MAP3K1, MCL1, MDM2, MDM4, MED12, MEF2B, MEN1, MET, MITF, MLH1, MLL, MLL2, MPL, MRE11A, MSH2, MSH6, MTOR, MUTYH, MYC, MYCL1, MYCN, MYD88, NF1, NF2, NFE2L2, NFKBIA, NKX2-1, NOTCH1, NOTCH2, NPM1, NRAS, NTRK1, NTRK2, NTRK3, NUP93, PAK3, PALB2, PAX5, PBRM1, PDGFRA, PDGFRB, PDK1, PIK3CA, PIK3CG, PIK3R1, PIK3R2, PPP2R1A, PRDM1, PRKAR1A, PRKDC, PTCH1, PTEN, PTPN11, RAD50, RAD51, RAF1, RARA, RB1, RET, RICTOR, RNF43, RPTOR, RUNX1, SETD2, SF3B1, SMAD2, SMAD4, SMARCA4, SMARCB1, SMO, SOCS1, SOX10, SOX2, SPEN, SPOP, SRC, STAG2, STAT4, STK11, SUFU, TET2, |

|  | |
| --- | --- |
|  | TGFBR2, TNFAIP3, TNFRSF14, TOP1, TP53, TSC1, TSC2, TSHR, VHL, WISP3, WT1, XPO1, ZNF217, ZNF703 |
| Gene rearrangement in cancer | ALK, BCR, BCL2, BRAF, EGFR, ETV1, ETV4, ETV5, ETV6, EWSR1, MLL, MYC, NTRK1, PDGFRA, RAF1, RARA, RET, ROS1, TMPRSS2 |
| Cancer Related | ABL1, ACE2, ADA, ADH1C, ADH4, AGT, AKT1, AKT2, AKT3, ALK, APC, AR, ARAF, AREG, ARFRP1, ARID1A, ARID2, ASNS, ASXL1, ATM, ATR, ATRX, AURKA, AURKB, AXL, BAP1, BARD1, BCL2, BCL2L2, BCL6, BCOR, BCORL1, BCR, BIRC5 (survivin), BLM, BRAF, BRCA1, BRCA2, BRIP1, BTK, CA2, CARD11, CAV, CBFB, CBL, CCND1, CCND2, CCND3, CCNE1, CD33, CD52 (CDW52), CD79A, CD79B, CDC73, CDH1, CDK12, CDK2, CDK4, CDK6, CDK8, CDKN1B, CDKN2A, CDKN2B, CDKN2C, CEBPA, CES2, CHEK1, CHEK2, CIC, CREBBP, CRKL, CRLF2, CSF1R, CTCF, CTNNA1, CTNNB1, DAXX, DCK, DDR2, DHFR, DNMT1, DNMT3A, DNMT3B, DOT1L, EGFR, EMSY (C11orf30) , EP300, EPHA2, EPHA3, EPHA5, EPHB1, ERBB2, ERBB3, ERBB4, ERBR2 (typo?), ERCC3, EREG, ERG, ESR1, ETV1, ETV4, ETV5, ETV6, EWSR1, EZH2, FAM123B (WTX), FAM46C, FANCA, FANCC, FANCD2, FANCE, FANCF, FANCG, FANCL, FBXW7, FGF10, FGF14, FGF19, FGF23, FGF3, FGF4, FGF6, FGFR1, FGFR2, FGFR3, FGFR4, FLT1, FLT3, FLT4, FOLR1, FOLR2, FOXL2, FSHB, FSHPRH1, FSHR, GART, GATA1, GATA2, GATA3, GID4 (C17orf39), GNA11, GNA13, GNAQ, GNAS, GNRH1, GNRHR1, GPR124, GRIN2A, GSK3B, GSTP1, HDAC1, HGF, HIG1, HNF1A, HRAS, HSPCA (HSP90), IDH1, IDH2, IGF1R, IKBKE, IKZF1, IL13RA1, IL2, IL2RA (CD25), IL7R, INHBA, IRF4, IRS2, JAK1, JAK2, JAK3, JUN, KAT6A (MYST3), KDM5A, KDM5C, KDM6A, KDR (VEGFR2), KEAP1, KIT, KLHL6, KRAS, LCK, LRP1B, LTB, LTBR, MAP2K1, MAP2K2, MAP2K4, MAP3K1, MAPK, MCL1, MDM2, MDM4, MED12, MEF2B, MEN1, MET, MGMT, MITF, MLH1, MLL, MLL2, MPL, MRE11A, MS4A1 (CD20), MSH2, MSH6, MTAP, MTOR, MUTYH, MYC, MYCL1, MYCN, MYD88, NF1, NF2, NFE2L2, NFKB1, NFKB2, NFKBIA, NGF, NKX2-1, NOTCH1, NOTCH2, NPM1, NRAS, NTRK1, NTRK2, NTRK3, NUP93, ODC1, OGFR, PAK3, PALB2, PAX5, PBRM1, PDGFC, PDGFRA, PDGFRB, PDK1, PGP, PGR (PR), PIK3CA, PIK3CG, PIK3R1, PIK3R2, POLA, PPARG, PPARGC1, PPP2R1A, PRDM1, PRKAR1A, PRKDC, PTCH1, PTEN, PTPN11, RAD50, RAD51, RAF1, RARA, RB1, RET, RICTOR, RNF43, ROS1, RPTOR, RRM1, RRM2, RRM2B, RUNX1, RXR, RXRB, RXRG, SETD2, SF3B1, SMAD2, SMAD4, SMARCA4, SMARCB1 , SMO, SOCS1, SOX10, SOX2, SPARC, SPEN, SPOP, SRC, SST, SSTR1, SSTR2, SSTR3, SSTR4, SSTR5, STAG2, STAT4, STK11, SUFU , TET2, TGFBR2, TK1, TLE3, TMPRSS2, TNF, TNFAIP3, TNFRSF14, TOP1, TOP2, TOP2A, TOP2B, TP53, TS, TSC1, TSC2, TSHR, TUBB3, TXN, TYMP, VDR, VEGF (VEGFA), VEGFC, VHL, WISP3, WT1, XDH, XPO1, YES1, ZAP70, ZNF217, ZNF703 |
| Cytohesions | cytohesin-1 (CYTH1), cytohesin-2 (CYTH2; ARNO), cytohesin-3 (CYTH3; Grp1; ARNO3), cytohesin-4 (CYTH4) |
| Cancer/Angio | Erb 2, Erb 3, Erb 4, UNC93a, B7H3, MUC1, MUC2, MUC16, MUC17, 5T4, RAGE, VEGF A, VEGFR2, FLT1, DLL4, Epcam |
| Tissue (Breast) | BIG H3, GCDFP-15, PR(B), GPR 30, CYFRA 21, BRCA 1, BRCA 2, ESR 1, ESR2 |
| Tissue (Prostate) | PSMA, PCSA, PSCA, PSA, TMPRSS2 |
| Inflammation/Immune | MFG-E8, IFNAR, CD40, CD80, MICB, HLA-DRb, IL-17-Ra |
| Common vesicle markers | HSPA8, CD63, Actb, GAPDH, CD9, CD81, ANXA2, HSP90AA1, ENO1, YWHAZ, PDCD6IP, CFL1, SDCBP, PKN2, MSN, MFGE8, EZR, YWHAG, PGK1, EEF1A1, PPIA, GLC1F, GK, ANXA6, ANXA1, ALDOA, ACTG1, TPI1, LAMP2, HSP90AB1, DPP4, YWHAB, TSG101, PFN1, LDHB, HSPA1B, HSPA1A, GSTP1, GNAI2, GDI2, CLTC, ANXA5, YWHAQ, TUBA1A, THBS1, PRDX1, LDHA, LAMP1, CLU, CD86 |

| Common vesicle membrane markers | CD63, GAPDH, CD9, CD81, ANXA2, ENO1, SDCBP, MSN, MFGE8, EZR, GK, ANXA1, LAMP2, DPP4, TSG101, HSPA1A, GDI2, CLTC, LAMP1, CD86, ANPEP, TFRC, SLC3A2, RDX, RAP1B, RAB5C, RAB5B, MYH9, ICAM1, FN1, RAB11B, PIGR, LGALS3, ITGB1, EHD1, CLIC1, ATP1A1, ARF1, RAP1A, P4HB, MUC1, KRT10, HLA-A, FLOT1, CD59, C1orf58, BASP1, TACSTD1, STOM |
|---|---|
| Common vesicle markers | MHC class I, MHC class II, Integrins, Alpha 4 beta 1, Alpha M beta 2, Beta 2, ICAM1/CD54, P-selection, Dipeptidylpeptidase IV/CD26, Aminopeptidase n/CD13, CD151, CD53, CD37, CD82, CD81, CD9, CD63, Hsp70, Hsp84/90 Actin, Actin-binding proteins, Tubulin, Annexin I, Annexin II, Annexin IV, Annexin V, Annexin VI, RAB7/RAP1B/RADGDI, Gi2alpha/14-3-3, CBL/LCK, CD63, GAPDH, CD9, CD81, ANXA2, ENO1, SDCBP, MSN, MFGE8, EZR, GK, ANXA1, LAMP2, DPP4, TSG101, HSPA1A, GDI2, CLTC, LAMP1, Cd86, ANPEP, TFRC, SLC3A2, RDX, RAP1B, RAB5C, RAB5B, MYH9, ICAM1, FN1, RAB11B, PIGR, LGALS3, ITGB1, EHD1, CLIC1, ATP1A1, ARF1, RAP1A, P4HB, MUC1, KRT10, HLA-A, FLOT1, CD59, C1orf58, BASP1, TACSTD1, STOM |
| Vesicle markers | A33, a33 n15, AFP, ALA, ALIX, ALP, AnnexinV, APC, ASCA, ASPH (246-260), ASPH (666-680), ASPH (A-10), ASPH (D01P), ASPH (D03), ASPH (G-20), ASPH (H-300), AURKA, AURKB, B7H3, B7H4, BCA-225, BCNP, BDNF, BRCA, CA125 (MUC16), CA-19-9, C-Bir, CD1.1, CD10, CD174 (Lewis y), CD24, CD44, CD46, CD59 (MEM-43), CD63, CD66e CEA, CD73, CD81, CD9, CDA, CDAC1 1a2, CEA, C-Erb2, C-erbB2, CRMP-2, CRP, CXCL12, CYFRA21-1, DLL4, DR3, EGFR, Epcam, EphA2, EphA2 (H-77), ER, ErbB4, EZH2, FASL, FRT, FRT c.f23, GDF15, GPCR, GPR30, Gro-alpha, HAP, HBD 1, HBD2, HER 3 (ErbB3), HSP, HSP70, hVEGFR2, iC3b, IL 6 Unc, IL-1B, IL6 Unc, IL6R, IL8, IL-8, INSIG-2, KLK2, L1CAM, LAMN, LDH, MACC-1, MAPK4, MART-1, MCP-1, M-CSF, MFG-E8, MIC1, MIF, MIS RII, MMG, MMP26, MMP7, MMP9, MS4A1, MUC1, MUC1 seq1, MUC1 seq11A, MUC17, MUC2, Ncam, NGAL, NPGP/NPFF2, OPG, OPN, p53, p53, PA2G4, PBP, PCSA, PDGFRB, PGP9.5, PIM1, PR (B), PRL, PSA, PSMA, PSME3, PTEN, R5-CD9 Tube 1, Reg IV, RUNX2, SCRN1, seprase, SERPINB3, SPARC, SPB, SPDEF, SRVN, STAT 3, STEAP1, TF (FL-295), TFF3, TGM2, TIMP-1, TIMP1, TIMP2, TMEM211, TMPRSS2, TNF-alpha, Trail-R2, Trail-R4, TrKB, TROP2, Tsg 101, TWEAK, UNC93A, VEGF A, YPSMA-1 |
| Vesicle markers | NSE, TRIM29, CD63, CD151, ASPH, LAMP2, TSPAN1, SNAIL, CD45, CKS1, NSE, FSHR, OPN, FTH1, PGP9, ANNEXIN 1, SPD, CD81, EPCAM, PTH1R, CEA, CYTO 7, CCL2, SPA, KRAS, TWIST1, AURKB, MMP9, P27, MMP1, HLA, HIF, CEACAM, CENPH, BTUB, INTG b4, EGFR, NACC1, CYTO 18, NAP2, CYTO 19, ANNEXIN V, TGM2, ERB2, BRCA1, B7H3, SFTPC, PNT, NCAM, MS4A1, P53, INGA3, MUC2, SPA, OPN, CD63, CD9, MUC1, UNCR3, PAN ADH, HCG, TIMP, PSMA, GPCR, RACK1, PSCA, VEGF, BMP2, CD81, CRP, PRO GRP, B7H3, MUC1, M2PK, CD9, PCSA, PSMA |
| Vesicle markers | TFF3, MS4A1, EphA2, GAL3, EGFR, N-gal, PCSA, CD63, MUC1, TGM2, CD81, DR3, MACC-1, TrKB, CD24, TIMP-1, A33, CD66 CEA, PRL, MMP9, MMP7, TMEM211, SCRN1, TROP2, TWEAK, CDACC1, UNC93A, APC, C-Erb, CD10, BDNF, FRT, GPR30, P53, SPR, OPN, MUC2, GRO-1, tsg 101, GDF15 |
| Vesicle markers | CD9, Erb2, Erb4, CD81, Erb3, MUC16, CD63, DLL4, HLA-Drpe, B7H3, IFNAR, 5T4, PCSA, MICB, PSMA, MFG-E8, Muc1, PSA, Muc2, Unc93a, VEGFR2, EpCAM, VEGF A, TMPRSS2, RAGE, PSCA, CD40, Muc17, IL-17-RA, CD80 |
| Benign Prostate Hyperplasia (BPH) | BCMA, CEACAM-1, HVEM, IL-1 R4, IL-10 Rb, Trappin-2, p53, hsa-miR-329, hsa-miR-30a, hsa-miR-335, hsa-miR-152, hsa-miR-151-5p, hsa-miR-200a, hsa-miR-145, hsa-miR-29a, hsa-miR-106b, hsa-miR-595, hsa-miR-142-5p, hsa-miR-99a, hsa-miR-20b, hsa-miR-373, hsa-miR-502-5p, hsa-miR-29b, hsa-miR-142-3p, hsa-miR-663, hsa-miR-423-5p, hsa-miR-15a, hsa-miR-888, hsa-miR-361-3p, hsa-miR-365, hsa-miR-10b, hsa-miR-199a-3p, hsa-miR-181a, hsa-miR-19a, hsa-miR-125b, hsa-miR-760, hsa-miR-7a, hsa-miR-671-5p, hsa-miR-7c, hsa-miR-1979, hsa-miR-103 |

| Metastatic Prostate Cancer | hsa-miR-100, hsa-miR-1236, hsa-miR-1296, hsa-miR-141, hsa-miR-146b-5p, hsa-miR-17*, hsa-miR-181a, hsa-miR-200b, hsa-miR-20a*, hsa-miR-23a*, hsa-miR-331-3p, hsa-miR-375, hsa-miR-452, hsa-miR-572, hsa-miR-574-3p, hsa-miR-577, hsa-miR-582-3p, hsa-miR-937, miR-10a, miR-134, miR-141, miR-200b, miR-30a, miR-32, miR-375, miR-495, miR-564, miR-570, miR-574-3p, miR-885-3p |
|---|---|
| Metastatic Prostate Cancer | hsa-miR-200b, hsa-miR-375, hsa-miR-141, hsa-miR-331-3p, hsa-miR-181a, hsa-miR-574-3p |
| Prostate Cancer | hsa-miR-574-3p, hsa-miR-141, hsa-miR-432, hsa-miR-326, hsa-miR-2110, hsa-miR-181a-2*, hsa-miR-107, hsa-miR-301a, hsa-miR-484, hsa-miR-625* |
| Metastatic Prostate Cancer | hsa-miR-582-3p, hsa-miR-20a*, hsa-miR-375, hsa-miR-200b, hsa-miR-379, hsa-miR-572, hsa-miR-513a-5p, hsa-miR-577, hsa-miR-23a*, hsa-miR-1236, hsa-miR-609, hsa-miR-17*, hsa-miR-130b, hsa-miR-619, hsa-miR-624*, hsa-miR-198 |
| Metastatic Prostate Cancer | FOXO1A, SOX9, CLNS1A, PTGDS, XPO1, LETMD1, RAD23B, ABCC3, APC, CHES1, EDNRA, FRZB, HSPG2, TMPRSS2_ETV1 fusion |
| Prostate Cancer | hsa-let-7b, hsa-miR-107, hsa-miR-1205, hsa-miR-1270, hsa-miR-130b, hsa-miR-141, hsa-miR-143, hsa-miR-148b*, hsa-miR-150, hsa-miR-154*, hsa-miR-181a*, hsa-miR-181a-2*, hsa-miR-18a*, hsa-miR-19b-1*, hsa-miR-204, hsa-miR-2110, hsa-miR-215, hsa-miR-217, hsa-miR-219-2-3p, hsa-miR-23b*, hsa-miR-299-5p, hsa-miR-301a, hsa-miR-301a, hsa-miR-326, hsa-miR-331-3p, hsa-miR-365*, hsa-miR-373*, hsa-miR-424, hsa-miR-424*, hsa-miR-432, hsa-miR-450a, hsa-miR-451, hsa-miR-484, hsa-miR-497, hsa-miR-517*, hsa-miR-517a, hsa-miR-518f, hsa-miR-574-3p, hsa-miR-595, hsa-miR-617, hsa-miR-625*, hsa-miR-628-5p, hsa-miR-629, hsa-miR-634, hsa-miR-769-5p, hsa-miR-93, hsa-miR-96 |
| Prostate Cancer | CD9, PSMA, PCSA, CD63, CD81, B7H3, IL 6, OPG-13, IL6R, PA2G4, EZH2, RUNX2, SERPINB3, EpCam |
| Prostate Cancer | A33, a33 n15, AFP, ALA, ALIX, ALP, AnnexinV, APC, ASCA, ASPH (246-260), ASPH (666-680), ASPH (A-10), ASPH (D01P), ASPH (D03), ASPH (G-20), ASPH (H-300), AURKA, AURKB, B7H3, B7H4, BCA-225, BCNP, BDNF, BRCA, CA125 (MUC16), CA-19-9, C-Bir, CD1.1, CD10, CD174 (Lewis y), CD24, CD44, CD46, CD59 (MEM-43), CD63, CD66e CEA, CD73, CD81, CD9, CDA, CDAC1 1a2, CEA, C-Erb2, C-erbB2, CRMP-2, CRP, CXCL12, CYFRA21-1, DLL4, DR3, EGFR, Epcam, EphA2, EphA2 (H-77), ER, ErbB4, EZH2, FASL, FRT, FRT c.f23, GDF15, GPCR, GPR30, Gro-alpha, HAP, HBD 1, HBD2, HER 3 (ErbB3), HSP, HSP70, hVEGFR2, iC3b, IL 6 Unc, IL-1B, IL6 Unc, IL6R, IL8, IL-8, INSIG-2, KLK2, L1CAM, LAMN, LDH, MACC-1, MAPK4, MART-1, MCP-1, M-CSF, MFG-E8, MIC1, MIF, MIS RII, MMG, MMP26, MMP7, MMP9, MS4A1, MUC1, MUC1 seq1, MUC1 seq11A, MUC17, MUC2, Ncam, NGAL, NPGP/NPFF2, OPG, OPN, p53, p53, PA2G4, PBP, PCSA, PDGFRB, PGP9.5, PIM1, PR (B), PRL, PSA, PSMA, PSME3, PTEN, R5-CD9 Tube 1, Reg IV, RUNX2, SCRN1, seprase, SERPINB3, SPARC, SPB, SPDEF, SRVN, STAT 3, STEAP1, TF (FL-295), TFF3, TGM2, TIMP-1, TIMP1, TIMP2, TMEM211, TMPRSS2, TNF-alpha, Trail-R2, Trail-R4, TrKB, TROP2, Tsg 101, TWEAK, UNC93A, VEGF A, YPSMA-1 |
| Prostate Cancer Vesicle Markers | 5T4, ACTG1, ADAM10, ADAM15, ALDOA, ANXA2, ANXA6, APOA1, ATP1A1, BASP1, C1orf58, C20orf114, C8B, CAPZA1, CAV1, CD151, CD2AP, CD59, CD9, CD9, CFL1, CFP, CHMP4B, CLTC, COTL1, CTNND1, CTSB, CTSZ, CYCS, DPP4, EEF1A1, EHD1, ENO1, F11R, F2, F5, FAM125A, FNBP1L, FOLH1, GAPDH, GLB1, GPX3, HIST1H1C, HIST1H2AB, HSP90AB1, HSPA1B, HSPA8, IGSF8, ITGB1, ITIH3, JUP, LDHA, LDHB, LUM, LYZ, MFGE8, MGAM, MMP9, MYH2, MYL6B, NME1, NME2, PABPC1, PABPC4, PACSIN2, PCBP2, PDCD6IP, PRDX2, PSA, PSMA, PSMA1, PSMA2, PSMA4, PSMA6, PSMA7, PSMB1, PSMB2, PSMB3, PSMB4, PSMB5, PSMB6, PSMB8, PTGFRN, RPS27A, SDCBP, SERINC5, SH3GL1, SLC3A2, SMPDL3B, SNX9, TACSTD1, TCN2, THBS1, TPI1, TSG101, TUBB, VDAC2, VPS37B, YWHAG, YWHAQ, YWHAZ |
| Prostate Cancer Vesicle Markers | FLNA, DCRN, HER 3 (ErbB3), VCAN, CD9, GAL3, CDADC1, GM-CSF, EGFR, RANK, CSA, PSMA, ChickenIgY, B7H3, PCSA, CD63, CD3, MUC1, TGM2, CD81, S100-A4, MFG-E8, Integrin, NK-2R(C-21), PSA, CD24, TIMP-1, IL6 Unc, PBP, PIM1, CA-19-9, Trail-R4, MMP9, PRL, EphA2, TWEAK, NY-ESO-1, |

| | | |
|---|---|---|
| | | Mammaglobin, UNC93A, A33, AURKB, CD41, XAGE-1, SPDEF, AMACR, seprase/FAP, NGAL, CXCL12, FRT, CD66e CEA, SIM2 (C-15), C-Bir, STEAP, PSIP1/LEDGF, MUC17, hVEGFR2, ERG, MUC2, ADAM10, ASPH (A-10), CA125, Gro-alpha, Tsg 101, SSX2, Trail-R4 |
| Prostate Cancer Vesicle Markers | | NT5E (CD73), A33, ABL2, ADAM10, AFP, ALA, ALIX, ALPL, AMACR, Apo J/CLU, ASCA, ASPH (A-10), ASPH (D01P), AURKB, B7H3, B7H4, BCNP, BDNF, CA125 (MUC16), CA-19-9, C-Bir (Flagellin), CD10, CD151, CD24, CD3, CD41, CD44, CD46, CD59(MEM-43), CD63, CD66e CEA, CD81, CD9, CDA, CDADC1, C-erbB2, CRMP-2, CRP, CSA, CXCL12, CXCR3, CYFRA21-1, DCRN, DDX-1, DLL4, EGFR, EpCAM, EphA2, ERG, EZH2, FASL, FLNA, FRT, GAL3, GATA2, GM-CSF, Gro-alpha, HAP, HER3 (ErbB3), HSP70, HSPB1, hVEGFR2, iC3b, IL-1B, IL6 R, IL6 Unc, IL7 R alpha/CD127, IL8, INSIG-2, Integrin, KLK2, Label, LAMN, Mammaglobin, M-CSF, MFG-E8, MIF, MIS RII, MMP7, MMP9, MS4A1, MUC1, MUC17, MUC2, Ncam, NDUFB7, NGAL, NK-2R(C-21), NY-ESO-1, p53, PBP, PCSA, PDGFRB, PIM1, PRL, PSA, PSIP1/LEDGF, PSMA, RAGE, RANK, Reg IV, RUNX2, S100-A4, seprase/FAP, SERPINB3, SIM2 (C-15), SPARC, SPC, SPDEF, SPP1, SSX2, SSX4, STEAP, STEAP4, TFF3, TGM2, TIMP-1, TMEM211, Trail-R2, Trail-R4, TrKB (poly), Trop2, Tsg 101, TWEAK, UNC93A, VCAN, VEGF A, wnt-5a(C-16), XAGE, XAGE-1 |
| Prostate Vesicle Membrane | | ADAM 9, ADAM10, AGR2, ALDOA, ALIX, ANXA1, ANXA2, ANXA4, ARF6, ATP1A3, B7H3, BCHE, BCL2L14 (Bcl G), BCNP1, BDKRB2, BDNFCAV1-Caveolin1, CCR2 (CC chemokine receptor 2, CD192), CCR5 (CC chemokine receptor 5), CCT2 (TCP1-beta), CD10, CD151, CD166/ALCAM, CD24, CD283/TLR3, CD41, CD46, CD49d (Integrin alpha 4, ITGA4), CD63, CD81, CD9, CD90/THY1, CDH1, CDH2, CDKN1A cyclin-dependent kinase inhibitor (p21), CGA gene (coding for the alpha subunit of glycoprotein hormones), CLDN3-Claudin3, COX2 (PTGS2), CSE1L (Cellular Apoptosis Susceptibility), CXCR3, Cytokeratin 18, Eag1 (KCNH1), EDIL3 (del-1), EDNRB - Endothelial Receptor Type B, EGFR, EpoR, EZH2 (enhancer of Zeste Homolog2), EZR, FABP5, Farnesyltransferase/geranylgeranyl diphosphate synthase 1 (GGPS1), Fatty acid synthase (FASN), FTL (light and heavy), GAL3, GDF15-Growth Differentiation Factor 15, GloI, GM-CSF, GSTP1, H3F3A, HGF (hepatocyte growth factor), hK2 / Kif2a, HSP90AA1, HSPA1A / HSP70-1, HSPB1, IGFBP-2, IGFBP-3, IL1alpha, IL-6, IQGAP1, ITGAL (Integrin alpha L chain), Ki67, KLK1, KLK10, KLK11, KLK12, KLK13, KLK14, KLK15, KLK4, KLK5, KLK6, KLK7, KLK8, KLK9, Lamp-2, LDH-A, LGALS3BP, LGALS8, MMP 1, MMP 2, MMP 25, MMP 3, MMP10, MMP-14/MT1-MMP, MMP7, MTA1nAnS, Nav1.7, NKX3-1, Notch1, NRP1 / CD304, PAP (ACPP), PGP, PhIP, PIP3 / BPNT1, PKM2, PKP1 (plakophilin1), PKP3 (plakophilin3), Plasma chromogranin-A (CgA), PRDX2, Prostate secretory protein (PSP94) / β-Microseminoprotein (MSP) / IGBF, PSAP, PSMA, PSMA1, PTENPTPN13/PTPL1, RPL19, seprase/FAPSET, SLC3A2 / CD98, SRVN, STEAP1, Syndecan / CD138, TGFB, TGM2, TIMP-1TLR4 (CD284), TLR9 (CD289), TMPRSS1 / hepsin, TMPRSS2, TNFR1, TNFα, Transferrin receptor/CD71/TRFR, Trop2 (TACSTD2), TWEAK uPA (urokinase plasminoge activator) degrades extracellular matrix, uPAR (uPA receptor) / CD87, VEGFR1, VEGFR2 |
| Prostate Vesicle Markers | | ADAM 34, ADAM 9, AGR2, ALDOA, ANXA1, ANXA 11, ANXA4, ANXA 7, ANXA2, ARF6, ATP1A1, ATP1A2, ATP1A3, BCHE, BCL2L14 (Bcl G), BDKRB2, CA215, CAV1-Caveolin1, CCR2 (CC chemokine receptor 2, CD192), CCR5 (CC chemokine receptor 5), CCT2 (TCP1-beta), CD166/ALCAM, CD49b (Integrin alpha 2, ITGA4), CD90/THY1, CDH1, CDH2, CDKN1A cyclin-dependent kinase inhibitor (p21), CGA gene (coding for the alpha subunit of glycoprotein hormones), CHMP4B, CLDN3- Claudin3, CLSTN1 (Calsyntenin-1), COX2 (PTGS2), CSE1L (Cellular Apoptosis Susceptibility), Cytokeratin 18, Eag1 (KCNH1) (plasma membrane-K+-voltage gated channel), EDIL3 (del-1), EDNRB- Endothelial Receptor Type B, Endoglin/CD105, ENOX2 – Ecto-NOX disulphide Thiol exchanger 2, EPCA-2 Early prostate cancer antigen2, EpoR, EZH2 (enhancer |

| | |
|---|---|
| | of Zeste Homolog2), EZR, FABP5, Farnesyltransferase/geranylgeranyl diphosphate synthase 1 (GGPS1), Fatty acid synthase (FASN, plasma membrane protein), FTL (light and heavy), GDF15-Growth Differentiation Factor 15, GloI, GSTP1, H3F3A, HGF (hepatocyte growth factor), hK2 (KLK2), HSP90AA1, HSPA1A / HSP70-1, IGFBP-2, IGFBP-3, IL1alpha, IL-6, IQGAP1, ITGAL (Integrin alpha L chain), Ki67, KLK1, KLK10, KLK11, KLK12, KLK13, KLK14, KLK15, KLK4, KLK5, KLK6, KLK7, KLK8, KLK9, Lamp-2, LDH-A, LGALS3BP, LGALS8, MFAP5, MMP 1, MMP 2, MMP 24, MMP 25, MMP 3, MMP10, MMP-14/MT1-MMP, MTA1, nAnS, Nav1.7, NCAM2 – Neural cell Adhesion molecule 2, NGEP/D-TMPP/IPCA-5/ANO7, NKX3-1, Notch1, NRP1 / CD304, PGP, PAP (ACPP), PCA3- Prostate cancer antigen 3, Pdia3/ERp57, PhIP, phosphatidylethanolamine (PE), PIP3, PKP1 (plakophilin1), PKP3 (plakophilin3), Plasma chromogranin-A (CgA), PRDX2, Prostate secretory protein (PSP94) / β-Microseminoprotein (MSP) / IGBF, PSAP, PSMA1, PTEN, PTGFRN, PTPN13/PTPL1, PKM2, RPL19, SCA-1 / ATXN1, SERINC5/TPO1, SET, SLC3A2 / CD98, STEAP1, STEAP-3, SRVN, Syndecan / CD138, TGFB, Tissue Polypeptide Specific antigen TPS, TLR4 (CD284), TLR9 (CD289), TMPRSS1 / hepsin, TMPRSS2, TNFR1, TNFα, CD283/TLR3, Transferrin receptor/CD71/TRFR, uPA (urokinase plasminoge activator) , uPAR (uPA receptor) / CD87, VEGFR1, VEGFR2 |
| Prostate Cancer Treatment | hsa-miR-1974, hsa-miR-27b, hsa-miR-103, hsa-miR-146a, hsa-miR-22, hsa-miR-382, hsa-miR-23a, hsa-miR-376c, hsa-miR-335, hsa-miR-142-5p, hsa-miR-221, hsa-miR-142-3p, hsa-miR-151-3p, hsa-miR-21, hsa-miR-16 |
| Prostate Cancer | let-7d, miR-148a, miR-195, miR-25, miR-26b, miR-329, miR-376c, miR-574-3p, miR-888, miR-9, miR1204, miR-16-2*, miR-497, miR-588, miR-614, miR-765, miR92b*, miR-938, let-7f-2*, miR-300, miR-523, miR-525-5p, miR-1182, miR-1244, miR-520d-3p, miR-379, let-7b, miR-125a-3p, miR-1296, miR-134, miR-149, miR-150, miR-187, miR-32, miR-324-3p, miR-324-5p, miR-342-3p, miR-378, miR-378*, miR-384, miR-451, miR-455-3p, miR-485-3p, miR-487a, miR-490-3p, miR-502-5p, miR-548a-5p, miR-550, miR-562, miR-593, miR-593*, miR-595, miR-602, miR-603, miR-654-5p, miR-877*, miR-886-5p, miR-125a-5p, miR-140-3p, miR-192, miR-196a, miR-2110, miR-212, miR-222, miR-224*, miR-30b*, miR-499-3p, miR-505* |
| Prostate (PCSA+ cMVs) | miR-182, miR-663, miR-155, mirR-125a-5p, miR-548a-5p, miR-628-5p, miR-517*, miR-450a, miR-920, hsa-miR-619, miR-1913, miR-224*, miR-502-5p, miR-888, miR-376a, miR-542-5p, miR-30b*, miR-1179 |
| Prostate Cancer | miR-183-96-182 cluster (miRs-183, 96 and 182), metal ion transporter such as hZIP1, SLC39A1, SLC39A2, SLC39A3, SLC39A4, SLC39A5, SLC39A6, SLC39A7, SLC39A8, SLC39A9, SLC39A10, SLC39A11, SLC39A12, SLC39A13, SLC39A14 |
| Prostate Cancer | RAD23B, FBP1, TNFRSF1A, CCNG2, NOTCH3, ETV1, BID, SIM2, LETMD1, ANXA1, miR-519d, miR-647 |
| Prostate Cancer | RAD23B, FBP1, TNFRSF1A, NOTCH3, ETV1, BID, SIM2, ANXA1, BCL2 |
| Prostate Cancer | ANPEP, ABL1, PSCA, EFNA1, HSPB1, INMT, TRIP13 |
| Prostate Cancer | E2F3, c-met, pRB, EZH2, e-cad, CAXII, CAIX, HIF-1α, Jagged, PIM-1, hepsin, RECK, Clusterin, MMP9, MTSP-1, MMP24, MMP15, IGFBP-2, IGFBP-3, E2F4, caveolin, EF-1A, Kallikrein 2, Kallikrein 3, PSGR |
| Prostate Cancer | A2ML1, BAX, C10orf47, C1orf162, CSDA, EIFC3, ETFB, GABARAPL2, GUK1, GZMH, HIST1H3B, HLA-A, HSP90AA1, NRGN, PRDX5, PTMA, RABAC1, RABGAP1L, RPL22, SAP18, SEPW1, SOX1 |
| Prostate Cancer | NY-ESO-1, SSX-2, SSX-4, XAGE-lb, AMACR, p90 autoantigen, LEDGF |
| Prostate Cancer | A33, ABL2, ADAM10, AFP, ALA, ALIX, ALPL, ApoJ/CLU, ASCA, ASPH(A-10), ASPH(D01P), AURKB, B7H3, B7H3, B7H4, BCNP, BDNF, CA125(MUC16), CA-19-9, C-Bir, CD10, CD151, CD24, CD41, CD44, CD46, CD59(MEM-43), CD63, CD63, CD66eCEA, CD81, CD81, CD9, CD9, CDA, CDADC1, CRMP-2, CRP, CXCL12, CXCR3, CYFRA21-1, DDX-1, DLL4, DLL4, EGFR, Epcam, EphA2, ErbB2, ERG, EZH2, FASL, FLNA, FRT, GAL3, GATA2, GM-CSF, Gro-alpha, HAP, HER3(ErbB3), HSP70, HSPB1, hVEGFR2, iC3b, IL-1B, IL6R, IL6Unc, |

| | |
|---|---|
| | IL7Ralpha/CD127, IL8, INSIG-2, Integrin, KLK2, LAMN, Mammoglobin, M-CSF, MFG-E8, MIF, MISRII, MMP7, MMP9, MUC1, Muc1, MUC17, MUC2, Ncam, NDUFB7, NGAL, NK-2R(C-21), NT5E (CD73), p53, PBP, PCSA, PCSA, PDGFRB, PIM1, PRL, PSA, PSA, PSMA, PSMA, RAGE, RANK, RegIV, RUNX2, S100-A4, seprase/FAP, SERPINB3, SIM2(C-15), SPARC, SPC, SPDEF, SPP1, STEAP, STEAP4, TFF3, TGM2, TIMP-1, TMEM211, Trail-R2, Trail-R4, TrKB(poly), Trop2, Tsg101, TWEAK, UNC93A, VEGFA, wnt-5a(C-16) |
| Prostate Vesicles | CD9, CD63, CD81, PCSA, MUC2, MFG-E8 |
| Prostate Cancer | miR-148a, miR-329, miR-9, miR-378*, miR-25, miR-614, miR-518c*, miR-378, miR-765, let-7f-2*, miR-574-3p, miR-497, miR-32, miR-379, miR-520g, miR-542-5p, miR-342-3p, miR-1206, miR-663, miR-222 |
| Prostate Cancer | hsa-miR-877*, hsa-miR-593, hsa-miR-595, hsa-miR-300, hsa-miR-324-5p, hsa-miR-548a-5p, hsa-miR-329, hsa-miR-550, hsa-miR-886-5p, hsa-miR-603, hsa-miR-490-3p, hsa-miR-938, hsa-miR-149, hsa-miR-150, hsa-miR-1296, hsa-miR-384, hsa-miR-487a, hsa-miRPlus-C1089, hsa-miR-485-3p, hsa-miR-525-5p |
| Prostate Cancer | hsa-miR-451, hsa-miR-223, hsa-miR-593*, hsa-miR-1974, hsa-miR-486-5p, hsa-miR-19b, hsa-miR-320b, hsa-miR-92a, hsa-miR-21, hsa-miR-675*, hsa-miR-16, hsa-miR-876-5p, hsa-miR-144, hsa-miR-126, hsa-miR-137, hsa-miR-1913, hsa-miR-29b-1*, hsa-miR-15a, hsa-miR-93, hsa-miR-1266 |
| Inflammatory Disease | miR-588, miR-1258, miR-16-2*, miR-938, miR-526b, miR-92b*, let-7d, miR-378*, miR-124, miR-376c, miR-26b, miR-1204, miR-574-3p, miR-195, miR-499-3p, miR-2110, miR-888 |
| Prostate Cancer | A33, ADAM10, AMACR, ASPH (A-10), AURKB, B7H3, CA125, CA-19-9, C-Bir, CD24, CD3, CD41, CD63, CD66e CEA, CD81, CD9, CDADC1, CSA, CXCL12, DCRN, EGFR, EphA2, ERG, FLNA, FRT, GAL3, GM-CSF, Gro-alpha, HER 3 (ErbB3), hVEGFR2, IL6 Unc, Integrin, Mammaglobin, MFG-E8, MMP9, MUC1, MUC17, MUC2, NGAL, NK-2R(C-21), NY-ESO-1, PBP, PCSA, PIM1, PRL, PSA, PSIP1/LEDGF, PSMA, RANK, S100-A4, seprase/FAP, SIM2 (C-15), SPDEF, SSX2, STEAP, TGM2, TIMP-1, Trail-R4, Tsg 101, TWEAK, UNC93A, VCAN, XAGE-1 |
| Prostate Cancer | A33, ADAM10, ALIX, AMACR, ASCA, ASPH (A-10), AURKB, B7H3, BCNP, CA125, CA-19-9, C-Bir (Flagellin), CD24, CD3, CD41, CD63, CD66e CEA, CD81, CD9, CDADC1, CRP, CSA, CXCL12, CYFRA21-1, DCRN, EGFR, EpCAM, EphA2, ERG, FLNA, GAL3, GATA2, GM-CSF, Gro alpha, HER3 (ErbB3), HSP70, hVEGFR2, iC3b, IL-1B, IL6 Unc, IL8, Integrin, KLK2, Mammaglobin, MFG-E8, MMP7, MMP9, MS4A1, MUC1, MUC17, MUC2, NGAL, NK-2R(C-21), NY-ESO-1, p53, PBP, PCSA, PIM1, PRL, PSA, PSMA, RANK, RUNX2, S100-A4, seprase/FAP, SERPINB3, SIM2 (C-15), SPC, SPDEF, SSX2, SSX4, STEAP, TGM2, TIMP-1, TRAIL R2, Trail-R4, Tsg 101, TWEAK, VCAN, VEGF A, XAGE |
| Prostate Vesicles | EpCam, CD81, PCSA, MUC2, MFG-E8 |
| Prostate Vesicles | CD9, CD63, CD81, MMP7, EpCAM |
| Prostate Cancer | let-7d, miR-148a, miR-195, miR-25, miR-26b, miR-329, miR-376c, miR-574-3p, miR-888, miR-9, miR1204, miR-16-2*, miR-497, miR-588, miR-614, miR-765, miR92b*, miR-938, let-7f-2*, miR-300, miR-523, miR-525-5p, miR-1182, miR-1244, miR-520d-3p, miR-379, let-7b, miR-125a-3p, miR-1296, miR-134, miR-149, miR-150, miR-187, miR-32, miR-324-3p, miR-324-5p, miR-342-3p, miR-378, miR-378*, miR-384, miR-451, miR-455-3p, miR-485-3p, miR-487a, miR-490-3p, miR-502-5p, miR-548a-5p, miR-550, miR-562, miR-593, miR-593*, miR-595, miR-602, miR-603, miR-654-5p, miR-877*, miR-886-5p, miR-125a-5p, miR-140-3p, miR-192, miR-196a, miR-2110, miR-212, miR-222, miR-224*, miR-30b*, miR-499-3p, miR-505* |
| Prostate Cancer | STAT3, EZH2, p53, MACC1, SPDEF, RUNX2, YB-1, AURKA, AURKB |
| Prostate Cancer (Ensembl ENSG identifiers) | E.001036, E.001497, E.001561, E.002330, E.003402, E.003756, E.004838, E.005471, E.005882, E.005893, E.006210, E.006453, E.006625, E.006695, E.006756, E.007264, E.007952, E.008118, E.008196, E.009694, E.009830, E.010244, E.010256, E.010278, E.010539, E.010810, E.011052, E.011114, E.011143, E.011304, E.011451, E.012061, E.012779, E.014216, E.014257, |

| |
|---|
| E.015133, E.015171, E.015479, E.015676, E.016402, E.018189, E.018699, E.020922, E.022976, E.023909, E.026508, E.026559, E.029363, E.029725, E.030582, E.033030, E.035141, E.036257, E.036448, E.038002, E.039068, E.039560, E.041353, E.044115, E.047410, E.047597, E.048544, E.048828, E.049239, E.049246, E.049883, E.051596, E.051620, E.052795, E.053108, E.054118, E.054938, E.056097, E.057252, E.057608, E.058729, E.059122, E.059378, E.059691, E.060339, E.060688, E.061794, E.061918, E.062485, E.063241, E.063244, E.064201, E.064489, E.064655, E.064886, E.065054, E.065057, E.065308, E.065427, E.065457, E.065485, E.065526, E.065548, E.065978, E.066455, E.066557, E.067248, E.067369, E.067704, E.068724, E.068885, E.069535, E.069712, E.069849, E.069869, E.069956, E.070501, E.070785, E.070814, E.071246, E.071626, E.071859, E.072042, E.072071, E.072110, E.072506, E.073050, E.073350, E.073584, E.073756, E.074047, E.074071, E.074964, E.075131, E.075239, E.075624, E.075651, E.075711, E.075856, E.075886, E.076043, E.076248, E.076554, E.076864, E.077097, E.077147, E.077312, E.077514, E.077522, E.078269, E.078295, E.078808, E.078902, E.079246, E.079313, E.079785, E.080572, E.080823, E.081087, E.081138, E.081181, E.081721, E.081842, E.082212, E.082258, E.082556, E.083093, E.083720, E.084234, E.084463, E.085224, E.085733, E.086062, E.086205, E.086717, E.087087, E.087301, E.088888, E.088899, E.088930, E.088992, E.089048, E.089127, E.089154, E.089177, E.089248, E.089280, E.089902, E.090013, E.090060, E.090565, E.090612, E.090615, E.090674, E.090861, E.090889, E.091140, E.091483, E.091542, E.091732, E.092020, E.092199, E.092421, E.092621, E.092820, E.092871, E.092978, E.093010, E.094755, E.095139, E.095380, E.095485, E.095627, E.096060, E.096384, E.099331, E.099715, E.099783, E.099785, E.099800, E.099821, E.099899, E.099917, E.099956, E.100023, E.100056, E.100065, E.100084, E.100142, E.100191, E.100216, E.100242, E.100271, E.100284, E.100299, E.100311, E.100348, E.100359, E.100393, E.100399, E.100401, E.100412, E.100442, E.100575, E.100577, E.100583, E.100601, E.100603, E.100612, E.100632, E.100714, E.100739, E.100796, E.100802, E.100815, E.100823, E.100836, E.100883, E.101057, E.101126, E.101152, E.101222, E.101246, E.101265, E.101365, E.101439, E.101557, E.101639, E.101654, E.101811, E.101812, E.101901, E.102030, E.102054, E.102103, E.102158, E.102174, E.102241, E.102290, E.102316, E.102362, E.102384, E.102710, E.102780, E.102904, E.103035, E.103067, E.103175, E.103194, E.103449, E.103479, E.103591, E.103599, E.103855, E.103978, E.104064, E.104067, E.104131, E.104164, E.104177, E.104228, E.104331, E.104365, E.104419, E.104442, E.104611, E.104626, E.104723, E.104760, E.104805, E.104812, E.104823, E.104824, E.105127, E.105220, E.105221, E.105281, E.105379, E.105402, E.105404, E.105409, E.105419, E.105428, E.105486, E.105514, E.105518, E.105618, E.105705, E.105723, E.105939, E.105948, E.106049, E.106078, E.106128, E.106153, E.106346, E.106392, E.106554, E.106565, E.106603, E.106633, E.107104, E.107164, E.107404, E.107485, E.107551, E.107581, E.107623, E.107798, E.107816, E.107833, E.107890, E.107897, E.107968, E.108296, E.108312, E.108375, E.108387, E.108405, E.108417, E.108465, E.108561, E.108582, E.108639, E.108641, E.108848, E.108883, E.108953, E.109062, E.109184, E.109572, E.109625, E.109758, E.109790, E.109814, E.109846, E.109956, E.110063, E.110066, E.110104, E.110107, E.110321, E.110328, E.110921, E.110955, E.111057, E.111218, E.111261, E.111335, E.111540, E.111605, E.111647, E.111785, E.111790, E.111801, E.111907, E.112039, E.112081, E.112096, E.112110, E.112144, E.112232, E.112234, E.112473, E.112578, E.112584, E.112715, E.112941, E.113013, E.113163, E.113282, E.113368, E.113441, E.113448, E.113522, E.113580, E.113645, E.113719, E.113739, E.113790, E.114054, E.114127, E.114302, E.114331, E.114388, E.114491, E.114861, E.114867, E.115053, E.115221, E.115234, E.115239, E.115241, E.115257, E.115339, E.115540, E.115541, E.115561, E.115604, |

E.115648, E.115738, E.115758, E.116044, E.116096, E.116127, E.116254,
E.116288, E.116455, E.116478, E.116604, E.116649, E.116726, E.116754,
E.116833, E.117298, E.117308, E.117335, E.117362, E.117411, E.117425,
E.117448, E.117480, E.117592, E.117593, E.117614, E.117676, E.117713,
E.117748, E.117751, E.117877, E.118181, E.118197, E.118260, E.118292,
E.118513, E.118523, E.118640, E.118898, E.119121, E.119138, E.119318,
E.119321, E.119335, E.119383, E.119421, E.119636, E.119681, E.119711,
E.119820, E.119888, E.119906, E.120159, E.120328, E.120337, E.120370,
E.120656, E.120733, E.120837, E.120868, E.120915, E.120948, E.121022,
E.121057, E.121068, E.121104, E.121390, E.121671, E.121690, E.121749,
E.121774, E.121879, E.121892, E.121903, E.121940, E.121957, E.122025,
E.122033, E.122126, E.122507, E.122566, E.122705, E.122733, E.122870,
E.122884, E.122952, E.123066, E.123080, E.123143, E.123154, E.123178,
E.123416, E.123427, E.123595, E.123901, E.123908, E.123983, E.123992,
E.124143, E.124164, E.124181, E.124193, E.124216, E.124232, E.124529,
E.124562, E.124570, E.124693, E.124749, E.124767, E.124788, E.124795,
E.124831, E.124942, E.125246, E.125257, E.125304, E.125352, E.125375,
E.125445, E.125492, E.125676, E.125753, E.125798, E.125844, E.125868,
E.125901, E.125944, E.125995, E.126062, E.126267, E.126653, E.126773,
E.126777, E.126814, E.126858, E.126883, E.126934, E.126945, E.126952,
E.127022, E.127328, E.127329, E.127399, E.127415, E.127554, E.127616,
E.127720, E.127824, E.127884, E.127914, E.127946, E.127948, E.128050,
E.128311, E.128342, E.128609, E.128626, E.128683, E.128708, E.128881,
E.129315, E.129351, E.129355, E.129514, E.129636, E.129657, E.129757,
E.129810, E.129990, E.130175, E.130177, E.130193, E.130255, E.130299,
E.130305, E.130338, E.130340, E.130402, E.130413, E.130612, E.130713,
E.130764, E.130770, E.130810, E.130826, E.130935, E.131351, E.131467,
E.131473, E.131771, E.131773, E.132002, E.132275, E.132323, E.132382,
E.132475, E.132481, E.132589, E.132646, E.132716, E.132881, E.133313,
E.133315, E.133687, E.133835, E.133863, E.133874, E.133961, E.134077,
E.134138, E.134207, E.134248, E.134308, E.134444, E.134452, E.134548,
E.134684, E.134759, E.134809, E.134851, E.134955, E.135052, E.135297,
E.135298, E.135387, E.135390, E.135476, E.135486, E.135525, E.135597,
E.135679, E.135740, E.135829, E.135842, E.135870, E.135900, E.135914,
E.135926, E.135940, E.135999, E.136044, E.136068, E.136152, E.136169,
E.136280, E.136371, E.136383, E.136450, E.136521, E.136527, E.136574,
E.136710, E.136750, E.136807, E.136874, E.136875, E.136930, E.136933,
E.136935, E.137055, E.137124, E.137312, E.137409, E.137497, E.137513,
E.137558, E.137601, E.137727, E.137776, E.137806, E.137814, E.137815,
E.137948, E.137955, E.138028, E.138031, E.138041, E.138050, E.138061,
E.138069, E.138073, E.138095, E.138160, E.138294, E.138347, E.138363,
E.138385, E.138587, E.138594, E.138621, E.138674, E.138756, E.138757,
E.138760, E.138772, E.138796, E.139211, E.139405, E.139428, E.139517,
E.139613, E.139626, E.139684, E.139697, E.139874, E.140263, E.140265,
E.140326, E.140350, E.140374, E.140382, E.140451, E.140481, E.140497,
E.140632, E.140678, E.140694, E.140743, E.140932, E.141002, E.141012,
E.141258, E.141378, E.141425, E.141429, E.141522, E.141543, E.141639,
E.141744, E.141873, E.141994, E.142025, E.142208, E.142515, E.142606,
E.142698, E.142765, E.142864, E.142875, E.143013, E.143294, E.143321,
E.143353, E.143374, E.143375, E.143390, E.143578, E.143614, E.143621,
E.143633, E.143771, E.143797, E.143816, E.143889, E.143924, E.143933,
E.143947, E.144136, E.144224, E.144306, E.144381, E.144410, E.144485,
E.144566, E.144671, E.144741, E.144935, E.145020, E.145632, E.145741,
E.145833, E.145888, E.145907, E.145908, E.145919, E.145990, E.146067,
E.146070, E.146281, E.146433, E.146457, E.146535, E.146701, E.146856,
E.146966, E.147044, E.147127, E.147130, E.147133, E.147140, E.147231,
E.147257, E.147403, E.147475, E.147548, E.147697, E.147724, E.148158,

E.148396, E.148488, E.148672, E.148737, E.148835, E.149182, E.149218,
E.149311, E.149480, E.149548, E.149646, E.150051, E.150593, E.150961,
E.150991, E.151092, E.151093, E.151247, E.151304, E.151491, E.151690,
E.151715, E.151726, E.151779, E.151806, E.152086, E.152207, E.152234,
E.152291, E.152359, E.152377, E.152409, E.152422, E.152582, E.152763,
E.152818, E.152942, E.153113, E.153140, E.153391, E.153904, E.153936,
E.154099, E.154127, E.154380, E.154639, E.154723, E.154781, E.154832,
E.154864, E.154889, E.154957, E.155368, E.155380, E.155508, E.155660,
E.155714, E.155959, E.155980, E.156006, E.156194, E.156282, E.156304,
E.156467, E.156515, E.156603, E.156650, E.156735, E.156976, E.157064,
E.157103, E.157502, E.157510, E.157538, E.157551, E.157637, E.157764,
E.157827, E.157992, E.158042, E.158290, E.158321, E.158485, E.158545,
E.158604, E.158669, E.158715, E.158747, E.158813, E.158863, E.158901,
E.158941, E.158987, E.159147, E.159184, E.159348, E.159363, E.159387,
E.159423, E.159658, E.159692, E.159761, E.159921, E.160049, E.160226,
E.160285, E.160294, E.160633, E.160685, E.160691, E.160789, E.160862,
E.160867, E.160948, E.160972, E.161202, E.161267, E.161649, E.161692,
E.161714, E.161813, E.161939, E.162069, E.162298, E.162385, E.162437,
E.162490, E.162613, E.162641, E.162694, E.162910, E.162975, E.163041,
E.163064, E.163110, E.163257, E.163468, E.163492, E.163530, E.163576,
E.163629, E.163644, E.163749, E.163755, E.163781, E.163825, E.163913,
E.163923, E.163930, E.163932, E.164045, E.164051, E.164053, E.164163,
E.164244, E.164270, E.164300, E.164309, E.164442, E.164488, E.164520,
E.164597, E.164749, E.164754, E.164828, E.164916, E.164919, E.164924,
E.165084, E.165119, E.165138, E.165215, E.165259, E.165264, E.165280,
E.165359, E.165410, E.165496, E.165637, E.165646, E.165661, E.165688,
E.165695, E.165699, E.165792, E.165807, E.165813, E.165898, E.165923,
E.165934, E.166263, E.166266, E.166329, E.166337, E.166341, E.166484,
E.166526, E.166596, E.166598, E.166710, E.166747, E.166833, E.166860,
E.166946, E.166971, E.167004, E.167085, E.167110, E.167113, E.167258,
E.167513, E.167552, E.167553, E.167604, E.167635, E.167642, E.167658,
E.167699, E.167744, E.167751, E.167766, E.167772, E.167799, E.167815,
E.167969, E.167978, E.167987, E.167996, E.168014, E.168036, E.168066,
E.168071, E.168148, E.168298, E.168393, E.168575, E.168653, E.168746,
E.168763, E.168769, E.168803, E.168916, E.169087, E.169093, E.169122,
E.169189, E.169213, E.169242, E.169410, E.169418, E.169562, E.169592,
E.169612, E.169710, E.169763, E.169789, E.169807, E.169826, E.169957,
E.170017, E.170027, E.170037, E.170088, E.170144, E.170275, E.170310,
E.170315, E.170348, E.170374, E.170381, E.170396, E.170421, E.170430,
E.170445, E.170549, E.170632, E.170703, E.170743, E.170837, E.170854,
E.170906, E.170927, E.170954, E.170959, E.171121, E.171155, E.171180,
E.171202, E.171262, E.171302, E.171345, E.171428, E.171488, E.171490,
E.171492, E.171540, E.171643, E.171680, E.171723, E.171793, E.171861,
E.171953, E.172115, E.172283, E.172345, E.172346, E.172466, E.172590,
E.172594, E.172653, E.172717, E.172725, E.172733, E.172831, E.172867,
E.172893, E.172939, E.173039, E.173230, E.173366, E.173473, E.173540,
E.173585, E.173599, E.173714, E.173726, E.173805, E.173809, E.173826,
E.173889, E.173898, E.173905, E.174021, E.174100, E.174332, E.174842,
E.174996, E.175063, E.175110, E.175166, E.175175, E.175182, E.175198,
E.175203, E.175216, E.175220, E.175334, E.175416, E.175602, E.175866,
E.175946, E.176102, E.176105, E.176155, E.176171, E.176371, E.176515,
E.176900, E.176971, E.176978, E.176994, E.177156, E.177239, E.177354,
E.177409, E.177425, E.177459, E.177542, E.177548, E.177565, E.177595,
E.177628, E.177674, E.177679, E.177694, E.177697, E.177731, E.177752,
E.177951, E.178026, E.178078, E.178104, E.178163, E.178175, E.178187,
E.178234, E.178381, E.178473, E.178741, E.178828, E.178950, E.179091,
E.179115, E.179119, E.179348, E.179388, E.179776, E.179796, E.179869,

| | |
|---|---|
| | E.179912, E.179981, E.180035, E.180198, E.180287, E.180318, E.180667, E.180869, E.180979, E.180998, E.181072, E.181163, E.181222, E.181234, E.181513, E.181523, E.181610, E.181773, E.181873, E.181885, E.181924, E.182013, E.182054, E.182217, E.182271, E.182318, E.182319, E.182512, E.182732, E.182795, E.182872, E.182890, E.182944, E.183048, E.183092, E.183098, E.183128, E.183207, E.183292, E.183431, E.183520, E.183684, E.183723, E.183785, E.183831, E.183856, E.184007, E.184047, E.184113, E.184156, E.184254, E.184363, E.184378, E.184470, E.184481, E.184508, E.184634, E.184661, E.184697, E.184708, E.184735, E.184840, E.184916, E.185043, E.185049, E.185122, E.185219, E.185359, E.185499, E.185554, E.185591, E.185619, E.185736, E.185860, E.185896, E.185945, E.185972, E.186198, E.186205, E.186376, E.186472, E.186575, E.186591, E.186660, E.186814, E.186834, E.186868, E.186889, E.187097, E.187323, E.187492, E.187634, E.187764, E.187792, E.187823, E.187837, E.187840, E.188021, E.188171, E.188186, E.188739, E.188771, E.188846, E.189060, E.189091, E.189143, E.189144, E.189221, E.189283, E.196236, E.196419, E.196436, E.196497, E.196504, E.196526, E.196591, E.196700, E.196743, E.196796, E.196812, E.196872, E.196975, E.196993, E.197081, E.197157, E.197217, E.197223, E.197299, E.197323, E.197353, E.197451, E.197479, E.197746, E.197779, E.197813, E.197837, E.197857, E.197872, E.197969, E.197976, E.198001, E.198033, E.198040, E.198087, E.198131, E.198156, E.198168, E.198205, E.198216, E.198231, E.198265, E.198366, E.198431, E.198455, E.198563, E.198586, E.198589, E.198712, E.198721, E.198732, E.198783, E.198793, E.198804, E.198807, E.198824, E.198841, E.198951, E.203301, E.203795, E.203813, E.203837, E.203879, E.203908, E.204231, E.204316, E.204389, E.204406, E.204560, E.204574 |
| Prostate Markers (Ensembl ENSG identifiers) | E.005893 (LAMP2), E.006756 (ARSD), E.010539 (ZNF200), E.014257 (ACPP), E.015133 (CCDC88C), E.018699 (TTC27), E.044115 (CTNNA1), E.048828 (FAM120A), E.051620 (HEBP2), E.056097 (ZFR), E.060339 (CCAR1), E.063241 (ISOC2), E.064489 (MEF2BNB-MEF2B), E.064886 (CHI3L2), E.066455 (GOLGA5), E.069535 (MAOB), E.072042 (RDH11), E.072071 (LPHN1), E.074047 (GLI2), E.076248 (UNG), E.076554 (TPD52), E.077147 (TM9SF3), E.077312 (SNRPA), E.081842 (PCDHA6), E.086717 (PPEF1), E.088888 (MAVS), E.088930 (XRN2), E.089902 (RCOR1), E.090612 (ZNF268), E.092199 (HNRNPC), E.095380 (NANS), E.099783 (HNRNPM), E.100191 (SLC5A4), E.100216 (TOMM22), E.100242 (SUN2), E.100284 (TOM1), E.100401 (RANGAP1), E.100412 (ACO2), E.100836 (PABPN1), E.102054 (RBBP7), E.102103 (PQBP1), E.103599 (IQCH), E.103978 (TMEM87A), E.104177 (MYEF2), E.104228 (TRIM35), E.105428 (ZNRF4), E.105518 (TMEM205), E.106603 (C7orf44; COA1), E.108405 (P2RX1), E.111057 (KRT18), E.111218 (PRMT8), E.112081 (SRSF3), E.112144 (ICK), E.113013 (HSPA9), E.113368 (LMNB1), E.115221 (ITGB6), E.116096 (SPR), E.116754 (SRSF11), E.118197 (DDX59), E.118898 (PPL), E.119121 (TRPM6), E.119711 (ALDH6A1), E.120656 (TAF12), E.121671 (CRY2), E.121774 (KHDRBS1), E.122126 (OCRL), E.122566 (HNRNPA2B1), E.123901 (GPR83), E.124562 (SNRPC), E.124788 (ATXN1), E.124795 (DEK), E.125246 (CLYBL), E.126883 (NUP214), E.127616 (SMARCA4), E.127884 (ECHS1), E.128050 (PAICS), E.129351 (ILF3), E.129757 (CDKN1C), E.130338 (TULP4), E.130612 (CYP2G1P), E.131351 (HAUS8), E.131467 (PSME3), E.133315 (MACROD1), E.134452 (FBXO18), E.134851 (TMEM165), E.135940 (COX5B), E.136169 (SETDB2), E.136807 (CDK9), E.137727 (ARHGAP20), E.138031 (ADCY3), E.138050 (THUMPD2), E.138069 (RAB1A), E.138594 (TMOD3), E.138760 (SCARB2), E.138796 (HADH), E.139613 (SMARCC2), E.139684 (ESD), E.140263 (SORD), E.140350 (ANP32A), E.140632 (GLYR1), E.142765 (SYTL1), E.143621 (ILF2), E.143933 (CALM2), E.144410 (CPO), E.147127 (RAB41), E.151304 (SRFBP1), E.151806 (GUF1), E.152207 (CYSLTR2), E.152234 (ATP5A1), E.152291 (TGOLN2), E.154723 (ATP5J), E.156467 (UQCRB), E.159387 (IRX6), E.159761 (C16orf86), E.161813 (LARP4), E.162613 (FUBP1), E.162694 (EXTL2), |

E.165264 (NDUFB6), E.167113 (COQ4), E.167513 (CDT1), E.167772 (ANGPTL4), E.167978 (SRRM2), E.168916 (ZNF608), E.169763 (PRYP3), E.169789 (PRY), E.169807 (PRY2), E.170017 (ALCAM), E.170144 (HNRNPA3), E.170310 (STX8), E.170954 (ZNF415), E.170959 (DCDC5), E.171302 (CANT1), E.171643 (S100Z), E.172283 (PRYP4), E.172590 (MRPL52), E.172867 (KRT2), E.173366 (TLR9), E.173599 (PC), E.177595 (PIDD), E.178473 (UCN3), E.179981 (TSHZ1), E.181163 (NPM1), E.182319 (Tyrosine-protein kinase SgK223), E.182795 (C1orf116), E.182944 (EWSR1), E.183092 (BEGAIN), E.183098 (GPC6), E.184254 (ALDH1A3), E.185619 (PCGF3), E.186889 (TMEM17), E.187837 (HIST1H1C), E.188771 (C11orf34), E.189060 (H1F0), E.196419 (XRCC6), E.196436 (NPIPL2), E.196504 (PRPF40A), E.196796, E.196993, E.197451 (HNRNPAB), E.197746 (PSAP), E.198131 (ZNF544), E.198156, E.198732 (SMOC1), E.198793 (MTOR), E.039068 (CDH1), E.173230 (GOLGB1), E.124193 (SRSF6), E.140497 (SCAMP2), E.168393 (DTYMK), E.184708 (EIF4ENIF1), E.124164 (VAPB), E.125753 (VASP), E.118260 (CREB1), E.135052 (GOLM1), E.010244 (ZNF207), E.010278 (CD9), E.047597 (XK), E.049246 (PER3), E.069849 (ATP1B3), E.072506 (HSD17B10), E.081138 (CDH7), E.099785 (MARCH2), E.104331 (IMPAD1), E.104812 (GYS1), E.120868 (APAF1), E.123908 (EIF2C2), E.125492 (BARHL1), E.127328 (RAB3IP), E.127329 (PTPRB), E.129514 (FOXA1), E.129657 (SEC14L1), E.129990 (SYT5), E.132881 (RSG1), E.136521 (NDUFB5), E.138347 (MYPN), E.141429 (GALNT1), E.144566 (RAB5A), E.151715 (TMEM45B), E.152582 (SPEF2), E.154957 (ZNF18), E.162385 (MAGOH), E.165410 (CFL2), E.168298 (HIST1H1E), E.169418 (NPR1), E.178187 (ZNF454), E.178741 (COX5A), E.179115 (FARSA), E.182732 (RGS6), E.183431 (SF3A3), E.185049 (WHSC2), E.196236 (XPNPEP3), E.197217 (ENTPD4), E.197813, E.203301, E.116833 (NR5A2), E.121057 (AKAP1), E.005471 (ABCB4), E.071859 (FAM50A), E.084234 (APLP2), E.101222 (SPEF1), E.103175 (WFDC1), E.103449 (SALL1), E.104805 (NUCB1), E.105514 (RAB3D), E.107816 (LZTS2), E.108375 (RNF43), E.109790 (KLHL5), E.112039 (FANCE), E.112715 (VEGFA), E.121690 (DEPDC7), E.125352 (RNF113A), E.134548 (C12orf39), E.136152 (COG3), E.143816 (WNT9A), E.147130 (ZMYM3), E.148396 (SEC16A), E.151092 (NGLY1), E.151779 (NBAS), E.155508 (CNOT8), E.163755 (HPS3), E.166526 (ZNF3), E.172733 (PURG), E.176371 (ZSCAN2), E.177674 (AGTRAP), E.181773 (GPR3), E.183048 (SLC25A10; MRPL12 SLC25A10), E.186376 (ZNF75D), E.187323 (DCC), E.198712 (MT-CO2), E.203908 (C6orf221; KHDC3L), E.001497 (LAS1L), E.009694 (ODZ1), E.080572 (CXorf41; PIH1D3), E.083093 (PALB2), E.089048 (ESF1), E.100065 (CARD10), E.100739 (BDKRB1), E.102904 (TSNAXIP1), E.104824 (HNRNPL), E.107404 (DVL1), E.110066 (SUV420H1), E.120328 (PCDHB12), E.121903 (ZSCAN20), E.122025 (FLT3), E.136930 (PSMB7), E.142025 (DMRTC2), E.144136 (SLC20A1), E.146535 (GNA12), E.147140 (NONO), E.153391 (INO80C), E.164919 (COX6C), E.171540 (OTP), E.177951 (BET1L), E.179796 (LRRC3B), E.197479 (PCDHB11), E.198804 (MT-CO1), E.086205 (FOLH1), E.100632 (ERH), E.100796 (SMEK1), E.104760 (FGL1), E.114302 (PRKAR2A), E.130299 (GTPBP3), E.133961 (NUMB), E.144485 (HES6), E.167085 (PHB), E.167635 (ZNF146), E.177239 (MAN1B1), E.184481 (FOXO4), E.188171 (ZNF626), E.189221 (MAOA), E.157637 (SLC38A10), E.100883 (SRP54), E.105618 (PRPF31), E.119421 (NDUFA8), E.170837 (GPR27), E.168148 (HIST3H3), E.135525 (MAP7), E.174996 (KLC2), E.018189 (RUFY3), E.183520 (UTP11L), E.173905 (GOLIM4), E.165280 (VCP), E.022976 (ZNF839), E.059691 (PET112), E.063244 (U2AF2), E.075651 (PLD1), E.089177 (KIF16B), E.089280 (FUS), E.094755 (GABRP), E.096060 (FKBP5), E.100023 (PPIL2), E.100359 (SGSM3), E.100612 (DHRS7), E.104131 (EIF3J), E.104419 (NDRG1), E.105409 (ATP1A3), E.107623 (GDF10), E.111335 (OAS2), E.113522 (RAD50), E.115053 (NCL), E.120837 (NFYB), E.122733 (KIAA1045), E.123178 (SPRYD7), E.124181 (PLCG1), E.126858 (RHOT1), E.128609 (NDUFA5), E.128683 (GAD1), E.130255 (RPL36), E.133874 (RNF122), E.135387 (CAPRIN1), E.135999 (EPC2), E.136383 (ALPK3), E.139405 (C12orf52),

E.141012 (GALNS), E.143924 (EML4), E.144671 (SLC22A14), E.145741 (BTF3), E.145907 (G3BP1), E.149311 (ATM), E.153113 (CAST), E.157538 (DSCR3), E.157992 (KRTCAP3), E.158901 (WFDC8), E.165259 (HDX), E.169410 (PTPN9), E.170421 (KRT8), E.171155 (C1GALT1C1), E.172831 (CES2), E.173726 (TOMM20), E.176515, E.177565 (TBL1XR1), E.177628 (GBA), E.179091 (CYC1), E.189091 (SF3B3), E.197299 (BLM), E.197872 (FAM49A), E.198205 (ZXDA), E.198455 (ZXDB), E.082212 (ME2), E.109956 (B3GAT1), E.169710 (FASN), E.011304 (PTBP1), E.057252 (SOAT1), E.059378 (PARP12), E.082258 (CCNT2), E.087301 (TXNDC16), E.100575 (TIMM9), E.101152 (DNAJC5), E.101812 (H2BFM), E.102384 (CENPI), E.108641 (B9D1), E.119138 (KLF9), E.119820 (YIPF4), E.125995 (ROMO1), E.132323 (ILKAP), E.134809 (TIMM10), E.134955 (SLC37A2), E.135476 (ESPL1), E.136527 (TRA2B), E.137776 (SLTM), E.139211 (AMIGO2), E.139428 (MMAB), E.139874 (SSTR1), E.143321 (HDGF), E.164244 (PRRC1), E.164270 (HTR4), E.165119 (HNRNPK), E.165637 (VDAC2), E.165661 (QSOX2), E.167258 (CDK12), E.167815 (PRDX2), E.168014 (C2CD3), E.168653 (NDUFS5), E.168769 (TET2), E.169242 (EFNA1), E.175334 (BANF1), E.175416 (CLTB), E.177156 (TALDO1), E.180035 (ZNF48), E.186591 (UBE2H), E.187097 (ENTPD5), E.188739 (RBM34), E.196497 (IPO4), E.197323 (TRIM33), E.197857 (ZNF44), E.197976 (AKAP17A), E.064201 (TSPAN32), E.088992 (TESC), E.092421 (SEMA6A), E.100601 (ALKBH1), E.101557 (USP14), E.103035 (PSMD7), E.106128 (GHRHR), E.115541 (HSPE1), E.121390 (PSPC1), E.124216 (SNAI1), E.130713 (EXOSC2), E.132002 (DNAJB1), E.139697 (SBNO1), E.140481 (CCDC33), E.143013 (LMO4), E.145020 (AMT), E.145990 (GFOD1), E.146070 (PLA2G7), E.164924 (YWHAZ), E.165807 (PPP1R36), E.167751 (KLK2), E.169213 (RAB3B), E.170906 (NDUFA3), E.172725 (CORO1B), E.174332 (GLIS1), E.181924 (CHCHD8), E.183128 (CALHM3), E.204560 (DHX16), E.204574 (ABCF1), E.146701 (MDH2), E.198366 (HIST1H3A), E.081181 (ARG2), E.185896 (LAMP1), E.077514 (POLD3), E.099800 (TIMM13), E.100299 (ARSA), E.105419 (MEIS3), E.108417 (KRT37), E.113739 (STC2), E.125868 (DSTN), E.145908 (ZNF300), E.168575 (SLC20A2), E.182271 (TMIGD1), E.197223 (C1D), E.186834 (HEXIM1), E.001561 (ENPP4), E.011451 (WIZ), E.053108 (FSTL4), E.064655 (EYA2), E.065308 (TRAM2), E.075131 (TIPIN), E.081087 (OSTM1), E.092020 (PPP2R3C), E.096384 (HSP90AB1), E.100348 (TXN2), E.100577 (GSTZ1), E.100802 (C14orf93), E.101365 (IDH3B), E.101654 (RNMT), E.103067 (ESRP2), E.104064 (GABPB1), E.104823 (ECH1), E.106565 (TMEM176B), E.108561 (C1QBP), E.115257 (PCSK4), E.116127 (ALMS1), E.117411 (B4GALT2), E.119335 (SET), E.120337 (TNFSF18), E.122033 (MTIF3), E.122507 (BBS9), E.122870 (BICC1), E.130177 (CDC16), E.130193 (C8orf55; THEM6), E.130413 (STK33), E.130770 (ATPIF1), E.133687 (TMTC1), E.136874 (STX17), E.137409 (MTCH1), E.139626 (ITGB7), E.141744 (PNMT), E.145888 (GLRA1), E.146067 (FAM193B), E.146433 (TMEM181), E.149480 (MTA2), E.152377 (SPOCK1), E.152763 (WDR78), E.156976 (EIF4A2), E.157827 (FMNL2), E.158485 (CD1B), E.158863 (FAM160B2), E.161202 (DVL3), E.161714 (PLCD3), E.163064 (EN1), E.163468 (CCT3), E.164309 (CMYA5), E.164916 (FOXK1), E.165215 (CLDN3), E.167658 (EEF2), E.170549 (IRX1), E.171680 (PLEKHG5), E.178234 (GALNT11), E.179869 (ABCA13), E.179912 (R3HDM2), E.180869 (C1orf180), E.180979 (LRRC57), E.182872 (RBM10), E.183207 (RUVBL2), E.184113 (CLDN5), E.185972 (CCIN), E.189144 (ZNF573), E.197353 (LYPD2), E.197779 (ZNF81), E.198807 (PAX9), E.100442 (FKBP3), E.111790 (FGFR1OP2), E.136044 (APPL2), E.061794 (MRPS35), E.065427 (KARS), E.068885 (IFT80), E.104164 (PLDN; BLOC1S6), E.105127 (AKAP8), E.123066 (MED13L), E.124831 (LRRFIP1), E.125304 (TM9SF2), E.126934 (MAP2K2), E.130305 (NSUN5), E.135298 (BAI3), E.135900 (MRPL44), E.136371 (MTHFS), E.136574 (GATA4), E.140326 (CDAN1), E.141378 (PTRH2), E.141543 (EIF4A3), E.150961 (SEC24D), E.155368 (DBI), E.161649 (CD300LG), E.161692 (DBF4B), E.162437 (RAVER2), E.163257 (DCAF16), E.163576 (EFHB), E.163781 (TOPBP1), E.163913 (IFT122), E.164597 (COG5), E.165359 (DDX26B), E.165646

(SLC18A2), E.169592 (INO80E), E.169957 (ZNF768), E.171492 (LRRC8D), E.171793 (CTPS; CTPS1), E.171953 (ATPAF2), E.175182 (FAM131A), E.177354 (C10orf71), E.181610 (MRPS23), E.181873 (IBA57), E.187792 (ZNF70), E.187823 (ZCCHC16), E.196872 (C2orf55; KIAA1211L), E.198168 (SVIP), E.160633 (SAFB), E.177697 (CD151), E.181072 (CHRM2), E.012779 (ALOX5), E.065054 (SLC9A3R2), E.074071 (MRPS34), E.100815 (TRIP11), E.102030 (NAA10), E.106153 (CHCHD2), E.126814 (TRMT5), E.126952 (NXF5), E.136450 (SRSF1), E.136710 (CCDC115), E.137124 (ALDH1B1), E.143353 (LYPLAL1), E.162490 (C1orf187; DRAXIN), E.167799 (NUDT8), E.171490 (RSL1D1), E.173826 (KCNH6), E.173898 (SPTBN2), E.176900 (OR51T1), E.181513 (ACBD4), E.185554 (NXF2), E.185945 (NXF2B), E.108848 (LUC7L3), E.029363 (BCLAF1), E.038002 (AGA), E.108312 (UBTF), E.166341 (DCHS1), E.054118 (THRAP3), E.135679 (MDM2), E.166860 (ZBTB39), E.183684 (THOC4; ALYREF), E.004838 (ZMYND10), E.007264 (MATK), E.020922 (MRE11A), E.041353 (RAB27B), E.052795 (FNIP2), E.075711 (DLG1), E.087087 (SRRT), E.090060 (PAPOLA), E.095139 (ARCN1), E.099715 (PCDH11Y), E.100271 (TTLL1), E.101057 (MYBL2), E.101265 (RASSF2), E.101901 (ALG13), E.102290 (PCDH11X), E.103194 (USP10), E.106554 (CHCHD3), E.107833 (NPM3), E.110063 (DCPS), E.111540 (RAB5B), E.113448 (PDE4D), E.115339 (GALNT3), E.116254 (CHD5), E.117425 (PTCH2), E.117614 (SYF2), E.118181 (RPS25), E.118292 (C1orf54), E.119318 (RAD23B), E.121022 (COPS5), E.121104 (FAM117A), E.123427 (METTL21B), E.125676 (THOC2), E.132275 (RRP8), E.137513 (NARS2), E.138028 (CGREF1), E.139517 (LNX2), E.143614 (GATAD2B), E.143889 (HNRPLL), E.145833 (DDX46), E.147403 (RPL10), E.148158 (SNX30), E.151690 (MFSD6), E.153904 (DDAH1), E.154781 (C3orf19), E.156650 (KAT6B), E.158669 (AGPAT6), E.159363 (ATP13A2), E.163530 (DPPA2), E.164749 (HNF4G), E.165496 (RPL10L), E.165688 (PMPCA), E.165792 (METTL17), E.166598 (HSP90B1), E.168036 (CTNNB1), E.168746 (C20orf62), E.170381 (SEMA3E), E.171180 (OR2M4), E.171202 (TMEM126A), E.172594 (SMPDL3A), E.172653 (C17orf66), E.173540 (GMPPB), E.173585 (CCR9), E.173809 (TDRD12), E.175166 (PSMD2), E.177694 (NAALADL2), E.178026 (FAM211B; C22orf36), E.184363 (PKP3), E.187634 (SAMD11), E.203837 (PNLIPRP3), E.169122 (FAM110B), E.197969 (VPS13A), E.136068 (FLNB), E.075856 (SART3), E.081721 (DUSP12), E.102158 (MAGT1), E.102174 (PHEX), E.102316 (MAGED2), E.104723 (TUSC3), E.105939 (ZC3HAV1), E.108883 (EFTUD2), E.110328 (GALNTL4), E.111785 (RIC8B), E.113163 (COL4A3BP), E.115604 (IL18R1), E.117362 (APH1A), E.117480 (FAAH), E.124767 (GLO1), E.126267 (COX6B1), E.130175 (PRKCSH), E.135926 (TMBIM1), E.138674 (SEC31A), E.140451 (PIF1), E.143797 (MBOAT2), E.149646 (C20orf152), E.157064 (NMNAT2), E.160294 (MCM3AP), E.165084 (C8orf34), E.166946 (CCNDBP1), E.170348 (TMED10), E.170703 (TTLL6), E.175198 (PCCA), E.180287 (PLD5), E.183292 (MIR5096), E.187492 (CDHR4), E.188846 (RPL14), E.015479 (MATR3), E.100823 (APEX1), E.090615 (GOLGA3), E.086062 (B4GALT1), E.138385 (SSB), E.140265 (ZSCAN29), E.140932 (CMTM2), E.167969 (ECI1), E.135486 (HNRNPA1), E.137497 (NUMA1), E.181523 (SGSH), E.099956 (SMARCB1), E.049883 (PTCD2), E.082556 (OPRK1), E.090674 (MCOLN1), E.107164 (FUBP3), E.108582 (CPD), E.109758 (HGFAC), E.111605 (CPSF6), E.115239 (ASB3), E.121892 (PDS5A), E.125844 (RRBP1), E.130826 (DKC1), E.132481 (TRIM47), E.135390 (ATP5G2), E.136875 (PRPF4), E.138621 (PPCDC), E.145632 (PLK2), E.150051 (MKX), E.153140 (CETN3), E.154127 (UBASH3B), E.156194 (PPEF2), E.163825 (RTP3), E.164053 (ATRIP), E.164442 (CITED2), E.168066 (SF1), E.170430 (MGMT), E.175602 (CCDC85B), E.177752 (YIPF7), E.182512 (GLRX5), E.188186 (C7orf59), E.198721 (ECI2), E.204389 (HSPA1A), E.010256 (UQCRC1), E.076043 (REXO2), E.102362 (SYTL4), E.161939 (C17orf49), E.173039 (RELA), E.014216 (CAPN1), E.054938 (CHRDL2), E.065526 (SPEN), E.070501 (POLB), E.078808 (SDF4), E.083720 (OXCT1), E.100084 (HIRA), E.101246 (ARFRP1), E.102241 (HTATSF1), E.103591

(AAGAB), E.104626 (ERI1), E.105221 (AKT2), E.105402 (NAPA), E.105705 (SUGP1), E.106346 (USP42), E.108639 (SYNGR2), E.110107 (PRPF19), E.112473 (SLC39A7), E.113282 (CLINT1), E.115234 (SNX17), E.115561 (CHMP3), E.119906 (FAM178A), E.120733 (KDM3B), E.125375 (ATP5S), E.125798 (FOXA2), E.127415 (IDUA), E.129810 (SGOL1), E.132382 (MYBBP1A), E.133313 (CNDP2), E.134077 (THUMPD3), E.134248 (HBXIP), E.135597 (REPS1), E.137814 (HAUS2), E.138041 (SMEK2), E.140382 (HMG20A), E.143578 (CREB3L4), E.144224 (UBXN4), E.144306 (SCRN3), E.144741 (SLC25A26), E.145919 (BOD1), E.146281 (PM20D2), E.152359 (POC5), E.152409 (JMY), E.154889 (MPPE1), E.157551 (KCNJ15), E.157764 (BRAF), E.158987 (RAPGEF6), E.162069 (CCDC64B), E.162910 (MRPL55), E.163749 (CCDC158), E.164045 (CDC25A), E.164300 (SERINC5), E.165898 (ISCA2), E.167987 (VPS37C), E.168763 (CNNM3), E.170374 (SP7), E.171488 (LRRC8C), E.178381 (ZFAND2A), E.180998 (GPR137C), E.182318 (ZSCAN22), E.198040 (ZNF84), E.198216 (CACNA1E), E.198265 (HELZ), E.198586 (TLK1), E.203795 (FAM24A), E.204231 (RXRB), E.123992 (DNPEP), E.184634 (MED12), E.181885 (CLDN7), E.186660 (ZFP91), E.126777 (KTN1), E.080823 (MOK), E.101811 (CSTF2), E.124570 (SERPINB6), E.148835 (TAF5), E.158715 (SLC45A3), E.110955 (ATP5B), E.127022 (CANX), E.142208 (AKT1), E.128881 (TTBK2), E.147231 (CXorf57), E.006210 (CX3CL1), E.009830 (POMT2), E.011114 (BTBD7), E.065057 (NTHL1), E.068724 (TTC7A), E.073584 (SMARCE1), E.079785 (DDX1), E.084463 (WBP11), E.091140 (DLD), E.099821 (POLRMT), E.101126 (ADNP), E.104442 (ARMC1), E.105486 (LIG1), E.110921 (MVK), E.113441 (LNPEP), E.115758 (ODC1), E.116726 (PRAMEF12), E.119681 (LTBP2), E.136933 (RABEPK), E.137815 (RTF1), E.138095 (LRPPRC), E.138294 (MSMB), E.141873 (SLC39A3), E.142698 (C1orf94), E.143390 (RFX5), E.148488 (ST8SIA6), E.148737 (TCF7L2), E.151491 (EPS8), E.152422 (XRCC4), E.154832 (CXXC1), E.158321 (AUTS2), E.159147 (DONSON), E.160285 (LSS), E.160862 (AZGP1), E.160948 (VPS28), E.160972 (PPP1R16A), E.165934 (CPSF2), E.167604 (NFKBID), E.167766 (ZNF83), E.168803 (ADAL), E.169612 (FAM103A1), E.171262 (FAM98B), E.172893 (DHCR7), E.173889 (PHC3), E.176971 (FIBIN), E.177548 (RABEP2), E.179119 (SPTY2D1), E.184378 (ACTRT3), E.184508 (HDDC3), E.185043 (CIB1), E.186814 (ZSCAN30), E.186868 (MAPT), E.196812 (ZSCAN16), E.198563 (DDX39B), E.124529 (HIST1H4B), E.141002 (TCF25), E.174100 (MRPL45), E.109814 (UGDH), E.138756 (BMP2K), E.065457 (ADAT1), E.105948 (TTC26), E.109184 (DCUN1D4), E.125257 (ABCC4), E.126062 (TMEM115), E.142515 (KLK3), E.144381 (HSPD1), E.166710 (B2M), E.198824 (CHAMP1), E.078902 (TOLLIP), E.099331 (MYO9B), E.102710 (FAM48A), E.107485 (GATA3), E.120948 (TARDBP), E.187764 (SEMA4D), E.103855 (CD276), E.117751 (PPP1R8), E.173714 (WFIKKN2), E.172115 (CYCS), E.005882 (PDK2), E.007952 (NOX1), E.008118 (CAMK1G), E.012061 (ERCC1), E.015171 (ZMYND11), E.036257 (CUL3), E.057608 (GDI2), E.058729 (RIOK2), E.071246 (VASH1), E.073050 (XRCC1), E.073350 (LLGL2), E.079246 (XRCC5), E.085733 (CTTN), E.091542 (ALKBH5), E.091732 (ZC3HC1), E.092621 (PHGDH), E.099899 (TRMT2A), E.099917 (MED15), E.101439 (CST3), E.103479 (RBL2), E.104611 (SH2D4A), E.105281 (SLC1A5), E.106392 (C1GALT1), E.107104 (KANK1), E.107798 (LIPA), E.108296 (CWC25), E.109572 (CLCN3), E.112110 (MRPL18), E.113790 (EHHADH), E.115648 (MLPH), E.117308 (GALE), E.117335 (CD46), E.118513 (MYB), E.118640 (VAMP8), E.119321 (FKBP15), E.122705 (CLTA), E.123983 (ACSL3), E.124232 (RBPJL), E.125901 (MRPS26), E.127399 (LRRC61), E.127554 (GFER), E.128708 (HAT1), E.129355 (CDKN2D), E.130340 (SNX9), E.130935 (NOL11), E.131771 (PPP1R1B), E.133863 (TEX15), E.134207 (SYT6), E.136935 (GOLGA1), E.141425 (RPRD1A), E.143374 (TARS2), E.143771 (CNIH4), E.146966 (DENND2A), E.148672 (GLUD1), E.150593 (PDCD4), E.153936 (HS2ST1), E.154099 (DNAAF1), E.156006 (NAT2), E.156282 (CLDN17), E.158545 (ZC3H18), E.158604 (TMED4), E.158813 (EDA), E.159184 (HOXB13), E.161267 (BDH1), E.163492 (CCDC141), E.163629 (PTPN13),

E.164163 (ABCE1), E.164520 (RAET1E), E.165138 (ANKS6), E.165923 (AGBL2), E.166484 (MAPK7), E.166747 (AP1G1), E.166971 (AKTIP), E.167744 (NTF4), E.168071 (CCDC88B), E.169087 (HSPBAP1), E.170396 (ZNF804A), E.170445 (HARS), E.170632 (ARMC10), E.170743 (SYT9), E.171428 (NAT1), E.172346 (CSDC2), E.173805 (HAP1), E.175175 (PPM1E), E.175203 (DCTN2), E.177542 (SLC25A22), E.177679 (SRRM3), E.178828 (RNF186), E.182013 (PNMAL1), E.182054 (IDH2), E.182890 (GLUD2), E.184156 (KCNQ3), E.184697 (CLDN6), E.184735 (DDX53), E.184840 (TMED9), E.185219 (ZNF445), E.186198 (SLC51B), E.186205 (MOSC1; MARC1), E.189143 (CLDN4), E.196700 (ZNF512B), E.196743 (GM2A), E.198087 (CD2AP), E.198951 (NAGA), E.204406 (MBD5), E.002330 (BAD), E.105404 (RABAC1), E.114127 (XRN1), E.117713 (ARID1A), E.123143 (PKN1), E.130764 (LRRC47), E.131773 (KHDRBS3), E.137806 (NDUFAF1), E.142864 (SERBP1), E.158747 (NBL1), E.175063 (UBE2C), E.178104 (PDE4DIP), E.186472 (PCLO), E.069956 (MAPK6), E.112941 (PAPD7), E.116604 (MEF2D), E.142875 (PRKACB), E.147133 (TAF1), E.157510 (AFAP1L1), E.006625 (GGCT), E.155980 (KIF5A), E.134444 (KIAA1468), E.107968 (MAP3K8), E.117592 (PRDX6), E.123154 (WDR83), E.135297 (MTO1), E.135829 (DHX9), E.149548 (CCDC15), E.152086 (TUBA3E), E.167553 (TUBA1C), E.169826 (CSGALNACT2), E.171121 (KCNMB3), E.198033 (TUBA3C), E.147724 (FAM135B), E.170854 (MINA), E.006695 (COX10), E.067369 (TP53BP1), E.089248 (ERP29), E.112096 (SOD2), E.138073 (PREB), E.146856 (AGBL3), E.159423 (ALDH4A1), E.171345 (KRT19), E.172345 (STARD5), E.111647 (UHRF1BP1L), E.117877 (CD3EAP), E.155714 (PDZD9), E.156603 (MED19), E.075886 (TUBA3D), E.167699 (GLOD4), E.121749 (TBC1D15), E.090861 (AARS), E.093010 (COMT), E.117676 (RPS6KA1), E.157502 (MUM1L1), E.159921 (GNE), E.169562 (GJB1), E.179776 (CDH5), E.071626 (DAZAP1), E.085224 (ATRX), E.116478 (HDAC1), E.117298 (ECE1), E.176171 (BNIP3), E.177425 (PAWR), E.179348 (GATA2), E.187840 (EIF4EBP1), E.033030 (ZCCHC8), E.049239 (H6PD), E.060688 (SNRNP40), E.075239 (ACAT1), E.095627 (TDRD1), E.109625 (CPZ), E.113719 (ERGIC1), E.126773 (C14orf135; PCNXL4), E.149218 (ENDOD1), E.162975 (KCNF1), E.183785 (TUBA8), E.198589 (LRBA), E.105379 (ETFB), E.011052 (NME2), E.011143 (MKS1), E.048544 (MRPS10), E.062485 (CS), E.114054 (PCCB), E.138587 (MNS1), E.155959 (VBP1), E.181222 (POLR2A), E.183723 (CMTM4), E.184661 (CDCA2), E.204316 (MRPL38), E.140694 (PARN), E.035141 (FAM136A), E.095485 (CWF19L1), E.115540 (MOB4), E.123595 (RAB9A), E.140678 (ITGAX), E.141258 (SGSM2), E.158941 (KIAA1967), E.169189 (NSMCE1), E.198431 (TXNRD1), E.016402 (IL20RA), E.112234 (FBXL4), E.125445 (MRPS7), E.128342 (LIF), E.164051 (CCDC51), E.175866 (BAIAP2), E.102780 (DGKH), E.203813 (HIST1H3H), E.198231 (DDX42), E.030582 (GRN), E.106049 (HIBADH), E.130810 (PPAN), E.132475 (H3F3B), E.158290 (CUL4B), E.166266 (CUL5), E.026559 (KCNG1), E.059122 (FLYWCH1), E.107897 (ACBD5), E.121068 (TBX2), E.125944 (HNRNPR), E.134308 (YWHAQ), E.137558 (PI15), E.137601 (NEK1), E.147548 (WHSC1L1), E.149182 (ARFGAP2), E.159658 (KIAA0494), E.165699 (TSC1), E.170927 (PKHD1), E.186575 (NF2), E.188021 (UBQLN2), E.167552 (TUBA1A), E.003756 (RBM5), E.134138 (MEIS2), E.008196 (TFAP2B), E.079313 (REXO1), E.089127 (OAS1), E.106078 (COBL), E.113645 (WWC1), E.116288 (PARK7), E.121940 (CLCC1), E.136280 (CCM2), E.141639 (MAPK4), E.147475 (ERLIN2), E.155660 (PDIA4), E.162298 (SYVN1), E.176978 (DPP7), E.176994 (SMCR8), E.178175 (ZNF366), E.196591 (HDAC2), E.127824 (TUBA4A), E.163932 (PRKCD), E.143375 (CGN), E.076864 (RAP1GAP), E.138772 (ANXA3), E.163041 (H3F3A), E.165813 (C10orf118), E.166337 (TAF10), E.178078 (STAP2), E.184007 (PTP4A2), E.167004 (PDIA3), E.039560 (RAI14), E.119636 (C14orf45), E.140374 (ETFA), E.143633 (C1orf131), E.144935 (TRPC1), E.156735 (BAG4), E.159348 (CYB5R1), E.170275 (CRTAP), E.172717 (FAM71D), E.172939 (OXSR1), E.176105 (YES1), E.078295 (ADCY2), E.119888 (EPCAM), E.141522 (ARHGDIA), E.184047 (DIABLO), E.109062

(SLC9A3R1), E.170037 (CNTROB), E.066557 (LRRC40), E.074964 (ARHGEF10L), E.078269 (SYNJ2), E.090013 (BLVRB), E.100142 (POLR2F), E.100399 (CHADL), E.104365 (IKBKB), E.111261 (MANSC1), E.111907 (TPD52L1), E.112578 (BYSL), E.121957 (GPSM2), E.122884 (P4HA1), E.124693 (HIST1H3B), E.126653 (NSRP1), E.130402 (ACTN4), E.138757 (G3BP2), E.150991 (UBC), E.164828 (SUN1), E.175216 (CKAP5), E.176155 (CCDC57), E.177459 (C8orf47), E.183856 (IQGAP3), E.185122 (HSF1), E.122952 (ZWINT), E.151093 (OXSM), E.067704 (IARS2), E.088899 (ProSAP-interacting protein 1), E.091483 (FH), E.114388 (NPRL2), E.114861 (FOXP1), E.135914 (HTR2B), E.197837 (HIST4H4), E.127720 (C12orf26; METTL25), E.123416 (TUBA1B), E.047410 (TPR), E.117748 (RPA2), E.133835 (HSD17B4), E.067248 (DHX29), E.121879 (PIK3CA), E.132589 (FLOT2), E.136750 (GAD2), E.160789 (LMNA), E.166329, E.170088 (TMEM192), E.175946 (KLHL38), E.178163 (ZNF518B), E.182217 (HIST2H4B), E.184470 (TXNRD2), E.110321 (EIF4G2), E.171861 (RNMTL1), E.065978 (YBX1), E.115738 (ID2), E.143294 (PRCC), E.158042 (MRPL17), E.169093 (ASMTL), E.090565 (RAB11FIP3), E.185591 (SP1), E.156304 (SCAF4), E.092978 (GPATCH2), E.100056 (DGCR14), E.100583 (SAMD15), E.105723 (GSK3A), E.107551 (RASSF4), E.107581 (EIF3A), E.107890 (ANKRD26), E.110104 (CCDC86), E.112584 (FAM120B), E.113580 (NR3C1), E.114491 (UMPS), E.137312 (FLOT1), E.137955 (RABGGTB), E.141994 (DUS3L), E.147044 (CASK), E.152818 (UTRN), E.180667 (YOD1), E.184916 (JAG2), E.196526 (AFAP1), E.198783 (ZNF830), E.108465 (CDK5RAP3), E.156515 (HK1), E.036448 (MYOM2), E.061918 (GUCY1B3), E.070785 (EIF2B3), E.116044 (NFE2L2), E.128311 (TST), E.131473 (ACLY), E.132716 (DCAF8), E.138363 (ATIC), E.166596 (WDR16), E.170027 (YWHAG), E.174021 (GNG5), E.203879 (GDI1), E.160049 (DFFA), E.010810 (FYN), E.051596 (THOC3), E.006453 (BAI1-associated protein 2-like 1), E.126945 (HNRNPH2), E.165695 (AK8), E.069869 (NEDD4), E.111801 (BTN3A3), E.112232 (KHDRBS2), E.128626 (MRPS12), E.129636 (ITFG1), E.137948 (BRDT), E.147257 (GPC3), E.155380 (SLC16A1), E.159692 (CTBP1), E.166833 (NAV2), E.172466 (ZNF24), E.175110 (MRPS22), E.176102 (CSTF3), E.179388 (EGR3), E.185359 (HGS), E.198001 (IRAK4), E.100603 (SNW1), E.162641 (AKNAD1), E.069712 (KIAA1107), E.073756 (PTGS2), E.077522 (ACTN2), E.101639 (CEP192), E.106633 (GCK), E.115241 (PPM1G), E.116649 (SRM), E.120370 (GORAB), E.124143 (ARHGAP40), E.127948 (POR), E.129315 (CCNT1), E.132646 (PCNA), E.135740 (SLC9A5), E.151726 (ACSL1), E.154380 (ENAH), E.157103 (SLC6A1), E.163930 (BAP1), E.164488 (DACT2), E.164754 (RAD21), E.175220 (ARHGAP1), E.180318 (ALX1), E.181234 (TMEM132C), E.197081 (IGF2R), E.092871 (RFFL), E.163644 (PPM1K), E.171723 (GPHN), E.108953 (YWHAE), E.072110 (ACTN1), E.077097 (TOP2B), E.090889 (KIF4A), E.114331 (ACAP2), E.114867 (EIF4G1), E.117593 (DARS2), E.118523 (CTGF), E.120915 (EPHX2), E.134759 (ELP2), E.138061 (CYP1B1), E.140743 (CDR2), E.151247 (EIF4E), E.152942 (RAD17), E.160685 (ZBTB7B), E.163923 (RPL39L), E.167642 (SPINT2), E.167996 (FTH1), E.185736 (ADARB2), E.198841 (KTI12), E.185860 (C1orf110), E.160226 (C21orf2), E.070814 (TCOF1), E.124749 (COL21A1), E.154639 (CXADR), E.065485 (PDIA5), E.023909 (GCLM), E.100714 (MTHFD1), E.108387 (SEPT4), E.160867 (FGFR4), E.134684 (YARS), E.123080 (CDKN2C), E.065548 (ZC3H15), E.116455 (WDR77), E.117448 (AKR1A1), E.100393 (EP300), E.138160 (KIF11), E.166263 (STXBP4), E.173473 (SMARCC1), E.124942 (AHNAK), E.174842 (GLMN), E.180198 (RCC1), E.185499 (MUC1), E.143947 (RPS27A), E.170315 (UBB), E.003402 (CFLAR), E.137055 (PLAA), E.142606 (MMEL1), E.147697 (GSDMC), E.163110 (PDLIM5), E.135842 (FAM129A), E.160691 (SHC1), E.197157 (SND1), E.029725 (RABEP1), E.127946 (HIP1), E.001036 (FUCA2), E.109846 (CRYAB), E.183831 (ANKRD45), E.189283 (FHIT), E.092820 (EZR), E.104067 (TJP1), E.120159 (C9orf82; CAAP1), E.154864 (PIEZO2), E.196975 (ANXA4), E.105220 (GPI), E.127914 (AKAP9), E.135870 (RC3H1), E.026508 (CD44), E.089154 (GCN1L1), E.100311 (PDGFB),

| | |
|---|---|
| | E.119383 (PPP2R4), E.075624 (ACTB), E.177409 (SAMD9L), E.177731 (FLII), E.015676 (NUDCD3), E.146457 (WTAP), E.178950 (GAK), E.167110 (GOLGA2) |
| Prostate vesicle | LAMP2, ACPP, CTNNA1, HEBP2, ISOC2, HNRNPC, HNRNPM, TOMM22, TOM1, ACO2, KRT18, HSPA9, LMNB1, SPR, PPL, ALDH6A1, HNRNPA2B1, ATXN1, SMARCA4, ECHS1, PAICS, ILF3, PSME3, COX5B, RAB1A, SCARB2, HADH, ESD, SORD, ILF2, CALM2, ATP5A1, TGOLN2, ANGPTL4, ALCAM, KRT2, PC, NPM1, C1orf116, GPC6, ALDH1A3, HIST1H1C, XRCC6, HNRNPAB, PSAP, CDH1, SCAMP2, VASP, CD9, ATP1B3, HSD17B10, APAF1, EIF2C2, RAB5A, CFL2, FARSA, XPNPEP3, ENTPD4, APLP2, NUCB1, RAB3D, VEGFA, HPS3, TSNAXIP1, HNRNPL, PSMB7, GNA12, NONO, FOLH1, PRKAR2A, PHB, HIST3H3, MAP7, VCP, U2AF2, FUS, FKBP5, NDRG1, ATP1A3, NCL, RPL36, KRT8, C1GALT1C1, FASN, PTBP1, TXNDC16, DNAJC5, SLC37A2, HNRNPK, VDAC2, PRDX2, TALDO1, USP14, PSMD7, HSPE1, DNAJB1, YWHAZ, RAB3B, CORO1B, MDH2, HIST1H3A, LAMP1, STC2, DSTN, SLC20A2, ENPP4, WIZ, HSP90AB1, IDH3B, ECH1, C1QBP, SET, TNFSF18, ITGB7, SPOCK1, EIF4A2, CCT3, CLDN3, EEF2, LRRC57, RUVBL2, CLDN5, APPL2, TM9SF2, EIF4A3, DBI, DBF4B, SVIP, CD151, ALOX5, SLC9A3R2, RAB27B, DLG1, ARCN1, CHCHD3, RAB5B, RPS25, RPL10, DDAH1, HSP90B1, CTNNB1, PSMD2, PKP3, FLNB, EFTUD2, GLO1, PRKCSH, TMBIM1, SEC31A, TMED10, RPL14, MATR3, APEX1, B4GALT1, HNRNPA1, CPD, HSPA1A, CAPN1, CHRDL2, SPEN, SDF4, NAPA, SYNGR2, CHMP3, CNDP2, CCDC64B, SERINC5, VPS37C, DNPEP, CLDN7, KTN1, SERPINB6, ATP5B, CANX, AKT1, TTBK2, DDX1, DLD, LNPEP, LTBP2, LRPPRC, EPS8, AZGP1, VPS28, DHCR7, CIB1, DDX39B, HIST1H4B, UGDH, HSPD1, B2M, TOLLIP, CD276, CYCS, CUL3, GDI2, LLGL2, XRCC5, CTTN, PHGDH, CST3, RBL2, SLC1A5, CD46, VAMP8, CLTA, ACSL3, MRPS26, SNX9, GLUD1, TMED4, PTPN13, AP1G1, SYT9, DCTN2, IDH2, GLUD2, TMED9, CLDN4, GM2A, CD2AP, MBD5, SERBP1, NBL1, PRKACB, GGCT, PRDX6, DHX9, TUBA3E, TUBA1C, TUBA3C, ERP29, SOD2, KRT19, TUBA3D, AARS, COMT, MUM1L1, CDH5, ECE1, ACAT1, ENDOD1, TUBA8, ETFB, NME2, CS, VBP1, RAB9A, TXNRD1, LIF, BAIAP2, HIST1H3H, GRN, HIBADH, H3F3B, CUL4B, HNRNPR, YWHAQ, PKHD1, TUBA1A, PARK7, ERLIN2, PDIA4, TUBA4A, PRKCD, ANXA3, H3F3A, PTP4A2, PDIA3, ETFA, CYB5R1, CRTAP, OXSR1, YES1, EPCAM, ARHGDIA, DIABLO, SLC9A3R1, BLVRB, P4HA1, HIST1H3B, ACTN4, UBC, FH, HIST4H4, TUBA1B, HSD17B4, PIK3CA, FLOT2, LMNA, TMEM192, HIST2H4B, YBX1, EIF3A, FLOT1, UTRN, HK1, ACLY, ATIC, YWHAG, GNG5, GDI1, HNRNPH2, NEDD4, BTN3A3, SLC16A1, HGS, ACTN2, SRM, PCNA, ACSL1, RAD21, ARHGAP1, IGF2R, YWHAE, ACTN1, EIF4G1, EPHX2, EIF4E, FTH1, CXADR, MTHFD1, AKR1A1, STXBP4, AHNAK, MUC1, RPS27A, UBB, PDLIM5, FAM129A, SND1, FUCA2, CRYAB, EZR, TJP1, ANXA4, GPI, AKAP9, CD44, GCN1L1, ACTB, FLII, NUDCD3 |
| Prostate Cancer vesicles | EGFR, GLUD2, ANXA3, APLP2, BclG, Cofilin 2 /cfL2, DCTN-50 / DCTN2, DDAH1, ESD, FARSLA, GITRL, PRKCSH, SLC20A2, Synaptogyrin 2 /SYNGR2, TM9SF2, Calnexin, TOMM22, NDRG1, RPL10, RPL14, USP14, VDAC2, LLGL2, CD63, CD81, uPAR / CD87, ADAM 9, BDKRB2, CCR5, CCT2 (TCP1-beta), PSMA, PSMA1, HSPB1, VAMP8, Rab1A, B4GALT1, Aspartyl Aminopeptidase /Dnpep, ATPase Na+/K+ beta 3/ATP1B3, BDNF, ATPB, beta 2 Microglobulin, Calmodulin 2 /CALM2, CD9, XRCC5 / Ku80, SMARCA4, TOM1, Cytochrome C, Hsp10 / HSPE1, COX2 / PTGS2, Claudin 4 /CLDN4, Cytokeratin 8, Cortactin/CTTN, DBF4B /DRF1, ECH1, ECHS1, GOLPH2, ETS1, DIP13B /appl2, EZH2 / KMT6, GSTP1, hK2 / Kif2a, IQGAP1, KLK13, Lamp-2, GM2A, Hsp40/DNAJB1, HADH/HADHSC, Hsp90B, Nucleophosmin, p130 /RBL2, PHGDH, RAB3B, ANXA1, PSMD7, PTBP1, Rab5a, SCARB2, Stanniocalcin 2 /STC2, TGN46 /TGOLN2, TSNAXIP1, ANXA2, CD46, KLK14, IL1alpha, hnRNP C1 + C2, hnRNP A1, hnRNP A2B1, Claudin 5, CORO1B, Integrin beta 7, CD41, CD49d, CDH2, COX5b, IDH2, ME1, PhIP, ALDOA, EDNRB/EDN3, MTA1, NKX3-1, TMPRSS2, CD10, CD24, CDH1, ADAM10, B7H3, CD276, CHRDL2, |

| | |
|---|---|
| | SPOCK1, VEGFA, BCHE, CD151, CD166/ALCAM, CSE1L, GPC6, CXCR3, GAL3, GDF15, IGFBP-2, HGF, KLK12, ITGAL, KLK7, KLK9, MMP 2, MMP 25, MMP10, TNFRI, Notch1, PAP - same as ACPP, PTPN13/PTPL1, seprase/FAP, TNFR1, TWEAK, VEGFR2, E-Cadherin, Hsp60, CLDN3- Claudin3, KLK6, KLK8, EDIL3 (del-1), APE1, MMP 1, MMP3, nAnS, PSP94 / MSP / IGBF, PSAP, RPL19, SET, TGFB, TGM2, TIMP-1, TNFRII, MDH2, PKP1, Cystatin C, Trop2 / TACSTD2, CCR2 / CD192, hnRNP M1-M4, CDKN1A, CGA, Cytokeratin 18, EpoR, GGPS1, FTL (light and heavy), GM-CSF, HSP90AA1, IDH3B, MKI67/Ki67, LTBP2, KLK1, KLK4, KLK5, LDH-A, Nav1.7/SCN9A, NRP1 / CD304, PIP3 / BPNT1, PKP3, CgA, PRDX2, SRVN, ATPase Na+/K+ alpha 3/ATP1A3, SLC3A2 / CD98, U2AF2, TLR4 (CD284), TMPRSS1, TNFα, uPA, GloI, ALIX, PKM2, FABP5, CAV1, TLR9 / CD289, ANXA4, PLEKHC1 / Kindlin-2, CD71 / TRFR, MBD5, SPEN/ RBM15, LGALS8, SLC9A3R2, ENTPD4, ANGPTL4, p97 / VCP, TBX5, PTEN, Prohibitin, LSP1, HOXB13, DDX1, AKT1, ARF6, EZR, H3F3A, CIB1, Ku70 (XRCC6), KLK11, TMBIM6, SYT9, APAF1, CLDN7, MATR3, CD90/THY1, Tollip, NOTCH4, 14-3-3 zeta/beta, ATP5A1, DLG1, GRP94, FKBP5/FKBP51, LAMP1, LGALS3BP, GDI2, HSPA1A, NCL, KLK15, Cytokeratin basic, EDN-3, AGR2, KLK10, BRG1, FUS, Histone H4, hnRNP L, Catenin Alpha 1, hnRNP K (F45)*, MMP7*, DBI*, beta catenin, CTH, CTNND2, Ataxin 1, Proteasome 20S beta 7, ADE2, EZH2, GSTP1, Lamin B1, Coatomer Subunit Delta, ERAB, Mortalin, PKM2, IGFBP-3, CTNND1 / delta 1-catenin / p120-catenin, PKA R2, NONO, Sorbitol Dehydrogenase, Aconitase 2, VASP, Lipoamide Dehydrogenase, AP1G1, GOLPH2, ALDH6A1, AZGP1, Ago2, CNDP2, Nucleobindin-1, SerpinB6, RUVBL2, Proteasome 19S 10B, SH3PX1, SPR, Destrin, MDM4, FLNB, FASN, PSME |
| Prostate Cancer vesicles | 14-3-3 zeta/beta, Aconitase 2, ADAM 9, ADAM10, ADE2, AFM, Ago2, AGR2, AKT1, ALDH1A3, ALDH6A1, ALDOA, ALIX, ANGPTL4, ANXA1, ANXA2, ANXA3, ANXA3, ANXA4, AP1G1, APAF1, APE1, APLP2, APLP2, ARF6, Aspartyl Aminopeptidase /Dnpep, Ataxin 1, ATP5A1, ATPase Na+/K+ alpha 3/ATP1A3, ATPase Na+/K+ beta 3/ATP1B3, ATPase Na+/K+ beta 3/ATP1B3, ATPB, AZGP1, B4GALT1, B7H3, BCHE, BclG, BDKRB2, BDNF, BDNF, beta 2 Microglobulin, beta catenin, BRG1, CALM2, Calmodulin 2 /CALM2, Calnexin, Calpain 1, Catenin Alpha 1, CAV1, CCR2 / CD192, CCR5, CCT2 (TCP1-beta), CD10, CD151, CD166/ALCAM, CD24, CD276, CD41, CD46, CD49d, CD63, CD71 / TRFR, CD81, CD9, CD9, CD90/THY1, CDH1, CDH2, CDKN1A, CGA, CgA, CHRDL2, CIB1, CIB1, Claudin 4 /CLDN4, Claudin 5, CLDN3, CLDN3- Claudin3, CLDN4, CLDN7, CNDP2, Coatomer Subunit Delta, Cofilin 2 /cfL2, CORO1B, Cortactin/CTTN, COX2 / PTGS2, COX5b, CSE1L, CTH, CTNND1 / delta 1-catenin / p120-catenin, CTNND2, CXCR3, CYCS, Cystatin C, Cytochrome C, Cytokeratin 18, Cytokeratin 8, Cytokeratin basic, DBF4B /DRF1, DBI*, DCTN-50 / DCTN2, DDAH1, DDAH1, DDX1, Destrin, DIP13B /appl2, DIP13B /appl2, DLG1, Dnpep, E-Cadherin, ECH1, ECHS1, ECHS1, EDIL3 (del-1), EDN-3, EDNRB/EDN3, EGFR, EIF4A3, ENTPD4, EpoR, EpoR, ERAB, ESD, ESD, ETS1, ETS1, ETS-2, EZH2, EZH2 / KMT6, EZR, FABP5, FARSLA, FASN, FKBP5/FKBP51, FLNB, FTL (light and heavy), FUS, GAL3, gamma-catenin, GDF15, GDI2, GGPS1, GGPS1, GITRL, GloI, GLUD2, GM2A, GM-CSF, GOLM1/GOLPH2 Mab; clone 3B10, GOLPH2, GOLPH2, GPC6, GRP94, GSTP1, GSTP1, H3F3A, HADH/HADHSC, HGF, HIST1H3A, Histone H4, hK2 / Kif2a, hnRNP A1, hnRNP A2B1, hnRNP C1 + C2, hnRNP K (F45)*, hnRNP L, hnRNP M1-M4, HOXB13, Hsp10 / HSPE1, Hsp40/DNAJB1, Hsp60, HSP90AA1, Hsp90B, HSPA1A, HSPB1, IDH2, IDH3B, IDH3B, IGFBP-2, IGFBP-3, IgG1, IgG2A, IgG2B, IL1alpha, IL1alpha, Integrin beta 7, IQGAP1, ITGAL, KLHL12/C3IP1, KLK1, KLK10, KLK11, KLK12, KLK13, KLK14, KLK15, KLK4, KLK5, KLK6, KLK7, KLK8, KLK9, Ku70 (XRCC6), Lamin B1, LAMP1, Lamp-2, LDH-A, LGALS3BP, LGALS8, Lipoamide Dehydrogenase, LLGL2, LSP1, LSP1, LTBP2, MATR3, MBD5, MDH2, MDM4, ME1, MKI67/Ki67, MMP 1, MMP 2, MMP 25, MMP10, MMP-14/MT1-MMP, MMP3, MMP7*, Mortalin, MTA1, nAnS, nAnS, |

| | |
|---|---|
| | Nav1.7/SCN9A, NCL, NDRG1, NKX3-1, NONO, Notch1, NOTCH4, NRP1 / CD304, Nucleobindin-1, Nucleophosmin, p130 /RBL2, p97 / VCP, PAP - same as ACPP, PHGDH, PhIP, PIP3 / BPNT1, PKA R2, PKM2, PKM2, PKP1, PKP3, PLEKHC1 / Kindlin-2, PRDX2, PRKCSH, Prohibitin, Proteasome 19S 10B, Proteasome 20S beta 7, PSAP, PSMA, PSMA1, PSMA1, PSMD7, PSMD7, PSME3, PSP94 / MSP / IGBF, PTBP1, PTEN, PTPN13/PTPL1, Rab1A, RAB3B, Rab5a, Rad51b, RPL10, RPL10, RPL14, RPL14, RPL19, RUVBL2, SCARB2, seprase/FAP, SerpinB6, SET, SH3PX1, SLC20A2, SLC3A2 / CD98, SLC9A3R2, SMARCA4, Sorbitol Dehydrogenase, SPEN/ RBM15, SPOCK1, SPR, SRVN, Stanniocalcin 2 /STC2, STEAP1, Synaptogyrin 2 /SYNGR2, Syndecan, SYNGR2, SYT9, TAF1B / GRHL1, TBX5, TGFB, TGM2, TGN46 /TGOLN2, TIMP-1, TLR3, TLR4 (CD284), TLR9 / CD289, TM9SF2, TMBIM6, TMPRSS1, TMPRSS2, TNFR1, TNFRI, TNFRII, TNFSF18 / GITRL, TNFα, TNFα, Tollip, TOM1, TOMM22, Trop2 / TACSTD2, TSNAXIP1, TWEAK, U2AF2, uPA, uPAR / CD87, USP14, USP14, VAMP8, VASP, VDAC2, VEGFA, VEGFR1/FLT1, VEGFR2, VPS28, XRCC5 / Ku80, XRCC5 / Ku80 |
| Prostate Vesicles / General Vesicles | EpCAM/TROP-1, HSA, Fibrinogen, GAPDH, Cholesterol Oxidase, MMP7, Complement Factor D/Adipsin, E-Cadherin, Transferrin Antibody, eNOS, IgM, CD9, Apolipoprotein B (Apo B), Ep-CAM, TBG, Kallekerin 3, IgA, IgG, Annexin V, IgG, Pyruvate Carboxylase, trypsin, AFP, TNF RI/TNFRSF1A, Aptamer CAR023, Aptamer CAR024, Aptamer CAR025, Aptamer CAR026 |
| Ribonucleoprotein complexes & vesicles | GW182, Ago2, miR-let-7a, miR-16, miR-22, miR-148a, miR-451, miR-92a, CD9, CD63, CD81 |
| Prostate Cancer vesicles | PCSA, Muc2, Adam10 |
| Prostate Cancer vesicles | Alkaline Phosphatase (AP), CD63, MyoD1, Neuron Specific Enolase, MAP1B, CNPase, Prohibitin, CD45RO, Heat Shock Protein 27, Collagen II, Laminin B1/b1, Gai1, CDw75, bcl-XL, Laminin-s, Ferritin, CD21, ADP-ribosylation Factor (ARF-6) |
| Prostate Cancer vesicles | CD56/NCAM-1, Heat Shock Protein 27/hsp27, CD45RO, MAP1B, MyoD1, CD45/T200/LCA, CD3zeta, Laminin-s, bcl-XL, Rad18, Gai1, Thymidylate Synthase, Alkaline Phosphatase (AP), CD63, MMP-16 / MT3-MMP, Cyclin C, Neuron Specific Enolase, SIRP a1, Laminin B1/b1, Amyloid Beta (APP), SODD (Silencer of Death Domain), CDC37, Gab-1, E2F-2, CD6, Mast Cell Chymase, Gamma Glutamylcysteine Synthetase (GCS) |
| Prostate Cancer vesicles | EpCAM, MMP7, PCSA, BCNP, ADAM10, KLK2, SPDEF, CD81, MFGE8, IL-8 |
| Prostate Cancer vesicles | EpCAM, KLK2, PBP, SPDEF, SSX2, SSX4 |
| Prostate Cancer vesicles | ADAM-10, BCNP, CD9, EGFR, EpCam, IL1B, KLK2, MMP7, p53, PBP, PCSA, SERPINB3, SPDEF, SSX2, SSX4 |
| Androgen Receptor (AR) pathway members in cMVs | GTF2F1, CTNNB1, PTEN, APPL1, GAPDH, CDC37, PNRC1, AES, UXT, RAN, PA2G4, JUN, BAG1, UBE2I, HDAC1, COX5B, NCOR2, STUB1, HIPK3, PXN, NCOA4 |
| EGFR1 pathway members in cMVs | RALBP1, SH3BGRL, RBBP7, REPS1, SNRPD2, CEBPB, APPL1, MAP3K3, EEF1A1, GRB2, RAC1, SNCA, MAP2K3, CEBPA, CDC42, SH3KBP1, CBL, PTPN6, YWHAB, FOXO1, JAK1, KRT8, RALGDS, SMAD2, VAV1, NDUFA13, PRKCB1, MYC, JUN, RFXANK, HDAC1, HIST3H3, PEBP1, PXN, TNIP1, PKN2 |
| TNF-alpha pathway members in cMVs | BCL3, SMARCE1, RPS11, CDC37, RPL6, RPL8, PAPOLA, PSMC1, CASP3, AKT2, MAP3K7IP2, POLR2L, TRADD, SMARCA4, HIST3H3, GNB2L1, PSMD1, PEBP1, HSPB1, TNIP1, RPS13, ZFAND5, YWHAQ, COMMD1, COPS3, POLR1D, SMARCC2, MAP3K3, BIRC3, UBE2D2, HDAC2, CASP8, MCM7, PSMD7, YWHAG, NFKBIA, CAST, YWHAB, G3BP2, PSMD13, FBL, RELB, YWHAZ, SKP1, UBE2D3, PDCD2, HSP90AA1, HDAC1, KPNA2, RPL30, GTF2I, PFDN2 |

| Colorectal cancer | CD9, EGFR, NGAL, CD81, STEAP, CD24, A33, CD66E, EPHA2, Ferritin, GPR30, GPR110, MMP9, OPN, p53, TMEM211, TROP2, TGM2, TIMP, EGFR, DR3, UNC93A, MUC17, EpCAM, MUC1, MUC2, TSG101, CD63, B7H3 |
|---|---|
| Colorectal cancer | DR3, STEAP, epha2, TMEM211, unc93A, A33, CD24, NGAL, EpCam, MUC17, TROP2, TETS |
| Colorectal cancer | A33, AFP, ALIX, ALX4, ANCA, APC, ASCA, AURKA, AURKB, B7H3, BANK1, BCNP, BDNF, CA-19-9, CCSA-2, CCSA-3&4, CD10, CD24, CD44, CD63, CD66 CEA, CD66e CEA, CD81, CD9, CDA, C-Erb2, CRMP-2, CRP, CRTN, CXCL12, CYFRA21-1, DcR3, DLL4, DR3, EGFR, Epcam, EphA2, FASL, FRT, GAL3, GDF15, GPCR (GPR110), GPR30, GRO-1, HBD 1, HBD2, HNP1-3, IL-1B, IL8, IMP3, L1CAM, LAMN, MACC-1, MGC20553, MCP-1, M-CSF, MIC1, MIF, MMP7, MMP9, MS4A1, MUC1, MUC17, MUC2, Ncam, NGAL, NNMT, OPN, p53, PCSA, PDGFRB, PRL, PSMA, PSME3, Reg IV, SCRN1, Sept-9, SPARC, SPON2, SPR, SRVN, TFF3, TGM2, TIMP-1, TMEM211, TNF-alpha, TPA, TPS, Trail-R2, Trail-R4, TrKB, TROP2, Tsg 101, TWEAK, UNC93A, VEGFA |
| Colorectal cancer | miR 92, miR 21, miR 9, miR 491 |
| Colorectal cancer | miR-127-3p, miR-92a, miR-486-3p, miR-378 |
| Colorectal cancer | TMEM211, MUC1, CD24 and/or GPR110 (GPCR 110) |
| Colorectal cancer | hsa-miR-376c, hsa-miR-215, hsa-miR-652, hsa-miR-582-5p, hsa-miR-324-5p, hsa-miR-1296, hsa-miR-28-5p, hsa-miR-190, hsa-miR-590-5p, hsa-miR-202, hsa-miR-195 |
| Colorectal cancer vesicle markers | A26C1A, A26C1B, A2M, ACAA2, ACE, ACOT7, ACP1, ACTA1, ACTA2, ACTB, ACTBL2, ACTBL3, ACTC1, ACTG1, ACTG2, ACTN1, ACTN2, ACTN4, ACTR3, ADAM10, ADSL, AGR2, AGR3, AGRN, AHCY, AHNAK, AKR1B10, ALB, ALDH16A1, ALDH1A1, ALDOA, ANXA1, ANXA11, ANXA2, ANXA2P2, ANXA4, ANXA5, ANXA6, AP2A1, AP2A2, APOA1, ARF1, ARF3, ARF4, ARF5, ARF6, ARHGDIA, ARPC3, ARPC5L, ARRDC1, ARVCF, ASCC3L1, ASNS, ATP1A1, ATP1A2, ATP1A3, ATP1B1, ATP4A, ATP5A1, ATP5B, ATP5I, ATP5L, ATP5O, ATP6AP2, B2M, BAIAP2, BAIAP2L1, BRI3BP, BSG, BUB3, C1orf58, C5orf32, CAD, CALM1, CALM2, CALM3, CAND1, CANX, CAPZA1, CBR1, CBR3, CCT2, CCT3, CCT4, CCT5, CCT6A, CCT7, CCT8, CD44, CD46, CD55, CD59, CD63, CD81, CD82, CD9, CDC42, CDH1, CDH17, CEACAM5, CFL1, CFL2, CHMP1A, CHMP2A, CHMP4B, CKB, CLDN3, CLDN4, CLDN7, CLIC1, CLIC4, CLSTN1, CLTC, CLTCL1, CLU, COL12A1, COPB1, COPB2, CORO1C, COX4I1, COX5B, CRYZ, CSPG4, CSRP1, CST3, CTNNA1, CTNNB1, CTNND1, CTTN, CYFIP1, DCD, DERA, DIP2A, DIP2B, DIP2C, DMBT1, DPEP1, DPP4, DYNC1H1, EDIL3, EEF1A1, EEF1A2, EEF1AL3, EEF1G, EEF2, EFNB1, EGFR, EHD1, EHD4, EIF3EIP, EIF3I, EIF4A1, EIF4A2, ENO1, ENO2, ENO3, EPHA2, EPHA5, EPHB1, EPHB2, EPHB3, EPHB4, EPPK1, ESD, EZR, F11R, F5, F7, FAM125A, FAM125B, FAM129B, FASLG, FASN, FAT, FCGBP, FER1L3, FKBP1A, FLNA, FLNB, FLOT1, FLOT2, G6PD, GAPDH, GARS, GCN1L1, GDI2, GK, GMDS, GNA13, GNAI2, GNAI3, GNAS, GNB1, GNB2, GNB2L1, GNB3, GNB4, GNG12, GOLGA7, GPA33, GPI, GPRC5A, GSN, GSTP1, H2AFJ, HADHA, hCG_1757335, HEPH, HIST1H2AB, HIST1H2AE, HIST1H2AJ, HIST1H2AK, HIST1H4A, HIST1H4B, HIST1H4C, HIST1H4D, HIST1H4E, HIST1H4F, HIST1H4H, HIST1H4I, HIST1H4J, HIST1H4K, HIST1H4L, HIST2H2AC, HIST2H4A, HIST2H4B, HIST3H2A, HIST4H4, HLA-A, HLA-A29.1, HLA-B, HLA-C, HLA-E, HLA-H, HNRNPA2B1, HNRNPH2, HPCAL1, HRAS, HSD17B4, HSP90AA1, HSP90AA2, HSP90AA4P, HSP90AB1, HSP90AB2P, HSP90AB3P, HSP90B1, HSPA1A, HSPA1B, HSPA1L, HSPA2, HSPA4, HSPA5, HSPA6, HSPA7, HSPA8, HSPA9, HSPD1, HSPE1, HSPG2, HYOU1, IDH1, IFITM1, IFITM2, IFITM3, IGH@, IGHG1, IGHG2, IGHG3, IGHG4, IGHM, IGHV4-31, IGK@, IGKC, IGKV1-5, IGKV2-24, IGKV3-20, IGSF3, IGSF8, IQGAP1, IQGAP2, ITGA2, ITGA3, ITGA6, ITGAV, ITGB1, ITGB4, JUP, KIAA0174, KIAA1199, KPNB1, KRAS, KRT1, KRT10, KRT13, KRT14, KRT15, KRT16, KRT17, KRT18, KRT19, KRT2, KRT20, KRT24, KRT25, KRT27, KRT28, KRT3, KRT4, KRT5, KRT6A, KRT6B, KRT6C, KRT7, |

| | KRT75, KRT76, KRT77, KRT79, KRT8, KRT9, LAMA5, LAMP1, LDHA, LDHB, LFNG, LGALS3, LGALS3BP, LGALS4, LIMA1, LIN7A, LIN7C, LOC100128936, LOC100130553, LOC100133382, LOC100133739, LOC284889, LOC388524, LOC388720, LOC442497, LOC653269, LRP4, LRPPRC, LRSAM1, LSR, LYZ, MAN1A1, MAP4K4, MARCKS, MARCKSL1, METRNL, MFGE8, MICA, MIF, MINK1, MITD1, MMP7, MOBKL1A, MSN, MTCH2, MUC13, MYADM, MYH10, MYH11, MYH14, MYH9, MYL6, MYL6B, MYO1C, MYO1D, NARS, NCALD, NCSTN, NEDD4, NEDD4L, NME1, NME2, NOTCH1, NQO1, NRAS, P4HB, PCBP1, PCNA, PCSK9, PDCD6, PDCD6IP, PDIA3, PDXK, PEBP1, PFN1, PGK1, PHB, PHB2, PKM2, PLEC1, PLEKHB2, PLSCR3, PLXNA1, PLXNB2, PPIA, PPIB, PPP2R1A, PRDX1, PRDX2, PRDX3, PRDX5, PRDX6, PRKAR2A, PRKDC, PRSS23, PSMA2, PSMC6, PSMD11, PSMD3, PSME3, PTGFRN, PTPRF, PYGB, QPCT, QSOX1, RAB10, RAB11A, RAB11B, RAB13, RAB14, RAB15, RAB1A, RAB1B, RAB2A, RAB33B, RAB35, RAB43, RAB4B, RAB5A, RAB5B, RAB5C, RAB6A, RAB6B, RAB7A, RAB8A, RAB8B, RAC1, RAC3, RALA, RALB, RAN, RANP1, RAP1A, RAP1B, RAP2A, RAP2B, RAP2C, RDX, REG4, RHOA, RHOC, RHOG, ROCK2, RP11-631M21.2, RPL10A, RPL12, RPL6, RPL8, RPLP0, RPLP0-like, RPLP1, RPLP2, RPN1, RPS13, RPS14, RPS15A, RPS16, RPS18, RPS20, RPS21, RPS27A, RPS3, RPS4X, RPS4Y1, RPS4Y2, RPS7, RPS8, RPSA, RPSAP15, RRAS, RRAS2, RUVBL1, RUVBL2, S100A10, S100A11, S100A14, S100A16, S100A6, S100P, SDC1, SDC4, SDCBP, SDCBP2, SERINC1, SERINC5, SERPINA1, SERPINF1, SETD4, SFN, SLC12A2, SLC12A7, SLC16A1, SLC1A5, SLC25A4, SLC25A5, SLC25A6, SLC29A1, SLC2A1, SLC3A2, SLC44A1, SLC7A5, SLC9A3R1, SMPDL3B, SNAP23, SND1, SOD1, SORT1, SPTAN1, SPTBN1, SSBP1, SSR4, TACSTD1, TAGLN2, TBCA, TCEB1, TCP1, TF, TFRC, THBS1, TJP2, TKT, TMED2, TNFSF10, TNIK, TNKS1BP1, TNPO3, TOLLIP, TOMM22, TPI1, TPM1, TRAP1, TSG101, TSPAN1, TSPAN14, TSPAN15, TSPAN6, TSPAN8, TSTA3, TTYH3, TUBA1A, TUBA1B, TUBA1C, TUBA3C, TUBA3D, TUBA3E, TUBA4A, TUBA4B, TUBA8, TUBB, TUBB2A, TUBB2B, TUBB2C, TUBB3, TUBB4, TUBB4Q, TUBB6, TUFM, TXN, UBA1, UBA52, UBB, UBC, UBE2N, UBE2V2, UGDH, UQCRC2, VAMP1, VAMP3, VAMP8, VCP, VIL1, VPS25, VPS28, VPS35, VPS36, VPS37B, VPS37C, WDR1, YWHAB, YWHAE, YWHAG, YWHAH, YWHAQ, YWHAZ |
|---|---|
| Colorectal Cancer | hsa-miR-16, hsa-miR-25, hsa-miR-125b, hsa-miR-451, hsa-miR-200c, hsa-miR-140-3p, hsa-miR-658, hsa-miR-370, hsa-miR-1296, hsa-miR-636, hsa-miR-502-5p |
| Breast cancer | miR-21, miR-155, miR-206, miR-122a, miR-210, miR-21, miR-155, miR-206, miR-122a, miR-210, let-7, miR-10b, miR-125a, miR-125b, miR-145, miR-143, miR-145, miR-1b |
| Breast cancer | GAS5 |
| Breast cancer | ER, PR, HER2, MUC1, EGFR, KRAS, B-Raf, CYP2D6, hsp70, MART-1, TRP, HER2, hsp70, MART-1, TRP, HER2, ER, PR, Class III b-tubulin, VEGFA, ETV6-NTRK3, BCA-225, hsp70, MART1, ER, VEGFA, Class III b-tubulin, HER2/neu (e.g., for Her2+ breast cancer), GPR30, ErbB4 (JM) isoform, MPR8, MISIIR, CD9, EphA2, EGFR, B7H3, PSM, PCSA, CD63, STEAP, CD81, ICAM1, A33, DR3, CD66e, MFG-E8, TROP-2, Mammaglobin, Hepsin, NPGP/NPFF2, PSCA, 5T4, NGAL, EpCam, neurokinin receptor-1 (NK-1 or NK-1R), NK-2, Pai-1, CD45, CD10, HER2/ERBB2, AGTR1, NPY1R, MUC1, ESA, CD133, GPR30, BCA225, CD24, CA15.3 (MUC1 secreted), CA27.29 (MUC1 secreted), NMDAR1, NMDAR2, MAGEA, CTAG1B, NY-ESO-1, SPB, SPC, NSE, PGP9.5, progesterone receptor (PR) or its isoform (PR(A) or PR(B)), P2RX7, NDUFB7, NSE, GAL3, osteopontin, CHI3L1, IC3b, mesothelin, SPA, AQP5, GPCR, hCEA-CAM, PTP IA-2, CABYR, TMEM211, ADAM28, UNC93A, MUC17, MUC2, IL10R-beta, BCMA, HVEM/TNFRSF14, Trappin-2, Elafin, ST2/IL1 R4, TNFRF14, CEACAM1, TPA1, LAMP, WF, WH1000, PECAM, BSA, TNFR |
| Breast cancer | CD9, MIS Rii, ER, CD63, MUC1, HER3, STAT3, VEGFA, BCA, CA125, CD24, EPCAM, ERB B4 |

| | |
|---|---|
| Breast cancer | CD10, NPGP/NPFF2, HER2/ERBB2, AGTR1, NPY1R, neurokinin receptor-1 (NK-1 or NK-1R), NK-2, MUC1, ESA, CD133, GPR30, BCA225, CD24, CA15.3 (MUC1 secreted), CA27.29 (MUC1 secreted), NMDAR1, NMDAR2, MAGEA, CTAG1B, NY-ESO-1 |
| Breast cancer | SPB, SPC, NSE, PGP9.5, CD9, P2RX7, NDUFB7, NSE, GAL3, osteopontin, CHI3L1, EGFR, B7H3, IC3b, MUC1, mesothelin, SPA, PCSA, CD63, STEAP, AQP5, CD81, DR3, PSM, GPCR, EphA2, hCEA-CAM, PTP IA-2, CABYR, TMEM211, ADAM28, UNC93A, A33, CD24, CD10, NGAL, EpCam, MUC17, TROP-2, MUC2, IL10R-beta, BCMA, HVEM/TNFRSF14, Trappin-2 Elafin, ST2/IL1 R4, TNFRF14, CEACAM1, TPA1, LAMP, WF, WH1000, PECAM, BSA, TNFR |
| Breast cancer | BRCA, MUC-1, MUC 16, CD24, ErbB4, ErbB2 (HER2), ErbB3, HSP70, Mammaglobin, PR, PR(B), VEGFA |
| Breast cancer | CD9, HSP70, Gal3, MIS, EGFR, ER, ICB3, CD63, B7H4, MUC1, DLL4, CD81, ERB3, VEGF, BCA225, BRCA, CA125, CD174, CD24, ERB2, NGAL, GPR30, CYFRA21, CD31, cMET, MUC2, ERBB4 |
| Breast cancer | CD9, EphA2, EGFR, B7H3, PSMA, PCSA, CD63, STEAP, CD81, STEAP1, ICAM1 (CD54), PSMA, A33, DR3, CD66e, MFG-8e, TMEM211, TROP-2, EGFR, Mammoglobin, Hepsin, NPGP/NPFF2, PSCA, 5T4, NGAL, NK-2, EpCam, NK-1R, PSMA, 5T4, PAI-1, CD45 |
| Breast cancer | PGP9.5, CD9, HSP70, gal3-b2c10, EGFR, iC3b, PSMA, PCSA, CD63, MUC1, DLL4, CD81, B7-H3, HER 3 (ErbB3), MART-1, PSA, VEGF A, TIMP-1, GPCR GPR110, EphA2, MMP9, mmp7, TMEM211, UNC93a, BRCA, CA125 (MUC16), Mammaglobin, CD174 (Lewis y), CD66e CEA, CD24 c.sn3, C-erbB2, CD10, NGAL, epcam, CEA (carcinoembryonic Antigen), GPR30, CYFRA21-1, OPN, MUC17, hVEGFR2, MUC2, NCAM, ASPH, ErbB4, SPB, SPC, CD9, MS4A1, EphA2, MIS RII, HER2 (ErbB2), ER, PR (B), MRP8, CD63, B7H4, TGM2, CD81, DR3, STAT 3, MACC-1, TrKB, IL 6 Unc, OPG - 13, IL6R, EZH2, SCRN1, TWEAK, SERPINB3, CDAC1, BCA-225, DR3, A33, NPGP/NPFF2, TIMP1, BDNF, FRT, Ferritin heavy chain, seprase, p53, LDH, HSP, ost, p53, CXCL12, HAP, CRP, Gro-alpha, Tsg 101, GDF15 |
| Breast cancer | CD9, HSP70, Gal3, MIS (RII), EGFR, ER, ICB3, CD63, B7H4, MUC1, CD81, ERB3, MART1, STAT3, VEGF, BCA225, BRCA, CA125, CD174, CD24, ERB2, NGAL, GPR30, CYFRA21, CD31, cMET, MUC2, ERB4, TMEM211 |
| Breast Cancer | 5T4 (trophoblast), ADAM10, AGER/RAGE, APC, APP (β-amyloid), ASPH (A-10), B7H3 (CD276), BACE1, BAI3, BRCA1, BDNF, BIRC2, C1GALT1, CA125 (MUC16), Calmodulin 1, CCL2 (MCP-1), CD9, CD10, CD127 (IL7R), CD174, CD24, CD44, CD63, CD81, CEA, CRMP-2, CXCR3, CXCR4, CXCR6, CYFRA 21, derlin 1, DLL4, DPP6, E-CAD, EpCAM, EphA2 (H-77), ER(1) ESR1 α, ER(2) ESR2 β, Erb B4, Erbb2, erb3 (Erb-B3), PA2G4, FRT (FLT1), Gal3, GPR30 (G-coupled ER1), HAP1, HER3, HSP-27, HSP70, IC3b, IL8, insig, junction plakoglobin, Keratin 15, KRAS, Mammaglobin, MART1, MCT2, MFGE8, MMP9, MRP8, Muc1, MUC17, MUC2, NCAM, NG2 (CSPG4), Ngal, NHE-3, NT5E (CD73), ODC1, OPG, OPN, p53, PARK7, PCSA, PGP9.5 (PARK5), PR(B), PSA, PSMA, RAGE, STXBP4, Survivin, TFF3 (secreted), TIMP1, TIMP2, TMEM211, TRAF4 (scaffolding), TRAIL-R2 (death Receptor 5), TrkB, Tsg 101, UNC93a, VEGF A, VEGFR2, YB-1, VEGFR1, GCDPF-15 (PIP), BigH3 (TGFb1-induced protein), 5HT2B (serotonin receptor 2B), BRCA2, BACE 1, CDH1-cadherin |
| Breast Cancer | AK5.2, ATP6V1B1, CRABP1 |
| Breast Cancer | DST.3, GATA3, KRT81 |
| Breast Cancer | AK5.2, ATP6V1B1, CRABP1, DST.3, ELF5, GATA3, KRT81, LALBA, OXTR, RASL10A, SERHL, TFAP2A.1, TFAP2A.3, TFAP2C, VTCN1 |
| Breast Cancer | TRAP; Renal Cell Carcinoma; Filamin; 14.3.3, Pan; Prohibitin; c-fos; Ang-2; GSTmu; Ang-1; FHIT; Rad51; Inhibin alpha; Cadherin-P; 14.3.3 gamma; p18INK4c; P504S; XRCC2; Caspase 5; CREB-Binding Protein; Estrogen Receptor; IL17; Claudin 2; Keratin 8; GAPDH; CD1; Keratin, LMW; Gamma Glutamylcysteine Synthetase(GCS)/Glutamate-cysteine Ligase; a-B-Crystallin; Pax- |

| | |
|---|---|
| | 5; MMP-19; APC; IL-3; Keratin 8 (phospho-specific Ser73); TGF-beta 2; ITK; Oct-2/; DJ-1; B7-H2; Plasma Cell Marker; Rad18; Estriol; Chk1; Prolactin Receptor; Laminin Receptor; Histone H1; CD45RO; GnRH Receptor; IP10/CRG2; Actin, Muscle Specific; S100; Dystrophin; Tubulin-a; CD3zeta; CDC37; GABA a Receptor 1; MMP-7 (Matrilysin); Heregulin; Caspase 3; CD56/NCAM-1; Gastrin 1; SREBP-1 (Sterol Regulatory Element Binding Protein-1); MLH1; PGP9.5; Factor VIII Related Antigen; ADP-ribosylation Factor (ARF-6); MHC II (HLA-DR) Ia; Survivin; CD23; G-CSF; CD2; Calretinin; Neuron Specific Enolase; CD165; Calponin; CD95 / Fas; Urocortin; Heat Shock Protein 27/hsp27; Topo II beta; Insulin Receptor; Keratin 5/8; sm; Actin, skeletal muscle; CA19-9; GluR1; GRIP1; CD79a mb-1; TdT; HRP; CD94; CCK-8; Thymidine Phosphorylase; CD57; Alkaline Phosphatase (AP); CD59 / MACIF / MIRL / Protectin; GLUT-1; alpha-1-antitrypsin; Presenillin; Mucin 3 (MUC3); pS2; 14-3-3 beta; MMP-13 (Collagenase-3); Fli-1; mGluR5; Mast Cell Chymase; Laminin B1/b1; Neurofilament (160kDa); CNPase; Amylin Peptide; Gai1; CD6; alpha-1-antichymotrypsin; E2F-2; MyoD1 |
| Ductal carcinoma in situ (DCIS) | Laminin B1/b1; E2F-2; TdT; Apolipoprotein D; Granulocyte; Alkaline Phosphatase (AP); Heat Shock Protein 27/hsp27; CD95 / Fas; pS2; Estriol; GLUT-1; Fibronectin; CD6; CCK-8; sm; Factor VIII Related Antigen; CD57; Plasminogen; CD71 / Transferrin Receptor; Keratin 5/8; Thymidine Phosphorylase; CD45/T200/LCA; Epithelial Specific Antigen; Macrophage; CD10; MyoD1; Gai1; bcl-XL; hPL; Caspase 3; Actin, skeletal muscle; IP10/CRG2; GnRH Receptor; p35nck5a; ADP-ribosylation Factor (ARF-6); Cdk4 ; alpha-1-antitrypsin; IL17; Neuron Specific Enolase; CD56/NCAM-1; Prolactin Receptor; Cdk7; CD79a mb-1; Collagen IV; CD94; Myeloid Specific Marker; Keratin 10; Pax-5; IgM (m-Heavy Chain); CD45RO; CA19-9; Mucin 2; Glucagon; Mast Cell Chymase; MLH1; CD1; CNPase; Parkin; MHC II (HLA-DR) Ia; B7-H2; Chk1; Lambda Light Chain; MHC II (HLA-DP and DR); Myogenin; MMP-7 (Matrilysin); Topo II beta; CD53; Keratin 19; Rad18; Ret Oncoprotein; MHC II (HLA-DP); E3-binding protein (ARM1); Progesterone Receptor; Keratin 8; IgG; IgA; Tubulin; Insulin Receptor Substrate-1; Keratin 15; DR3; IL-3; Keratin 10/13; Cyclin D3; MHC I (HLA25 and HLA-Aw32); Calmodulin; Neurofilament (160kDa) |
| Ductal carcinoma in situ (DCIS) v. other Breast cancer | Macrophage; Fibronectin; Granulocyte; Keratin 19; Cyclin D3; CD45/T200/LCA; EGFR; Thrombospondin; CD81/TAPA-1; Ruv C; Plasminogen; Collagen IV; Laminin B1/b1; CD10; TdT; Filamin; bcl-XL; 14.3.3 gamma; 14.3.3, Pan; p170; Apolipoprotein D; CD71 / Transferrin Receptor; FHIT |
| Breast cancer | 5HT2B, 5T4 (trophoblast), ACO2, ACSL3, ACTN4, ADAM10, AGR2, AGR3, ALCAM, ALDH6A1, ANGPTL4, ANO9, AP1G1, APC, APEX1, APLP2, APP (_-amyloid), ARCN1, ARHGAP35, ARL3, ASAH1, ASPH (A-10), ATP1B1, ATP1B3, ATP5I, ATP5O, ATXN1, B7H3, BACE1, BAI3, BAIAP2, BCA-200, BDNF, BigH3, BIRC2, BLVRB, BRCA, BST2, C1GALT1, C1GALT1C1, C20orf3, CA125, CACYBP, Calmodulin, CAPN1, CAPNS1, CCDC64B, CCL2 (MCP-1), CCT3, CD10(BD), CD127 (IL7R), CD174, CD24, CD44, CD80, CD86, CDH1, CDH5, CEA, CFL2, CHCHD3, CHMP3, CHRDL2, CIB1, CKAP4, COPA, COX5B, CRABP2, CRIP1, CRISPLD1, CRMP-2, CRTAP, CTLA4, CUL3, CXCR3, CXCR4, CXCR6, CYB5B, CYB5R1, CYCS, CYFRA 21, DBI, DDX23, DDX39B, derlin 1, DHCR7, DHX9, DLD, DLL4, DNAJB1, DPP6, DSTN, eCadherin, EEF1D, EEF2, EFTUD2, EIF4A2, EIF4A3, EpCAM, EphA2, ER(1) ESR1 _, ER(2) ESR2 _, Erb B4, Erb2, erb3 (Erb-B3?), ERLIN2, ESD, FARSA, FASN, FEN1, FKBP5, FLNB, FOXP3, FUS, Gal3, GCDPF-15, GCNT2, GNA12, GNG5, GNPTG, GPC6, GPD2, GPER (GPR30), GSPT1, H3F3B, H3F3C, HADH, HAP1, HER3, HIST1H1C, HIST1H2AB, HIST1H3A, HIST1H3C, HIST1H3D, HIST1H3E, HIST1H3F, HIST1H3G, HIST1H3H, HIST1H3I, HIST1H3J, HIST2H2BF, HIST2H3A, HIST2H3C, HIST2H3D, HIST3H3, HMGB1, HNRNPA2B1, HNRNPAB, HNRNPC, HNRNPD, HNRNPH2, HNRNPK, HNRNPL, HNRNPM, HNRNPU, HPS3, HSP-27, HSP70, HSP90B1, HSPA1A, HSPA2, HSPA9, HSPE1, IC3b, IDE, IDH3B, IDO1, IFI30, IL1RL2, IL7, IL8, ILF2, ILF3, IQCG, ISOC2, IST1, ITGA7, ITGB7, junction plakoglobin, Keratin 15, KRAS, KRT19, KRT2, |

| | |
|---|---|
| | KRT7, KRT8, KRT9, KTN1, LAMP1, LMNA, LMNB1, LNPEP, LRPPRC, LRRC57, Mammaglobin, MAN1A1, MAN1A2, MART1, MATR3, MBD5, MCT2, MDH2, MFGE8, MFGE8, MGP, MMP9, MRP8, MUC1, MUC17, MUC2, MYO5B, MYOF, NAPA, NCAM, NCL, NG2 (CSPG4), Ngal, NHE-3, NME2, NONO, NPM1, NQO1, NT5E (CD73), ODC1, OPG, OPN (SC), OS9, p53, PACSIN3, PAICS, PARK7, PARVA, PC, PCNA, PCSA, PD-1, PD-L1, PD-L2, PGP9.5, PHB, PHB2, PIK3C2B, PKP3, PPL, PR(B)?, PRDX2, PRKCB, PRKCD, PRKDC, PSA, PSAP, PSMA, PSMB7, PSMD2, PSME3, PYCARD, RAB1A, RAB3D, RAB7A, RAGE, RBL2, RNPEP, RPL14, RPL27, RPL36, RPS25, RPS4X, RPS4Y1, RPS4Y2, RUVBL2, SET, SHMT2, SLAIN1, SLC39A14, SLC9A3R2, SMARCA4, SNRPD2, SNRPD3, SNX33, SNX9, SPEN, SPR, SQSTM1, SSBP1, ST3GAL1, STXBP4, SUB1, SUCLG2, Survivin, SYT9, TFF3 (secreted), TGOLN2, THBS1, TIMP1, TIMP2, TMED10, TMED4, TMED9, TMEM211, TOM1, TRAF4 (scaffolding), TRAIL-R2, TRAP1, TrkB, Tsg 101, TXNDC16, U2AF2, UEVLD, UFC1, UNC93a, USP14, VASP, VCP, VDAC1, VEGFA, VEGFR1, VEGFR2, VPS37C, WIZ, XRCC5, XRCC6, YB-1, YWHAZ |
| Lung cancer | Pgrmc1 (progesterone receptor membrane component 1)/sigma-2 receptor, STEAP, EZH2 |
| Lung cancer | Prohibitin, CD23, Amylin Peptide, HRP, Rad51, Pax-5, Oct-3/, GLUT-1, PSCA, Thrombospondin, FHIT, a-B-Crystallin, LewisA, Vacular Endothelial Growth Factor(VEGF), Hepatocyte Factor Homologue-4, Flt-4, GluR6/7, Prostate Apoptosis Response Protein-4, GluR1, Fli-1, Urocortin, S100A4, 14-3-3 beta, P504S, HDAC1, PGP9.5, DJ-1, COX2, MMP-19, Actin, skeletal muscle, Claudin 3, Cadherin-P, Collagen IX, p27Kip1, Cathepsin D, CD30 (Reed-Sternberg Cell Marker), Ubiquitin, FSH-b, TrxR2, CCK-8, Cyclin C, CD138, TGF-beta 2, Adrenocorticotrophic Hormone, PPAR-gamma, Bcl-6, GLUT-3, IGF-I, mRANKL, Fas-ligand, Filamin, Calretinin, O ct-1, Parathyroid Hormone, Claudin 5, Claudin 4, Raf-1 (Phospho-specific), CDC14A Phosphatase, Mitochondria, APC, Gastrin 1, Ku (p80), Gai1, XPA, Maltose Binding Protein, Melanoma (gp100), Phosphotyrosine, Amyloid A, CXCR4 / Fusin, Hepatic Nuclear Factor-3B, Caspase 1, HPV 16-E7, Axonal Growth Cones, Lck, Ornithine Decarboxylase, Gamma Glutamylcysteine Synthetase(GCS)/Glutamate-cysteine Ligase, ERCC1, Calmodulin, Caspase 7 (Mch 3), CD137 (4-1BB), Nitric Oxide Synthase, brain (bNOS), E2F-2, IL-10R, L-Plastin, CD18, Vimentin, CD50/ICAM-3, Superoxide Dismutase, Adenovirus Type 5 E1A, PHAS-I, Progesterone Receptor (phospho-specific) - Serine 294, MHC II (HLA-DQ), XPG, ER Ca+2 ATPase2, Laminin-s, E3-binding protein (ARM1), CD45RO, CD1, Cdk2, MMP-10 (Stromilysin-2), sm, Surfactant Protein B (Pro), Apolipoprotein D, CD46, Keratin 8 (phospho-specific Ser73), PCNA, PLAP, CD20, Syk, LH, Keratin 19, ADP-ribosylation Factor (ARF-6), Int-2 Oncoprotein, Luciferase, AIF (Apoptosis Inducing Factor), Grb2, bcl-X, CD16, Paxillin, MHC II (HLA-DP and DR), B-Cell, p21WAF1, MHC II (HLA-DR), Tyrosinase, E2F-1, Pds1, Calponin, Notch, CD26/DPP IV, SV40 Large T Antigen, Ku (p70/p80), Perforin, XPF, SIM Ag (SIMA-4D3), Cdk1/p34cdc2, Neuron Specific Enolase, b-2-Microglobulin, DNA Polymerase Beta, Thyroid Hormone Receptor, Human, Alkaline Phosphatase (AP), Plasma Cell Marker, Heat Shock Protein 70/hsp70, TRP75 / gp75, SRF (Serum Response Factor), Laminin B1/b1, Mast Cell Chymase, Caldesmon, CEA / CD66e, CD24, Retinoid X Receptor (hRXR), CD45/T200/LCA, Rabies Virus, Cytochrome c, DR3, bcl-XL, Fascin, CD71 / Transferrin Receptor |
| Lung Cancer | miR-497 |
| Lung Cancer | Pgrmc1 |
| Ovarian Cancer | CA-125, CA 19-9, c-reactive protein, CD95(also called Fas, Fas antigen, Fas receptor, FasR, TNFRSF6, APT1 or APO-1), FAP-1, miR-200 microRNAs, EGFR, EGFRvIII, apolipoprotein AI, apolipoprotein CIII, myoglobin, tenascin C, MSH6, claudin-3, claudin-4, caveolin-1, coagulation factor III, CD9, CD36, CD37, CD53, CD63, CD81, CD136, CD147, Hsp70, Hsp90, Rab13, Desmocollin-1, EMP-2, CK7, CK20, GCDF15, CD82, Rab-5b, Annexin V, MFG-E8, HLA-DR. MiR-200 microRNAs (miR-200a, miR-200b, miR-200c), miR-141, miR-429, JNK, Jun |

| | |
|---|---|
| Prostate Cancer v normal | AQP2, BMP5, C16orf86, CXCL13, DST, ERCC1, GNAO1, KLHL5, MAP4K1, NELL2, PENK, PGF, POU3F1, PRSS21, SCML1, SEMG1, SMARCD3, SNAI2, TAF1C, TNNT3 |
| Prostate Cancer v Breast Cancer | ADRB2, ARG2, C22orf32, CYorf14, EIF1AY, FEV, KLK2, KLK4, LRRC26, MAOA, NLGN4Y, PNPLA7, PVRL3, SIM2, SLC30A4, SLC45A3, STX19, TRIM36, TRPM8 |
| Prostate Cancer v Colorectal Cancer | ADRB2, BAIAP2L2, C19orf33, CDX1, CEACAM6, EEF1A2, ERN2, FAM110B, FOXA2, KLK2, KLK4, LOC389816, LRRC26, MIPOL1, SLC45A3, SPDEF, TRIM31, TRIM36, ZNF613 |
| Prostate Cancer v Lung Cancer | ASTN2, CAB39L, CRIP1, FAM110B, FEV, GSTP1, KLK2, KLK4, LOC389816, LRRC26, MUC1, PNPLA7, SIM2, SLC45A3, SPDEF, TRIM36, TRPV6, ZNF613 |
| Prostate Cancer | miRs-26a+b, miR-15, miR-16, miR-195, miR-497, miR-424, miR-206, miR-342-5p, miR-186, miR-1271, miR-600, miR-216b, miR-519 family, miR-203 |
| Integrins | ITGA1 (CD49a, VLA1), ITGA2 (CD49b, VLA2), ITGA3 (CD49c, VLA3), ITGA4 (CD49d, VLA4), ITGA5 (CD49e, VLA5), ITGA6 (CD49f, VLA6), ITGA7 (FLJ25220), ITGA8, ITGA9 (RLC), ITGA10, ITGA11 (HsT18964), ITGAD (CD11D, FLJ39841), ITGAE (CD103, HUMINAE), ITGAL (CD11a, LFA1A), ITGAM (CD11b, MAC-1), ITGAV (CD51, VNRA, MSK8), ITGAW, ITGAX (CD11c), ITGB1 (CD29, FNRB, MSK12, MDF20), ITGB2 (CD18, LFA-1, MAC-1, MFI7), ITGB3 (CD61, GP3A, GPIIIa), ITGB4 (CD104), ITGB5 (FLJ26658), ITGB6, ITGB7, ITGB8 |
| Glycoprotein | GpIa-IIa, GpIIb-IIIa, GpIIIb, GpIb, GpIX |
| Transcription factors | STAT3, EZH2, p53, MACC1, SPDEF, RUNX2, YB-1 |
| Kinases | AURKA, AURKB |
| Disease Markers | 6Ckine, Adiponectin, Adrenocorticotropic Hormone, Agouti-Related Protein, Aldose Reductase, Alpha-1-Antichymotrypsin, Alpha-1-Antitrypsin, Alpha-1-Microglobulin, Alpha-2-Macroglobulin, Alpha-Fetoprotein, Amphiregulin, Angiogenin, Angiopoietin-2, Angiotensin-Converting Enzyme, Angiotensinogen, Annexin A1, Apolipoprotein A-I, Apolipoprotein A-II, Apolipoprotein A-IV, Apolipoprotein B, Apolipoprotein C-I, Apolipoprotein C-III, Apolipoprotein D, Apolipoprotein E, Apolipoprotein H, Apolipoprotein(a), AXL Receptor Tyrosine Kinase, B cell-activating Factor, B Lymphocyte Chemoattractant, Bcl-2-like protein 2, Beta-2-Microglobulin, Betacellulin, Bone Morphogenetic Protein 6, Brain-Derived Neurotrophic Factor, Calbindin, Calcitonin, Cancer Antigen 125, Cancer Antigen 15-3, Cancer Antigen 19-9, Cancer Antigen 72-4, Carcinoembryonic Antigen, Cathepsin D, CD 40 antigen, CD40 Ligand, CD5 Antigen-like, Cellular Fibronectin, Chemokine CC-4, Chromogranin-A, Ciliary Neurotrophic Factor, Clusterin, Collagen IV, Complement C3, Complement Factor H, Connective Tissue Growth Factor, Cortisol, C-Peptide, C-Reactive Protein, Creatine Kinase-MB, Cystatin-C, Endoglin, Endostatin, Endothelin-1, EN-RAGE, Eotaxin-1, Eotaxin-2, Eotaxin-3, Epidermal Growth Factor, Epiregulin, Epithelial cell adhesion molecule, Epithelial-Derived Neutrophil-Activating Protein 78, Erythropoietin, E-Selectin, Ezrin, Factor VII, Fas Ligand, FASLG Receptor, Fatty Acid-Binding Protein (adipocyte), Fatty Acid-Binding Protein (heart), Fatty Acid-Binding Protein (liver), Ferritin, Fetuin-A, Fibrinogen, Fibroblast Growth Factor 4, Fibroblast Growth Factor basic, Fibulin-1C, Follicle-Stimulating Hormone, Galectin-3, Gelsolin, Glucagon, Glucagon-like Peptide 1, Glucose-6-phosphate Isomerase, Glutamate-Cysteine Ligase Regulatory subunit, Glutathione S-Transferase alpha, Glutathione S-Transferase Mu 1, Granulocyte Colony-Stimulating Factor, Granulocyte-Macrophage Colony-Stimulating Factor, Growth Hormone, Growth-Regulated alpha protein, Haptoglobin, HE4, Heat Shock Protein 60, Heparin-Binding EGF-Like Growth Factor, Hepatocyte Growth Factor, Hepatocyte Growth Factor Receptor, Hepsin, Human Chorionic Gonadotropin beta, Human Epidermal Growth Factor Receptor 2, Immunoglobulin A, Immunoglobulin E, Immunoglobulin M, Insulin, Insulin-like Growth Factor I, Insulin-like Growth Factor-Binding Protein 1, Insulin-like Growth Factor-Binding Protein 2, Insulin-like Growth Factor-Binding Protein 3, |

| | |
|---|---|
| | Insulin-like Growth Factor Binding Protein 4, Insulin-like Growth Factor Binding Protein 5, Insulin-like Growth Factor Binding Protein 6, Intercellular Adhesion Molecule 1, Interferon gamma, Interferon gamma Induced Protein 10, Interferon-inducible T-cell alpha chemoattractant, Interleukin-1 alpha, Interleukin-1 beta, Interleukin-1 Receptor antagonist, Interleukin-2, Interleukin-2 Receptor alpha, Interleukin-3, Interleukin-4, Interleukin-5, Interleukin-6, Interleukin-6 Receptor, Interleukin-6 Receptor subunit beta, Interleukin-7, Interleukin-8, Interleukin-10, Interleukin-11, Interleukin-12 Subunit p40, Interleukin-12 Subunit p70, Interleukin-13, Interleukin-15, Interleukin-16, Interleukin-25, Kallikrein 5, Kallikrein-7, Kidney Injury Molecule-1, Lactoylglutathione lyase, Latency-Associated Peptide of Transforming Growth Factor beta 1, Lectin-Like Oxidized LDL Receptor 1, Leptin, Luteinizing Hormone, Lymphotactin, Macrophage Colony-Stimulating Factor 1, Macrophage Inflammatory Protein-1 alpha, Macrophage Inflammatory Protein-1 beta, Macrophage Inflammatory Protein-3 alpha, Macrophage inflammatory protein 3 beta, Macrophage Migration Inhibitory Factor, Macrophage-Derived Chemokine, Macrophage-Stimulating Protein, Malondialdehyde-Modified Low-Density Lipoprotein, Maspin, Matrix Metalloproteinase-1, Matrix Metalloproteinase-2, Matrix Metalloproteinase-3, Matrix Metalloproteinase-7, Matrix Metalloproteinase-9, Matrix Metalloproteinase-9, Matrix Metalloproteinase-10, Mesothelin, MHC class I chain-related protein A, Monocyte Chemotactic Protein 1, Monocyte Chemotactic Protein 2, Monocyte Chemotactic Protein 3, Monocyte Chemotactic Protein 4, Monokine Induced by Gamma Interferon, Myeloid Progenitor Inhibitory Factor 1, Myeloperoxidase, Myoglobin, Nerve Growth Factor beta, Neuronal Cell Adhesion Molecule, Neuron-Specific Enolase, Neuropilin-1, Neutrophil Gelatinase-Associated Lipocalin, NT-proBNP, Nucleoside diphosphate kinase B, Osteopontin, Osteoprotegerin, Pancreatic Polypeptide, Pepsinogen I, Peptide YY, Peroxiredoxin-4, Phosphoserine Aminotransferase, Placenta Growth Factor, Plasminogen Activator Inhibitor 1, Platelet-Derived Growth Factor BB, Pregnancy-Associated Plasma Protein A, Progesterone, Proinsulin (inc. Total or Intact), Prolactin, Prostasin, Prostate-Specific Antigen (inc. Free PSA), Prostatic Acid Phosphatase, Protein S100-A4, Protein S100-A6, Pulmonary and Activation-Regulated Chemokine, Receptor for advanced glycosylation end products, Receptor tyrosine-protein kinase erbB-3, Resistin, S100 calcium-binding protein B, Secretin, Serotransferrin, Serum Amyloid P-Component, Serum Glutamic Oxaloacetic Transaminase, Sex Hormone-Binding Globulin, Sortilin, Squamous Cell Carcinoma Antigen-1, Stem Cell Factor, Stromal cell-derived Factor-1, Superoxide Dismutase 1 (soluble), T Lymphocyte-Secreted Protein I-309, Tamm-Horsfall Urinary Glycoprotein, T-Cell-Specific Protein RANTES, Tenascin-C, Testosterone, Tetranectin, Thrombomodulin, Thrombopoietin, Thrombospondin-1, Thyroglobulin, Thyroid-Stimulating Hormone, Thyroxine-Binding Globulin, Tissue Factor, Tissue Inhibitor of Metalloproteinases 1, Tissue type Plasminogen activator, TNF-Related Apoptosis-Inducing Ligand Receptor 3, Transforming Growth Factor alpha, Transforming Growth Factor beta-3, Transthyretin, Trefoil Factor 3, Tumor Necrosis Factor alpha, Tumor Necrosis Factor beta, Tumor Necrosis Factor Receptor I, Tumor necrosis Factor Receptor 2, Tyrosine kinase with Ig and EGF homology domains 2, Urokinase-type Plasminogen Activator, Urokinase-type plasminogen activator Receptor, Vascular Cell Adhesion Molecule-1, Vascular Endothelial Growth Factor, Vascular endothelial growth Factor B, Vascular Endothelial Growth Factor C, Vascular endothelial growth Factor D, Vascular Endothelial Growth Factor Receptor 1, Vascular Endothelial Growth Factor Receptor 2, Vascular endothelial growth Factor Receptor 3, Vitamin K-Dependent Protein S, Vitronectin, von Willebrand Factor, YKL-40 |
| Disease Markers | Adiponectin, Adrenocorticotropic Hormone, Agouti-Related Protein, Alpha-1-Antichymotrypsin, Alpha-1-Antitrypsin, Alpha-1-Microglobulin, Alpha-2-Macroglobulin, Alpha-Fetoprotein, Amphiregulin, Angiopoietin-2, Angiotensin-Converting Enzyme, Angiotensinogen, Apolipoprotein A-I, Apolipoprotein A-II, Apolipoprotein A-IV, Apolipoprotein B, Apolipoprotein C-I, Apolipoprotein C-III, Apolipoprotein D, Apolipoprotein E, Apolipoprotein H, Apolipoprotein(a), AXL |

| | |
|---|---|
| | Receptor Tyrosine Kinase, B Lymphocyte Chemoattractant, Beta-2-Microglobulin, Betacellulin, Bone Morphogenetic Protein 6, Brain-Derived Neurotrophic Factor, Calbindin, Calcitonin, Cancer Antigen 125, Cancer Antigen 19-9, Carcinoembryonic Antigen, CD 40 antigen, CD40 Ligand, CD5 Antigen-like, Chemokine CC-4, Chromogranin-A, Ciliary Neurotrophic Factor, Clusterin, Complement C3, Complement Factor H, Connective Tissue Growth Factor, Cortisol, C-Peptide, C-Reactive Protein, Creatine Kinase-MB, Cystatin-C, Endothelin-1, EN-RAGE, Eotaxin-1, Eotaxin-3, Epidermal Growth Factor, Epiregulin, Epithelial-Derived Neutrophil-Activating Protein 78, Erythropoietin, E-Selectin, Factor VII, Fas Ligand, FASLG Receptor, Fatty Acid-Binding Protein (heart), Ferritin, Fetuin-A, Fibrinogen, Fibroblast Growth Factor 4, Fibroblast Growth Factor basic, Follicle-Stimulating Hormone, Glucagon, Glucagon-like Peptide 1, Glutathione S-Transferase alpha, Granulocyte Colony-Stimulating Factor, Granulocyte-Macrophage Colony-Stimulating Factor, Growth Hormone, Growth-Regulated alpha protein, Haptoglobin, Heat Shock Protein 60, Heparin-Binding EGF-Like Growth Factor, Hepatocyte Growth Factor, Immunoglobulin A, Immunoglobulin E, Immunoglobulin M, Insulin, Insulin-like Growth Factor I, Insulin-like Growth Factor-Binding Protein 2, Intercellular Adhesion Molecule 1, Interferon gamma, Interferon gamma Induced Protein 10, Interleukin-1 alpha, Interleukin-1 beta, Interleukin-1 Receptor antagonist, Interleukin-2, Interleukin-3, Interleukin-4, Interleukin-5, Interleukin-6, Interleukin-6 Receptor, Interleukin-7, Interleukin-8, Interleukin-10, Interleukin-11, Interleukin-12 Subunit p40, Interleukin-12 Subunit p70, Interleukin-13, Interleukin-15, Interleukin-16, Interleukin-25, Kidney Injury Molecule-1, Lectin-Like Oxidized LDL Receptor 1, Leptin, Luteinizing Hormone, Lymphotactin, Macrophage Colony-Stimulating Factor 1, Macrophage Inflammatory Protein-1 alpha, Macrophage Inflammatory Protein-1 beta, Macrophage Inflammatory Protein-3 alpha, Macrophage Migration Inhibitory Factor, Macrophage-Derived Chemokine, Malondialdehyde-Modified Low-Density Lipoprotein, Matrix Metalloproteinase-1, Matrix Metalloproteinase-2, Matrix Metalloproteinase-3, Matrix Metalloproteinase-7, Matrix Metalloproteinase-9, Matrix Metalloproteinase-9, Matrix Metalloproteinase-10, Monocyte Chemotactic Protein 1, Monocyte Chemotactic Protein 2, Monocyte Chemotactic Protein 3, Monocyte Chemotactic Protein 4, Monokine Induced by Gamma Interferon, Myeloid Progenitor Inhibitory Factor 1, Myeloperoxidase, Myoglobin, Nerve Growth Factor beta, Neuronal Cell Adhesion Molecule, Neutrophil Gelatinase-Associated Lipocalin, NT-proBNP, Osteopontin, Pancreatic Polypeptide, Peptide YY, Placenta Growth Factor, Plasminogen Activator Inhibitor 1, Platelet-Derived Growth Factor BB, Pregnancy-Associated Plasma Protein A, Progesterone, Proinsulin (inc. Intact or Total), Prolactin, Prostate-Specific Antigen (inc. Free PSA), Prostatic Acid Phosphatase, Pulmonary and Activation-Regulated Chemokine, Receptor for advanced glycosylation end products, Resistin, S100 calcium-binding protein B, Secretin, Serotransferrin, Serum Amyloid P-Component, Serum Glutamic Oxaloacetic Transaminase, Sex Hormone-Binding Globulin, Sortilin, Stem Cell Factor, Superoxide Dismutase 1 (soluble), T Lymphocyte-Secreted Protein I-309, Tamm-Horsfall Urinary Glycoprotein, T-Cell-Specific Protein RANTES, Tenascin-C, Testosterone, Thrombomodulin, Thrombopoietin, Thrombospondin-1, Thyroid-Stimulating Hormone, Thyroxine-Binding Globulin, Tissue Factor, Tissue Inhibitor of Metalloproteinases 1, TNF-Related Apoptosis-Inducing Ligand Receptor 3, Transforming Growth Factor alpha, Transforming Growth Factor beta-3, Transthyretin, Trefoil Factor 3, Tumor Necrosis Factor alpha, Tumor Necrosis Factor beta, Tumor necrosis Factor Receptor 2, Vascular Cell Adhesion Molecule-1, Vascular Endothelial Growth Factor, Vitamin K-Dependent Protein S, Vitronectin, von Willebrand Factor |
| Oncology | 6Ckine, Aldose Reductase, Alpha-Fetoprotein, Amphiregulin, Angiogenin, Annexin A1, B cell-activating Factor, B Lymphocyte Chemoattractant, Bcl-2-like protein 2, Betacellulin, Cancer Antigen 125, Cancer Antigen 15-3, Cancer Antigen 19-9, Cancer Antigen 72-4, Carcinoembryonic Antigen, Cathepsin D, Cellular Fibronectin, Collagen IV, Endoglin, Endostatin, Eotaxin-2, Epidermal Growth Factor, Epiregulin, |

| | |
|---|---|
| | Epithelial cell adhesion molecule, Ezrin, Fatty Acid-Binding Protein (adipocyte), Fatty Acid-Binding Protein (liver), Fibroblast Growth Factor basic, Fibulin-1C, Galectin-3, Gelsolin, Glucose-6-phosphate Isomerase, Glutamate-Cysteine Ligase Regulatory subunit, Glutathione S-Transferase Mu 1, HE4, Heparin-Binding EGF-Like Growth Factor, Hepatocyte Growth Factor, Hepatocyte Growth Factor Receptor, Hepsin, Human Chorionic Gonadotropin beta, Human Epidermal Growth Factor Receptor 2, Insulin-like Growth Factor-Binding Protein 1, Insulin-like Growth Factor-Binding Protein 2, Insulin-like Growth Factor-Binding Protein 3, Insulin-like Growth Factor Binding Protein 4, Insulin-like Growth Factor Binding Protein 5, Insulin-like Growth Factor Binding Protein 6, Interferon gamma Induced Protein 10, Interferon-inducible T-cell alpha chemoattractant, Interleukin-2 Receptor alpha, Interleukin-6, Interleukin-6 Receptor subunit beta, Kallikrein 5, Kallikrein-7, Lactoylglutathione lyase, Latency-Associated Peptide of Transforming Growth Factor beta 1, Leptin, Macrophage inflammatory protein 3 beta, Macrophage Migration Inhibitory Factor, Macrophage-Stimulating Protein, Maspin, Matrix Metalloproteinase-2, Mesothelin, MHC class I chain-related protein A, Monocyte Chemotactic Protein 1, Monokine Induced by Gamma Interferon, Neuron-Specific Enolase, Neuropilin-1, Neutrophil Gelatinase-Associated Lipocalin, Nucleoside diphosphate kinase B, Osteopontin, Osteoprotegerin, Pepsinogen I, Peroxiredoxin-4, Phosphoserine Aminotransferase, Placenta Growth Factor, Platelet-Derived Growth Factor BB, Prostasin, Protein S100-A4, Protein S100-A6, Receptor tyrosine-protein kinase erbB-3, Squamous Cell Carcinoma Antigen-1, Stromal cell-derived Factor-1, Tenascin-C, Tetranectin, Thyroglobulin, Tissue type Plasminogen activator, Transforming Growth Factor alpha, Tumor Necrosis Factor Receptor I, Tyrosine kinase with Ig and EGF homology domains 2, Urokinase-type Plasminogen Activator, Urokinase-type plasminogen activator Receptor, Vascular Endothelial Growth Factor, Vascular endothelial growth Factor B, Vascular Endothelial Growth Factor C, Vascular endothelial growth Factor D, Vascular Endothelial Growth Factor Receptor 1, Vascular Endothelial Growth Factor Receptor 2, Vascular endothelial growth Factor Receptor 3, YKL-40 |
| Disease | Adiponectin, Alpha-1-Antitrypsin, Alpha-2-Macroglobulin, Alpha-Fetoprotein, Apolipoprotein A-I, Apolipoprotein C-III, Apolipoprotein H, Apolipoprotein(a), Beta-2-Microglobulin, Brain-Derived Neurotrophic Factor, Calcitonin, Cancer Antigen 125, Cancer Antigen 19-9, Carcinoembryonic Antigen, CD 40 antigen, CD40 Ligand, Complement C3, C-Reactive Protein, Creatine Kinase-MB, Endothelin-1, EN-RAGE, Eotaxin-1, Epidermal Growth Factor, Epithelial-Derived Neutrophil-Activating Protein 78, Erythropoietin, Factor VII, Fatty Acid-Binding Protein (heart), Ferritin, Fibrinogen, Fibroblast Growth Factor basic, Granulocyte Colony-Stimulating Factor, Granulocyte-Macrophage Colony-Stimulating Factor, Growth Hormone, Haptoglobin, Immunoglobulin A, Immunoglobulin E, Immunoglobulin M, Insulin, Insulin-like Growth Factor I, Intercellular Adhesion Molecule 1, Interferon gamma, Interleukin-1 alpha, Interleukin-1 beta, Interleukin-1 Receptor antagonist, Interleukin-2, Interleukin-3, Interleukin-4, Interleukin-5, Interleukin-6, Interleukin-7, Interleukin-8, Interleukin-10, Interleukin-12 Subunit p40, Interleukin-12 Subunit p70, Interleukin-13, Interleukin-15, Interleukin-16, Leptin, Lymphotactin, Macrophage Inflammatory Protein-1 alpha, Macrophage Inflammatory Protein-1 beta, Macrophage-Derived Chemokine, Matrix Metalloproteinase-2, Matrix Metalloproteinase-3, Matrix Metalloproteinase-9, Monocyte Chemotactic Protein 1, Myeloperoxidase, Myoglobin, Plasminogen Activator Inhibitor 1, Pregnancy-Associated Plasma Protein A, Prostate-Specific Antigen (inc. Free PSA), Prostatic Acid Phosphatase, Serum Amyloid P-Component, Serum Glutamic Oxaloacetic Transaminase, Sex Hormone-Binding Globulin, Stem Cell Factor, T-Cell-Specific Protein RANTES, Thrombopoietin, Thyroid-Stimulating Hormone, Thyroxine-Binding Globulin, Tissue Factor, Tissue Inhibitor of Metalloproteinases 1, Tumor Necrosis Factor alpha, Tumor Necrosis Factor beta, Tumor Necrosis Factor Receptor 2, Vascular Cell Adhesion Molecule-1, Vascular Endothelial Growth Factor, von Willebrand Factor |

| Neurological | Alpha-1-Antitrypsin, Apolipoprotein A-I, Apolipoprotein A-II, Apolipoprotein B, Apolipoprotein C-I, Apolipoprotein H, Beta-2-Microglobulin, Betacellulin, Brain-Derived Neurotrophic Factor, Calbindin, Cancer Antigen 125, Carcinoembryonic Antigen, CD5 Antigen-like, Complement C3, Connective Tissue Growth Factor, Cortisol, Endothelin-1, Epidermal Growth Factor Receptor, Ferritin, Fetuin-A, Follicle-Stimulating Hormone, Haptoglobin, Immunoglobulin A, Immunoglobulin M, Intercellular Adhesion Molecule 1, Interleukin-6 Receptor, Interleukin-7, Interleukin-10, Interleukin-11, Interleukin-17, Kidney Injury Molecule-1, Luteinizing Hormone, Macrophage-Derived Chemokine, Macrophage Migration Inhibitory Factor, Macrophage Inflammatory Protein-1 alpha, Matrix Metalloproteinase-2, Monocyte Chemotactic Protein 2, Peptide YY, Prolactin, Prostatic Acid Phosphatase, Serotransferrin, Serum Amyloid P-Component, Sortilin, Testosterone, Thrombopoietin, Thyroid-Stimulating Hormone, Tissue Inhibitor of Metalloproteinases 1, TNF-Related Apoptosis-Inducing Ligand Receptor 3, Tumor necrosis Factor Receptor 2, Vascular Endothelial Growth Factor, Vitronectin |
|---|---|
| Cardiovascular | Adiponectin, Apolipoprotein A-I, Apolipoprotein B, Apolipoprotein C-III, Apolipoprotein D, Apolipoprotein E, Apolipoprotein H, Apolipoprotein(a), Clusterin, C-Reactive Protein, Cystatin-C, EN-RAGE, E-Selectin, Fatty Acid-Binding Protein (heart), Ferritin, Fibrinogen, Haptoglobin, Immunoglobulin M, Intercellular Adhesion Molecule 1, Interleukin-6, Interleukin-8, Lectin-Like Oxidized LDL Receptor 1, Leptin, Macrophage Inflammatory Protein-1 alpha, Macrophage Inflammatory Protein-1 beta, Malondialdehyde-Modified Low-Density Lipoprotein, Matrix Metalloproteinase-1, Matrix Metalloproteinase-10, Matrix Metalloproteinase-2, Matrix Metalloproteinase-3, Matrix Metalloproteinase-7, Matrix Metalloproteinase-9, Monocyte Chemotactic Protein 1, Myeloperoxidase, Myoglobin, NT-proBNP, Osteopontin, Plasminogen Activator Inhibitor 1, P-Selectin, Receptor for advanced glycosylation end products, Serum Amyloid P-Component, Sex Hormone-Binding Globulin, T-Cell-Specific Protein RANTES, Thrombomodulin, Thyroxine-Binding Globulin, Tissue Inhibitor of Metalloproteinases 1, Tumor Necrosis Factor alpha, Tumor necrosis Factor Receptor 2, Vascular Cell Adhesion Molecule-1, von Willebrand Factor |
| Inflammatory | Alpha-1-Antitrypsin, Alpha-2-Macroglobulin, Beta-2-Microglobulin, Brain-Derived Neurotrophic Factor, Complement C3, C-Reactive Protein, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 Receptor antagonist, Interleukin-2, Interleukin-3, Interleukin-4, Interleukin-5, Interleukin-6, Interleukin-7, Interleukin-8, Interleukin-10, Interleukin-12 Subunit p40, Interleukin-12 Subunit p70, Interleukin-15, Interleukin-17, Interleukin-23, Macrophage Inflammatory Protein-1 alpha, Macrophage Inflammatory Protein-1 beta, Matrix Metalloproteinase-2, Matrix Metalloproteinase-3, Matrix Metalloproteinase-9, Monocyte Chemotactic Protein 1, Stem Cell Factor, T-Cell-Specific Protein RANTES, Tissue Inhibitor of Metalloproteinases 1, Tumor Necrosis Factor alpha, Tumor Necrosis Factor beta, Tumor necrosis Factor Receptor 2, Vascular Cell Adhesion Molecule-1, Vascular Endothelial Growth Factor, Vitamin D-Binding Protein, von Willebrand Factor |
| Metabolic | Adiponectin, Adrenocorticotropic Hormone, Angiotensin-Converting Enzyme, Angiotensinogen, Complement C3 alpha des arg, Cortisol, Follicle-Stimulating Hormone, Galanin, Glucagon, Glucagon-like Peptide 1, Insulin, Insulin-like Growth Factor I, Leptin, Luteinizing Hormone, Pancreatic Polypeptide, Peptide YY, Progesterone, Prolactin, Resistin, Secretin, Testosterone |
| Kidney | Alpha-1-Microglobulin, Beta-2-Microglobulin, Calbindin, Clusterin, Connective Tissue Growth Factor, Creatinine, Cystatin-C, Glutathione S-Transferase alpha, Kidney Injury Molecule-1, Microalbumin, Neutrophil Gelatinase-Associated Lipocalin, Osteopontin, Tamm-Horsfall Urinary Glycoprotein, Tissue Inhibitor of Metalloproteinases 1, Trefoil Factor 3, Vascular Endothelial Growth Factor |
| Cytokines | Granulocyte-Macrophage Colony-Stimulating Factor, Interferon gamma, Interleukin-2, Interleukin-3, Interleukin-4, Interleukin-5, Interleukin-6, Interleukin-7, |

| | | Interleukin-8, Interleukin-10, Macrophage Inflammatory Protein-1 alpha, Macrophage Inflammatory Protein-1 beta, Matrix Metalloproteinase-2, Monocyte Chemotactic Protein 1, Tumor Necrosis Factor alpha, Tumor Necrosis Factor beta, Brain-Derived Neurotrophic Factor, Eotaxin-1, Intercellular Adhesion Molecule 1, Interleukin-1 alpha, Interleukin-1 beta, Interleukin-1 Receptor antagonist, Interleukin-12 Subunit p40, Interleukin-12 Subunit p70, Interleukin-15, Interleukin-17, Interleukin-23, Matrix Metalloproteinase-3, Stem Cell Factor, Vascular Endothelial Growth Factor |
|---|---|---|
| Protein | | 14.3.3 gamma, 14.3.3 (Pan), 14-3-3 beta, 6-Histidine, a-B-Crystallin, Acinus, Actin beta, Actin (Muscle Specific), Actin (Pan), Actin (skeletal muscle), Activin Receptor Type II, Adenovirus, Adenovirus Fiber, Adenovirus Type 2 E1A, Adenovirus Type 5 E1A, ADP-ribosylation Factor (ARF-6), Adrenocorticotrophic Hormone, AIF (Apoptosis Inducing Factor), Alkaline Phosphatase (AP), Alpha Fetoprotein (AFP), Alpha Lactalbumin, alpha-1-antichymotrypsin, alpha-1-antitrypsin, Amphiregulin, Amylin Peptide, Amyloid A, Amyloid A4 Protein Precursor, Amyloid Beta (APP), Androgen Receptor, Ang-1, Ang-2, APC, APC11, APC2, Apolipoprotein D, A-Raf, ARC, Ask1 / MAPKKK5, ATM, Axonal Growth Cones, b Galactosidase, b-2-Microglobulin, B7-H2, BAG-1, Bak, Bax, B-Cell, B-cell Linker Protein (BLNK), Bcl10 / CIPER / CLAP / mE10, bcl-2a, Bcl-6, bcl-X, bcl-XL, Bim (BOD), Biotin, Bonzo / STRL33 / TYMSTR, Bovine Serum Albumin, BRCA2 (aa 1323-1346), BrdU, Bromodeoxyuridine (BrdU), CA125, CA19-9, c-Abl, Cadherin (Pan), Cadherin-E, Cadherin-P, Calcitonin, Calcium Pump ATPase, Caldesmon, Calmodulin, Calponin, Calretinin, Casein, Caspase 1, Caspase 2, Caspase 3, Caspase 5, Caspase 6 (Mch 2), Caspase 7 (Mch 3), Caspase 8 (FLICE), Caspase 9, Catenin alpha, Catenin beta, Catenin gamma, Cathepsin D, CCK-8, CD1, CD10, CD100/Leukocyte Semaphorin, CD105, CD106 / VCAM, CD115/c-fms/CSF-1R/M-CSFR, CD137 (4-1BB), CD138, CD14, CD15, CD155/PVR (Polio Virus Receptor), CD16, CD165, CD18, CD1a, CD1b, CD2, CD20, CD21, CD23, CD231, CD24, CD25/IL-2 Receptor a, CD26/DPP IV, CD29, CD30 (Reed-Sternberg Cell Marker), CD32/Fcg Receptor II, CD35/CR1, CD36GPIIIb/GPIV, CD3zeta, CD4, CD40, CD42b, CD43, CD45/T200/LCA, CD45RB, CD45RO, CD46, CD5, CD50/ICAM-3, CD53, CD54/ICAM-1, CD56/NCAM-1, CD57, CD59 / MACIF / MIRL / Protectin, CD6, CD61 / Platelet Glycoprotein IIIA, CD63, CD68, CD71 / Transferrin Receptor, CD79a mb-1, CD79b, CD8, CD81/TAPA-1, CD84, CD9, CD94, CD95 / Fas, CD98, CDC14A Phosphatase, CDC25C, CDC34, CDC37, CDC47, CDC6, cdh1, Cdk1/p34cdc2, Cdk2, Cdk3, Cdk4, Cdk5, Cdk7, Cdk8, CDw17, CDw60, CDw75, CDw78, CEA / CD66e, c-erbB-2/HER-2/neu Ab-1 (21N), c-erbB-4/HER-4, c-fos, Chk1, Chorionic Gonadotropin beta (hCG-beta), Chromogranin A, CIDE-A, CIDE-B, CITED1, c-jun, Clathrin, claudin 11, Claudin 2, Claudin 3, Claudin 4, Claudin 5, CLAUDIN 7, Claudin-1, CNPase, Collagen II, Collagen IV, Collagen IX, Collagen VII, Connexin 43, COX2, CREB, CREB-Binding Protein, Cryptococcus neoformans, c-Src, Cullin-1 (CUL-1), Cullin-2 (CUL-2), Cullin-3 (CUL-3), CXCR4 / Fusin, Cyclin B1, Cyclin C, Cyclin D1, Cyclin D3, Cyclin E, Cyclin E2, Cystic Fibrosis Transmembrane Regulator, Cytochrome c, D4-GDI, Daxx, DcR1, DcR2 / TRAIL-R4 / TRUNDD, Desmin, DFF40 (DNA Fragmentation Factor 40) / CAD, DFF45 / ICAD, DJ-1, DNA Ligase I, DNA Polymerase Beta, DNA Polymerase Gamma, DNA Primase (p49), DNA Primase (p58), DNA-PKcs, DP-2, DR3, DR5, Dysferlin, Dystrophin, E2F-1, E2F-2, E2F-3, E2F-4, E2F-5, E3-binding protein (ARM1), EGFR, EMA/CA15-3/MUC-1, Endostatin, Epithelial Membrane Antigen (EMA / CA15-3 / MUC-1), Epithelial Specific Antigen, ER beta, ER Ca+2 ATPase2, ERCC1, Erk1, ERK2, Estradiol, Estriol, Estrogen Receptor, Exo1, Ezrin/p81/80K/Cytovillin, F.VIII/VWF, Factor VIII Related Antigen, FADD (FAS-Associated death domain-containing protein), Fascin, Fas-ligand, Ferritin, FGF-1, FGF-2, FHIT, Fibrillin-1, Fibronectin, Filaggrin, Filamin, FITC, Fli-1, FLIP, Flk-1 / KDR / VEGFR2, Flt-1 / VEGFR1, Flt-4, Fra2, FSH, FSH-b, Fyn, Ga0, Gab-1, GABA a Receptor 1, GAD65, Gai1, Gamma Glutamyl Transferase (gGT), Gamma Glutamylcysteine Synthetase(GCS)/Glutamate-cysteine Ligase, GAPDH, Gastrin 1, |

GCDFP-15, G-CSF, GFAP, Glicentin, Glucagon, Glucose-Regulated Protein 94, GluR 2/3, GluR1, GluR4, GluR6/7, GLUT-1, GLUT-3, Glycogen Synthase Kinase 3b (GSK3b), Glycophorin A, GM-CSF, GnRH Receptor, Golgi Complex, Granulocyte, Granzyme B, Grb2, Green Fluorescent Protein (GFP), GRIP1, Growth Hormone (hGH), GSK-3, GST, GSTmu, H.Pylori, HDAC1, HDJ-2/DNAJ, Heat Shock Factor 1, Heat Shock Factor 2, Heat Shock Protein 27/hsp27, Heat Shock Protein 60/hsp60, Heat Shock Protein 70/hsp70, Heat Shock Protein 75/hsp75, Heat Shock Protein 90a/hsp86, Heat Shock Protein 90b/hsp84, Helicobacter pylori, Heparan Sulfate Proteoglycan, Hepatic Nuclear Factor-3B, Hepatocyte, Hepatocyte Factor Homologue-4, Hepatocyte Growth Factor, Heregulin, HIF-1a, Histone H1, hPL, HPV 16, HPV 16-E7, HRP, Human Sodium Iodide Symporter (hNIS), I-FLICE / CASPER, IFN gamma, IgA, IGF-1R, IGF-I, IgG, IgM (m-Heavy Chain), I-Kappa-B Kinase b (IKKb), IL-1 alpha, IL-1 beta, IL-10, IL-10R, IL17, IL-2, IL-3, IL-30, IL-4, IL-5, IL-6, IL-8, Inhibin alpha, Insulin, Insulin Receptor, Insulin Receptor Substrate-1, Int-2 Oncoprotein, Integrin beta5, Interferon-a(II), Interferon-g, Involucrin, IP10/CRG2, IPO-38 Proliferation Marker, IRAK, ITK, JNK Activating kinase (JKK1), Kappa Light Chain, Keratin 10, Keratin 10/13, Keratin 14, Keratin 15, Keratin 16, Keratin 18, Keratin 19, Keratin 20, Keratin 5/6/18, Keratin 5/8, Keratin 8, Keratin 8 (phospho-specific Ser73), Keratin 8/18, Keratin (LMW), Keratin (Multi), Keratin (Pan), Ki67, Ku (p70/p80), Ku (p80), L1 Cell Adhesion Molecule, Lambda Light Chain, Laminin B1/b1, Laminin B2/g1, Laminin Receptor, Laminin-s, Lck, Lck (p56lck), Leukotriene (C4, D4, E4), LewisA, LewisB, LH, L-Plastin, LRP / MVP, Luciferase, Macrophage, MADD, MAGE-1, Maltose Binding Protein, MAP1B, MAP2a,b, MART-1/Melan-A, Mast Cell Chymase, Mcl-1, MCM2, MCM5, MDM2, Medroxyprogesterone Acetate (MPA), Mek1, Mek2, Mek6, Mekk-1, Melanoma (gp100), mGluR1, mGluR5, MGMT, MHC I (HLA25 and HLA-Aw32), MHC I (HLA-A), MHC I (HLA-A,B,C), MHC I (HLA-B), MHC II (HLA-DP and DR), MHC II (HLA-DP), MHC II (HLA-DQ), MHC II (HLA-DR), MHC II (HLA-DR) Ia, Microphthalmia, Milk Fat Globule Membrane Protein, Mitochondria, MLH1, MMP-1 (Collagenase-I), MMP-10 (Stromilysin-2), MMP-11 (Stromelysin-3), MMP-13 (Collagenase-3), MMP-14 / MT1-MMP, MMP-15 / MT2-MMP, MMP-16 / MT3-MMP, MMP-19, MMP-2 (72kDa Collagenase IV), MMP-23, MMP-7 (Matrilysin), MMP-9 (92kDa Collagenase IV), Moesin, mRANKL, Muc-1, Mucin 2, Mucin 3 (MUC3), Mucin 5AC, MyD88, Myelin / Oligodendrocyte, Myeloid Specific Marker, Myeloperoxidase, MyoD1, Myogenin, Myoglobin, Myosin Smooth Muscle Heavy Chain, Nck, Negative Control for Mouse IgG1, Negative Control for Mouse IgG2a, Negative Control for Mouse IgG3, Negative Control for Mouse IgM, Negative Control for Rabbit IgG, Neurofilament, Neurofilament (160kDa), Neurofilament (200kDa), Neurofilament (68kDa), Neuron Specific Enolase, Neutrophil Elastase, NF kappa B / p50, NF kappa B / p65 (Rel A), NGF-Receptor (p75NGFR), brain Nitric Oxide Synthase (bNOS), endothelial Nitric Oxide Synthase (eNOS), nm23, NOS-i, NOS-u, Notch, Nucleophosmin (NPM), NuMA, O ct-1, Oct-2/, Oct-3/, Ornithine Decarboxylase, Osteopontin, p130, p130cas, p14ARF, p15INK4b, p16INK4a, p170, p170 / MDR-1, p18INK4c, p19ARF, p19Skp1, p21WAF1, p27Kip1, p300 / CBP, p35nck5a, P504S, p53, p57Kip2 Ab-7, p63 (p53 Family Member), p73, p73a, p73a/b, p95VAV, Parathyroid Hormone, Parathyroid Hormone Receptor Type 1, Parkin, PARP, PARP (Poly ADP-Ribose Polymerase), Pax-5, Paxillin, PCNA, PCTAIRE2, PDGF, PDGFR alpha, PDGFR beta, Pds1, Perforin, PGP9.5, PHAS-I, PHAS-II, Phospho-Ser/Thr/Tyr, Phosphotyrosine, PLAP, Plasma Cell Marker, Plasminogen, PLC gamma 1, PMP-22, Pneumocystis jiroveci, PPAR-gamma, PR3 (Proteinase 3), Presenillin, Progesterone, Progesterone Receptor, Progesterone Receptor (phospho-specific) - Serine 190, Progesterone Receptor (phospho-specific) - Serine 294, Prohibitin, Prolactin, Prolactin Receptor, Prostate Apoptosis Response Protein-4, Prostate Specific Acid Phosphatase, Prostate Specific Antigen, pS2, PSCA, Rabies Virus, RAD1, Rad51, Raf1, Raf-1 (Phospho-specific), RAIDD, Ras, Rad18, Renal Cell Carcinoma, Ret Oncoprotein, Retinoblastoma, Retinoblastoma (Rb) (Phospho-specific Serine608), Retinoic Acid Receptor (b),

| | Retinoid X Receptor (hRXR), Retinol Binding Protein, Rhodopsin (Opsin), ROC, RPA/p32, RPA/p70, Ruv A, Ruv B, Ruv C, S100, S100A4, S100A6, SHP-1, SIM Ag (SIMA-4D3), SIRP a1, sm, SODD (Silencer of Death Domain), Somatostatin Receptor-I, SRC1 (Steroid Receptor Coactivator-1) Ab-1, SREBP-1 (Sterol Regulatory Element Binding Protein-1), SRF (Serum Response Factor), Stat-1, Stat3, Stat5, Stat5a, Stat5b, Stat6, Streptavidin, Superoxide Dismutase, Surfactant Protein A, Surfactant Protein B, Surfactant Protein B (Pro), Survivin, SV40 Large T Antigen, Syk, Synaptophysin, Synuclein, Synuclein beta, Synuclein pan, TACE (TNF-alpha converting enzyme) / ADAM17, TAG-72, tau, TdT, Tenascin, Testosterone, TGF beta 3, TGF-beta 2, Thomsen-Friedenreich Antigen, Thrombospondin, Thymidine Phosphorylase, Thymidylate Synthase, Thymine Glycols, Thyroglobulin, Thyroid Hormone Receptor beta, Thyroid Hormone Receptor, Thyroid Stimulating Hormone (TSH), TID-1, TIMP-1, TIMP-2, TNF alpha, TNFa, TNR-R2, Topo II beta, Topoisomerase IIa, Toxoplasma Gondii, TR2, TRADD, Transforming Growth Factor a, Transglutaminase II, TRAP, Tropomyosin, TRP75 / gp75, TrxR2, TTF-1, Tubulin, Tubulin-a, Tubulin-b, Tyrosinase, Ubiquitin, UCP3, uPA, Urocortin, Vacular Endothelial Growth Factor(VEGF), Vimentin, Vinculin, Vitamin D Receptor (VDR), von Hippel-Lindau Protein, Wnt-1, Xanthine Oxidase, XPA, XPF, XPG, XRCC1, XRCC2, ZAP-70, Zip kinase |
|---|---|
| Known Cancer Genes | ABL1, ABL2, ACSL3, AF15Q14, AF1Q, AF3p21, AF5q31, AKAP9, AKT1, AKT2, ALDH2, ALK, ALO17, APC, ARHGEF12, ARHH, ARID1A, ARID2, ARNT, ASPSCR1, ASXL1, ATF1, ATIC, ATM, ATRX, BAP1, BCL10, BCL11A, BCL11B, BCL2, BCL3, BCL5, BCL6, BCL7A, BCL9, BCOR, BCR, BHD, BIRC3, BLM, BMPR1A, BRAF, BRCA1, BRCA2, BRD3, BRD4, BRIP1, BTG1, BUB1B, C12orf9, C15orf21, C15orf55, C16orf75, CANT1, CARD11, CARS, CBFA2T1, CBFA2T3, CBFB, CBL, CBLB, CBLC, CCNB1IP1, CCND1, CCND2, CCND3, CCNE1, CD273, CD274, CD74, CD79A, CD79B, CDH1, CDH11, CDK12, CDK4, CDK6, CDKN2A, CDKN2a(p14), CDKN2C, CDX2, CEBPA, CEP1, CHCHD7, CHEK2, CHIC2, CHN1, CIC, CIITA, CLTC, CLTCL1, CMKOR1, COL1A1, COPEB, COX6C, CREB1, CREB3L1, CREB3L2, CREBBP, CRLF2, CRTC3, CTNNB1, CYLD, D10S170, DAXX, DDB2, DDIT3, DDX10, DDX5, DDX6, DEK, DICER1, DNMT3A, DUX4, EBF1, EGFR, EIF4A2, ELF4, ELK4, ELKS, ELL, ELN, EML4, EP300, EPS15, ERBB2, ERCC2, ERCC3, ERCC4, ERCC5, ERG, ETV1, ETV4, ETV5, ETV6, EVI1, EWSR1, EXT1, EXT2, EZH2, FACL6, FAM22A, FAM22B, FAM46C, FANCA, FANCC, FANCD2, FANCE, FANCF, FANCG, FBXO11, FBXW7, FCGR2B, FEV, FGFR1, FGFR1OP, FGFR2, FGFR3, FH, FHIT, FIP1L1, FLI1, FLJ27352, FLT3, FNBP1, FOXL2, FOXO1A, FOXO3A, FOXP1, FSTL3, FUBP1, FUS, FVT1, GAS7, GATA1, GATA2, GATA3, GMPS, GNA11, GNAQ, GNAS, GOLGA5, GOPC, GPC3, GPHN, GRAF, HCMOGT-1, HEAB, HERPUD1, HEY1, HIP1, HIST1H4I, HLF, HLXB9, HMGA1, HMGA2, HNRNPA2B1, HOOK3, HOXA11, HOXA13, HOXA9, HOXC11, HOXC13, HOXD11, HOXD13, HRAS, HRPT2, HSPCA, HSPCB, IDH1, IDH2, IGH@, IGK@, IGL@, IKZF1, IL2, IL21R, IL6ST, IL7R, IRF4, IRTA1, ITK, JAK1, JAK2, JAK3, JAZF1, JUN, KDM5A, KDM5C, KDM6A, KDR, KIAA1549, KIT, KLK2, KRAS, KTN1, LAF4, LASP1, LCK, LCP1, LCX, LHFP, LIFR, LMO1, LMO2, LPP, LYL1, MADH4, MAF, MAFB, MALT1, MAML2, MAP2K4, MDM2, MDM4, MDS1, MDS2, MECT1, MED12, MEN1, MET, MITF, MKL1, MLF1, MLH1, MLL, MLL2, MLL3, MLLT1, MLLT10, MLLT2, MLLT3, MLLT4, MLLT6, MLLT7, MN1, MPL, MSF, MSH2, MSH6, MSI2, MSN, MTCP1, MUC1, MUTYH, MYB, MYC, MYCL1, MYCN, MYD88, MYH11, MYH9, MYST4, NACA, NBS1, NCOA1, NCOA2, NCOA4, NDRG1, NF1, NF2, NFE2L2, NFIB, NFKB2, NIN, NKX2-1, NONO, NOTCH1, NOTCH2, NPM1, NR4A3, NRAS, NSD1, NTRK1, NTRK3, NUMA1, NUP214, NUP98, OLIG2, OMD, P2RY8, PAFAH1B2, PALB2, PAX3, PAX5, PAX7, PAX8, PBRM1, PBX1, PCM1, PCSK7, PDE4DIP, PDGFB, PDGFRA, PDGFRB, PER1, PHOX2B, PICALM, PIK3CA, PIK3R1, PIM1, PLAG1, PML, PMS1, PMS2, PMX1, PNUTL1, POU2AF1, POU5F1, PPARG, PPP2R1A, PRCC, PRDM1, PRDM16, PRF1, PRKAR1A, |

| | |
|---|---|
| | PRO1073, PSIP2, PTCH, PTEN, PTPN11, RAB5EP, RAD51L1, RAF1, RALGDS, RANBP17, RAP1GDS1, RARA, RB1, RBM15, RECQL4, REL, RET, ROS1, RPL22, RPN1, RUNDC2A, RUNX1, RUNXBP2, SBDS, SDH5, SDHB, SDHC, SDHD, SEPT6, SET, SETD2, SF3B1, SFPQ, SFRS3, SH3GL1, SIL, SLC45A3, SMARCA4, SMARCB1, SMO, SOCS1, SOX2, SRGAP3, SRSF2, SS18, SS18L1, SSH3BP1, SSX1, SSX2, SSX4, STK11, STL, SUFU, SUZ12, SYK, TAF15, TAL1, TAL2, TCEA1, TCF1, TCF12, TCF3, TCF7L2, TCL1A, TCL6, TET2, TFE3, TFEB, TFG, TFPT, TFRC, THRAP3, TIF1, TLX1, TLX3, TMPRSS2, TNFAIP3, TNFRSF14, TNFRSF17, TNFRSF6, TOP1, TP53, TPM3, TPM4, TPR, TRA@, TRB@, TRD@, TRIM27, TRIM33, TRIP11, TSC1, TSC2, TSHR, TTL, U2AF1, USP6, VHL, VTI1A, WAS, WHSC1, WHSC1L1, WIF1, WRN, WT1, WTX, XPA, XPC, XPO1, YWHAE, ZNF145, ZNF198, ZNF278, ZNF331, ZNF384, ZNF521, ZNF9, ZRSR2 |
| Known Cancer Genes | AR, androgen receptor; ARPC1A, actin-related protein complex 2/3 subunit A; AURKA, Aurora kinase A; BAG4, BCl-2 associated anthogene 4; BCl2l2, BCl-2 like 2; BIRC2, Baculovirus IAP repeat containing protein 2; CACNA1E, calcium channel voltage dependent alpha-1E subunit; CCNE1, cyclin E1; CDK4, cyclin dependent kinase 4; CHD1L, chromodomain helicase DNA binding domain 1-like; CKS1B, CDC28 protein kinase 1B; COPS3, COP9 subunit 3; DCUN1D1, DCN1 domain containing protein 1; DYRK2, dual specificity tyrosine phosphorylation regulated kinase 2; EEF1A2, eukaryotic elongation transcription factor 1 alpha 2; EGFR, epidermal growth factor receptor; FADD, Fas-associated via death domain; FGFR1, fibroblast growth factor receptor 1, GATA6, GATA binding protein 6; GPC5, glypican 5; GRB7, growth factor receptor bound protein 7; MAP3K5, mitogen activated protein kinase kinase kinase 5; MED29, mediator complex subunit 5; MITF, microphthalmia associated transcription factor; MTDH, metadherin; NCOA3, nuclear receptor coactivator 3; NKX2-1, NK2 homeobox 1; PAK1, p21/CDC42/RAC1-activated kinase 1; PAX9, paired box gene 9; PIK3CA, phosphatidylinositol-3 kinase catalytic a; PLA2G10, phopholipase A2, group X; PPM1D, protein phosphatase magnesium-dependent 1D; PTK6, protein tyrosine kinase 6; PRKCI, protein kinase C iota; RPS6KB1, ribosomal protein s6 kinase 70kDa; SKP2, s-phase kinase associated protein; SMURF1, sMAD specific E3 ubiquitin protein ligase 1; SHH, sonic hedgehog homologue; STARD3, sTAR-related lipid transfer domain containing protein 3; YWHAQ, tyrosine 3-monooxygenase/tryptophan 5-monooxygenase activation protein, zeta isoform; ZNF217, zinc finger protein 217 |
| Mitotic Related Cancer Genes | Aurora kinase A (AURKA); Aurora kinase B (AURKB); Baculoviral IAP repeat-containing 5, survivin (BIRC5); Budding uninhibited by benzimidazoles 1 homolog (BUB1); Budding uninhibited by benzimidazoles 1 homolog beta, BUBR1 (BUB1B); Budding uninhibited by benzimidazoles 3 homolog (BUB3); CDC28 protein kinase regulatory subunit 1B (CKS1B); CDC28 protein kinase regulatory subunit 2 (CKS2); Cell division cycle 2 (CDC2)/CDK1 Cell division cycle 20 homolog (CDC20); Cell division cycle-associated 8, borealin (CDCA8); Centromere protein F, mitosin (CENPF); Centrosomal protein 110 kDa (CEP110); Checkpoint with forkhead and ring finger domains (CHFR); Cyclin B1 (CCNB1); Cyclin B2 (CCNB2); Cytoskeleton-associated protein 5 (CKAP5/ch-TOG); Microtubule-associated protein RP/ EB family member 1. End-binding protein 1, EB1 (MAPRE1); Epithelial cell transforming sequence 2 oncogene (ECT2); Extra spindle poles like 1, separase (ESPL1); Forkhead box M1 (FOXM1); H2A histone family, member X (H2AFX); Kinesin family member 4A (KIF4A); Kinetochore-associated 1 (KNTC1/ROD); Kinetochore-associated 2; highly expressed in cancer 1 (KNTC2/HEC1); Large tumor suppressor, homolog 1 (LATS1); Large tumor suppressor, homolog 2 (LATS2); Mitotic arrest deficient-like 1; MAD1 (MAD1L1); Mitotic arrest deficient-like 2; MAD2 (MAD2L1); Mps1 protein kinase (TTK); Never in mitosis gene a-related kinase 2 (NEK2); Ninein, GSK3b interacting protein (NIN); Non-SMC condensin I complex, subunit D2 (NCAPD2/CNAP1); Non-SMC condensin I complex, subunit H (NACPH/CAPH); Nuclear mitotic apparatus protein |

| | |
|---|---|
| | 1 (NUMA1); Nucleophosmin (nucleolar phosphoprotein B23, numatrin); (NPM1); Nucleoporin (NUP98); Pericentriolar material 1 (PCM1); Pituitary tumor-transforming 1, securin (PTTG1); Polo-like kinase 1 (PLK1); Polo-like kinase 4 (PLK4/SAK); Protein (peptidylprolyl cis/trans isomerase) NIMA-interacting 1 (PIN1); Protein regulator of cytokinesis 1 (PRC1); RAD21 homolog (RAD21); Ras association (RalGDS/AF-6); domain family 1 (RASSF1); Stromal antigen 1 (STAG1); Synuclein-c, breast cancer-specific protein 1 (SNCG, BCSG1); Targeting protein for Xklp2 (TPX2); Transforming, acidic coiled-coil containing protein 3 (TACC3); Ubiquitin-conjugating enzyme E2C (UBE2C); Ubiquitin-conjugating enzyme E2I (UBE2I/UBC9); ZW10 interactor, (ZWINT); ZW10, kinetochore-associated homolog (ZW10); Zwilch, kinetochore-associated homolog (ZWILCH) |
| Ribonucleoprotein complexes | Argonaute family member, Ago1, Ago2, Ago3, Ago4, GW182 (TNRC6A), TNRC6B, TNRC6C, HNRNPA2B1, HNRPAB, ILF2, NCL (Nucleolin), NPM1 (Nucleophosmin), RPL10A, RPL5, RPLP1, RPS12, RPS19, SNRPG, TROVE2, apolipoprotein, apolipoprotein A, apo A-I, apo A-II, apo A-IV, apo A-V, apolipoprotein B, apo B48, apo B100, apolipoprotein C, apo C-I, apo C-II, apo C-III, apo C-IV, apolipoprotein D (ApoD), apolipoprotein E (ApoE), apolipoprotein H (ApoH), apolipoprotein L, APOL1, APOL2, APOL3, APOL4, APOL5, APOL6, APOLD1 |
| Cytokine Receptors | 4-1BB, ALCAM, B7-1, BCMA, CD14, CD30, CD40 Ligand, CEACAM-1, DR6, Dtk, Endoglin, ErbB3, E-Selectin, Fas, Flt-3L, GITR, HVEM, ICAM-3, IL-1 R4, IL-1 RI, IL-10 Rbeta, IL-17R, IL-2Rgamma, IL-21R, LIMPII, Lipocalin-2, L-Selectin, LYVE-1, MICA, MICB, NRG1-beta1, PDGF Rbeta, PECAM-1, RAGE, TIM-1, TRAIL R3, Trappin-2, uPAR, VCAM-1, XEDAR |
| Prostate and colorectal cancer vesicles | ErbB3, RAGE, Trail R3 |
| Colorectal cancer vesicles | IL-1 alpha, CA125, Filamin, Amyloid A |
| Colorectal cancer v adenoma vesicles | Involucrin, CD57, Prohibitin, Thrombospondin, Laminin B1/b1, Filamin, 14.3.3 gamma, 14.3.3 Pan |
| Colorectal adenoma vesicles | Involucrin, Prohibitin, Laminin B1/b1, IL-3, Filamin, 14.3.3 gamma, 14.3.3 Pan, MMP-15 / MT2-MMP, hPL, Ubiquitin, and mRANKL |
| Brain cancer vesicles | Prohibitin, CD57, Filamin, CD18, b-2-Microglobulin, IL-2, IL-3, CD16, p170, Keratin 19, Pds1, Glicentin, SRF (Serum Response Factor), E3-binding protein (ARM1), Collagen II, SRC1 (Steroid Receptor Coactivator-1) Ab-1, Caldesmon, GFAP, TRP75 / gp75, alpha-1-antichymotrypsin, Hepatic Nuclear Factor-3B, PLAP, Tyrosinase, NF kappa B / p50, Melanoma (gp100), Cyclin E, 6-Histidine, Mucin 3 (MUC3), TdT, CD21, XPA, Superoxide Dismutase, Glycogen Synthase Kinase 3b (GSK3b), CD54/ICAM-1, Thrombospondin, Gai1, CD79a mb-1, IL-1 beta, Cytochrome c, RAD1, bcl-X, CD50/ICAM-3, Neurofilament, Alkaline Phosphatase (AP), ER Ca+2 ATPase2, PCNA, F.VIII/VWF, SV40 Large T Antigen, Paxillin, Fascin, CD165, GRIP1, Cdk8, Nucleophosmin (NPM), alpha-1-antitrypsin, CD32/Fcg Receptor II, Keratin 8 (phospho-specific Ser73), DR5, CD46, TID-1, MHC II (HLA-DQ), Plasma Cell Marker, DR3, Calmodulin, AIF (Apoptosis Inducing Factor), DNA Polymerase Beta, Vitamin D Receptor (VDR), Bcl10 / CIPER / CLAP / mE10, Neuron Specific Enolase, CXCR4 / Fusin, Neurofilament (68kDa), PDGFR, beta, Growth Hormone (hGH), Mast Cell Chymase, Ret Oncoprotein, and Phosphotyrosine |
| Melanoma vesicles | Caspase 5, Thrombospondin, Filamin, Ferritin, 14.3.3 gamma, 14.3.3 Pan, CD71 / Transferrin Receptor, and Prostate Apoptosis Response Protein-4 |
| Head and neck cancer vesicles | 14.3.3 Pan, Filamin, 14.3.3 gamma, CD71 / Transferrin Receptor, CD30, Cdk5, CD138, Thymidine Phosphorylase, Ruv 5, Thrombospondin, CD1, Von Hippel-Lindau Protein, CD46, Rad51, Ferritin, c-Abl, Actin, Muscle Specific, LewisB |
| Membrane proteins | carbonic anhydrase IX, B7, CCCL19, CCCL21, CSAp, HER-2/neu, BrE3, CD1, CD1a, CD2, CD3, CD4, CD5, CD8, CD11A, CD14, CD15, CD16, CD18, CD19, |

| | |
|---|---|
| | CD20, CD21, CD22, CD23, CD25, CD29, CD30, CD32b, CD33, CD37, CD38, CD40, CD40L, CD44, CD45, CD46, CD52, CD54, CD55, CD59, CD64, CD67, CD70, CD74, CD79a, CD80, CD83, CD95, CD126, CD133, CD138, CD147, CD154, CEACAM5, CEACAM-6, alpha-fetoprotein (AFP), VEGF, ED-B fibronectin, EGP-1, EGP-2, EGF receptor (ErbB1), ErbB2, ErbB3, Factor H, FHL-1, Flt-3, folate receptor, Ga 733,GROB, HMGB-1, hypoxia inducible factor (HIF), HM1.24, HER-2/neu, insulin-like growth factor (ILGF), IFN-$\gamma$, IFN-$\alpha$, IL-$\beta$, IL-2R, IL-4R, IL-6R, IL-13R, IL-15R, IL-17R, IL-18R, IL-2, IL-6, IL-8, IL-12, IL-15, IL-17, IL-18, IL-25, IP-10, IGF-1R, Ia, HM1.24, gangliosides, HCG, HLA-DR, CD66a-d, MAGE, mCRP, MCP-1, MIP-1A, MIP-1B, macrophage migration-inhibitory factor (MIF), MUC1, MUC2, MUC3, MUC4, MUC5, placental growth factor (P1GF), PSA (prostate-specific antigen), PSMA, PSMA dimer, PAM4 antigen, NCA-95, NCA-90, A3, A33, Ep-CAM, KS-1, Le(y), mesothelin, S100, tenascin, TAC, Tn antigen, Thomas-Friedenreich antigens, tumor necrosis antigens, tumor angiogenesis antigens, TNF-$\alpha$, TRAIL receptor (R1 and R2), VEGFR, RANTES, T101, cancer stem cell antigens, complement factors C3, C3a, C3b, C5a, C5 |
| Cluster of Differentiation (CD) proteins | CD1, CD2, CD3, CD4, CD5, CD6, CD7, CD8, CD9, CD10, CD11a, CD11b, CD11c, CD12w, CD13, CD14, CD15, CD16, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD43, CD44, CD45, CD46, CD47, CD48, CD49a, CD49b, CD49c, CD49d, CD49e, CD49f, CD53, CD54, CD55, CD56, CD57, CD58, CD59, CD61, CD62E, CD62L, CD62P, CD63, CD68, CD69, CD71, CD72, CD73, CD74, CD80, CD81, CD82, CD83, CD86, CD87, CD88, CD89, CD90, CD91, CD95, CD96, CD100, CD103, CD105, CD106, CD107, CD107a, CD107b, CD109, CD117, CD120, CD127, CD133, CD134, CD135, CD138, CD141, CD142, CD143, CD144, CD147, CD151, CD152, CD154, CD156, CD158, CD163, CD165, CD166, CD168, CD184, CDw186, CD195, CD197, CD209, CD202a, CD220, CD221, CD235a, CD271, CD303, CD304, CD309, CD326 |
| Interleukin (IL) proteins | IL-1, IL-2, IL-3, IL-4, IL-5, IL-6, IL-7, IL-8 or CXCL8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-35, IL-36 |
| IL receptors | CD121a/IL1R1, CD121b/IL1R2, CD25/IL2RA, CD122/IL2RB, CD132/IL2RG, CD123/IL3RA, CD131/IL3RB, CD124/IL4R, CD132/IL2RG, CD125/IL5RA, CD131/IL3RB, CD126/IL6RA, CD130/IR6RB, CD127/IL7RA, CD132/IL2RG, CXCR1/IL8RA, CXCR2/IL8RB/CD128, CD129/IL9R, CD210/IL10RA, CDW210B/IL10RB, IL11RA, CD212/IL12RB1, IR12RB2, IL13R, IL15RA, CD4, CDw217/IL17RA, IL17RB, CDw218a/IL18R1, IL20R, IL20R, IL21R, IL22R, IL23R, IL20R, LY6E, IL20R1, IL27RA, IL28R, IL31RA |
| Mucin (MUC) proteins | MUC1, MUC2, MUC3A, MUC3B, MUC4, MUC5AC, MUC5B, MUC6, MUC7, MUC8, MUC12, MUC13, MUC15, MUC16, MUC17, MUC19, and MUC20 |
| MUC1 isoforms | mucin-1 isoform 2 precursor or mature form (NP_001018016.1), mucin-1 isoform 3 precursor or mature form (NP_001018017.1), mucin-1 isoform 5 precursor or mature form (NP_001037855.1), mucin-1 isoform 6 precursor or mature form (NP_001037856.1), mucin-1 isoform 7 precursor or mature form (NP_001037857.1), mucin-1 isoform 8 precursor or mature form (NP_001037858.1), mucin-1 isoform 9 precursor or mature form (NP_001191214.1), mucin-1 isoform 10 precursor or mature form (NP_001191215.1), mucin-1 isoform 11 precursor or mature form (NP_001191216.1), mucin-1 isoform 12 precursor or mature form (NP_001191217.1), mucin-1 isoform 13 precursor or mature form (NP_001191218.1), mucin-1 isoform 14 precursor or mature form (NP_001191219.1), mucin-1 isoform 15 precursor or mature form (NP_001191220.1), mucin-1 isoform 16 precursor or mature form (NP_001191221.1), mucin-1 isoform 17 precursor or mature form (NP_001191222.1), mucin-1 isoform 18 precursor or mature form (NP_001191223.1), mucin-1 isoform 19 precursor or mature form (NP_001191224.1), mucin-1 isoform 20 precursor or mature form |

| | |
|---|---|
| | (NP_001191225.1), mucin-1 isoform 21 precursor or mature form (NP_001191226.1), mucin-1 isoform 1 precursor or mature form (NP_002447.4), ENSP00000357380, ENSP00000357377, ENSP00000389098, ENSP00000357374, ENSP00000357381, ENSP00000339690, ENSP00000342814, ENSP00000357383, ENSP00000357375, ENSP00000338983, ENSP00000343482, ENSP00000406633, ENSP00000388172, ENSP00000357378, P15941-1, P15941-2, P15941-3, P15941-4, P15941-5, P15941-6, P15941-7, P15941-8, P15941-9, P15941-10, secreted isoform, membrane bound isoform, CA 27.29 (BR 27.29), CA 15-3, PAM4 reactive antigen, underglycosylated isoform, unglycosylated isoform, CanAg antigen |
| MUC1 interacting proteins | ABL1, SRC, CTNND1, ERBB2, GSK3B, JUP, PRKCD, APC, GALNT1, GALNT10, GALNT12, JUN, LCK, OSGEP, ZAP70, CTNNB1, EGFR, SOS1, ERBB3, ERBB4, GRB2, ESR1, GALNT2, GALNT4, LYN, TP53, C1GALT1, C1GALT1C1, GALNT3, GALNT6, GCNT1, GCNT4, MUC12, MUC13, MUC15, MUC17, MUC19, MUC2, MUC20, MUC3A, MUC4, MUC5B, MUC6, MUC7, MUCL1, ST3GAL1, ST3GAL3, ST3GAL4, ST6GALNAC2, B3GNT2, B3GNT3, B3GNT4, B3GNT5, B3GNT7, B4GALT5, GALNT11, GALNT13, GALNT14, GALNT5, GALNT8, GALNT9, ST3GAL2, ST6GAL1, ST6GALNAC4, GALNT15, MYOD1, SIGLEC1, IKBKB, TNFRSF1A, IKBKG, MUC1 |
| Tumor markers | Alphafetoprotein (AFP), Carcinoembryonic antigen (CEA), CA-125, MUC-1, Epithelial tumor antigen (ETA), Tyrosinase, Melanoma-associated antigen (MAGE), p53 |
| Tumor markers | Alpha fetoprotein (AFP), CA15-3, CA27-29, CA19-9, CA-125, Calretinin, Carcinoembryonic antigen, CD34, CD99, CD117, Chromogranin, Cytokeratin (various types), Desmin, Epithelial membrane protein (EMA), Factor VIII, CD31 FL1, Glial fibrillary acidic protein (GFAP), Gross cystic disease fluid protein (GCDFP-15), HMB-45, Human chorionic gonadotropin (hCG), immunoglobulin, inhibin, keratin (various types), PTPRC (CD45), lymphocyte marker (various types, MART-1 (Melan-A), Myo D1, muscle-specific actin (MSA), neurofilament, neuron-specific enolase (NSE), placental alkaline phosphatase (PLAP), prostate-specific antigen, S100 protein, smooth muscle actin (SMA), synaptophysin, thyroglobulin, thyroid transcription factor-1, Tumor M2-PK, vimentin |
| Cell adhesion molecule (CAMs) | Immunoglobulin superfamily CAMs (IgSF CAMs), N-CAM (Myelin protein zero), ICAM (1, 5), VCAM-1, PE-CAM, L1-CAM, Nectin (PVRL1, PVRL2, PVRL3), Integrins, LFA-1 (CD11a+CD18), Integrin alphaXbeta2 (CD11c+CD18), Macrophage-1 antigen (CD11b+CD18), VLA-4 (CD49d+CD29), Glycoprotein IIb/IIIa (ITGA2B+ITGB3), Cadherins, CDH1, CDH2, CDH3, Desmosomal, Desmoglein (DSG1, DSG2, DSG3, DSG4), Desmocollin (DSC1, DSC2, DSC3), Protocadherin, PCDH1, T-cadherin, CDH4, CDH5, CDH6, CDH8, CDH11, CDH12, CDH15, CDH16, CDH17, CDH9, CDH10, Selectins, E-selectin, L-selectin, P-selectin, Lymphocyte homing receptor: CD44, L-selectin, integrin (VLA-4, LFA-1), Carcinoembryonic antigen (CEA), CD22, CD24, CD44, CD146, CD164 |
| Annexins | ANXA1; ANXA10; ANXA11; ANXA13; ANXA2; ANXA3; ANXA4; ANXA5; ANXA6; ANXA7; ANXA8; ANXA8L1; ANXA8L2; ANXA9 |
| Cadherins ("calcium-dependent adhesion") | CDH1, CDH2, CDH12, CDH3, Deomoglein, DSG1, DSG2, DSG3, DSG4, Desmocollin, DSC1, DSC2, DSC3, Protocadherins, PCDH1, PCDH10, PCDH11x, PCDH11y, PCDH12, FAT, FAT2, FAT4, PCDH15, PCDH17, PCDH18, PCDH19; PCDH20; PCDH7, PCDH8, PCDH9, PCDHA1, PCDHA10, PCDHA11, PCDHA12, PCDHA13, PCDHA2, PCDHA3, PCDHA4, PCDHA5, PCDHA6, PCDHA7, PCDHA8, PCDHA9, PCDHAC1, PCDHAC2, PCDHB1, PCDHB10, PCDHB11, PCDHB12, PCDHB13, PCDHB14, PCDHB15, PCDHB16, PCDHB17, PCDHB18, PCDHB2, PCDHB3, PCDHB4, PCDHB5, PCDHB6, PCDHB7, PCDHB8, PCDHB9, PCDHGA1, PCDHGA10, PCDHGA11, PCDHGA12, PCDHGA2; PCDHGA3, PCDHGA4, PCDHGA5, PCDHGA6, PCDHGA7, PCDHGA8, PCDHGA9, PCDHGB1, PCDHGB2, PCDHGB3, PCDHGB4, PCDHGB5, PCDHGB6, PCDHGB7, PCDHGC3, PCDHGC4, PCDHGC5, CDH9 (cadherin 9, type 2 (T1-cadherin)), CDH10 (cadherin 10, type 2 (T2-cadherin)), CDH5 (VE-cadherin (vascular endothelial)), CDH6 (K-cadherin (kidney)), CDH7 (cadherin 7, |

| | type 2), CDH8 (cadherin 8, type 2), CDH11 (OB-cadherin (osteoblast)), CDH13 (T-cadherin - H-cadherin (heart)), CDH15 (M-cadherin (myotubule)), CDH16 (KSP-cadherin), CDH17 (LI cadherin (liver-intestine)), CDH18 (cadherin 18, type 2), CDH19 (cadherin 19, type 2), CDH20 (cadherin 20, type 2), CDH23 (cadherin 23, (neurosensory epithelium)), CDH10, CDH11, CDH13, CDH15, CDH16, CDH17, CDH18, CDH19, CDH20, CDH22, CDH23, CDH24, CDH26, CDH28, CDH4, CDH5, CDH6, CDH7, CDH8, CDH9, CELSR1, CELSR2, CELSR3, CLSTN1, CLSTN2, CLSTN3, DCHS1, DCHS2, LOC389118, PCLKC, RESDA1, RET |
|---|---|
| ECAD (CDH1) downregulators | SNAI1/SNAIL, ZFHX1B/SIP1, SNAI2/SLUG, TWIST1, DeltaEF1 |
| ECAD upregulators | AML1, p300, HNF3 |
| ECAD interacting proteins | ACADVL, ACTG1, ACTN1, ACTN4, ACTR3, ADAM10, ADAM9, AJAP1, ANAPC1, ANAPC11, ANAPC4, ANAPC7, ANK2, ANP32B, APC2, ARHGAP32, ARPC2, ARVCF, BOC, C1QBP, CA9, CASP3, CASP8, CAV1, CBLL1, CCNB1, CCND1, CCT6A, CDC16, CDC23, CDC26, CDC27, CDC42, CDH2, CDH3, CDK5R1, CDON, CDR2, CFTR, CREBBP, CSE1L, CSNK2A1, CTNNA1, CTNNB1, CTNND1, CTNND2, DNAJA1, DRG1, EGFR, EP300, ERBB2, ERBB2IP, ERG, EZR, FER, FGFR1, FOXM1, FRMD5, FYN, GBAS, GNA12, GNA13, GNB2L1, GSK3B, HDAC1, HDAC2, HSP90AA1, HSPA1A, HSPA1B, HSPD1, IGHA1, IQGAP1, IRS1, ITGAE, ITGB7, JUP, KIFC3, KLRG1, KRT1, KRT9, LIMA1, LMNA, MAD2L2, MAGI1, MAK, MDM2, MET, MYO6, MYO7A, NDRG1, NEDD9, NIPSNAP1, NKD2, PHLPP1, PIP5K1C, PKD1, PKP4, PLEKHA7, POLR2E, PPP1CA, PRKD1, PSEN1, PTPN1, PTPN14, PTPRF, PTPRM, PTPRQ, PTTG1, PVR, PVRL1, RAB8B, RRM2, SCRIB, SET, SIX1, SKI, SKP2, SRC, TACC3, TAS2R13, TGM2, TJP1, TK1, TNS3, TTK, UBC, USP9X, VCL, VEZT, XRCC5, YAP1, YES1, ZC3HC1 |
| Epithelial-mesenchymal transition (EMT) | SERPINA3, ACTN1, AGR2, AKAP12, ALCAM, AP1M2, AXL, BSPRY, CCL2, CDH1, CDH2, CEP170, CLDN3, CLDN4, CNN3, CYP4X1, DNMT3A, DSG3, DSP, EFNB2, EHF, ELF3, ELF5, ERBB3, ETV5, FLRT3, FOSB, FOSL1, FOXC1, FX YD 5, GPDIL, HMGA1, HMGA2, HOPX, IFI16, IGFBP2, IHH, IKBIP, IL-11, IL-18, IL6, IL8, ITGA5, ITGB3, LAMB1, LCN2, MAP7, MB, MMP7, MMP9, MPZL2, MSLN, MTA3, MTSS1, OCLN, PCOLCE2, PECAM1, PLAUR, PLXNB1, PPL, PPP1R9A, RASSF8, SCNN1A, SERPINB2, SERPINE1, SFRP1, SH3YL1, SLC27A2, SMAD7, SNAI1, SNAI2, SPARC, SPDEF, SRPX, STAT5A, TBX2, TJP3, TMEM125, TMEM45B, TWIST1, VCAN, VIM, VWF, XBP1, YBX1, ZBTB10, ZEB1, ZEB2 |

Examples of additional biomarkers that can be incorporated into the methods of the invention include without limitation those disclosed in International Patent Application Nos. PCT/US2012/042519 (WO 2012/174282), filed June 14, 2012 and PCT/US2012/050030 (WO 2013/022995), filed August 8, 2012.

The biomarkers or biosignature used to detect or assess any of the conditions or diseases disclosed herein can comprise one or more biomarkers in one of several different categories of markers, wherein the categories include without limitation one or more of: 1) disease specific biomarkers; 2) cell- or tissue-specific biomarkers; 3) vesicle-specific markers (e.g., general vesicle biomarkers); 4. angiogenesis-specific biomarkers; and 5) immunomodulatory biomarkers. Examples of all such markers are disclosed herein and known to a person having ordinary skill in the art. Furthermore, a biomarker known in the art that is characterized to have a role in a particular disease or condition can be adapted for use as a target in methods of the invention. In further embodiments, such biomarkers that are associated with vesicles can be all vesicle surface markers, or a combination of vesicle surface markers and vesicle payload markers (i.e., molecules enclosed by a vesicle). The biomarkers assessed can be from a combination of sources. For example, a disease or disorder may be detected or characterized by assessing a combination of proteins, nucleic acids, vesicles, circulating biomarkers, biomarkers from a tissue sample, and the like. In addition, as noted herein, the biological sample assessed can be any biological fluid, or can comprise individual components present within such biological fluid (e.g., vesicles, nucleic acids, proteins, or complexes thereof).

EpCAM is a pan-epithelial differentiation antigen that is expressed on many tumor cells. It is intricately linked with the Cadherin-Catenin pathway and hence the fundamental WNT pathway responsible for intracellular signalling and polarity. It has been used as an immunotherapeutic target in the treatment of gastrointestinal, urological and other carcinomas. (Chaudry MA, Sales K, Ruf P, Lindhofer H, Winslet MC (April 2007). Br. J. Cancer 96 (7): 1013-9.). It is expressed in undifferentiated pluripotent stem cells. EpCAM is a member of a family that includes at least two type I membrane proteins and functions as a homotypic calcium-independent cell adhesion molecule. Mutations in this gene result in congenital tufting enteropathy. EpCAM has been observed on the surface of microvesicles derived from cancer cell of various lineages. EpCAM is used as an exemplary surface antigen in various examples herein. One of skill will appreciate that various embodiments and examples using EpCAM can be applied to other microvesicle surface antigens as well.

**Therapeutics**

As used herein "therapeutically effective amount" refers to an amount of a composition that relieves (to some extent, as judged by a skilled medical practitioner) one or more symptoms of the disease or condition in a mammal. Additionally, by "therapeutically effective amount" of a composition is meant an amount that returns to normal, either partially or completely, physiological or biochemical parameters associated with or causative of a disease or condition. A clinician skilled in the art can determine the therapeutically effective amount of a composition in order to treat or prevent a particular disease condition, or disorder when it is administered, such as intravenously, subcutaneously, intraperitoneally, orally, or through inhalation. The precise amount of the composition required to be therapeutically effective will depend upon numerous factors, e.g., such as the specific activity of the active agent, the delivery device employed, physical characteristics of the agent, purpose for the administration, in addition to many patient specific considerations. But a determination of a therapeutically effective amount is within the skill of an ordinarily skilled clinician upon the appreciation of the disclosure set forth herein.

The terms "treating," "treatment," "therapy," and "therapeutic treatment" as used herein refer to curative therapy, prophylactic therapy, or preventative therapy. An example of "preventative therapy" is the prevention or lessening the chance of a targeted disease (e.g., cancer or other proliferative disease) or related condition thereto. Those in need of treatment include those already with the disease or condition as well as those prone to have the disease or condition to be prevented. The terms "treating," "treatment," "therapy," and "therapeutic treatment" as used herein also describe the management and care of a mammal for the purpose of combating a disease, or related condition, and includes the administration of a composition to alleviate the symptoms, side effects, or other complications of the disease, condition. Therapeutic treatment for cancer includes, but is not limited to, surgery, chemotherapy, radiation therapy, gene therapy, and immunotherapy.

As used herein, the term "agent" or "drug" or "therapeutic agent" refers to a chemical compound, a mixture of chemical compounds, a biological macromolecule, or an extract made from biological materials such as bacteria, plants, fungi, or animal (particularly mammalian) cells or tissues that are suspected of having therapeutic properties. The agent or drug can be purified, substantially purified or partially purified. An "agent", also includes a radiation therapy agent or a "chemotherapuetic agent."

As used herein, the term "diagnostic agent" refers to any chemical used in the imaging of diseased tissue, such as, e.g., a tumor.

As used herein, the term "chemotherapuetic agent" refers to an agent with activity against cancer, neoplastic, and/or proliferative diseases, or that has ability to kill cancerous cells directly.

As used herein, "pharmaceutical formulations" include formulations for human and veterinary use with no significant adverse toxicological effect. "Pharmaceutically acceptable formulation" as used herein refers to a composition or formulation that allows for the effective distribution of the nucleic acid molecules in the physical location most suitable for their desired activity.

As used herein the term "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated.

***Aptamer-Toxin Conjugates as a Cancer Therapeutic***

Extensive previous work has developed the concept of antibody-toxin conjugates ("immunoconjugates") as potential therapies for a range of indications, mostly directed at the treatment of cancer with a primary focus on hematological tumors. A variety of different payloads for targeted delivery have been tested in pre-clinical and clinical studies, including protein toxins, high potency small molecule cytotoxics, radioisotopes, and liposome-encapsulated drugs. While these efforts have successfully yielded three FDA-approved therapies for hematological tumors, immunoconjugates as a class (especially for solid tumors) have historically yielded disappointing results that have been attributable to multiple different properties of antibodies, including tendencies to develop neutralizing antibody responses to non-humanized antibodies, limited penetration in solid tumors, loss of target binding affinity as a result of toxin conjugation, and imbalances between antibody half-life and toxin conjugate half-life that limit the overall therapeutic index (reviewed by Reff and Heard, Critical Reviews in Oncology/Hematology, 40 (2001):25-35).

Aptamers are functionally similar to antibodies, except their absorption, distribution, metabolism, and excretion ("ADME") properties are intrinsically different and they generally lack many of the immune effector functions generally associated with antibodies (e.g., antibody-dependent cellular cytotoxicity, complement-dependent cytotoxicity). In comparing many of the properties of aptamers and antibodies previously described, several factors suggest that toxin-delivery via aptamers offers several concrete advantages over delivery with antibodies, ultimately affording them better potential as therapeutics. Several examples of the advantages of toxin-delivery via aptamers over antibodies are as follows:

1) Aptamer-toxin conjugates are entirely chemically synthesized. Chemical synthesis provides more control over the nature of the conjugate. For example, the stoichiometry (ratio of toxins per aptamer) and site of attachment can be precisely defined. Different linker chemistries can be readily tested. The reversibility of aptamer folding means that loss of activity during conjugation is unlikely and provides more flexibility in adjusting conjugation conditions to maximize yields.

2) Smaller size allows better tumor penetration. Poor penetration of antibodies into solid tumors is often cited as a factor limiting the efficacy of conjugate approaches. See Colcher, D., Goel, A., Pavlinkova, G., Beresford, G., Booth, B., Batra, S. K. (1999) "Effects of genetic engineering on the pharmacokinetics of antibodies," Q. J. Nucl. Med., 43: 132-139. Studies comparing the properties of unPEGylated anti-tenascin C aptamers with corresponding antibodies demonstrate efficient uptake into tumors (as defined by the tumor:blood ratio) and evidence that aptamer localized to the tumor is unexpectedly long-lived ($t_{1/2}$>12 hours) (Hicke, B. J., Stephens, A. W., "Escort aptamers: a delivery service for diagnosis and therapy", J. Clin. Invest., 106:923-928 (2000)).

3) Tunable PK. Aptamer half-life/metabolism can be easily tuned to match properties of payload, optimizing the ability to deliver toxin to the tumor while minimizing systemic exposure. Appropriate modifications to the aptamer backbone and addition of high molecular weight PEGs should make it possible to match the half-life of the aptamer to the intrinsic half-life of the conjugated toxin/linker, minimizing systemic exposure to non-functional toxin-bearing metabolites (expected if $t_{1/2}$(aptamer)<<$t_{1/2}$(toxin)) and reducing the likelihood that persisting unconjugated aptamer will functionally block uptake of conjugated aptamer (expected if $t_{1/2}$(aptamer)>>$t_{1/2}$ (toxin)).

4) Relatively low material requirements. It is likely that dosing levels will be limited by toxicity intrinsic to the cytotoxic payload. As such, a single course of treatment will likely entail relatively small (<100 mg) quantities of aptamer, reducing the likelihood that the cost of oligonucleotide synthesis will be a barrier for aptamer-based therapies.

5) Parenteral administration is preferred for this indication. There will be no special need to develop alternative formulations to drive patient/physician acceptance.

The disclosure provides a pharmaceutical composition comprising a therapeutically effective amount of an aptamer or a salt thereof, and a pharmaceutically acceptable carrier or diluent. The disclosure also provides a pharmaceutical composition comprising a therapeutically effective amount of the aptamer or a salt thereof, and a pharmaceutically acceptable carrier or diluent. Relatedly, the disclosure provides a method of treating or ameliorating a disease or disorder, comprising administering the pharmaceutical composition to a subject in need thereof. Administering a therapeutically effective amount of the composition to the subject may result in: (a) an enhancement of the delivery of the active agent to a disease site relative to delivery of the active agent alone; or (b) an enhancement of microvesicles clearance resulting in a decrease of at least 10%, 20%, 30%, 40%, 50%, 60%, 70%, 80%, or 90% in a blood level of microvesicles targeted by the aptamer; or (c) an decrease in biological activity of microvesicles targeted by the aptamer of at least 10%, 20%, 30%, 40%, 50%, 60%, 70%, 80%, or 90%. The biological activity of microvesicles may comprise immune suppression or transfer of genetic information. The disease or disorder can include without limitation those disclosed herein. For example, the disease or disorder may comprise a neoplastic, proliferative, or inflammatory, metabolic, cardiovascular, or neurological disease or disorder. See, e.g., section "Phenotypes."

## Aptamer Identification Methods

Nucleic acid sequences fold into secondary and tertiary motifs particular to their nucleotide sequence. These motifs position the positive and negative charges on the nucleic acid sequences in locations that enable the sequences to bind to specific locations on target molecules, e.g., proteins and other amino acid sequences. These binding sequences are known in the field as aptamers. Due to the trillions of possible unique nucleotide sequences in even a relatively short stretch of nucleotides (e.g., 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides), a large variety of motifs can be generated, resulting in aptamers for almost any desired protein or other target.

Aptamers are created by randomly generating oligonucleotides of a specific length, typically 20-80 base pairs long, e.g., 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 or 80 base pairs. These random oligonucleotides are then incubated with the protein target of interest. After several wash steps, the oligonucleotides that bind to the target are collected and amplified. The amplified aptamers are then added to the target and the process is repeated, often 15-20 times. A common version of this process known to those of skill in the art as the SELEX method.

The end result comprises one or more aptamer with high affinity to the target. The disclosure provides further processing of such resulting aptamers that can be use to provide desirable characteristics: 1) competitive binding assays to identify aptamers to a desired epitope; 2) motif analysis to identify high affinity binding aptamers *in silico*; and 3) microvesicle-based aptamer selection assays to identify aptamers that can be used to detect a particular disease. The methods are described in more detail below and further in the Examples.

The disclosure further contemplates aptamer sequences that are highly homologous to the sequences that are discovered by the methods. "High homology" typically refers to a homology of 40% or higher, preferably 60% or higher, more preferably 80% or higher, even more preferably 85%, 90%, 91%, 92%, 93%, 94%, 95%, 96%, 97%, 98%, 99% or higher between a polynucleotide sequence sequence and a reference sequence. The reference sequence may comprise the sequence of one or more aptamer provided herein. Percent homologies (also referred to as percent identity) are typically carried out between two optimally aligned sequences. Methods of alignment of sequences for comparison are well-known in the art. Optimal alignment of sequences and comparison can be conducted, e.g., using the algorithm in "Wilbur and Lipman, Proc Natl Acad Sci USA 80: 726-30 (1983)". Homology calculations can also be performed using BLAST, which can be found on the NCBI server at: www.ncbi.nlm.nih.gov/BLAST/ (Altschul S F, et al, Nucleic Acids Res. 1997; 25(17):3389-402; Altschul S F, et al, J Mol. Biol. 1990; 215(3):403-10). In the case of an isolated polynucleotide which is longer than or equivalent in length to the reference sequence, e.g., a sequence identified by the methods herein, the comparison is made with the full length of the reference sequence. Where the isolated polynucleotide is shorter than the reference sequence, e.g., shorter than a sequence identified by the methods herein, the comparison is made to a segment of the reference sequence of the same length (excluding any loop required by the homology calculation).

The disclosure further contemplates aptamer sequences that are functional fragments of the sequences that are discovered by the methods. In the context of an aptamer sequence, a "functional fragment" of the aptamer sequence may comprise a subsequence that binds to the same target as the full length sequence. In some instances, a candidate aptamer sequence is from a member of a library that contains a 5' leader sequences and/or a 3' tail sequence. Such leader sequences or tail sequences may serve to facilitate primer binding for amplification or capture, etc. The functional fragment of the full length sequence may comprise the subsequence of the candidate aptamer sequence absent the leader and/or tail sequences.

## Competitive Antibody Addition

Known aptamer production methods may involve eluting all bound aptamers from the target sequence. In some cases, this is not sufficient to identify the desired aptamer sequence. For example, when trying to replace an antibody in an assay, it may be desirable to only collect aptamers that bind to the specific epitope of the antibody being replaced. The disclosure provides a method comprising addition of an antibody that is to be replaced to the aptamer/target reaction in order to allow for the selective collection of aptamers which bind to the antibody epitope. The method may comprise incubating a reaction mixture comprising randomly generated oligonucleotides with a target of interest, removing unbound aptamers from the reaction mixture that do not bind the target, adding an antibody to the reaction mixture that binds to that epitope of interest, and collecting the aptamers that are displaced by the antibody. The target can be a protein. See, e.g., **FIG. 1,** which illustrates the method for identifying an aptamer to a specific epitope of EpCam.

## Motif Analysis

In most aptamer experiments, multiple aptamer sequences are identified that bind to the target. These aptamers will have various binding affinities. It can be time consuming and laborious to generate quantities of these many aptamers sufficient to assess the affinities of each. To identify large numbers of aptamers with the highest affinities without physically screening large subsets, the disclosure provides a method comprising the analysis of the two dimensional structure of one or more high affinity aptamers to the target of interest. The method may comprise screening the database for aptamers that have similar two-dimensional structures, or motifs, but not necessarily similar primary sequences. The method may comprise identifying a high affinity aptamer using traditional methods such as disclosed herein or known in the art (e.g. surface plasmon resonance binding assay, see **FIG. 5),** approximating the two-dimensional structure of the high affinity aptamer, and identifying aptamers from a pool of sequences that are predicted to have a similar two-dimensional structure to the high affinity aptamer. The method thereby provides a pool of candidates that also bind the target of interest. The two-dimensional structure of an oligo can be predicting using methods known in the art, e.g., via free energy ($\Delta$G) calculations performed using a commercially available software program such as Vienna or mFold, for example as described in Mathews, D., Sabina, J., Zucker, M. & Turner, H. Expanded sequence dependence of thermodynamic parameters provides robust prediction of RNA secondary structure. J. Mol. Biol. 288, 911-940 (1999); Hofacker et al., Monatshefte f. Chemie 125: 167-188 (1994); and Hofacker, I. L. Vienna RNA secondary structure server. Nucleic Acids Res. 31, 3429-3431 (2003). See **FIG. 3.** The pool of sequences can be sequenced from a pool of randomly generated aptamer candidates using a high-throughput sequencing platform, such as the Ion Torrent platform from Life Technologies. Identifying aptamers from a pool of sequences that are predicted to have a similar two-dimensional structure to the high affinity aptamer may comprise loading the resulting sequences into the software program of choice to identify members of the pool of sequences with similar two-dimensional structures as the high affinity aptamer. The affinities of the pool of sequences can then be determined in situ, e.g., surface plasmon resonance binding assay or the like.

**Aptamer Subtraction Methods**

In order to develop an assay to detect a disease, for example, cancer, one typically screens a large population of known biomarkers from normal and diseased patients in order to identify markers that correlate with disease. This process only works if discriminating markers are already described. In order to address this problem, the disclosure provides a method comprising subtracting out non-discriminating aptamers from a large pool of aptamers by incubating them initially with non-target microvesicles or cells. The non-target cells can be normal cells or microvesicles shed therefrom. The aptamers that did not bind to the normal microvesicles or cells are then incubated with diseased microvesicles or cells. The aptamers that bind to the diseased microvesicles or cells but that did not bind to the normal cells are then possible candidates for an assay to detect the disease. This process is independent of knowing the existence of a particular marker in the diseased sample.

Subtraction methods can be used to identify aptamers that preferentially recognize a desired population of targets. The subtraction method may be used to identify aptamers that preferentially recognize target from a diseased target population over a control (e.g., normal or non-diseased) population. The diseased target population may be a population of vesicles from a diseased individual or individuals, whereas the control population comprises vesicles from a non-diseased individual or individuals. The disease can be a cancer or other disease disclosed herein or known in the art. Accordingly, the method provides aptamers that preferentially identify disease targets versus control targets.

Circulating microvesicles can be isolated from control samples, e.g., plasma from "normal" individuals that are absent a disease of interest, such as an absence of cancer. Vesicles in the sample are isolated using a method disclosed herein or as known in the art. For example, vesicles can be isolated from the plasma by one of the following methods: filtration, ultrafiltration, nanomembrane ultrafiltration, the ExoQuick reagent (System Biosciences, Inc., Mountain View, CA), centrifugation, ultracentrifugation, using a molecular crowding reagent (e.g., TEXIS from Life Technologies), polymer precipitation (e.g., polyethylene glycol (PEG)), affinity isolation, affinity selection, immunoprecipitation, chromatography, size exclusion, or a combination of any of these methods. The microvesicles isolated in each case will be a mixture of vesicle types and will be various sizes although ultracentrifugation methods may have more tendency to produce exosomal-sized vesicles. Randomly generated oligonucleotide libraries (e.g., produced as described in the **Examples** herein) are incubated with the isolated normal vesicles. The aptamers that do not bind to these vesicles are isolated, e.g., by spinning down the vesicles and collecting the supernatant containing the non-binding aptamers. These non-binding aptamers are then contacted with vesicles isolated from diseased patients (e.g., using the same methods as described above) to allow the aptamers to recognize the disease vesicles. Next, aptamers that are bound to the diseased vesicles are collected. The vesicles may be isolated then lysed using a chaotropic agent (e.g., SDS or a similar detergent), and the aptamers are then captured by running the lysis mixture over an affinity column. The affinity column may comprise streptavidin beads in the case of biotin conjugated aptamer pools. The isolated aptamers are the amplified. The process can then then repeated, e.g., 1, 2, 3, 4, 5, 6, 7, 8, 9, 10, 11, 12, 13, 14, 15, 16, 17, 18, 19 or 20 or more times.

In one aspect, an aptamer profile is identified that can be used to characterize a biological sample of interest. A pool of randomly generated oligonucleotides, e.g., at least 10, $10^2$, $10^3$, $10^4$, $10^5$, $10^6$, $10^7$, $10^8$, $10^9$, $10^{10}$, $10^{11}$, $10^{12}$, $10^{13}$, $10^{14}$, $10^{15}$, $10^{16}$, $10^{17}$, $10^{18}$, $10^{19}$ or at least $10^{20}$ oligonucleotides, may be contacted with a biological component or target of interest from a control population. The oligonucleotides that do not bind the biological component or target of interest from the control population are isolated and then contacted with a biological component or target of interest from a test population. The oligonucleotides that bind the biological component or target of interest from the test population are retained. The retained oligonucleotides can be used to repeat the process by contacting the retained oligonucleotides with the biological component or target of interest from the control population, isolating the retained oligonucleotides that do not bind the the biological component or target of interest from the control population, and again contacting these isolated oligonucleotides with the biological component or target of interest from the test population and isolating the binding oligonucleotides. The "component" or "target" can be anything that is present in sample to which the oligonucleotides are capable of binding (e.g., polypeptides, peptide, nucleic acid molecules, carbodyhrates, lipids, etc.). The process can then then repeated, e.g., 1, 2, 3, 4, 5, 6, 7, 8, 9, 10, 11, 12, 13, 14, 15, 16, 17, 18, 19 or 20 or more times. The resulting oligonucleotides comprise aptamers that can differentially detect the test population versus the control. These aptamers provide an aptamer profile, which comprises a biosignature that is determined using one or more aptamer, e.g., a biosignature comprising a presense or level of the component or target which is detected using the one or more aptamer.

An exemplary process is illustrated in **FIG. 4,** which demonstrates the method to identify aptamer that preferentially recognize cancer vesicles using vesicles from normal (non-cancer) individuals as a control. In the figure, exosomes are exemplified but one of skill will appreciate that other microvesicles can be used in the same manner. The resulting aptamers can provide a profile that can differentially detect the cancer vesicles from the normal vesicles. One of skill will appreciate that the same steps can be used to derive an aptamer profile to characterize any disease or condition of interest.

The disclosure provides an isolated polynucleotide that encodes a polypeptide, or a fragment thereof, identified by the methods above. The disclosure further provides an isolated polynucleotide having a nucleotide sequence that is at least 60% identical to the nucleotide sequence identified by the methods above. More preferably, the isolated nucleic acid molecule is at least 65%, 70%, 75%, 80%, 85%, 90%, 91%, 92%, 93%, 94%, 95%, 96%, 97%, 98%, 99% or more, identical to the nucleotide sequence identified by the methods above. In the case of an isolated polynucleotide which is longer than or equivalent in length to the reference sequence, e.g., a sequence identified by the methods above, the comparison is made with the full length of the reference sequence. Where the isolated polynucleotide is shorter than the reference sequence, e.g., shorter than a sequence identified by the methods above, the comparison is made to a segment of the reference sequence of the same length (excluding any loop required by the homology calculation).

In a related aspect, the disclosure provides a method of characterizing a biological phenotype using an aptamer profile. The aptamer profile can be determined using the method above. The aptamer profile can be determined for a test sample and compared to a control aptamer profile. The phenotype may be a disease or disorder such as a cancer. Characterizing the phenotype can include without limitation providing a diagnosis, prognosis, or theranosis. Thus, the aptamer profile can provide a diagnostic, prognostic and/or theranostic readout for the subject from whom the test sample is obtained.

An aptamer profile may be determined for a test sample by contacting a pool of aptamer molecules to the test sample, contacting the same pool of aptamers to a control sample, and identifying one or more aptamer molecules that differentially bind a component or target in the test sample but not in the control sample (or vice versa). A "component" or "target" as used in the context of the biological test sample or control sample can be anything that is present in sample to which the aptamers are capable of binding (e.g., polypeptides, peptide, nucleic acid molecules, carbodyhrates, lipids, etc.). For example, if a sample is a plasma or serum sample, the aptamer molecules may bind a polypeptide biomarker that is solely expressed or differentially expressed (over- or underexpressed) in a disease state as compared to a non-diseased subject. Comparison of the aptamer profile in the test sample as compared to the control sample may be based on qualitative and quantitative measure of aptamer binding (e.g., binding versus no binding, or level of binding in test sample versus different level of binding in the reference control sample).

In an aspect, the disclosure provides a method of identifying a target-specific aptamer profile, comprising contacting a biological test sample with a pool of aptamer molecules, contacting the pool to a control biological sample, identifying one or more aptamers that bind to a component in said test sample but not to the control sample, thereby identifying an aptamer profile for said biological test sample. A pool of aptamers may be selected against a disease sample and compared to a reference sample, the aptamers in a subset that bind to a component(s) in the disease sample but not in the reference sample can be sequenced using conventional sequencing techniques to identify the subset that bind, thereby identifying an aptamer profile for the particular disease sample. In this way, the aptamer profile provides an individualized platform for detecting disease in other samples that are screened. Furthermore, by selecting an appropriate reference or control sample, the aptamer profile can provide a diagnostic, prognostic and/or theranostic readout for the subject from whom the test sample is obtained.

In a related aspect, the disclosure provides a method of selecting a pool of aptamers, comprising: (a) contacting a biological control sample with a pool of oligonucleotides; (b) isolating a first subset of the pool of oligonucleotides that do not bind the biological control sample; (c) contacting the biological test sample with the first subset of the pool of oligonucleotides; and (d) isolating a second subset of the pool of oligonucleotides that bind the biological test sample, thereby selecting the pool of aptamers. The pool of oligonucleotides may comprise any number of desired sequences, e.g., at least 10, $10^2$, $10^3$, $10^4$, $10^5$, $10^6$, $10^7$, $10^1$, $10^9$, $10^{10}$, $10^{11}$, $10^{11}$, $10^{11}$, $10^{14}$, $10^{11}$, $10^{16}$, $10^{17}$, $10^{18}$, $10^{19}$ or at least $10^{20}$ oligonucleotides may be present in the starting pool. Steps (a)-(d) may be repeated to further hone the pool of aptamers. These steps may be repeated at least 1, 2, 3, 4, 5, 6, 7, 8, 9, 10, 11, 12, 13, 14, 15, 16, 17, 18, 19 or at least 20 times.

As described herein, the biological test sample and biological control sample may comprise microvesicles. In an embodiment, the biological test sample and optionally biological control sample comprise a bodily fluid. The bodily fluid may comprise without limitation peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, Cowper's fluid, pre-ejaculatory fluid, female ejaculate, sweat, fecal matter, hair, tears, cyst fluid, pleural fluid, peritoneal fluid, malignant fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids. Tthe biological test sample and optionally biological control may also comprise a tumor sample, e.g., cells from a tumor or tumor tissue. In other embodiments, the biological test sample and optionally biological control sample comprise a cell culture medium. In embodiments, the biological test sample comprises a diseased sample and the biological control sample comprises a non-diseased sample. Accordingly, the pool of aptamers may be used to provide a diagnostic, prognostic and/or theranostic readout a disease.

As noted, the invention can be used to assess microvesicles. Microvesicles are powerful biomarkers because the vesicles provide one biological entity that comprises multiple pieces of information. For example as described, a vesicle can have multiple surface antigens, each of which provides complementary information. Consider a cancer marker and a tissue specific marker. If both markers are individually present in a sample, e.g., both are circulating proteins or nucleic acids, it may not be ascertainable whether the cancer marker and the tissue specific marker are derived from the same anatomical locale. However, if both the cancer marker and the tissue specific marker are surface antigens on a single microvesicle, the vesicle itself links the two markers and provides an indication of a disease (via the cancer marker) and origin of the disease (via the tissue specific marker). Furthermore, the vesicle can have any number of surface antigens and also payload that can be assessed. Accordingly, the disclosure provides a method for identifying binding agents comprising contacting a plurality of extracellular microvesicles with a randomly generated library of binding agents, identifying a subset of the library of binding agents that have an affinity to one or more components of the extracellular microvesicles. The binding agents may comprise aptamers, antibodies, and/or any other useful type of binding agent disclosed herein or known in the art.

In a related aspect, the disclosure provides a method for identifying a plurality of target ligands comprising, (a) contacting a reference microvesicle population with a plurality of ligands that are capable of binding one or more microvesicle surface markers, (b) isolating a plurality of reference ligands, wherein the plurality of reference ligands comprise a subset of the plurality of ligands that do not have an affinity for the reference microvesicle population; (c) contacting one or more test microvesicle with the plurality of reference ligands; and (d) identifying a subset of ligands from the the plurality of reference ligands that form complexes with a surface marker on the one or more test microvesicle, thereby identifying the plurality of target ligands. The term "ligand" can refer a molecule, or a molecular group, that binds to another chemical entity to form a larger complex. Accordingly, a binding agent comprises a ligand. The plurality of ligands may comprise aptamers, antibodies and/or other useful binding agents described herein or known in the art.

The disclosure further provides kits comprising one or more reagent to carry out the methods above. The one or more reagent may comprise a library of potential binding agents that comprises one or more of an aptamer, antibody, and other useful binding agents described herein or known in the art.

### Negative and Positive Aptamer Selection

Aptamers can be used in various biological assays, including numerous types of assays which rely on a binding agent. For example, aptamers can be used instead of or along side antibodies in immune-based assays. The disclosure provides an aptamer screening method that identifies aptamers that do not bind to any surfaces (substrates, tubes, filters, beads, other antigens, etc.) throughout the assay steps and bind specifically to an antigen of interest. The assay relies on negative selection to remove aptamers that bind non-target antigen components of the final assay. The negative selection is followed by positive selection to identify aptamers that bind the desired antigen.

In an aspect, the disclosure provides a method of identifying an aptamer specific to a target of interest, comprising (a) contacting a pool of candidate aptamers with one or more assay components, wherein the assay components do not comprise the target of interest; (b) recovering the members of the pool of candidate aptamers that do not bind to the one or more assay components in (a); (c) contacting the members of the pool of candidate aptamers recovered in (b) with the target of interest in the presence of one or more confounding target; and (d) recovering a candidate aptamer that binds to the target of interest in step (c), thereby identifying the aptamer specific to the target of interest. In the method, steps (a) and (b) provide negative selection to remove aptamers that bind non-target entities. Conversely, steps (c) and (d) provide positive selection by identifying aptamers that bind the target of interest but not other confounding targets, e.g., other antigens that may be present in a biological sample which comprises the target of interest. The pool of candidate aptamers may comprise at least $10$, $10^2$, $10^3$, $10^4$, $10^5$, $10^6$, $10^7$, $10^8$, $10^9$, $10^{10}$, $10^{11}$, $10^{12}$, $10^{13}$, $10^{14}$, $10^{15}$, $10^{16}$, $10^{17}$, $10^{18}$, $10^{19}$ or at least $10^{20}$ nucleic acid sequences. One illustrative approach for performing the method is provided in **Example 7.**

Steps (a)-(b) may be optional. Steps (a)-(b) may be repeated at least 1, 2, 3, 4, 5, 6, 7, 8, 9, 10, 11, 12, 13, 14, 15, 16, 17, 18, 19 or at least 20 times before positive selection in step (c) is performed. The positive selection can also be performed in multiple rounds. Steps (c)-(d) can be repeated at least 1, 2, 3, 4, 5, 6, 7, 8, 9, 10, 11, 12, 13, 14, 15, 16, 17, 18, 19 or at least 20 times before identifying the aptamer specific to the target of interest. Multiple rounds may provide improved stringency of selection.

The one or more assay components contacted with the aptamer pool during negative selection may comprise one or more of a substrate, a bead, a planar array, a column, a tube, a well, or a filter. One of skill will appreciate that the assay components can include any substance that may be part of a biological assay.

The target of interest can be any appropriate entity that can be detected when recognized by an aptamer. In an embodiment, the target of interest comprises a protein or polypeptide. As used herein, "protein," "polypeptide" and "peptide" are used interchangeably unless stated otherwise. The target of interest can be a nucleic acid, including DNA, RNA, and various subspecies of any thereof as disclosed herein or known in the art. The target of interest can comprise a lipid. The target of interest can comprise a carbohydrate. The target of interest can also be a complex, e.g., a complex comprising protein, nucleic acids, lipids and/or carbohydrates. The target of interest may comprise a microvesicle. In such cases, the aptamer can be a binding agent to a microvesicle surface antigen, e.g., a protein. General microvesicle surface antigens include tetraspanin, CD9, CD63, CD81, CD63, CD9, CD81, CD82, CD37, CD53, Rab-5b, Annexin V, and MFG-E8. Additional general microvesicle surface antigens are provided in **Table 3** herein.

The microvesicle surface antigen can also be a biomarker of a disease or disorder. In such cases, the aptamer may be used to provide a diagnosis, prognosis or theranosis of the disease or disorder. For example, the one or more protein may comprise one or more of PSMA, PCSA, B7H3, EpCam, ADAM-10, BCNP, EGFR, IL1B, KLK2, MMP7, p53, PBP, SERPINB3, SPDEF, SSX2, and SSX4. These markers can be used detect a prostate cancer. Additional microvesicle surface antigens are provided in **Tables 3-4** herein.

The one or more confounding target can be an antigen other than the target of interest. For example, a confounding target can be another entity that may be present in a sample to be assayed. As a non-limiting example, consider that the sample to be assessed is a plasma sample from an individual. The target of interest may be a protein, e.g., a microvesicle surface antigen, which is present in the sample. In this case, a confounding target could be selected from any other antigen that is likely to be present in the plasma sample. Accordingly, the positive selection should provide candidate aptamers that recognize the target of interest but have minimal, if any, interactions with the confounding targets. The target of interest and the one or more confounding target may comprise the same type of biological entity, e.g., all protein, all nucleic acid, all carbohydrate, or all lipids. As a non-limiting example, the target of interest can be a protein selected from the group consisting of SSX4, SSX2, PBP, KLK2, SPDEF, and EpCAM, and the one or more confounding target comprises the other members of this group. The target of interest and the one or more confounding target may comprise different types of biological entities, e.g., any combination of protein, nucleic acid, carbohydrate, and lipids. The one or more confounding targets may also comprise different types of biological entities, e.g., any combination of protein, nucleic acid, carbohydrate, and lipids.

The disclosure provides an isolated polynucleotide, or a fragment thereof, identified by the methods above. The disclosure further provides an isolated polynucleotide having a nucleotide sequence that is at least 60% identical to the nucleotide sequence identified by the methods above. More preferably, the isolated nucleic acid molecule is at least 65%, 70%, 75%, 80%, 85%, 90%, 91%, 92%, 93%, 94%, 95%, 96%, 97%, 98%, 99% or more, identical to the nucleotide sequence identified by the methods above. In the case of an isolated polynucleotide which is longer than or equivalent in length to the reference sequence, e.g., a sequence identified by the methods above, the comparison is made with the full length of the reference sequence. Where the isolated polynucleotide is shorter than the reference sequence, e.g., shorter than a sequence identified by the methods above, the comparison is made to a segment of the reference sequence of the same length (excluding any loop required by the homology calculation).

In a related aspect, the disclosure provides a method of selecting a group of aptamers, comprising: (a) contacting a pool of aptamers to a population of microvesicles from a first sample; (b) enriching a subpool of aptamers that show affinity to the population of microvesicles from the first sample; (c) contacting the subpool to a second population of microvesicles from a second sample; and (d) depleting a second subpool of aptamers that show affinity to the second population of microvesicles from the second sample, thereby selecting the group of aptamers that have preferential affinity for the population of microvesicles from the first sample.

The first sample and/or second sample may comprise a biological fluid such as disclosed herein. For example, the biological fluid may include without limitation blood, a blood derivative, plasma, serum or urine. The first sample and/or second sample may also be derived from a cell culture.

The first sample may comprise a cancer sample and the second sample may comprise a control [2]sample, such as a non-cancer sample. The first sample and/or and the second sample may each comprise a pooled sample. For example, the first sample and/or second sample can comprise bodily fluid from 2, 3, 4, 5, 6, 7, 8, 9, 10, 11, 12, 13, 14, 15, 16, 17, 18, 19, 20, 25, 30, 40, 50, 60, 70, 80, 90, 100 or more than 100 individuals. In such cases, the members of a pool may be chosen to represent a desired phenotype. In a non-limiting example, the members of the first sample pool may be from patients with a cancer and the members of the second sample pool may be from non-cancer controls.

Steps (a)-(d) can be repeated a desired number of times in order to further enrich the pool in aptamers that have preferential affinity for the population of microvesicles from the first sample. For example, steps (a)-(d) can be repeated 1, 2, 3, 4, 5, 6, 7, 8, 9, 10, 11, 12, 13, 14, 15, 16, 17, 18, 19, 20 or more than 20 times. The output from step (d) can be used as the input to repeated step (a). The first sample and/or second sample may be replaced with a different sample before repeating steps (a)-(d). In a non-limiting example, members of a first sample pool may be from patients with a cancer and members of a second sample pool may be from non-cancer controls. During subsequent repetitions of steps (a)-(d), the first sample pool may comprise samples from different cancer patients than in the prior round/s. Similarly, the second sample pool may comprise samples from different controls than in the prior round/s.

The method may further comprise identifying the members of the selected group of aptamers, e.g., by DNA sequencing. The sequencing may be performed by Next Generation sequencing as desired.

The method may also comprise identifying the targets of the selected group of aptamers. Methods to identify aptamer targets are disclosed herein.

### *Aptamer Target Identification*

The methods and kits above can be used to identify binding agents that differentiate between two biomarker populations. The disclosure further provides methods of identifying the targets of the binding agents, as described in this section. For example, the methods may further comprise identifying a surface marker of a target microvesicle that is recognized by the binding agent.

The disclosure provides a method of identifying a target of a binding agent comprising: (a) contacting the binding agent with the target to bind the target with the binding agent, wherein the target comprises a surface antigen of a microvesicle; (b) disrupting the microvesicle under conditions which do not disrupt the binding of the target with the binding agent; (c) isolating the complex between the target and the binding agent; and (d) identifying the target bound by the binding agent. The binding agent can be a binding agent identified by the methods above, e.g., an aptamer, ligand, antibody, or other useful binding agent that can differentiate between two populations of biomarkers.

An illustrative schematic for carrying on the method is shown in **FIG. 9.** The figure shows a binding agent **902,** here an aptamer for purposes of illustration, tethered to a substrate **901.** The binding agent **902** can be covalently attached to substrate **901.** The binding agent **902** may also be non-covalently attached. For example, binding agent **902** can comprise a label which can be attracted to the substrate, such as a biotin group which can form a complex with an avidin/streptavidin molecule that is covalently attached to the substrate. This can allow a complex to be formed between the aptamer and the microvesicle while in solution, followed by capture of the aptamer using the biotin label. The binding agent **902** binds to a surface antigen **903** of microvesicle **904.** In the step signified by arrow (i), the microvesicle is disrupted while leaving the complex between the binding agent **902** and surface antigen **903** intact. Disrupted microvesicle **905** is removed, e.g., via washing or buffer exchange, in the step signified by arrow (ii). In the step signified by arrow (iii), the surface antigen **903** is released from the binding agent **902.** The surface antigen **903** can be analyzed to determine its identity using methods disclosed herein and/or known in the art. The target of the method can be any useful biological entity associated with a microvesicle. For example, the target may comprise a protein, nucleic acid, lipid or carbohydrate, or other biological entity disclosed herein or known in the art.

The target may be cross-linked to the binding agent prior disrupting the microvesicle. Without being bound by theory, this step may assist in maintaining the complex between the binding agent and the target while the vesicle is disrupted. Any useful method of crosslinking disclosed herein or known in the art can be used. The cross-linking may comprise photocrosslinking, an imidoester crosslinker, dimethyl suberimidate, an N-Hydroxysuccinimide-ester crosslinker, bissulfosuccinimidyl suberate (BS3), an aldehyde, acrolein, crotonaldehyde, formaldehyde, a carbodiimide crosslinker, N,N'-dicyclohexylcarbodiimide (DDC), N,N'-diisopropylcarbodiimide (DIC), 1-Ethyl-3-[3-dimethylaminopropyl]carbodiimide hydrochloride (EDC or EDAC), Succinimidyl-4-(N-maleimidomethyl)cyclohexane-1-carboxylate (SMCC), a Sulfosuccinimidyl-4-(N-maleimidomethyl)cyclohexane-1-carboxylate (Sulfo-SMCC), a Sulfo-N-hydroxysuccinimidyl-2-(6-[biotinamido]-2-(p-azido benzamido)-hexanoamido) ethyl-1,3'-dithioproprionate (Sulfo-SBED), 2-[N2-(4-Azido-2,3,5,6-tetrafluorobenzoyl)-N6-(6-biotin-amidocaproyl)-L-lysinyl]ethyl methanethiosulfonate (Mts-Atf-Biotin; available from Thermo Fisher Scientific Inc, Rockford IL.), 2-{N2-[N6-(4-Azido-2,3,5,6-tetrafluorobenzoyl-6-amino-caproyl)-N6-(6-biotinamidocaproyl)-L-lysinylamido]}ethyl methanethiosultonate (Mts-Atf-LC-Biotin; available from Thermo Fisher Scientific Inc), a photoreactive amino acid (e.g., L-Photo-Leucine and L-Photo-Methionine, see, e.g., Suchanek, M., et al. (2005). Photo-leucine and photo-methionine allow identification of protein-protein interactions. Nat. Methods 2:261-267), an N-Hydroxysuccinimide (NHS) crosslinker, an NHS-Azide reagent (e.g., NHS-Azide, NHS-PEG4-Azide, NHS-PEG12-Azide; each available from Thermo Fisher Scientific, Inc.), an NHS-Phosphine reagent (e.g., NHS-Phosphine, Sulfo-NHS-Phosphine; each available from Thermo Fisher Scientific, Inc.), or any combination or modification thereof.

A variety of methods can be used to disrupt the microvesicle. For example, the vesicle membrane can be disrupted using mechanical forces, chemical agents, or a combination thereof. Disrupting the microvesicle may comprise use of one or more of a detergent, a surfactant, a solvent, an enzyme, or any useful combination thereof. The enzyme may comprise one or more of lysozyme, lysostaphin, zymolase, cellulase, mutanolysin, a glycanase, a protease, and mannase. The detergent or surfactant may comprise one or more of a octylthioglucoside (OTG), octyl beta-glucoside (OG), a nonionic detergent, Triton X, Tween 20, a fatty alcohol, a cetyl alcohol, a stearyl alcohol, cetostearyl alcohol, an oleyl alcohol, a polyoxyethylene glycol alkyl ether (Brij), octaethylene glycol monododecyl ether, pentaethylene glycol monododecyl ether, a polyoxypropylene glycol alkyl ether, a glucoside alkyl ether, decyl glucoside, lauryl glucoside, octyl glucoside, a polyoxyethylene glycol octylphenol ethers, a polyoxyethylene glycol alkylphenol ether, nonoxynol-9, a glycerol alkyl ester, glyceryl laurate, a polyoxyethylene glycol sorbitan alkyl esters, polysorbate, a sorbitan alkyl ester, cocamide MEA, cocamide DEA, dodecyldimethylamine oxide, a block copolymers of polyethylene glycol and polypropylene glycol, poloxamers, polyethoxylated tallow amine (POEA), a zwitterionic detergent, 3-[(3-cholamidopropyl)dimethylammonio]-1-propanesulfonate (CHAPS), a linear alkylbenzene sulfonate (LAS), a alkyl phenol ethoxylate (APE), cocamidopropyl hydroxysultaine, a betaine, cocamidopropyl betaine, lecithin, an ionic detergent, sodium dodecyl sulfate (SDS), cetrimonium bromide (CTAB), cetyl trimethylammonium chloride (CTAC), octenidine dihydrochloride, cetylpyridinium chloride (CPC), benzalkonium chloride (BAC), benzethonium chloride (BZT), 5-Bromo-5-nitro-1,3-dioxane, dimethyldioctadecylammonium chloride, dioctadecyldimethylammonium bromide (DODAB), sodium deoxycholate, nonyl phenoxypolyethoxylethanol (Tergitol-type NP-40; NP-40), ammonium lauryl sulfate, sodium laureth sulfate (sodium lauryl ether sulfate (SLES)), sodium myreth sulfate, an alkyl carboxylate, sodium stearate, sodium lauroyl sarcosinate, a carboxylate-based fluorosurfactant, perfluorononanoate, perfluorooctanoate (PFOA or PFO), and a biosurfactant. Mechanical methods of disruption that can be used comprise without limitation mechanical shear, bead milling, homogenation, microfluidization, sonication, French Press, impingement, a colloid mill, decompression, osmotic shock, thermolysis, freeze-thaw, desiccation, or any combination thereof.

As shown in **FIG. 9,** the binding agent may be tethered to a substrate. The binding agent can be tethered before or after the complex between the binding agent and target is formed. The substrate can be any useful substrate such as disclosed herein or known in the art. The substrate may comprise a microsphere. In another embodiment, the substrate comprises a planar substrate. The binding agent can also be labeled. Isolating the complex between the target and the binding agent may comprise capturing the binding agent via the label. For example, the label can be a biotin label. In such cases, the binding agent can be attached to the substrate via a biotin-avidin binding event.

Methods of identifying the target after release from the binding agent will depend on the type of target of interest. For example, when the target comprises a protein, identifying the target may comprise use of mass spectrometry (MS), peptide mass fingerprinting (PMF; protein fingerprinting), sequencing, N-terminal amino acid analysis, C-terminal amino acid analysis, Edman degradation, chromatography, electrophoresis, two-dimensional gel electrophoresis (2D gel), antibody array, and immunoassay. Nucleic acids can be identified by sequencing.

One of skill will appreciate that the method can be used to identify any appropriate target, including those not associated with a vesicle. For example, with respect to the **FIG. 9,** all steps except for the step signified by arrow (i) (i.e., disrupting the microvesicle), could be performed for a circulating target such as a protein, nucleic acid, lipid, carbohydrate, or combination thereof.

***Sample Characterization***

The aptamers can be used to characterize a biological sample. For example, an aptamer can be used to bind a biomarker in the sample. The presence or level of the bound biomarker can indicate a characteristic of the example, such as a diagnosis, prognosis or theranosis of a disease or disorder associated with the sample.

In an aspect, the disclosure provides an aptamer comprising a nucleic acid sequence that is at least about 50, 55, 60, 65, 70, 75, 80, 85, 90, 95, 96, 97, 98, 99 or 100 percent homologous of any of: a) SEQ ID NOs. 8-21 or a variable sequence thereof as described in **Table 8;** b) SEQ ID NOs. 24-43 or a variable sequence thereof as described in **Table 11;** c) SEQ ID NOs. 44-10527; d) SEQ ID NOs. 10528-10557 or a variable sequence thereof as described in **Table 12;** or e) a functional fragment of any preceding sequence. In a related aspect, the disclosure provides a method of characterizing a disease or disorder, comprising: (a) contacting a biological test sample with one or more aptamer, e.g., any of those in this paragraph or modifications thereof; (b) detecting a presence or level of a complex between the one or more aptamer and the target bound by the one or more aptamer in the biological test sample formed in step (a); (c) contacting a biological control sample with the one or more aptamer; (d) detecting a presence or level of a complex between the one or more aptamer and the target bound by the one or more aptamer in the biological control sample formed in step (c); and (e) comparing the presence or level detected in steps (b) and (d), thereby characterizing the disease or disorder.

The biological test sample and biological control sample can each comprise a tissue sample, a cell culture, or a biological fluid. In some embodiments, the biological test sample and biological control sample comprise the same sample type, e.g., both are tissue samples or both are fluid samples. In other embodiments, different sample types may be used for the test and control samples. For example, the control sample may comprise an engineered or otherwise artificial sample.

The biological fluid may comprise a bodily fluid. The bodily fluid may include without limitation one or more of peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood. In some embodiments, the bodily fluid comprises blood, serum or plasma.

The biological fluid may comprise microvesicles. For example, the biological fluid can be a tissue, cell culture, or bodily fluid which comprises microvesicles released from cells in the sample. The microvesicles can be circulating microvesicles.

The one or more aptamer can bind a target biomarker, e.g., a biomarker useful in characterizing the sample. The biomarker may comprise a polypeptide or fragment thereof, or other useful biomarker described herein or known in the art (lipid, carbohydrate, complex, nucleic acid, etc). In embodiments, the polypeptide or fragment thereof is soluble or membrane bound. Membrane bound polypeptides may comprise a cellular surface antigen or a microvesicle surface antigen. The biomarker can be a biomarker selected from **Table 3** or **Table 4.**

The characterizing can comprises a diagnosis, prognosis or theranosis of the disease or disorder. Various diseases and disorders can be characterized using the methods of the invention, including without limitation a cancer, a premalignant condition, an inflammatory disease, an immune disease, an autoimmune disease or disorder, a cardiovascular disease or disorder, a neurological disease or disorder, an infectious disease, and/or pain. See, e.g., section herein "Phenotypes" for further details. In embodiments, the disease or disorder comprises a proliferative or neoplastic disease or disorder. For example, the disease or disorder can be a cancer. In some embodiments, the cancer comprises a breast cancer, ovarian cancer, prostate cancer, lung cancer, colorectal cancer, melanoma, or brain cancer.

**FIG. 16A** is a schematic **1600** showing an assay configuration that can be used to detect and/or quantify a target of interest using one or more aptamer. Capture aptamer **1602** is attached to substrate **1601.** The substrate can be a planar substrate, well, microbead, or other useful substrate as disclosed herein or known in the art. Target of interest **1603** is bound by capture aptamer **1602.** The target of interest can be any appropriate entity that can be detected when recognized by an aptamer or other binding agent. The target of interest may comprise a protein or polypeptide, a nucleic acid, including DNA, RNA, and various subspecies thereof, a lipid, a carbohydrate, a complex, e.g., a complex comprising protein, nucleic acids, lipids and/or carbohydrates. In some embodiments, the target of interest comprises a microvesicle. The target of interest can be a microvesicle surface antigen. The target of interest may be a biomarker, including a vesicle associated biomarker, in **Tables 3** or **4.** The microvesicle input can be isolated from a sample using various techniques as described herein, e.g., chromatography, filtration, centrifugation, flow cytometry, affinity capture (e.g., to a planar surface, column or bead), and/or using microfluidics. Detection aptamer **1604** is also bound to target of interest **1603.** Detection aptamer **1604** carries label **1605** which can be detected to identify target captured to substrate **1601** via capture aptamer **1602.** The label can be a fluorescent, radiolabel, enzyme, or other detectable label as disclosed herein. Either capture aptamer **1602** or detection aptamer **1604** can be substituted with another binding agent, e.g., an antibody. For example, the target may be captured with an antibody and detected with an aptamer, or vice versa. When the target of interest comprises a complex, the capture and detection agents (aptamer, antibody, etc) can recognize the same or different targets. For example, when the target is a microvesicle, the capture agent may recognize one microvesicle surface antigen while the detection agent recognizes another microvesicle surface antigen. Alternately, the capture and detection agents can recognize the same surface antigen.

The aptamers may be identified and/or used for various purposes in the form of DNA or RNA. Unless otherwise specified, one of skill in the art will appreciate that an aptamer may generally be synthesized in various forms of nucleic acid. The aptamers may also carry various chemical modifications.

An aptamer may be modified to comprise at least one chemical modification. The modification may include without limitation a chemical substitution at a sugar position; a chemical substitution at a phosphate position; and a chemical substitution at a base position of the nucleic acid.The modification may be selected from the group consisting of: biotinylation, incorporation of a fluorescent label, incorporation of a modified nucleotide, a 2'-modified pyrimidine, 3' capping, conjugation to an amine linker, conjugation to a high molecular weight, non-immunogenic compound, conjugation to a lipophilic compound, conjugation to a drug, conjugation to a cytotoxic moiety, and labeling with a radioisotope, or other modification as disclosed herein. The position of the modification can be varied as desired. For example, the biotinylation, fluorescent label, or cytotoxic moiety can be conjugated to the 5' end of the aptamer. The biotinylation, fluorescent label, or cytotoxic moiety can also be conjugated to the 3' end of the aptamer.

The cytotoxic moiety may be encapsulated in a nanoparticle. The nanoparticle can be selected from the group consisting of: liposomes, dendrimers, and comb polymers. The cytotoxic moiety may comprise a small molecule cytotoxic moiety. The small molecule cytotoxic moiety can include without limtation vinblastine hydrazide, calicheamicin, vinca alkaloid, a cryptophycin, a tubulysin, dolastatin-10, dolastatin-15, auristatin E, rhizoxin, epothilone B, epithilone D, taxoids, maytansinoids and any variants and derivatives thereof. The cytotoxic moiety may comprise a protein toxin. For example, the protein toxin can be selected from the group consisting of diphtheria toxin, ricin, abrin, gelonin, and Pseudomonas exotoxin A. Non-immunogenic, high molecular weight compounds for use may include polyalkylene glycols, e.g., polyethylene glycol. Appropriate radioisotopes include yttrium-90, indium-111, iodine-131, lutetium-177, copper-67, rhenium-186, rhenium-188, bismuth-212, bismuth-213, astatine-211, and actinium-225. The aptamer may be labeled with a gamma-emitting radioisotope.

An active agent may be conjugated to the aptamer. For example, the active agent may be a therapeutic agent or a diagnostic agent. The therapeutic agent may be selected from the group consisting of tyrosine kinase inhibitors, kinase inhibitors, biologically active agents, biological molecules, radionuclides, adriamycin, ansamycin antibiotics, asparaginase, bleomycin, busulphan, cisplatin, carboplatin, carmustine, capecotabine, chlorambucil, cytarabine, cyclophosphamide, camptothecin, dacarbazine, dactinomycin, daunorubicin, dexrazoxane, docetaxel, doxorubicin, etoposide, epothilones, floxuridine, fludarabine, fluorouracil, gemcitabine, hydroxyurea, idarubicin, ifosfamide, irinotecan, lomustine, mechlorethamine, mercaptopurine, melphalan, methotrexate, rapamycin (sirolimus), mitomycin, mitotane, mitoxantrone, nitrosurea, paclitaxel, pamidronate, pentostatin, plicamycin, procarbazine, rituximab, streptozocin, teniposide, thioguanine, thiotepa, taxanes, vinblastine, vincristine, vinorelbine, taxol, combretastatins, discodermolides, transplatinum, antivascular endothelial growth factor compounds ("anti-VEGFs"), anti-epidermal growth factor receptor compounds ("anti-EGFRs"), 5-fluorouracil and derivatives, radionuclides, polypeptide toxins, apoptosis inducers, therapy sensitizers, enzyme or active fragment thereof, and combinations thereof.

### Oligonucleotide Pools to Characterize a Sample

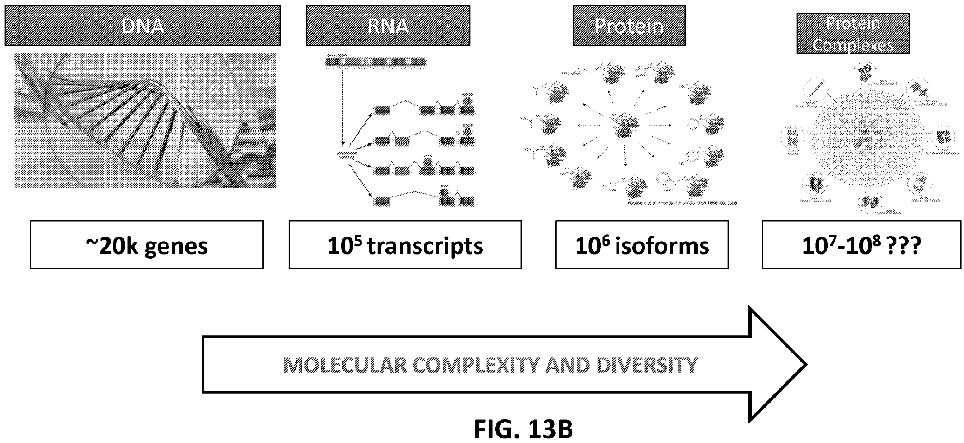

The complexity and heterogeneity present in biology challenges the understanding of biological systems and disease. Diversity exists at various levels, e.g., within and between cells, tissues, individuals and disease states. See, e.g., **FIG. 13A. FIG. 13B** overviews various biological entities that can be assessed to characterize such samples. As shown in the Figure, as one moves from assessing DNA, to RNA, to protein, and finally to protein complexes, the amount of diversity and complexity increases dramatically. The oligonucleotide probe library method can be used characterize complex biological sources, e.g., tissue samples, cells, circulating tumor cells, microvesicles, and complexes such as protein and proteolipid complexes.

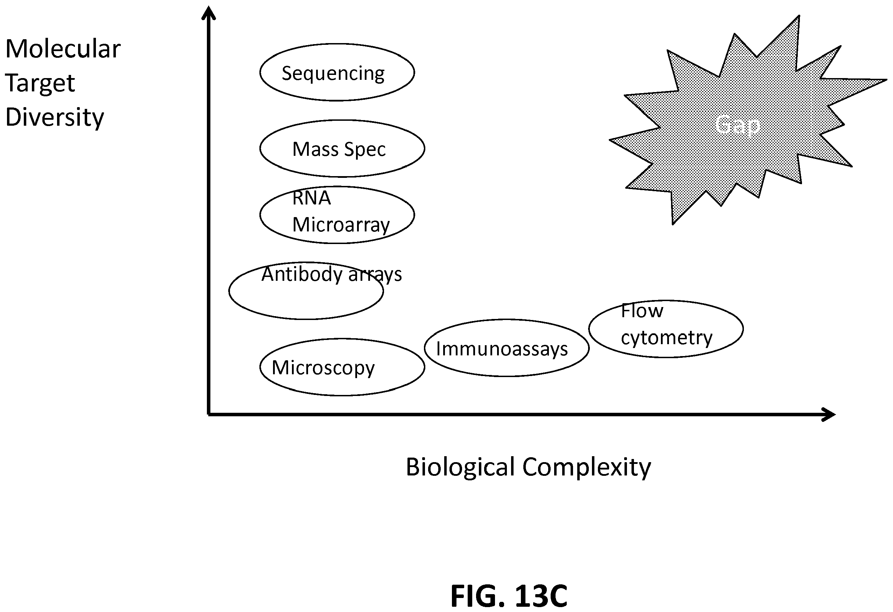

Current methods to characterize biological samples may not adequately address such complexity and diversity. As shown in **FIG. 13C,** such current methods often have a trade off between measuring diversity and complexity. As an example, consider high throughput sequencing technology. Next generation approaches may query many 1000s of molecular targets in a single assay. However, such approaches only probe individual DNA and/or RNA molecules, and thus miss out on the great diversity of proteins and biological complexes. On the other hand, flow cytometry can probe biological complexes, but are limited to a small number of pre-defined ligands. For example, a single assay can probe a handful of differentially labeled antibodies to pre-defined targets.

The oligonucleotide probe library can overcome the above challenges with current biological detection technologies. The size of the starting library can be adjusted to measure as many different entities as there are library members. In this Example, the initial untrained oligonucleotide library has the potential to measure $10^{12}$ or more biological features. A larger and/or different library can be constructed as desired. The technology is adapted to find differences between samples without assumptions about what "should be different." For example, the probe library may distinguish based on individual proteins, protein modifications, protein complexes, lipids, nucleic acids, different folds or conformations, or whatever is there that distinguishes a sample of interest. Thus, the method provides an unbiased approach to identify differences in biological samples that can be used to identify different populations of interest.

In the context of micro vesicles whose surface may comprise any number of biomarkers in various configurations and complexes, the use of the oligonucleotide library probe to assess a sample can be referred to as Topological Oligonucleotide Profiling: TOP™. Although as noted the terms aptamer and oligonucleotides are typically used interchangeably herein, some differences between "classic" individual aptamers and TOP probes are as follows. Individual pptamers may comprise individual oligonucleotides selected to bind to a known specific target in an antibody-like "key-in-lock" binding mode. They may be evaluated individually based on specificity and binding affinity to the intended target. However, TOP probes may comprise a library of oligonucleotides intended to produce multi-probe signatures. The TOP probes comprise numerous potential binding modalities (electrostatic, hydrophobic, Watson-Crick, multi-oligo complexes, etc.). The TOP probe signatures have the potential to identify heterogeneous patient subpopulations. For example, a single TOP probe library can be assembled to differentiate multiple disease states, as demonstrated herein. Unlike classic single aptamers, the binding targets may or may not be isolated or identified. It will be understood that screening methods that identify individual aptamers, e.g., SELEX, can also be used to enrich a naive library of oligonucleotides to identify a TOP probe library.

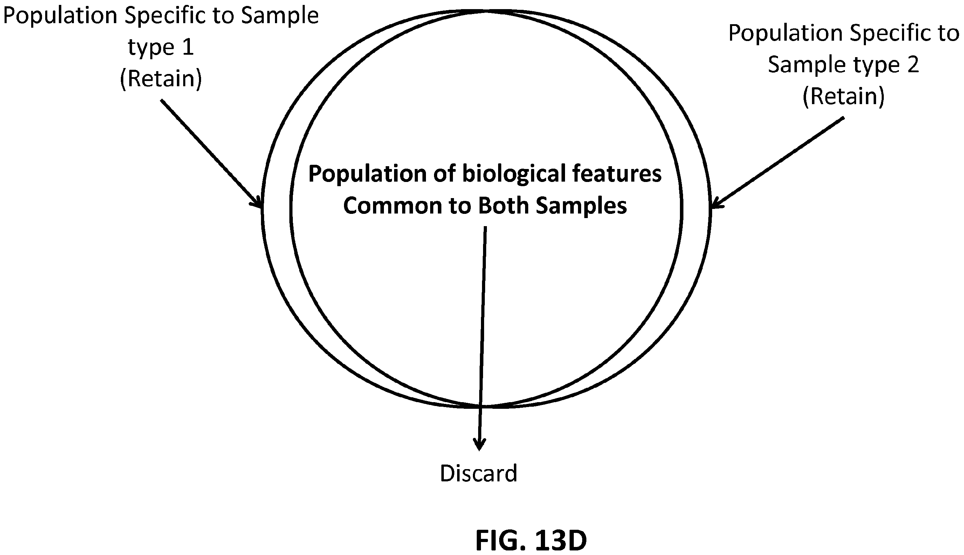

The general method is outlined in **FIG. 13D.** One input to the method comprises a randomized oligonucleotide library with the potential to measure $10^{12}$ or more biological features. As outlined in the figure, the method identifies a desired number (e.g., $\sim 10^5$-$10^6$) that are different between two input sample types. The randomized oligonucleotide library is contacted with a first and a second sample type, and oligonucleotides that bind to each sample are identified.

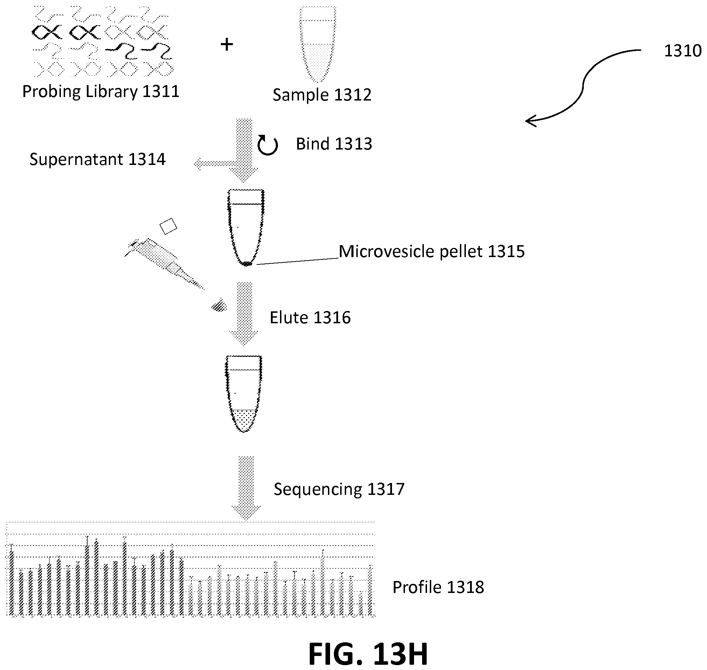

The bound oligonucleotide populations are compared and oligonucleotides that specifically bind to one or the other biological input sample are retained for the oligonucleotide probe library, whereas oligonucleotides that bind both biological input samples are discarded. This trained oligonucleotide probe library can then be contacted with a new test sample and the identities of oligonucleotides that bind the test sample are determined. The test sample is characterized based on the profile of oligonucleotides that bound. See, e.g., **FIG. 13H.**

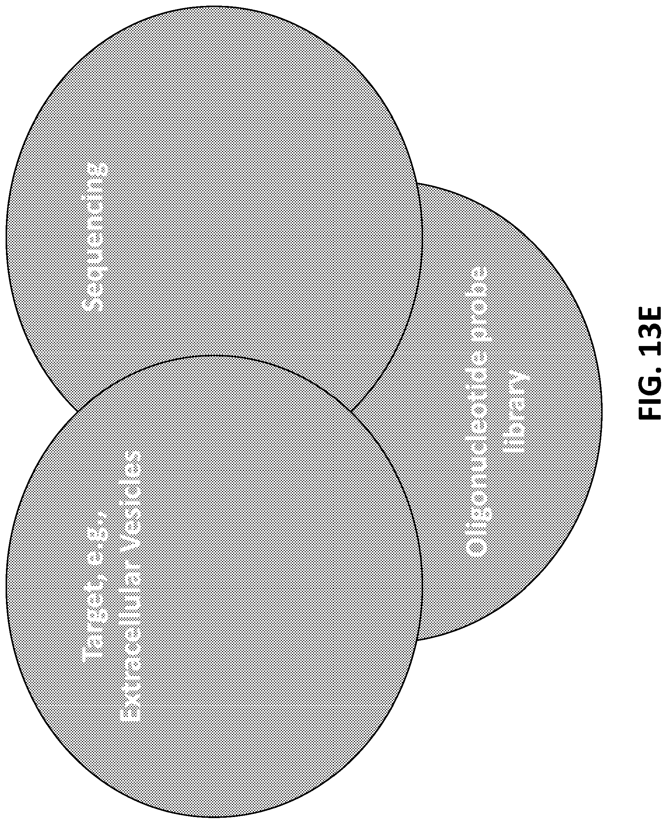

Extracellular vesicles provide an attractive vehicle to profile the biological complexity and diversity driven by many inter-related sources. There can be a great deal of heterogeneity between patient-to-patient microvesicle populations, or even in microvesicle populations from a single patient under different conditions (e.g., stress, diet, exercise, rest, disease, etc). Diversity of molecular phenotypes within microvesicle populations in various disease states, even after microvesicle isolation and sorting by vesicle biomarkers, can present challenges identifying surface binding ligands. This situation is further complicated by vesicle surface-membrane protein complexes. The oligonucleotide probe library can be used to address such challenges and allow for characterization of biological phenotypes. The approach combines the power of diverse oligonucleotide libraries and high throuput (next-generation) sequencing technologies to probe the complexity of extracellular microvesicles. See **FIG. 13E.**

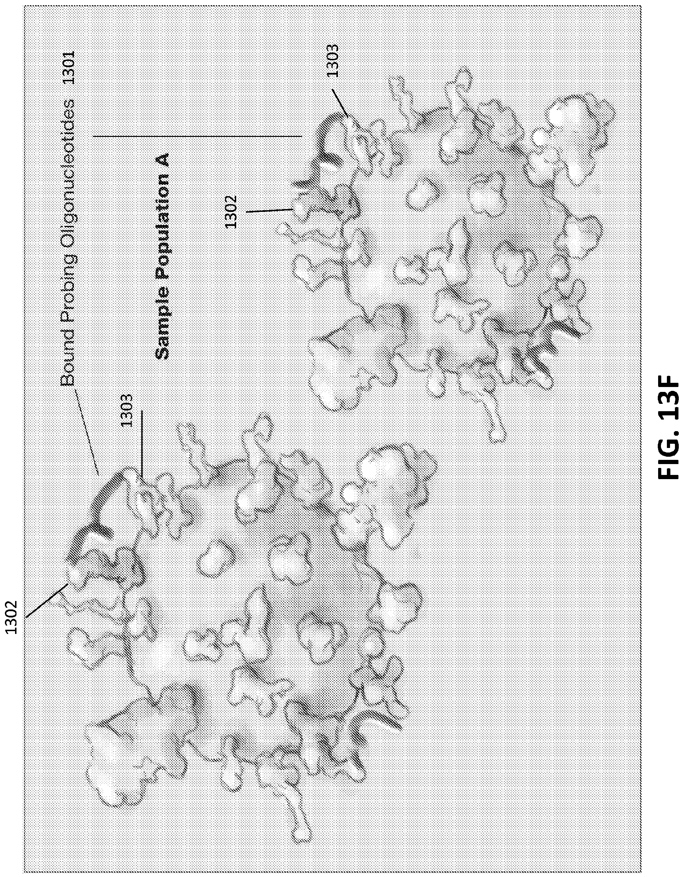

TOP™ profiling may provide quantitative measurements of dynamic events in addition to detection of presence/absence of various biomarkers in a sample. For example, the binding probes may detect protein complexes or other post-translation modifications, allowing for differentiation of samples with the same proteins but in different biological configurations. Such configurations are illustrated in **FIGs. 13F-G.** In **FIG. 13F,** microvesicles with various surface markers are shown from an example microvesicle sample population: Sample Population A. The indicated Bound Probing Oligonucleotides **1301** are contacted to two surface markers **1302** and **1303** in a given special relationship. Here, probes unique to these functional complexes and spatial relationships may be retained. In contrast, in microvesicle Sample Population B shown in **FIG. 13F,** the two surface markers **1302** and **1303** are found in disparate spacial relationship. Here, probes **1301** are not bound due to absence of the spatial relationship of the interacting components **1302** and **1303.**

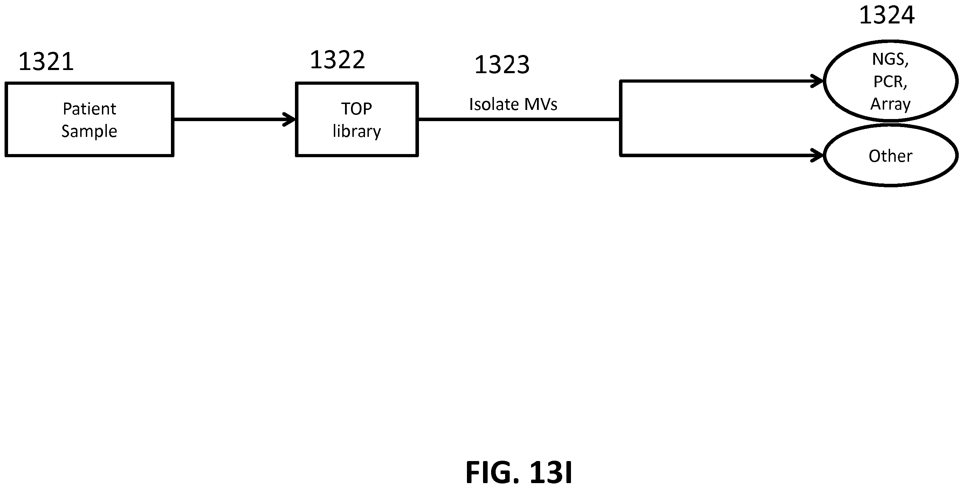

An illustrative approach **1310** for using TOP™ profiling to assess a sample is shown in **FIG. 13H.** The probing library **1311** is mixed with sample **1312.** The sample can be as described herein, e.g., a bodily fluid from a subject having or suspected of having a disease. The probes are allowed to bind the sample **1313** and the microvesicles are pelleted **1315.** The supernatant **1314** comprising unbound oligonucleotides is discarded. Oligonucleotide probes bound to the pellet **1315** are eluted **1316** and sequenced **1317.** The profile **1318** generated by the bound oligonucleotide probes as determined by the sequening **1317** is used to characterize the sample **1312.** For example, the profile **1318** can be compared to a reference, e.g., to determine if the profile is similar or different from a reference profile indicative of a disease or healthy state, or other phenotypic characterization of interest. The comparison may indicate the presence of a disease, provide a diagnosis, prognosis or theranosis, or otherwise characterize a phenotype associated with the sample **1312.** **FIG. 13I** illustrates another schematic for using TOP™ profiling to characterize a phenotype. A patient sample such as a bodily fluid disclosed herein is collected **1321.** The sample is contacted with the TOP™ library pool **1322.** Microvesicles (MVs) are isolated from the contacted sample **1323,** e.g., using ultracentrifugation, filtration, polymer precipitation or other appropriate technique or combination of techniques disclosed herein. Oligonucleotides that bound the isolated microvesicles are collected and identity is determined **1324.** The identity of the bound oligonucleotides can be determined by any useful technique such as sequencing, high throughput sequencing (e.g., NGS), amplification including without limitation qPCR, or hybridization such as to a planar or particle based array. The identity of the bound oligonucleotides is used to characterize the sample, e.g., as containing disease related microvesicles.

In an aspect, the disclosure provides a method of characterizing a sample by contacting the sample with a pool of different oligonucleotides (e.g., an aptamer pool), and determining the frequency at which various oligonucleotides in the pool bind the sample. For example, a pool of oligonucleotides is identified that preferentially bind to microvesicles from cancer patients as compared to non-cancer patients. A test sample, e.g., from a patient suspected of having the cancer, is collected and contacted with the pool of oligonucleotides. Oligonucleotides that bind the test sample are eluted from the test sample, collected and identified, and the composition of the bound oligonucleotides is compared to those known to bind cancer samples. Various sequencing, amplification and hybridization techinques can be used to identify the eluted oligonucleotides. For example, when a large pool of oligonucleotides is used, oligonucleotide identification can be performed by high throughput methods such as next generation sequencing or via hybridization. If the test sample is bound by the oligonucleotide pool in a similar manner (e.g., as determined by bioinformatics classification methods) to the microvesicles from cancer patients, then the test sample is indicative of cancer as well. Using this method, a pool of oligonucleotides that bind one or more microvesicle antigen can be used to characterize the sample without necessarily knowing the precise target of each member of the pool of oligonucleotides.

In an aspect, the disclosure provides a method for characterizing a condition for a test sample comprising: contacting a microvesicle sample with a plurality of oligonucleotide capable of binding one or more target(s) present in said microvesicle sample, identifying a set of oligonucleotides that form a complex with the sample wherein the set is predetermined to characterize a condition for the sample, thereby characterizing a condition for a sample.

In an related aspect, the disclosure provides a method for identifying a set of oligonucleotides associated with a test sample, comprising: (a) contacting a microvesicle sample with a plurality of oligonucleotides, isolating a set of oligonucleotides that form a complex with the microvesicle sample, (b) determining sequence and/or copy number for each of the oligonucleotides, thereby identifying a set of oligonucleotides associated with the test sample.

In still another related aspect, the disclosure provides a method of diagnosing a sample as cancerous or predisposed to be cancerous, comprising contacting a microvesicle sample with a plurality of oligonucleotides that are predetermined to preferentially form a complex with microvesicles from a cancer sample as compared to microvesicles from a non-cancer sample.

The oligonucleotides can be identified by sequencing, e.g., by dye termination (Sanger) sequencing or high throughput methods. High throughput methods can comprise techiques to rapidly sequence a large number of nucleic acids, including next generation techniques such as Massively parallel signature sequencing (MPSS; Polony sequencing; 454 pyrosequencing; Illumina (Solexa) sequencing; SOLiD sequencing; Ion Torrent semiconductor sequencing; DNA nanoball sequencing; Heliscope single molecule sequencing; Single molecule real time (SMRT) sequencing, or other methods such as Nanopore DNA sequencing; Tunnelling currents DNA sequencing; Sequencing by hybridization; Sequencing with mass spectrometry; Microfluidic Sanger sequencing; Microscopy-based techniques; RNAP sequencing; In vitro virus high-throughput sequencing. The oligonucleotides may also be identified by hybridization techniques. For example, a microarray having addressable locals to hybridize and thereby detect the various members of the pool can be used.

The plurality or pool of oligonucleotides can comprise any desired number of oligonucleotides to allow characterization of the sample. The pool may comprise at least 2, 3, 4, 5, 6, 7, 8, 9, 10, 11, 12, 13, 14, 15, 16, 17, 18, 19, 20, 25, 30, 40, 50, 60, 70, 80, 90, 100, 150, 200, 250, 300, 350, 400, 450, 500, 600, 700, 800, 900, 1000, 2000, 3000, 4000, 5000, 6000, 7000, 8000, 9000, or at least 10000 different oligonucleotide members.

The plurality of oligonucleotides can be pre-selected through one or more steps of positive or negative selection, wherein positive selection comprises selection of oligonucleotides against a sample having substantially similar characteristics compared to the test sample, and wherein negative selection comprises selection of oligonucleotides against a sample having substantially different characteristics compared to the test sample. Substantially similar characteristics mean that the samples used for positive selection are representative of the test sample in one or more characteristic of interest. For example, the samples used for positive selection can be from cancer patients or cell lines and the test sample can be a sample from a patient having or suspected to have a cancer. Substantially different characteristics mean that the samples used for negative selection differ from the test sample in one or more characteristic of interest. For example, the samples used for negative selection can be from individuals or cell lines that do not have cancer (e.g., "normal" or otherwise "control" samples) and the test sample can be a sample from a patient having or suspected to have a cancer. The cancer can be a breast cancer, ovarian cancer, prostate cancer, lung cancer, colorectal cancer, melanoma, brain cancer, or other cancer.

By selecting samples representative of the desired phenotypes to detect and/or distinguish, the characterizing can comprise a diagnosis, prognosis or theranosis for any number of diseases or disorders. Various diseases and disorders can be characterized using the methods of the invention, including without limitation a cancer, a premalignant condition, an inflammatory disease, an immune disease, an autoimmune disease or disorder, a cardiovascular disease or disorder, a neurological disease or disorder, an infectious disease, and/or pain. See, e.g., section herein "Phenotypes" for further details. In embodiments, the disease or disorder comprises a proliferative or neoplastic disease or disorder. For example, the disease or disorder can be a cancer. In some embodiments, the cancer comprises a breast cancer, ovarian cancer, prostate cancer, lung cancer, colorectal cancer, melanoma, or brain cancer.

FIG. 16B is a schematic 1610 showing use of an oligonucleotide pool to characterize a phenotype of a sample, such as those listed above. A pool of oligonucleotides to a target of interest is provided 1611. For example, the pool of oligonucleotides can be enriched to target one or more microvesicle. The members of the pool may bind different targets (e.g., a microvesicle surface antigen) or different epitopes of the same target present on the one or more microvesicle. The pool is contacted with a test sample to be characterized 1612. For example, the test sample may be a biological sample from an individual having or suspected of having a given disease or disorder. The mixture is washed to remove unbound oligonucleotides. The remaining oligonucleotides are eluted or otherwise disassociated from the sample and collected 1613. The collected oligonucleotides are identified, e.g., by sequencing or hybridization 1614. The presence and/or copy number of the identified is used to characterize the phenotype 1615. For example, the pool of oligonucleotides may be chosen as oligonucleotides that preferentially recognize microvesicles shed from cancer cells. The method can be employed to detect whether the sample retains oligonucleotides that bind the cancer-related microvesicles, thereby allowing the sample to be characterized as cancerous or not.

FIG. 16C is a schematic 1620 showing an implementation of the method in FIG. 16B. A pool of oligonucleotides identified as binding a microvesicle population is provided 1621. The input sample comprises a test sample comprising microvesicles 1622. For example, the test sample may be a biological sample from an individual having or suspected of having a given disease or disorder. The pool is contacted with the isolated microvesicles to be characterized 1623. The microvesicle population can be isolated before or after the contacting 1623 from the sample using various techniques as described herein, e.g., chromatography, filtration, ultrafiltration, centrifugation, ultracentrifugation, flow cytometry, affinity capture (e.g., to a planar surface, column or bead), polymer precipitation, and/or using microfluidics. The mixture is washed to remove unbound oligonucleotides and the remaining oligonucleotides are eluted or otherwise disassociated from the sample and collected **1624**. The collected oligonucleotides are identified **1625** and the presence and/or copy number of the retained oligonucleotides is used to characterize the phenotype **1626** as above.

As noted, in **FIG. 16C,** the pool of oligonucleotides **1620** may be directly contacted with a biological sample that comprises or is expected to comprise microvesicles. Microvesicles are thereafter isolated from the sample and the mixture is washed to remove unbound oligonucleotides and the remaining oligonucleotides are disassociated and collected **1624.** The following steps are performed as above. As an example of this alternate configuration, a biological sample, e.g., a blood, serum or plasma sample, is directly contacted with the pool of oligonucleotides. Microvesicles are then isolated by various techniques disclosed herein, including without limitation ultracentrifugation, ultrafiltration, flow cytometry, affinity isolation, polymer precipitation, chromatography, various combinations thereof, or the like. Remaining oligonucleotides are then identified, e.g., by sequencing, hybridization or amplification.

In a related aspect, the disclosure provides a composition of matter comprising a plurality of oligonucleotides that can be used to carry out the methods comprising use of an oligonucleotide pool to characterize a phenotype. The plurality of oligonucleotides can comprise any of those described herein.

In a related aspect, the disclosure provides a method of performing high-throughput sequencing comprising: performing at least one (i) negative selection or (ii) one positive selection of a plurality of oligonucleotides with a microvesicle sample; obtaining a set of oliognucleotides to provide a negative binder subset or positive binder subset of the plurality of oligonucleotides, wherein the negative binder subset of the plurality of oligonucleotides does not bind the microvesicle sample and wherein the positive binder subset of the plurality of oligonucleotides does bind the microvesicle sample; contacting the negative binder subset or positive binder subset with a test sample; eluting oligonucleotides that bound to the test sample to provide a plurality of eluate oligonucleotides; and performing high-throughput sequencing of the plurality of eluate oligonucleotides to identify sequence and/or copy number of the members of the plurality of eluate oligonucleotides. Negative and positive selection of the plurality of oligonucleotides using microvesicle sample can be performed as disclosed herein. The oligonucleotide profile revealed by the sequence and/or copy number of the members of the plurality of eluate oligonucleotides can be used to characterize a phenotype of the test sample as described herein.

In a similar aspect, the disclosure provides a method for identifying oligonucleotides specific for a test sample. The method comprises: (a) enriching a plurality of oligonucleotides for a sample to provide a set of oligonucleotides predetermined to form a complex with a target sample; (b) contacting the plurality in (a) with a test sample to allow formation of complexes of oligonucleotides with test sample; (c) recovering oligonucleotides that formed complexes in (b) to provide a recovered subset of oligonucleotides; and (d) profiling the recovered subset of oligonucleotides by high-throughput sequencing or hybridization, thereby identifying oligonucleotides specific for a test sample. The test sample may comprise a plurality of microvesicles. The oligonucleotides may comprise RNA, DNA or both. The method may further comprise performing informatics analysis to identify a subset of oligonucleotides comprising sequence identity of at least 50%, 55%, 60%, 65%, 70%, 75%, 80%, 85%, 90%, 91%, 92%, 93%, 94%, 95%, 96%, 97%, 98%, or at least 99% to the oligonucleotides predetermined to form a complex with the target sample.

In an aspect, the disclosure provides an oligonucleotide at least 50, 55, 60, 65, 70, 75, 80, 85, 90, 95, 96, 97, 98, 99 or 100 percent homologous to SEQ ID NO. 10558. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NO. 10558.

In another aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, or 500000 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to a sequence selected from SEQ ID NOs. 10559-510558. In a related aspect, the disclosure provides an oligonucleotide probe library comprising at least 50%, 60%, 70%, 80%, 90%, 95%, 96%, 97%, 98% or at least 99% of the oligonucleotides listed in SEQ ID NOs. 10559-510558.

In still another aspect, the disclosure provides an oligonucleotide comprising a nucleic acid sequence or a portion thereof that is at least 50, 55, 60, 65, 70, 75, 80, 85, 90, 95, 96, 97, 98, 99 or 100 percent homologous to a sequence selected from SEQ ID NOs. 510559-510578. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NOs. 510559-510578.

In yet another aspect, the disclosure provides an oligonucleotide comprising a nucleic acid sequence or a portion thereof that is at least 50, 55, 60, 65, 70, 75, 80, 85, 90, 95, 96, 97, 98, 99 or 100 percent homologous to a sequence selected from SEQ ID NOs. 510579-510598. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NOs. 510579-510598.

In an aspect, the disclosure provides an oligonucleotide comprising a nucleic acid sequence or a portion thereof that is at least 50, 55, 60, 65, 70, 75, 80, 85, 90, 95, 96, 97, 98, 99 or 100 percent homologous to a sequence selected from SEQ ID NOs. 510599-510763. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 110, 120, 125, 130, 140, 150, 160 or 165 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to SEQ ID NOs. 510599-510763. In still another related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 110, 120, 125, 130, 140, 150, 160 or 165 different oligonucleotide sequences, wherein each of the oligonucleotide sequences or a portion thereof is at least 50, 55, 60, 65, 70, 75, 80, 85, 90, 95, 96, 97, 98, 99 or 100 percent homologous to the SEQ ID NOs. in a row in **Table 16.**

In an aspect, the disclosure also provides a method comprising contacting an oligonucleotide or plurality of oligonucleotides with a sample and detecting the presence or level of binding of the oligonucleotide or plurality of oligonucleotides to a target in the sample, wherein the oligonucleotide or plurality of oligonucleotides can be those provided by the disclosure above. The sample may comprise a biological sample, an organic sample, an inorganic sample, a tissue, a cell culture, a bodily fluid, blood, serum, a cell, a microvesicle, a protein complex, a lipid complex, a carbohydrate, or any combination, fraction or variation thereof. The target may comprise a cell, an organelle, a protein complex, a lipoprotein, a carbohydrate, a microvesicle, a membrane fragment, a small molecule, a heavy metal, a toxin, or a drug.

In a related aspect, the disclosure provides a method comprising: a) contacting a biological sample comprising microvesicles with an oligonucleotide probe library, wherein optionally the oligonucleotide probe library comprises an oligonucleotide or plurality of oligonucleotides those disclosed above; b) identifying oligonucleotides bound to at least a portion of the microvesicles; and c) characterizing the sample based on a profile of the identified oligonucleotides.

In another aspect, the disclosure provides a method comprising: a) contacting a sample with an oligonucleotide probe library comprising at least $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences oligonucleotides to form a mixture in solution, wherein the oligonucleotides are capable of binding a plurality of entities in the sample to form complexes, wherein optionally the oligonucleotide probe library comprises an oligonucleotide or plurality of oligonucleotides as disclosed above; b) partitioning the complexes formed in step (a) from the mixture; and c) detecting oligonucleotides present in the complexes partitioned in step (b) to identify an oligonucleotide profile for the sample. The detecting step may comprise performing sequencing of all or some of the oligonucleotides in the complexes, amplification of all or some of the oligonucleotides in the complexes, and/or hybridization of all or some of the oligonucleotides in the complexes to an array. The array can be any useful array, such as a planar or particle-based array.

In still another aspect, the disclosure provides a method for generating an enriched oligonucleotide probe library comprising: a) contacting a first oligonucleotide library with a biological test sample and a biological control sample, wherein complexes are formed between biological entities present in the biological samples and a plurality of oligonucleotides present in the first oligonucleotide library; b) partitioning the complexes formed in step (a) and isolating the oligonucleotides in the complexes to produce a subset of oligonucleotides for each of the biological test sample and biological control sample; c) contacting the subsets of oligonucleotides in (b) with the biological test sample and biological control sample wherein complexes are formed between biological entities present in the biological samples and a second plurality of oligonucleotides present in the subsets of oligonucleotides to generate a second subset group of oligonucleotides; and d) optionally repeating steps b)-c), one, two, three or more times to produce a respective third, fourth, fifth or more subset group of oligonucleotides, thereby producing the enriched oligonucleotide probe library. In a related aspect, the disclosure provides a plurality of oligonucleotides comprising at least 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, or 500000 different oligonucleotide sequences, wherein the plurality results from the method in this paragraph, wherein the library is capable of distinguishing a first phenotype from a second phenotype. The first phenotype may comprise a disease or disorder and the second phenotype may comprise a healthy state; or the first phenotype may comprise a disease or disorder and the second phenotype may comprise a different disease or disorder; or the first phenotype may comprise a stage or progression of a disease or disorder and the second phenotype may comprise a different stage or progression of the same disease or disorder; or the first phenotype may comprise a positive response to a therapy and the second phenotype may comprise a negative response to the same therapy.

In yet another aspect, the disclosure provides a method of characterizing a disease or disorder, comprising: a) contacting a biological test sample with the oligonucleotide or plurality of oligonucleotides provided; b) detecting a presence or level of complexes formed in step (a) between the oligonucleotide or plurality of oligonucleotides provided by the disclosure and a target in the biological test sample; and c) comparing the presence or level detected in step (b) to a reference level from a biological control sample, thereby characterizing the disease or disorder. The step of detecting may comprise performing sequencing of all or some of the oligonucleotides in the complexes, amplification of all or some of the oligonucleotides in the complexes, and/or hybridization of all or some of the oligonucleotides in the complexes to an array. The sequencing may be high-throughput or next generation sequencing.

In the methods of the invention, the biological test sample and biological control sample may each comprise a tissue sample, a cell culture, or a biological fluid. In some embodiments, the biological fluid comprises a bodily fluid. Useful bodily fluids within the method of the invention comprise peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood. In some preferred embodiments, the bodily fluid comprises blood, serum or plasma. The biological fluid may comprise microvesicles. In such case, the complexes may be formed between the oligonucleotide or plurality of oligonucleotides and at least one of the microvesicles.

The biological test sample and biological control sample may further comprise isolated microvesicles, wherein optionally the microvesicles are isolated using at least one of chromatography, filtration, ultrafiltration, centrifugation, ultracentrifugation, flow cytometry, affinity capture (e.g., to a planar surface, column or bead), polymer precipitation, and using microfluidics. The vesicles can also be isolated after contact with the oligonucleotide or plurality of oligonucleotides.

In various embodiments of the methods of the invention, the oligonucleotide or plurality of oligonucleotides binds a polypeptide or fragment thereof. The polypeptide or fragment thereof can be soluble or membrane bound, wherein optionally the membrane comprises a microvesicle membrane. The membrane could also be from a cell or a fragment of a cell of vesicle. In some embodiments, the polypeptide or fragment thereof comprises a biomarker in **Table 3** or **Table 4.** For example, the polypeptide or fragment thereof could be a general vesicle marker such as in **Table 3** or a tissue-related or disease-related marker such as in **Table 4.** The oligonucleotide or plurality of oligonucleotides may bind a microvesicle surface antigen in the biological sample. For example, the oligonucleotide or plurality of oligonucleotides can be enriched from a naive library against microvesicles.

The disease or disorder detected by the oligonucleotide, plurality of oligonucleotides, or methods provided here may comprise any appropriate disease or disorder of interest, including without limitation Breast Cancer, Alzheimer's disease, bronchial asthma, Transitional cell carcinoma of the bladder, Giant cellular osteoblastoclastoma, Brain Tumor, Colorectal adenocarcinoma, Chronic obstructive pulmonary disease (COPD), Squamous cell carcinoma of the cervix, acute myocardial infarction (AMI) / acute heart failure, Chron's Disease, diabetes mellitus type II, Esophageal carcinoma, Squamous cell carcinoma of the larynx, Acute and chronic leukemia of the bone marrow, Lung carcinoma, Malignant lymphoma, Multiple Sclerosis, Ovarian carcinoma, Parkinson disease, Prostate adenocarcinoma, psoriasis, Rheumatoid Arthritis, Renal cell carcinoma, Squamous cell carcinoma of skin, Adenocarcinoma of the stomach, carcinoma of the thyroid gland, Testicular cancer, ulcerative colitis, or Uterine adenocarcinoma.

In some embodiments, the disease or disorder comprises a cancer, a premalignant condition, an inflammatory disease, an immune disease, an autoimmune disease or disorder, a cardiovascular disease or disorder, neurological disease or disorder, infectious disease or pain. The cancer can include without limitation one of acute lymphoblastic leukemia; acute myeloid leukemia; adrenocortical carcinoma; AIDS-related cancers; AIDS-related lymphoma; anal cancer; appendix cancer; astrocytomas; atypical teratoid/rhabdoid tumor; basal cell carcinoma; bladder cancer; brain stem glioma; brain tumor (including brain stem glioma, central nervous system atypical teratoid/rhabdoid tumor, central nervous system embryonal tumors, astrocytomas, craniopharyngioma, ependymoblastoma, ependymoma, medulloblastoma, medulloepithelioma, pineal parenchymal tumors of intermediate differentiation, supratentorial primitive neuroectodermal tumors and pineoblastoma); breast cancer; bronchial tumors; Burkitt lymphoma; cancer of unknown primary site; carcinoid tumor; carcinoma of unknown primary site; central nervous system atypical teratoid/rhabdoid tumor; central nervous system embryonal tumors; cervical cancer; childhood cancers; chordoma; chronic lymphocytic leukemia; chronic myelogenous leukemia; chronic myeloproliferative disorders; colon cancer; colorectal cancer; craniopharyngioma; cutaneous T-cell lymphoma; endocrine pancreas islet cell tumors; endometrial cancer; ependymoblastoma; ependymoma; esophageal cancer; esthesioneuroblastoma; Ewing sarcoma; extracranial germ cell tumor; extragonadal germ cell tumor; extrahepatic bile duct cancer; gallbladder cancer; gastric (stomach) cancer; gastrointestinal carcinoid tumor; gastrointestinal stromal cell tumor; gastrointestinal stromal tumor (GIST); gestational trophoblastic tumor; glioma; hairy cell leukemia; head and neck cancer; heart cancer; Hodgkin lymphoma; hypopharyngeal cancer; intraocular melanoma; islet cell tumors; Kaposi sarcoma; kidney cancer; Langerhans cell histiocytosis; laryngeal cancer; lip cancer; liver cancer; lung cancer; malignant fibrous histiocytoma bone cancer; medulloblastoma; medulloepithelioma; melanoma; Merkel cell carcinoma; Merkel cell skin carcinoma; mesothelioma; metastatic squamous neck cancer with occult primary; mouth cancer; multiple endocrine neoplasia syndromes; multiple myeloma; multiple myeloma/plasma cell neoplasm; mycosis fungoides; myelodysplastic syndromes; myeloproliferative neoplasms; nasal cavity cancer; nasopharyngeal cancer; neuroblastoma; Non-Hodgkin lymphoma; nonmelanoma skin cancer; non-small cell lung cancer; oral cancer; oral cavity cancer; oropharyngeal cancer; osteosarcoma; other brain and spinal cord tumors; ovarian cancer; ovarian epithelial cancer; ovarian germ cell tumor; ovarian low malignant potential tumor; pancreatic cancer; papillomatosis; paranasal sinus cancer; parathyroid cancer; pelvic cancer; penile cancer; pharyngeal cancer; pineal parenchymal tumors of intermediate differentiation; pineoblastoma; pituitary tumor; plasma cell neoplasm/multiple myeloma; pleuropulmonary blastoma; primary central nervous system (CNS) lymphoma; primary hepatocellular liver cancer; prostate cancer; rectal cancer; renal cancer; renal cell (kidney) cancer; renal cell cancer; respiratory tract cancer; retinoblastoma; rhabdomyosarcoma; salivary gland cancer; Sezary syndrome; small cell lung cancer; small intestine cancer; soft tissue sarcoma; squamous cell carcinoma; squamous neck cancer; stomach (gastric) cancer; supratentorial primitive neuroectodermal tumors; T-cell lymphoma; testicular cancer; throat cancer; thymic carcinoma; thymoma; thyroid cancer; transitional cell cancer; transitional cell cancer of the renal pelvis and ureter; trophoblastic tumor; ureter cancer; urethral cancer; uterine cancer; uterine sarcoma; vaginal cancer; vulvar cancer; Waldenström macroglobulinemia; or Wilm's tumor. The premalignant condition can include without limitation Barrett's Esophagus. The autoimmune disease can include without limitation one of inflammatory bowel disease (IBD), Crohn's disease (CD), ulcerative colitis (UC), pelvic inflammation, vasculitis, psoriasis, diabetes, autoimmune hepatitis, multiple sclerosis, myasthenia gravis, Type I diabetes, rheumatoid arthritis, psoriasis, systemic lupus erythematosis (SLE), Hashimoto's Thyroiditis, Grave's disease, Ankylosing Spondylitis Sjogrens Disease, CREST syndrome, Scleroderma, Rheumatic Disease, organ rejection, Primary Sclerosing Cholangitis, or sepsis. The cardiovascular disease can include without limitation one of atherosclerosis, congestive heart failure, vulnerable plaque, stroke, ischemia, high blood pressure, stenosis, vessel occlusion or a thrombotic event. The neurological disease can include without limitation one of Multiple Sclerosis (MS), Parkinson's Disease (PD), Alzheimer's Disease (AD), schizophrenia, bipolar disorder, depression, autism, Prion Disease, Pick's disease, dementia, Huntington disease (HD), Down's syndrome, cerebrovascular disease, Rasmussen's encephalitis, viral meningitis, neurospsychiatric systemic lupus erythematosus (NPSLE), amyotrophic lateral sclerosis, Creutzfeldt-Jacob disease, Gerstmann-Straussler-Scheinker disease, transmissible spongiform encephalopathy, ischemic reperfusion damage (e.g. stroke), brain trauma, microbial infection, or chronic fatigue syndrome. The pain can include without limitation one of fibromyalgia, chronic neuropathic pain, or peripheral neuropathic pain. The infectious disease can include without limitation one of a bacterial infection, viral infection, yeast infection, Whipple's Disease, Prion Disease, cirrhosis, methicillin-resistant staphylococcus aureus, HIV, HCV, hepatitis, syphilis, meningitis, malaria, tuberculosis, or influenza. One of skill will appreciate that the methods of the invention can be used to assess any number of these or other related diseases and disorders.

In some embodiments of the invention, the methods of use thereof are useful for characterizing certain diseases or disease states. As desired, a pool of oligonucleotides useful for characterizing various diseases is assembled to create a master pool that can be used to probe useful for characterizing the various diseases. For example, the combination of SEQ ID NOs. 510599-510763 provided herein comprise such a pool. One of skill will also appreciate that pools of oligonucleotides useful for characterizing specific diseases or disorders can be created as well. The sequences provided herein can also be modified as desired so long as the functional aspects are still maintained (e.g., binding to various targets or ability to characterize a phenotype). For example, the oligonucleotides may comprise DNA or RNA, incorporate various non-natural nucleotides, incorporate other chemical modifications, or comprise various deletions or insertions. Such modifications may facilitate synthesis, stability, delivery, labeling, etc, or may have little to no effect in practice. In some cases, some nucleotides in an oligonucleotide may be substituted while maintaining functional aspects of the oligonucleotide. Similarly, 5' and 3' flanking regions may be substituted. In still other cases, only a portion of an oligonucleotide may be determined to direct its functionality such that other portions can be deleted or substituted. Numerous techniques to synthesize and modify nucleotides and polynucleotides are disclosed herein or are known in the art.

In an embodiment, the disease or disorder comprises a breast cancer and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30 or at least 40 of SEQ ID NOs. 510559-510598. The oligonucleotides may incorporate various chemical modifications, additions, deletions, insertions, substitutions or other modifications so long as functional aspects of the oligonucleotides are enhanced or maintained in whole or in part.

In another embodiment, and the disease or disorder comprises Alzheimer's disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510604, 510605, 510608, 510609, 510612, 510614, 510629, 510632, 510633, 510634, 510641, 510642, 510643, 510646, 510648, 510649, 510651, 510652, 510653, 510655, 510661, 510667, 510673, 510675, 510676, 510677, 510678, 510679, 510681, 510683, 510685, 510687, 510688, 510690, 510694, 510696, 510702, 510707, 510709, 510726, 510727, 510728, 510729, 510730, 510731, 510732, 510737, 510740, 510748, 510749, 510751, 510752, 510754, 510756, 510757, 510758, 510761, and 510762. In a related embodiment, the disease or disorder comprises Alzheimer's disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510601, 510603, 510606, 510608, 510609, 510611, 510613, 510614, 510615, 510619, 510621, 510625, 510628, 510629, 510630, 510632, 510634, 510635, 510636, 510637, 510644, 510647, 510648, 510651, 510652, 510654, 510657, 510665, 510666, 510667, 510668, 510677, 510678, 510679, 510687, 510692, 510693, 510696, 510698, 510699, 510701, 510702, 510707, 510708, 510710, 510713, 510716, 510725, 510726, 510728, 510731, 510732, 510733, 510734, 510736, 510741, 510748, 510749, 510755, 510757, 510758, 510761, and 510762. The oligonucleotides may incorporate various chemical modifications, additions, deletions, insertions, substitutions or other modifications so long as functional aspects of the oligonucleotides are enhanced or maintained in whole or in part.

The disease or disorder may comprise bronchial asthma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510614, 510619, 510623, 510627, 510631, 510633, 510635, 510647, 510655, 510656, 510660, 510672, 510689, 510690, 510693, 510698, 510701, 510702, 510707, 510709, 510710, 510713, 510720, 510723, 510724, 510726, 510728, 510729, 510730, 510731, 510734, 510735, 510738, 510743, 510744, 510745, 510746, 510748, 510749, 510750, 510751, 510752, 510754, 510755, 510757, 510758, 510759, 510760, 510761, and 510762. The disease or disorder may also comprise bronchial asthma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510608, 510609, 510610, 510611, 510617, 510619, 510622, 510631, 510632, 510634, 510635, 510637, 510642, 510643, 510644, 510652, 510655, 510657, 510658, 510665, 510668, 510673, 510675, 510676, 510677, 510678, 510679, 510683, 510691, 510701, 510703, 510704, 510706, 510708, 510709, 510714, 510725, 510736, 510737, 510740, 510741, 510742, and 510756. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises a transitional cell carcinoma of the bladder and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510619, 510623, 510631, 510632, 510635, 510641, 510642, 510647, 510656, 510657, 510658, 510659, 510673, 510674, 510683, 510686, 510693, 510695, 510701, 510702, 510707, 510708, 510711, 510716, 510722, 510725, 510726, 510728, 510731, 510734, 510737, 510744, 510748, 510749, 510751, 510752, 510753, 510756, 510757, 510758, 510761, and 510762. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a giant cellular osteoblastoclastoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510612, 510620, 510635, 510637, 510641, 510644, 510648, 510658, 510662, 510663, 510667, 510668, 510670, 510676, 510678, 510679, 510682, 510683, 510685, 510686, 510699, 510708, 510712, 510722, 510737, and 510753. The oligonucleotides may incorporate various chemical modifications, additions, deletions, insertions, substitutions or other modifications so long as functional aspects of the oligonucleotides are enhanced or maintained in whole or in part.

The disease or disorder may comprise a brain tumor and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510619, 510624, 510628, 510639, 510641, 510645, 510647, 510648, 510655, 510657, 510665, 510668, 510673, 510674, 510689, 510695, 510698, 510699, 510705, 510710, 510711, 510712, 510713, 510716, 510734, 510737, 510738, and 510762. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a colorectal adenocarcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510603, 510607, 510611, 510616, 510618, 510619, 510623, 510624, 510641, 510644, 510646, 510647, 510655, 510656, 510672, 510673, 510690, 510693, 510695, 510698, 510701, 510702, 510709, 510711, 510714, 510716, 510719, 510723, 510724, 510725, 510726, 510730, 510731, 510734, 510735, 510737, 510738, 510743, 510744, 510748, 510749, 510751, 510752, 510754, 510755, 510757, 510758, 510760, 510761, 510762, and 510763. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises a chronic obstructive pulmonary disease (COPD) and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510604, 510608, 510609, 510612, 510614, 510616, 510619, 510620, 510629, 510634, 510637, 510640, 510647, 510649, 510653, 510661, 510666, 510667, 510669, 510673, 510678, 510682, 510689, 510690, 510701, 510707, 510715, 510723, 510727, 510728, 510749, 510754, 510755, 510757, 510758, 510762, and 510763. In a related embodiment, the disease or disorder comprises a chronic obstructive pulmonary disease (COPD) and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510609, 510611, 510613, 510620, 510634, 510637, 510647, 510648, 510654, 510664, 510668, 510679, 510694, 510696, 510698, 510699, 510701, 510705, 510706, 510710, 510718, 510734, and 510741. The oligonucleotides may incorporate various chemical modifications, additions, deletions, insertions, substitutions or other modifications so long as functional aspects of the oligonucleotides are enhanced or maintained in whole or in part.

The disease or disorder can be a squamous cell carcinoma of the cervix and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510615, 510619, 510623, 510626, 510630, 510632, 510633, 510635, 510638, 510639, 510641, 510642, 510644, 510647, 510650, 510655, 510656, 510657, 510661, 510673, 510674, 510677, 510683, 510688, 510693, 510695, 510698, 510711, 510712, 510714, 510716, 510717, 510722, 510725, 510729, 510731, 510734, 510737, 510743, 510745, 510747, 510753, 510755, 510756, 510758, 510759, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise an acute myocardial infarction (AMI) or acute heart failure and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510608, 510613, 510626, 510629, 510631, 510634, 510635, 510638, 510639, 510646, 510648, 510656, 510667, 510669, 510671, 510672, 510675, 510682, 510689, 510698, 510701, 510707, 510710, 510715, 510721, 510723, 510727, 510728, 510730, 510731, 510734, 510735, 510743, 510744, 510748, 510749, 510751, 510752, 510757, 510758, 510760, 510761, and 510762. In a related embodiment, the disease or disorder comprises an acute myocardial infarction (AMI) or acute heart failure and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510601, 510606, 510607, 510608, 510609, 510611, 510614, 510616, 510619, 510622, 510624, 510626, 510635, 510636, 510637, 510640, 510641, 510643, 510644, 510648, 510651, 510665, 510668, 510669, 510672, 510675, 510677, 510678, 510679, 510682, 510692, 510695, 510696, 510698, 510699, 510701, 510703, 510707, 510710, 510725, 510726, 510728, 510729, 510730, 510731, 510733, 510734, 510736, 510743, 510750, 510751, 510752, 510755, 510757, 510758, 510759, 510760, 510761, and 510762. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises Chron's Disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510602, 510608, 510611, 510624, 510644, 510653, 510656, 510659, 510669, 510671, 510676, 510686, 510689, 510690, 510697, 510698, 510700, 510713, 510727, 510728, 510729, 510731, 510734, 510744, 510751, and 510757. In a related embodiment, the disease or disorder comprises Chron's Disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510610, 510611, 510615, 510618, 510621, 510623, 510625, 510626, 510628, 510631, 510632, 510635, 510637, 510638, 510643, 510647, 510648, 510649, 510653, 510654, 510655, 510657, 510658, 510666, 510667, 510668, 510672, 510675, 510677, 510678, 510679, 510680, 510682, 510684, 510689, 510691, 510694, 510696, 510698, 510701, 510707, 510708, 510709, 510710, 510713, 510714, 510715, 510719, 510725, 510727, 510728, 510729, 510730, 510731, 510734, 510736, 510738, 510743, 510744, 510748, 510750, 510751, 510755, 510757, 510758, 510759, 510760, 510761, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise diabetes mellitus type II and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510604, 510608, 510610, 510611, 510614, 510616, 510620, 510624, 510629, 510632, 510634, 510640, 510649, 510667, 510669, 510670, 510671, 510678, 510685, 510700, 510701, 510702, 510707, 510709, 510718, 510721, 510723, 510726, 510727, 510728, 510729, 510730, 510731, 510733, 510735, 510743, 510748, 510749, 510752, 510754, 510755, 510757, 510758, 510760, 510761, 510762, and 510763. Relatedly, the disease or disorder may comprise diabetes mellitus type II and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510613, 510632, 510635, 510636, 510641, 510645, 510647, 510648, 510654, 510660, 510664, 510667, 510668, 510670, 510675, 510684, 510691, 510695, 510696, 510706, 510710, 510734, and 510749. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises an esophageal carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510602, 510619, 510623, 510632, 510635, 510638, 510641, 510644, 510653, 510656, 510661, 510671, 510682, 510689, 510693, 510698, 510714, 510722, 510725, 510731, 510734, 510738, 510753, and 510761. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a squamous cell carcinoma of the larynx and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510605, 510607, 510612, 510614, 510619, 510623, 510632, 510635, 510641, 510642, 510655, 510656, 510657, 510659, 510661, 510668, 510673, 510674, 510689, 510690, 510693, 510695, 510698, 510708, 510712, 510732, 510734, 510737, 510738, 510745, 510747, 510753, and 510755. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises an acute or chronic leukemia of the bone marrow and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510605, 510607, 510610, 510612, 510628, 510631, 510633, 510641, 510644, 510650, 510664, 510670, 510673, 510674, 510675, 510681, 510684, 510685, 510686, 510701, 510711, 510712, 510717, 510718, 510719, 510720, 510721, 510724, 510729, 510732, 510739, 510740, 510743, 510745, 510746, 510747, 510752, 510754, and 510763. The oligonucleotides may incorporate various chemical modifications, additions, deletions, insertions, substitutions or other modifications so long as functional aspects of the oligonucleotides are enhanced or maintained in whole or in part.

In yet another embodiment, the disease or disorder comprises a lung carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510604, 510626, 510628, 510631, 510633, 510635, 510649, 510650, 510654, 510668, 510672, 510674, 510677, 510699, 510701, 510702, 510710, 510712, 510715, 510717, 510719, 510720, 510721, 510723, 510724, 510726, 510727, 510733, 510735, 510738, 510743, 510744, 510745, 510746, 510747, 510750, 510751, 510754, 510755, 510758, 510760, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise a malignant lymphoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510611, 510618, 510623, 510624, 510631, 510632, 510636, 510638, 510641, 510644, 510645, 510647, 510656, 510660, 510662, 510672, 510673, 510675, 510690, 510693, 510701, 510702, 510707, 510708, 510713, 510717, 510719, 510721, 510723, 510724, 510725, 510726, 510728, 510729, 510730, 510731, 510734, 510735, 510737, 510743, 510744, 510749, 510751, 510752, 510754, 510755, 510756, 510757, 510758, 510760, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder may also comprise multiple sclerosis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510607, 510612, 510620, 510646, 510653, 510655, 510661, 510663, 510669, 510675, 510682, 510685, 510686, 510690, 510699, 510701, 510710, 510713, 510731, 510738, 510747, 510758, and 510762. Relatedly, the disease or disorder may comprise multiple sclerosis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510604, 510609, 510610, 510613, 510617, 510618, 510622, 510632, 510635, 510647, 510670, 510675, 510677, 510687, 510690, 510692, 510695, 510701, 510706, 510708, 510731, and 510733. The oligonucleotides may incorporate various chemical modifications, additions, deletions, insertions, substitutions or other modifications so long as functional aspects of the oligonucleotides are enhanced or maintained in whole or in part.

In an embodiment, the disease or disorder comprises an ovarian carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510618, 510626, 510628, 510633, 510638, 510641, 510642, 510643, 510645, 510646, 510647, 510650, 510652, 510658, 510665, 510666, 510673, 510674, 510677, 510682, 510683, 510689, 510705, 510707, 510712, 510717, 510722, 510724, 510729, 510732, 510735, 510737, 510745, 510746, 510747, 510753, and 510756. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises Parkinson disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510601, 510604, 510608, 510609, 510612, 510614, 510624, 510631, 510633, 510634, 510640, 510641, 510642, 510649, 510650, 510651, 510653, 510667, 510673, 510675, 510676, 510677, 510683, 510686, 510689, 510694, 510700, 510703, 510704, 510705, 510706, 510707, 510709, 510713, 510715, 510721, 510723, 510724, 510726, 510727, 510729, 510730, 510731, 510732, 510734, 510735, 510736, 510737, 510739, 510740, 510741, 510742, 510744, 510745, 510746, 510747, 510748, 510751, 510752, 510754, 510756, 510757, 510758, 510759, 510760, 510761, and 510762. In a related embodiment, the disease or disorder comprises Parkinson disease and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510606, 510608, 510609, 510610, 510614, 510616, 510632, 510634, 510643, 510644, 510647, 510648, 510654, 510662, 510664, 510665, 510667, 510668, 510670, 510677, 510678, 510679, 510687, 510692, 510696, 510698, 510699, 510701, 510703, 510704, 510706, 510708, 510710, 510722, 510727, 510734, 510736, 510741, 510742, 510753, and 510756. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises a prostate adenocarcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510603, 510607, 510619, 510623, 510624, 510628, 510641, 510642, 510644, 510647, 510650, 510655, 510656, 510657, 510669, 510673, 510674, 510677, 510684, 510688, 510689, 510690, 510695, 510698, 510701, 510709, 510710, 510718, 510726, 510734, 510737, 510738, 510739, 510757, and 510762. The oligonucleotides may incorporate various chemical modifications, additions, deletions, insertions, substitutions or other modifications so long as functional aspects of the oligonucleotides are enhanced or maintained in whole or in part.

In yet another embodiment, the disease or disorder comprises psoriasis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510601, 510608, 510609, 510611, 510614, 510620, 510624, 510626, 510631, 510633, 510634, 510635, 510638, 510647, 510649, 510650, 510653, 510656, 510665, 510666, 510667, 510669, 510672, 510674, 510675, 510680, 510685, 510689, 510698, 510702, 510707, 510711, 510712, 510715, 510717, 510719, 510720, 510721, 510723, 510724, 510726, 510727, 510728, 510729, 510730, 510731, 510734, 510735, 510743, 510744, 510745, 510746, 510747, 510748, 510749, 510750, 510751, 510752, 510754, 510755, 510757, 510758, 510759, 510760, 510761, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder can be psoriasis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510601, 510604, 510613, 510621, 510627, 510637, 510641, 510644, 510648, 510650, 510652, 510663, 510667, 510668, 510676, 510680, 510681, 510699, 510701, 510703, 510705, 510709, 510710, 510722, 510734, 510739, 510750, and 510753. The oligonucleotides may incorporate various chemical modifications, additions, deletions, insertions, substitutions or other modifications so long as functional aspects of the oligonucleotides are enhanced or maintained in whole or in part.

The disease or disorder can also be rheumatoid arthritis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510603, 510604, 510607, 510608, 510609, 510614, 510616, 510622, 510625, 510627, 510629, 510630, 510634, 510635, 510636, 510637, 510640, 510642, 510646, 510649, 510650, 510651, 510653, 510656, 510664, 510665, 510667, 510671, 510675, 510677, 510678, 510679, 510682, 510689, 510699, 510707, 510726, 510727, 510728, 510729, 510731, 510733, 510737, 510738, 510748, 510755, 510758, 510761, and 510762. The oligonucleotides may incorporate various chemical modifications, additions, deletions, insertions, substitutions or other modifications so long as functional aspects of the oligonucleotides are enhanced or maintained in whole or in part.

In some embodiments, the disease or disorder comprises rheumatoid arthritis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510601, 510603, 510604, 510606, 510607, 510608, 510609, 510610, 510611, 510612, 510613, 510614, 510616, 510617, 510618, 510622, 510623, 510625, 510629, 510630, 510634, 510635, 510636, 510637, 510638, 510639, 510640, 510641, 510643, 510644, 510645, 510646, 510648, 510649, 510651, 510652, 510653, 510654, 510658, 510662, 510663, 510664, 510665, 510666, 510667, 510668, 510669, 510671, 510673, 510677, 510678, 510679, 510682, 510685, 510692, 510696, 510697, 510698, 510699, 510701, 510703, 510710, 510716, 510722, 510725, 510726, 510727, 510730, 510733, 510734, 510738, 510741, 510743, 510753, 510755, and 510757. The oligonucleotides may incorporate various chemical modifications, additions, deletions, insertions, substitutions or other modifications so long as functional aspects of the oligonucleotides are enhanced or maintained in whole or in part.

In another embodiment, the disease or disorder comprises a renal cell carcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510603, 510604, 510606, 510608, 510614, 510616, 510622, 510628, 510629, 510630, 510632, 510634, 510635, 510640, 510644, 510645, 510646, 510652, 510653, 510656, 510664, 510665, 510666, 510667, 510671, 510675, 510677, 510683, 510685, 510687, 510690, 510701, 510722, 510726, 510728, 510729, 510730, 510731, 510732, 510733, 510734, 510738, 510748, 510749, 510752, 510753, 510758, 510761, and 510762. The oligonucleotides may incorporate various chemical modifications, additions, deletions, insertions, substitutions or other modifications so long as functional aspects of the oligonucleotides are enhanced or maintained in whole or in part.

In still another embodiment, the disease or disorder comprises a squamous cell carcinoma of skin and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510618, 510622, 510626, 510639, 510641, 510642, 510650, 510658, 510673, 510674, 510683, 510696, 510708, 510712, 510717, 510720, 510722, 510723, 510724, 510727, 510729, 510743, 510745, 510746, 510747, 510752, 510753, 510754, 510755, 510756, 510759, 510761, and 510763. The oligonucleotides may incorporate various chemical modifications, additions, deletions, insertions, substitutions or other modifications so long as functional aspects of the oligonucleotides are enhanced or maintained in whole or in part.

In various embodiments, the disease or disorder comprises an adenocarcinoma of the stomach and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510604, 510608, 510609, 510612, 510626, 510631, 510632, 510634, 510639, 510653, 510658, 510663, 510667, 510681, 510689, 510692, 510693, 510696, 510698, 510699, 510705, 510711, 510715, 510717, 510719, 510723, 510725, 510729, 510731, 510734, 510735, 510736, 510743, 510746, 510748, 510751, 510754, 510757, 510760, 510762, and 510763. The oligonucleotides may incorporate various chemical modifications, additions, deletions, insertions, substitutions or other modifications so long as functional aspects of the oligonucleotides are enhanced or maintained in whole or in part.

The disease or disorder may comprise a carcinoma of the thyroid gland and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510602, 510603, 510614, 510626, 510638, 510641, 510644, 510646, 510653, 510658, 510659, 510661, 510662, 510674, 510689, 510693, 510695, 510698, 510699, 510701, 510704, 510705, 510708, 510717, 510718, 510722, 510727, 510731, 510734, 510736, 510739, 510740, 510741, 510747, 510753, and 510755. The oligonucleotides may incorporate various chemical modifications, additions, deletions, insertions, substitutions or other modifications so long as functional aspects of the oligonucleotides are enhanced or maintained in whole or in part.

The disease or disorder may also comprise a testicular cancer and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510600, 510603, 510607, 510615, 510616, 510618, 510619, 510621, 510622, 510623, 510624, 510630, 510636, 510637, 510641, 510644, 510645, 510647, 510650, 510654, 510655, 510656, 510657, 510659, 510662, 510665, 510670, 510673, 510674, 510681, 510684, 510685, 510687, 510688, 510689, 510690, 510692, 510693, 510695, 510696, 510698, 510700, 510701, 510704, 510707, 510712, 510713, 510717, 510718, 510726, 510734, 510737, 510738, 510739, 510740, 510742, 510747, 510756, and 510757. The oligonucleotides may incorporate various chemical modifications, additions, deletions, insertions, substitutions or other modifications so long as functional aspects of the oligonucleotides are enhanced or maintained in whole or in part.

The disease or disorder can be ulcerative colitis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510599, 510607, 510611, 510612, 510616, 510620, 510621, 510622, 510624, 510626, 510632, 510633, 510636, 510646, 510655, 510659, 510672, 510673, 510675, 510676, 510677, 510682, 510684, 510691, 510692, 510702, 510703, 510704, 510705, 510706, 510707, 510709, 510710, 510715, 510721, 510723, 510724, 510728, 510729, 510730, 510731, 510735, 510736, 510737, 510739, 510740, 510741, 510743, 510744, 510748, 510751, 510752, 510754, 510757, 510758, 510759, 510760, 510761, and 510762. In a related embodiment, the disease or disorder comprises ulcerative colitis and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510610, 510611, 510612, 510619, 510622, 510623, 510634, 510635, 510641, 510647, 510648, 510654, 510657, 510664, 510668, 510670, 510671, 510676, 510677, 510678, 510679, 510689, 510691, 510696, 510701, 510703, 510704, 510710, 510722, 510734, 510742, and 510753. The oligonucleotides may incorporate various chemical modifications, additions, deletions, insertions, substitutions or other modifications so long as functional aspects of the oligonucleotides are enhanced or maintained in whole or in part.

The disease or disorder can also be a uterine adenocarcinoma and the oligonucleotide or plurality of oligonucleotides useful for characterization thereof comprises at least 1, 2, 3, 4, 5, 6, 7, 8, 9, 10, 11, 12, 13, 14, 15, 16, 17, 18, 19, 20, 25, 30, 40, 45, 50 or all of SEQ ID NOs. 510601, 510612, 510621, 510626, 510637, 510641, 510644, 510650, 510653, 510669, 510675, 510684, 510686, 510687, 510696, 510714, 510717, 510722, 510739, 510743, 510745, 510746, 510753, 510755, and 510762. The oligonucleotides may incorporate various chemical modifications, additions, deletions, insertions, substitutions or other modifications so long as functional aspects of the oligonucleotides are enhanced or maintained in whole or in part.

In an aspect, the invention provides a kit comprising a reagent for carrying out the methods of the invention provided herein, wherein the reagent comprises a plurality of oligonucleotides. The oligonucleotide or plurality of oligonucleotides are those provided herein. The reagent may comprise various other useful components including without limitation one or more of: a) a reagent configured to isolate a microvesicle, optionally wherein the at least one reagent configured to isolate a microvesicle comprises a binding agent to a microvesicle antigen, a column, a substrate, a filtration unit, a polymer, polyethylene glycol, PEG4000, PEG8000, a particle or a bead; b) at least one oligonucleotide configured to act as a primer or probe in order to amplify, sequence, hybridize or detect the oligonucleotide or plurality of oligonucleotides; and c) a reagent configured to remove one or more abundant protein from a sample, wherein optionally the one or more abundant protein comprises at least one of albumin, immunoglobulin, fibrinogen and fibrin.

***Optimized Oligonucleotide Library Constructon***

As described herein, individual aptamers and oligonucleotide probe libraries are generated by screening or enriching an input oligonucleotide library against one or more target of interest. The input library may be referred to as a starting or naive oligonucleotide library and may comprise at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences. The library typically comprises known sequences on the 5' and/or 3' ends to facilitate primer binding, probe binding, hybridization, amplification, sequencing and the like. These flanking regions may surround a section comprising randomly generated sequences that create the library diversity. The disclosure provides oligonucleotide sequences that enhance both the flanking and variable regions used for library construction. Such constructs may enhance any desired characteristic of the naive library for use in screening and enrichment applications, including without limitation synthesis, diversity, and sequence determination. The enhanced flanking and variable regions may be used separately or together. See **Examples 19** and **23** herein for illustrative examples.

In an aspect, the disclosure provides a composition comprising an input oligonucleotide library for assessing a cellular or extracellular vesicle sample comprising at least two subset oligonucleotide libraries to generate the input oligonucleotide library, wherein at least one of the at least two subset oligonucleotide libraries are manufactured with amounts of nucleotides with a total G and C content that is not equal to 50%. For example, the total G and C content can be less than 50%, or the the total G and C content can be more than 50%. At least one subset library may be manufactured with a total G and C content that is more than 50% and another subset library is manufactured with a total G and C content less than 50%. The at least two subset libraries may comprise three subset libraries manufactured with 25%, 50% and 75% G and C content, respectively. The at least two subset libraries can be manufactured with amounts of nucleotides similar or equal to at least two rows in **Table 13**. The nucleotides can consist of naturally occurring nucleotides or modified naturally occurring nucleotides as desired. The nucleotides can also comprise non-naturally occurring nucleotides. Such input oligonucleotide libraries may be referred to as "GC" libraries herein. The GC libraries may be generated as the variable region in a naive input library for further screening or enrichment purposes. See **Example 19.**

In a related aspect, the disclosure provides a method of generating an input oligonucleotide library, comprising contacting the at least two subset oligonucleotide libraries to generate the input oligonucleotide library. The input oligonucleotide library can be screened or enriched to provide various aptamers or oligonucleotide probe libraries using the methods described herein.

In an aspect, the disclosure provides an oligonucleotide having a sequence that comprises a 5' transposon adapter region, an offset region comprising 0 or more nucleotides located 5' to the transposon adapter region, a variable region located 5' to the offset region, and a left primer region located 5' to the variable region. In a related aspect, the disclosure provides a plurality of oligonucleotides comprising at least 1, 2, 3, 4, 5, 6, 7, 8, 9, 10, 11, 12, 13, 14, 15, 16, 17, 18, 19, 20, 25, 30, 40, 45, 50, 55, 60, 65, 70, 75, 80, 85, 90, 95, 100, 125, 150, 175, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences, wherein each of the oligonucleotide sequences comprises a 5' transposon adapter region, an offset region comprising 0 or more nucleotides located 5' to the transposon adapter region, a variable region located 5' to the offset region, and a left primer region located 5' to the variable region. Such oligonucleotides may be referred to herein as "balanced" oligonucleotides. See **Example 23.**

The balanced oligonucleotide or plurality of balanced oligonucleotides can be such that: a) the transposon adapter region comprises 20-40 nucleotides; b) the offset region compries 0, 1, 2, 3, 4, 5, 6, 7, 8, 9 or 10 nucleotides; c) the variable region comprises 20-50 nucleotides; and/or d) the left primer region comprises 20-40 nucleotides. The balanced oligonucleotides may be such that: a) the transposon adapter region comprises a nucleic acid sequence that is at least about 50, 55, 60, 65, 70, 75, 80, 85, 90, 95, 96, 97, 98, 99 or 100 percent homologous to the sequence 5'-TCGTCGGCAGCGTCAGATGTGTATAAGAGACAG (SEQ ID NO. 510764); b) the offset region compries 1, 2, 3, 4, 5, 6, 7, 8, 9 or 10 nucleotides that are at least about 50, 55, 60, 65, 70, 75, 80, 85, 90, 95, 96, 97, 98, 99 or 100 percent homologous to at least one of the following sequences: 5'-T (SEQ ID NO. 510765), 5'-CT (SEQ ID NO. 510766) and 5'-ACT (SEQ ID NO. 510767); c) the variable region comprises 10, 11, 12, 13, 14, 15,1 6, 17, 18, 19, 20, 21, 22, 23, 24, 25 26 27, 28, 29, 30, 31, 32, 33, 34, 35, 36, 37, 38, 39, 40, 41, 42, 43, 44, 45, 46, 47, 48, 49, 50, or more than 50 nucleotides; and/or d) the left primer region comprises a nucleic acid sequence that is at least about 50, 55, 60, 65, 70, 75, 80, 85, 90, 95, 96, 97, 98, 99 or 100 percent homologous to a sequence selected from SEQ ID NOs. 510768 and 510769. The variable region may comprise "GC" library sequences as provided herein.

In an aspect, the disclosure provides a method of identifying an aptamer, comprising performing a selection or enrichment process using a balanced oligonucleotide or plurality of balanced oligonucleotides as a naive input library. Such selection or enrichment processes are described or provided herein, including without limitation SELEX and variations thereof. The selection process may comprise at least one round of positive selection against a target of interest and optionally at least one round of negative selection against a target other than the target of interest.

In another aspect, the disclosure provides a method comprising contacting a balanced oligonucleotide or plurality of balanced oligonucleotides with a sample and detecting the presence or level of binding of the oligonucleotide or plurality of oligonucleotides to a target in the sample. The sample may comprise a biological sample, such as an organic sample, an inorganic sample, a tissue, a cell culture, a bodily fluid, blood, serum, a cell, a microvesicle, a protein complex, a lipid complex, a carbohydrate, or any combination, fraction or variation thereof. The target can be any useful target, including without limitation a cell, an organelle, a protein complex, a lipoprotein, a carbohydrate, a microvesicle, a membrane fragment, a small molecule, a heavy metal, a toxin, or a drug.

In a related aspect, the disclosure provides a method comprising: a) contacting a biological sample comprising microvesicles with an oligonucleotide probe library, wherein the oligonucleotide probe library comprises a balanced oligonucleotide or plurality of balanced oligonucleotides; b) identifying oligonucleotides bound to at least a portion of the microvesicles; and c) characterizing the sample based on a profile of the identified oligonucleotides.

In another related aspect, the disclosure provides a method comprising: a) contacting a sample with an oligonucleotide probe library comprising at least $10^6$, $10^7$, $10^8$, $10^9$, $10^{10}$, $10^{11}$, $10^{12}$, $10^{13}$, $10^{14}$, $10^{15}$, $10^{16}$, $10^{17}$, or at least $10^{18}$ different oligonucleotide sequences oligonucleotides to form a mixture in solution, wherein the oligonucleotides are capable of binding a plurality of entities in the sample to form complexes, wherein the oligonucleotide probe library comprises a balanced oligonucleotide or plurality of balanced oligonucleotides; d) partitioning the complexes formed in step (a) from the mixture; and c) detecting oligonucleotides present in the complexes partitioned in step (b) to identify an oligonucleotide profile for the sample. In a related aspect, the disclosure provides a method of characterizing a disease or disorder, comprising: a) contacting a biological test sample with a balanced oligonucleotide or plurality of balanced oligonucleotides; b) detecting a presence or level of complexes formed in step (a) between the balanced oligonucleotide or plurality of balanced oligonucleotides and a target in the biological test sample; and c) comparing the presence or level detected in step (b) to a reference level from a biological control sample, thereby characterizing the disease or disorder. The step of detecting may comprise performing sequencing of all or some of the oligonucleotides in the complexes, amplification of all or some of the oligonucleotides in the complexes, and/or hybridization of all or some of the oligonucleotides in the complexes to an array. In some embodiments, the sequencing comprises high-throughput sequencing.

In the methods comprising use of a balanced oligonucleotide or plurality of balanced oligonucleotides, the biological test sample and biological control sample may each comprise a tissue sample, a cell culture, or a biological fluid. In some embodiments, the biological fluid comprises a bodily fluid. Useful bodily fluids within the method of the invention comprise peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood. In some preferred embodiments, the bodily fluid comprises blood, serum or plasma. The biological fluid may comprise microvesicles. In such case, the complexes may be formed between the oligonucleotide or plurality of oligonucleotides and at least one of the microvesicles. The biological test sample and biological control sample may further comprise isolated microvesicles, wherein optionally the microvesicles are isolated using at least one of chromatography, filtration, ultrafiltration, centrifugation, ultracentrifugation, flow cytometry, affinity capture (e.g., to a planar surface, column or bead), polymer precipitation, and using microfluidics. The vesicles can also be isolated after contact with the oligonucleotide or plurality of oligonucleotides.

In various aspects of the methods comprising use of a balanced oligonucleotide or plurality of balanced oligonucleotides, the oligonucleotide or plurality of oligonucleotides binds a polypeptide or fragment thereof. The polypeptide or fragment thereof can be soluble or membrane bound, wherein optionally the membrane comprises a microvesicle membrane. The membrane could also be from a cell or a fragment of a cell of vesicle. The polypeptide or fragment thereof may comprise a biomarker in **Table 3** or **Table 4.** For example, the polypeptide or fragment thereof could be a general vesicle marker such as in **Table 3** or a tissue-related or disease-related marker such as in **Table 4.** The oligonucleotide or plurality of oligonucleotides may bind a microvesicle surface antigen in the biological sample. For example, the oligonucleotide or plurality of oligonucleotides can be enriched from a naive library against microvesicles.

The disease or disorder detected by the oligonucleotide, plurality of oligonucleotides, or methods provided herein comprising use of a balanced oligonucleotide or plurality of balanced oligonucleotides may comprise any appropriate disease or disorder of interest. For example, the disease or disorder may comprise a cancer, a premalignant condition, an inflammatory disease, an immune disease, an autoimmune disease or disorder, a cardiovascular disease or disorder, a neurological disease or disorder, an infectious disease, and/or pain. See, e.g., section "Phenotypes" herein. The disease or disorder may include without limitation Breast Cancer, Alzheimer's disease, bronchial asthma, Transitional cell carcinoma of the bladder, Giant cellular osteoblastoclastoma, Brain Tumor, Colorectal adenocarcinoma, Chronic obstructive pulmonary disease (COPD), Squamous cell carcinoma of the cervix, acute myocardial infarction (AMI) / acute heart failure, Chron's Disease, diabetes mellitus type II, Esophageal carcinoma, Squamous cell carcinoma of the larynx, Acute and chronic leukemia of the bone marrow, Lung carcinoma, Malignant lymphoma, Multiple Sclerosis, Ovarian carcinoma, Parkinson disease, Prostate adenocarcinoma, psoriasis, Rheumatoid Arthritis, Renal cell carcinoma, Squamous cell carcinoma of skin, Adenocarcinoma of the stomach, carcinoma of the thyroid gland, Testicular cancer, ulcerative colitis, or Uterine adenocarcinoma.

The disclosure further provides a kit comprising a reagent for carrying out the methods herein and also use of the reagent for carrying out the methods related to GC libraries or balanced oligonucleotides. For example, the reagent may comprise one or more oligonucleotide having a sequence derived from a GC libraries and/or a balanced oligonucleotide library. The reagent may further comprise other useful components disclosed herein including without limitation at least one of: a) a reagent configured to isolate a microvesicle, optionally wherein the at least one reagent configured to isolate a microvesicle comprises a binding agent to a microvesicle antigen, a column, a substrate, a filtration unit, a polymer, polyethylene glycol, PEG4000, PEG8000, a particle or a bead; b) at least one oligonucleotide configured to act as a primer or probe in order to amplify, sequence, hybridize or detect the oligonucleotide or plurality of oligonucleotides; and c) a reagent configured to remove one or more abundant protein from a sample, wherein optionally the one or more abundant protein comprises at least one of albumin, immunoglobulin, fibrinogen and fibrin.

**Kits**

The invention also provides a kit comprising one or more reagent to carry out the methods of the invention, wherein the at least one reagent comprises the plurality of oligonucleotides. The one or more reagent may comprise any useful reagents for carrying out the subject methods, including without limitation aptamer libraries, substrates such as microbeads or planar arrays or wells, reagents for biomarker and/or microvesicle isolation (e.g., via chromatography, filtration, ultrafiltration, centrifugation, ultracentrifugation, flow cytometry, affinity capture (e.g., to a planar surface, column or bead), polymer precipitation, and/or using microfluidics), aptamers directed to specific targets, aptamer pools that facilitate detection of a biomarker/microvesicle population, reagents such as primers for nucleic acid sequencing or amplification, arrays for nucleic acid hybridization, detectable labels, solvents or buffers and the like, various linkers, various assay components, blockers, and the like. The one or more reagent may also comprise various compositions disclosed above. The one or more reagent comprises one or more aptamer. The one or more reagent can comprise a substrate, such as a planar substate, column or bead. The kit can contain instructions to carry out various assays using the one or more reagent.

In an aspect, the kit comprises an aptamer or composition provided herein. The kit can be configured to carry out the methods provided herein. For example, the kit can include an aptamer, a substrate, or both an aptamer and a substrate.

In an embodiment, the kit is configured to carry out an assay. For example, the kit can contain one or more reagent and instructions for detecting the presence or level of a biological entity in a biological sample. In such cases, the kit can include one or more binding agent to a biological entity of interest. The one or more binding agent can be bound to a substrate.

In an aspect, the kit comprises a set of aptamers that provide a particular aptamer profile for a biological sample. An aptamer profile can include, without limitation, a profile that can be used to characterize a particular disease or disorder. For example, the disease or disorder can be a proliferative disease or disorder, including without limitation a cancer. The disease or disorder can be selected from a disease or disorder in **Table 16.**

## EXAMPLES

### Example 1: Identification of DNA oligonucleotides that bind a target

The target is affixed to a solid substrate, such as a glass slide or a magnetic bead. For a magnetic bead preparation, beads are incubated with a concentration of target protein ranging from 0.1 to 1 mg/ml. The target protein is conjugated to the beads according to a chemistry provided by the particular bead manufacturer. Typically, this involves coupling via an N-hydroxysuccinimide (NHS) functional group process. Unoccupied NHS groups are rendered inactive following conjugation with the target.

Randomly generated oligonucleotides (oligos) of a certain length, such as 32 base pairs long, are added to a container holding the stabilized target. Each oligo contains 6 thymine nucleotides (a "thymine tail") at either the 5 or 3 prime end, along with a single molecule of biotin conjugated to the thymine tail. Additional molecules of biotin could be added. Each oligo is also manufactured with a short stretch of nucleotides on each end (5-10 base pairs long) corresponding to amplification primers for PCR ("primer tails"). The sequences are shown absent the thymine tails or primer tails.

The oligonucleotides are incubated with the target at a specified temperature and time in phosphate-buffered saline (PBS) at 37 degrees Celsius in 500 microliter reaction volume.

The target/oligo combination is washed 1-10 times with buffer to remove unbound oligo. The number of washes increases with each repetition of the process (as noted below).

The oligos bound to the target are eluted using a buffer containing a chaotropic agent such as 7 M urea or 1% SDS and collected using the biotin tag. The oligos are amplified using the polymerase chain reaction using primers specific to 5' and 3' sequences added to the randomized region of the oligos. The amplified oligos are added to the target again for another round of selection. This process is repeated as desired to observe binding enrichment.

### Example 2: Competitive assay

The process is performed as in **Example 1** above, except that a known ligand to the target, such as an antibody, is used to elute the bound oligo species (as opposed to or in addition to the chaotropic agent). In this case, anti-EpCAM antibody from Santa Cruz Biotechnology, Inc. was used to elute the aptamers from the target EpCAM.

### Example 3: Screening and Affinity Analysis

All aptamers generated from the binding assays described above are sequenced using a high-throughput sequencing platform, such as the Ion Torrent from Life Technologies:

Library Preparation - Aptamers were pooled after ligating barcodes and adapter sequences (Life Technologies) according to manufacturer protocols. In brief, equimolar pools of the aptamers were made using the following steps: Analyzed an aliquot of each library with a Bioanalyzer™ instrument and Agilent DNA 1000 Kit or Agilent High Sensitivity Kit, as appropriate for the final library concentration. The molar concentration (nmol/L) of each amplicon library was detetrmined using the commercially available software (Agilent).

An equimolar pool of the library was prepared at the highest possible concentration.

The combined concentration of the pooled library stock was calculated.

The template dilution factor of the library pool was determined using the following equation:

Template Dilution Factor = (Library pool concentration [pM])/26 pM).

Template Preparation - Using a freshly diluted library, the aptamer pool resulting from binding assays provided above were sequenced using conventional sequencing protocols. High throughput (NextGen) sequencing methods can be used as desired.

Twenty aptamers were selected based on direct or competitive assays assessing binding to EpCAM (as described above).

Affinity Measurements - These twenty aptamers were then tested for binding affinity using an *in vitro* binding platform. SPR can be used for this step, e.g., a Biacore SPR machine using the T200 control software, as follows: Dilute the antigen to a concentration of 32 nM.

Prepare necessary dilutions for kinetics, starting at 32nM prepare two-fold dilutions of antigen down to 0.5nM.

The Biacore 200 control software is programmed with the following conditions: Solution: HBS-EP+ Buffer; Number of cycles: 3; Contact time: 120s; Flow rate: 30μl/min; Dissociation time: 300s; Solution: Glycine-HCl pH 2.5; Contact time: 120s; Flow rate: 20μl/min; Stabilization period: 0s. The binding affinities of these aptamers are then measured using the SPR assay above, or an alternate in vitro assay assessing the aptamer for a desired function.

**FIG. 5** shows the SPR data for aptamer BTX176881 (SEQ ID NO: 3). The figure comprises an association and dissociation graph of 1:1 fitting model of the biotinylated aptamers to EpCAM protein at the indicated concentrations (nM). **Table 5** shows the calculated $K_d$ values from the SPR measurements that are illustrated in **FIG. 5.** In addition, **Table 5** shows the SPR data and calculated $K_d$ values for BTX187269 (SEQ ID NO: 6) and Aptamer 4 (SEQ ID NO. 1).

Table 5: Calculated $K_D$ values from SPR measurements

| Immobilized aptamer | Analyte | Conc (nM) | Response | $K_d$ (nM) | Full $R^2$ | Full $Chi^2$ |
|---|---|---|---|---|---|---|
| BTX176881 (SEQ ID No: 3) | EpCAM protein | 500 | 0.2434 | 8.40 | 0.989322 | 0.179008 |
| | | 250 | 0.136 | 8.40 | 0.989322 | 0.179008 |
| | | 100 | 0.0776 | 8.40 | 0.989322 | 0.179008 |
| BTX187269 (SEQ ID NO: 6) | EpCAM protein | 500 | 0.2575 | 7.12 | 0.990323 | 0.215697 |
| | | 250 | 0.1584 | 7.12 | 0.990323 | 0.215697 |
| | | 100 | 0.0551 | 7.12 | 0.990323 | 0.215697 |
| Aptamer 4 (SEQ ID NO. 1) | EpCAM protein | 500 | 0.2742 | 10.10 | 0.986276 | 0.299279 |
| | | 250 | 0.1618 | 10.10 | 0.986276 | 0.299279 |
| | | 100 | 0.0809 | 10.10 | 0.986276 | 0.299279 |
| *$K_d$, $R^2$ and $Chi^2$ values by Global fitting for single reference method. | | | | | | |

## Example 4: Motif analysis

The process of **Example 3** is followed to identity a high affinity aptamer to a target of interest. Once a high affinity aptamer is identified, its sequence is then analyzed using a software program to estimate its two-dimensional folding structure. Well-known sequence alignment programs and algorithms for motif identification can be used to identify sequence motifs and reduce the dimensionality of even large data sets of sequences. Further, software programs such as Vienna and mfold are well-known to those skilled in the art of aptamer selection and can be used to further group sequences based on secondary structure motifs (shared shapes). See **FIG. 3A** and **FIG. 3B** for example structure predictions. Shared secondary structure of course, does not guarantee identical three-dimensional structure. Therefore "wet-lab" validation of aptamers is still useful as no one set of *in silico* tools has yet been able to fully predict the optimal aptamer among a set of aptamer candidates.

## Example 5: Microvesicle-based aptamer subtraction assay

Circulating microvesicles are isolated from normal plasma (e.g., from individuals without cancer) using one of the following methods: 1) Isolation using the ExoQuick reagent according to manufacturer's protocol; 2) Ultracentrifugation comprising spin at 50,000 to 150,000g for 1 to 20 hours then resuspending the pellet in PBS; 3) Isolation using the TEXIS reagent from Life Technologies according to manufacturer's protocol; and 4) filtration methodology. The filtration method is described in more detail as follows:

Place syringe and filter (1.2 μm Acrodisc Syringe Filter Versapor Membrane Non-Pyrogenic Ref: 4190, Pall Life Sciences) on open 7 ml 150K MWCO column (Pierce concentrators, 150K MWCO (molecular weight cut off) 7 ml. Part number: 89922). Fill open end of syringe with 5.2 ml of filtered IX PBS prepared in sterile molecular grade water.

Pipette patient plasma (900-1000 μl) into the PBS in the syringe, pipette mix twice

Filter the plasma into the 7 ml 150K MWCO column.

Centrifuge 7 ml 150K MWCO columns at 2000 x g at 20°C (16°C to 24°C) for 1 hour.

After 1 hour spin, pour the flow-through into 10% bleach to be discarded.

Visually inspect sample volume. If plasma concentrate is above the 8.5 ml graduation on the concentrator tube, continue to spin plasma sample at 10 minute increments at 2000 x g at 20°C (16°C to 24°C) checking volume after each spin until plasma concentrate is between 8.0 and 8.5 mls.

Pipette mix slowly on the column a minimum of 6 times and adjust pipette to determine plasma concentrate volume. If volume is between 100 μl and Target Volume, transfer plasma concentrate to previously labeled co-polymer 1.5 ml tube. If volume is still greater than Target Volume, repeat the above centrifugation step.

Pour ~45 mls of filtered IX PBS prepared in sterile molecular grade water into 50 ml conical tube for use in the next step.

Add the appropriate amount of filtered IX PBS to reconstitute the sample to the Target Volume.

The microvesicles produced using any of the isolation methods will comprise a mixture of vesicle types and will be various sizes with the possible exception of the ultracentrifugation methods, which may favor isolating exosome size particles.

Randomly generated oligonucleotides (produced as described in Example 1 above) are incubated with the isolated normal vesicles in PBS overnight at room temperature or at 4 degrees Celsius.

The aptamers that do not bind to these vesicles are isolated by spinning down the vesicles at 50,000 to 150,000 X g for 1 to 20 hours and collecting the supernatant.

The aptamer oligonucleotides are collected from the supernatant by running the mixture over a column containing streptavidin-coated beads. These aptamers are then added to vesicles isolated from diseased patients (using the same methods as above) and incubated overnight in PBS at room temperature or 4 degrees Celsius.

The vesicles are then spun at 50,000 to 150,000 X g for 1 to 20 hours and the supernatant is discarded. The vesicles are resuspended in PBS and lysed using SDS or some similar detergent.

The aptamers are then captured by running the lysis mixture over a column of streptavidin-coated beads. The isolated aptamers are then subjected to a round of PCR to amplify the products.

The process is then repeated for a set number of times, e.g., 5 times. The remaining aptamer pool has been depleted of aptamers that recognize microvesicles found in "normal" plasma. Accordingly, this method can be used to enrich the pool in aptamers that recognize cancer vesicles. See **FIG. 4.**

### Example 6: Detection of Microvesicles using anti-EpCAM aptamers

Aptamers can be used as binding agents to detect a biomarker. In this Example, aptamers are used as binding agents to detect EpCAM protein associated with microvesicles.

**FIG. 6** illustrates the use of an anti-EpCAM aptamer (Aptamer 4; SEQ ID NO. 1) to detect a microvesicle population in plasma samples. Plasma samples were obtained from three men with prostate cancer and three men without prostate cancer (referred to as controls or normals). Antibodies to the following microvesicle surface protein antigens of interest were conjugated to microbeads (Luminex Corp, Austin, TX): **FIG. 6A)** EGFR (epidermal growth factor receptor); **FIG. 6B)** PBP (prostatic binding protein; also known as PEBP1 (phosphatidylethanolamine binding protein 1)); **FIG. 6C)** EpCAM (epithelial cell adhesion molecule); and **FIG. 6D)** KLK2 (kallikrein-related peptidase 2). Microvesicles in the plasma samples were captured using the bead-conjugated antibodies. Fluorescently labeled Aptamer 4 was used as a detector in the microbead assay. **FIG. 6** shows the average median fluorescence values (MFI values) detected for the bead-captured and Aptamer 4 detected microvesicles. Each plot individually shows the three cancer (C1-C3) and three normal samples (N1-N3). These data show that, on average, the prostate cancer samples have higher levels of microvesicles containing the target proteins than the normals.

### Example 7: Negative and Positive Selection of Aptamers

Aptamers can be used in various biological assays, including numerous types of assays which rely on a binding agent. For example, aptamers can be used instead of antibodies in immune-based assays. This Example provides an aptamer screening method that identifies aptamers that do not bind to any surfaces (substrates, tubes, filters, beads, other antigens, etc.) throughout the assay steps and bind specifically to an antigen of interest. The assay relies on negative selection to remove aptamers that bind non-target antigen components of the final assay. The negative selection is followed by positive selection to identify aptamers that bind the desired antigen.

Preliminary experiments were done with five DNA aptamer libraries with $10^{15}$ sequences each and variable lengths (60, 65, 70, 75, 80-mers) were pre-amplified and strand separated so that forward strand (non-biotinylated) serves as an aptamer. Multiple rounds of negative selection and positive selection were performed. Before each round, the recovered aptamer products were PCR amplified and strand separated using standard methodology. Selections were performed as follows:

Negative selection

1. Prepare bead negative Selection Mix: Incubate 1200 non-magnetic beads with standard blocking agent for 20 min.
2. Add 50 µl of aptamer library (5 libraries total) to a PCR strip tube with 4.5 µl of each bead mixture. Incubate for 2 h at 37°C with agitation at 550 rpm.
3. Pre-wet filter plate (1.2 µm, Millipore) with PBS-BN buffer. Add 150 µl PBS-BN.
4. Transfer samples from the PCR strip tubes to the filter plate, incubate for 1 h at room temperature with agitation at 550 rpm.
5. Collect flow-through from filter plate into a collection (NBS) plate using a vacuum manifold.
6. Concentrate and clean samples to remove excess materials as desired.

The negative selection process is repeated up to 6-7 times.

Positive selection

Before starting, conjugate the protein biomarkers of interest (here, SSX4, SSX2, PBP, KLK2, SPDEF) to desired non-magnetic microbeads using conditions known in the art. The recombinant purified starting material included: SPDEF recombinant protein from Novus Biologicals (Littleton, CO, USA), catalog number H00025803-P01; KLK2 recombinant protein from Novus, catalog number H00003817-P02; SSX2 recombinant protein from Novus, catalog number H00006757-P01; PBP recombinant protein from Fitzgerald Industries International (Action, MA, USA), catalog number 30R-1382; SSX4 recombinant protein from GenWay Biotech, Inc. (San Diego, CA, USA), catalog number GWB-E219AC.

1. Bead blocking: Incubate a desired number of each bead (8400 x number of aptamer libraries (5) x an overage factor of (1.2)) with a starting block for 20 min.
2. Mix 50 µl of each aptamer library sample to PCR strip tubes add 2.3 µl of bead sample with particular antigen. Incubate for 2 h at 37°C with agitation at 550 rpm.
3. Pre-wet filter plate (1.2 µm, Millipore) with PBS-BN buffer. Add 150 µl PBS-BN.
4. Transfer samples from the PCR strip tubes to the filter plate, incubate for 1 h at room temperature with agitation at 550 rpm.
5. Wash 3x with PBS-BN, add 50 µl of PBS and collect samples from the top of the filter to the 1.5 ml tubes.

The positive selection is repeated up to 16 times. Certain rounds of positive selection have additional steps to treat the recovered RNA (i.e., remaining aptamer candidates) as follows:

Round 8 of positive selection was modified as follows:

1. After the third wash (PBS-BN) 25 µl of sample were collected from the top of the filter into 1.5 ml tubes.
2. The filter plate was incubated at 45°C for -10 min and washed immediately using vacuum. The plate was washed three more times with PBS-BN.
3. 50 µl of PBS were added to the plate and step 2 was repeated.
4. After the last wash, 25 µl of PBS was added to the wells. The samples were mixed well and collected from the top of the filter into 1.5 ml tubes.

Round 9 of positive selection was modified as follows:

1. After the final wash in step 5), 5 $\mu$g/ml Streptavidin-PE was added to the aptamer mixture and incubated for 30 min at room temperature with agitation at 550 rpm.

2. Samples on filter plate were washed 3x with PBS-BN (+ additional 500 mM NaCl).

3. One additional wash with regular PBS-BN was performed.

4. 50 $\mu$l of PBS was added to the samples followed by collection as above into 1.5 ml tubes.

5. Samples stored at -20°C.

Round 14 of positive selection was modified as follows:

Before start this round, the antigens of interest (SSX4, SSX2, PBP, KLK2, SPDEF) were conjugated to carboxylated magnetic beads using methods known in the art.

1. Bead blocking: take desired number of each non-magnetic bead (3000 x number of aptamer libraries (5) x an overage factor of 1.2), add starting block (3:1, blocking per 1200 beads), make 5 mixes of 4 antigens and supplement each with different target antigen conjugated to magnetic beads (see **Table 6** below, wherein the antigens are conjugated to non-magnetic beads except as indicated), incubate 20 min.

**Table 6: Bead blocking mixtures**

| Blocking Mix | Non-magnetic bead antigens | Magnetic bead antigens |
|---|---|---|
| 1 | SSX4 + PBP + KLK2 + SPDEF | SSX2 |
| 2 | SSX2 + PBP + KLK2 + SPDEF | SSX4 |
| 3 | SSX2 + SSX4 + KLK2 + SPDEF | PBP |
| 4 | SSX2 + SSX4 + PBP + SPDEF | KLK2 |
| 5 | SSX2 + SSX4 + PBP + KLK2 | SPDEF |

2. Add 50 $\mu$l of aptamer libraries to PCR strip tubes, add bead mixtures with target antigen on magnetic beads to the tubes with pre-selected corresponding aptamer library and incubate for 2 h at 37°C with agitation at 550 rpm.

3. Pre-wet filter plate with PBS-BN buffer, add 150 $\mu$l PBS-BN.

4. Transfer samples from PCR strip tubes to filter plate, incubate 1h room temperature with agitation at 550 rpm.

5. After last (standard) wash, add 5$\mu$g/ml Streptavidin-PE, incubate for 30 min room temperature with agitation at 550 rpm;

6. Wash 3x with PBS-BN (+ additional 500 mM NaCl).

7. Perform one additional wash with regular PBS-BN.

8. 50 $\mu$l of PBS was added to the samples followed by collection as above into 1.5 ml tubes.

9. Remove the magnetic beads using a magnetic stand, and replace with fresh PBS buffer.

10. Samples stored at -20°C for subsequent DNA extraction and strand separation.

Optional steps implemented in the later round of positive selection are intended to increase stringency of aptamer binding (e.g., increased heat or salt concentration).

## Example 8: Discovery and Characterization of anti-EpCAM aptamers

In this Example, an aptamer to EpCAM identified using the technique in the Example above is characterized. After selection for a pool of EpCAM binding aptamers as described above, the aptamer library was sequenced using the Ion Torrent standard protocol (Life Technologies, Carlsbad, CA). Lead candidates were selected as those having (a) high abundant motifs across all read sequences with full expected length product and (b) strong secondary structure (**FIG. 7B**).

Aptamers wre selected for EPCAM protein conjugated to MicroPlex beads in competition with SSX4, SSX2, PBP, KLK2, and SPDEF recombinant proteins. A portion of the aptamers was selected in initial rounds against EpCAM that was attached to an Fc tag, and after round 8 the selection was switched to EPCAM with a Histidine tag. Another portion of the aptamers was selected in initial rounds against EpCAM that was attached to a Histidine tag, and after round 8 the selection was switched to EPCAM with an Fc tag. Methods of using Fc and histidine tags for protein purification and capture are known to those of skill in the art.

Aptamer Characterization

CAR003 is an aptamer candidate identified using the above methodology. As an RNA aptamer, CAR003 with alternate tail sequence has the following RNA sequence (SEQ ID NO. 5):

$$5'\text{-auccagagug acgcagcagu cuuuucugau ggacacgugg uggucuagua}$$

$$\text{ucacuaagcc accgugucca-}3'$$

CAR003 was further characterized. EpCAM aptamer CAR003 is modified as desired by attachment of a biotin moiety on the 5'(CAR003.2_5'-B (SEQ ID NO. 4)) end or 3' end (CAR003.2_3'-B (SEQ ID NO. 7)). The biotin can be used to bind the aptamer using a streptavidin-biotin system, e.g., for labeling, capture and/or anchoring. **FIG. 7B** illustrates the optimal secondary structure of CAR003 with a minimum free energy ($\Delta$G) of -30.00 kcal/mol. For purposes of illustration, the aptamer is shown as an RNA aptamer (SEQ ID NO. 5) corresponding to the CAR003 DNA sequence (SEQ ID NOs. 4, 7).

Synthesis and purification. The selected CAR003 aptamer was re-synthesized using AKTA OligoPilot 100 Synthesizer (GE Healthcare Life Sciences Corp., Piscataway, NJ) with a 3'Biotin and final detritylation. The product was purified with anion exchange chromatography by FPLC. Several fractions after FPLC were combined as shown as the indicated Pools 1-3 in **FIG. 7C.** The figure comprises an FPLC chromatogram with all product and fra"ctions assigned in pools after checking quality on gel. **FIG. 7D** illustrates a SYBR GOLD stained gel with different FPLC fractions of CAR003 aptamer after synthesis. Different fractions were combined in pools based on amount of unfinished chains in order high to low (pool 1 - pool 3). The pools 1-3 correspond to those indicated in **FIG. 7C.**

CAR003 aptamer characterization. Purified CAR003 aptamer was tested for binding to recombinant EPCAM protein with a polyhistidine tag ("His tagged") using the following internally developed assay. Anti-His tag conjugated beads were mixed with EPCAM-His tagged protein. The aptamer to be tested was labeled with streptavidin-phycoerythrin (SA-PE). The EpCAM-beads and SA-PE labeled aptamers were mixed. Binding was determined as median flourescent value in a bead assay as described herein. MFI values **(FIG. 7E-F)** increase with increased binding of the SA-PE labeled aptamer to the recombinant EpCAM. **FIG. 7E-F** illustrate binding of CAR003 to EPCAM protein in 25 mM HEPES with PBS-BN (PBS, 1% BSA, 0.05% Azide, pH 7.4) **(FIG. 7E)** or in 25 mM HEPES with 1 mM $MgCl_2$ **(FIG. 7F)**. EPCAM aptamer Aptamer 4 (see above) was used for comparison. As shown in the figures, CAR003 pool 3 more efficiently binds its target in the presence of $MgCl_2$ **(FIG. 7F)** than in the presence of BSA **(FIG. 7E)**.

To understand its performance further, CAR003 binding was tested in the presence of both BSA and $MgCl_2$ in various buffers. **FIG. 7G** illustrates CAR003 binding to EpCAM in the indicated salts with and without addition of bovine serum albumin (BSA). Again, CAR003 binding to EpCAM is more efficient when BSA is not present. Additionally, 150 mM NaCl was tested but did not appear to improve CAR003 performance over $MgCl_2$.

Another factor which might influence performance of aptamer is denaturing with different salt compositions. **FIG. 7H** illustrates the effect of denaturing on CAR003 binding to EPCAM protein. As seen from the chart, denaturing of the aptamer has a postive effect on CAR003 binding to EpCAM similar as the effect on CAR003 from $MgCl_2$. However, denaturing in the presence of $MgCl_2$ may not synergistically improve binding of CAR003 to EpCAM. Interestingly, CAR003 appeared more stable compared to control Aptamer 4 in the conditions tested.

CAR003 affinity to EpCAM in the bead assay environment was assessed in the same assay as above with aptamer titrated across a constant input of antigen. **FIG. 7I** illustrates titration of aptamers against EPCAM recombinant protein (constant input 5 $\mu$g). Under the conditions tested, Aptamer 4 had a higher affinity to EPCAM protein compared to CAR003 as suggested from saturation level starting at 5 $\mu$g of aptamer input.

In order to evaluate specificity of CAR003, it was tested using Western Blot against EPCAM recombinant protein, and controls comprising bovine serum albumin (BSA) and human serum albumin (HSA). **FIG. 7J** illustrates a Western blot with CAR003 aptamer versus EPCAM his-tagged protein, BSA, and HSA (5 $\mu$g each). The gel was blocked 0.5% F127 and probed with ~50 $\mu$g/ml CAR003 biotinylated aptamer, fraction 3. The blot was visualized with NeutrAvidin-HRP followed by SuperSignal West Femto Chemiluminescent Substrate. The Western blot probed with CAR003 aptamer showed a clear preference of the aptamer to EPCAM protein over the albumins.

CAR003 test with plasma samples. Plasma samples from five prostate cancer and five normal subjects were tested with CAR003 to detect microvesicles using bead-conjugated proteins to capture the microvesicles and SA-PE labeled aptamer to detect the vesicles. SA-PE labeled Aptamer 4 detector was used as control. Fold changes of Cancer over Normal are shown in **Table 7.** The fold changes are shown without normalization ("Raw") or with normalization to a negative control. The vesicles were captured with bead conjugated antibodies to SSX4, PBP, SPDEF, EPCAM, KLK2 and SSX2 as indicated.

**Table 7: CAR003 to detect microvesicles**

| | | SSX4 | PBP | SPDEF | EPCAM | KLK2 | SSX2 |
|---|---|---|---|---|---|---|---|
| Raw | Standard protocol | 0.87 | 0.39 | 0.71 | 0.63 | 0.93 | 0.87 |
| | Incubation in presence of 1mM MgCl2 and absence of PBS-BN | 0.77 | 0.39 | 0.69 | 0.6 | 0.91 | 0.81 |
| | Aptamer 4 control (standard protocol) | 0.78 | 0.67 | 0.81 | 0.72 | 1.19 | 0.79 |
| Normalized to Negative control | Standard protocol | 1.49 | 0.84 | 1.13 | 1.17 | 1.5 | 1.38 |
| | Incubation in presence of 1mM MgCl2 and absence of PBS-BN | 1.27 | 0.83 | 1.08 | 1.1 | 1.46 | 1.29 |
| | Aptamer 4 control (standard protocol) | 1.18 | 0.96 | 1.11 | 1.04 | 1.8 | 1.1 |

Under the conditions tested, the samples detected with CAR003 had lower MFI values as compared to detection with Aptamer 4, whereas CAR003 had a better signal-to-noise ratio and showed better separation between cancer and normal samples with SSX4, SPDEF, EPCAM and SSX2 capturing markers.

Control Aptamer

The characteristics of the aptamers (size, stability, binding affinity and specificity, etc) can be compared against control aptamers specific to EpCAM or other targets. For example, the aptamers are compared to the anti-VEGF aptamer 5' biotin-CA ATT GGG CCC GTC CGT ATG GTG GGT (SEQ ID NO. 22) as described in Kaur and Yung, 2012.

References:

1. Müller, J., et al. "Selection of high affinity DNA-aptamer for activated protein C using capillary electrophoresis." Research in Pharmaceutical Sciences 7.5 (2012): S987.
2. Cerchia, L., and V. de Franciscis. "Nucleic Acid Aptamers Against Protein Kinases." Current medicinal chemistry 18.27 (2011): 4152-4158.
3. Wu, Jie, et al. "Identification, Characterization and Application of a G-Quadruplex Structured DNA Aptamer against Cancer Biomarker Protein Anterior Gradient Homolog 2." PloS ONE 7.9 (2012): e46393
4. Mitkevich, Olga V., et al. "DNA aptamers detecting generic amyloid epitopes." Prion 6.4 (2012): 400-406.
5. Kaur H, Yung L-YL (2012) Probing High Affinity Sequences of DNA Aptamer against VEGF165. PLoS ONE 7(2): e31196. doi:10.1371/journal.pone.0031196.

**Example 9: Aptamer Target Identification**

In this Example, aptamers conjugated to microspheres are used to assist in determining the target of two aptamers identified by library screening methods as described above. The general approach is shown in **FIG. 9.** The approach is used to verify the targets of CAR003, an aptamer identified by library screening to recognize EpCAM. See decription above for CAR003. In this approach, the sequence of CAR003 is randomly rearranged before linkage to the microspheres. The microspheres are used as controls to bind to targets that are similar but not identical to the intended target molecule.

The protocol used is as follows:

1) The candidate aptamers (here, CAR003) and negative control aptamers (here, randomly arranged CAR003) are synthesized with modifications to allow capture (here, the aptamers are biotinylated) and crosslinking (here, using the Sulfo-SBED Biotin Label Transfer Reagent and Kit, Catalog Number 33073 from Thermo Fisher Scientific Inc., Rockford, IL, to allow photocrosslinking).

2) Each of the aptamers is individually mixed with microvesicles having the target of interest (here, BrCa cell line microvesicles).

3) After incubation to allow the aptamers to bind target, ultraviolet light is applied to the mixtures to trigger crosslinking of the aptamers with the microvesicle targets.

4) The microvesicles are lysed, thereby releasing the crosslinked aptamer-target complex into solution.

5) The crosslinked aptamer-target complexes are captured from solution using a streptavidin coated substrate.

6) The crosslinked aptamer-target complexes for each aptamer are run individually on SDS-PAGE gel electrophoresis. The captured protein targets are visualized with Coomasie Blue staining.

7) The crosslinking and binding steps may be promiscuous so that multiple bands including the intended target but also random proteins will appear on each of the gels. The intended target will be found in a band that appears on the gel with the candidate aptamer (here, CAR003) but not the related negative control aptamers (here, randomly arranged CAR003). The bands corresponding to the target are excised from the gel.

8) Mass spectrometry (MS) is used to identify the aptamer target from the excised bands.

## Example 10: Aptamers to Breast Cancer (BrCa) derived microvesicles

In this Example, an aptamer library is screened to identify aptamers that distinguish between microvesicles circulating in the blood of breast cancer patients and microvesicles circulating in the blood of healthy, control individuals (i.e., without breast cancer).

Microvesicles were isolated from plasma of a pool of 60 breast cancer patients (BrCa+). Microvesicles were also isolated from pool of 60 non-cancer samples (BrCa-). Microvesicles were isolated from the plasma using ultracentrifugation (120,000 x g). Microvesicles were in the pellet from the ultracentrifugation. The supernatant from the ultracentrifugation was saved to use as a control. The microvesicles from both sample types were conjugated to MagPlex beads (Luminex Corp, Austin TX). Optionally, the isolated microvesicles are incubated with anti-HSA/IgG/Fibrinogen beads to remove these highly abundant blood proteins. However, the conjugation step can be optimized to favor conjugation of the microvesicles such that removal of highly abundant proteins is less of an issue.

The aptamer library used consisted of a 2'F SUL1 RNA aptamer library. The sequence is 5'-GGGAGGACGAUGCGG-N40-CAGACGACUCGCUGAGGAUCCGAGA-3' (SEQ ID NO. 23). The aptamer library consists of three sections: Forward primer - 15 nucleotides, variable region - 40 nucleotides; reverse primer - 25 nucleotides. All pyrimidines (C and U) were 2'Fluoro modified.

The aptamer library was incubated with either the cancer or control microvesicle-conjugated beads. Thirteen rounds of positive selection for aptamers that bind the microvesicles were performed in parallel for both types of samples. See **FIG. 11A "Selection A"** for selection schemes. See **Example 11** below for detailed protocol of the positive selection steps. Negative selection was not performed.

The aptamers that were retained from the above positive selection were sequenced using Next Generation sequencing technology consisting of Ion Torrent NGS (Life Technologies, Inc., Carlsbad, CA). The MiSeq system may be used also (Illumina, Inc., San Diego, CA). The sequences are compared to identify aptamers that are found in the cancer samples and not the control samples, and vice versa. Such aptamers provide candidates that can be used to distinguish between BrCa and non-BrCa samples.

A number of representative sequences obtained from these procedures are shown in **Table 8.** The sequences in the table were identified in the aptamer pools from selection against BrCa microvesicles but were not in the aptamer pools selected against non-cancer samples. In **Table 8,** the sequences are shown 5' to 3' from left to right, wherein each complete sequence consists of the indicated 5' leader sequence followed by the indicated Variable Sequence followed by the indicated 3' tail sequence. Each sequence is derived from a library having a leader and tail (see description above) with a variable sequence between. It is understood that the nucleotide sequences that are disclosed in **Table 8** can also be modified to the extent that resulting modifications result in an aptamer having about 50, 55, 60, 65, 70, 75, 80, 85, 90, 95, 96, 97, 98, and 99 percent homology to the disclosed sequence and retain the functionality of binding to microvesicle antigens or functional fragments thereof.

**Table 8: BrCa microvesicle aptamer candidate sequences**

| ID | SEQ ID NO. | 5'-Leader | Variable Sequence | Tail-3' |
|---|---|---|---|---|
| BRCA_APT1_RNA | 8 | GGGAGGACGAUGCGG | CGCGUCUUCCCCGCAUUGCCGCAAUUGCCAUACAUUAAUA | CAGACGACUCGCUGAGGAUCCGAGA |
| BRCA_APT2_RNA | 10 | GGGAGGACGAUGCGG | GUCCGGAACGCCUCGAUCCUCGCAUAAUAUGAUACGUCUG | CAGACGACUCGCUGAGGAUCCGAGA |
| BRCA_APT3_RNA | 12 | GGGAGGACGAUGCGG | GUCCAUGGUACGCCUCGAUUCCGCCCAUACAUGCAUGUAA | CAGACGACUCGCUGAGGAUCCGAGA |
| BRCA_APT4_RNA | 14 | GGGAGGACGAUGCGG | CACUAUCCGUUUGUCCGUCCUCUUGUGGUAUUGCGCAUGC | CAGACGACUCGCUGAGGAUCCGAGA |
| BRCA_APT5_RNA | 16 | GGGAGGACGAUGCGG | UCUUCCAUCUGGUCGCGAUACAGAAUACGAUUAACAUAAA | CAGACGACUCGCUGAGGAUCCGAGA |
| BRCA_APT6_RNA | 18 | GGGAGGACGAUGCGG | GAUCACGCUGCCCUUUGUUUAAGGCCUUUAUACAAACGCA | CAGACGACUCGCUGAGGAUCCGAGA |
| BRCA_APT7_RNA | 20 | GGGAGGACGAUGCGG | UAUUCGCCAGUCACAUCAACUAUGAUGACGCUUGACUGGA | CAGACGACUCGCUGAGGAUCCGAGA |

Each sequence in **Table 8** is synthesized in two variants for further investigation: 5' biotinylated and 3' biotinylated. This provides aptamer variants that can be captured at the 5' end or the 3' end as desired. The aptamers are further synthesized with each pyrimidine (C and U) 2'Fluoro modified.

The DNA sequence corresponding to each RNA sequence in **Table 8** is provided in the sequence listing, where the DNA sequence directly follows its corresponding RNA sequence. For example SEQ ID NO. 9 is the DNA sequence corresponding to RNA sequence SEQ ID NO. 8, etc. The DNA forms of the aptamers are synthesized for further characterization as well.

The aptamers above were identified using positive selection for aptamers which recognize BrCa and non-BrCa microvesicles conjugated to microspheres.

### Example 11: Aptamer library selection protocol

This Example provides the protocol for SUL1 RNA library selection performed in the Example above. The protocol can be followed for other aptamer libraries and sample input as desired.

### Preparation

The working space is cleaned with 80 % EtOH before working.

Beads are MagPlex beads (Luminex Corp., Austin, TX). Other beads can be substituted as desired.

Buffers/Reagents to Prepare:

- MilliQ water
- 100mM $MgCl_2$
- 5x Transcription Buffer (200mM Tris pH 7.9)
- 1x PBS
- 1x PBS with 3mM $MgCl_2$
- 10x PBS
- Selection buffer (1x PBS with 0.1% BSA and 3mM $MgCl_2$)

Before starting with selection, remove the bead storage buffer, and wash beads with 1x PBS w/ 3mM $MgCl_2$ 1 times (200uL total in all 4 tubes). 200,000 beads per selection are used.

### Binding 2'F SUL1 RNA pool to microvesicle coated magnetic beads

Abbreviations: TK- Transcription; NTC- No template control.

Steps:

1. **1st Round:** Mix 1nmol purified 2'F SUL1 RNA with 20 $\mu$l of resuspended beads (conjugated with microvesicle). 10uL of 10x PBS +1% BSA, 3$\mu$l 100 mM $MgCl_2$, and 47uL $H_2O$. This gives a final concentration of 1x PBS, 0.1% BSA, 3mM $MgCl_2$.

1.1 The addition of $MgCl_2$ in this step gives a concentration of 3mM $MgCl_2$. This is the binding concentration for the entire process.

1.2 **Following Rounds:** Mix 20 $\mu$l of transcription product (15 mM $MgCl_2$ inside) with 20 $\mu$l of washed microvesicle coated beads, plus 9uL 10x PBS with 1% BSA, 51uL $H_2O$. No additional $MgCl_2$ is needed because the $MgCl_2$ in the diluted transcription product (TK) provides a final concentration of 3mM $MgCl_2$.

2. Incubate for 30 min at 37 °C, shake at 1000rpm, and pipet mix every 10 minutes.

3. Wash the beads:

3.1 **One washing cycle comprises:**

3.1.1 Remove the beads from the magnet
3.1.2 Resuspend beads in 100$\mu$l 1x PBS +3mM $MgCl_2$ off the magnet.
3.1.3 Incubate sample for 30 seconds off of the magnet.

3.1.4 Place the sample back onto the magnet, and wait until the beads are on the side.

3.1.5 Remove and discard the supernatant.

3.1.6 Resuspend in 100$\mu$l 1x PBS +3 mM MgCl$_2$ + 0.1% BSA off of the magnet.

3.1.7 Incubate sample for 3 minutes off of the magnet.

3.1.8 Place the sample back onto the magnet, and wait until the beads are on the side.

3.1.9 Remove and discard the supernatant

3.2 **1st Round:** Place bead mixture on a magnet and remove the supernatant. Wash once with 100 $\mu$l 1x PBS +3 mM MgCl$_2$ +0.1% BSA (by pipette mixing the beads), and discard buffer.

3.3 **Following Rounds:** Increase the washing steps every second round by one more washing step up to 3 washing steps.

4. Add 55 $\mu$l MilliQ water to the bead sample.

5. Elute the RNA by incubating the bead sample for 5 min at 80 °C

5.1 Check if there is 50 $\mu$l, if not spin the sample down to spin down the condensed water off the top.

5.2 Transfer the supernatant to a new vial. Work quickly to avoid the strands rebinding the beads.

5.2.1 Use 50 $\mu$l eluate for the following RT-PCR and store the rest at -20°C

### RT-PCR of recovered aptamer candidates

Tips

- The rest of the RT-PCR sample and the TK-PCR sample is stored at -20 °C
- RNA can be stored at 4°C for ~1h
- RT-PCR product can be stored overnight at 4°C
- Proceed to the next selection cycle for optimal RNA quality immediately after transcription.
- Avoid vortexing RNA
- Mix on ice
- Use 0.5ml PCR tubes
- Every RT-PCR should have a no-template control (NTC) with water instead of template
- Do not freeze-thaw DTT more than one time

6. Prepare a Master Mix before the first round, check it with 0.5 pmol RNA and store aliquots of 48 $\mu$l at -20°C until usage.

**Table 9: RT-PCR master mix**

| Reagent | Volume ($\mu$l)/reaction | Final concentration |
|---|---|---|
| 5x Colorless GoTaq Flexi Buffer Promega cat# M890A | 20 | 1x |
| 5 x first strand buffer (Invitrogen) lot# 1300427 | 4 | 0.2 x |
| 100 mM DTT | 2 | 2mM |
| 100 $\mu$M SUL1 F primer | 1 | 1 $\mu$M |
| 100 $\mu$M SUL1 R primer | 1 | 1 $\mu$M |
| 100 mM MgCl$_2$ | 1.5 | 1.5 mM |
| 25 mM **(each)** dNTPs | 1.2 | 300 $\mu$M |
| MilliQ water | 17.3 | |
| | | |
| Total | 48 | |

7. Add 50 μl MilliQ water as negative control (NTC) (pipette this first) **or** 50 μl selection eluate. Pipet mix.

8. Incubate at 65 °C for 5 min.

9. After cooling to 4°C, add:

9.1 1 μl Superscript II Reverse Transcriptase (Invitrogen, cat# 18064) (200 U/μl)

9.2 1 μl GoTaqFlexi DNA polymerase (5 units/μl) Promega cat# M8305.

PCR -Program (SARTPCR)

a) 10 min 54 °C
(This step is only for reverse transcriptase, should more rounds be needed, do not repeat step A.)

b) 1 min 95 °C

c) 1 min 60 °C

d) 1 min 72 °C

10. Cycle steps b-d for

10.1 **1st round** b-d 4 cycles. Run 5 μL PCR products on a 4% agarose gel.

10.1.1 Subsequent rounds: The amount of RNA is decreased after the first round, leading to an increase in required PCR-cycles. To determine the number of cycles needed each time, check the band intensity from the agarose gel from the previous round of selection. Use that number of cycles to start the next round of RT-PCR. Note: Always check results on an agarose gel.

10.1.1.1 Agarose gel results: product band should be seen at the target length. The band intensity should be about the same as the 50bp ladder band (if not a little less intense). If the band is not intense enough (barely visible), cycle an appropriate amount more and re-check on an agarose gel.

Transcription

All mixing performed on ice. Prepare transcription Master Mix and store aliquots of 85.7 μl at - 20°C until use.

11. Verify pH of stock 200 mM Tris pH 7.9 before use. A change in pH over time may cause problems with the transcription.

**Table 10: Transcription (TK) Master Mix for SUL1 library**

| Reagent | Volume (μl) for one reaction | Volume (μl) for 20 reactions | Final concentration |
|---|---|---|---|
| 5x Transcription buffer (200 mMTris, pH 7.9) | 20 | 400 | 1x |
| 100 mM DTT | 5 | 100 | 5 mM |
| 100mM ATP | 1 | 20 | 1 mM |
| 100mM GTP | 1 | 20 | 1 mM |
| 100mM 2'F-dUTP | 3 | 60 | 3 mM |
| 100mM 2'F-dCTP | 3 | 60 | 3 mM |
| 100 mM MgCl$_2$ | 15 | 300 | 15 mM |
| MilliQ water | 37 | 740 | |
| | | | |
| Total volume | 85 | 1700 μl | |

12. Add 10 μl RT-PCR product to the mastermix.

13. Add 1 μl RNasin (40 units/μl)

13.1 Promega Recombinant RNasin Ribonuclease Inhibitor cat# N2515/N2511

14. Add 4 μl T7 Y639F mutant polymerase (25U/μl use: 100U total per reaction)

15. Perform the reaction for 30 min at 37 °C

16. Use the transcription-product directly for the next selection round. If the next step is not feasible, freeze transcription product at -20C.

Subsequent Rounds

Repeat the bead incubation, the RT-PCR and transcription as often as needed. Try to have similar band intensity of the RT-PCR product for the sample in all rounds as noted above.

Binding Assay

A binding assay is performed after desired rounds of selection to determine to assess non-specific binding of cancer selected aptamers to control beads (conjugated to supernatant from plasma ultracentrifugation, see above) and likewise for non-cancer control samples. Binding assays can also be performed to assess binding of selected aptamers against the intended target microvesicles.

Cherenkov protocol: Performed using $^{32}$P radioactively labeled aptamer library.

Final concentration of selection buffer: 1x PBS+ 3mM $MgCl_2$ + 0.01% BSA pH 7.4

Wash buffer: 1x PBS + 3mM $MgCl_2$ pH 7.4

1. Remove microvesicle samples from -80°C freezer and thaw.

2. Place beads on magnet (200,000 per sample experiment), remove bead storage buffer.

3. Wash 1x 200μL for 1 minute each with 1x PBS, 3mM $MgCl_2$ buffer. Pool beads to make 200,000 in one tube.

4. Resuspend beads in 70μL of the selection buffer. (10μl of 10x PBS, 1% BSA + 3μL 100mM $MgCl_2$ +57μL $H_2O$ per sample).

5. Add 30μL radioactively labeled RNA aptamer library to their respective sample.

6. Incubate shaking at 1000 rpm at 37°C for 30 min.

7. Place samples on a magnet.

8. Remove and save supernatant.

9. Wash beads with 200μL wash buffer 1x PBS 3mM $MgCl_2$ pH 7.4, incubating off the magnet for 3 minute.

10. Place samples on the magnet, remove and save wash solution.

11. Repeat steps 9, 10.

12. Add 100μL water to the sample, pipette mix.

13. Heat at 80°C for 5 minutes.

14. Place samples on a magnet, remove supernatant, and save.

15. Resuspend beads in 100μL water.

16. Measure radioactivity of every fraction using scintillation counter.

17. Analyze amount of background binding present.

Negative Selection

As desired, a negative selection step is added prior to incubating the aptamer library with the beads conjugated to the target microvesicles (i.e., procedure **"Binding 2'F SUL1 RNA pool to microvesicle coated magnetic beads"** above). The negative selection can be performed using beads conjugated to the supernatant or the input samples (e.g., plasma) after microvesicles are filtered or sedimented from the sample (referred to as "no microvesicle coated beads," "microvesicle depleted samples," or similar). The steps are:

1) Start with aptamer library product from the desired round after transcription as described above. Wash the beads before start: remove storage buffer, wash beads with 200μL wash buffer, then replace buffer as stated below:

2) Negative selection step: Add and pipet mix 20 μl of transcription product (15 mM $MgCl_2$) with freshly washed 'no microvesicle' coated beads with 10μL 10x PBS with 1% BSA, 70μL $H_2O$. No additional $MgCl_2$ is needed because the $MgCl_2$ in the diluted transcription product (TK) provides a final concentration of 3mM $MgCl_2$.

3) Incubate for 30 min at 37 °C, shake at 1000rpm.

4) Remove supernatant and add it to the positive selection beads (directly), which are washed microvesicle coated beads.

Continue with positive selection incubation. See **Binding 2'F SUL1 RNA pool to microvesicle coated magnetic beads** above, starting at step 2. Additional steps through transcription are as detailed above.

**Example 12: Additional Aptamers to Breast Cancer (BrCa) derived microvesicles**

In this Example, an aptamer library is screened to identify aptamers that distinguish between microvesicles circulating in the blood of breast cancer patients and microvesicles circulating in the blood of healthy, control individuals (i.e., without breast cancer). The procedure used the same samples and aptamer library as in **Example 10** above. The procedure in this Example differs in that negative selection was performed before each positive selection starting after the third round of positive selection.

Negative selection serves to remove aptamers that bind soluble/abundant/non-informative and common proteins for cancer and non-cancer proteins. Negative selection include performing negative selection on the aptamer candidates selected against BrCa+ microvesicles as follows: (i) using microbeads conjugated to the supernatant from the BrCa+ plasma ultracentrifugation step (which should not contain microvesicles); (ii) using microbeads conjugated to the supernatant from the BrCa- plasma ultracentrifugation step (which should not contain microvesicles); (iii) using microbeads conjugated to BrCa- microvesicles. Negative selection can also be performed on the aptamer candidates selected against BrCa- microvesicles as follows: (i) using microbeads conjugated to the supernatant from the BrCa+ plasma ultracentrifugation step (which should not contain microvesicles); (ii) using microbeads conjugated to the supernatant from the BrCa- plasma ultracentrifugation step (which should not contain microvesicles); (iii) using microbeads conjugated to BrCa+ microvesicles. Negative selection rounds are performed between rounds of positive selection as described herein.

Microvesicles were isolated from plasma of a pool of 60 breast cancer patients (BrCa+). Microvesicles were also isolated from pool of 60 non-cancer samples (BrCa-). Microvesicles were isolated from the plasma using ultracentrifugation (120,000 x g). Microvesicles were in the pellet from the ultracentrifugation. The supernatant from the ultracentrifugation was saved to use as a control. The microvesicles from both sample types were conjugated to MagPlex beads (Luminex Corp, Austin TX).

The aptamer library used consisted of a 2'F SUL1 RNA aptamer library. The sequence is 5'-GGGAGGACGAUGCGG-N40-CAGACGACUCGCUGAGGAUCCGAGA-3' (SEQ ID NO. 23). The aptamer library consists of three sections: Forward primer - 15 nucleotides, variable region - 40 nucleotides; reverse primer - 25 nucleotides. All pyrimidines (C and U) were 2'Fluoro modified.

The aptamer library was incubated with either the cancer or control microvesicle-conjugated beads. Nine rounds of positive selection for aptamers that bind the microvesicles were performed in parallel for both types of samples. Negative selection against beads conjugated to the input plasma supernatant after ultracentrifugation before positive selection in rounds 4-9. See **FIG. 11A "Selection B"** and **FIGs. 11D-11E** for positive/negative selection schemes. See **Example 11** above for protocol of the positive selection and negative selection steps.

The aptamers that were retained from the above positive selection were sequenced using Next Generation sequencing technology consisting of Ion Torrent NGS (Life Technologies, Inc., Carlsbad, CA). The MiSeq system may be used also (Illumina, Inc., San Diego, CA). The sequences are compared to identify aptamers that are found in the cancer samples and not the control samples, and vice versa. Such aptamers provide candidates that can be used to distinguish between BrCa and non-BrCa samples.

The sequencing data was analyzed according to the following procedure:

Step 1: Sequences were ranked according to frequencies in entire aptamer pool recovered in round 9 after negative selection against beads conjugated to microvesicle-depleted cancer plasma followed by positive selection against beads conjugated to cancer microvesicles.
**Step 2:** Fold changes were calculated between noted sample in Step 1 and: (i) same sample after additional negative selection against microvesicle depleted cancer plasma; (ii) same sample after additional negative selection against non-cancer microvesicles; (iii) same sample after additional negative selection against microvesicles depleted non-cancer plasma.
Step 3: Sequences were ranked based on fold changes calculated in Step 2 to identify sequences which are abundant or deficient in aptamer pool selected for breast cancer derived microvesicles.
Step 4: Possible mutant sequences (e.g., due to PCR or other errors) were removed based on results of consolidation analysis.
Step 5: Sequences were identified with fold changes greater than 3 and minimum frequency 50 in all three variants (i, ii and iii in step 2).

The same selection schemes as in steps 1-5 were performed for aptamers selected against beads conjugated to non-cancer microvesicles.

A number of representative sequences obtained from these procedures are shown in **Table 11.** The sequences in the table were identified in the aptamer pools from selection against BrCa microvesicles but were not in the aptamer pools selected against non-cancer samples. In **Table 11,** the sequences are shown 5' to 3' from left to right, wherein each complete sequence consists of the indicated 5' leader sequence followed by the indicated Variable Sequence followed by the indicated 3' tail sequence. Each sequence is derived from a library having a leader and tail (see description above) with a variable sequence between. It is understood that the nucleotide sequences that are disclosed in **Table 11** can also be modified to the extent that resulting modifications result in an aptamer having about 50, 55, 60, 65, 70, 75, 80, 85, 90, 95, 96, 97, 98, and 99 percent homology to the disclosed sequence and retain the functionality of binding to microvesicle antigens or functional fragments thereof.

**Table 11: BrCa microvesicle aptamer candidate sequences**

| ID (Figure) | SEQ ID NO. | 5'-Leader | Variable Sequence | Tail-3' |
|---|---|---|---|---|
| BCE8 **(FIG. 10Ai)** | 24 | GGGAGGACGAUGCGG | UACCGCCUCAUCAUCG GACACGACGUGUAUCA GUUGGCUG | CAGACGACUCGCUGAG GAUCCGAGA |
| BCE9 **(FIG. 10Aii)** | 25 | GGGAGGACGAUGCGG | GUUCUCGCCUCUGUCC UCAUGGUUCGAACCGG UAUGCAUG | CAGACGACUCGCUGAG GAUCCGAGA |
| BCE10 **(FIG. 10Aiii)** | 26 | GGGAGGACGAUGCGG | GCGGUUUCUUCUCCUG ACUACAUGAGAUUAAU AAACGCGC | CAGACGACUCGCUGAG GAUCCGAGA |
| BCE11 **(FIG. 10Aiv)** | 27 | GGGAGGACGAUGCGG | CCGCCUCGAACACUGA CGUCGUGGAACCUUCG AUUGCUAG | CAGACGACUCGCUGAG GAUCCGAGA |
| BCE12 **(FIG. 10Av)** | 28 | GGGAGGACGAUGCGG | AAUCACAGUAAUUCUG CCCCUCUGAUGAAACC GGUUACUU | CAGACGACUCGCUGAG GAUCCGAGA |
| BCE13 **(FIG. 10Avi)** | 29 | GGGAGGACGAUGCGG | CUUAGUGAUUUCGCCG CCCCUCUGUUUAGUGG CCAUUGGA | CAGACGACUCGCUGAG GAUCCGAGA |
| BCE14 **(FIG. 10Avii)** | 30 | GGGAGGACGAUGCGG | ACACUAUUCCGGUAAG UCAUCGUUUAACCGUU UGUUGCAA | CAGACGACUCGCUGAG GAUCCGAGA |
| BCE15 **(FIG. 10Aviii)** | 31 | GGGAGGACGAUGCGG | UGCGCAACGCCUUGAU UCACUCCUACAGUGUG UCUAUAGA | CAGACGACUCGCUGAG GAUCCGAGA |
| BCE16 **(FIG. 10Aix)** | 32 | GGGAGGACGAUGCGG | AAUGUUAAGCUUACAU ACGCCUGGGUCACUCU UUGUUCUG | CAGACGACUCGCUGAG GAUCCGAGA |
| BCE17 **(FIG. 10Bi)** | 33 | GGGAGGACGAUGCGG | GUAAAUAUUCACGUUG AAUCGCCUUGCUCCUC UUAGUCUG | CAGACGACUCGCUGAG GAUCCGAGA |
| BCE18 **(FIG. 10Bii)** | 34 | GGGAGGACGAUGCGG | CCGCCUCGGAUCGUUC CCAAUGGUGGUACCCC UAUUAAUG | CAGACGACUCGCUGAG GAUCCGAGA |
| BCE19 **(FIG. 10Biii)** | 35 | GGGAGGACGAUGCGG | UGUAGAUCGUUCUUAU CCGCCUCGGUCUUCCC CAGGUUAA | CAGACGACUCGCUGAG GAUCCGAGA |

(continued)

| ID (Figure) | SEQ ID NO. | 5'-Leader | Variable Sequence | Tail-3' |
|---|---|---|---|---|
| BCE20 (FIG. 10Biv) | 36 | GGGAGGACGAUGCGG | AUCGUCGGGCCCCUUU UAUGAAACUUACAUGA AAGCGCAC | CAGACGACUCGCUGAG GAUCCGAGA |
| BCE21 (FIG. 10Bv) | 37 | GGGAGGACGAUGCGG | UAAGAGUGCACAGUAC UGCCUCGAUCCUCCAU GGCUUAAG | CAGACGACUCGCUGAG GAUCCGAGA |
| BCE22 (FIG. 10Bvi) | 38 | GGGAGGACGAUGCGG | GAAUUAGUACUGACGG CCGCCUUGAUCCUCCG UUAGUCUG | CAGACGACUCGCUGAG GAUCCGAGA |
| BCE23 (FIG. 10Bvii) | 39 | GGGAGGACGAUGCGG | GCCCGCCUCCGAAGCC CUCCUAAGUGCACUUU AAACCGCG | CAGACGACUCGCUGAG GAUCCGAGA |
| BCE24 (FIG. 10Bviii) | 40 | GGGAGGACGAUGCGG | CCGCCUGGGAUCACUC UCUACGCGUAUAAAUG CUCUGUCA | CAGACGACUCGCUGAG GAUCCGAGA |
| BCE25 (FIG. 10Bix) | 41 | GGGAGGACGAUGCGG | AGUCUGACCCUGUUAU GGACUACCAUAUCAGA AAGGUACU | CAGACGACUCGCUGAG GAUCCGAGA |
| BCE26 (FIG. 10Ci) | 42 | GGGAGGACGAUGCGG | GGUGAUCCUCCCCCCC GCCUCGAAGAUUUGUG CACAUAUC | CAGACGACUCGCUGAG GAUCCGAGA |
| BCE27 (FIG. 10Cii) | 43 | GGGAGGACGAUGCGG | GCUACCAUCGUCUAGU GAGUCACCCUUAGUUC AUCAAGGC | CAGACGACUCGCUGAG GAUCCGAGA |

Each sequence in **Table 11** is synthesized in two variants for further investigation: 5' biotinylated and 3' biotinylated. This provides aptamer variants that can be captured at the 5' end or the 3' end as desired. The aptamers are further synthesized with each pyrimidine (C and U) 2'Fluoro modified. The aptamers may also be synthesized as the DNA sequence corresponding to each RNA sequence in Table **11**. The aptamer libraries can also be filtered based on predicted secondary sequence, free energy, and other parameters as described herein.

**FIGs. 10A-C** show binding of the aptamers in **Table 11** against microbeads conjugated to various input samples. The aptamer is indicated above each plot and the plot for each aptamer is indicated in the ID column in **Table 11.** See **Table 11.** The input sample is indicated on the X axis from left to right as follows: 1) Cancer Exosome: aptamer binding to microbeads conjugated to microvesicles isolated from plasma samples from breast cancer patients; 2) Cancer Non-exosome: aptamer binding to microbeads conjugated to plasma samples from breast cancer patients after removal of microvesicles by ultracentrifugation; 3) Non-Cancer Exosome: aptamer binding to microbeads conjugated to microvesicles isolated from plasma samples from normal (i.e., non-breast cancer) patients; 4) Non-Cancer Non-Exosome: aptamer binding to microbeads conjugated to plasma samples from breast cancer patients after removal of microvesicles by ultracentrifugation. As shown in **FIGs. 10A-C,** the aptamers were each able to distinguish between the cancer microvesicle samples versus the supernatant control samples and the non-cancer microvesicles. Further, all sequences in **Table 11** were observed as binding more abundantly to cancer derived microvesicles as compared to non-cancer derived microvesicles with the exception of BCE10 and BCE14, which were observed as binding more abundantly to non-cancer derived microvesicles as compared to cancer derived microvesicles.

SEQ ID NOs. 44-10527 comprise a listing of sequences identified in this Example as binding to microbeads conjugated to microvesicles isolated from plasma samples from breast cancer patients preferentially over other samples described in this Example (e.g., beads conjugated to supernatant controls or non-cancer microvesicles). These sequences comprise the conjunction of sequences selected as in Step 2 described above (i.e., selected against microbeads conjugated to microvesicles from breast cancer patients as well as: (i) same after additional negative selection against microvesicle depleted cancer plasma; (ii) same after additional negative selection against non-cancer microvesicles; (iii) same after additional negative selection against microvesicles depleted non-cancer plasma). SEQ ID NOs. 44-10527 indicate the 40 nucleotide variable region from the starting library with 5' and 3' tails as described above (see, e.g., SEQ ID NO. 23 and **Table 11).** These aptamers can be used to detect microvesicles that are derived from breast cancer cells or from non-breast cancer cells. In various applications as described herein, the aptamers can be used for diagnosis, prognosis or theranosis of a cancer.

Based on the comparisons performed in this Example, aptamers that bind different starting input are obtained, including: 1) aptamers that preferentially bind cancer-derived microvesicles over non-cancer derived microvesicles; 2) aptamers that preferentially bind non-cancer-derived microvesicles over cancer derived microvesicles; 3) aptamers that bind both non-cancer-derived microvesicles and cancer derived microvesicles (e.g., "universal" binders); and 4) aptamers that bind plasma components that have been depleted of microvesicles.

The aptamer libraries in this Example are further subjected to four rounds of additional negative and positive selection. See **FIG. 11G "SELEX C"** for positive/negative selection schemes. The positive selection is performed as described in this Example. The negative selection rounds are performed using the beads conjugated to non-cancer microvesicles as negative selection for aptamers obtained by positive selection against beads conjugated to cancer microvesicles. Similarly, the negative selection rounds are performed using the beads conjugated to cancer microvesicles as negative selection for aptamers obtained by positive selection against beads conjugated to non-cancer microvesicles.

## Example 13: Disease Diagnosis

This example illustrates the use of the aptamers to diagnose a proliferative disease.

A suitable quantity of an aptamer that binds a BrCa-derived microvesicle such as identified in **Example 10** or **Example 12** is synthesized via chemical means known in the art. The aptamers are conjugated to a diagnostic agent suitable for detection, such as a fluorescent moiety, using a conjugation method known in the art.

The composition is applied to microvesicles isolated from blood samples taken from a test cohort of patients suffering from a proliferative disease associated with the overexpression of microvesicles, e.g. breast cancer. The composition is likewise applied to microvesicles isolated from blood samples taken from a negative control cohort, not suffering from a proliferative disease.

The use of appropriate detection techniques (e.g., microbead assay or flow cytometry) on the test cohort samples indicates the presence of disease, while the same techniques applied to the control cohort samples indicate the absence of disease.

The results show that the aptamers are useful in diagnosing proliferative diseases.

## Example 14: Therapeutic Aptamers

This example illustrates the use of the aptamers to treat a proliferative disease in a mouse.

A suitable quantity of an aptamer that binds a BrCa-derived microvesicle such as identified in **Example 10** or **Example 12** is synthesized via chemical means known in the art. The aptamers are conjugated to a chemotherapeutic agent, such as Doxil, using a conjugation method known in the art. The conjugate is formulated in an aqueous composition.

The composition is administered intravenously, in one or more doses, to a test cohort of mice suffering from a proliferative disease associated with the overexpression of the microvesicles, e.g. a breast cancer model. A control cohort, not suffering from a proliferative disease is administered the identical composition intravenously, according to a corresponding dosage regimen.

Pathological analysis of tumor samples and/or mouse survival indicates that mortality and/or morbidity are improved in the test cohort over the control cohort.

The results show that the aptamers are useful in treating proliferative diseases.

Useful aptamers can be used to treat breast cancer in other organisms, e.g., a human.

## Example 15: Oligonucleotide - Sequencing Detection Method

This example illustrates the use of an oligonucleotide pool to detect microvesicles that are indicative of a phenotype of interest. The method makes use of a pool of oligonucleotides that has been enriched against a target of interest that is indicative of a phenotype of interest. The method in this Example allows efficient use of a library of oligonucleotides to preferentially recognize a target entity.

For purposes of illustration, the method is described in the Example with a microvesicle target from a bodily fluid sample. One of skill will appreciate that the method can be extended to other types of target entity (e.g., cells, proteins, and various other biological complexes), sample (e.g., tissue, cell culture, biopsy, and other bodily fluids) and other phenotypes (other cancers, other diseases, etc) by enriching an oligonucleotide library against the desired input samples.

General workflow:

1) Obtain sample (plasma, serum, urine or any other biological sample) of patients with unknown medical etymology and pre-treating them accordingly to ensure availability of the target of interest (see below). Where the target of interest is a microvesicle population, the microvesicles can be isolated and optionally tethered to a solid support such as a microbead.
2) Expose pre-treated sample to an oligonucleotide pool carrying certain specificity against target of interest. As described herein, an oligonucleotide pool carrying certain specificity against the target of interest can be enriched using various selection schemes, e.g., using non-cancer microvesicles for negative selection and cancer microvesicles for positive selection as described above. DNA or RNA oligonucleotides can be used as desired.
3) Contact oligonucleotide library with the sample.
4) Elute any oligonucleotides bound to the target.
5) Sequence the eluted oligonucleotides. Next generation sequencing methods can be used.
6) Analyze oligonucleotide profile from the sequencing. A profile of oligonucleotides known to bind the target of interest indicates the presence of the target within the input sample. The profile can be used to characterize the sample, e.g., as cancer or non-cancer.

Protocol varitions:

Various configurations of the assay can be performed. Below are four examples protocols serving the purpose of oligonucleotide-sequencing assay. Samples can be any biological sample.

Protocol 1:

Ultracentrifugation of 1-5 ml bodily fluid samples (e.g., plasma/serum/urine) (120K x g, no sucrose) with two washes of the precipitate to isolate microvesicles.
Measure total protein concentration of recovered sample containing the isolated microvesicles.
Conjugate the isolated microvesicles to magnetic beads (for example MagPlex beads (Luminex Corp. Austin TX)).
Incubate conjugated microvesicles with oligonucleotide pool of interest.
Wash unbound oligonucleotides by retaining beads using magnet.
Elute oligonucleotides bound to the microvesicles.
Amplify and purify the eluted oligonucleotides.
Oligonucleotide sequencing (for example, Next generation methods; Ion Torrent: fusion PCR, emulsion PCR, sequencing).
Assess oligonucleotide profile.

Protocol 2:

This alternate protocol does not include a microvesicle isolation step, microvesicles conjugation to the beads, or separate partitioning step. This may present non-specific binding of the oligonucleotides agains the input sample.
Remove cells/debris from bodily fluid sample and dilute sample with PBS containing $MgCl_2$ (2mM).
Pre-mix sample prepared above with oligonucleotide library.
Ultracentrifugation of oligonucleotide/sample mixture (120K x g, no sucrose). Wash precipitated microvesicles.
Recover precipitate and elute oligonucleotides bound to microvesicles.
Amplify and purify the eluted oligonucleotides.
Oligonucleotide sequencing (for example, Next generation methods; Ion Torrent: fusion PCR, emulsion PCR, sequencing).
Assess oligonucleotide profile.

Protocol 3:

This protocol uses filtration instead of ultracentrifugation and should require less time and sample volume.

Remove cells/debris from bodily fluid sample and dilute it with PBS containing $MgCl_2$ (2mM).

Pre-mix sample prepared above with oligonucleotide library.

Load sample into filter (i.e., 150K or 300K MWCO filter or any other that can eliminate unbound or unwanted oligonucleotides). Centrifuge sample to concentrate. Concentrated sample should contain microvesicles.

Wash concentrate. Variant 1: Dilute concentrate with buffer specified above to the original volume and repeat centrifugation. Variant 2: Dilute concentrate with buffer specified above to the original volume and transfer concentrate to new filter unit and centrifuge. Repeat twice.

Recover concentrate and elute oligonucleotides bound to microvesicles.

Amplify and purify the eluted oligonucleotides.

Oligonucleotide sequencing (for example, Next generation methods; Ion Torrent: fusion PCR, emulsion PCR, sequencing).

Assess oligonucleotide profile.

Protocol 4:

Ultracentrifugation of 1-5 ml bodily fluid sample (120K x g, no sucrose) with 2 washes of the precipitate to isolate microvesicles.

Pre-mix microvesicles with oligonucleotide pool.

Load sample into 300K MWCO filter unite and centrifuge (2000xg). Concentration rate is ~3x.

Wash concentrate. Variant 1: Dilute concentrate with buffer specified above to the original volume and centrifuge. Repeat twice. Variant 2: Dilute concentrate with buffer specified above to the original volume and transfer concentrate to new filter unit and centrifuge. Repeat twice

Recover concentrate and elute oligonucleotides bound to microvesicles.

Amplify and purify the eluted oligonucleotides.

Oligonucleotide sequencing (for example, Next generation methods; Ion Torrent: fusion PCR, emulsion PCR, sequencing).

Assess oligonucleotide profile.

In alterations of the above protocols, polymer precipitation is used to isolate microvesicles from the patient samples. For example, the oligonucleotides are added to the sample and then PEG4000 or PEG8000 at 4% or 8% concentration is used to precipitate and thereby isolate microvesicles. Elution, recovery and sequence analysis continues as above.

**Example 16: Detection of Breast Cancer derived Microvesicle using Oligonucleotide Sequencing**

The method in **Example 15** above is used to detect microvesicles in a bodily fluid sample that are derived from breast cancer cells. The oligonucleotide library comprises a subset of oligonucleotides described in **Example 12** above that preferentially bind to microvesicles from breast cancer patients versus non-cancer individuals. The sequences are listed herein as SEQ ID NOs. 24-10527. The method is used to detect the presence or absence of microvesicles that are indicative of breast cancer.

The test sample comprises plasma samples that are collected from patients scheduled to undergo a breast biopsy. A test sample is a sample to be characterized by the methods of the invention. The method is performed to determine the presence or absence of breast-cancer derived microvesicles.

**Example 17: Aptamer Selection for Microvesicles**

Classic aptamer selection techniques (i.e., SELEX) are typically performed to enrich aptamers for the particular target in "clean" conditions when interference with non-target molecules (competitors) is minimized. However, various applications for the selected aptamers require that the aptamers work to bind their target in diverse environments, e.g., in a biological sample or tissue culture media with an abundance of potentially interfering molecules. In this case, such interfering molecules which were not present during the selection process may interfere with target recognition by aptamers selected in more ideal conditions. This Example provides an aptamer selection process to screen an aptamer library in order to select aptamers highly specific for the targets and also able to perform in a biological sample comprising the potentially interfering molecules.

In this Example, the target comprises a microvesicle such that an aptamer library is screened for members that bind cancer-derived microvesicles as compared to normal (non-cancer controls) microvesicles. One of skill will appreciate that the method can be extended to other types of target entity (e.g., cells, proteins, various other biological complexes). The method can employ various sample types (e.g., tissue, cell culture, biopsy, and various bodily fluids) and can be directed to target molecules that can be used to characterize various phenotypes (various cancers, other diseases, etc) by enriching an aptamer library against the desired input samples. In addition, the cancer and normal samples can be switched to screen for aptamers that preferentially bind the normal samples.

<u>**Variant 1:**</u> Microvesicle based aptamer selection in competition with plasma depleted from microvesicles. Workflow:

-> Microvesicles are isolated from cancer and normal plasma samples using ultracentrifugation. The supernatant comprising microvesicle depleted sample is stored as a negative control sample.

-> Capture/conjugate the isolated microvesicles to magnetic beads.

-> Start the aptamer screening and selection process using microvesicle conjugated beads mixed with supernatant from above (i.e., microvesicle depleted plasma). Supplement supernatant with $MgCl_2$. Aptamer screening and selection for cancer-derived and normal (non-cancer derived) microvesicles can be done in parallel. Each round consists of negative and positive selections as desired.

Since the initial aptamer library is exposed simultaneously to microvesicle targets as well as competitors (soluble proteins) from plasma, aptamers recovered from the microvesicles/beads may be more specific for the targets on microvesicle membrane. Such aptamers may perform effectively in biological samples without requiring extensive purification of the target prior to target binding/detection.

<u>Variant 2:</u> Aptamer selection for cancer-derived microvesicles (e.g., shed from cancer cells) in competition with microvesicles isolated from normal samples. Workflow:

-> Microvesicles are isolated from cancer and normal plasma samples using ultracentrifugation.

-> Capture/conjugate the isolated cancer-derived microvesicles to magnetic beads.

-> Start the aptamer screening and selection process using cancer microvesicles conjugated beads mixed with normal microvesicles (non-conjugated) supplemented with $MgCl_2$. Aptamer screening and selection is performed for the cancer-derived microvesicles only by retaining only the aptamers that are retained with the magnetic beads.

Since aptamer library will be exposed simultaneously to both types of microvesicles (cancer and normal), the method selects aptamers that preferentially bind cancer-derived microvesicles in the presence of non-cancer microvesicles.

<u>Variant 3:</u> Aptamer selection for cancer-derived microvesicles (e.g., shed from cancer cells) in competition with normal plasma (non microvesicles depleted). Workflow:

-> Microvesicles are isolated from cancer plasma samples using ultracentrifugation.

-> Capture/conjugate the isolated cancer-derived microvesicles to magnetic beads.

-> Start the aptamer screening and selection process using cancer microvesicles conjugated beads mixed with normal plasma (non-conjugated) supplemented with $MgCl_2$. Aptamer screening and selection is performed for cancer microvesicles only by retaining only the aptamers that are retained with the magnetic beads.

This approach combines advantages form Variants 1 and 2 above. Each round includes competition with normal plasma. Aptamers should be more selective for target in the presence of interfering plasma proteins and other biological entities.

<u>**Variant 4:**</u> Parallel Cancer/Normal aptamer selection on mixed beads. Workflow:

-> Capture/conjugate cancer-derived microvesicles to magnetic beads

-> Capture/conjugate normal (non-cancer) microvesicles to non-magnetic beads

-> Mix both bead sets and perform the aptamer screening and selection process with the starting aptamer library

-> Separate the magnetic and non-magnetic beads after incubation with aptamer library. Use a magnet to capture the magnetic beads then centrifuge to separate the nonmagnetic beads.

-> Wash the separated magnetic and non-magnetic beads separately.

-> Elute and re-amplify aptamers from both types of beads separately.

-> Round 2: mix amplified aptamer pools from both types of beads and add to the mixed beads

-> Repeat from above

Potential advantages of variant 4 include: (i) using same aliquot of aptamer library to start the aptamer screening and selection process for both cancer and non-cancer microvesicles; (ii) using competition between cancer and non-cancer microvesicles directly in one mixture in each round; (iii) supernatant can be added as additional competitor to increase selection stringency; (iv) parallel enrichment for cancer and non-cancer specific aptamers in competition to result in aptamers identifying only normals or only cancer (since there is a choice to bind either one).

**Example 18: Identifying Aptamers to Breast Cancer (BrCa) derived microvesicles**

In this Example, an aptamer library is screened to identify aptamers that distinguish between microvesicles circulating in the blood of breast cancer patients and microvesicles circulating in the blood of healthy, control individuals (i.e., without breast cancer). The procedure used the similar samples, starting aptamer library, and rounds of positive and negative selection as in **Example 12** above. The procedure in this Example differs in the analysis logic and workflow presented below:

Analysis Logic

An alternative variant of sequencing data analysis versus that in **Example 12** was applied to the data from cancer/non-cancer enriched SUL1 RNA library after 10 rounds of positive and negative selection (R10) probed on Cancer/Non-Cancer exosomes and corresponding microvesicle depleted plasma (see **Example 12).** Specific modifications so analyze sequencing data used in this Example are as follows:

- Frequency of particular aptamer was used rather than raw read counts which may be impacted by the total recovery and re-amplifications. Normalization of read count of particular aptamer to the total count (all aptamer length with correct primers) takes into account the relative abundance of particular aptamer in entire population which may enhance consistency in all steps between elution from the target and sequencing.
- Enrichment of particular aptamer (frequency of aptamer eluted from the target/frequency of same aptamer in input library) used first time as a criterion for aptamer selection, allows quick estimation of the binding ability for all aptamers disregarding the count. Note, all aptamer selection strategies applied before were considering the highest frequency aptamers only.
- Fold change is used to measure the difference in binding to the target vs non-target. Here fold-changes are applied only to the frequencies of aptamers which have demonstrated enrichment.
- RNA vs DNA: since selection was done with the mixture of dsDNA and RNA. Either form of aptamer may be a binder. To address this, four samples were probed with a dsDNA aptamer library (precursor to round 10 selection) and frequency of particular RNA aptamer was divided by frequency of the same aptamer in DNA version.

Analysis workflow

1) Calculate frequency of each aptamer with correct primers in total number of reads.
2) Overlap sequences recovered from binding to cancer microvesicles with input library as well as with those recovered from binding to other three types of samples (cancer plasma supernatant depleted of microvesicles, non-cancer microvesicles, non-cancer supernatant depleted of microvesicles) as well as those recovered from dsDNA binding.
3) Calculate "Enrichment" of particular aptamers in RNA library bound to cancer microvesicles compared to the input library. Consider three threshold classes based difference in aptamer frequency between input and bound populations: 2-5 times, 5-10 times and more than 10 times higher frequency compared to the input.
4) Calculate "Fold changes" of particular aptamers in RNA library bound to cancer microvesicles compared to the same sequences in library bound to cancer plasma depleted of microvesicles, non-cancer microvesicles and non-cancer plasma depleted of microvesicles. Consider fold changes equal or greater than 2.
5) Calculate the difference in frequences of particular RNA aptamers on cancer microvesicles compared to enrichment of corresponding DNA aptamers. Aptamers which have RNA/dsDNA frequences ratios between 0.5 and 1.5 may be binding microvesicles in both versions dsDNA and RNA. Aptamers which have RNA/dsDNA frequences ratios equal or exceeding 2 may bind more efficiently as RNA aptamers.
6) Filter data on "Enrichment" greater than 10, fold changes with three control types of samples (cancer plasma supernatant depleted of microvesicles, non-cancer microvesicles, non-cancer supernatant depleted of microvesicles) are >=2. This provides a first list of aptamers. Repeat filtration on "Enrichment" in the range 5-10 and 2-5 will give the second and third lists of aptamers accordingly.
7) Consider the RNA/dsDNA ratio in the final list of aptamers to identify which aptamer version (i.e., DNA or RNA) may have more efficient binding.

A number of representative sequences obtained from these procedures are shown in **Table 12.** The sequences in the table were identified using the workflow above to selectively bind microvesicles isolated from BrCa plasma as compared to other plasma components or non-cancer plasma. In **Table 12,** the sequences are shown 5' to 3' from left to right, wherein each complete sequence consists of the indicated 5' leader sequence followed by the indicated Variable Sequence followed by the indicated 3' tail sequence. Each sequence is derived from a library having a leader and tail (see description above) with a variable sequence between. It is understood that the nucleotide sequences that are disclosed in **Table 12** can also be modified to the extent that resulting modifications result in an aptamer having about 50, 55, 60, 65, 70, 75, 80, 85, 90, 95, 96, 97, 98, and 99 percent homology to the disclosed sequence and retain the functionality of binding to microvesicle antigens or functional fragments thereof.

**Table 12: BrCa microvesicle aptamer candidate sequences**

| ID | SEQ ID NO. | 5'-Leader | Variable Sequence | Tail-3' |
|---|---|---|---|---|
| BCE28_rna | 10528 | GGGAGGACGAUGCGG | GCGGUUUCUUCUCCU GACUACAUGAGAUUAA UAAACGCGC | CAGACGACUCGCUGAG GAUCCGAGA |
| BCE28_dna | 10529 | GGGAGGACGATGCGG | GCGTTTCTTCTCCT GACTACATGAGATTAA TAAACGCGC | CAGACGACTCGCTGAG GATCCGAGA |
| BCE29_rna | 10530 | GGGAGGACGAUGCGG | CACUAUCCGUUUGUCC CGUCCCUUGUGGGUAU UGCGCAUGC | CAGACGACUCGCUGAG GAUCCGAGA |
| BCE29_dna | 10531 | GGGAGGACGATGCGG | CACTATCCGTTTGTCC CGTCCCTTGTGGGTAT TGCGCATGC | CAGACGACTCGCTGAG GATCCGAGA |
| BCE30_rna | 10532 | GGGAGGACGAUGCGG | CACUAUCCGUUUUGUC CCGUCCCUUGUGGGUA UUGCGCAUGC | CAGACGACUCGCUGAG GAUCCGAGA |
| BCE30_dna | 10533 | GGGAGGACGATGCGG | CACTATCCGTTTTGTC CCGTCCCTTGTGGGTA TTGCGCATGC | CAGACGACTCGCTGAG GATCCGAGA |
| BCE31_rna | 10534 | GGGAGGACGAUGCGG | AAACCGGGGCCCGCCU CAAGUCUAGCUACCUC CAUUGUCGUG | CAGACGACUCGCUGAG GAUCCGAGA |
| BCE31_dna | 10535 | GGGAGGACGATGCGG | AAACCGGGGCCCGCCT CAAGTCTAGCTACCTC CATTGTCGTG | CAGACGACTCGCTGAG GATCCGAGA |
| BCE32_rna | 10536 | GGGAGGACGAUGCGG | AAAACCGGGGCCCCG CCUCAAAGCUAGCUAC CUCCAUUGUCUG | CAGACGACUCGCUGAG GAUCCGAGA |
| BCE32_dna | 10537 | GGGAGGACGATGCGG | AAAACCGGGGCCCCG CCTCAAAGCTAGCTAC CTCCATTGTCTG | CAGACGACTCGCTGAG GATCCGAGA |

(continued)

| ID | SEQ ID NO. | 5'-Leader | Variable Sequence | Tail-3' |
|---|---|---|---|---|
| BCE33_rna | 10538 | GGGAGGACGAUGCGG | CGACCUAUCCGUUUGU CCGUCCUCUUGUGGUA UUGCGCAUGC | CAGACGACUCGCUGAG GAUCCGAGA |
| BCE33_dna | 10539 | GGGAGGACGATGCGG | CGACCTATCCGTTTGT CCGTCCTCTTGTGGTA TTGCGCATGC | CAGACGACTCGCTGAG GATCCGAGA |
| BCE34_rna | 10540 | GGGAGGACGAUGCGG | GUCCAUGGUACGCCUC GAUUUCCGCCCAUCAC AUGCAUUGUAA | CAGACGACUCGCUGAG GAUCCGAGA |
| BCE34_dna | 10541 | GGGAGGACGATGCGG | GTCCATGGTACGCCTC GATTTCCGCCCATCAC ATGCATTGTAA | CAGACGACTCGCTGAG GATCCGAGA |
| BCE35_rna | 10542 | GGGAGGACGAUGCGG | AAACCGGGCCCGCCUC AAGUCUAGCUACCUCC AUUGUCGUG | CAGACGACUCGCUGAG GAUCCGAGA |
| BCE35_dna | 10543 | GGGAGGACGATGCGG | AAACCGGGCCCGCCTC AAGTCTAGCTACCTCC ATTGTCGTG | CAGACGACTCGCTGAG GATCCGAGA |
| BCE36_rna | 10544 | GGGAGGACGAUGCGG | AUGGUCCAUGAGUCUU CUCGGCACUAUCCAGU UGACGCUCA | CAGACGACUCGCUGAG GAUCCGAGA |
| BCE36_dna | 10545 | GGGAGGACGATGCGG | ATGGTCCATGAGTCTT CTCGGCACTATCCAGT TGACGCTCA | CAGACGACTCGCTGAG GATCCGAGA |
| BCE37_rna | 10546 | GGGAGGACGAUGCGG | AAACCGGGCCCGCCU CAAGCUUAGCUACCUC CAUUGUCCUG | CAGACGACUCGCUGAG GAUCCGAGA |
| BCE37_dna | 10547 | GGGAGGACGATGCGG | AAACCGGGCCCGCCT CAAGCTTAGCTACCTC CATTGTCCTG | CAGACGACTCGCTGAG GATCCGAGA |

| ID | SEQ ID NO. | 5'-Leader | Variable Sequence | Tail-3' |
|---|---|---|---|---|
| BCE38_rna | 10548 | GGGAGGACGAUGCGG | GACGGGUUUCCUUCUC CUGACUACAUGAGAUU AAUAAACGCGC | CAGACGACUCGCUGAG GAUCCGAGA |
| BCE38_dna | 10549 | GGGAGGACGATGCGG | GACGGGTTTCCTTCTC CTGACTACATGAGATT AATAAACGCGC | CAGACGACTCGCTGAG GATCCGAGA |
| BCE39_rna | 10550 | GGGAGGACGAUGCGG | CACUAUCCGUUUGUCC CGUCCUCUCGUGGUAU UGCGCAUGC | CAGACGACUCGCUGAG GAUCCGAGA |
| BCE39_dna | 10551 | GGGAGGACGATGCGG | CACTATCCGTTTGTCC CGTCCTCTCGTGGTAT TGCGCATGC | CAGACGACTCGCTGAG GATCCGAGA |
| BCE40_rna | 10552 | GGGAGGACGAUGCGG | CACUAUCCGUUUCGUC CGUCCUCUUGUGGUAU UGCGCAUGC | CAGACGACUCGCUGAG GAUCCGAGA |
| BCE40_dna | 10553 | GGGAGGACGATGCGG | CACTATCCGTTTCGTC CGTCCTCTTGTGGTAT TGCGCATGC | CAGACGACTCGCTGAG GATCCGAGA |
| BCE41_rna | 10554 | GGGAGGACGAUGCGG | CACUAUCGUUUGUCCG UCCUCUUGUGGUAUUG CGCAUGC | CAGACGACUCGCUGAG GAUCCGAGA |
| BCE41_dna | 10555 | GGGAGGACGATGCGG | CACTATCGTTTGTCCG TCCTCTTGTGGTATTG CGCATGC | CAGACGACTCGCTGAG GATCCGAGA |
| BCE42_rna | 10556 | GGGAGGACGAUGCGG | UAUGGGGGCUUUUUAA GCACUUCUCGGUAAAC UUGGGCAGUUAC | CAGACGACUCGCUGAG GAUCCGAGA |
| BCE42_dna | 10557 | GGGAGGACGATGCGG | TATGGGGGCTTTTTAA GCACTTCTCGGTAAAC TTGGGCAGTTAC | CAGACGACTCGCTGAG GATCCGAGA |

The aptamers in **Table 12** were identified as having enrichment compared to the input library of at least 10-fold and at least 5-fold more prevalent in the aptamer pools selected against cancer microvesicles versus non-cancer microvesicles.

Each sequence in **Table 12** is synthesized in two variants for further investigation: 5' biotinylated and 3' biotinylated. This provides aptamer variants that can be captured at the 5' end or the 3' end as desired. The aptamers are further synthesized with each pyrimidine (C and U) 2'Fluoro modified. The DNA sequence corresponding to each RNA sequence in **Table 12** is provided in the table, where the DNA sequence directly follows its corresponding RNA sequence. For example SEQ ID NO. 10529 is the DNA sequence corresponding to RNA sequence SEQ ID NO. 10528, etc. As noted, both forms of the aptamers are synthesized for further characterization.

**Example 19: Aptamer Library Generation**

In this Example, an aptamer library with high diversity is generated using varying concentrations of nucleotides in lieu of the typical equal molar concentrations. See **FIG. 12.** In providing such varying nucleotide concentrations, the resulting random sequence library comprises oligonucleotides having different ratio between A/T and G/C depending on particular concentrations of nucleotides input. Furthermore, the resulting library provides greater chemical and structural diversity of oligonucleotide molecules with the benefit of providing more potential binding partners for a plurality of targets that would be present in a complex input sample (e.g., tissue or tissue components, cells or cell components, extracellular vesicles, etc.). Such highly diverse random libraries are used as input to enrich for oligonucleotides having high affinity for desired targets present in an input sample. Unexpectedly, aptamers exhibiting differential binding to disease versus non-disease samples (e.g., cancer versus non-cancer) are discovered that are not available when utilizing standard equa-molar concentrations of nucleotides.

As described herein, a starting aptamer input library is processed through several rounds of selection to enrich for a subset library that provides a differential readout, such as through sequencing. The starting input library may itself comprise of multiple subset libraries wherein each of the subset libraries is generated using a different concentration of G and C nucleotides, in order to generate nucleic acids with higher or lower final GC content. In this aspect, varying concentrations of nucleotides beyond 25% each (e.g., 12.5% each A/T and 37.5% each G/C, merely as one example), may include increasing/decreasing concentrations of A/T in relation to G/C nucleotides. For example, one or more random sequence libraries are generated, using a G and/or C nucleotide concentration for a particular library that is anywhere from about 5 to about 95 percent (including any single unit of measurement between the range 5 to 95) of the total nucleotide concentration. Exemplary but non-limiting nucleotide concentrations are shown in **Table 13.**

**Table 13: Exemplary nucleotide percentages for aptamer library generation**

| A | T | C | G |
|---|---|---|---|
| 5 | 5 | 45 | 45 |
| 10 | 10 | 40 | 40 |
| 15 | 15 | 35 | 35 |
| 20 | 20 | 30 | 30 |
| 25 | 25 | 25 | 25 |
| 30 | 30 | 20 | 20 |
| 35 | 35 | 15 | 15 |
| 40 | 40 | 10 | 10 |
| 45 | 45 | 5 | 5 |
| 5 | 5 | 5 | 85 |
| 10 | 10 | 10 | 70 |
| 15 | 15 | 15 | 55 |
| 20 | 20 | 20 | 40 |
| 25 | 25 | 25 | 25 |
| 30 | 30 | 30 | 10 |
| 5 | 5 | 85 | 5 |

(continued)

| A | T | C | G |
|---|---|---|---|
| 10 | 10 | 70 | 10 |
| 15 | 15 | 55 | 15 |
| 20 | 20 | 40 | 20 |
| 25 | 25 | 25 | 25 |
| 30 | 30 | 10 | 30 |
| 5 | 85 | 5 | 5 |
| 10 | 70 | 10 | 10 |
| 15 | 55 | 15 | 15 |
| 20 | 40 | 20 | 20 |
| 25 | 25 | 25 | 25 |
| 30 | 10 | 30 | 30 |
| 85 | 5 | 5 | 5 |
| 70 | 10 | 10 | 10 |
| 55 | 15 | 15 | 15 |
| 40 | 20 | 20 | 20 |
| 25 | 25 | 25 | 25 |
| 10 | 30 | 30 | 30 |

A starting input library for selection of one or a group of aptamers may be comprised of at least one (e.g., 1, 2, 3, 4, 5, 6, 7, 8, 9, 10 or more) subset libraries having differing nucleotide content. Each subset library may comprise, for example, $10^{12}$ different oligonucleotides (for 1.7 pmols of 80 bases library). In one non-limiting example, an input library is comprised of three subset libraries, each having GC content of 25%, 50% and 75%, respectively. Based on Applicant's teachings herein, it will be apparent to one of ordinary skill in the art that both the number of subset libraries and GC content of each subset library can be varied as disclosed herein to provide different input libraries. A subset library used in an input library can be generated using nucleotides that are less than, equal to or greater than 50% G and/or C.

Therefore, the disclosure provides a method of generating a starting oligonucleotide library with high diversity. Such libraries provide advancement in assessing a disease or condition using an aptamer library as the basis for differentiating between two samples/states. This is clearly distinguishable from conventional techniques of identifying aptamer(s) that are specific for a specific known or unknown target. The nucleotides used may be natural nucleotides, modified nucleotides, use of unnatural nucleotides or combinations thereof. Such species of modified or unnatural nucleotides are known.

**Example 20: Oligonucleotide Probe Library**

In this Example, the oligonucleotide detection method (see, e.g., **Example 15** above) is applied to distinguish breast cancer patient samples from normal (i.e., non-breast cancer as confirmed by negative biopsy) biological samples. The oligonucleotide library used to detect and/or distinguish the samples may be referred to herein as an "oligonucleotide probe library" or "oligonucleotide profiling library." A randomized oligonucleotide library was trained against microvesicles derived from the blood of breast cancer patients and normal (non-cancer) individuals. The approach described above with respect to **FIG. 13D** was applied. See **FIG. 14A.**

The naive input oligonucleotide library consisted of a 3'Phosphorylated ssDNA oligonucleotide library with the following sequence: 5'-ATCCAGAGTGACGCAGCA-30n-TGGACACGGTGGCTTAGT-3' (SEQ ID NO. 10558), where n comprises A, T, C, G, U or other nucleotide. As shown, each oligonucleotide has 18 nucleotide primer regions with a central 30 nucleotide variable region. The only modification is the single 3' phosphorylated nucleotide. Positive selection was performed wherein plasma samples from the breast cancer patients were contacted with the untrained input library. Microvesicles in the samples were collected by ultracentrifugation and oligos that were retained with the microvesicles were eluted and collected. These steps were repeated using the retained oligos as the input for the next round of selection. The partially trained library was then subjected to negative selection by contacting the library with the non-cancer patient plasma. Microvesicles in the samples were collected by ultracentrifugation and oligos that bound the non-cancer microvesicles were discarded and those found in the ultracentrifugation supernatant (i.e., those that did not bind the non-cancer microvesicles) were retained. Postive selection against microvesicles from cancer patients was performed again with repetition. The trained library was processed to remove PCR and other non-specific artifacts. The remaining oligos comprised the trained oligonucleotide probe library that preferentially recognized cancer-derived microvesicles. This same process was repeated in reverse to identify a trained oligonucleotide probe library that preferentially recognized non-cancer-derived microvesicles (i.e., positive selection against non-cancer samples and negative selection against cancer samples). A conjoint library was created by mixing half of each library by weight. Using this process and determining the library composition via next generation sequencing (Illumina HiSeq platform), an oligonucleotide pool of 1,395,706 unique sequences was created. Of these, 843,729 sequences were detected a single time. The 500,000 most commonly occurring sequences in the oligonucleotide pool ranked in order of most to least occurrence are incorporated herein in SEQ ID NOs. 10559-510558.

The protocol to test the samples with the oligonucleotide probe library is described below in **Example 21.** Briefly, plasma samples were contacted with the trained oligonucleotide probe library. Microvesicles were isolated from contacted plasma samples using ultracentrifugation. Oligos that were retained with the microvesicles were eluted and sequenced using next generation sequencing methodology. The frequency of retained oligos that bound the microvesicles was determined.

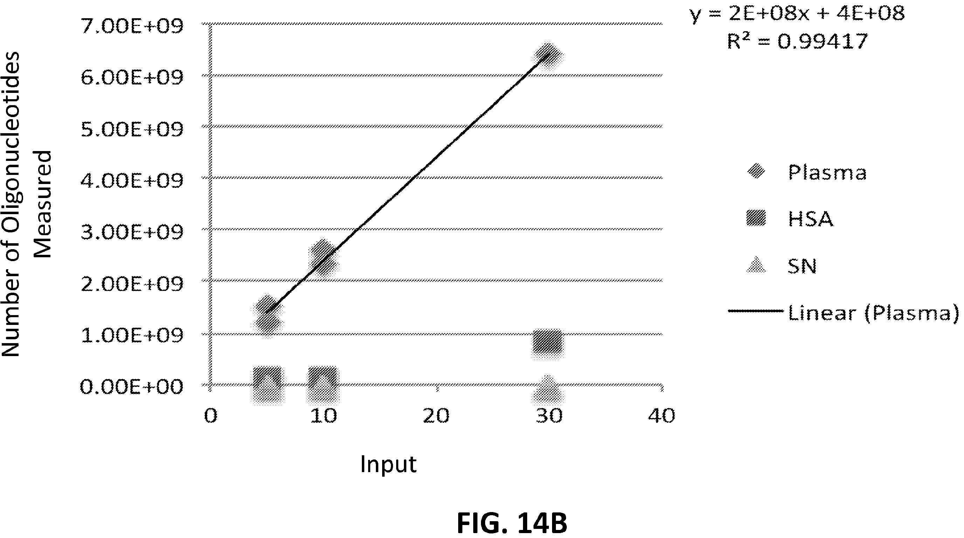

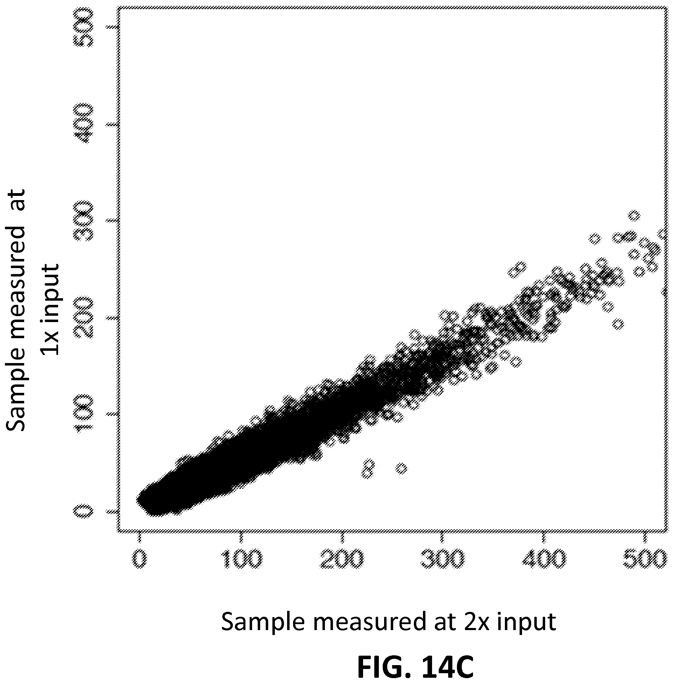

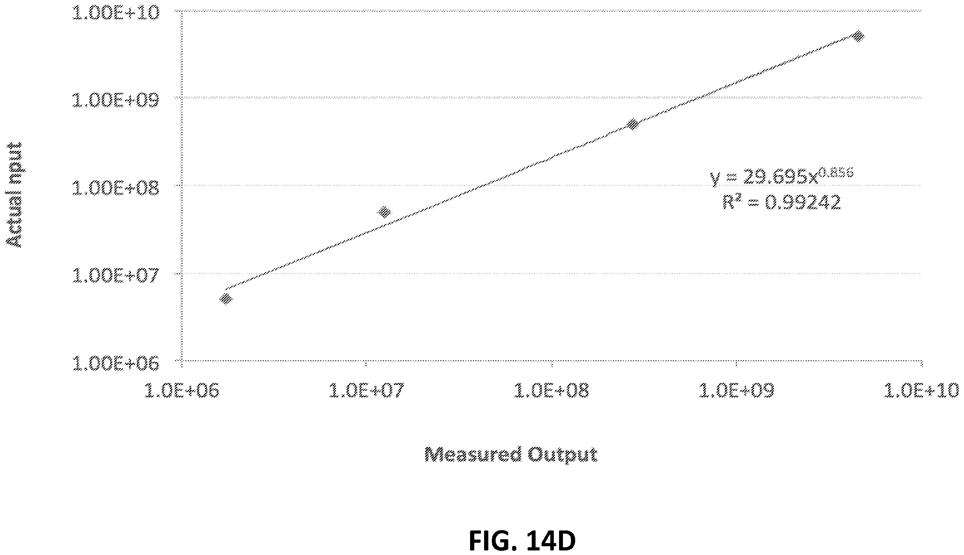

The oligonucleotide probe library developed using the above approach showed good recovery, signal to noise, and reproducibility. **FIG. 14B** shows a plot of the number of oligonucleotides that bound to the input sample (i.e., plasma microvesicles). A linear relationship ($r^2 = 0.99$) was observed between the input sample (labeled "Plasma") and number of bound oligonucleotides. In contrast, few if any oligonucleotides bound to control samples comprising human serum albumin (labeled "HSA") or the plasma supernatant without microvesicles (labeled "SN"). **FIG. 14C** shows that the method was highly reproducible with multiple sequencing runs displaying high linear correlation. In this Figure, microvesicles from a single sample were probed at a 1x and 2x concentration. A linear relationship was observed with twice as many oligos binding the 2x input. This figure is one of many such examples. Similar linear relationship was observed when comparing different sequencing runs on the same sample or different ultracentrifugation preparation of the same plasma sample. In contrast, a large spread was observed when comparing sequencing profiles between different patient samples. Thus, the differences between patient samples were significantly larger than sources of technical variance showing that this method is capable of measuring differences in the underlying biological system. **FIG. 14D** shows the formation of a standard curve by comparing known actual input versus the measured output. Inclusion of a standard curve with every sample allows for absolute quantification and normalization between samples.

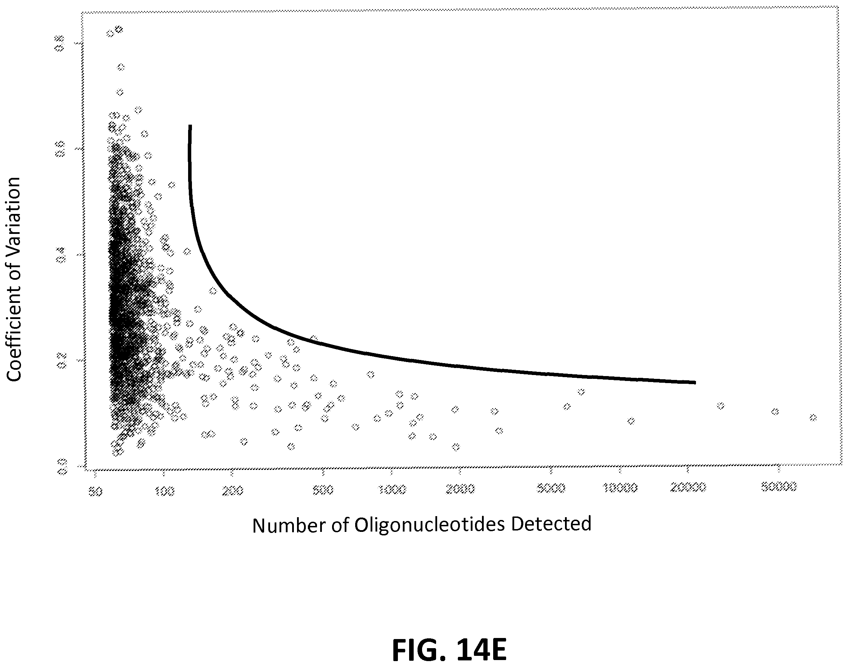

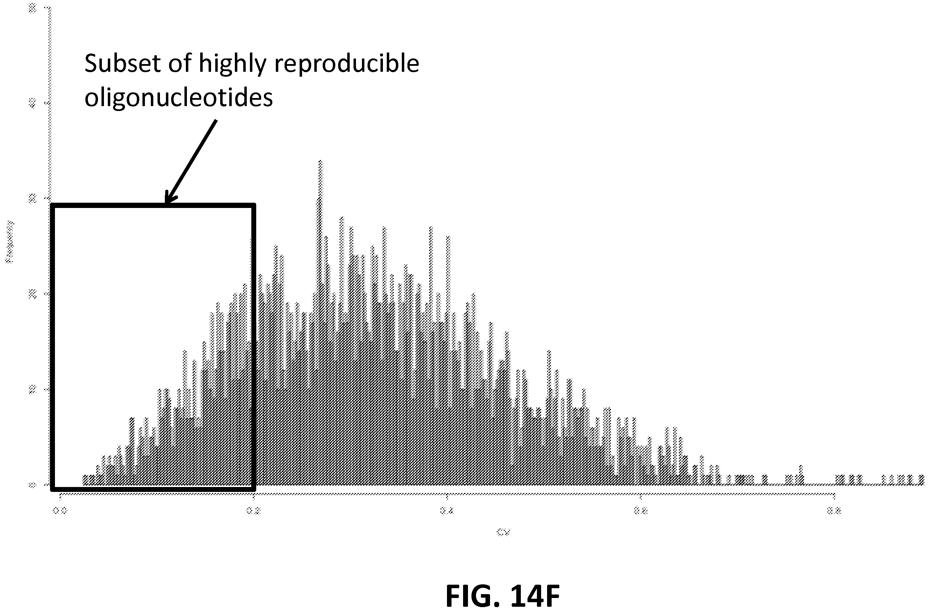

Importantly, we observed that the variance of the method is a function of the sequencing depth and therefore can be controlled by the number of sequencing runs performed. **FIG. 14E** illustrates this point. Results were obtained by sequencing of the probe library that bound the input samples. The figure shows the coefficient of variation (CV) plotted against the number of bound oligonucleotides detected. As seen, the greater the number of oligonucleotides sequenced allows detection of sequences with lower CV. Accordingly, the assay is reproducible as the variance can be controlled by increasing the amount or sequencing data collected. **FIG. 14F** reveals the distribution of high frequency oligonucleotides detected. The aptamers within the box indicate a subset of reproducible oligos (CV < 0.2). These oligos have potential use as individual markers.

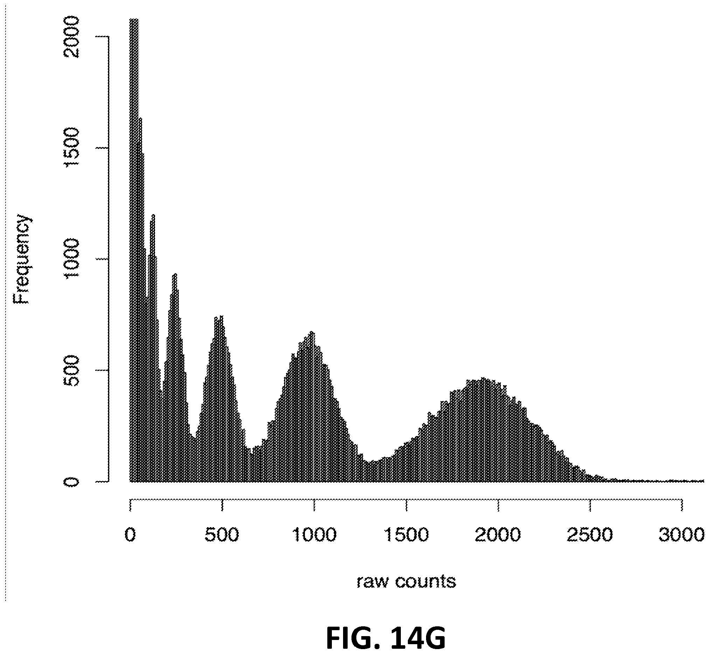

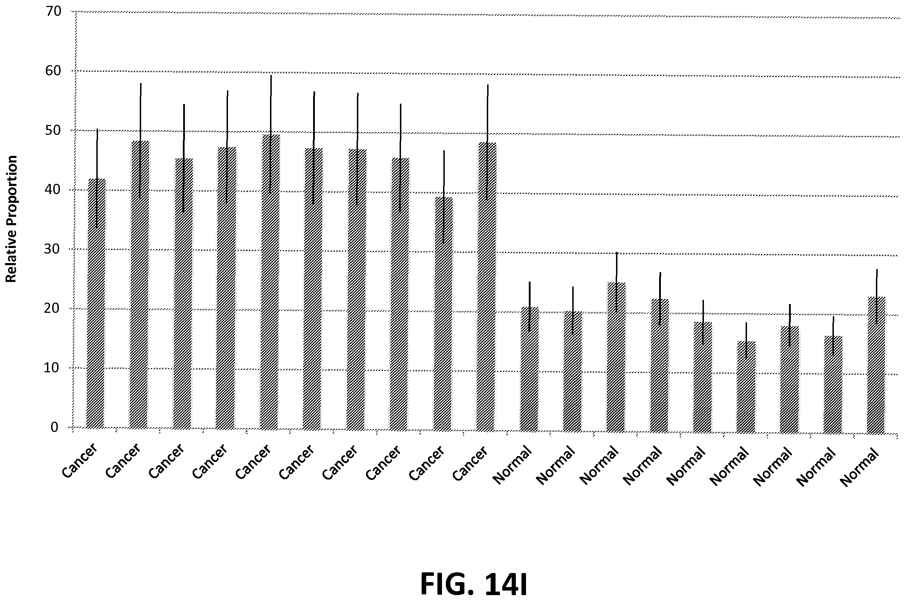

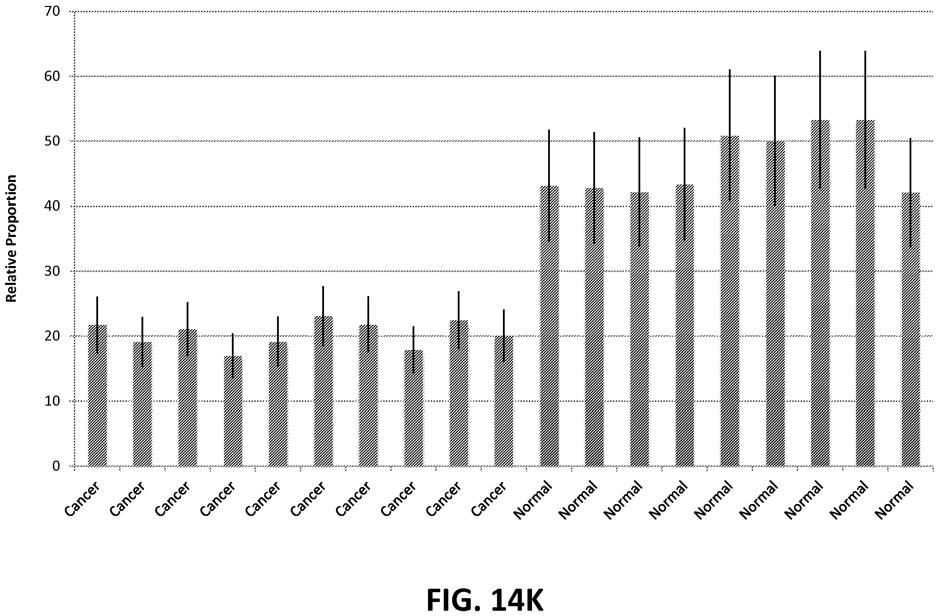

Based on the results in **FIGs. 14B-14F,** the detection of the oligonucleotide probe library trained against breast cancer and normal samples was reproducible with low noise. Individual members of the oligonucleotide probe library were identified that showed the most separation of breast cancer and normal samples. **FIG. 14G** shows a plot of the sequencing profile (frequency vs. count) of the oligonucleotide probe library ies enriched for breast cancer microvesicles in plasma. Multiple sub-populations of markers were observed in the plot. **FIG. 14H** shows plots of four individual oligonucleotides that showed differences between breast cancer patients and biopsy negative controls. These oligonucleotides were identified as preferentially binding the cancer samples. Multiple individual oligonucleotides can also be combined. **FIG. 14I** shows plots of the combination of 20 individual oligonucleotides that preferentially binding the cancer samples. As seen in the plot, complete separation was observed between the breast cancer patients and biopsy negative controls. The sequences of these 20 individual oligonucleotides are shown in **Table 14.** Similarly, **FIG. 14J** shows results obtained by four representative individual oligonucleotides that preferentially bound the normal samples, and **FIG. 14K** shows plots of the combination of 20 individual oligonucleotides that preferentially bound the normals. The sequences of these 20 individual oligonucleotides are shown in **Table 14.** As in **FIG. 14I,** complete separation was observed between the breast cancer patients and biopsy negative controls.

**Table 14: Use of oligonucleotide probe library to detect breast cancer**

| Preferentially detect | Sequences (5'->3') | SEQ ID NO. |
|---|---|---|
| Breast Cancer microvesicles | CGGGGCTGTTTGTCGGGACCTGGGTCCCTG | 510559 |
| | GTCTAGCACGCCCACCTCCAGGCAGGCTGA | 510560 |
| | CAGTGACATGTAAATTCAATGCCCTGCATA | 510561 |
| | TGATAATCGTTTGTTTGTAGCAAAGCGCAG | 510562 |
| | GGGGAACACGCATATTGAGTACAAATGATG | 510563 |
| | TCGTTGTAGGATATCGGCCCGCCGTACACA | 510564 |
| | GCCAATCCGCAGCATCGGAGGGCTAAGGAA | 510565 |
| | CTTGAGCGCTGGTCCCCGGTTCAGCATGAT | 510566 |
| | GGTACGTTTGATTTAACTACGGGTTATACT | 510567 |
| | AGAGTATATTCGGGACCAATAGTACGATGG | 510568 |
| | CGTACATCGGATTGCTCTAGAATGCAGAAT | 510569 |
| | GATTGTAACGCCAACCTTATATCCATTCAG | 510570 |
| | TAAAGCATGCTACAATTCACGCAGTAGTAA | 510571 |
| | TCCGATCGGTTGGTCACCGAGTGATTTCGA | 510572 |
| | GAAACGATCGCCTGTACAATTAGGTTAAGG | 510573 |
| | ATGCAGTTCTATACAATGCCAGTGGGATAG | 510574 |
| | CAAAAATGTCACGAGAAAAGGAAAGGTTGG | 510575 |
| | TAACCAGTGGGGGGATCTAAAAGATAAACG | 510576 |
| | GAATACCCGGCAGGGATGGTTGATACTCAG | 510577 |
| | ATCGGACGGAAGTAGCAGTTCACGCGTAAC | 510578 |
| Non-Breast Cancer microvesicles | TCCTGAATTGCCTCAGTCGAAAGGTCAGTC | 510579 |
| | AACTTAGCGCGAGTTGAAAAGACGGTTTAC | 510580 |
| | AATTGATACGATGAAATTCGGTCGGTTGCA | 510581 |

(continued)

| Preferentially detect | Sequences (5'->3') | SEQ ID NO. |
|---|---|---|
| | GGCGGGCAAAGGGGGTAATGAGTCCGAAGG | 510582 |
| | TAGCTACGTACGCATAGGATTTGAGCCGGT | 510583 |
| | TAGGGATGTGACCAAGGATTATCCAGGTGG | 510584 |
| | TCCGAAATCTCCTCCGCAATGGACGCGTCG | 510585 |
| | GGGCCTACGCCCTGTGACGATGCTCCCAAG | 510586 |
| | GGAGGGGTGTTAGTTCAGTGTTGAGAGTTT | 510587 |
| | TTGTAATAATGGTCCGCGGTGGAGTTTCGA | 510588 |
| | GGGTCGGTAAACCAGGGTGGTATCTGTAAA | 510589 |
| | GATGTTAAACTACTTCGTAAGAGTTGGTGA | 510590 |
| | AGCATGAGGGAGGATGGGTCGTGGGGGGCC | 510591 |
| | GACAGCTCACAATGAGACAGTATCAGCTAC | 510592 |
| | CATCCTTGAGTGAGATCGTCTGTCCCACTA | 510593 |
| | AGGTTTAGGAGGATGGCGGGAGTAGATTAG | 510594 |
| | AATTACGTGAGAATCCCGGTGACCCTAAGC | 510595 |
| | AACTTGCCTTCCGCAATGACAAATACATGT | 510596 |
| | CATTGTCGAGGCCACGTGCGGATAAATAGT | 510597 |
| | CGGACTCGAGGGGGAGGACATCGGAGGCAA | 510598 |

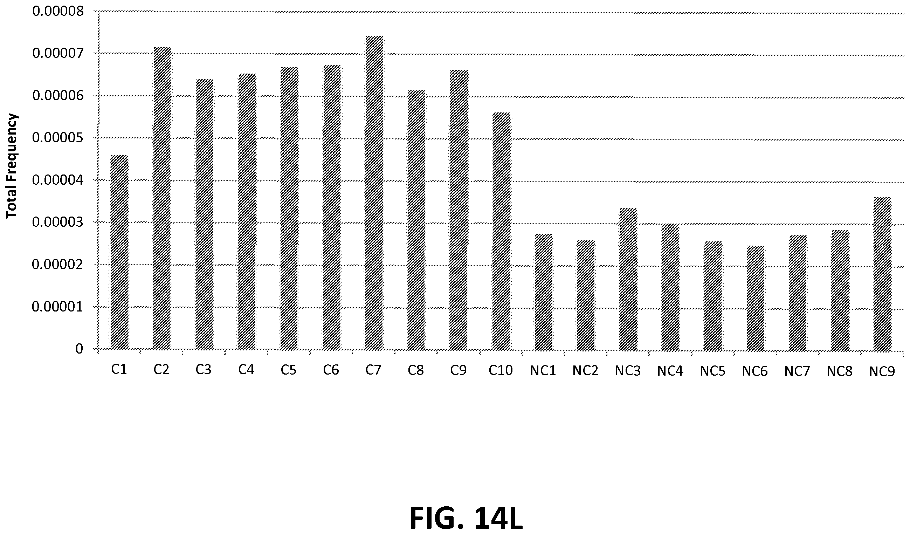

The results of any number of individual oligonucleotides can be combined. For example, **FIG. 14L** shows results obtained by combining 46 individual oligonucleotides that preferentially bound the cancer samples (C1-10) versus normal (NC1-9). These 46 oligonucleotides were selected as showing a fold-change in frequency between cancers and normal of 2.2. As above the observed sensitivity and specificity were both 100% in these samples.

This Example illustrates the development and use of an oligonucleotide probe library to distinguish plasma samples and cancer or non-cancer. As shown above, the method provided 100% separation between the samples. One of skill will appreciate that the method can be applied to distinguish other types of biological entities (e.g., cells, protein complexes) and phenotypes (e.g., different cancers, different diseases).

**Example 21: Oligonucleotide Probe Library for Multiple Indications**

In this Example, an oligonucleotide probe library as described above in **Example 20** was tested for its ability to distinguish between multiple types of cancers and other diseases versus normal (i.e., non-diseased) samples. For this Example, plasma and serum were used as input samples based on sample availability.

The general approach to library enrichment is as follows. The starting naive library was based on SEQ ID NO. 10558 as described above in **Example 20.** This library comprises $\sim 10^{12}$ unique representatives (e.g., single-stranded DNA oligonucleotides, ssDNA). Several positive enrichment (binding) as well as counter-enrichment (subtractive) steps were performed. For enrichment of oligonucleotides that bind to microvesicles of a specific sample population (e. g., breast cancer patients in this Example), the starting library was mixed with a pool of plasma obtained from patient samples and incubated. A centrifugation step is performed to isolate the microvesicles in the sample. Ultracentrifugation or polymer precipitation (e.g., PEG4000, PEG8000) with low speed centrifugation have been successfully used. Oligonucleotides with affinity and specificity to the microvesicles remain bound during the centrifugation whereas unspecific binding is suppressed by the addition of competitors (e.g., salmon sperm DNA, tRNA, S1, CM-D, combinations thereof, or the like). Non-binding oligonucleotides and binders to non-exosomal proteins (such as HSA) are removed when the supernatant is discarded. Bound oligonucleotides are recovered (eluted) from the resuspended microvesicle pellet. For depletion of the library from binders to a second sample population (e. g., healthy control such as non-cancer patients), the recovered oligo pool is mixed with a pool of samples from the second sample population and binders to microvesicles in the control samples are removed by discarding the pellet resulting from centrifugation. In a second positive enrichment, the supernatant (which now comprises oligos which bound the first sample population but not the second sample population) is mixed with isolated microvesicles from another aliquot of the first sample population and binders are recovered representing a single-round enriched library. This scheme may be iterated multiple times to further enrich the library and reduce its complexity. In a final step, the library is amplified by PCR and reverted to ssDNA by lambda exonuclease digestion and cleaned up. The same procedure performed to enrich for binders to the second (control) population as opposed to the first (cancer) population. A combination of the two enriched libraries comprises binders to targets characteristic of both sample populations, e. g., binders to both up- and down-regulated cancer markers. The combination of enriched libraries is then used as the oligonucleotide probing library. The course of the enrichment (copy and species numbers contained in a library) is followed by qPCR and high throughput sequencing.

The enriched oligonucleotide probe library was tested against individual samples as in **Example 20.** The protocol is described below in **Example 22.** Briefly, the plasma or serum samples were contacted with the trained oligonucleotide probe library. Microvesicles were isolated from the contacted samples using ultracentrifugation. Oligos that were retained with the microvesicles were eluted and sequenced using next generation sequencing methodology. The frequency of retained oligos that bound the microvesicles was determined. The method was used to distinguish normal serum or plasma from serum or plasma from a variety of indications as indicated in **Table 16.** Heat maps were created using cluster analysis and are shown for the indications in **FIGs. 15A-AN.** The oligonucleotides were selected as those having $p < 0.1$ between the conditions and normal samples and also as appearing at least 100 times in all samples (as a quality measure). After this filtering, 165 sequences remained, as shown in **Table 15.** In the plots in **FIGs. 15A-AN,** individual oligonucleotides are shown on the Y-axis and samples are shown on the X-axis (normals are indicated as such). The sequences in **Table 15** are the variable regions of the input library without flanking sequencing primer regions. **Table 16** indicates the sequences from **Table 15** that are shown in the corresponding heat maps in **FIGs. 15A-AN** for the various indications.

**Table 15: Filtered selection of oligonucleotide probe library to detect various conditions**

| Sequence (5'->3') | SEQ ID NO. |
|---|---|
| AAAGAATCGCCAACCAACACGACCTGAGAG | 510599 |
| AAAGGCGATTGTATATCCAGGTGCCGCCAA | 510600 |
| AACGTAGATAACCAAAAGAGTGATACAAAG | 510601 |
| ACAACAACGAATACAATGCGTAAGAGTGCA | 510602 |
| ACACACCCGGAAGAGAGAGCGGAAACCGAA | 510603 |
| ACCCAAACAACAACCTTGGAATCCAGCAGA | 510604 |
| ACGACAGTATCTAAGAGAGAACCGAAGTAA | 510605 |
| ACGTCCACCGAAAAAACTCGCCACGGCAGA | 510606 |
| ACTCTAACCAGAGAGATCCAGCCAACCGAA | 510607 |
| AGAAACAATGCCACCCATCCAAACCAATAG | 510608 |
| AGACCACAACTCGACCCTTTCACAGAAGAG | 510609 |
| AGATCCCTAGCAATGAATGAAGTATCCAGG | 510610 |
| AGATCGTTGGTGCGACTTATCAGGGTGAAT | 510611 |
| AGCAAAGGAATCACAGAGTATAGGACCACA | 510612 |
| AGCCTGGCGCCCCGGGGAGGGTGAACCTGT | 510613 |
| AGCGCACCACGAAGACAACCACAATTAGCA | 510614 |
| AGGAAAGAAGAGGAGGTCCGACACACCAGG | 510615 |
| AGGAAGAACCAACAACTGAAACGTCTAGAG | 510616 |
| AGGCGTGAACTACACACAATGAGTGACTAA | 510617 |
| AGGGCACACCAAGATAGCGGTAACGTGAGT | 510618 |
| AGGGCCATCACATACAGCAACGAATTAGAG | 510619 |
| AGGGGCACAGTGAAAAACATGCTAACCAGG | 510620 |
| AGGTGTAGAAGTCAATCTGAGAATCCTAGA | 510621 |

(continued)

| Sequence (5'->3') | SEQ ID NO. |
|---|---|
| ATAACCAGAAGACAGGAAGAACGAGACGAG | 510622 |
| ATGCCCAGATAGCAGAGCACAACGGGAGGA | 510623 |
| ATGCCGAGAGACGCCGATCTGCGTGGTTGG | 510624 |
| ATTGAACAGAAACCACATGCCAATCCAGTG | 510625 |
| ATTGTATATCCCTGTTGGCATTACGCCAT | 510626 |
| ATTTAACGCAGTTTATCCGTTCACCTAACT | 510627 |
| CAAAAGGGTAACCAACAGAAATAGAGGACG | 510628 |
| CAAGTAGACCACACTGACACACCAGCAGAG | 510629 |
| CACACCCCAAGATCGATAAACGAGTAGCCA | 510630 |
| CACTAGAACTTCTCAGAGATCGTCGCCCAA | 510631 |
| CAGGGCGTGTATATGATGGTGTTGTATGGT | 510632 |
| CATAACAAGACTTCATTCTTTCGCTCCATT | 510633 |
| CATGCAATACAACCATCCTAACAGAACCAG | 510634 |
| CATTAAGAACAAACACAAGAAAAGAGGGCG | 510635 |
| CCAAAAAACAAGTCGAATATGTCACACAGA | 510636 |
| CCAACAAGACGCAAAAACAAAGTACCAGA | 510637 |
| CCACACAACGCAACAGAGTGATCTAACGCG | 510638 |
| CCATCACGAAAAATAAGCGCAGAGAAAGCA | 510639 |
| CCATTAACCATTATCAGAGGACCGCGAATC | 510640 |
| CCCCCCTCTGTCTCTTGTTTCTCTTTTCTA | 510641 |
| CCCCTCTCGCTTTCTATTCCTTTGTGCCCT | 510642 |
| CCCGCCTGATCCAAAGTACTGACTCTGTTA | 510643 |
| CCGAAAACAGGAGCCAAAAGGGTAAGAGGA | 510644 |
| CCGAATCCAGACAGGAGCGAGCCCAACAGG | 510645 |
| CCGACACCAGAACTAGGAGCATCGAGCACA | 510646 |
| CCGGACGGAAGATAGGAGGAACAGCTGCAG | 510647 |
| CCGTAAAGGAGGGGGAGGATCCAGCGAATT | 510648 |
| CCTATCGAAAACCCCCCTCATCACA | 510649 |
| CCTTTTGTTGAAAATGAATCCGCTCTTTAT | 510650 |
| CGAACAAATCGATACAATACGGATCAAGGG | 510651 |
| CGACACAAACCAGTGGGAAAGCACTAAGCA | 510652 |
| CGACCAACCTACCACGAACACTCCCCTGGA | 510653 |
| CGAGCCGTCGGAGGGAAAAAGCTAAGGGAG | 510654 |
| CGAGGAAAGATCATGACTCCATACCAGATG | 510655 |
| CGAGGAACAACTCACAGATAGAAAAGGAGG | 510656 |
| CGATCAGCCGAGAAAGAAGGAAGAGTGGCA | 510657 |
| CGCAAGAAGAGGACGCATATAGTACTACCG | 510658 |
| CGCATTGCAAGAGGGAAAATACAGAGTGCC | 510659 |

(continued)

| Sequence (5'->3') | SEQ ID NO. |
|---|---|
| CGCCGTCAAAACAGGATGAACGTATAAGAG | 510660 |
| CGCGTTAGACCAGAGGACAAATAGCATGAA | 510661 |
| CGGGACATGCAGAAACCAGAGAGGAACGCG | 510662 |
| CGGGCACACAAGAAGCAAAGAGAGGATGCA | 510663 |
| CGGTAAGGAGGGTCACCGACGTCGCGCCGT | 510664 |
| CGTAATACATCACACCGAAACAAGGCCAGA | 510665 |
| CGTCACCAATCCCAGAACAAGGAAGCCTCG | 510666 |
| CGTCCCACAAAGAACACCTACCTGCCAAGA | 510667 |
| CGTCGGGCAATCGAGGAGGGGGAGTTCGCA | 510668 |
| CGTGCAGTAAATACCAGGAGTACCGCACAC | 510669 |
| CGTGTCAAGGAAGGTAACCAGGGAGACGAA | 510670 |
| CTAGAAAGAACCACCGAAGAGTTACGCGTG | 510671 |
| CTCGCAGTTACCGCCTTGTCATCCAAATCA | 510672 |
| CTCTTCTCTGCCCCCTGTTTTTCTATCTCT | 510673 |
| CTGGAACTATTACTATACTGATCTCTAAAT | 510674 |
| CTGGACAAACAGCGAAATGAAGAGGGATCG | 510675 |
| CTTATTTCTTTTATAAGTCGTTTACTCAGA | 510676 |
| CTTCTCTATCCAGATTGCCCCTTTATTCTT | 510677 |
| GATACTAGACCACCACGTGTCCATCACAAAAGAC | 510678 |
| GATACTAGACCACCACGTGTCCATCAGAAAAGAC | 510679 |
| GCAGAGTAATCGACCGTCTACCTTCCAGCG | 510680 |
| GCCACGAAGAAAGAGGGAATCAAGCAGAGA | 510681 |
| GCCAGAAGCGAACACACAACCAGATATGAG | 510682 |
| GCCCTATTCCCTCGCTTTCTCCCCTTTTGT | 510683 |
| GCCGAGGGATACCGGAAACAAAGGGAAGCA | 510684 |
| GCGGAAAATTTACCAGAGTGAGGATAACAA | 510685 |
| GCGGAACAAGCAGAAACCAGGAGTAGCACC | 510686 |
| GCGTCAATACCAGAGGGCGATAGTCGTTAG | 510687 |
| GGAGCGCCAAAATAAACGAATCCTCCATAG | 510688 |
| GGAGGAGAGGTAAGGAACACAACAGAACCA | 510689 |
| GGATGGCGTCGACAAACCTCAACTCCAGAA | 510690 |
| GGCAACGTCTACCGAAACAGAGGAGCACTA | 510691 |
| GGCACAACAACGAATAACTACGAAGATACG | 510692 |
| GGCATCAATAAACACGATCCCCGCAAGGAG | 510693 |
| GGCCAAAATGAACCAGAAAGCATCGAGCGA | 510694 |
| GGCGAAGGAAGGATAACAAGATTCGGGTGGA | 510695 |
| GGGAACGGGAACAATGAATAGGAAGCTGAG | 510696 |
| GGGAACGGTAGAATAACACTTAAGAACAGA | 510697 |

(continued)

| Sequence (5'->3') | SEQ ID NO. |
|---|---|
| GGGTGGGCGCGGGAGGAGGTGGGGGAGGGT | 510698 |
| GTACACCAAACACCGCATATCCGCGCCAGA | 510699 |
| GTAGTCAATGGGAAACAGAGTTACGATCGA | 510700 |
| GTGATGTATTTGCCTGTGTTTATCGCTTTT | 510701 |
| TACGTCTTTTTTCGCTTTTGATCATCTCTG | 510702 |
| TACTTATCCAGGAGGATAAGTCGATTACTA | 510703 |
| TACTTATCCCCTCATGATAAGTCGAGTACT | 510704 |
| TACTTATTCCATTAAATAAGTCGATTACTT | 510705 |
| TACTTATTCTTATAAGTCGAGTACTTCCCA | 510706 |
| TATTGAGTTGTGTATTCTTTTTGGTGCTCC | 510707 |
| TCAACAGAGGATGCAGTAATAAAGCCGCAA | 510708 |
| TCACACACCGGGATTCGGACCTACTACCTT | 510709 |
| TCACGCAAGAGGAGGAGGAGGGACACACGA | 510710 |
| TCATAAGACCGAGCCAGAAGCGTACCCGAG | 510711 |
| TCATATCTGTTGTGTTTTCCTCTTGTGTAT | 510712 |
| TCATTCAAACAGAGGGCATAGCACATTCGA | 510713 |
| TCCGTAAAAGAGAGTAGACGTCCAGACCCA | 510714 |
| TCCTTACACCATACCTATCTACAATTTACT | 510715 |
| TCGACCACCTTCAGAACTAACCGCACTGAG | 510716 |
| TCGATATATGGTCGGGTATTCGATATTT | 510717 |
| TCGGAACGTAATTCAGGACCAGAGCACGCA | 510718 |
| TCTATGCCGCAATGTTTCGAAACGTTGCTT | 510719 |
| TCTCATGAGTGTCTTTCAACCTCTCATTCT | 510720 |
| TCTTATCTGTTGTGTTTTCCTATTGTGTAT | 510721 |
| TCTTATCTGTTGTGTTTTCCTCTTGTGTAG | 510722 |
| TCTTATCTGTTGTGTTTTCCTCTTGTGTAT | 510723 |
| TCTTCACCGTTTATTCTAAGGATTTTTTTC | 510724 |
| TCTTTCGTCTTGTTATGTATTGCTGCGTCACTCT | 510725 |
| TCTTTCTTTTGTGTAGTCCTCTTTTCTTGT | 510726 |
| TGACCCCAACCATACCAACAACATCCATAG | 510727 |
| TGATCTGTTGTGTTTTCCTCTTGTGTAT | 510728 |
| TGATCTGTTGTGTTTTCCTTTGGGTTTTCT | 510729 |
| TGATGTGTAGGTTTACTCCCGTATAAA | 510730 |
| TGATGTGTAGGTTTACTCCTCTACCTTGCG | 510731 |
| TGCGCATAAAATAAGAAAAGAGGGGTGCAC | 510732 |
| TGGACAAGACCACCGAAAAAATAGCAGAGT | 510733 |
| TGGGAGGGTAGTGGTGGCGGTGGGGGAGGG | 510734 |
| TGTGCCTGTTTCCTTATTGTTGTGTTTTCC | 510735 |

(continued)

| Sequence (5'->3') | SEQ ID NO. |
|---|---|
| TGTTTTCCTTCTTACCTTTATGCTGCGTCACTCT | 510736 |
| TGTTTTCTCTTTCTACTCTTCCCCCCTCTG | 510737 |
| TTACGATAAGAAAATAAGGAAGATAACAGA | 510738 |
| TTACTTATTACTGAGAATAAGTCGTTTACT | 510739 |
| TTACTTATTCAAGGTATAAGTCGTTTACTG | 510740 |
| TTACTTATTCACCGTAATAAGTCGAGTACT | 510741 |
| TTACTTATTTTGTAATAAGTTGATTACTTG | 510742 |
| TTATGTGTAGGTTCACTCATATATTTCTTT | 510743 |
| TTATGTGTAGGTTTACTCCTCTACCTTGCG | 510744 |
| TTCCACTTTCCCTTCGTTGTATTCTCCTTT | 510745 |
| TTCCCTTGTTTCCTGTTGTGTTGTCCTCCT | 510746 |
| TTCCTTTTGTGTAGTCCTCTTCTCTTTACT | 510747 |
| TTGCGCTTTTGTGTAGGTTTACTCCCTTTT | 510748 |
| TTGTGTAGGTTTACTCCTCTACCTTGCG | 510749 |
| TTGTGTGTTTCTTTGCTGGGTGAATCCCTT | 510750 |
| TTGTTGTGTAGGTTTACTCCCGTATAAA | 510751 |
| TTGTTGTGTAGGTTTACTCCTCTACCTTGCG | 510752 |
| TTGTTGTGTTCTCTTTTTGTGTTTTCCTAG | 510753 |
| TTGTTGTGTTCTCTTTTTGTGTTTTCCTAT | 510754 |
| TTGTTGTGTTCTCTTTTTTTGTTTTCCTAT | 510755 |
| TTTCTATTCCCCCCTGCCCTTTTGTCTCGC | 510756 |
| TTTGTGTAGGTTTACTCCCGTATAAA | 510757 |
| TTTGTGTAGGTTTACTCCTCTACCTTGCG | 510758 |
| TTTGTTGTGTAGGTTCACTCATATATTTCTTT | 510759 |
| TTTGTTGTGTAGGTTTACTCCCGTATAAA | 510760 |
| TTTGTTGTGTAGGTTTACTCCCTTTT | 510761 |
| TTTGTTGTGTAGGTTTACTCCTCTACCTTGCG | 510762 |
| TTTGTTGTGTTCTCTTTTTGTGTTTTCCTAT | 510763 |

**Table 16: Use of oligonucleotide probe library to detect various conditions**

| Indication | Sample Type | Figure | SEQ ID NOs. |
|---|---|---|---|
| Alzheimer's disease | Plasma | **FIG. 15A** | 510600, 510604, 510605, 510608, 510609, 510612, 510614, 510629, 510632, 510633, 510634, 510641, 510642, 510643, 510646, 510648, 510649, 510651, 510652, 510653, 510655, 510661, 510667, 510673, 510675, 510676, 510677, 510678, 510679, 510681, 510683, 510685, 510687, 510688, 510690, 510694, 510696, 510702, 510707, 510709, 510726, 510727, 510728, 510729, 510730, 510731, 510732, 510737, 510740, 510748, 510749, 510751, 510752, 510754, 510756, 510757, 510758, 510761, 510762 |

(continued)

| Indication | Sample Type | Figure | SEQ ID NOs. |
|---|---|---|---|
| Alzheimer's disease | Serum | FIG. 15B | 510599, 510601, 510603, 510606, 510608, 510609, 510611, 510613, 510614, 510615, 510619, 510621, 510625, 510628, 510629, 510630, 510632, 510634, 510635, 510636, 510637, 510644, 510647, 510648, 510651, 510652, 510654, 510657, 510665, 510666, 510667, 510668, 510677, 510678, 510679, 510687, 510692, 510693, 510696, 510698, 510699, 510701, 510702, 510707, 510708, 510710, 510713, 510716, 510725, 510726, 510728, 510731, 510732, 510733, 510734, 510736, 510741, 510748, 510749, 510755, 510757, 510758, 510761, 510762 |
| Bronchial asthma | Plasma | FIG. 15C | 510601, 510614, 510619, 510623, 510627, 510631, 510633, 510635, 510647, 510655, 510656, 510660, 510672, 510689, 510690, 510693, 510698, 510701, 510702, 510707, 510709, 510710, 510713, 510720, 510723, 510724, 510726, 510728, 510729, 510730, 510731, 510734, 510735, 510738, 510743, 510744, 510745, 510746, 510748, 510749, 510750, 510751, 510752, 510754, 510755, 510757, 510758, 510759, 510760, 510761, 510762 |
| Bronchial asthma | Serum | FIG. 15D | 510600, 510608, 510609, 510610, 510611, 510617, 510619, 510622, 510631, 510632, 510634, 510635, 510637, 510642, 510643, 510644, 510652, 510655, 510657, 510658, 510665, 510668, 510673, 510675, 510676, 510677, 510678, 510679, 510683, 510691, 510701, 510703, 510704, 510706, 510708, 510709, 510714, 510725, 510736, 510737, 510740, 510741, 510742, 510756 |
| Transitional cell carcinoma of the bladder | Plasma | FIG. 15E | 510607, 510619, 510623, 510631, 510632, 510635, 510641, 510642, 510647, 510656, 510657, 510658, 510659, 510673, 510674, 510683, 510686, 510693, 510695, 510701, 510702, 510707, 510708, 510711, 510716, 510722, 510725, 510726, 510728, 510731, 510734, 510737, 510744, 510748, 510749, 510751, 510752, 510753, 510756, 510757, 510758, 510761, 510762 |
| Giant cellular osteoblastoclastoma | Serum | FIG. 15F | 510612, 510620, 510635, 510637, 510641, 510644, 510648, 510658, 510662, 510663, 510667, 510668, 510670, 510676, 510678, 510679, 510682, 510683, 510685, 510686, 510699, 510708, 510712, 510722, 510737, 510753 |
| Brain Tumor | Plasma | FIG. 15G | 510607, 510619, 510624, 510628, 510639, 510641, 510645, 510647, 510648, 510655, 510657, 510665, 510668, 510673, 510674, 510689, 510695, 510698, 510699, 510705, 510710, 510711, 510712, 510713, 510716, 510734, 510737, 510738, 510762 |
| Colorectal adenocarcinoma | Plasma | FIG. 15H | 510603, 510607, 510611, 510616, 510618, 510619, 510623, 510624, 510641, 510644, 510646, 510647, 510655, 510656, 510672, 510673, 510690, 510693, 510695, 510698, 510701, 510702, 510709, 510711, 510714, 510716, 510719, 510723, 510724, 510725, 510726, 510730, 510731, 510734, 510735, 510737, 510738, 510743, 510744, 510748, 510749, 510751, 510752, 510754, 510755, 510757, 510758, 510760, 510761, 510762, 510763 |
| Chronic obstructive pulmonary disease (COPD) | Plasma | FIG. 15I | 510604, 510608, 510609, 510612, 510614, 510616, 510619, 510620, 510629, 510634, 510637, 510640, 510647, 510649, 510653, 510661, 510666, 510667, 510669, 510673, 510678, 510682, 510689, 510690, 510701, 510707, 510715, 510723, 510727, 510728, 510749, 510754, 510755, 510757, 510758, 510762, 510763 |

(continued)

| Indication | Sample Type | Figure | SEQ ID NOs. |
|---|---|---|---|
| Chronic obstructive pulmonary disease (COPD) | Serum | FIG. 15J | 510601, 510609, 510611, 510613, 510620, 510634, 510637, 510647, 510648, 510654, 510664, 510668, 510679, 510694, 510696, 510698, 510699, 510701, 510705, 510706, 510710, 510718, 510734, 510741 |
| Squamous cell carcinoma of the cervix | Plasma | FIG. 15K | 510615, 510619, 510623, 510626, 510630, 510632, 510633, 510635, 510638, 510639, 510641, 510642, 510644, 510647, 510650, 510655, 510656, 510657, 510661, 510673, 510674, 510677, 510683, 510688, 510693, 510695, 510698, 510711, 510712, 510714, 510716, 510717, 510722, 510725, 510729, 510731, 510734, 510737, 510743, 510745, 510747, 510753, 510755, 510756, 510758, 510759, 510763 |
| Acute myocardial infarction (AMI) / acute heart failure | Plasma | FIG. 15L | 510607, 510608, 510613, 510626, 510629, 510631, 510634, 510635, 510638, 510639, 510646, 510648, 510656, 510667, 510669, 510671, 510672, 510675, 510682, 510689, 510698, 510701, 510707, 510710, 510715, 510721, 510723, 510727, 510728, 510730, 510731, 510734, 510735, 510743, 510744, 510748, 510749, 510751, 510752, 510757, 510758, 510760, 510761, 510762 |
| Acute myocardial infarction (AMI) / acute heart failure | Serum | FIG. 15M | 510599, 510601, 510606, 510607, 510608, 510609, 510611, 510614, 510616, 510619, 510622, 510624, 510626, 510635, 510636, 510637, 510640, 510641, 510643, 510644, 510648, 510651, 510665, 510668, 510669, 510672, 510675, 510677, 510678, 510679, 510682, 510692, 510695, 510696, 510698, 510699, 510701, 510703, 510707, 510710, 510725, 510726, 510728, 510729, 510730, 510731, 510733, 510734, 510736, 510743, 510750, 510751, 510752, 510755, 510757, 510758, 510759, 510760, 510761, 510762 |
| Chron's Disease | Plasma | FIG. 15N | 510600, 510602, 510608, 510611, 510624, 510644, 510653, 510656, 510659, 510669, 510671, 510676, 510686, 510689, 510690, 510697, 510698, 510700, 510713, 510727, 510728, 510729, 510731, 510734, 510744, 510751, 510757 |
| Chron's Disease | Serum | FIG. 15O | 510600, 510610, 510611, 510615, 510618, 510621, 510623, 510625, 510626, 510628, 510631, 510632, 510635, 510637, 510638, 510643, 510647, 510648, 510649, 510653, 510654, 510655, 510657, 510658, 510666, 510667, 510668, 510672, 510675, 510677, 510678, 510679, 510680, 510682, 510684, 510689, 510691, 510694, 510696, 510698, 510701, 510707, 510708, 510709, 510710, 510713, 510714, 510715, 510719, 510725, 510727, 510728, 510729, 510730, 510731, 510734, 510736, 510738, 510743, 510744, 510748, 510750, 510751, 510755, 510757, 510758, 510759, 510760, 510761, 510762, 510763 |
| Diabetes mellitus type II | Plasma | FIG. 15P | 510600, 510604, 510608, 510610, 510611, 510614, 510616, 510620, 510624, 510629, 510632, 510634, 510640, 510649, 510667, 510669, 510670, 510671, 510678, 510685, 510700, 510701, 510702, 510707, 510709, 510718, 510721, 510723, 510726, 510727, 510728, 510729, 510730, 510731, 510733, 510735, 510743, 510748, 510749, 510752, 510754, 510755, 510757, 510758, 510760, 510761, 510762, 510763 |
| Diabetes mellitus type II | Serum | FIG. 15Q | 510613, 510632, 510635, 510636, 510641, 510645, 510647, 510648, 510654, 510660, 510664, 510667, 510668, 510670, 510675, 510684, 510691, 510695, 510696, 510706, 510710, 510734, 510749 |
| Esophageal carcinoma | Plasma | FIG. 15R | 510602, 510619, 510623, 510632, 510635, 510638, 510641, 510644, 510653, 510656, 510661, 510671, 510682, 510689, 510693, 510698, 510714, 510722, 510725, 510731, 510734, 510738, 510753, 510761 |

(continued)

| Indication | Sample Type | Figure | SEQ ID NOs. |
|---|---|---|---|
| Squamous cell carcinoma of the larynx | Plasma | FIG. 15S | 510605, 510607, 510612, 510614, 510619, 510623, 510632, 510635, 510641, 510642, 510655, 510656, 510657, 510659, 510661, 510668, 510673, 510674, 510689, 510690, 510693, 510695, 510698, 510708, 510712, 510732, 510734, 510737, 510738, 510745, 510747, 510753, 510755 |
| Acute and chronic leukemia of the bone marrow | Plasma | FIG. 15T | 510600, 510605, 510607, 510610, 510612, 510628, 510631, 510633, 510641, 510644, 510650, 510664, 510670, 510673, 510674, 510675, 510681, 510684, 510685, 510686, 510701, 510711, 510712, 510717, 510718, 510719, 510720, 510721, 510724, 510729, 510732, 510739, 510740, 510743, 510745, 510746, 510747, 510752, 510754, 510763 |
| Lung carcinoma | Plasma | FIG. 15U | 510604, 510626, 510628, 510631, 510633, 510635, 510649, 510650, 510654, 510668, 510672, 510674, 510677, 510699, 510701, 510702, 510710, 510712, 510715, 510717, 510719, 510720, 510721, 510723, 510724, 510726, 510727, 510733, 510735, 510738, 510743, 510744, 510745, 510746, 510747, 510750, 510751, 510754, 510755, 510758, 510760, 510763 |
| Malignant lymphoma | Plasma | FIG. 15V | 510601, 510611, 510618, 510623, 510624, 510631, 510632, 510636, 510638, 510641, 510644, 510645, 510647, 510656, 510660, 510662, 510672, 510673, 510675, 510690, 510693, 510701, 510702, 510707, 510708, 510713, 510717, 510719, 510721, 510723, 510724, 510725, 510726, 510728, 510729, 510730, 510731, 510734, 510735, 510737, 510743, 510744, 510749, 510751, 510752, 510754, 510755, 510756, 510757, 510758, 510760, 510762, 510763 |
| Multiple Sclerosis | Plasma | FIG. 15W | 510607, 510612, 510620, 510646, 510653, 510655, 510661, 510663, 510669, 510675, 510682, 510685, 510686, 510690, 510699, 510701, 510710, 510713, 510731, 510738, 510747, 510758, 510762 |
| Multiple Sclerosis | Serum | FIG. 15X | 510599, 510604, 510609, 510610, 510613, 510617, 510618, 510622, 510632, 510635, 510647, 510670, 510675, 510677, 510687, 510690, 510692, 510695, 510701, 510706, 510708, 510731, 510733 |
| Ovarian carcinoma | Plasma | FIG. 15Y | 510618, 510626, 510628, 510633, 510638, 510641, 510642, 510643, 510645, 510646, 510647, 510650, 510652, 510658, 510665, 510666, 510673, 510674, 510677, 510682, 510683, 510689, 510705, 510707, 510712, 510717, 510722, 510724, 510729, 510732, 510735, 510737, 510745, 510746, 510747, 510753, 510756 |
| Parkinson disease | Plasma | FIG. 15Z | 510600, 510601, 510604, 510608, 510609, 510612, 510614, 510624, 510631, 510633, 510634, 510640, 510641, 510642, 510649, 510650, 510651, 510653, 510667, 510673, 510675, 510676, 510677, 510683, 510686, 510689, 510694, 510700, 510703, 510704, 510705, 510706, 510707, 510709, 510713, 510715, 510721, 510723, 510724, 510726, 510727, 510729, 510730, 510731, 510732, 510734, 510735, 510736, 510737, 510739, 510740, 510741, 510742, 510744, 510745, 510746, 510747, 510748, 510751, 510752, 510754, 510756, 510757, 510758, 510759, 510760, 510761, 510762 |
| Parkinson disease | Serum | FIG. 15AA | 510601, 510606, 510608, 510609, 510610, 510614, 510616, 510632, 510634, 510643, 510644, 510647, 510648, 510654, 510662, 510664, 510665, 510667, 510668, 510670, 510677, 510678, 510679, 510687, 510692, 510696, 510698, 510699, 510701, 510703, 510704, 510706, 510708, 510710, 510722, 510727, 510734, 510736, 510741, 510742, 510753, 510756 |

(continued)

| Indication | Sample Type | Figure | SEQ ID NOs. |
|---|---|---|---|
| Prostate adenocarcinoma | Plasma | FIG. 15AB | 510600, 510603, 510607, 510619, 510623, 510624, 510628, 510641, 510642, 510644, 510647, 510650, 510655, 510656, 510657, 510669, 510673, 510674, 510677, 510684, 510688, 510689, 510690, 510695, 510698, 510701, 510709, 510710, 510718, 510726, 510734, 510737, 510738, 510739, 510757, 510762 |
| Psoriasis | Plasma | FIG. 15AC | 510600, 510601, 510608, 510609, 510611, 510614, 510620, 510624, 510626, 510631, 510633, 510634, 510635, 510638, 510647, 510649, 510650, 510653, 510656, 510665, 510666, 510667, 510669, 510672, 510674, 510675, 510680, 510685, 510689, 510698, 510702, 510707, 510711, 510712, 510715, 510717, 510719, 510720, 510721, 510723, 510724, 510726, 510727, 510728, 510729, 510730, 510731, 510734, 510735, 510743, 510744, 510745, 510746, 510747, 510748, 510749, 510750, 510751, 510752, 510754, 510755, 510757, 510758, 510759, 510760, 510761, 510762, 510763 |
| Psoriasis | Serum | FIG. 15AD | 510600, 510601, 510604, 510613, 510621, 510627, 510637, 510641, 510644, 510648, 510650, 510652, 510663, 510667, 510668, 510676, 510680, 510681, 510699, 510701, 510703, 510705, 510709, 510710, 510722, 510734, 510739, 510750, 510753 |
| Rheumatoid Arthritis | Plasma | FIG. 15AE | 510599, 510603, 510604, 510607, 510608, 510609, 510614, 510616, 510622, 510625, 510627, 510629, 510630, 510634, 510635, 510636, 510637, 510640, 510642, 510646, 510649, 510650, 510651, 510653, 510656, 510664, 510665, 510667, 510671, 510675, 510677, 510678, 510679, 510682, 510689, 510699, 510707, 510726, 510727, 510728, 510729, 510731, 510733, 510737, 510738, 510748, 510755, 510758, 510761, 510762 |
| Rheumatoid Arthritis | Serum | FIG. 15AF | 510599, 510601, 510603, 510604, 510606, 510607, 510608, 510609, 510610, 510611, 510612, 510613, 510614, 510616, 510617, 510618, 510622, 510623, 510625, 510629, 510630, 510634, 510635, 510636, 510637, 510638, 510639, 510640, 510641, 510643, 510644, 510645, 510646, 510648, 510649, 510651, 510652, 510653, 510654, 510658, 510662, 510663, 510664, 510665, 510666, 510667, 510668, 510669, 510671, 510673, 510677, 510678, 510679, 510682, 510685, 510692, 510696, 510697, 510698, 510699, 510701, 510703, 510710, 510716, 510722, 510725, 510726, 510727, 510730, 510733, 510734, 510738, 510741, 510743, 510753, 510755, 510757 |
| Renal cell carcinoma | Plasma | FIG. 15AG | 510600, 510603, 510604, 510606, 510608, 510614, 510616, 510622, 510628, 510629, 510630, 510632, 510634, 510635, 510640, 510644, 510645, 510646, 510652, 510653, 510656, 510664, 510665, 510666, 510667, 510671, 510675, 510677, 510683, 510685, 510687, 510690, 510701, 510722, 510726, 510728, 510729, 510730, 510731, 510732, 510733, 510734, 510738, 510748, 510749, 510752, 510753, 510758, 510761, 510762 |
| Squamous cell carcinoma of skin | Plasma | FIG. 15AH | 510618, 510622, 510626, 510639, 510641, 510642, 510650, 510658, 510673, 510674, 510683, 510696, 510708, 510712, 510717, 510720, 510722, 510723, 510724, 510727, 510729, 510743, 510745, 510746, 510747, 510752, 510753, 510754, 510755, 510756, 510759, 510761, 510763 |

(continued)

| Indication | Sample Type | Figure | SEQ ID NOs. |
|---|---|---|---|
| Adenocarcinoma of the stomach | Plasma | FIG. 15AI | 510600, 510604, 510608, 510609, 510612, 510626, 510631, 510632, 510634, 510639, 510653, 510658, 510663, 510667, 510681, 510689, 510692, 510693, 510696, 510698, 510699, 510705, 510711, 510715, 510717, 510719, 510723, 510725, 510729, 510731, 510734, 510735, 510736, 510743, 510746, 510748, 510751, 510754, 510757, 510760, 510762, 510763 |
| Carcinoma of the thyroid gland | Plasma | FIG. 15AJ | 510602, 510603, 510614, 510626, 510638, 510641, 510644, 510646, 510653, 510658, 510659, 510661, 510662, 510674, 510689, 510693, 510695, 510698, 510699, 510701, 510704, 510705, 510708, 510717, 510718, 510722, 510727, 510731, 510734, 510736, 510739, 510740, 510741, 510747, 510753, 510755 |
| Testicular cancer | Plasma | FIG. 15AK | 510600, 510603, 510607, 510615, 510616, 510618, 510619, 510621, 510622, 510623, 510624, 510630, 510636, 510637, 510641, 510644, 510645, 510647, 510650, 510654, 510655, 510656, 510657, 510659, 510662, 510665, 510670, 510673, 510674, 510681, 510684, 510685, 510687, 510688, 510689, 510690, 510692, 510693, 510695, 510696, 510698, 510700, 510701, 510704, 510707, 510712, 510713, 510717, 510718, 510726, 510734, 510737, 510738, 510739, 510740, 510742, 510747, 510756, 510757 |
| Ulcerative colitis | Plasma | FIG. 15AL | 510599, 510607, 510611, 510612, 510616, 510620, 510621, 510622, 510624, 510626, 510632, 510633, 510636, 510646, 510655, 510659, 510672, 510673, 510675, 510676, 510677, 510682, 510684, 510691, 510692, 510702, 510703, 510704, 510705, 510706, 510707, 510709, 510710, 510715, 510721, 510723, 510724, 510728, 510729, 510730, 510731, 510735, 510736, 510737, 510739, 510740, 510741, 510743, 510744, 510748, 510751, 510752, 510754, 510757, 510758, 510759, 510760, 510761, 510762 |
| Ulcerative colitis | Serum | FIG. 15AM | 510610, 510611, 510612, 510619, 510622, 510623, 510634, 510635, 510641, 510647, 510648, 510654, 510657, 510664, 510668, 510670, 510671, 510676, 510677, 510678, 510679, 510689, 510691, 510696, 510701, 510703, 510704, 510710, 510722, 510734, 510742, 510753 |
| Uterine adenocarcinoma | Plasma | FIG. 15AN | 510601, 510612, 510621, 510626, 510637, 510641, 510644, 510650, 510653, 510669, 510675, 510684, 510686, 510687, 510696, 510714, 510717, 510722, 510739, 510743, 510745, 510746, 510753, 510755, 510762 |

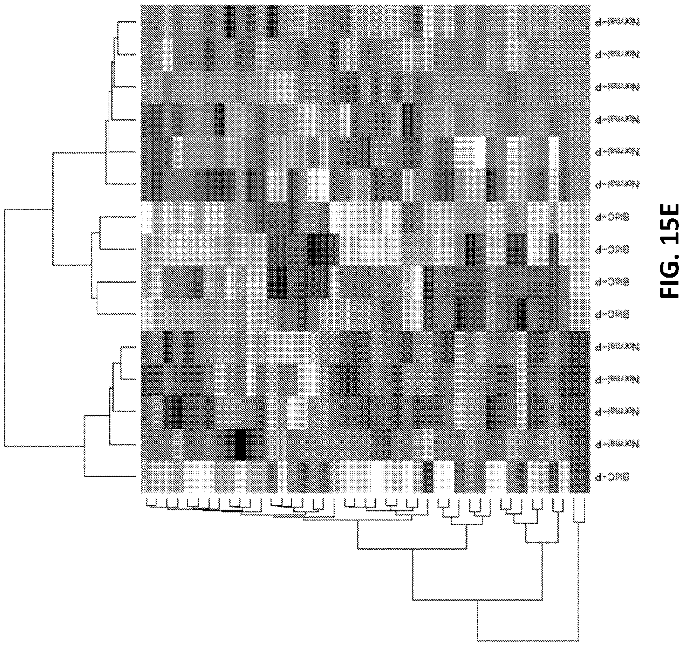

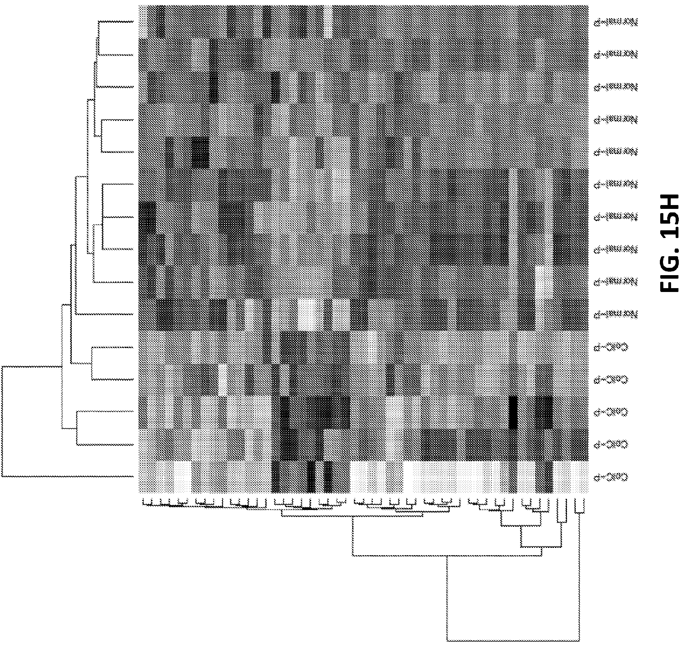

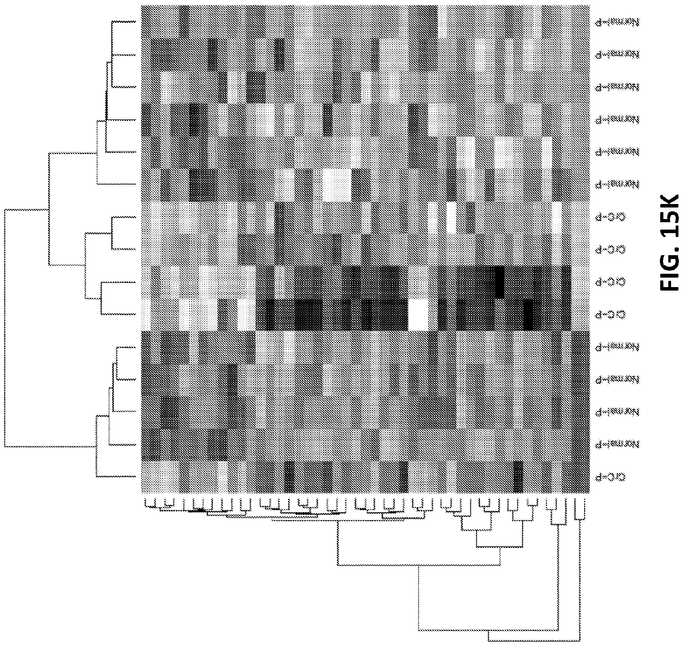

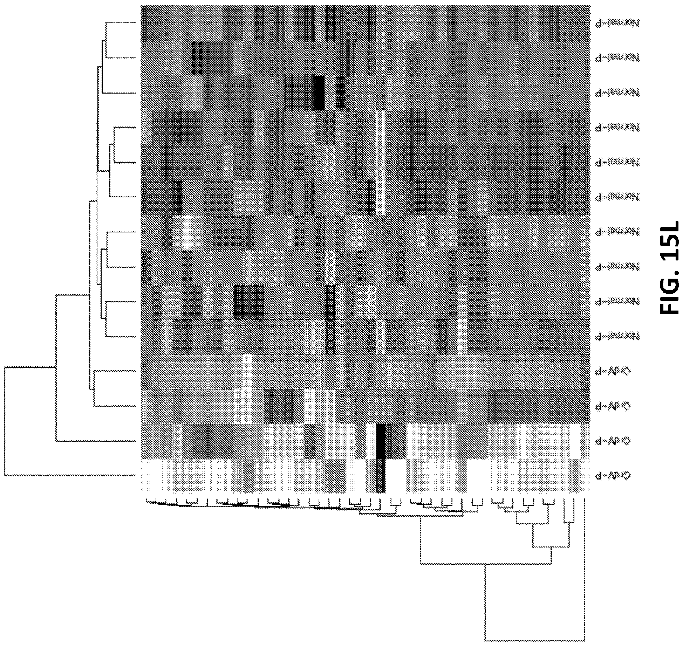

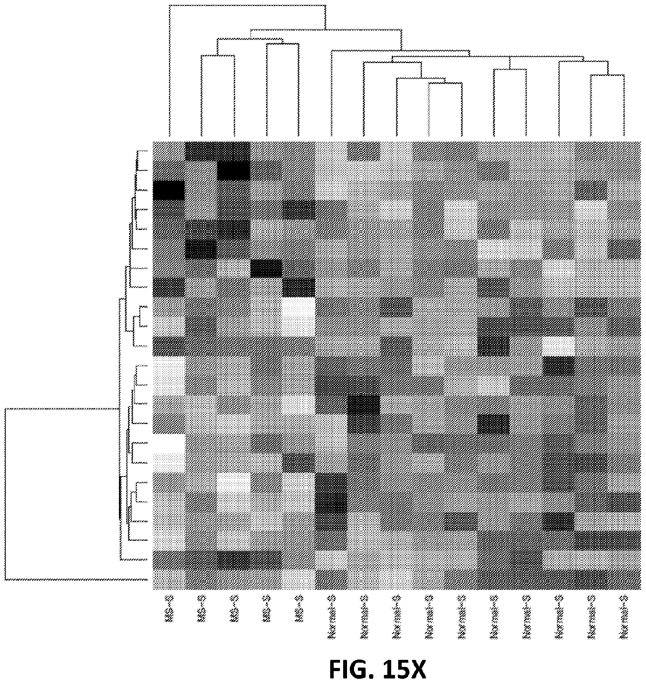

The data in **FIGs. 15A-AN** show that the oligonucleotide probe library was able to distinguish between the disease and normal samples. This can be seen by the clustering of the diseased samples in the figures. For example, in **FIG. 15A** the Alzheimer's samples clustered to the left in the plot whereas the normal samples clustered to the right. Similar results are shown in **FIG. 15H** for colorectal cancer, **FIG. 15L** for cardiovascular disease, and **FIG. 15X** for multiple sclerosis. In **FIG. 15E,** all but one of the bladder cancer samples was found in a central cluster. **FIG. 15K** is similar for cervical cancer. One of skill will appreciate the universal nature of the oligonucleotide probe library can be enhanced by training against multiple input samples and combining resulting oligonucleotide pools.

In this Example, the oligonucleotide probe library trained in the breast cancer setting was used as universal probe to distinguish normal samples against a variety of diseased samples (e.g., cancer, autoimmune, neurological, and cardiovascular). Unexpectedly, the library was efficiently differentiated most of the diseased samples from normal. Without being bound by theory, it is possible that this approach works because the oligonucleotide library was trained against diverse microvesicle populations and still contains a great deal of diversity.

**Example 22: Bodily Fluid probing with Oligonucleotide Probe Library**

The following protocol is used to probe a bodily fluid such as a plasma or serum sample using an oligonucleotide probe library, e.g., as in **Examples 15** and **20-21** above.

Input oligonucleotide library:

Use 2 ng input of oligonucleotide library per sample.

Input oligonucleotide library is a mixture of two libraries, cancer and non-cancer enriched, concentration is 16.3 ng/ul.

Dilute to 0.2ng/ul working stock using Aptamer Buffer (3mM $MgCl_2$ in IX PBS)

Add 10ul from working stock (equal to 2 ng library) to each optiseal tube

Materials:

PBS, Hyclone SH30256.01, LN: AYG165629, bottle# 8237, exp. 7/2015
Round Bottom Centrifuge Tubes, Beckman 326820, LN:P91207
OptiSeal Centrifuge tubes and plugs, polyallomer Konical, Beckman 361621, lot# Z10804SCA
Ultracentrifuge rotor: 50.4 TI
Ultracentrifuge rotor: 50.4 TI, Beckman Caris ID# 0478

Protocol:

1 Pre-chill tabletop centrifuge, ultracentrifuge, buckets, and rotor at 4°C.
2 Thaw plasma or serum samples
3 Dilute 1ml of samples with 1:2 with Aptamer Buffer (3mM $MgCl_2$ in IX PBS)
4 Spin at 2000xg, 30 min, 4°C to remove debris (tabletop centrifuge)
5 Transfer supernatants for all samples to a round bottom conical
6 Spin at 12,000xg, 45 min, 4°C in ultracentrifuge to remove additional debris.
7 Transfer supernatant about 1.8ml for all samples into new OptiSeal bell top tubes (uniquely marked).
8 Add 2ng (in 10 ul) of DNA Probing library to each optiseal tube
9 QS to 4.5 ml with Aptamer Buffer
10 Fix caps onto the OptiSeal bell top tubes
11 Apply Parafilm around caps to prevent leakage
12 Incubate plasma and oligonucleotide probe library for 1 hour at room temperature with rotation
13 Remove parafilm (but not caps)
14 Place correct spacer on top of each plugged tube
15 Mark pellet area on the tubes, insure this marking is facing outwards from center.
16 Spin tubes at 120,000 x g, 2hr, 4°C (inner row, 33,400 rpm) to pellet microvesicles.
17 Check marking is still pointed away from center.
18 Completely remove supernatant from pellet, by collecting liquid from opposite side of pellet marker and using a 10 ml syringe barrel and 21G2 needle
19 Discard supernatant in appropriate biohazard waste container
20 Add 1 ml of 3 mM MgCl2 diluted with IX PBS
21 Gentle vortex, 1600rpm for 5 sec and incubate 5 min at RT.
22 QS to ~4.5 mL with 3 mM Mg Cl2 diluted with IX PBS
23 Fix caps onto the OptiSeal bell top tubes.
24 Place correct spacer on top of each plugged tube.
25 Mark pellet area on the tubes, insure this marking is facing outwards from center.
26 Spin tubes at 120,000 x g, 70 min, 4°C (inner row 33,400 rpm) to pellet microvesicles
27 Check marking in still pointed away from center.
28 Completely remove supernatant from pellet, by collecting liquid from opposite side of pellet marker and using a 10 ml syringe barrel and 21G2 needle
29 Discard supernatant in appropriate biohazard waste container

30 Add 1 ml of 3 mM MgCl2 diluted with IX PBS

31 Gentle vortex, 1600rpm for 5 sec and incubate 5 min at RT.

32 QS to ~4.5 mL with 3 mM Mg Cl2 diluted with IX PBS

33 Fix caps onto the OptiSeal bell top tubes.

34 Place correct spacer on top of each plugged tube.

35 Mark pellet area on the tubes, insure this marking is facing outwards from center.

36 Spin tubes at 120,000 x g, 70 min, 4°C (inner row 33,400 rpm) to pellet microvesicles

37 Check marking is still pointed away from center.

38 Save an aliquot of the supernatant (100ul into a 1.5ml tube)

39 Completely remove supernatant from pellet, by collecting liquid from opposite side of pellet marker and using a 10 ml syringe barrel and 21G2 needle

40 Add 50 ul of Rnase-free water to the side of the pellet

41 Leave for 15min incubation on bench top

42 Cut top off tubes using clean scissors.

43 Resuspend pellet, pipette up and down on the pellet side

44 Measure the volume, make a note on the volume in order to normalize all samples

45 Transfer the measured resuspended eluted microvesicles with bound oligonucleotides to a Rnase free 1.5ml Eppendorf tube

46 Normalize all samples to 100ul to keep it even across samples and between experiments.

Next Generation Sequencing Sample Preparation:

I) Use 50 ul of sample from above, resuspended in 100 ul H2O and containing microvesicle/oligo complexes, as template in Transposon PCR, 14 cycles.

II) AMPure transposon PCR product, use entire recovery for indexing PCR, 10 cycles.

III) Check indexing PCR product on gel, proceed with AMPure if band is visible. Add 3 cylces if band is invisible, check on gel. After purification quantify product with QuBit and proceed with denaturing and dilting for loading on HiSeq flow cell (Illumina Inc., San Diego, CA).

IV) 5 samples will be multiplexed per one flow cell. 10 samples per HiSeq.

Data Analysis (e.g., as in **Example 21):**

All sequence reads from the HiSeq analysis that match both primer sequences are compared to each other. The counts for each unique sequence read are calculated and used as signal for binding. The sequence counts are normalized by total number of sequence reads for each sample. The normalized counts for each unique sequence are compared to identify the oligonucleotides that show different level of binding to samples between a disease condition to the normal control from the same sample type, i.e. Serum and plasma. Only sequences with average counts of all samples greater than 100 are considered. The oligonucleotides data from aptamers with differential binding are clustered using hierarchical clustering and the degree if similarity between samples is shown in heat maps and dendrograms.

**Example 23: Optimized aptamer library design for High Throughput sequencing**

In classical aptamer library construction, e.g., for selection purposes such as SELEX, each member of the library comprises a variable nucleotide sequence region inserted between two conserved flanking sequences. Among various uses, the conserved regions may be used as priming sites for amplification, such as for PCR amplification between selection rounds, priming sites for sequencing, and for hybridization. The forward and reverse primers may be referred to herein as the left (5') and right (3') end primers. As both ends of the aptamer library sequences are identical, the library is considered to be low-diversity for sequencing purposes. This may result in the introduction of bias in amplification or other such biases. In the case of some high throughput sequencing methods, e.g., for Illumina based next generation sequencing (NGS), first sequencing reads establish clusters on the surface of flow cell after clonal amplification. Having regions of identical bases at the beginning of a library member makes it more difficult to distinguish fluorescent signals and, correspondingly, proceed with sequencing. Illumina suggests various solutions for sequencing low-diversity libraries: (i) mix library containing sample with 50% of bacteriophage PhiX genome to introduce diversity; (ii) add control sample to a different lane in the same flow cell; and (iii) stagger the libraries within one sample. Options (i) and (ii) reduce the total sequencing output by half and are thus not optimal. Option (iii) shows variable results: it was used successfully on a MiSeq NGS instrument but not on a HiSeq NGS instrument.

This Example describes an alternate aptamer library design which allows more efficient sequencing of aptamer libraries by introducing diversity to the library itself. The aptamer library design in this Example addresses the following: 1) sequencing of aptamer libraries on high throughput sequencing instruments without spiking in control sample PhiX; 2) sequencing of aptamer libraries on high throughput sequencing instruments at maximum capacity; and 3) sequencing of aptamer libraries on high throughput sequencing instruments with maximum quality. As an illustration, this method was applied in this Example to facilitate use of a HiSeq instrument at its maximum capacity. Although this Example focuses on the HiSeq platform for illustrative purposes, the approach can be used in any appropriate setting, e.g., where oligonucleotide library diversity may present an issue.

A naive oligonucleotide library (i.e., aptamer candidate library) was designed so that the sequencing starting point shifted from the conserved outer region (primer) to the variable region. The left primer of the library was replaced with a transposon adapter, which is complementary to the sequencing primer. Inserted between the transposon adapter and the 35 nucleotide variable region are 0, 1, 2, or 3 nucleotides which server to create on offset in the library design. **FIG. 17A** shows the right arm primer of the aptamer library which follows the variable region (i.e., SEQ ID NO. 510768). In addition to enhancing diversity, a single round of PCR can be used to prepare a sample for NGS with this approach (e.g., adding indexes and adaptor for hybridization to flow cell). This leads to more efficient sample preparation and may decrease the number of potential mutants generated by PCR.

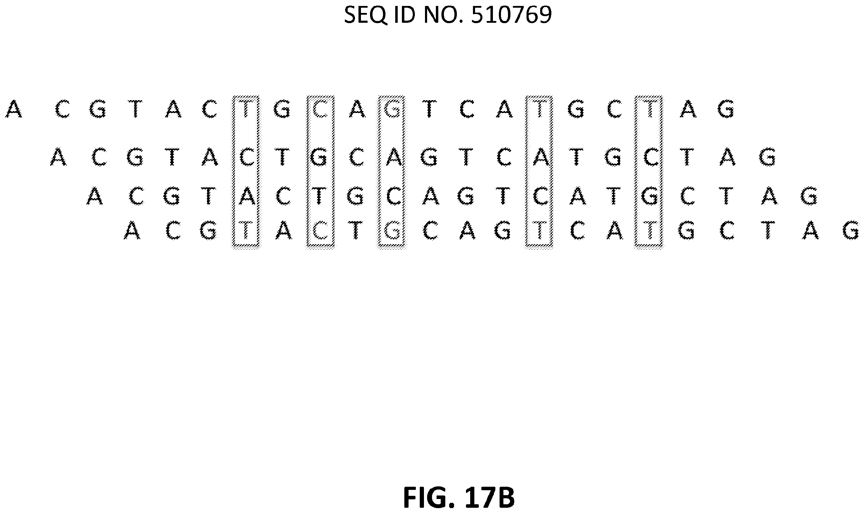

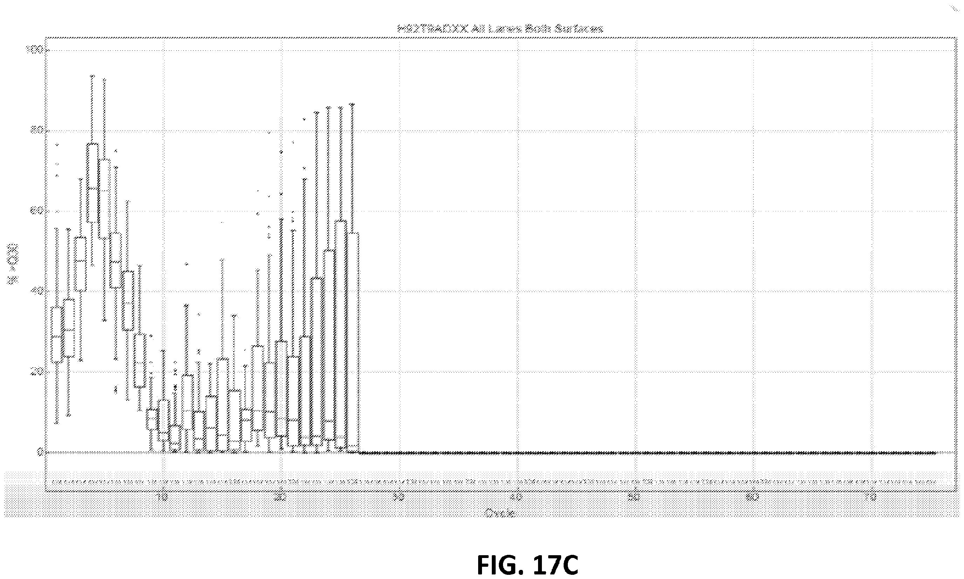

This change in the library design to incorporate the transposon adapter and offset improved the sequencing quality in the variable region of the sequenced library. Compare **FIGs. 17C-D** as discussed further below. To further improve the library, the sequence of the left primer was also designed to reduce potential sequence overlaps, e.g., as indicated by the boxes in **FIG. 17A,** which indicate 14 positions with overlapping nucleotides in the staggered design using the single sequence (SEQ ID NO. 510768) in the figure. The redesigned primer sequence with balanced design contains only five positions where two bases are overlapping. See **FIG. 17B** (showing SEQ ID NO. 510769).

**FIGs. 17C-17F** illustrate sequencing quality reflected in %>Q30 per each HiSeq SBS sequencing cycle. Q30 is equivalent to the probability of an incorrect base call 1 in 1000 times. **FIG. 17C** presents an example of lower quality sequencing of an oligonucleotide library with the starting design where the sequencing was initiated from the conservative (identical) part of the library. No PhiX DNA was added to the sequencing run. Even though the library has a staggered design, the base calling software had difficultly calling these regions.

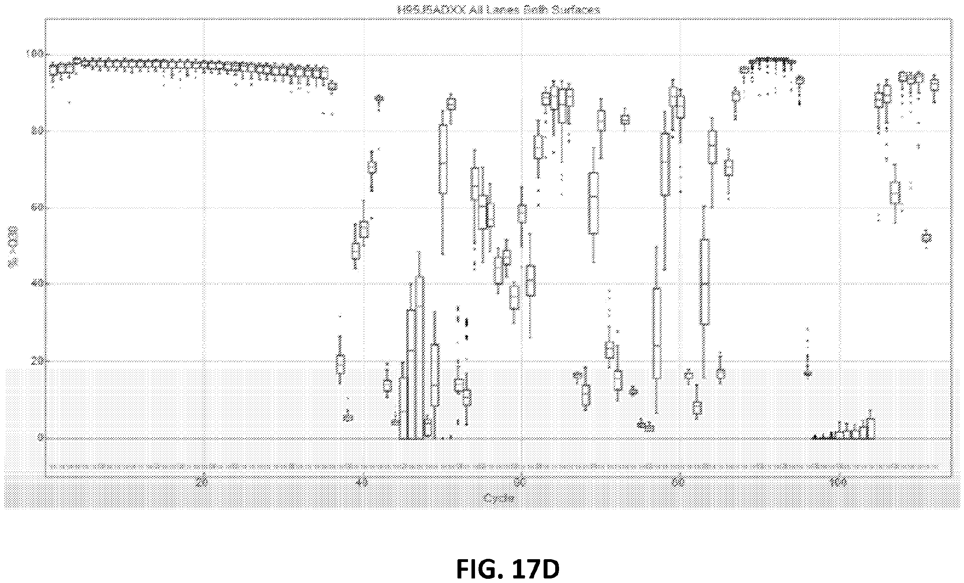

**FIG. 17D** shows an example of the sequencing quality of a standard library wherein a transposon adapter was incorporated into the library as right arm primer. In this case sequencing is starting directly at the variable region, resulting in greatly improved sequencing quality. However, once sequencing reaches the left arm primer, the quality falls off.

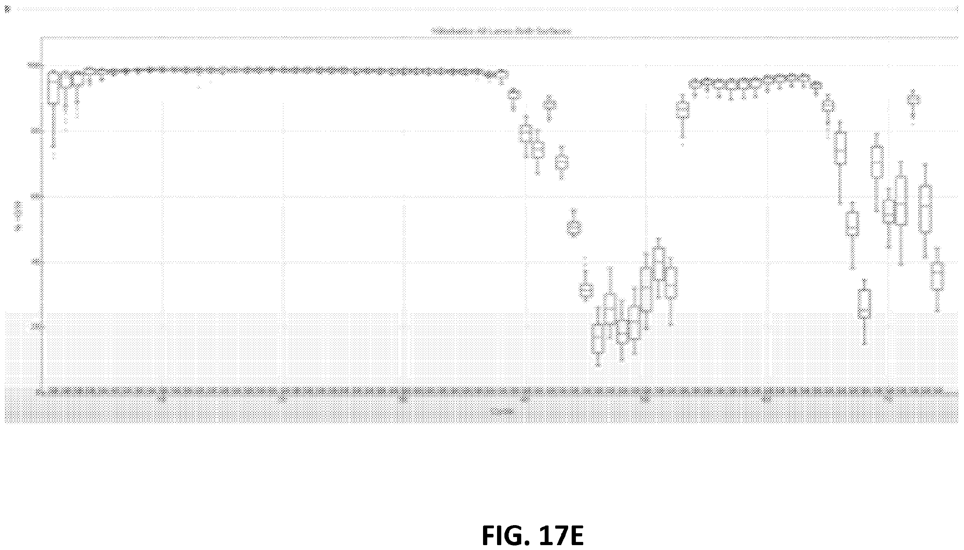

**FIG. 17E** shows an example of sequencing quality with library after introducing the staggered design of **FIG. 17A.** As seen in the figure, sequencing quality is improved compared to the previous, non-staggered design. However, several bases in the middle of the primer region are potentially miscalled.

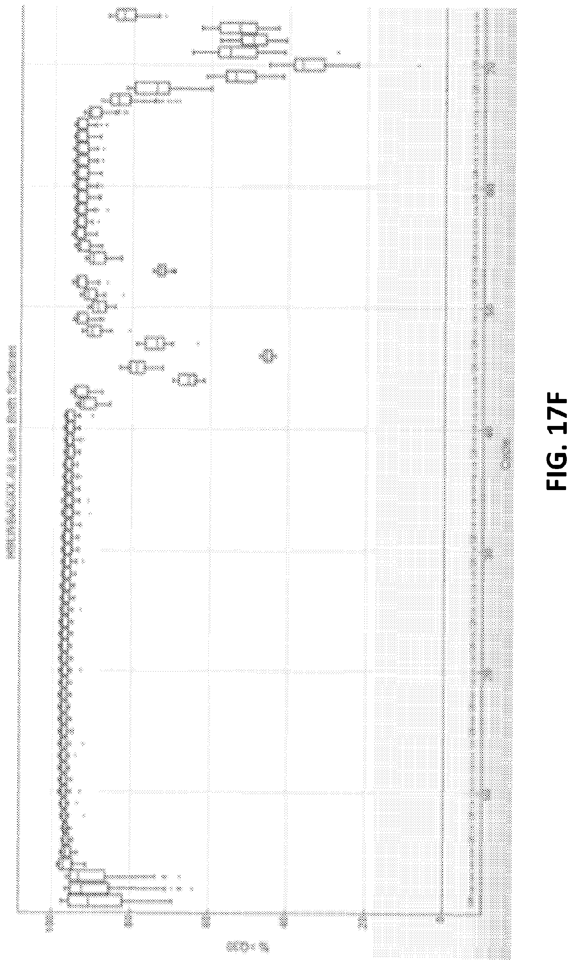

For final tuning of the sequencing quality, the sequence of the left arm primer was rebalanced as shown in **FIG. 17B. FIG. 17F** shows that, for most of the lower Q30 score bases on **FIG. 17E,** quality was improved bu about 50%. This design improved the base calling of the primer sequence and allowed the most efficient match to the starting aptamer library pattern.

Comparing the graphs in **FIG. 17C** (original) and **FIG. 17F** (optimized design) demonstrates the improvement in the aptamer library design and sequencing quality/output correspondingly.

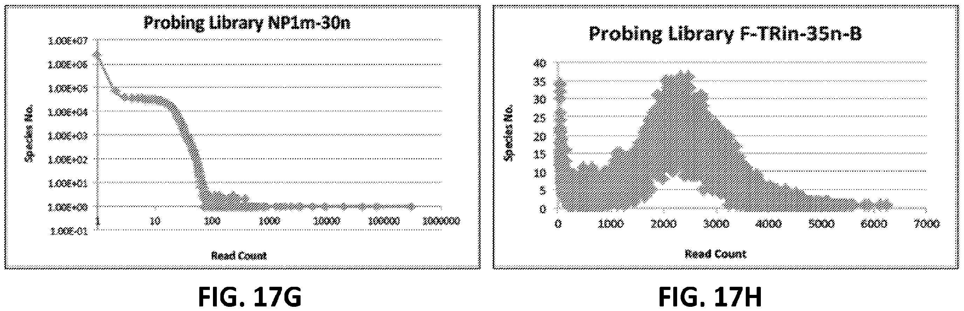

Additional advantages of the improved aptamer library design are demonstrated by the efficacy of enrichment of aptamer sequences against biological targets of interest and by the possibility to manage library effectively with current capacity of high throughput sequencing. **FIGs. 17G-H** shows the classical aptamer library design ("NP1m-30n"; **FIG. 17G)** compared to new one-step balanced library design ("F-TRin-35n-B"; **FIG. 17H)** after enrichment against breast cancer plasma exosomes. As shown in these figures, the improved design allows more efficient enrichment as demonstrated by: (i) presence of distinct population with normal distribution of species number across different read counts; (ii) improved separation of species with low counts from the rest of the library, which allows to exclude sequences from the analysis as most likely they got created by PCR bias or during HiSeq SBS steps; (iii) average read count per species in the enriched population is in the range of 2000-3000 copies, which allows increased sequencing throughput; and (iv) minimized technical variability while probing biological speciments.

The resulting aptamer library can be used to develop individual aptamers or oligonucleotide probe libraries as described herein, e.g., to develop diagnostic assays.

**Claims**

1. A method of characterizing a disease or disorder, comprising:

  (a) contacting a biological test sample with a plurality of oligonucleotides, wherein the plurality of oligonucleotides comprises 164 different oligonucleotide sequences, and wherein each different member of the plurality of oligonucleotides comprises a different sequence that is any one of SEQ ID NOs. 510599-510763 or a sequence that is 95 percent homologous to any one of SEQ ID NOs. 510599-510763;

(b) detecting a presence or level of complexes formed in step (a) between the plurality of oligonucleotides and a target in the biological test sample; and

(c) comparing the presence or level detected in step (b) to a reference level from a biological control sample, thereby characterizing the disease or disorder.

2. The method of claim 1, wherein the detecting in step (b) comprises performing at least one of sequencing, amplification , and hybridization of the members of the plurality of oligonucleotides within the complexes.

3. The method of claim 1 or 2, wherein the detecting in step (b) comprises high-throughput sequencing.

4. The method of any one of claims 1-3, wherein the biological test sample and biological control sample each comprise a tissue sample, a cell culture, or a biological fluid, wherein optionally the biological fluid comprises a bodily fluid.

5. The method of claim 4, wherein the bodily fluid comprises peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, or umbilical cord blood.

6. The method of claim 4, wherein the bodily fluid comprises blood, serum or plasma.

7. The method of any one of claims 4-6, wherein the biological fluid comprises microvesicles, wherein optionally the complexes are formed between the oligonucleotide or plurality of oligonucleotides and at least one of the microvesicles.

8. The method of claim 7, wherein the microvesicles are isolated using at least one of chromatography, filtration, ultrafiltration, centrifugation, ultracentrifugation, flow cytometry, affinity capture (e.g., to a planar surface, column or bead), polymer precipitation, and using microfluidics.

9. The method of any of claims 1-8, wherein at least one member of the plurality of oligonucleotides binds a polypeptide or fragment thereof, optionally wherein the polypeptide or fragment thereof is soluble or membrane bound, wherein optionally the membrane comprises a microvesicle membrane.

10. The method of any of claims 1-9, wherein at least one member of the plurality of oligonucleotides binds a microvesicle surface antigen.

11. The method of any of claims 1-10, wherein the disease or disorder comprises Breast Cancer, Alzheimer's disease, bronchial asthma, Transitional cell carcinoma of the bladder, Giant cellular osteoblastoclastoma, Brain Tumor, Colorectal adenocarcinoma, Chronic obstructive pulmonary disease (COPD), Squamous cell carcinoma of the cervix, acute myocardial infarction (AMI) / acute heart failure, Chron's Disease, diabetes mellitus type II, Esophageal carcinoma, Squamous cell carcinoma of the larynx, Acute and chronic leukemia of the bone marrow, Lung carcinoma, Malignant lymphoma, Multiple Sclerosis, Ovarian carcinoma, Parkinson disease, Prostate adenocarcinoma, psoriasis, Rheumatoid Arthritis, Renal cell carcinoma, Squamous cell carcinoma of skin, Adenocarcinoma of the stomach, carcinoma of the thyroid gland, Testicular cancer, ulcerative colitis, or Uterine adenocarcinoma.

12. The method of any of claims 1-10, wherein the disease or disorder comprises a cancer, a premalignant condition, an inflammatory disease, an immune disease, an autoimmune disease or disorder, a cardiovascular disease or disorder, neurological disease or disorder, infectious disease or pain.

13. The method of any of claims 1-12, wherein at least one member of the plurality of oligonucleotides comprises at least one functional modification.

14. A kit comprising at least one reagent for carrying out the method of any of claims 1-13, wherein the at least one reagent comprises the plurality of oligonucleotides.

15. The kit of claim 14, wherein the at least one reagent further comprises at least one of:

> (a) a reagent configured to isolate a microvesicle, wherein optionally the at least one reagent configured to isolate a microvesicle comprises a binding agent to a microvesicle antigen, a column, a substrate, a filtration unit, a polymer, polyethylene glycol, PEG4000, PEG8000, a particle or a bead;
> (b) at least one oligonucleotide configured to act as a primer or probe in order to amplify, sequence, hybridize or detect the oligonucleotide or plurality of oligonucleotides; and
> (c) a reagent configured to remove one or more abundant protein from the biological test sample, wherein optionally the one or more abundant protein comprises at least one of albumin, immunoglobulin, fibrinogen and fibrin.

**Patentansprüche**

1. Verfahren eines Kennzeichnens einer Erkrankung oder Störung, umfassend:

> (a) Kontaktieren einer biologischen Testprobe mit einer Vielzahl von Oligonukleotiden, wobei die Vielzahl von Oligonukleotiden 164 verschiedene Oligonukleotidsequenzen umfasst und wobei jedes verschiedene Element der Vielzahl von Oligonukleotiden eine andere Sequenz, die eine von SEQ ID NO 510599-510763 ist, oder eine Sequenz, die 95 Prozent homolog zu einer von SEQ ID NO 510599-510763 ist, umfasst;
> (b) Nachweisen einer Anwesenheit oder eines Niveaus von Komplexen, die in Schritt (a) gebildet sind, zwischen der Vielzahl von Oligonukleotiden und einem Target in der biologischen Testprobe; und
> (c) Vergleichen der Anwesenheit oder des Niveaus, die/das in Schritt (b) nachgewiesen wurde, mit einem Referenzniveau aus einer biologischen Kontrollprobe, wodurch die Erkrankung oder Störung gekennzeichnet wird.

2. Verfahren nach Anspruch 1, wobei das Nachweisen in Schritt (b) ein Durchführen von mindestens einem von Sequenzierung, Amplifikation und Hybridisierung der Elemente der Vielzahl von Olignonukleotiden in den Komplexen umfasst.

3. Verfahren nach Anspruch 1 oder 2, wobei das Nachweisen in Schritt (b) eine hohe Durchsatzsequenzierung umfasst.

4. Verfahren nach einem der Ansprüche 1 bis 3, wobei die biologische Testprobe und biologische Kontrollprobe jeweils eine Gewebeprobe, eine Zellkultur oder eine biologische Flüssigkeit umfassen, wobei die biologische Flüssigkeit optional eine Körperflüssigkeit umfasst.

5. Verfahren nach Anspruch 4, wobei die Körperflüssigkeit peripheres Blut, Sera, Plasma, Aszites, Harn, Zerebrospinalflüssigkeit (CSF), Sputum, Speichelflüssigkeit, Knochenmark, Synovialflüssigkeit, Kammerwasser, Fruchtwasser, Zerumen, Muttermilch, bronchoalveoläre Lavageflüssigkeit, Samenflüssigkeit, Prostataflüssigkeit, Präejakulat oder Vorsekret, weibliches Ejakulat, Schweiß, Fäkalien, Tränen, Zystenflüssigkeit, Pleura- und Peritonealflüssigkeit, Perikardflüssigkeit, Lymphflüssigkeit, Chymus, Chylus, Gallenflüssigkeit, interstitielle Flüssigkeit, Menses, Eiter, Talg, Erbrochenes, Vaginalsekrete, Schleimhautsekret, Stuhlwasser, Pankreassaft, Lavageflüssigkeiten aus Nasennebenhöhlen, bronchopulmonale Aspirate, Blastozystenhöhlenflüssigkeit oder Nabelschnurblut umfasst.

6. Verfahren nach Anspruch 4, wobei die Körperflüssigkeit Blut, Serum oder Plasma umfasst.

7. Verfahren nach einem der Ansprüche 4 bis 6, wobei die biologische Flüssigkeit Mikrovesikel umfasst, wobei optional die Komplexe zwischen dem Oligonukleotid oder der Vielzahl von Oligonukleotiden und mindestens einem der Mikrovesikel gebildet sind.

8. Verfahren nach Anspruch 7, wobei die Mikrovesikel unter Verwendung mindestens eines von Chromatografie, Filtration, Ultrafiltration, Zentrifugation, Ultrazentrifugation, Durchflusszytometrie, Affinitätseinfang (z. B. an einer planaren Oberfläche, einer Säule oder einem Kügelchen), Polymerausfällung und unter Verwendung von Mikrofluidik isoliert werden.

9. Verfahren nach einem der Ansprüche 1 bis 8, wobei mindestens ein Element der Vielzahl von Oligonukleotiden ein Polypeptid oder ein Fragment davon bindet, optional wobei das Polypeptid oder Fragment davon löslich oder membrangebunden ist, wobei optional die Membran eine Mikrovesikelmembran umfasst.

**10.** Verfahren nach einem der Ansprüche 1 bis 9, wobei mindestens ein Element der Vielzahl von Oligonukleotiden ein Mikrovesikel-Oberflächenantigen bindet.

**11.** Verfahren nach einem der Ansprüche 1 bis 10, wobei die Erkrankung oder Störung Brustkrebs, Alzheimer-Krankheit, Bronchialasthma, Übergangszellkarzinom der Blase, Riesenzellen-Osteoblastoklastom, Gehirntumor, kolorektales Adenokarzinom, chronische obstruktive Lungenerkrankung (chronic obstructive pulmonary disease, COPD), Plattenepithelkarzinom des Zervix, akuter Myocardinfarkt (AMI)/akute Herzinsuffizienz, Morbus Crohn, Typ-2-Diabetes mellitus, Ösophaguskarzinom, Plattenepithelkarzinom des Kehlkopfs, akute und chronische Leukämie des Knochenmarks, Lungenkarzinom, malignes Lymphom, multiple Sklerose, Ovarialkarzinom, Parkinson-Krankheit, Adenokarzinom der Prostata, Psoriasis, rheumatoide Arthritis, Nierenzellkarzinom, Plattenepithelkarzinom der Haut, Adenokarzinom des Magens, Karzionom der Schilddrüse, Hodenkrebs, Colitis ulcerosa oder uterines Adenokarzinom umfasst.

**12.** Verfahren nach einem der Ansprüche 1 bis 10, wobei die Erkrankung oder Störung einen Krebs, einen prämalignen Zustand, eine entzündliche Erkrankung, eine Immunerkrankung, eine Autoimmunerkrankung oder -störung, eine kardiovaskuläre Erkrankung oder Störung, eine neurologische Erkrankung oder Störung, eine Infektionserkrankung oder Schmerz umfasst.

**13.** Verfahren nach einem der Ansprüche 1 bis 12, wobei mindestens ein Element der Vielzahl von Oligonukleotiden mindestens eine funktionelle Modifikation umfasst.

**14.** Kit, umfassend mindestens ein Reagens zum Ausführen des Verfahrens nach einem der Ansprüche 1 bis 13, wobei das mindestens eine Reagens die Vielzahl von Oligonukleotiden umfasst.

**15.** Kit nach Anspruch 14, wobei das mindestens eine Reagens mindestens eines von Folgendem umfasst:

(a) ein Reagens, das konfiguriert ist, ein Mikrovesikel zu isolieren, wobei optional das mindestens eine Reagens, das konfiguriert ist, ein Mikrovesikel zu isolieren, ein Bindemittel an ein Mikrovesikelantigen, eine Säule, ein Substrat, eine Filtrationseinheit, ein Polymer, Polyethylenglykol, PEG4000, PEG8000, ein Partikel oder ein Kügelchen umfasst;
(b) mindestens ein Oligonukleotid, das konfiguriert ist, als ein Primer oder eine Sonde zu agieren, um das Oligonukleotid oder eine Vielzahl von Olignonukleotiden zu amplifizieren, zu sequenzieren, zu hybridisieren oder nachzuweisen; und
(c) ein Reagens, das konfiguriert ist, ein oder mehrere abundante Proteine aus der biologischen Testprobe zu entfernen, wobei optional das eine oder die mehreren abundanten Proteine mindestens eines von Albumin, Immunglobulin, Fibrinogen und Fibrin umfassen.

**Revendications**

**1.** Procédé de caractérisation d'une maladie ou d'un trouble, comprenant :

(a) la mise en contact d'un échantillon de test biologique avec une pluralité d'oligonucléotides, la pluralité d'oligonucléotides comprenant 164 séquences différentes d'oligonucléotides, et chaque élément différent de la pluralité d'oligonucléotides comprenant une séquence différente qui est l'une quelconque parmi les SEQ ID n° : 510599 à 510763 ou une séquence qui est à 95 pour cent homologue à l'une quelconque des SEQ ID n° : 510599 à 510763 ;
(b) la détection d'une présence ou d'un niveau de complexes formés dans l'étape (a) entre la pluralité d'oligonucléotides et une cible dans l'échantillon de test biologique ; et
(c) la comparaison de la présence ou du niveau détecté dans l'étape (b) à un niveau de référence d'un échantillon biologique témoin, permettant ainsi de caractériser la maladie ou le trouble.

**2.** Procédé selon la revendication 1, dans lequel la détection dans l'étape (b) comprend la réalisation du séquençage, de l'amplification et/ou de l'hybridation des éléments de la pluralité d'oligonucléotides à l'intérieur des complexes.

**3.** Procédé selon la revendication 1 ou 2, dans lequel la détection dans l'étape (b) comprend le séquençage haut débit.

**4.** Procédé selon l'une quelconque des revendications 1 à 3, dans lequel l'échantillon de test biologique et l'échantillon biologique témoin comprennent chacun un échantillon de tissu, une culture cellulaire ou un liquide biologique, le liquide biologique comprenant éventuellement un liquide corporel.

5. Procédé selon la revendication 4, dans lequel le fluide corporel comprend le sang périphérique, le sérum, le plasma, les ascites, l'urine, le fluide céphalorachidien (CSF), une expectoration, la salive, la moelle épinière, le liquide synovial, l'humeur aqueuse, le liquide amniotique, le cérumen, le lait maternel, le liquide de lavage bronchoalvéolaire, le sperme, le liquide prostatique, le liquide de Cowper ou le liquide prééjaculatoire, l'éjaculat féminin, la sueur, la matière fécale, les larmes, le fluide kystique, le liquide pleural et péritonéal, le liquide péricardique, la lymphe, le chyme, le chyle, la bile, le liquide interstitiel, les menstrues, le pus, le sébum, le vomi, les sécrétions vaginales, la sécrétion de muqueuse, l'eau des selles, le suc pancréatique, les liquides de lavage provenant de cavités sinusales, les aspirats bronchopulmonaires, le liquide de la cavité du blastocyste ou le sang du cordon ombilical.

6. Procédé selon la revendication 4, dans lequel le fluide corporel comprend le sang, le sérum ou le plasma.

7. Procédé selon l'une quelconque des revendications 4 à 6, dans lequel le liquide biologique comprend des micro-vésicules, les complexes étant éventuellement formés entre l'oligonucléotide et une pluralité d'oligonucléotides et au moins une des micro-vésicules.

8. Procédé selon la revendication 7, dans lequel les micro-vésicules sont isolées à l'aide de la chromatographie, la filtration, l'ultrafiltration, la centrifugation, l'ultracentrifugation, la cytométrie de flux, la capture par affinité (par ex., vers une surface plane, une colonne ou une bille), la précipitation de polymères et/ou à l'aide de micro fluides.

9. Procédé selon l'une quelconque des revendications 1 à 8, dans lequel au moins un élément de la pluralité d'oligonucléotides se lie à un polypeptide ou un fragment de celui-ci, le polypeptide ou fragment de celui-ci étant éventuellement soluble ou lié à la membrane, la membrane comprenant éventuellement une membrane de micro-vésicule.

10. Procédé selon l'une quelconque des revendications 1 à 9, dans lequel au moins un élément de la pluralité d'oligonucléotides se lie à un antigène de surface de microvésicule.

11. Procédé selon l'une quelconque des revendications 1 à 10, dans lequel la maladie ou le trouble comprend le cancer du sein, la maladie d'Alzheimer, l'asthme bronchique, le carcinome à cellules transitionnelles de la vessie, l'ostéoblastoclastome à cellules géantes, la tumeur cérébrale, l'adénocarcinome colorectal, la maladie pulmonaire obstructive chronique (MPOC), le carcinome épidermoïde du col de l'utérus, l'infarctus du myocarde aigu (AMI) / l'insuffisance cardiaque aiguë, la maladie de Crohn, le diabète sucré de type II, le carcinome de l'oesophage, le carcinome épidermoïde du larynx, la leucémie aiguë et chronique de la moelle épinière, le carcinome du poumon, le lymphome malin, la sclérose en plaques, le carcinome ovarien, la maladie de Parkinson, l'adénocarcinome de la prostate, le psoriasis, la polyarthrite rhumatoïde, le carcinome à cellules rénales, le carcinome épidermoïde de la peau, l'adénocarcinome de l'estomac, le carcinome de la glande thyroïdienne, le cancer des testicules, la colite ulcéreuse ou l'adénocarcinome utérin.

12. Procédé selon l'une quelconque des revendications 1 à 10, dans lequel la maladie ou le trouble comprend un cancer, une affection précancéreuse, une maladie inflammatoire, une maladie immunitaire, une maladie ou un trouble auto-immun, une maladie ou un trouble cardiovasculaire, une maladie ou un trouble neurologique, une maladie infectieuse ou une douleur liée à une infection.

13. Procédé selon l'une quelconque des revendications 1 à 12, dans lequel un élément de la pluralité d'oligonucléotides comprend au moins une modification fonctionnelle.

14. Trousse comprenant au moins un réactif destiné à effectuer le procédé selon l'une quelconque des revendications 1 à 13, dans laquelle l'au moins un réactif comprend la pluralité d'oligonucléotides.

15. Trousse selon la revendication 14, dans lequel l'au moins un réactif comprend en outre au moins un de :

(a) un réactif conçu pour isoler une microvésicule, l'au moins un réactif conçu pour isoler une microvésicule comprenant éventuellement un agent de liaison à un antigène de micro-vésicule, une colonne, un substrat, une unité de filtration, un polymère, un polyéthylène glycol, PEG4000, PEG8000, une particule ou une bille ;
(b) au moins un oligonucléotide conçu pour agir en tant qu'amorce ou sonde afin d'amplifier, de séquencer, d'hybrider ou de détecter l'oligonucléotide ou une pluralité d'oligonucléotides ; et

(c) un réactif conçu pour retirer au moins une protéine abondante de l'échantillon de test biologique, l'au moins une protéine abondante comprenant l'albumine, l'immunoglobuline, le fibrinogène et/ou la fibrine.

# Selection strategy

Single round of binding with
extensive washing
"Rnd1 direct"

Additional round of binding,
washing and competitive
displacement with anti-
EpCAM antibody
"Rnd2 competitive"

**FIG. 1**

EP 3 039 174 B1

FIG. 2A

FIG. 2B

## Screening Scheme

| 5 | X | 20 | = | 100 |
|---|---|---|---|---|
| Detection Antibodies | | Capture Antibodies | | Combinations Screened |

| | | |
|---|---|---|
| CD63 | CD9 | Rab |
| CD9 | PSCA | IgG |
| CD81 | TNFR | CD81 |
| B7H3 | CD63 2X | STEAP |
| EpCam | B7H3 | PCSA |
| | Rab IgG | PSMA |
| | MFG-E8 | 5T4 |
| | EpCam 2X | CD24 |
| | CD63 | TMEM211 |

**General vesicle biomarker antibodies: CD9, CD63, CD81**
**Cell of Origin biomarker antibodies: PSCA, MFG-E8, Rab, STEAP, PCSA, PSMA, 5T4, TMEM211**
**Cancer biomarker antibodies: EpCam, B7H3, CD24**
**Control antibodies: Rab IgG, IgG**

## FIG. 2C

FIG. 2D

**FIG. 2E**

**FIG. 2F**

SEQ ID NO. 1

**FIG. 3A**

SEQ ID NO. 3840

**FIG. 3B**

FIG. 4

**FIG. 5**

# Aptamer 4   (SEQ ID NO. 1)

FIG. 6

EP 3 039 174 B1

**A)** SEQ ID No. 230822

5′-ATCCAGAGTGACGCAGCAGTCTTTTCTGATGGACAC
GTGGTGGTCTAGTATCTGGACACGGTGGCTTAGT

**B)**

SEQ ID No. 230847

**FIGs. 7A-B**

EP 3 039 174 B1

FIG. 7C

FIG. 7D

FIG. 7E

EP 3 039 174 B1

**FIG. 7F**

EP 3 039 174 B1

**FIG. 7G**

**FIG. 7H**

EP 3 039 174 B1

FIG. 7I

FIG. 7J

A) Direct conjugation of cMVs to a substrate

substrate

cMV

B) Biotin functionalized lipid crosslinking of cMVs to a substrate

substrate

cMV

Streptavidin

Lipid chains

FIGs. 8A-B

EP 3 039 174 B1

C) Immuno-precipitation of cMVs with cMV specific antibodies immobilized to a substrate

substrate
antibody
cMV

D) Immuno-precipitation of cMVs with cMV specific aptamer immobilized to a substrate

substrate
aptamer
cMV

**FIGs. 8C-D**

FIG. 9

FIG. 10A

FIG. 10B

**FIG. 10C**

Cancer Microvesicles selection round 1

Selection A (Cancer ("C"))

Ca1 > Ca2 > Ca3 > (Ca4) > Ca5 > Ca6 > Ca7 > Ca8 > Ca9 > Ca10 > Ca11 > Ca12 > Ca13 •

non-Cancer Microvesicles selection round 1

Selection A (non-Cancer ("N"))

nCa1 > nCa2 > nCa3 > (nCa4) > nCa5 > nCa6 > nCa7 > nCa8 > nCa9 > nCa10 > nCa11 > nCa12 > nCa13 •

Cancer/no Microvesicles negative selection

Selection B (Cancer)

noE > Ca4 > noE > Ca5 > noE > Ca6 > noE > Ca7 > noE > Ca8 > noE > (Ca9) > noE > Ca10 > noE > Ca11 > noE > Ca12 •

non-Cancer/no Microvesicles negative selection

Selection B (non-Cancer)

noE > nCa4 > noE > nCa5 > noE > nCa6 > noE > nCa7 > noE > nCa8 > noE > nCa9 > noE > nCa10 > noE > nCa11 > noE > nCa12 •

**FIG. 11A**

FIG. 11B

**Enrichment on Microvesicles Beads (% bound)**

**FIG. 11C**

EP 3 039 174 B1

# Selection against Cancer derived microvesicles

## SELEX 'B'

R8 RNA library

For Round 9 **cancer exosome** selected library

Negative selection (cancer or non cancer non-exosome coated beads)

Unbound | aptamer used

Positive selection (cancer or non-cancer exosome coated beads)

Bound | aptamers eluted & saved

RT-PCR= **Round 9**

2'Fluoro Transcription

R9C sample

A. Cancer non-exosome beads
or
B. Non-cancer exosome beads
or
C. Non-cancer no-exosome beads

Bound aptamers eluted and RT-PCR amplified
= 9*C A
= 9*C B
= 9*C C

SUL1 RNA library

# FIG. 11D

EP 3 039 174 B1

# Selection against Non-Cancer derived microvesicles

## SELEX 'B'

R8 RNA library

↓

Negative selection (cancer or non-cancer non-exosome coated beads)

Unbound | aptamer used

↓

Positive selection (cancer or non-cancer exosome coated beads)

Bound | aptamers eluted & saved

RT-PCR= **Round 9**

↓

2'Fluoro Transcription

For Round 9 **non-cancer exosome** selected library

R9N sample

↓

D. Cancer exosome beads

or

E. Cancer no-exosome beads

or

F. Non-cancer no-exosome beads

↓

Bound aptamers eluted and RT-PCR amplified
= 9*N D
= 9*N E
= 9*N F

SUL1 RNA library

## FIG. 11E

# Additional analyses on R9 SELEX B (Cancer cMVs)

EP 3 039 174 B1

Same for non-Cancer

NGS → 9C          Bind to non-Cancer microvesicles          NGS → 9N

Bind to Cancer/no microvesicles → | Ca/noE > • | → NGS → 9C-A          Bind to Cancer microvesicles          NGS → 9N-D

Bind to non-Cancer/ microvesicles → | nCa > • | → NGS → 9C-B          Bind to Cancer/no microvesicles          NGS → 9N-E

Bind to non-Cancer/no microvesicles → | nCa/NoE > • | → NGS → 9C-C          Bind to non Cancer/no microvesicles          NGS → 9N-F

**FIG. 11F**

# SELEX C

Selection C (Cancer)

non-Cancer Microvesicle counter-selection

nCa > Ca10 > nCa4 > Ca11 > nCa4 > Ca12 > nCa4 > Ca13 > nCa4 > Ca14 •

• Counter-selection with non-Cancer microvesicles

EP 3 039 174 B1

**FIG. 11G**

**FIG. 12**

FIG. 13A

DNA     RNA     Protein     Protein Complexes

~20k genes     $10^5$ transcripts     $10^6$ isoforms     $10^7$-$10^8$ ???

MOLECULAR COMPLEXITY AND DIVERSITY

FIG. 13B

**FIG. 13C**

FIG. 13D

**FIG. 13E**

Sequencing

Oligonucleotide probe library

Target, e.g., Extracellular Vesicles

FIG. 13F

**FIG. 13G**

EP 3 039 174 B1

Probing Library 1311 + Sample 1312

1310

Bind 1313

Supernatant 1314

Microvesicle pellet 1315

Elute 1316

Sequencing 1317

Profile 1318

**FIG. 13H**

**1321**

Patient Sample

**1322**

TOP library

**1323**

Isolate MVs

**1324**

NGS, PCR, Array

Other

**FIG. 13I**

Population Specific to
Breast Cancer Plasma
(Retain)

Population Specific to
Non-cancer Control Sample/s
(Retain)

Population Present in Both
Cancer and Control Plasma

Discard

**FIG. 14A**

EP 3 039 174 B1

**FIG. 14B**

FIG. 14C

Sample measured at 2x input

Sample measured at 1x input

$y = 29.695x^{0.856}$
$R^2 = 0.99242$

Actual Input

Measured Output

1.0E+06  1.0E+07  1.0E+08  1.0E+09  1.0E+10

1.00E+06  1.00E+07  1.00E+08  1.00E+09  1.00E+10

**FIG. 14D**

**FIG. 14E**

FIG. 14F

**FIG. 14G**

FIG. 14H

**FIG. 14I**

FIG. 14J

FIG. 14K

**FIG. 14L**

FIG. 15A

FIG. 15B

FIG. 15C

**FIG. 15D**

FIG. 15E

FIG. 15F

FIG. 15G

FIG. 15H

FIG. 15I

FIG. 15J

**FIG. 15K**

FIG. 15L

FIG. 15M

FIG. 15N

FIG. 150

FIG. 15P

**FIG. 15Q**

FIG. 15R

**FIG. 15S**

FIG. 15T

**FIG. 15U**

**FIG. 15V**

**FIG. 15W**

**FIG. 15X**

**FIG. 15Y**

FIG. 15Z

**FIG. 15AA**

**FIG. 15AB**

FIG. 15AC

FIG. 15AD

FIG. 15AE

FIG. 15AF

FIG. 15AG

FIG. 15AH

**FIG. 15AI**

**FIG. 15AJ**

**FIG. 15AK**

**FIG. 15AL**

FIG. 15AM

FIG. 15AN

**FIG. 16A**

FIG. 16B

1610

Provide Aptamer Pool to Target of Interest 1611

Contact Pool with Sample 1612

Wash, Retain Aptamers that Bind Sample 1613

Identify Retained Aptamers 1614

Characterize Sample 1615

FIG. 16C

EP 3 039 174 B1

**FIG. 17A**

SEQ ID NO. 510769

FIG. 17B

**FIG. 17C**

**FIG. 17D**

**FIG. 17E**

**FIG. 17F**

FIG. 17H

FIG. 17G

## REFERENCES CITED IN THE DESCRIPTION

### Patent documents cited in the description

- WO 2010056337 A2
- US 53642890 A
- US 5475096 A
- US 5270163 A
- WO 9119813 A
- US 5958691 A
- US 5660985 A
- US 5698687 A
- US 5817635 A
- US 5672695 A
- WO 9207065 A
- US 5707796 A
- US 5763177 A
- US 5567588 A
- US 5861254 A
- US 5496938 A
- US 5705337 A
- US 5580737 A
- US 5756703 A
- US 5637459 A
- US 5683867 A
- US 6011020 A
- US 6051698 A
- WO 9818480 A
- US 5648214 A
- US 43076102 P
- US 48747403 P
- US 51703903 P
- US 72958103 A
- US 87385604 A
- US 65877010 A
- WO 2010093465 PCT
- WO 2011056688 A
- US 61427788 A
- WO 2011127219 A
- US 20050158708 A
- US 4275149 A
- US 4737456 A
- US 6329209 B
- US 6365418 B
- US 6406921 B
- US 6475808 B
- US 6475809 B
- US 884269 A
- US 4551435 A
- US 4795698 A
- US 4925788 A
- US 5108933 A

- US 5186827 A
- US 5200084 A
- US 5158871 A
- US 7399632 B
- US 8124015 B
- US 8008019 B
- US 7955802 B
- US 7445844 B
- US 7274316 B
- US 6773812 B
- US 6623526 B
- US 6599331 B
- US 6057107 A
- US 5736330 A
- WO 2012174282 A
- WO 1993022684 A
- US 7591936 B
- US 7581429 B
- US 7579136 B
- US 7575722 B
- US 7568399 B
- US 7552741 B
- US 7544506 B
- US 7541578 B
- US 7518726 B
- US 7488596 B
- US 7485214 B
- US 7467928 B
- US 7452713 B
- US 7452509 B
- US 7449096 B
- US 7431887 B
- US 7422725 B
- US 7422669 B
- US 7419822 B
- US 7419639 B
- US 7413709 B
- US 7411184 B
- US 7402229 B
- US 7390463 B
- US 7381471 B
- US 7357864 B
- US 7351592 B
- US 7351380 B
- US 7338637 B
- US 7329391 B
- US 7323140 B
- US 7261824 B
- US 7258837 B

- US 7253003 B
- US 7238324 B
- US 7238255 B
- US 7233865 B
- US 7229538 B
- US 7201881 B
- US 7195986 B
- US 7189581 B
- US 7189580 B
- US 7189368 B
- US 7141978 B
- US 7138062 B
- US 7135147 B
- US 7125711 B
- US 7118910 B
- US 7118661 B
- US 7640947 B
- US 7666361 B
- US 7704735 B
- WO 2010072410 A
- US 5376252 A
- US 6408878 B
- US 6645432 B
- US 6719868 B
- US 6793753 B
- US 6899137 B
- US 6929030 B
- US 7040338 B
- US 7144616 B
- US 7216671 B
- US 7250128 B
- US 7494555 B
- US 7501245 B
- US 7601270 B
- US 7691333 B

- US 7754010 B
- US 7837946 B
- US 20030061687 A
- US 20050084421 A
- US 20050112882 A
- US 20050129581 A
- US 20050145496 A
- US 20050201901 A
- US 20050214173 A
- US 20050252773 A
- US 20060006067 A
- EP 0527905 A
- EP 1065378 A
- US 4666828 A
- US 4683202 A
- US 4801531 A
- US 5192659 A
- US 5272057 A
- US 7751053 B
- US 7399600 B
- US 6899863 B
- US 6812023 B
- US 7198923 B
- WO 2014082083 A
- US 63294605 A
- US 6269957 B
- US 6357601 B
- WO 2011066589 A
- WO 2010065765 A
- WO 2010141862 A
- WO 2007103572 A
- US 2012042519 W
- US 2012050030 W
- WO 2013022995 A

**Non-patent literature cited in the description**

- **TUCKER et al.** *J. Chromatography B.,* 1999, vol. 732, 203-212
- **FROEHLER et al.** *Nucl. Acid Res.,* 1986, vol. 14, 5399-5467
- **FROEHLER et al.** *Tet. Lett.,* 1986, vol. 27, 5575-5578
- **SOOD et al.** *Nucl. Acid Res.,* 1977, vol. 4, 2557
- **HIROSE et al.** *Tet. Lett.,* 1978, vol. 28, 2449
- **SPROAT et al.** *Nucl. Acid Res.,* 1991, vol. 19, 733-738
- **COTTEN et al.** *Nucl. Acid Res.,* 1991, vol. 19, 2629-2635
- **HOBBS et al.** *Biochemistry,* 1973, vol. 12, 5138-5145
- **ROKHLIN et al.** 5E10: a prostate-specific surface-reactive monoclonal antibody. *Cancer Lett.,* 1998, vol. 131, 129-36

- The Handbook--A Guide to Fluorescent Probes and Labeling Technologies
- **MERE L et al.** Miniaturized FRET assays and microfluidics: key components for ultra-high-throughput screening. *Drug Discovery Today,* 1999, vol. 4 (8), 363-369
- **LAKOWICZ J R.** Principles of Fluorescence Spectroscopy. Plenum Press, 1999
- Proteomics of Human Body Fluids: Principles, Methods and Applications. **JAIN KK:.** Integrative Omics, Pharmacoproteomics, and Human Body Fluids. Humana Press, 2007, vol. 1
- Proteomics of Human Body Fluids: Principles, Methods and Applications. **JAIN KK.** Integrative Omics, Pharmacoproteomics, and Human Body Fluids. Humana Press, 2007, vol. 1
- **UNGER MET.** *Biotechniques,* 1999, vol. 27 (5), 1008-14

- **KARTALOV EP et al.** *Biotechniques,* 2006, vol. 40 (1), 85-90
- **CUTILLAS et al.** *Proteomics,* 2005, vol. 5, 101-112
- **CUTILLAS et al.** *Mol Cell Proteomics,* 2005, vol. 4, 1038-1051
- **PIPPER et al.** *Angewandte Chemie,* 2008, vol. 47 (21), 3900-3904
- **LI et al.** *Adv Dent Res,* 2005, vol. 18 (1), 3-5
- **SRINIVAS et al.** Aptamer functionalized Microgel Particles for Protein Detection. *Anal. Chem.,* 21 October 2011
- **BRODY ; GOLD.** *Rev. Mol. Biotech.,* 2000, vol. 74, 5-13
- **ORMEROD.** Flow Cytometry. Springer-Verlag, 1999
- **NIDA et al.** *Gynecologic Oncology,* 2005, vol. 4, 889-894
- **CHEN et al.** Microfluidic isolation and transcriptome analysis of serum vesicles. *Lab on a Chip,* 08 December 2009
- Harrison's Principles of Internal Medicine. 2005
- **SAMBROOK et al.** Molecular Cloning: A Laboratory Manual. Cold Spring Harbor Laboratory Press, 1989
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- PCR Protocols: A Guide to Methods and Applications. Academic Press, 1990
- **THERY et al.** *Nat Rev Immunol.,* August 2009, vol. 9 (8), 581-93
- **KELLER et al.** *Immunol. Lett.,* 2006, vol. 107 (2), 102-8
- **CHARRAS et al.** *Nature Reviews Molecular and Cell Biology,* 2008, vol. 9 (11), 730-736
- **LIU et al.** TiGER: a database for tissue-specific gene expression and regulation. *BMC Bioinformatics.,* 2008, vol. 9, 271, dkfz-heidelberg.de/menu/tissue_db/index.html
- **KING et al.** *Breast Cancer Res,* 2005, vol. 7 (5), 198-204
- **CHROMY et al.** *J Proteome Res,* 2004, vol. 3, 1120-1127
- **ZHANG et al.** *Mol Cell Proteomics,* 2005, vol. 4, 144-155
- **PISITKUN et al.** *Proc Natl Acad Sci USA,* 2004, vol. 101, 13368-13373
- **GRANT, R. et al.** A filtration-based protocol to isolate human Plasma Membrane-derived Vesicles and exosomes from blood plasma. *J Immunol Methods,* 2011, vol. 371, 143-51
- **BOYD et al.** *Antimicrob Agents Chemother,* 1997, vol. 41 (7), 1521-1530
- **HAMMAR et al.** *Ann N Y Acad Sci,* 1994, vol. 724, 166-169
- **KAKU et al.** *Arch Biochem Biophys,* 1990, vol. 279 (2), 298-304
- **CHERVENAK et al.** *Biochemistry,* 1995, vol. 34 (16), 5685-5695
- **FAN et al.** Illumina universal bead arrays. *Methods Enzymol.,* 2006, vol. 410, 57-73
- **SRINIVAS et al.** Aptamerfunctionalized Microgel Particles for Protein Detection. *Anal. Chem.,* 21 October 2011
- **CHAUDRY MA ; SALES K ; RUF P ; LINDHOFER H ; WINSLET MC.** *Br. J. Cancer,* April 2007, vol. 96 (7), 1013-9
- **REFF ; HEARD.** *Critical Reviews in Oncology/Hematology,* 2001, vol. 40, 25-35
- **COLCHER, D. ; GOEL, A. ; PAVLINKOVA, G. ; BERESFORD, G. ; BOOTH, B. ; BATRA, S. K.** Effects of genetic engineering on the pharmacokinetics of antibodies. *Q. J. Nucl. Med.,* 1999, vol. 43, 132-139
- **HICKE, B. J. ; STEPHENS, A. W.** Escort aptamers: a delivery service for diagnosis and therapy. *J. Clin. Invest.,* 2000, vol. 106, 923-928
- **WILBUR ; LIPMAN.** *Proc Natl Acad Sci USA,* 1983, vol. 80, 726-30
- **ALTSCHUL S F et al.** *Nucleic Acids Res.,* 1997, vol. 25 (17), 3389-402
- **ALTSCHUL S F et al.** *J Mol. Biol.,* 1990, vol. 215 (3), 403-10
- **MATHEWS, D. ; SABINA, J. ; ZUCKER, M. ; TURNER, H.** Expanded sequence dependence of thermodynamic parameters provides robust prediction of RNA secondary structure. *J. Mol. Biol.,* 1999, vol. 288, 911-940
- **HOFACKER et al.** *Monatshefte f. Chemie,* 1994, vol. 125, 167-188
- **HOFACKER, I. L.** Vienna RNA secondary structure server. *Nucleic Acids Res.,* 2003, vol. 31, 3429-3431
- **SUCHANEK, M. et al.** Photo-leucine and photo-methionine allow identification of protein-protein interactions. *Nat. Methods,* 2005, vol. 2, 261-267
- **MÜLLER, J. et al.** Selection of high affinity DNA-aptamer for activated protein C using capillary electrophoresis. *Research in Pharmaceutical Sciences,* 2012, vol. 7.5, 987
- **CERCHIA, L. ; V. DE FRANCISCIS.** Nucleic Acid Aptamers Against Protein Kinases. *Current medicinal chemistry,* 2011, vol. 18.27, 4152-4158

- **WU, JIE et al.** Identification, Characterization and Application of a G-Quadruplex Structured DNA Aptamer against Cancer Biomarker Protein Anterior Gradient Homolog 2. *PloS ONE,* 2012, vol. 7.9, e46393

- **MITKEVICH, OLGA V. et al.** DNA aptamers detecting generic amyloid epitopes. *Prion,* 2012, vol. 6.4, 400-406
- **KAUR H ; YUNG L-YL.** Probing High Affinity Sequences of DNA Aptamer against VEGF. *PLoS ONE,* 2012, vol. 7 (2), e31196